# EUROPEAN PATENT APPLICATION

(11) **EP 1 947 193 A1**
(43) Date of publication of application: **23.07.2008**
(21) Application number: 07000920.4
(22) Date of filing: 17.01.2007
(51) Int. Cl.: C12Q 1/48, G01N 33/50, G01N 33/74

(54) **Screening method for anti-diabetic compounds**

(71) Applicant: Max-Planck-Gesellschaft zur Förderung der Wissenschaften e.V., 80539 München (DE)
(72) Inventor: Lammert, Eckhard, 01097 Dresden (DE); Konstantinova, Irena, 01309 Dresden (DE)
(74) Representative: Vossius & Partner

(57) **Abstract**

The present invention relates to a method of identifying a compound suitable as a lead compound and/or as a medicament for the treatment and/or prevention of diabetes, the method comprising the steps of (a) contacting a test compound with a cell comprising a protein, wherein said protein (i) comprises or consists of the amino acid sequence of any one of SEQ ID NOs: 1 to 29; (ii) is encoded by a nucleic acid molecule comprising or consisting of the sequence of any one of SEQ ID NOs: 59 to 87; (iii) is a fragment of the protein according to (i) or (ii) and exhibits Eph-Receptor-Tyrosine-Kinase activity; or (iv) has a sequence at least 75% identical with the protein according to (i) or (ii) or with the fragment according to (iii) and exhibits Eph-Receptor-Tyrosine-Kinase activity; and (b) determining whether said test compound, upon contacting in step (a) inhibits the Eph receptor tyrosine kinase activity of said protein wherein said inhibition indicates a compound suitable as a lead compound and/or as a medicament for the treatment and/or prevention of diabetes.

## Description

The present invention relates to a method of identifying a compound suitable as a lead compound and/or as a medicament for the treatment and/or prevention of diabetes, the method comprising the steps of (a) contacting a test compound with a cell comprising a protein, wherein said protein (i) comprises or consists of the amino acid sequence of any one of SEQ ID NOs: 1 to 29; (ii) is encoded by a nucleic acid molecule comprising or consisting of the sequence of any one of SEQ ID NOs: 59 to 87; (iii) is a fragment of the protein according to (i) or (ii) and exhibits Eph-Receptor-Tyrosine-Kinase activity; or (iv) has a sequence at least 75% identical with the protein according to (i) or (ii) or with the fragment according to (iii) and exhibits Eph-Receptor-Tyrosine-Kinase activity; and (b) determining whether said test compound, upon contacting in step (a) inhibits the Eph receptor tyrosine kinase activity of said protein wherein said inhibition indicates a compound suitable as a lead compound and/or as a medicament for the treatment and/or prevention of diabetes.

Throughout this specification, several documents are cited. The disclosure content of these documents is herewith incorporated by reference (including all product descriptions and manufacturers' instructions).

The treatment of type II diabetes mellitus will be a major challenge in the near future since its global prevalence has dramatically increased over the past years and is expected to increase further. Furthermore, the fact that the pathogenic basis of this disease is complex and not well understood requires multiple therapies to be employed. Owing to estimations, which expect the number of patients suffering from type II diabetes to increase from currently 190 million to over 350 million individuals worldwide within the next 15 to 20 years, there is an urgent need for effective therapeutics. This increase is supposed to be caused by a number of factors including global population growth, aging, increasing urbanization and increased obesity. The aforementioned pathophysiologic complexity renders type II diabetes mellitus a heterogeneous disease where therapeutic interventions are complex and difficult. The rise in the prevalence of obesity appears to be closely associated with an increased insulin resistance in the population. Therefore, the improvement of insulin sensitivity is thought to be a promising approach for treatment. However, besides reduced insulin sensitivity, it appears that also impairment of beta-cell function is involved in the pathogenesis of type II diabetes. It has been shown that impaired glucose regulation is associated with defects of both insulin secretion and action. In light of these discoveries, the use of therapeutic agents that are capable of improving the glucose-mediated insulin secretion is desired. Up to now, sulfonylureas have been considered to be the first-line monotherapy of choice. Also their combination with other oral anti-diabetics is suggested by many guidelines on the pharmacological treatment of diabetes worldwide although they have been discovered more than 50 years ago (Prato and Pulizzi (2006) Metabolism 55, 20). Incretins such as glucagon-like peptide-1 (GLP-1), and dipeptidyl peptidase-4 (DPP-4) inhibitors, are currently developed as alternative treatments of type II diabetes with fewer side effects (Drucker and Nauck (2006) Lancet 368, 1696).

As evident from the foregoing, proper insulin secretion is crucial to the body's ability to cope with blood glucose. Insulin is the only blood glucose-lowering hormone and is secreted by the pancreatic beta-cells of the islets of Langerhans. After its synthesis, the hormone is processed into its biologically active form and stored in vesicles ready for release. In the event insulin secretion becomes necessary, the hormone is released via a complex process involving the interplay of several proteins with the vesicle (Rorsman and Renström (2003) Diabetologia 46, 1029).

Thus, pancreatic islets are strictly required for maintaining glucose homeostasis, and defects in their ability to adequately secrete insulin in response to glucose result in diabetes mellitus (Bell and Polonsky (2001) Nature 414, 788).

During embryonic development, beta-cells initially develop as scattered single cells (Lammert et al. (2001) Science 294, 564). However, they do not stay single, but aggregate with other beta-cells and endocrine cells to form pancreatic islets. While glucose metabolism cell-autonomously triggers insulin secretion (Maechler and Wollheim (2001) 414, 807), communication between beta-cells suppresses basal insulin secretion, but enhances glucose-stimulated insulin secretion. It therefore ensures that beta-cells secrete low amounts of insulin during times of starvation, but sufficient insulin after food up-take. In comparison with intact islets, dispersed islet cells display increased basal insulin secretion and decreased glucose-stimulated insulin secretion (Halban et al. (1982) Endocrinol. 111, 86; Bosco et al. (1989) Exp. Cell Res. 184, 72; Matta et al. (1994) Pancreas 9, 439; Hopcroft et al. (1985) Endocrinol. 117, 2073). Conversely, reaggregation of islet cells suppresses basal insulin secretion and enhances glucose-stimulated insulin secretion (Halban et al. (1982) Endocrinol. 111, 86; Maes and Pipeleers (1984) Endocrinol. 114, 2205; Meda et al. (1990) J. Clin. Invest. 86, 759). Similarly, aggregation of immortalized beta-cell lines, such as mouse or rat insulinoma cells, enhances glucose - stimulated insulin secretion (Luther et al. (2006) Biochem. Biophys. Res. Commun. 343, 99). Finally, inhibition of adherent junctions and gap junctions in beta-cells provides evidence that direct cell-cell communication between beta-cells is required for physiological insulin secretion and glucose homeostasis (Dahl et al. (1996) Development 1222, 2895; Hauge-Evans et al. (1999) Diabetes 48, 1402; Yamagata et al. (2002) Diabetes 51, 114; Ravier et al. (2005) Diabetes 54, 1798). In spite of all these observations, the molecular mechanism that allows beta-cell communication to suppress basal insulin secretion, but enhance glucose-stimulated insulin secretion remains elusive.

In view of the increasing prevalence of type II diabetes mellitus in the world's population, there is an increasing need for an appropriate treatment suitable to overcome the limitations of current therapeutics. Thus, the technical problem underlying the present invention is the provision of novel means and methods useful in the treatment and/or prevention of type II diabetes mellitus.

The solution to this problem is achieved by providing the embodiments as characterized in the claims.

Accordingly, the present invention relates to a method of identifying a compound suitable as a lead compound and/or as a medicament for the treatment and/or prevention of diabetes, the method comprising the steps of (a) contacting a test compound with a cell comprising a protein, wherein said protein (i) comprises or consists of the amino acid sequence of any one of SEQ ID NOs: 1 to 29; (ii) is encoded by a nucleic acid molecule comprising or consisting of the sequence of any one of SEQ ID NOs: 59 to 87; (iii) is a fragment of the protein according to (i) or (ii) and exhibits Eph-Receptor-Tyrosine-Kinase activity; or (iv) has a sequence at least 75% identical with the protein according to (i) or (ii) or with the fragment according to (iii) and exhibits Eph-Receptor-Tyrosine-Kinase activity; and (b) determining whether said test compound, upon contacting in step (a) inhibits the Eph receptor tyrosine kinase activity of said protein wherein said inhibition indicates a compound suitable as a lead compound and/or as a medicament for the treatment and/or prevention of diabetes.

In accordance with the present invention, the term "compound" denotes, in one alternative a small molecule. Such a small molecule may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively the compound may be an inorganic compound. Inorganic compounds are derived from mineral sources and include all compounds without carbon atoms (except carbon dioxide, carbon monoxide and carbonates). Alternatively, the compound may be a macromolecule of natural or synthetic origin. Natural macromolecules are, for example peptides, proteins such as antibodies, nucleic acid molecules such as DNA, RNA or apatmers or polysaccharides. Synthetic macromolecules are for example polymers consisting of covalently linked small organic molecules.

Small molecules have a molecular weight below 10000 Da, preferably less than 1000 Da, more preferably less than 500 Da and most preferably between 200 Da and 400 Da. Macromolecules have a molecular weight in the range of a few thousand up to several million Da. Preferably their molecular weight is more than 10000 Da, more preferably more than 100000 Da and most preferably between 150000 Da and 250000 Da.

"Nucleic acid molecules", in accordance with the present invention, include DNA, such as cDNA or genomic DNA, and RNA. It is understood that the term "RNA" as used herein comprises all forms of RNA including mRNA, ncRNA (non-coding RNA), tRNA and rRNA. The term "non-coding RNA" includes siRNA (small interfering RNA), miRNA (micro RNA), rasiRNA (repeat associated RNA), snoRNA (small nucleolar RNA), and snRNA (small nuclear RNA). Further included are nucleic acid mimicking molecules known in the art such as synthetic or semisynthetic derivatives of DNA or RNA and mixed polymers, both sense and antisense strands. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey (2001) Chem Biol. 8, 1). LNA is an RNA derivative in which the ribose ring is constrained by a methylene linkage between the 2'-oxygen and the 4'-carbon. They may contain additional non-natural or derivatized nucleotide bases, as will be readily appreciated by those skilled in the art.

The term "antibody", in accordance with the present invention, comprises polyclonal and monoclonal antibodies as well as derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The antibody of the invention also includes embodiments such as chimeric, single chain and humanized antibodies, as well as antibody fragments, like, inter alia, Fab fragments, as well as fusion proteins consisting of Eph, ephrin or phosphatase extracellular domains and Fc. Antibody fragments or derivatives further comprise F(ab')₂, Fv or scFv fragments; see, for example, Harlow and Lane (1988) and (1999), loc. cit. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals may be used to express humanized antibodies specific for polypeptides and fusion proteins of this invention. Most preferably, the antibody of this invention is a monoclonal antibody. For the preparation of monoclonal antibodies, any technique, which provides antibodies produced by continuous cell line cultures, can be used. Examples for such techniques include the hybridoma technique (Köhler and Milstein (1975) Nature 256, 495), the trioma technique, the human B-cell hybridoma technique (Kozbor (1983) Immunology Today 4, 72) and the EBV-hybridoma technique to produce human monoclonal antibodies (Cole et al. (1985) Monoclonal Antibodies and Cancer Therapy, Alan R. Liss, Inc., 77). Surface plasmon resonance as employed in the BlAcore system can be used to increase the efficiency of phage antibodies, which bind to an epitope of a polypeptide of the invention (Schier (1996) Human Antibodies Hybridomas 7, 97; Malmborg (1995) J. lmmunol. Methods 183, 7). It is also envisaged in the context of this invention that the term "antibody" comprises antibody constructs, which may be expressed in cells, e.g. antibody constructs which may be transfected and/or transduced via, amongst others, viruses or plasmid vectors. The antibody described in the context of the invention is capable to specifically bind/interact with an epitope of Eph receptor tyrosine kinases. The term "specifically binding/interacting with" as used in accordance with the present invention means that the antibody does not or essentially does not cross-react with an epitope of similar structure. Thus, the antibody does not bind to prior art carboxylesterase of the present invention. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or do not essentially bind to any of the other epitope are considered specific for the epitope of interest and thus to be antibodies in accordance with this invention. Corresponding methods are described e.g. in Harlow and Lane, 1988 and 1999, loc cit. The antibody specifically binds to/interacts with conformational or continuous epitopes, which are unique Eph receptor tyrosine kinases. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela (1969) Science 166, 1365; Laver (1990) Cell 61, 553). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain.

The term "aptamer" in accordance with the present invention refers to DNA or RNA molecules that have been selected from random pools based on their ability to bind other molecules. Aptamers have been selected which bind nucleic acid, proteins, small organic compounds, and even entire organisms. A database of aptamers is maintained at http://aptamer.icmb.utexas.edu/.

The term "lead compound" in accordance with the present invention refers to a compound discovered with step (b) of the method of the invention which will be e.g. further optimized, in particular to be pharmaceutically more acceptable. The identified lead compounds may be optimized to arrive at a compound, which may be for example used in a pharmaceutical composition. Methods for the optimization of the pharmacological properties of compounds identified in screens, the lead compounds, are known in the art and comprise a method of modifying a compound identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiffs bases, oximes, acetales, ketales, enolesters, oxazolidines, thiazolidines or combinations thereof.

The various steps recited above are generally known in the art. They include or rely on quantitative structure-activity relationship (QSAR) analyses (Kubinyi (1992) "Hausch-Analysis and Related Approaches", VCH Verlag, Weinheim), combinatorial biochemistry, classical chemistry and others (see, for example, Holzgrabe and Bechtold (2000) Deutsche Apotheker Zeitung 140(8), 813).

The term "diabetes" as used throughout this specification refers to a disease in which the body does not properly control the amount of sugar in the blood. As a result, the level of sugar in the blood is too high. This disease occurs when the body does not produce enough insulin or does not use it properly. Diabetes is divided into two types, type I and type II diabetes. Type I diabetes has its onset in juvenile age and is characterized by a complete destruction of the islets of Langerhans. Type II diabetes correlates with obesity and insulin resistance, and has its onset in the adult age. It is characterized by an inability of the islets of Langerhans to secrete sufficient insulin to compensate for the body's increased demand for insulin.

The "cell" as recited throughout this specification refers to a primary cell or a cell from a cell line. Primary cells are cells which are directly obtained from an organism and which are not immortalized. Suitable primary cells are, for example, cells that are capable of secreting insulin such as pancreatic beta-cells from species such as mouse, rat, human, guinea pig, hamster, pig, dog, sheep, goat, donkey or cow, and are used either in the form of islets of Langerhans or isolated cells. The cell line may be, for example, selected from the rat insulinoma (RIN) cell lines such as INS-1 (Asfari et al. (1992) Endocrinol.130, 167), INS-2 (Asfari et al. (1992) Endocrinol.130, 167), RIN-r (Philippe et al. (1986) Endocrinol. 119, 2833) and RIN-m (Bathena et al. (1982) Diabetes 31, 521; Praz et al. (1983) Biochem. J. 210, 345; Philippe et al. (1987) J. Clin. Invest. 79, 351) or from the hamster insulinoma (HIT) cell lines such as HIT-T15 (Santerre et al. (1981) Proc. Natl. Acad. Sci. U.S.A. 78, 4339) or from mouse beta-cell lines expressing the SV40 large T-antigen (beta-TC lines) such as betaTC1, betaTC2, betaTC3 (Efrat et al. (1988) Proc. Natl. Acad. Sci. U.S.A. 85, 9037), betaTC6 (Poitout et al. (1995) Diabetes 44, 306), betaTC7 (Efrat et al. (1993) Diabetes 42, 901), betaTCtet (Efrat et al. (1995) Proc. Natl. Acad. Sci. U.S.A. 92, 3576) or from mouse insulinoma (MIN) cell lines such as MlN6 (Myazaki et al. (1990) Endocrinol. 127, 126).

As recited hereinabove, said cell comprises a protein wherein the protein comprises or consists of the amino acid sequence of any one of SEQ ID NOs: 1 to 29 or is encoded by a nucleic acid comprising or consisting of any one of SEQ ID NOs: 59 to 87. These sequences refer to the amino acid sequence or the nucleic acid sequence of different Eph receptor tyrosine kinases (see Table 1).

**Table 1: Listing of the different amino acid and nucleic acid sequences referred to in the present invention together with the corresponding GenBank Accession-No, species and gene name.**

| Name | Species | Accession No. | SEQ ID NO (amino acid sequence) | Accession No. | SEQ ID NO (nucleic acid sequence) |
|---|---|---|---|---|---|
| EphA1 | human | NP_005223 | 1 | NM_005232.3 | 59 |
| | mouse | NP_076069 | 2 | NM_023580.2 | 60 |
| | rat | XP_001072130 | 3 | XM_001072130 | 61 |
| EphA2 | human | NP_004422 | 4 | NM_004431.2 | 62 |
| | mouse | NP_034269 | 5 | NM_010139.2 | 63 |
| | rat | XP_001072610 | 6 | XM_001072610.1 | 64 |
| EphA4 | human | NP_004429 | 7 | NM_004438.3 | 65 |
| | mouse | NP_031962 | 8 | NM_007936.3 | 66 |
| | rat | XP_244186 | 9 | XM_244186.4 | 67 |
| EphA5 | human | NP_004430 | 10 | NM_004439.4 | 68 |
| | mouse | NP_031963 | 11 | NM_007937.2 | 69 |
| | rat | XP_001075222 | 12 | XM_001075222.1 | 70 |
| EphA7 | human | NP_004431 | 13 | NM_004440.2 | 71 |
| | mouse | NP_034271 | 14 | NM_010141.2 | 72 |
| EphA8 | human | NP_065387 | 15 | NM_020526.3 | 73 |
| | mouse | NP_031965 | 16 | NM_007939.1 | 74 |
| | rat | XP_001069777 | 17 | XM_001069777.1 | 75 |
| EphB2 | human | NP_004433 | 18 | NM_004442.6 | 76 |
| | mouse | NP_034272 | 19 | NM_010142 | 77 |
| | rat | XP_001069612 | 20 | XM_001069612.1 | 78 |
| EphB3 | human | NP_004434 | 21 | NM_004443.3 | 79 |
| | mouse | NP_034273 | 22 | NM_010143.1 | 80 |
| | rat | XP 221311 | 23 | XM_221311.4 | 81 |
| EphB4 | human | NP 004435 | 24 | NM_004444.4 | 82 |
| | mouse | NP_034274 | 25 | NM_010144.4 | 83 |
| | rat | XP_001069363 | 26 | XM_001069363.1 | 84 |
| EphB6 | human | NP_004436 | 27 | NM_004445.2 | 85 |
| | mouse | NP_031706 | 28 | NM_007680.2 | 86 |
| | rat | XP_001068490 | 29 | XM_001068490.1 | 87 |
| ephrin-A1 | mouse | NP_034237 | 30 | NM_010107.2 | 88 |
| | human | NP_004419 | 31 | NM_004428.2 | 89 |
| | rat | NP_446051 | 32 | NM_053599.2 | 90 |
| ephrin-A2 | mouse | NP_031935 | 33 | NM_007909.2 | 91 |
| | human | NP_001396 | 34 | NM_001405.2 | 92 |
| | rat | XP_001076723 | 35 | XM_001076723.1 | 93 |
| ephrin-A4 | mouse | NP_031936 | 36 | NM_007910.2 | 94 |
| | human | NP_005218 | 37 | NM_005227.2 | 95 |
| | rat | XP_001074474 | 38 | XM_001074474.1 | 96 |
| ephrin-A5 | human | NP_001953 | 39 | NM_001962.1 | 97 |
| | mouse | NP_997537 | 40 | NM_207654.1 | 98 |
| | rat | NP_446355 | 41 | NM_053903.1 | 99 |
| ephrin-B2 | mouse | NP_034241 | 42 | NM_010111.2 | 100 |
| | human | NP_004084 | 43 | NM_004093.2 | 101 |
| | rat | XP_001067856 | 44 | XM_001067856.1 | 102 |
| ephrin-B3 | human | NP_001397 | 45 | NM_001406.3 | 103 |
| | mouse | NP_031937 | 46 | NM_007911.3 | 104 |
| | rat | XP_001079433 | 47 | XM_001079433.1 | 105 |
| ACP-1d | human | NP_001035739 | 48 | NM_001040649.1 | 106 |
| ACP-1b | human | NP_009030 | 49 | NM_007099.2 | 107 |
| ACP-1c | human | NP_004291 | 50 | NM_004300.2 | 108 |
| ACP | mouse | NP_067305 | 51 | NM_021330.2 | 109 |
| ACP | rat | NP_067085 | 52 | NM_021262.2 | 110 |
| PTP-RO a | human | NP_109592 | 53 | NM_030667.1 | 111 |
| PTP-RO b | human | NP_002839 | 54 | NM_002848.2 | 112 |
| PTP-RO c | human | NP_109596 | 55 | NM_030671.1 | 113 |
| PTP-RO d | human | NP_109595 | 56 | NM_030670.1 | 114 |
| PTP-RO | mouse | NP_035346 | 57 | NM_011216.2 | 115 |
| PTP-D30 | rat | NP_059032 | 58 | NM_017336.1 | 116 |

It is of note that also all the sequences accessible through the GenBank Accession Nos. of Table 1 are within the scope of the present invention irrespective of whether the entry of the respective Accession No. is completely identical to the sequence displayed by the corresponding SEQ ID NO due to potential future updates in the database. Thus, this is to account for future corrections and modifications in the entries of GenBank, which might occur due to the continuing progress of science.

The term "fragment of the protein" as defined herein in connection with the various proteins refers to a portion of the protein comprising at least the amino acid residues necessary to maintain the biological activity of the protein.
In accordance with the invention, the term a "fragment of the protein... and exhibits Eph-Receptor-Tyrosine-Kinase activity" refers to a portion of the protein comprising at least the amino acid residues of the juxtamembrane region and kinase domain. Preferably, the fragment has a length of at least 200 amino acids, more preferably between 250 and 500 amino acids and most preferably between 300 and 350 amino acids. Furthermore, said fragment exhibits Eph-Receptor-Tyrosine-Kinase (Eph) activity. Eph-Receptor-Tyrosine-Kinase activity is characterized by transphosphorylation of Eph receptor intracellular domains and results in F-actin polymerization and suppression of Rac1 activity via GTP-exchange factors (GEFs) (Murai and Pasquale (2005) Neuron 46, 161).

Also encompassed by the present invention are sequences that at least exhibit 75% identity with the above-recited protein. Preferably, the identity is between 75 and 98% such as at least 80%, more preferred at least 90%, more preferred 95% and most preferred the identity is at least 98%. Such molecules may be splice forms, homologous molecules from other species, such as orthologs, or mutated sequences from the same species to mention the most prominent examples. To evaluate the identity level between two nucleotide or protein sequences, they can be aligned electronically using suitable computer programs known in the art. Such programs comprise BLAST (Altschul et al. (1990) J. Mol. Biol. 215, 403), variants thereof such as WU-BLAST (Altschul and Gish (1996) Methods Enzymol. 266, 460), FASTA (Pearson and Lipman (1988) Proc. Natl. Acad. Sci. USA 85, 2444) or implementations of the Smith-Waterman algorithm (SSEARCH, Smith and Waterman (1981) J. Mol. Biol., 147, 195). These programs, in addition to providing a pairwise sequence alignment, also report the sequence identity level (usually in percent identity) and the probability for the occurrence of the alignment by chance (P-value). Programs such as CLUSTALW (Higgins et al. (1994) Nucleic Acids Res. 22, 4673) can be used to align more than two sequences.

In accordance with the present invention an "Eph receptor tyrosine kinase" is a protein comprising extracellularly an ephrin-binding domain and two fibronectin type-III repeats, and a cytosolic portion comprising a juxtamembrane region, kinase domain, SAM (sterile alpha-motif) domain and a PSD-95,Deg,ZO-1/2 (PDZ) binding motif. These two portions are linked via a transmembrane domain. "Eph receptor tyrosine kinase activity" is, in accordance with the present invention, characterized by transphosphorylation (transfer of a phosphate group from a kinase molecule to another kinase molecule). Eph receptor tyrosine kinase activity is also characterized by interactions of Eph receptor tyrosine kinases with other proteins such as PDZ-domain-containing proteins and Rho-family guanine nucleotide exchange factors. Lastly, as discovered in the present invention, Eph receptor tyrosine kinase activity is characterized by decreased insulin secretion and Rac1 activity, as well as increased F-actin content.

"Inhibition of Eph receptor tyrosine kinase activity" is defined as a reduction of the above defined Eph receptor tyrosine kinase activity by performing one or more of the following effects: (i) the transcription of the gene encoding the protein to be inhibited is lowered, i.e. the level of mRNA is lowered, (ii) the translation of the mRNA encoding the protein to be inhibited is lowered, (iii) the protein performs its biochemical function with lowered efficiency in presence of the inhibitor, and (iv) the protein performs its cellular function with lowered efficiency in presence of the inhibitor.

In a preferred embodiment, the level of activity is less than 90%, more preferred less than 80%, 70%, 60% or 50% of the activity in absence of the inhibitor. Yet more preferred are inhibitors lowering the level down to less than 25%, less than 10%, less than 5% or less than 1% of the activity in absence of the inhibitor.

In case inhibition is the decrease of expression level of the protein, determination of the expression level of a protein can for example be carried out on the nucleic acid or protein level.

Methods for determining the expression of a protein on the nucleic acid level include, but are not limited to, northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, the person skilled in the art knows how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidiumbromide or SybrGreen. A lower band intensity of the sample derived from the cell treated with the test compound as compared to a non-treated cell indicates a compound that inhibits the protein.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real time RT-PCR experiments is a DNA intercalating dye such as SybrGreen for monitoring the de novo synthesis of double stranded DNA molecules.

Methods for the determination of the expression of a protein on the amino acid level include but are not limited to western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest, here the Eph receptor tyrosine kinase. After washing, a second antibody specifically recognizing the first antibody and carrying a readout system such as a fluorescent dye is applied. The amount of the protein of interest is determined by comparing the fluorescence intensity of the protein derived from the cell treated with the test compound and the protein derived from a non-treated cell. A lower fluorescence intensity of the protein derived from the cell treated with the test compound indicates an inhibitor of the protein. Also of use in protein quantification is the Agilent Bioanalyzer technique.

In accordance with the present invention the term "determining whether said test compound inhibits the Eph receptor tyrosine kinase activity" refers to the determination of secreted insulin from said cell. Furthermore, it also refers to determination of Eph receptor phosphorylation, F-actin polymerization, Rac1 activity and cell-cell adhesion.

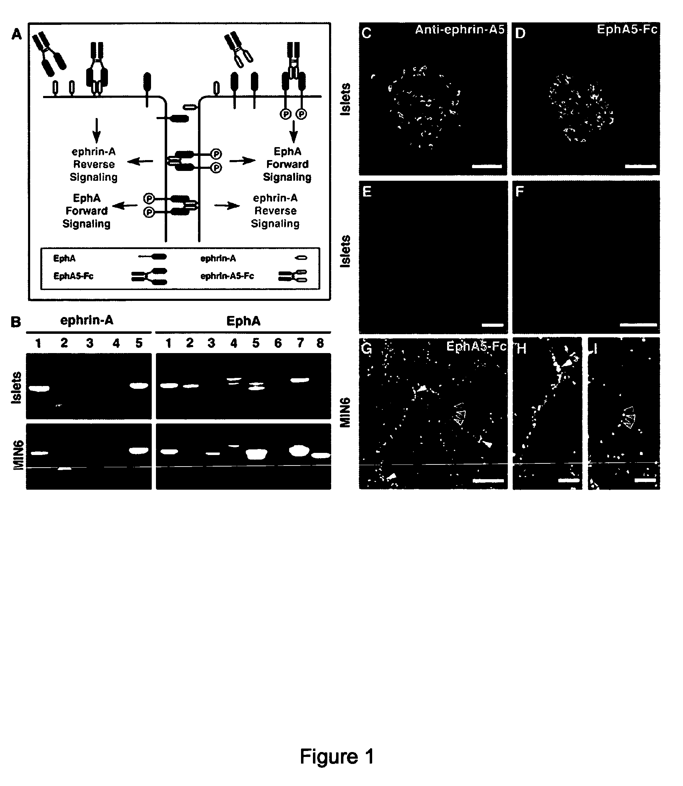

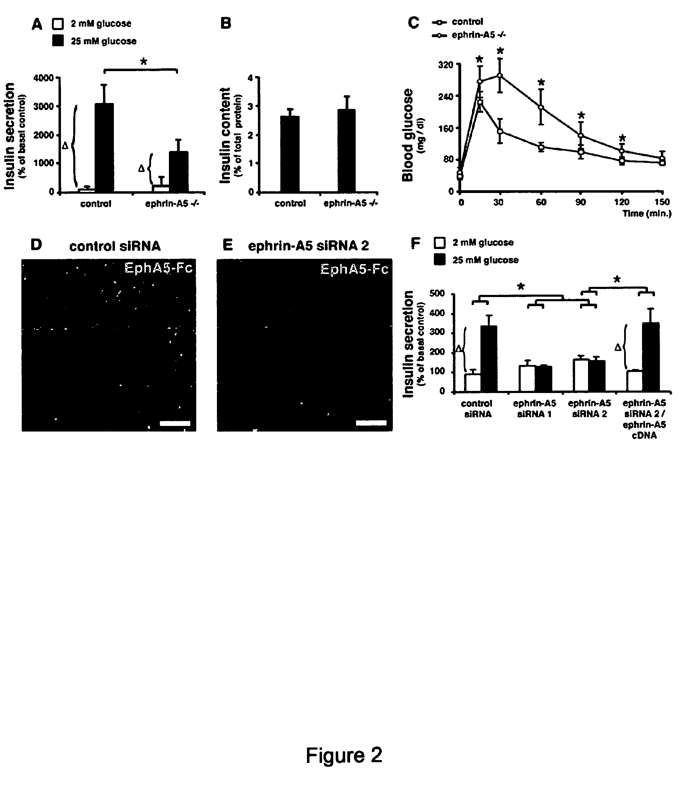

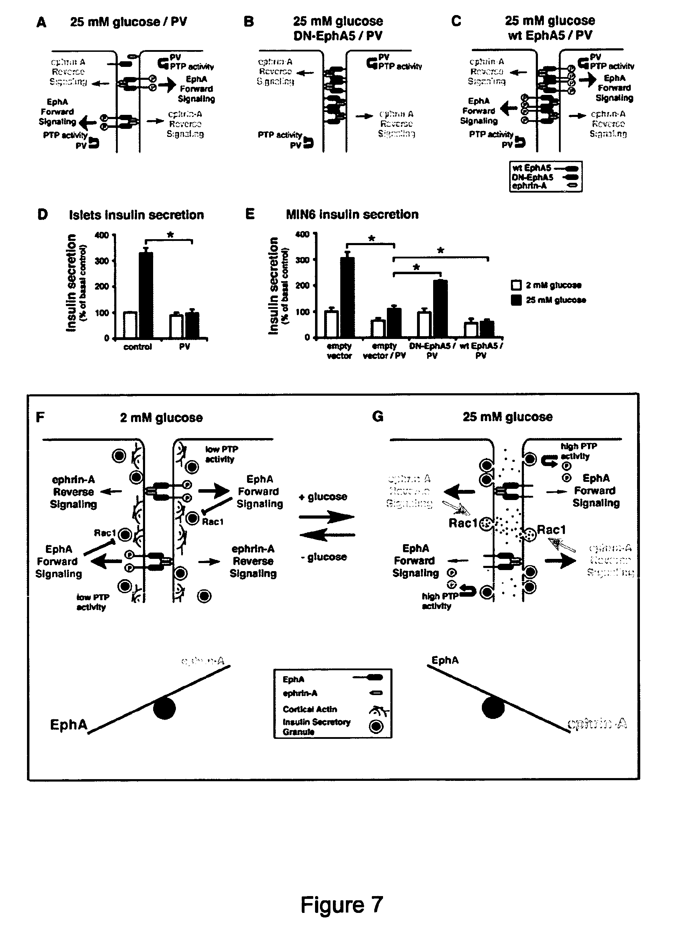

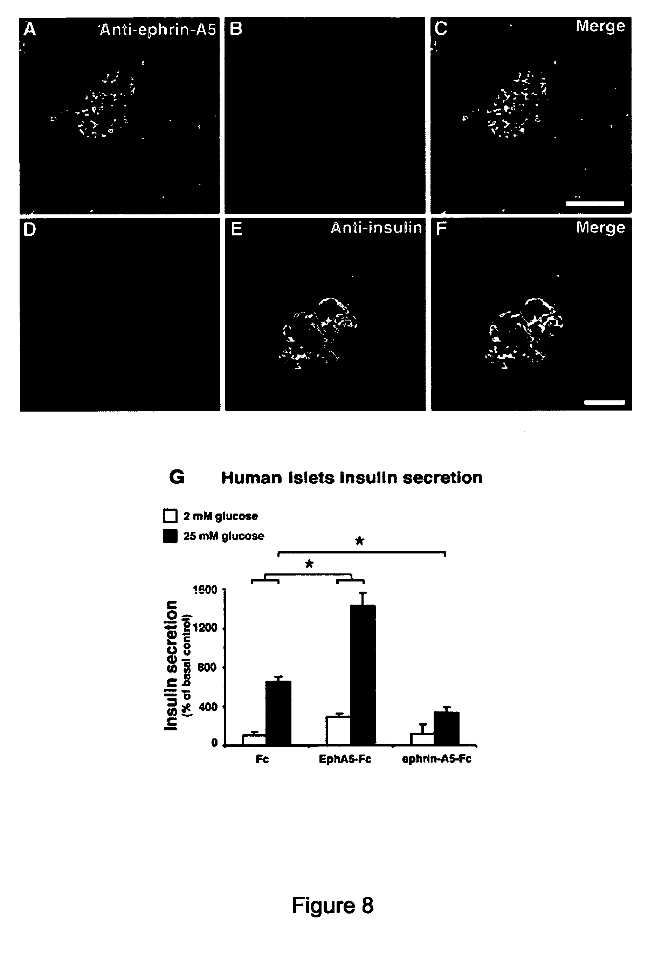

In accordance with the present invention, it was surprisingly found that, whereas the prior art provides no indication of the involvement of Eph-ephrin signalling in insulin secretion and glucose homeostasis, the interplay between Eph receptor tyrosine kinases and their corresponding ephrin ligands regulates insulin secretion in aggregated pancreatic beta-cells. The present invention for the first time shows that it is the communication between pancreatic beta-cells via Eph receptor tyrosine kinases and their ligands, the ephrins, which accounts for an optimal insulin secretory response to glucose, i.e. suppression of basal insulin secretion and enhancement of glucose-stimulated insulin secretion. Ephs and ephrins exhibit bidirectional signalling. Signalling events occur through both of the molecules. Conventionally, signalling through Ephs is referred to as forward signalling whereas signalling through ephrins is referred to as reverse signalling. Until the discoveries of the present invention, Ephs and ephrins were believed to be mainly involved in morphogenesis and pattern formation or in cell fate decisions (Pasquale (2005) Nat Rev Mol Cell Biol 6, 462). Ephs and ephrins are both present in mouse pancreatic islet cells, MIN6 cells (Example 1 and Figure 1) as well as human pancreatic islets (Example 1 and Figure 8). As evident from Example 2, ephrin-A5 is required for the regulation of insulin secretion from pancreatic beta-cells (see also Figure 2). It could further be shown that Eph forward signalling, in contrast to ephrin reverse signalling, significantly decreases insulin secretion in pancreatic islets and beta-cells (see Examples 3, 5 and 7). Further evidence for the involvement of Eph in glucose stimulated insulin secretion is corroborated by phosphorylation level changes of Eph upon glucose stimulation (see Examples 9 and 10). Eph is dephosphorylated under glucose stimulation and this dephosphorylation is required for insulin secretion under stimulatory conditions. Whereas the applicants do not wish to be bound by any scientific theory, a model as outlined in Figure 7 is proposed explaining the mechanisms underlying Eph-ephrin interactions in glucose-mediated insulin secretion. Thus, Eph receptor tyrosine kinases offer a new possibility to screen for anti-diabetic compounds as recited hereinabove.

Accordingly, the compounds identified by the above-recited method are capable of inhibiting transphosphorylation of Eph receptor tyrosine kinases. Said inhibition may be carried out, for example, by binding to the extracellular ephrin-binding site and thereby blocking said binding site without inducing the kinase activity. Inhibition may also be carried out by blocking the intracellular kinase domains of the Eph receptor tyrosine kinase and thereby preventing transphosphorylation of the receptor upon binding of the ligand. In both cases, no signalling will occur and consequently no inhibition of insulin secretion will take place. Thus, compounds that inhibit Eph receptor tyrosine kinase activity will also and in accordance with findings of the present invention increase insulin secretion. Based on the reported interaction of Eph receptors with Rho GTP-exchange factors (GEFs) (Murai and Pasquale (2005) Neuron 46, 161), an inhibition of such GEFs is also anticipated to increase insulin secretion.

To exert the above described effects, the compounds identified with the method of the present invention may act extracellularly or intracellularly. Compounds acting intracellularly may for example be an siRNA regulating down the expression level of the protein or compounds acting via inhibition of the cytosolic kinase domains. Compounds exerting their effects extracellularly may, for example, be compounds interfering with ephrin binding to the extracellular part of the Eph receptor tyrosine kinase. One example of such a compound may be an aptamer binding to the ephrin binding site.

Suitably, the method of the present invention is carried out in vitro. In vitro methods offer the possibility of establishing high-throughput assays, which are capable of screening up to several thousand compounds in parallel. High-throughput assays, independently of being biochemical, cellular or other assays, generally may be performed in wells of microtitre plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example, 10, 20, 30, 40, 50 or 100 test compounds may be added to each well. In case a well shows inhibition of Eph receptor tyrosine kinase activity, said mixture of test compounds may be de-convoluted to identify the one or more test compounds in said mixture giving rise to said inhibition.

In another embodiment, the present invention relates to a method of identifying a compound suitable as a lead compound and/or as a medicament for the treatment and/or prevention of diabetes, the method comprising the steps of (a) contacting a test compound with a protein, wherein said protein (i) comprises or consists of the amino acid sequence of any one of SEQ ID NOs: 1 to 29, or (ii) is encoded by a nucleic acid molecule comprising or consisting of the sequence of any one of SEQ ID NOs: 59 to 87, or (iii) is a fragment of the protein according to (i) or (ii) and exhibits Eph-Receptor-Tyrosine-Kinase activity, or (iv) has a sequence at least 75% identical with the protein according to (i) or (ii) or with the fragment according to (iii) and exhibits Eph-Receptor-Tyrosine-Kinase activity; and (b) determining whether said test compound, upon contacting in step (a) inhibits the Eph receptor tyrosine kinase activity of said protein wherein said inhibition indicates a compound suitable as a lead compound and/or as a medicament for the treatment and/or prevention of diabetes.

The present invention thus also provides a biochemical assay for assessing the anti-diabetic properties of a given compound. Biochemical assays offer the advantage of being carried out without the need for extensive cell culture work as compared to a cellular assay as recited above. The protein may be purified from a natural source such as tissue obtained from a mammal like mouse, rat or human. Protein purification techniques are well known to those skilled in the art. Alternatively, the protein may be recombinantly expressed in bacteria or in a cell culture. For example, nucleic acid sequences comprising a nucleic acid molecule encoding the protein can be synthesized by PCR and inserted into an expression vector (for a detailed description of the PCR process see below). Non-limiting examples of expression vectors include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146) or pBC12MI (ATCC 67109). Also suitable are prokaryotic plasmid vectors such as as the pUC-series, pBluescript (Stratagene), the pET-series of expression vectors (Novagen) or pCRTOPO (Invitrogen).

After purification of the protein, said protein can be brought into contact with the test compound by combining the test compound and the protein under conditions suitable for assessing the activity of the protein. For biological assays in many cases the presence of further substances, including other salts than sodium chloride, trace elements, amino acids, vitamins, growth factors, ubiquitous co-factors such as ATP or GTP, is required. Said further substances may either be added individually or provided in complex mixtures such as serum or cell extracts. These and further accessory substances are well known in the art as are concentrations suitable for biological assays.

Conditions suitable for assessing the activity of the protein in accordance with the present invention may vary significantly, for example when comparing the interior of a cell to the extracellular space. Exemplary intracellular conditions comprise 14 mM Na⁺, 140 mM K⁺, 10⁻⁷ mM Ca²⁺, 20 mM Mg²⁺, 4 mM Cl⁻, 10 mM HCO₃⁻, 11 mM HPO₄²⁻ and H₂PO₄⁻, 1 mM SO₄²⁻, 45 mM phosphocreatine, 14 mM carnosine, 8 mM amino acids, 9 mM creatine, 1.5 mM lactate, 5 mM ATP, 3.7 mM hexose monophosphate, 4 mM protein and 4 mM urea.

After contacting the protein with the test compound, the activity of the Eph receptor tyrosine kinase is determined. To do so, a kinase assay well known to those skilled in the art may be suitable. If the test compound inhibits the kinase activity of the Eph receptor tyrosine kinase, it is suitable as a medicament or as a lead compound in accordance with the present invention. Another particularly suitable assay is the binding of down-stream effectors, which are part of the Eph forward signalling pathway, to the Eph receptors. Down-stream proteins include Guanine nucleotide exchange factors (GEFs) such as ephexin (Shamah et al. (2001) Cell 105: 233) or Eph receptor tyrosine phosphatases (PTPs) such as ACP-1 (Stein et al. (1998) Genes&Dev 12:667) or PTP-RO (Shintani et al. (2006) Nat. Neurosci. 9: 761). The binding can be detected in vitro by using radioimmunoassay or ELISA.

In another embodiment, the present invention relates to a method of identifying a compound suitable as a lead compound and/or as a medicament for the treatment and/or prevention of diabetes, the method comprising the steps of (a) contacting a test compound with a cell comprising a protein, wherein said protein (i) comprises or consists of the amino acid sequence of any one of SEQ ID NOs: 48 to 58, or (ii) is encoded by a nucleic acid molecule comprising or consisting of the sequence of any one of SEQ ID NOs: 106 to 116, or (iii) is a fragment of the protein according to (i) or (ii) and acts as a protein tyrosine phosphatase for Eph receptor tyrosine kinases; or (iv) has a sequence at least 75% identical with the protein according to (i) or (ii) or with the fragment according to (iii) and acts as a protein tyrosine phosphatase for Eph receptor tyrosine kinases; and (b) determining whether said test compound, upon contacting in step (a) activates the expression and/or activity of said protein wherein said activation indicates a compound suitable as a lead compound and/or as a medicament for the treatment and/or prevention of diabetes. As regards the definition of sequence identity in the context of the present invention, it is referred to the discussion of sequence identity above.

In accordance with the present invention the term "a protein tyrosine phosphatase for Eph receptor tyrosine kinases" refers to proteins, which dephosphorylate Eph receptor tyrosine kinases in vitro or in vivo. Examples of such protein tyrosine phosphatases (PTP) are acid phosphatase-1 (ACP-1) and its isoforms ACP-1d (protein SEQ ID NO: 48; nucleic acid sequence SEQ ID NO: 106), ACP-1b (protein SEQ ID NO: 49; nucleic acid sequence SEQ ID NO: 107), ACP-1c (protein SEQ ID NO: 50; nucleic acid sequence SEQ ID NO: 108), mouse ACP (protein SEQ ID NO: 51; nucleic acid sequence SEQ ID NO: 109), rat ACP (protein SEQ ID NO: 52; nucleic acid sequence SEQ ID NO: 110) as well as protein tyrosine phosphatase receptor type O (PTP-RO) and its isoforms PTP-RO isoform a (protein SEQ ID NO: 53; nucleic acid sequence SEQ ID NO: 111), PTP-RO isoform b (protein SEQ ID NO: 54; nucleic acid sequence SEQ ID NO: 112), PTP-RO isoform c (protein SEQ ID NO: 55; nucleic acid sequence SEQ ID NO: 113), PTP-RO isoform d (protein SEQ ID NO: 56; nucleic acid sequence SEQ ID NO: 114), mouse PTP-RO (protein SEQ ID NO: 57; nucleic acid sequence SEQ ID NO: 115) or rat receptor-type protein tyrosine phosphatase D30 (protein SEQ ID NO: 58; nucleic acid sequence SEQ ID NO: 116).

As stated above, the phosphorylation state of Eph receptor tyrosine kinases depends on the glucose concentration. Eph receptor dephosphorylation is reported to occur through acid phosphatase-1 (ACP-1, also called low molecular weight protein tyrosine phosphatase LMW-PTP) (Parri et al. (2005) J. Biol. Chem. 280, 34008) and protein tyrosine phosphatase receptor type O (PTP-RO) (Shintani et al. (2006) Nat. Neurosci. 9, 761). In accordance with the present invention, it has been discovered that glucose stimulation induces a protein tyrosine phosphatase (PTP) activity that dephosphorylates Eph in pancreatic islets and insulinoma cells (see Examples 9 and 10). Accordingly, PTPs are also useful as a target to screen for compounds modulating insulin secretion by acting via the activation of PTPs. This assay has the advantage of being performed with a cytosolic protein or protein fragment, which is often easier to handle and analyze than transmembrane proteins such as Ephs and ephrins.

Determination of the activity of said protein is accomplished, for example, by measuring the Eph receptor phosphorylation rate in cells expressing ACP-1 and/or PTP-RO. This can be done by immunoprecipitation of EphA5 from cell lysates and subsequent Western blot with a phospho-tyrosine-specific antibody (4G10) (Santa Cruz). Alternatively, Eph receptor phosphorylation is determined by using a phospho-Eph-specific antiserum (Sharma et al. (2001) Cell 105, 233). Determination of the expression level of the PTP can be carried out as described above in the context of other embodiments of the present invention.

In accordance with the invention, the term a "fragment of the protein... and acts as a protein tyrosine phosphatase for Eph-Receptor-Tyrosine-Kinase activity" refers to a portion of the protein that exhibits protein tyrosine phosphatase activity for Eph-Receptor-Tyrosine-Kinase. Activity of said protein has been defined above. Generally, a preferred length of the fragment in particular for PTP-RO is at least 150 amino acids, more preferably between 200 and 400 amino acids and most preferably between 220 and 250 amino acids. A preferred length of the fragment in particular for ACP is, generally, at least 100 amino acids, more preferably at least 110 amino acids and most preferably at least 112 amino acids.

ln another embodiment, the invention relates to a method of identifying a compound suitable as a lead compound and/or as a medicament for the treatment and/or prevention of diabetes, the method comprising the steps of (a) contacting a test compound with a protein, wherein said protein (i) comprises or consists of the amino acid sequence of any one of SEQ ID NOs: 48 to 58, or (ii) is encoded by a nucleic acid molecule comprising or consisting of the sequence of any one of SEQ ID NOs: 106 to 116, or (iii) is a fragment of the protein according to (i) or (ii) and acts as a protein tyrosine phosphatase for Eph receptor tyrosine kinases; or (iv) has a sequence at least 75% identical with the protein according to (i) or (ii) or with the fragment according to (iii) and acts as a protein tyrosine phosphatase for Eph receptor tyrosine kinases; and (b) determining whether said test compound, upon contacting in step (a) enhances the activity of said protein wherein said enhancement indicates a compound suitable as a lead compound and/or as a medicament for the treatment and/or prevention of diabetes.

As recited above in the context of another embodiment, a biochemical assay which uses the PTP directly is also envisaged by the present invention. As outlined above, a biochemical assay offers the possibility to circumvent extensive cell culture work. A possible readout for assessing the enhancement of the activity is already provided above in the context of the corresponding cellular screening method. Detailed methods for performing all necessary steps to arrive at a suitable setup for performing the assay are also provided above in the context of the other embodiments relating to biochemical assays. A particularly suitable method is to determine the activity of the phosphatase in vitro by using a phosphopeptide of the juxtamembrane domain of Eph receptors, e.g. VDPFT('P'-Y)EDPN of EphA4 (Shintani et al. (2006) Nat. Neurosci. 9, 761) together with the recombinant phosphatase in a conventional protein tyrosine phosphatase assay (e.g. SIGMA). The latter method could be used for compound screens by using radioimmunoassay or ELISA.

In another embodiment, the invention relates to a method of identifying a compound suitable as a lead compound and/or as a medicament for the treatment and/or prevention of diabetes, the method comprising the steps of (a) contacting a test compound with a cell comprising a protein, wherein said protein (i) comprises or consists of the amino acid sequence of any one of SEQ ID NOs: 30 to 47, or (ii) is encoded by a nucleic acid molecule comprising or consisting of the sequence of any one of SEQ lD NOs: 88 to 105, or (iii) is a fragment of the protein according to (i) or (ii) and exhibits ephrin activity, or (iv) has a sequence at least 75% identical with the protein according to (i) or (ii) or with the fragment according to (iii) and exhibits ephrin activity; and (b) determining whether said test compound, upon contacting in step (a) activates the expression and/or activity of said protein wherein said activation indicates a compound suitable as a lead compound and/or as a medicament for the treatment and/or prevention of diabetes.

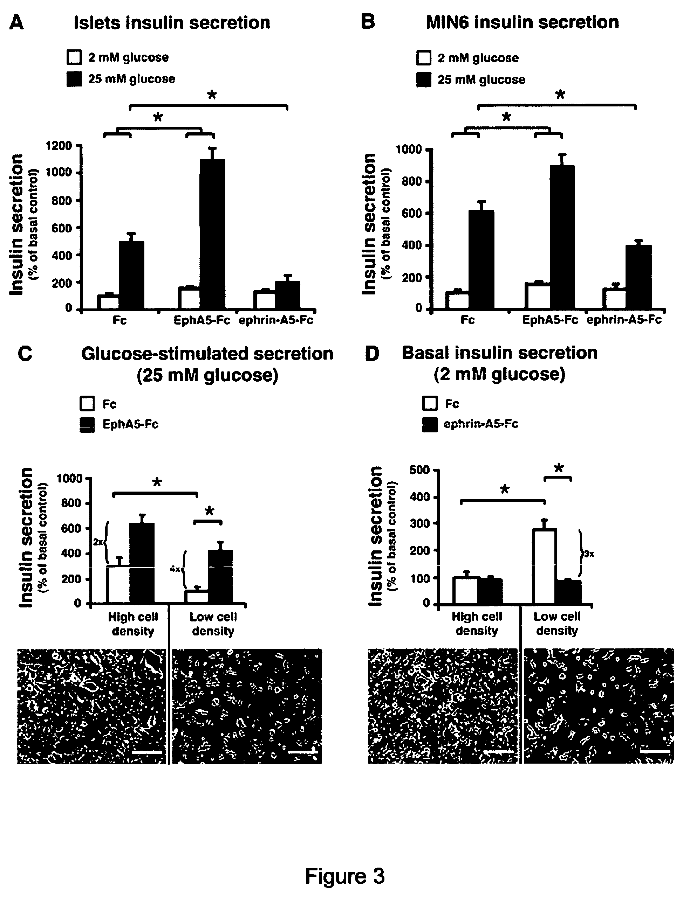

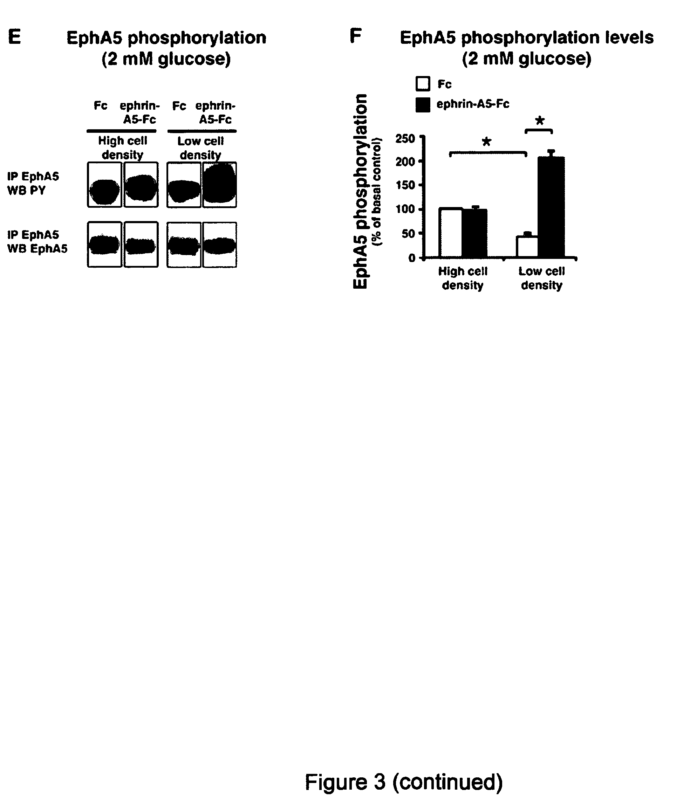

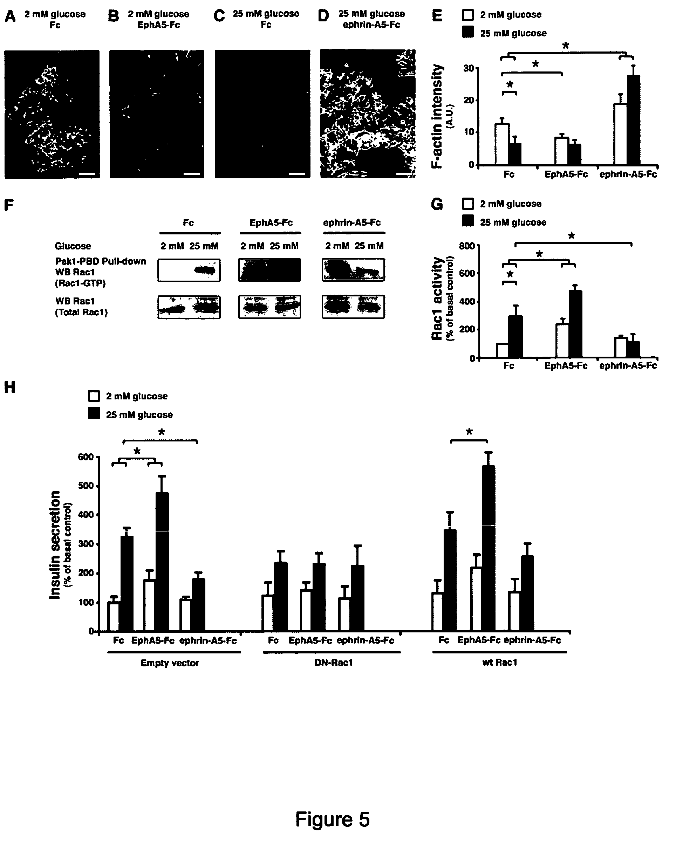

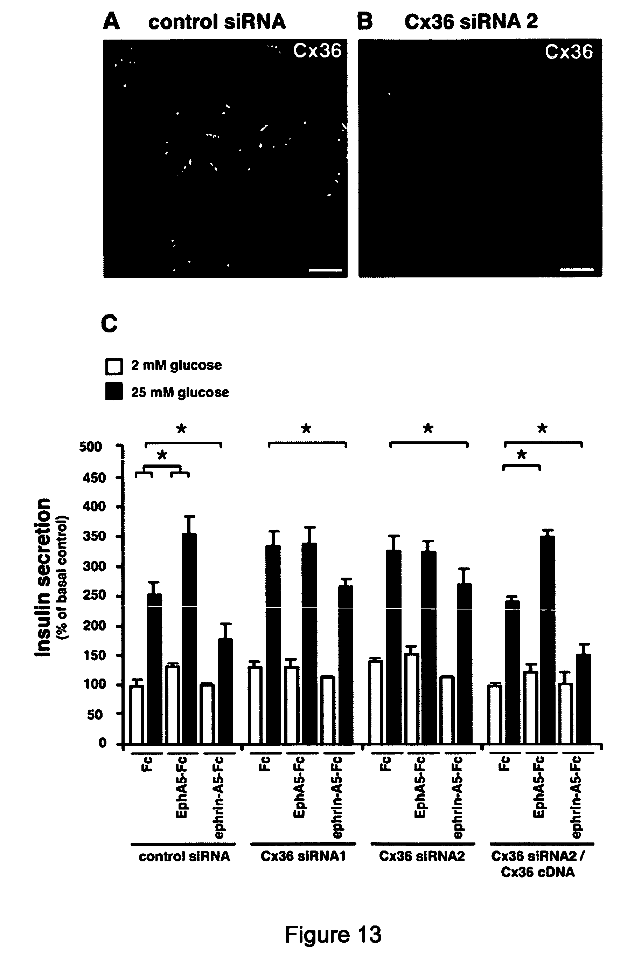

The term "ephrin activity" refers to an activity that, on the one hand induces transphosphorylation of Eph receptor tyrosine kinases and on the other hand induces ephrin reverse signalling. Ephrin reverse signalling results in enhanced activity of Rac1 as well as decreased cortical F-actin (Example 8, Figure 5). Since ephrin-A reverse signalling is mimicked by reduced expression of the beta-cell gap junction protein connexin-36 (Example 8, Figure 13), it is conceivable that ephrin-A reverse signalling inhibits gap junction communication between beta-cells. The main effect of ephrin-A reverse signalling is an increase in insulin secretion (Examples 3, 4 and 6, Figures 3, 4 and 9).

In accordance with the invention, the term a "fragment of the protein... and exhibits ephrin activity" refers to a portion of the protein that exhibits ephrin activity. Ephrin activity has been defined above. Generally, a preferred length of the fragment is at least 100 amino acids, more preferably at least 150 amino acids and most preferably at least 186 amino acids.

As stated above, ephrins are involved in insulin secretion from pancreatic beta-cells. Therefore, ephrins whose amino acid sequences are represented by SEQ !D NOs: 30 to 47 and whose encoding nucleic acid sequences are represented by SEQ ID NOs: 88 to 105 offer the possibility to screen for compounds in a cellular assay as described above. Ephrins represent a functionally different target as compared to Eph receptor tyrosine kinases since ephrins act in an opposite manner to Eph receptor tyrosine kinases with respect to insulin secretion (see above and Examples 3 to 7). Therefore, compounds stimulating insulin secretion via ephrins may be structurally different to compounds acting via Eph receptor tyrosine kinases.

In a preferred embodiment, the cell is a pancreatic beta-cell or a cell from a beta-cell line.

Suitable pancreatic beta-cells are, for example, from species such as mouse, rat, human, guinea pig, hamster, pig, dog, sheep, goat, donkey or cow, and are used either in the form of islets of Langerhans or isolated cells. The cell line may be, for example, selected from the rat insulinoma (RIN) cell lines such as INS-1 (Asfari et al. (1992) Endocrinol.130, 167), INS-2 (Asfari et al. (1992) Endocrinol.130, 167), RIN-r (Philippe et al. (1986) Endocrinol. 119, 2833) and RIN-m (Bathena et al. (1982) Diabetes 31, 521; Praz et al. (1983) Biochem. J. 210, 345; Philippe et al. (1987) J. Clin. Invest. 79, 351) or from the hamster insulinoma (HIT) cell lines such as HIT-T15 (Santerre et al. (1981) Proc. Natl. Acad. Sci. U.S.A. 78, 4339) or from mouse beta-cell lines expressing the SV40 large T-antigen (beta-TC lines) such as beta TC1, betaTC2, betaTC3 (Efrat et al. (1988) Proc. Natl. Acad. Sci. U.S.A. 85, 9037), betaTC6 (Poitout et al. (1995) Diabetes 44, 306), betaTC7 (Efrat et al. (1993) Diabetes 42, 901), betaTCtet (Efrat et al. (1995) Proc. Natl. Acad. Sci. U.S.A. 92, 3576) or from mouse insulinoma (MIN) cell lines such as MIN6 (Myazaki et al. (1990) Endocrinol. 127, 126).

In a preferred embodiment, determining in step (b) comprises quantifying insulin secretion.

The direct quantification of insulin secretion has the advantage of delivering a direct physiological read-out of the effect of the screened compound since insulin secretion is the desired result when screening for anti-diabetic compounds.

Preferably, determination of insulin secretion is carried out by methods including but not limited to ELISA kits (such as the one from Crystal Chem Inc.) or radioimmunoassays (such as the one from Linco, St. Charles, MO, USA).

Generally, determination of insulin secretion is carried out at glucose concentrations in the range of 6 to 30 mM, more preferably 10 to 30 mM, and most preferably 20 to 25 mM, or in the presence of, for example, a combination of glucose and glucagon-like peptide-1 (GLP-1) or glucose and 3-isobutyl-1-methylxanthine (IBMX).

In another preferred embodiment, determining in step (b) comprises quantifying F-actin in said cell, wherein the quantity of F-actin is determined at stimulatory glucose concentrations and wherein a decreased quantity of F-actin in the cell contacted with the test compound indicates a compound suitable as a lead compound and/or as a medicament for the treatment and/or prevention of diabetes.

The term "stimulatory glucose concentrations" generally refers to glucose concentrations in the range of 5.5 to 30 mM, more preferably 10 to 30 mM, and most preferably 20 to 25 mM. A preferred range in particular for studying activation of ephrin-A reverse signalling is 6 to 20 mM, most preferably 6 to 8 mM.

In addition to their involvement in glucose stimulated insulin secretion, the present invention shows opposite effects of Eph and ephrin signalling on F-actin. Moderate destabilization of F-actin was shown to enhance insulin secretion, while F-actin stabilization was shown to inhibit insulin secretion (Cable et al. (1995) Biochem. J. 307: 169; Wang et al. (1990) Biochem. Biophys. Res. Commun. 171: 424; Wilson et al. (2001) FEBS Lett 492: 101; Lawrence et al. (2003) Proc. Natl. Acad. Sci. U.S.A. 100: 13320; Tomas et al. (2006) J. Cell Sci. 119: 2156; Orci et al. (1972) Science 175: 1128; Jijakli et al. (2002) Int. J. Mol. Med. 9: 165; Thurmond et al. (2003) Mol. Endocrinol. 17: 732; Tsuboi et al. (2003) J. Biol. Chem. 278: 52042). The present invention now, for the first time, establishes a link between the F-actin content of a cell in response to a glucose stimulus and the molecular interaction between Ephs and ephrins (see Example 5). Quantification of F-actin is much easier than direct quantification of insulin secretion since no time-consuming assays, such as ELISA, are required but rather simple fluorescence measurements. Therefore, using the F-actin content as a readout renders the establishment of a high throughput assay for screening for anti-diabetic compounds less difficult.

Quantification of F-actin in said cell may, for example, be carried out by methods including but not limited to fluorescence measurements or F-actin ELISA.

In another preferred embodiment, the protein as referred to above is comprised in a cell expressing, or transfected with, a nucleic acid encoding said protein.

The cell is preferably a primary cell such as a pancreatic beta-cell or a cell from a cell line. Examples of suitable cell lines are referred to above.

Transfection of a cell or a cell line with a nucleic acid encoding the protein referred to above allows for adaptation of the screening method of the invention to the experimental set-up, such as the use of a different cell line capable of growing under culture conditions which are, for example, easier to implement.

In more preferred embodiment, the cell comprising the protein referred to above is comprised in a non-human animal.

Introduction or expression of the protein referred to above in a non-human animal allows for the screening method of the invention to be performed under physiological conditions and opens the way for the identification of compounds which are, besides their anti-diabetic activity, capable of being efficiently absorbed, distributed, eventually metabolized to their biologically active form and excreted in a pharmacologically acceptable manner. Preferably, the animal is selected from, for example, rat, mouse, hamster, guinea pig, dog, cow, pig, goat, sheep or donkey.

A method for the production of a transgenic non-human animal, for example a transgenic mouse, comprises introduction of the nucleic acid molecules referred to above into a germ cell, an embryonic cell, stem cell or an egg or a cell derived therefrom. The non-human animal can be used in accordance with the invention in a method for identification of compounds useful for the treatment and/or prevention of diabetes. Production of transgenic embryos and screening of those can be performed, e.g., as described by A. L. Joyner Ed., Gene Targeting, A Practical Approach (1993), Oxford University Press. The DNA of the embryonic membranes of embryos can be analyzed using, e.g., Southern blots with an appropriate probe. A general method for making transgenic non-human animals is described in the art, see for example WO 94/24274. For making transgenic non-human organisms (which include homologously targeted non-human animals), embryonic stem cells (ES cells) are preferred. Murine ES cells, such as AB-1 line grown on mitotically inactive SNL76/7 cell feeder layers (McMahon and Bradley (1990) Cell 62,1073) essentially as described (Robertson, E. J. (1987) in Teratocarcinomas and Embryonic Stem Cells: A Practical Approach. E. J. Robertson, ed. (Oxford: IRL Press), p. 71) may be used for homologous gene targeting. Other suitable ES cell lines include, but are not limited to, the E14 line (Hooper et al. (1987) Nature 326, 292), the D3 line (Doetschman et al. (1985) J. Embryol. Exp. Morph. 87, 27), the CCE line (Robertson et al. (1986) Nature 323, 445), the AK-7 line (Zhuang et al. (1994) Cell 77, 875). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are born, e.g. as chimeric mice. The resultant transgenic mice are chimeric for cells having either the recombinase or reporter loci and are backcrossed and screened for the presence of the correctly targeted transgene(s) by PCR or Southern blot analysis on tail biopsy DNA of offspring so as to identify transgenic mice heterozygous for either the recombinase or reporter locus/loci. For example, for beta-cell specific and pancreas-specific gene manipulation, the rat insulin promoter (RIP-1 and RIP-2) and pancreas-duodenum-homeobox-1 (Pdx1) promoter can be used, respectively. These promoters are generally used to drive the gene of interest in a constitutive or inducible fashion.

In more preferred embodiment, determining in step (b) involves quantifying insulin secretion in said non-human animal.

The invention also relates to the use of an activator of ephrin activity for the manufacture of a pharmaceutical composition for treating diabetes wherein said activator is selected from the group consisting of (a) a fusion protein consisting of the extracellular domain of an Eph receptor tyrosine kinase and an Fc chain; (b) a dominant-negative deletion mutant protein of an Eph receptor tyrosine kinase lacking the cytoplasmic tail; (c) a nucleic acid molecule encoding the protein of (a) or (b); (d) a vector comprising the nucleic acid molecule of (c); and (e) a host comprising the vector of (d).

The term "activator", in accordance with the present invention, is defined as a compound enhancing the activity of a target molecule, preferably by performing one or more of the following effects: (i) the transcription of the gene encoding the protein to be activated is enhanced, (ii) the translation of the mRNA encoding the protein to be activated is enhanced, (iii) the protein performs its biochemical function with enhanced efficiency in presence of the activator, and (iv) the protein performs its cellular function with enhanced efficiency in presence of the activator.

In another embodiment, the activator is a small molecule. Small molecules are compounds of natural origin or chemically synthesized compounds, preferably with a molecular weight below 10000 Da, more preferred below 1000 Da, more preferred below 500 Da and most preferred between 200 Da and 400 Da.

Preferably, the small molecule may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively the compound may be an inorganic compound. Inorganic compounds are derived from mineral sources and include all compounds without carbon atoms (except carbon dioxide, carbon monoxide and carbonates).

The efficiency of the activator can be quantified by comparing the level of activity in the presence of the activator to that in the absence of the activator. For example, as an activity measure may be used: the change in amount of mRNA formed, the change in amount of protein formed, the change in amount of phosphorylation, and/or the change in the cellular phenotype or in the phenotype of an organism.

In a preferred embodiment, the level of activity is 10% more than the activity in absence of the activator, more preferred, the level of activity is 25% or 50% more than the activity in absence of the activator. Yet more preferred are activators enhancing the level of activity to 75%, 80%, 90% or 100% more than the activity in absence of the activator.

In accordance with the present invention, the term "pharmaceutical composition" relates to a composition for administration to a patient, preferably a human patient.

The pharmaceutical composition of the invention comprises the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range 0.01 mg to 100 mg, even more preferably 0.01 mg to 50 mg and most preferably 0.01 mg to 10 mg per day.

In accordance with the present invention, "the extracellular domain of an Eph receptor tyrosine kinase" is defined as a protein comprising the extracellular domain of an Eph receptor tyrosine kinase, which generally comprises the first 200 amino acid residues of the N-terminus, preferably the first 300 amino acid residues, more preferably the first 400 amino acid residues, more preferably the first 530 amino acid residues, more preferably the first 590 amino acid residues and most preferably the first 600 amino acid residues.

The term "Fc chain" is defined as the region of an antibody composed of two heavy chains that each contribute two to three constant domains, depending on the class of the antibody. Fc chains bind to various cell receptors and complement proteins.

The "Eph receptor tyrosine kinase lacking the cytoplasmic tail" is defined as a protein which lacks the last 200-850 C-terminal amino acid residues, preferably the protein lacks the last 400-600 C-terminal amino acid residues, more preferably it lacks the last 450-550 amino acid residues and most preferably it lacks the last 470-520 C-terminal amino acid residues. Alternatively, said "Eph receptor tyrosine kinase lacking the cytoplasmic tail" may be defined as an Eph receptor tyrosine kinase comprising at least the extracellular portion and preferably said Eph receptor tyrosine kinase comprises at least the extracellular portion and the transmembrane region.

The term "dominant negative mutant" refers to a mutant that produces a protein that interacts with and/or interferes with the function of a wild-type protein.

ln a preferred embodiment the nucleic acid molecule(s) is/are DNA.

The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146) or pBC12MI (ATCC 67109).

The nucleic acid molecule referred to above may also be inserted into vectors such that a translational fusion with another nucleic acid molecule is generated. The vectors may also contain an additional expressible polynucleotide coding for one or more chaperones to facilitate correct protein folding.

For vector modification techniques, see Sambrook and Russel (2001), loc. cit. Generally, vectors can contain one or more origin of replication (ori) and inheritance systems for cloning or expression, one or more markers for selection in the host, e. g., antibiotic resistance, and one or more expression cassettes.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens (2001) Proc. Natl. Acad. Sci. USA 98, 1471) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule is operatively linked to such expression control sequences allowing expression in eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication, drug resistance gene, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).

The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. (1991) Biochem J. 227, 277; Bebbington et al. (1992) Bio/Technology 10, 169). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture.

The nucleic acid molecules as described hereinabove may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention.

Mammalian host cells that could be used include, human Hela, HEK293, H9 and Jurkat cells, mouse NIH3T3 and C127 cells, Cos 1, Cos 7 and CV1, quail QC1-3 cells, mouse L cells and Chinese hamster ovary (CHO) cells. Furthermore, also primary cells are within the scope of the present invention. Said primary cells may be derived, for example, from mammals such as mouse, rat or human.

In a preferred embodiment, the nucleic acid molecule referred to above is comprised in a cell that is comprised in a mammal.

Preferably, said mammal is a human.

In a preferred embodiment, the pharmaceutical composition further comprises an inhibitor of an Eph receptor tyrosine kinase wherein said inhibitor is selected from the group consisting of an antisense nucleic acid molecule, a miRNA, an siRNA or an shRNA binding specifically a nucleic acid encoding said Eph receptor tyrosine kinase or from the group consisting of an antibody or aptamer.

The term "inhibitor" designates a compound lowering the activity of a target molecule, preferably by performing one or more of the following effects: (i) the transcription of the gene encoding the protein to be inhibited is lowered, (ii) the translation of the mRNA encoding the protein to be inhibited is lowered, (iii) the protein performs its biochemical function with lowered efficiency in presence of the inhibitor, and (iv) the protein performs its cellular function with lowered efficiency in presence of the inhibitor.

Compounds falling in class (i) include compounds interfering with the transcriptional machinery and/or its interaction with the promoter of said gene and/or with expression control elements remote from the promoter such as enhancers. Compounds of class (ii) comprise antisense constructs and constructs for performing RNA interference (e.g. siRNA) well known in the art (see, e.g. Zamore (2001) Nat Struct Biol. 8(9), 746; Tuschl (2001) Chembiochem. 2(4), 239). Compounds of class (iii) interfere with molecular function of the protein to be inhibited, in case of Eph receptor tyrosine kinases with its enzymatic activity, in particular with the protein kinase activity. Accordingly, active site binding compounds, in particular compounds capable of binding to the active site of any protein kinase, are envisaged. More preferred are compounds specifically binding to an active site of Eph receptor tyrosine kinases. Class (iv) includes compounds which do not necessarily bind directly to Eph receptor tyrosine kinases, but still interfere with Eph receptor tyrosine kinase activity, for example by binding to and/or inhibiting the function or inhibiting expression of members of a pathway which comprises Eph receptor tyrosine kinases. These members may be either upstream or downstream of Eph receptor tyrosine kinases within said pathway.

The efficiency of the inhibitor can be quantified by comparing the level of activity in the presence of the inhibitor to that in the absence of the inhibitor. For example, as an activity measure may be used: the change in amount of mRNA formed, the change in amount of protein formed, the change in amount of phosphorylation, and/or the change in the cellular phenotype or in the phenotype of an organism.

In a preferred embodiment, the level of activity is less than 90%, more preferred less than 80%, 70%, 60% or 50% of the activity in absence of the inhibitor. Yet more preferred are inhibitors lowering the level down to less than 25%, less than 10%, less than 5% or less than 1% of the activity in absence of the inhibitor.

The addition of an inhibitor of Eph receptor tyrosine kinases may, for example, allow for the enhancement of insulin secretion since Eph receptor tyrosine kinases inhibit insulin secretion when active (see Examples 3, 7, 9 and 10). Therefore, the therapeutic window may be, for example, wider when using a combination of ephrin activators and Eph receptor tyrosine kinase inhibitors with, for example, reduced side effects as compared to the desired effect of insulin secretion.

The present invention also relates to the use of an inhibitor of Eph receptor tyrosine kinase activity for the manufacture of a pharmaceutical composition for treating diabetes wherein said inhibitor is selected from the group consisting of (a) an antibody, aptamer, or a fragment or derivative thereof binding specifically to said Eph receptor tyrosine kinase; (b) a ribozyme specifically cleaving a nucleic acid encoding the intracellular domain of said Eph receptor tyrosine kinase; (c) a protein tyrosine phosphatase for Eph receptor tyrosine kinase; (d) a reducing agent of a protein tyrosine phosphatase for Eph receptor tyrosine kinase; (e) a nucleic acid molecule encoding the protein tyrosine phosphatase of (c); (f) a vector comprising the nucleic acid molecule of (e); (g) a host comprising the vector of (f); and (h) an inhibitor of Eph receptor tyrosine kinases which competes with ATP.

The term "Ribozymes" refers to RNA molecules that act as enzymes in the absence of proteins. These RNA molecules act catalytic or autocatalytic and are capable of cleaving e.g. other RNAs at specific target sites. Selection of appropriate target sites and corresponding ribozymes can be done as described for example in Steinecke et al. ((1995) Methods in Cell Biology 50, 449).

Said ribozyme specifically cleaves e.g. the cytoplasmic tail of a nucleic acid encoding an Eph receptor tyrosine kinase thereby causing a truncated Eph receptor tyrosine kinase which consists, for example, only of the extracellular part and the transmembrane portion. Preferably, said ribozyme cleaves at least 500 nucleotides from 3' end, more preferably at least 1000 nucleotides and most preferably at least 2500 from the 3' end of the nucleic acid molecule encoding said Eph receptor tyrosine kinase.

The vector and the host are defined as recited hereinabove.

The "inhibitor of Eph receptor tyrosine kinases that competes with ATP" refers to small molecules as recited above which are additionally characterized by their ability to bind to the ATP binding site of e.g. kinases and inhibit their activity by rendering this site inaccessible for ATP. Examples include, but are not limited to, staurosporine.

Inhibition of kinase activity results in the Eph receptor tyrosine kinase's inability to exert transphosphorylation which leads to a loss of Eph receptor tyrosine kinase activity.

In a preferred embodiment, the type of diabetes to be treated is type II diabetes.

The Figures show:
**Figure 1****. Expression and localization of ephrin-As and EphAs in pancreatic islets and MIN6 cells**
   (A) Model of ephrin-A and EphA bidirectional signaling between two adjacent β-cells, and exogenous activation of ephrin-A reverse signaling and EphA forward signaling by EphA5-Fc and ephrin-A5-Fc fusion proteins, respectively. Circled P represents tyrosine phosphorylation.
   (B) RT-PCR products for ephrin-A1 to -A5 and EphA1 to A8, performed with mRNA isolated from mouse islets and mouse insulinoma cells (MIN6).
   (C - F) Confocal images of mouse pancreas sections show an islet surrounded by exocrine pancreatic tissue. Sections were stained with (C) Anti-ephrin-A5 antibody,
   (D) EphA5-Fc that binds to ephrin-As, (E) Anti-EphA5 antibody and (F) ephrin-A5-Fc that binds to EphAs. Scale bars, 50 µm.
   (G - I) Confocal images of a group of MlN6 cells stained for ephrin-As and EphA5.
   (G) Low magnification, (H, I) High magnification of regions in (G): (H) Contact region between two MlN6 cells with EphA-ephrin-A colocalization (white arrowhead) and (I) free surfaces of MlN6 cells with no colocalization between ephrin-As (light grey arrowheads) and EphA5 (dark grey arrowheads). Note that the anti-EphA5 antibody was directed against the C-terminus of EphA5 and could not bind to the EphA5-Fc used to stain ephrin-As.
      Scale bars in G, 10 µm, in H and 1, 2 µm.
**Figure 2****. Ephrin-A5 is required for glucose-stimulated insulin secretion**
   (A) Insulin secretion from control and ephrin-A5-/- islets at 2mM glucose (white columns) and 25mM glucose (black columns). Secreted insulin is normalized to insulin content and total protein content. Δ, difference between basal and glucose-stimulated insulin secretion. N = 3 experiments. *p < 0.05. All values are means ± SD.
   (B) Insulin content of islets isolated from control mice and ephrin-A5-/- mice presented as % of total protein content. N = 3 experiments. *p < 0.05. All values are means ± SD.
   (C) Glucose tolerance tests of control and ephrin-A5-/- mice. N = 7 mice each. *p < 0.05. All values are means ± SD.
   (D, E) EphA5-Fc immunostaining (white) of MIN6 cells transfected with (D) control siRNA and (E) ephrin-A5 siRNA 2. Cell nuclei are stained with DAPI (grey). Scale bars, 20 µm.
   (F) Insulin secretion from MIN6 cells transfected with control siRNA, ephrin-A5 siRNA 1, ephrin-A5 siRNA 2, or co-transfected with ephrin-A5 siRNA 2 and ephrin-A5 cDNA without the targeted 3'-UTR. White columns, 2mM glucose; black columns, 25mM glucose. Secreted insulin is normalized to insulin content and total protein content. N = 6 experiments. *p < 0.05. All values are means ± SD.
**Figure 3****. Opposite effects of EphA5-Fc and ephrin-A5-Fc on insulin secretion**
   (A, B) Insulin secretion from (A) mouse pancreatic islets and (B) MIN6 cells treated with Fc (as control), EphA5-Fc (to activate ephrin-A reverse signaling) and ephrin-A5-Fc (to activate EphA forward signaling) at 2mM glucose (white columns) and 25mM glucose (black columns), normalized to insulin content and total protein content. N = 3 experiments. _{*}p < 0.05. All values are means ± SD.
   (C) Glucose-stimulated insulin secretion from MIN6 cells at high cell density (lower left image) and low cell density (lower right image) after treatment with Fc (white columns) or EphA5-Fc (black columns). N = 4 experiments. *p < 0.05. All values are means ± SD. Scale bars, 100 µm.
   (D) Basal insulin secretion from MIN6 cells at high cell density (lower left image) and low cell density (lower right image) after treatment with Fc (white columns) or ephrin-A5-Fc (black columns). N = 3 experiments. *p < 0.05. All values are means ± SD. Scale bars, 100 µm.
   (E) Detection of tyrosine-phosphorylated EphA5 (PY) and total EphA5 in Western blots after EphA5 immunoprecipitation from lysates of MlN6 cells that were grown at high and low cell density, and kept under basal conditions for 1 h + Fc or + ephrin-A5-Fc.
   (F) EphA5 tyrosine phosphorylation, relative to total EphA5 protein, is shown in a histogram for the experiment shown in (E). N = 2 experiments. *p < 0.05. All values are means ± SD.

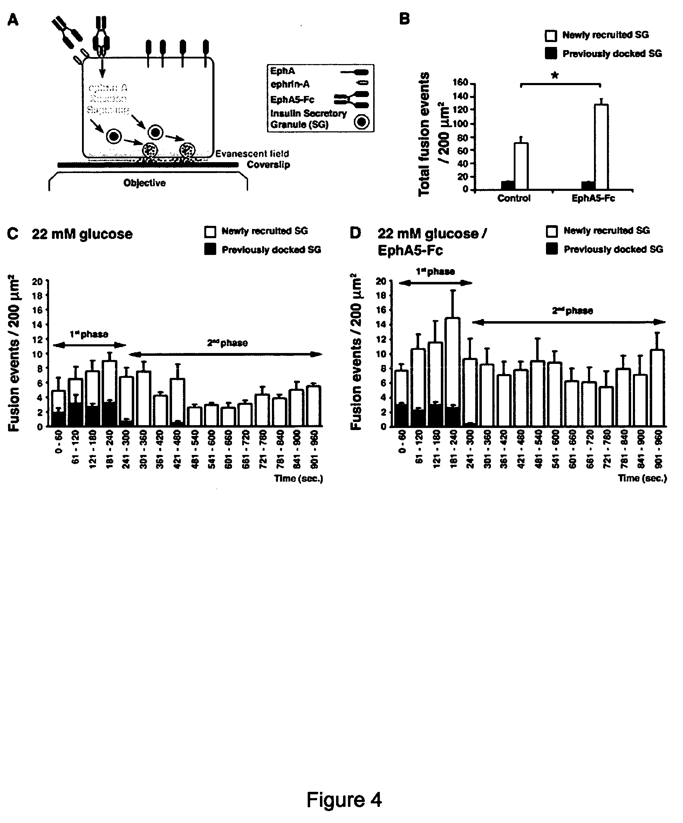

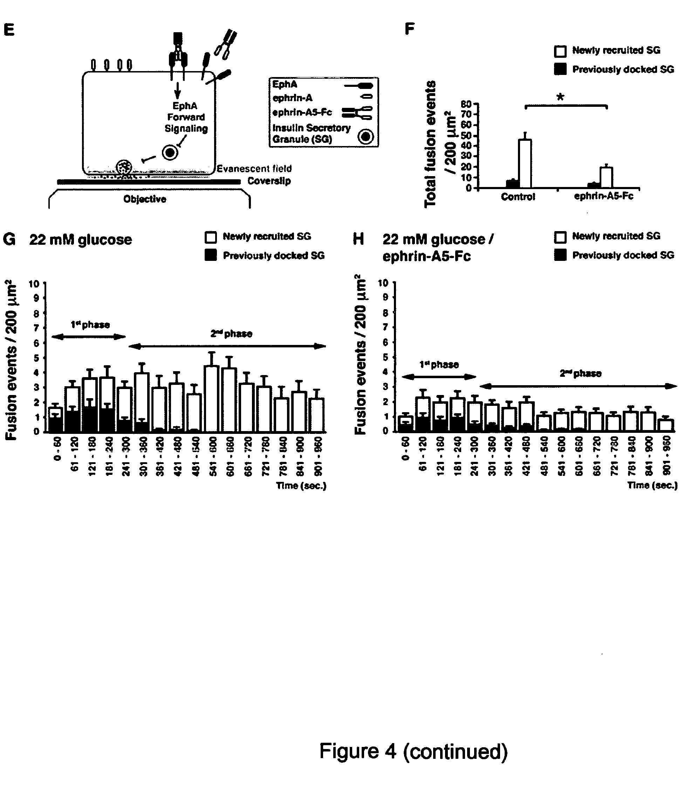

**Figure 4****. Opposite effects of ephrin-A reverse signaling and EphA forward signaling on insulin secretory granule fusion**
   (A) Model showing activation of ephrin-A reverse signaling by EphA5-Fc in a single insulin-GFP expressing mouse pancreatic β-cell. Total internal reflection - fluorescence microscopy (TIR-FM) was used to detect fusion of insulin secretory granules (SG) with the plasma membrane.
   (B) Histogram showing all secretory fusion events in single β-cells detected during 16 min. treatment with 22mM glucose in the absence (control) or presence of EphA5-Fc. Fusion events of previously docked SG (black columns) and newly recruited SG (white columns) were normalized to the area. N = 5-7 cells each. *p < 0.05. All values are means ± SEM.
   (C, D) Time-course of fusion events in single β-cells. Cells treated with 22mM glucose, (C) in the absence or (D) presence of EphA5-Fc. Fusion events of newly recruited SG (white columns) and previously docked SG (black columns) are shown. N = 5-7 cells each.
   (E) Model showing activation of EphA forward signaling by ephrin-A5-Fc in a single insulin-GFP expressing mouse pancreatic β-cell. TIR-FM was used to detect fusion of SG with the plasma membrane.
   (F) Histogram showing all secretory fusion events in single β-cells detected during 16 min. treatment with 22mM glucose in the absence (control) or presence of ephrin-A5-Fc. Fusion events of previously docked SG (black columns) and newly recruited SG (white columns) were normalized to the area. N = 20-21 cells each. *p < 0.05. All values are means ± SEM.
   (G, H) Time-course of fusion events in single β-cells. Cells treated with 22mM glucose, (G) in the absence or (H) presence of ephrin-A5-Fc. Fusion events of newly recruited SG (white columns) and previously docked SG (black columns) are shown. N = 20-21 cells each.
**Figure 5****. Opposite effects of EphA5-Fc and ephrin-A5-Fc on F-actin and Rac1 activity**
   (A - D) F-actin staining of MIN6 cells treated for 10 min. with (A) 2mM glucose and Fc (control), (B) 2mM glucose and EphA5-Fc (to activate ephrin-A reverse signaling), (C) 25mM glucose and Fc (control), (D) 25mM glucose and ephrin-A5-Fc (to activate EphA forward signaling). Scale bars, 20 µm.
   (E) Quantification of F-actin fluorescence intensities in MIN6 cells treated for 10 min. with Fc, EphA5-Fc and ephrin-A5-Fc at 2mM glucose (white columns) and 25mM glucose (black columns). N = 10 images of each N = 3 coverslips were quantified. *p < 0.05. All values are means ± SD.
   (F) Detection of active Rac1-GTP (upper bands) and total Rac1 (lower bands) in Western blots after Pak1-PBD pull-down from lysates of confluent MIN6 cells. Cells were treated for 10 min. with Fc, EphA5-Fc and ephrin-A5-Fc at 2mM glucose (white columns) and 25mM glucose (black columns).
   (G) Corresponding histograms of Rac1 activity, relative to total Rac1 protein. N = 3 experiments. *p < 0.05. All values are means ± SD.
   (H) Insulin secretion from MlN6 cells transfected with empty vector, DN-Rac1 and wtRac1 after treatment with Fc, EphA5-Fc and ephrin-A5-Fc at 2mM glucose (white columns) and 25mM glucose (black columns). N = 3 experiments. *p < 0.05. All values are means ± SD.

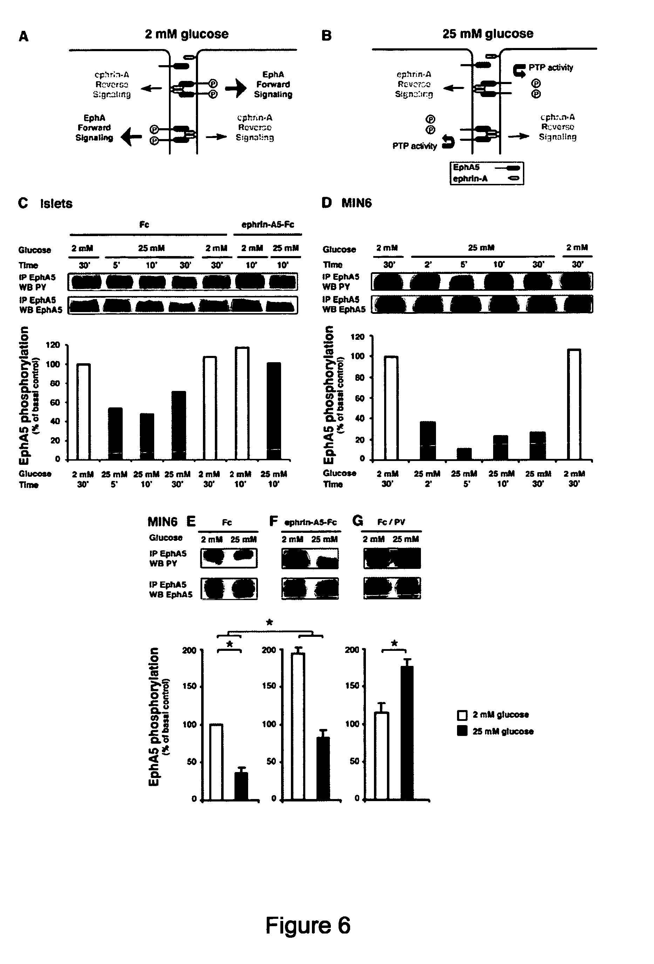

**Figure 6****. Glucose-induced dephosphorylation of EphA5**
   (A, B) Model showing how glucose changes the outcome of EphA - ephrin-A bidirectional signaling. (A) At low glucose concentration, EphA forward signaling is active. (B) Upon glucose stimulation, EphA forward signaling is attenuated by EphA dephosphorylation, involving a protein tyrosine phosphatase (PTP) activity.
   (C - G) Detection of tyrosine-phosphorylated EphA5 (PY) and total EphA5 in Western blots after immunoprecipitation of EphA5 from cell lysates. The EphA5 phosphorylation level, relative to total EphA5 protein, is shown in histograms. 2mM glucose (white columns); 25mM glucose (black columns).
   (C) Pancreatic islets treated with 2mM glucose for 30 min., then with 25mM glucose for 5, 10 and 30 min., followed by a switch from 25mM to 2mM glucose for 30 min. Islets treated with ephrin-A5-Fc (to activate EphA forward signaling) for 10 min. at 2mM and 25mM glucose are also shown.
   (D) MIN6 cells treated with 2mM glucose for 30 min., then with 25mM glucose for 2, 5, 10 and 30 min., followed by a switch from 25mM to 2mM glucose for 30 min.
   (E - G) MIN6 cells treated for 5 min. with (E) Fc (control), (F) ephrin-A5-Fc (to activate EphA forward signaling) and (G) Fc + 10µM peroxovanadate (PV) (to inhibit glucose-induced EphA5 dephosphorylation) at 2mM and 25mM glucose. N = 2 experiments. *p < 0.05. All values are means ± SD.
Figure 7. EphA dephosphorylation is required for insulin secretion
   (A) Model showing that peroxovanadate (PV) blocks a PTP activity, which normally dephosphorylates EphAs at high glucose concentration. (B) Model showing that overexpression of EphA5 lacking its cytoplasmic domain (DN-EphA5) rescues inhibition of EphA forward signaling in PV-treated cells at high glucose concentration.
   (C) Model showing that overexpression of wt EphA5 potentiates EphA forward signaling in PV-treated cells.
   (D) Insulin secretion from mouse pancreatic islets treated with Fc in the absence or presence of 10µM peroxovanadate (PV) at 2mM glucose (white columns) and 25mM glucose (black columns). Secreted insulin is normalized to insulin content and total protein content. N = 3 experiments. *p < 0.05. All values are means ± SD.
   (E) Insulin secretion from MIN6 cells transfected with an empty vector (as a control), DN-EphA5 or wt EphA5 in the absence or presence of 10µM peroxovanadate (PV) at 2mM glucose (white columns) and 25mM glucose (black columns). Secreted insulin is normalized to insulin content and total protein content. N = 2 experiments. *p < 0.05. All values are means ± SD.
   (F, G) Model. (F) At low glucose concentration, interactions between EphAs and ephrin-As on adjacent β-cell plasma membranes result in EphA phosphorylation and kinase-dependent EphA forward signaling. This inhibits Rac1 activity, increases cortical F-actin and suppresses insulin secretion. (G) Upon glucose stimulation, a PTP activity dephosphorylates EphAs, and EphA - ephrin-A interactions do not lead to kinase-dependent EphA forward signaling. Instead, EphAs predominantly activate ephrin-A reverse signaling, which enhances Rac1 activity, rearranges cortical F-actin and stimulates insulin secretion.
**Figure 8****. Ephrin-A5 and EphA5 expression in the human pancreas: EphA5-Fc and ephrin-A5-Fc conversely affect insulin secretion from human pancreatic islets**
   (A-F) Confocal images of human pancreas sections show an islet surrounded by exocrine pancreatic tissue. Sections are stained for (A) ephrin-A5, (B) insulin, (C) ephrin-A5 and insulin (merge), (D) EphA5, (E) insulin, (F) EphA5 and insulin (merge). Scale bars in C, 100 µm and F, 50 µm.
   (G) Insulin secretion from human pancreatic islets treated with Fc (as control), EphA5-Fc (to activate ephrin-A reverse signaling) and ephrin-A5-Fc (to activate EphA forward signaling) at 2mM glucose (white columns) and 25mM glucose (black columns), normalized to insulin content and total protein content. N = 3 experiments. *p < 0.05. All values are means ± SD.

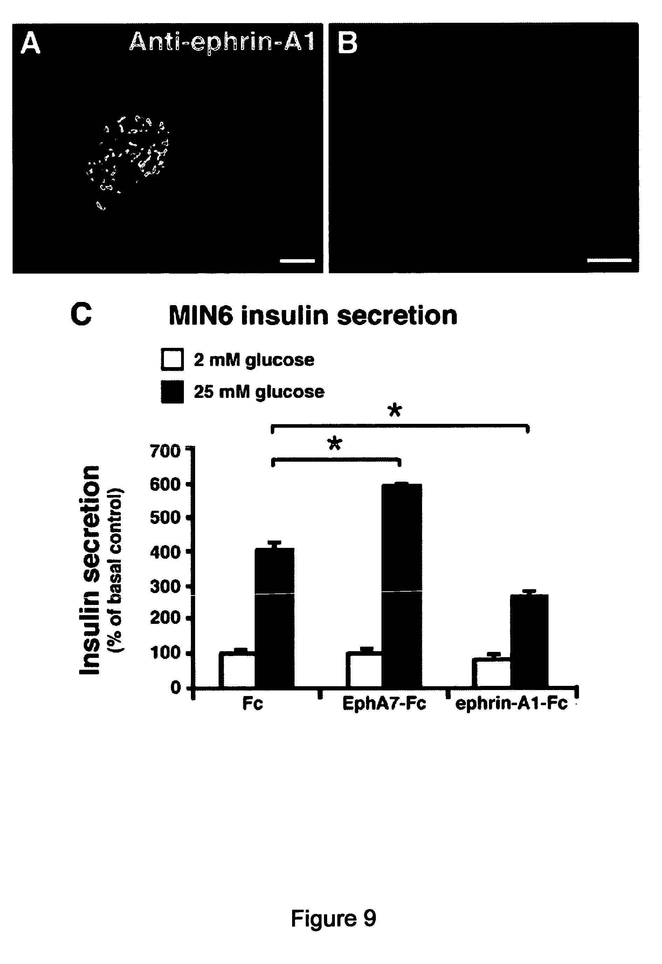

**Figure 9****. Ephrin-A1 and EphA7 expression in the mouse pancreas: EphA7-Fc and ephrin-A1-Fc conversely affect glucose-stimulated insulin secretion**
   (A, B) Confocal images of mouse pancreas sections showing an islet surrounded by exocrine tissue. Sections were stained for (A) ephrin-A1, and (B) EphA7. Scale bars, 20 µm.
   (C) Insulin secretion from MIN6 cells treated with Fc (as control), EphA7-Fc (to activate ephrin-A reverse signaling) and ephrin-A1-Fc (to activate EphA forward signaling) at 2mM glucose (white columns) and 25mM glucose (black columns), normalized to insulin content and total protein content. N = 3 experiments. *p < 0.05. All values are means ± SD.

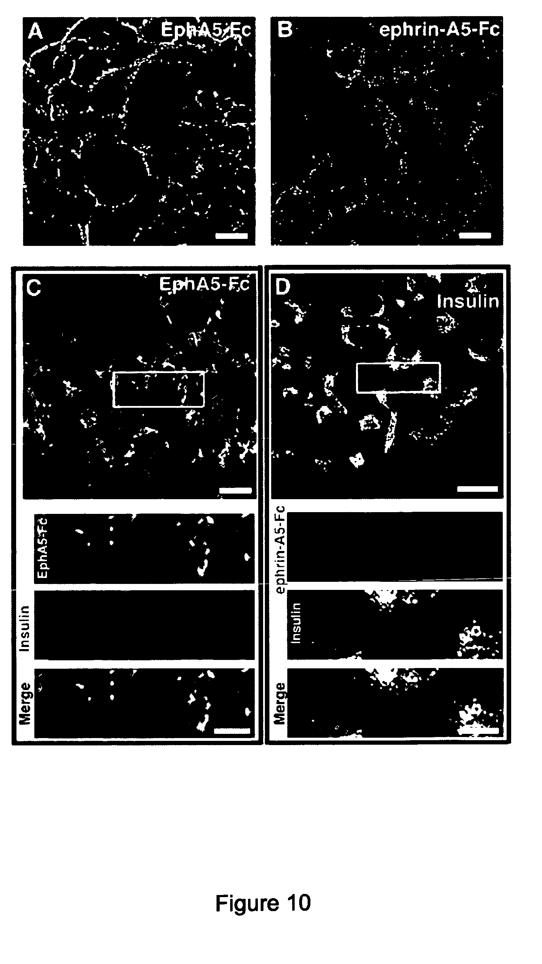

**Figure 10****. Segregation of EphAs and ephrin-As in mouse insulinoma cells**
   (A, B) Confocal images of non-permeabilized MIN6 cells stained for (A) ephrin-As with EphA5-Fc, and (B) EphAs with ephrin-A5-Fc. Scale bars, 20 µm.
   (C) Confocal image of permeabilized MIN6 cells stained for ephrin-As with EphA5-Fc (white) and insulin (grey). Low magnification image, scale bar, 10 µm, and high magnification images, scale bar, 2 µm.
   (D) Confocal image of permeabilized MIN6 cells stained for EphAs with ephrin-A5-Fc (grey) and insulin (white). Low magnification image, scale bar, 10 µm, and high magnification images, scale bar, 2 µm.

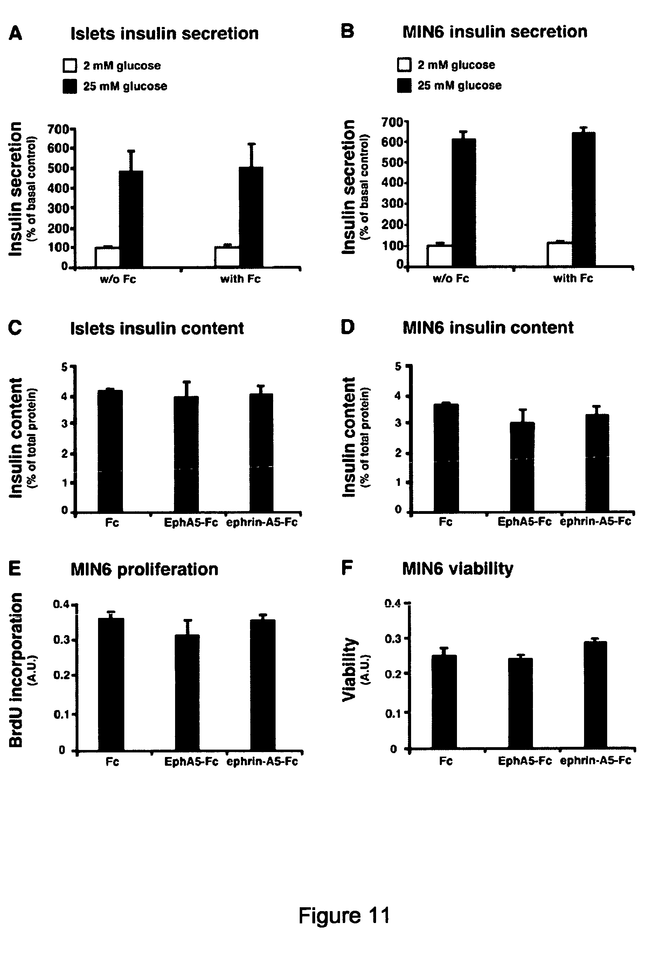

**Figure 11****. Controls for insulin secretion assays**
   (A, B) Insulin secretion from (A) mouse pancreatic islets and (B) MIN6 cells without (w/o) or with Fc control fragment at 2mM glucose (white columns) and 25mM glucose (black columns). Secreted insulin is normalized to insulin content and total protein content. N = 3 experiments. *p < 0.05. All values are means ± SD.
   (C, D) Insulin content of (C) mouse pancreatic islets and (D) MlN6 cells used for the insulin secretion measurements shown in Figures 3A and 3B, respectively. Insulin content is presented as % of total protein content. N = 3 experiments. All values are means ± SD.
   (E) BrdU incorporation assay in MIN6 cells treated with Fc, EphA5-Fc and ephrin-A5-Fc for 1 h. N = 3 experiments. All values are means ± SD.
   (F) WST-1 (Water-Soluble-Tetrazolium-1) viability assay of MIN6 cells treated with Fc, EphA5-Fc and ephrin-A5-Fc for 1 h. N = 3 experiments. All values are means ± SD.

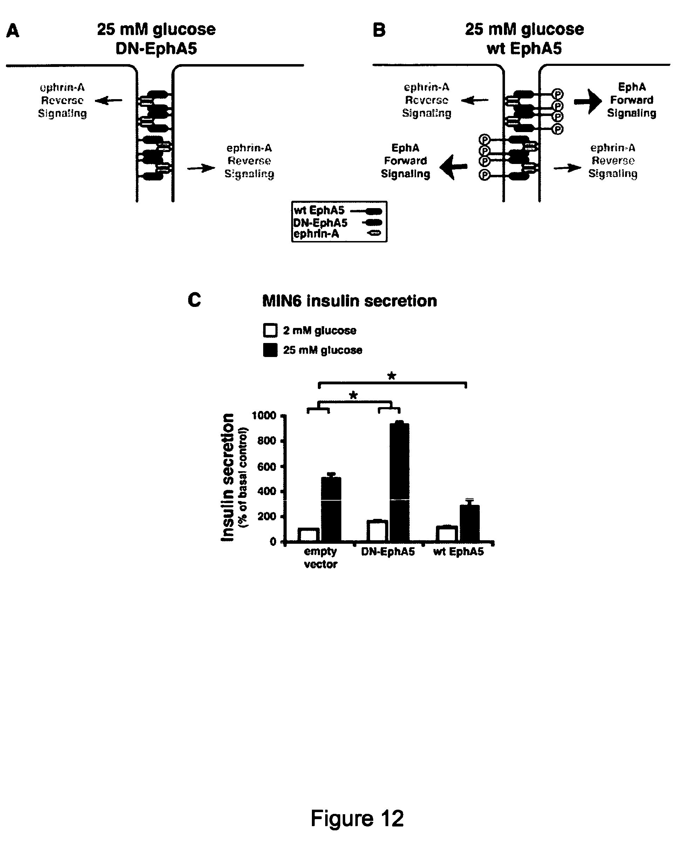

**Figure 12****. EphA forward signaling inhibits insulin secretion**
   (A, B) Model showing a possible role of the EphA5 cytoplasmic domain.
   (A) Over-expression of dominant-negative EphA5 with no cytoplasmic domain (DN-EphA5) blocks EphA5 forward signaling and activates ephrin-A reverse signaling. (B) Over-expression of full-length EphA5 (wt EphA5) activates EphA forward signaling and ephrin-A reverse signaling.
   (C) Insulin secretion from MIN6 cells transfected with empty vector as control, DN-EphA5 and wt EphA5 at 2mM glucose (white columns) and 25mM glucose (black columns). N = 3 experiments. *p < 0.05. All values are means ± SD.
**Figure 13****. Connexin-36 is required for the EphA5-Fc mediated effect on glucose-stimulated insulin secretion**
   (A, B) Connexin-36 (Cx36) staining (white) of MIN6 cells transfected with (A) control siRNA and (B) Cx36 siRNA 2. Cell nuclei were stained with DAPI (grey). Scale bars, 20 µm.
   (C) Insulin secretion from MIN6 cells transfected with control siRNA, Cx36 siRNA 1, Cx36 siRNA 2, Cx36 siRNA2 and Cx36 cDNA at 2mM glucose (white columns) and 25mM glucose (black columns). N = 3 experiments. *p ≤ 0.05. All values are means ± SD.

The Examples illustrate the invention. In particular, the examples demonstrate the surprising finding that the interaction between Eph receptor tyrosine kinases and ephrins is the molecular basis for enhanced insulin secretion under glucose stimulation which forms the necessary scientific basis for the embodiments of the invention.

### Experimental Procedures

### Cell culture and transfection procedure

MIN6 cells (Miyazaki et al. (1990) Endocrinol. 127, 126) at passages 37-47 were maintained as previously described (Nikolova et al. (2006) Dev. Cell 10, 397). The cells were electroporated with pEGFP, pEGFP-wtEphA5 (Gao et al. (1998) Proc. Natl. Acad. Sci. U.S.A. 95, 5329), pEGFP-DN-EphA5 (Gao et al. (1998) Proc. Natl. Acad. Sci. U.S.A. 95, 5329), pEGFP-ephrin-A5, (Wimmer-Kleikamp et al. (2004) J. Cell Biol. 164, 661), pEGFP-wtRac1, pEGFP-DN-Rac1 (Rac1N17) (Hall (2005) Biochem. Soc. Trans. 33, 891) and pcDNA-Cx36 constructs (Ravier et al. (2005) Diabetes 54, 1798); siRNA against firefly luciferase (control siRNA), ephrin-A5 and Cx36 by using Amaxa nucleofection (Amaxa biosystems).

### Mouse models, pancreatic islet culture and glucose tolerance test

Pancreatic islets were isolated from NMRI mice for all experiments, except for the experiments with male ephrin-A5-/- and control littermates, which were C57BU6 (Knoll et al. (2001) Development 128, 895). All mice used were 8-10 weeks old. Human pancreatic islets were isolated from a perfused human pancreas by using a protocol approved by the Ethical Research Committee of the Technical University Medical School Dresden and according to the law of the State of Saxony. Mouse and human islets were cultured overnight in Dulbecco's modified Eagle's medium (DMEM) containing 11mM glucose (Gibco), supplemented with 10% heat inactivated fetal calf serum (FCS), 100U/ml Penicillin, 100µg/ml Streptomycin, 1.5g/L NaHCO₃ in a humidified atmosphere (5% CO₂, 37°C). The glucose tolerance test was performed as previously described (Lammert et al. (2003) Curr. Biol. 13, 1070).

### RT-PCR, siRNA syntheses and real-time RT-PCR

Total RNA was extracted from MIN6 cells and mouse pancreatic islets by using RNeasy Mini kit (Qiagen) and transcribed into cDNA, which was used for RT-PCR. All Ephs and ephrins primers were initially tested on cDNA isolated from different mouse embryonic and adult organs as positive controls.
Small interfering RNA against the 3'-UTR of ephrin-A5 and Cx36 was prepared as previously described (Nikolova et al. (2006) Dev. Cell 10, 397). Ephrin-A5 and Cx36 knockdown efficiencies were monitored at the mRNA level using real-time RT-PCR (data not shown) and immunocytochemistry.

### Insulin secretion from pancreatic islets and MIN6 cells

For insulin secretion measurements, islets or MIN6 cells were starved for 1 h in Krebs Ringer Buffer (KRB) containing 115mM NaCl, 5mM KCI, 1.2mM KH₂PO₄, 1mM MgSO₄, 2.5mM CaCl₂, 24mM NaHCO₃, 2mM glucose, 25mM HEPES (pH 7.4), and 0.1% bovine serum albumin. After starvation, medium was exchanged for the same buffer +/- Fc fusion proteins to measure basal secretion, or for KRB containing 25mM glucose +/- Fc fusion proteins to measure glucose-stimulated insulin secretion, during 1 h incubation. When Fc fusion proteins were used, islets were continuously shaken (300-500 rpm) to facilitate access of Fc fusion proteins to islet β-cells. The amount of secreted insulin was measured in the medium, and islets or MIN6 cells were subsequently dissolved in RIPA buffer to measure insulin content and total protein content. Secreted insulin was normalized to total insulin content and total protein content, and presented as % of basal control insulin secretion. In all histograms, the first column represents the basal control (=100%), except in Figure 3C, in which the third column is taken as basal control. Secreted insulin and insulin content were measured by using ultra-sensitive rat insulin ELISA kit (Crystal Chem inc.). The total protein content was measured using BCA kit (Molecular Probes). Fc fusion proteins (R&D systems) and peroxovanadate (Sigma) were used at concentrations of 4µg/ml and 10µM, respectively. For determining viability and proliferation of islets and MIN6 cells during 1 h insulin secretion assay, a water-soluble tetrazolium (WST-1) reagent (Roche) and BrdU ELISA assay (Roche) were used, respectively.

### Confocal light microscopy and TIR-FM

Adult mouse pancreata, MIN6 cells and a biopsy of human pancreas were fixed in 4% PFA. After stepwise sucrose infiltration (9, 18, and 30%), the pancreatic tissues were embedded in O.C.T., deep frozen, and cut in 10µm thick cryosections. 1:100-diluted rabbit-anti-EphA5, rabbit-anti-EphA7, rabbit-anti-ephrin-A1 (Santa Cruz), goat-anti-ephrin-A5 (R&D systems), guinea pig-anti-insulin (DAKO), rabbit anti-connexin-36 antibodies and 4µg/ml Fc fusion proteins (R&D systems) were used for staining. 1:500-diluted secondary antibodies were used, conjugated with AlexaFluor488 (Molecular Probes) and with Cy5 (Dianova). 1µg/ml DAPI (Sigma) was used to stain cell nuclei. Monolayers of MIN6 cells were treated for 10 min. with or without Fc fusion proteins at 2mM or 25mM glucose, fixed and stained with 1:500-diluted phalloidin-rhodamine (Molecular Probes). Confocal images were acquired by using a Zeiss confocal microscope and intensities were quantified by using ImageJ software (NIH).
The Olympus total internal reflection-fluorescence microscope (TIR-FM) was used with a high-aperture objective lens (Apo 100x OHR; NA 1.65, Olympus) (Ohara-Imaizumi et al. (2004) Biochem. J. 381, 13). To monitor single insulin granules, adenovirus (insulin-GFP) infected primary mouse β-cells on high-refractive-index glass were mounted in an open chamber and incubated for 60 min at 37°C in KRB containing 2.2mM glucose (starvation). Cells were pre-incubated for 15 min. +/- Fc fusion proteins under basal conditions, transferred to a thermostat-controlled stage (37°C) and stimulated with glucose by the addition of 22mM glucose-KRB +/- 4µg/ml EphA5-Fc or ephrin-A5-Fc. Images were acquired every 300 ms, and images were analyzed by using Metamorph software (Molecular Devices).

### Immunoprecipitation and Western blot

MIN6 cells and pancreatic islets were lysed in RIPA buffer, supplemented with protease and phosphatase inhibitors. 1µg total protein, isolated from MIN6 cell lysates or 500ng total protein, isolated from islet lysates, were used for immunoprecipitation with 1:1,000-diluted rabbit-anti-EphA5 (Santa Cruz) and protein-A beads (Amersham). For immunoprecipitation/pull-down of Rac1-GTP with Pak1-PBD (P21-activated kinase-1 - P21 Binding Domain) and Western blots, a Rac1 Activation StressXPress Kit (Biomol) was used. The immunoprecipitates were separated on 4-12% gradient SDS-polyacrylamide gels (Invitrogen) and transferred onto a PVDF membrane (Amersham). For western blots, 1:500-diluted rabbit-anti-EphA5 antibody (Santa Cruz), 1:1,000-diluted mouse-anti-PY antibody (Biomol) and 1:200-diluted rabbit-anti-Rac1 antibody (Santa Cruz) were used. 1:10,000-diluted HRP-conjugated donkey-anti-rabbit antibody (Dianova) and 1:5,000-diluted HRP-conjugated goat-anti-mouse antibody (Dianova) were used as secondary antibodies. Western blots were developed using an ECL system (Amersham). The intensities of EphA5 and Rac1 bands were normalized to the intensities of total EphA5 and Rac1, respectively, and presented as % of basal control. The intensities of the bands were quantified by using TotalLab software (Stratagene).

### Statistical analysis

All values are means ± SD. Statistical significance was determined by using the two - tailed unpaired Student's t - test, and differences were considered to be statistically significant when p < 0.05.

**Table 2: Primer sequences (5' to 3') used for RT-PCR and siRNA production**

| SEQ ID NO | type | Name | DNA |
|---|---|---|---|
| 117 | artificial | ephrin- A1f | TTCAAATCCCAAGTTCCGTGAG |
| 118 | artificial | ephrin- A2f | AACTCTTCACCCCCTTTTCCC |
| 119 | artificial | ephrin- A3f | TACATCTCCACGCCCACTCACAAC |
| 120 | artificial | ephrin- A4f | CGATGCCTTTTGCCCCTTTC |
| 121 | artificial | ephrin- A5f | AACAGCAGCAACCCCAGATTC |
| 122 | artificial | EphA1f | GCCTTACGCCAACTACACATTTACC |
| 123 | artificial | EphA2f | TGAGGATGTCCGTTTTTCCAAG |
| 124 | artificial | EphA3f | GCAATGCTGGGTATGAAGAACG |
| 125 | artificial | EphA4f | GAACAACTGGCTGCGAACTGAC |
| 126 | artificial | EphA5f | GCAAGTATTATGGGGCAGTTCG |
| 127 | artificial | EphA6f | TCCTCTTTGGTTGAAGTGCGGG |
| 128 | artificial | EphA7f | AGCAGTCTCCAGTGAACAGAATCC |
| 129 | artificial | EphA8f | TCCATCAACGAGGTAGACGAGTCC |
| 130 | artificial | ephrin- A1r | CAGCAGCAGTGGTAGGAGCAATAC |
| 131 | artificial | ephrin- A2r | CTCATTGGTTGGACGCACATAAAC |
| 132 | artificial | ephrin- A3r | TCCCTCAAAGTCTTCCAACACG |
| 133 | artificial | ephrin- A4r | ATGTGATGACCCGCTCTCCTTG |
| 134 | artificial | ephrin-A5r | AAGCATCGCCAGGAGGAACAGTAG |
| 135 | artificial | EphA1r | GTCCACATAGGGTTTTAGCCACAG |
| 136 | artificial | EphA2r | TGCTGTTGACGAGGATGTTGCG |
| 137 | artificial | EphA3r | TAGTTGTGATGCTGACTGCGGC |
| 138 | artificial | EphA4r | TTCACCATTCTGCTCCTCGTGC |
| 139 | artificial | EphA5r | ATAGAGAGCAGCAGGGCAATCC |
| 140 | artificial | EphA6r | CAGTGTGTCTTGGGATGAAGCG |
| 141 | artificial | EphA7r | ATCCCAGCGGCAATACCTCTCAAC |
| 142 | artificial | EphA8r | GGGGCACTTCTTGTAGTAGATTCG |
| 143 | artificial | ephrin- A5 siRNA 1f | ATTAATACGACTCACTATAGGCAAGCCAGGGTTGATGAGTAG |
| 144 | artificial | ephrin- A5 siRNA 2f | ATTAATACGACTCACTATAGGAGCTGATTGCCAGGAAACAC |
| 145 | artificial | ephrin- A5 siRNA 1r | CGTAATACGACTCACTATAGGTCACAAGTTTAAATAGGACAAGCA |
| 146 | artificial | ephrin- A5 siRNA 2r | CGTAATACGACTCACTATAGGAAATGTTGGGTGCCTTCTGT |
| 147 | artificial | ephrin- A5f | TGTTGACGCTGCTCTTTCTGGTG |
| 148 | artificial | a- tubulinf | CTTTGAGCCAGCCAACCAGAT |
| 149 | artificial | ephrin- A5r | AATCTGGGGTTGCTGCTGTTCC . |
| 150 | artificial | a- tubulinr | TTGATGGTGGCAATGGCAG |
| 151 | artificial | Cx36 siRNA 1f | ATTAATACGACTCACTATAGGAGGGCAGGTTTGGGGAAG |
| 152 | artificial | Cx36 siRNA 2f | ATTAATACGACTCACTATAGGGCATGCCAGCTTTTCTTTTT |
| 153 | artificial | Cx36 siRNA 1 r | CGTAATACGACTCACTATAGGGGCCAGATGAAGGAAAAAGA |
| 154 | artificial | Cx36 siRNA 2r | CGTAATACGACTCACTATAGGCGGGCTGATTAAGGTCTCTG |
| 155 | artificial | Cx36f | ACGGTGTACGATGATGAGCA |
| 156 | artificial | Cx36r | GTAGAGTACCGGCGTTCTCG |

### Example 1: Ephrin-A and EphA expression in mouse and human pancreatic islets

We investigated the presence of Ephs and ephrins in pancreatic islets as possible candidate molecules involved in cell-cell communication (Figure 1 A). First, we studied the transcriptional profiles of Ephs and ephrins in mouse islets and mouse insulinoma cells, MIN6 (Figure 1 B). We detected similar transcription of ephrin-As and EphAs in islets and MIN6 cells (Figure 1 B). We also detected expression of ephrin-Bs and EphBs (data not shown), but focused on ephrin-As and EphAs due to their strong abundance and similar expression in both islets and MIN6 cells.
Immunostaining mouse pancreas sections showed co-expression of ephrin-A5 and EphA5 proteins in islets, and this expression was stronger in the islets than in the surrounding exocrine tissue (Figure 1C and 1E). A similar expression was observed in the human pancreas (Figure 8A-8F). Other ephrin-As and EphAs, such as ephrin-A1 and EphA7, were also expressed in mouse pancreatic islets (Figure 9A and 9B).
Next we stained mouse pancreas sections with EphA5-Fc and ephrin-A5-Fc fusion proteins, which bind with different affinity to virtually all ephrin-As and EphAs, respectively (Figure 1A) (Flanagan and Vanderhaeghen (1998) Annu. Rev. Neurosci. 21, 309). The staining showed that islet ephrin-As bind to EphA5 (Figure 1D) and that islet EphAs bind to ephrin-A5 (Figure 1 F).
EphA5 and ephrin-As co-localized in regions where MlN6 cells were in contact with each other (Figure 1G and 1H). ln line with the findings that EphAs and ephrin-As segregate in motor neurons (Marquardt et al. (2005) Cell 121, 127), and that several transmembrane proteins also segregate in insulinoma cells (Ohara-Imaizumi et al. (2004) J. Biol. Chem. 279, 8403; Uhles et al. (2003) J. Cell Biol. 163, 1327), we observed little co-localization of EphA5 and ephrin-As on the free surfaces of MIN6 cells (Figure 1I). Moreover, based on the EphA5-Fc and ephrin-A5-Fc staining of non-permeabilized MIN6 cells, we found that ephrin-As were more strongly localized to the plasma membrane compared to the EphAs (Figure 10A and 10B). Conversely, EphAs strongly localized to insulin secretory granules (Figure 10D), whereas ephrin-As did not (Figure 10C).
These results show that EphAs and ephrin-As are co-expressed in β-cells, and suggest that EphA-ephrin-A bidirectional signaling may take place between adjacent β-cells (Figure 1A).

### Example 2: Ephrin-A5 is required for glucose-stimulated insulin secretion

To investigate whether ephrin-A5, a possible participant in bidirectional signaling between β-cells, was required for insulin secretion, we compared control islets with ephrin-A5 deficient islets (ephrin-A5-/-) (Figure 2A-2C). In comparison with control islets, ephrin-A5-/- islets had a significantly reduced glucose-stimulated insulin secretion (compare black columns in Figure 2A). These results show that ephrin-A5 is required for normal β-cell insulin secretory response to glucose (compare Δs in Figure 2A). In contrast, we did not detect any differences in the insulin content between control and ephrin-A5-/- islets (Figure 2B), thus excluding an effect of ephrin-A5 on insulin production. In line with the insulin secretion defects observed in ephrin-A5-/- mouse islets (Figure 2A), we also detected impaired glucose tolerance in the ephrin-A5 deficient mice (Figure 2C).
To rule out the possibility that the observed defects were due to changes in non-β-cells, which are also present in islets, we knocked down ephrin-A5 in MlN6 cells (Figure 2D and 2E), a frequently used model for β-cells (Miyazaki et al. (1990) Endocrinol. 127, 126). We noticed that ephrin-A5 knockdown led to significantly increased basal insulin secretion (white columns in Figure 2F), as well as significantly reduced glucose-stimulated insulin secretion (black columns in Figure 2F). In addition, we could reproduce this effect with a second set of siRNA molecules. To provide evidence for the specificity of the knockdown, we rescued the insulin secretory response to glucose by cotransfecting cells with an ephrin-A5 cDNA, which lacked the 3'-UTR targeted by the siRNA (Figure 2F). These results show that ephrin-A5 is involved in suppression of basal insulin secretion and is required for glucose-stimulated insulin secretion.

### Example 3: Opposite effects of EphA5-Fc and ephrin-A5-Fc on insulin secretion

Communication between β-cells was shown to inhibit insulin secretion at low glucose concentrations, but stimulate insulin secretion at high glucose concentrations, and our data suggest that ephrin-A5 is involved in these communication effects. To uncover the underlying molecular mechanism, we first investigated the effects of ephrin-A reverse signaling, and EphA forward signaling, on insulin secretion (Figure 3). We used EphA5-Fc to stimulate ephrin-A reverse signaling, and ephrin-A5-Fc to stimulate EphA forward signaling (Figure 3A and 3B). However, it is worth noting that EphA5-Fc and ephrin-A5-Fc fusion proteins may also interfere with endogenous EphA forward signaling and ephrin-A reverse signaling, respectively, when β-cells interact with each other.
Because ephrin-As and EphAs are promiscuous (Flanagan and Vanderhaeghen (1998) Annu. Rev. Neurosci. 21, 309), the EphA5-Fc and ephrin-A5-Fc fusion proteins allowed us to manipulate virtually all relevant ephrin-As and EphAs in β-cells, respectively. We first confirmed that control Fc fragments did not affect insulin secretion from either pancreatic islets or MIN6 cells (Figures 11A and 11B). We then showed that EphA5-Fc significantly increased basal and glucose-stimulated insulin secretion (compare EphA5-Fc with Fc in Figure 3A), whereas ephrin-A5-Fc significantly decreased glucose-stimulated insulin secretion from pancreatic islets (compare ephrin-A5-Fc with Fc in Figure 3A).
Islets consist mainly of β-cells, but also harbor other cell types, such as α-cells and endothelial cells that might have been affected in these experiments. Therefore, we tested the role of ephrin-A reverse signaling and EphA forward signaling in MIN6 cells (Figure 3B), and obtained similar results: EphA5-Fc increased basal and glucose-stimulated insulin secretion (compare EphA5-Fc with Fc in Figure 3B), whereas ephrin-A5-Fc decreased glucose-stimulated insulin secretion (compare ephrin-A5-Fc with Fc in Figure 3B). Moreover, EphA7-Fc and ephrin-A1-Fc had similar effects on glucose-stimulated insulin secretion (Figure 9C).
The effects on insulin secretion did not result from any changes in insulin content, cell division or cell viability during the 1-hour incubation time with EphA5-Fc and ephrin-A5-Fc (Figures 11C-11F). In addition, we also showed in human islets that EphA5-Fc strongly increased basal and glucose-stimulated insulin secretion, whereas ephrin-A5-Fc suppressed glucose-stimulated insulin secretion (Figure 8G). These results therefore demonstrate that EphA5-Fc and ephrin-A5-Fc effectively and conversely change insulin secretion from mouse and human pancreatic islets.

### Example 4: EphA5-Fc partially rescues glucose-stimulated insulin secretion in cells with reduced cell-cell contact

Glucose-stimulated insulin secretion is decreased in situations of reduced β-cell communication. Therefore we asked whether EphA5-Fc restored glucose-stimulated insulin secretion in non-confluent MIN6 cells (Figure 3C). We first showed that non-confluent cells ("Low cell density") had lower glucose-stimulated insulin secretion compared to confluent cells ("High cell density") (compare white columns in Figure 3C). We then showed that EphA5-Fc partially restored glucose-stimulated insulin secretion in non-confluent cells (compare last two columns in Figure 3C). These results suggest that endogenous ephrin-A reverse signaling enhances glucose-stimulated insulin secretion.

### Example 5: Ephrin-A5-Fc fully rescues suppression of basal insulin secretion in cells with reduced cell-cell contact

In the experiments with islets, as well as confluent MIN6 cell monolayers, we noticed that ephrin-A5-Fc reduced glucose-stimulated insulin secretion, but did not suppress basal insulin secretion (Figure 3A and 3B). We hypothesized that basal insulin secretion already was maximally suppressed by endogenous EphA - ephrin-A interactions and, therefore, we could not further decrease basal insulin secretion by using ephrin-A5-Fc. If this were the case, one would expect that ephrin-A5-Fc was capable of decreasing basal insulin secretion, when the extent of endogenous EphA - ephrin-A interactions was less. Thus, we performed the experiments with non-confluent MIN6 cells, which had lower levels of endogenous EphA forward signaling as a result of less cell-cell contact.
As shown in Figure 3D, non-confluent MIN6 cells had significantly enhanced basal insulin secretion (compare white columns). In support of the hypothesis that this was due to reduced endogenous EphA forward signaling, we observed that EphA5 phosphorylation levels were lower in non-confluent MIN6 cells compared to confluent cells (Figure 3E; compare white columns in Figure 3F). Importantly, ephrin-A5-Fc decreased basal insulin secretion in non-confluent cells to a level characteristic of confluent MlN6 cells (compare last two columns in Figure 3D). Consistent with the idea that this was due to exogenous activation of EphA forward signaling, EphA5 phosphorylation levels were significantly increased upon ephrin-A5-Fc treatment of non-confluent MIN6 cells (Figure 3E; compare last two columns in Figure 3F). Ephrin-A5-Fc treatment even induced a stronger EphA5 phosphorylation in non-confluent MIN6 cells than in confluent ones (Figure 3F), possibly due to increased fusion of insulin secretory granules, which carry EphAs to the plasma membrane, where they can bind to ephrin-A5-Fc. These results suggest that endogenous EphA forward signaling inhibits basal insulin secretion.

### Example 6: Ephrin-A reverse signaling stimulates insulin secretion

The previous experiments strongly suggested that ephrin-A reverse signaling enhanced insulin secretion (Figure 3). Here we used single primary β-cells to stimulate ephrin-A reverse signaling with EphA5-Fc, without simultaneously affecting endogenous EphA forward signaling (Figure 4A). This was possible because the cells did not contact each other and, therefore, their ephrin-As could not interact with the EphAs of any adjacent cells (Figure 4A).
We expressed GFP-tagged insulin in primary mouse β-cells and stimulated these cells with glucose, in the presence or absence of EphA5-Fc (Figure 4A). We then monitored the secretory events in single β-cells by using total internal reflection-fluorescence microscopy (TIR-FM). Time-lapse TIR-FM allowed us to discriminate between fusion events of previously docked and newly recruited insulin secretory granules (Ohara-Imaizumi et al. (2004) Biochem. J. 381, 13). As shown in Figure 4B, EphA5-Fc treatment significantly increased fusion events of newly recruited insulin secretory granules (white columns).
Glucose-stimulated insulin secretion, in response to a sudden increase in glucose concentration, follows a biphasic time course consisting of a rapid 1^{st} phase followed by a sustained 2^{nd} phase (Rorsman and Renstrom (2003) Diabetologia 46, 1029). We found that EphA5-Fc increased the number of secretory events during both phases of insulin secretion with a stronger effect on the 2^{nd} phase (compare Figure 4C and 4D). We conclude from these experiments that ephrin-A reverse signaling stimulates insulin secretion.

### Example 7: EphA forward signaling suppresses insulin secretion

The experiments, shown in Figure 3, strongly suggested that EphA forward signaling suppressed insulin secretion. Here we used TIR-FM of single ephrin-A5-Fc treated β-cells to provide evidence of a suppressive effect of EphA forward signaling (Figure 4E). As shown in Figure 4F, ephrin-A5-Fc treatment significantly reduced the number of fusion events of newly recruited insulin secretory granules (white columns). In addition, ephrin-A5-Fc reduced the number of secretory events during both phases of insulin secretion with a stronger effect on the 2^{nd} phase (compare Figure 4G and 4H). As additional evidence of an inhibitory effect of EphA forward signaling on insulin secretion, a dominant-negative EphA5 protein (DN-EphA5), lacking its cytoplasmic domain, was expressed in MIN6 cells (Figure 12A). This DN-EphA5 binds to ephrin-As and this binding is expected to induce ephrin-A reverse signaling, but not EphA forward signaling. In contrast, over-expression of a full-length EphA5 protein (wt EphA5) is expected to induce ephrin-A reverse signaling, and also EphA forward signaling (Figure 12B). In support of an inhibitory effect of EphA forward signaling, we found that DN-EphA5 increased basal and glucose-stimulated insulin secretion, whereas wt EphA5 over-expression decreased glucose-stimulated insulin secretion in confluent MIN6 cell monolayers (Figure 12C). We conclude that EphA forward signaling suppresses insulin secretion.

### Example 8: Downstream targets of EphA-ephrin-A signaling and their impact on insulin secretion

It has been shown that destabilization of F-actin enhances insulin secretion, whereas its stabilization inhibits insulin secretion (Tomas et al. (2006) J. Cell Sci. 119, 2156). This led to the hypothesis that dense cortical F-actin network limits access of insulin secretory granules to the plasma membrane. However, since complete F-actin depolymerization inhibits glucose-stimulated insulin secretion (Li et al. (1994) Mol. Biol. Cell 5, 1199), certain actin filaments appear to be required for the secretory process.
Because ephrin-A reverse signaling, and EphA forward signaling, were shown to rearrange F-actin in several cell types (Marquardt et al. (2005) Cell 121, 127), we tested whether EphA5-Fc and ephrin-A5-Fc also changed the F-actin state of mouse insulinoma cells (Figure 5A-5E). We found that EphA5-Fc resulted in F-actin rearrangements with a subtle decrease in F-actin intensity under basal conditions (compare Figure 5A and 5B), consistent with its stimulatory effect on insulin secretion. In contrast, ephrin-A5-Fc strongly increased F-actin polymerization under basal and stimulatory conditions (compare Figure 5C and 5D), consistent with its inhibitory effect on insulin secretion.
EphAs modulate activity of Rac1, a Rho-GTPase involved in F-actin remodeling and endocytosis (Murai and Pasquale (2005) Neuron 46, 161). Since Rac1 is also involved in glucose-stimulated insulin secretion (Kowluru and Veluthakal (2005) Diabetes 54, 3523; Li et al. (2004) Am. J. Physiol. Endocrinol. Metab. 286, E818), we investigated whether EphA5-Fc and ephrin-A5-Fc modulated Rac1 activity in MlN6 cells. ln line with previous reports (Kowluru and Veluthakal (2005) Diabetes 54, 3523; Li et al. (2004) Am. J. Physiol. Endocrinol. Metab. 286, E818), glucose-stimulation significantly enhanced Rac1 activity (Fc, Figure 5F and 5G). Consistent with its stimulatory effects on insulin secretion, we found that EphA5-Fc stimulated Rac1 activity under basal and stimulatory conditions (compare Fc with EphA5-Fc in Figure 5G). In contrast, ephrin-A5-Fc inhibited Rac1 activity in confluent MIN6 cells under stimulatory conditions, consistent with its inhibitory effect on insulin secretion (compare Fc with ephrin-A5-Fc in Figure 5G). In addition, EphA5-Fc and ephrin-A5-Fc required Rac1 activity for their effects on glucose-stimulated insulin secretion (compare DN-Rac1 with empty vector in Figure 5H). These results suggest that EphA5-Fc and ephrin-A5-Fc modulate glucose-stimulated insulin secretion by affecting Rac1 activity and F-actin in β-cells.
Since Eph-ephrin signaling was also shown to affect connexin localization and gap junction communication (Mellitzer et al. (1999) Nature 400: 77; Davy et al. (2006) PLOS Biol. 4: 1763), we investigated whether connexin-36, the gap junction protein of β-cells (Ravier et al. (2005) Diabetes 54: 1798), was required for the effects of Eph-ephrin signaling on insulin secretion (Figure 13). We showed that the stimulatory effect of EphA5-Fc on basal and glucose-stimulated insulin secretion required connexin-36 (compare Fc with EphA5-Fc in Figure 13C), whereas the inhibitory effect of ephrin-A5-Fc did not (compare Fc with ephrin-A5-Fc in Figure 13F).
Taken together, these results show that EphA-ephrin-A signaling affects several β-cell components required for regulating insulin secretion: F-actin, Rac1 and connexin-36.

### Example 9: Glucose-induced dephosphorylation of EphA5

EphAs are receptor tyrosine kinases (RTKs) that oligomerize upon ephrin-A binding (Kullander and Klein (2002) Nat. Rev. Mol. Cell Biol. 3, 475). The oligomerization brings the cytoplasmic EphA RTK domains in close proximity to each other, thereby allowing their trans-phosphorylation, which promotes EphA forward signaling. The level of kinase-dependent EphA forward signaling can therefore be determined by measuring the levels of EphA tyrosine phosphorylation. Here we investigated the hypothesis that EphA forward signaling was active at low glucose concentrations in order to suppress insulin secretion (Figure 6A). We also investigated whether, upon glucose stimulation, EphA forward signaling was downregulated by dephosphorylation (Figure 6B), thus allowing predominance of ephrin-A reverse signaling, which stimulated insulin secretion.
In support of this hypothesis, we found that the EphA5 phosphorylation levels were higher at a low glucose concentration (2mM) compared to all depicted time points after glucose stimulation (25mM) in both islets and MIN6 cells (Figures 6C and 6D). In islets, the presence of phosphorylated EphA5 decreased shortly after glucose stimulation (compare column 1 with columns 2 to 4 in Figure 6C). However, when the islets were brought back to low glucose concentration, EphA5 phosphorylation reached basal levels again (compare column 1 with column 5 in Figure 6C).
The level of EphA5 phosphorylation was easier to determine in MIN6 cells, because these cells were not mixed with non-β-cells and could be obtained in large quantities (Figure 6D). As with islets, the amount of phosphorylated EphA5 decreased shortly after glucose stimulation (compare column 1 with columns 2 to 5 in Figure 6D). At the 5^{th} minute, EphA5 phosphorylation was reduced to about 20% of basal levels (compare column 1 with column 3 in Figure 6D). Similar to the islets, bringing back glucose-stimulated MIN6 cells to a low glucose concentration fully restored basal EphA5 phosphorylation levels (compare last column with first column in Figure 6D).
To demonstrate that EphA5 was not the only EphA to be dephosphorylated in β-cells, we performed experiments with other members of the EphA family and observed a similar dephosphorylation upon glucose stimulation (data not shown).
Since ephrin-A5-Fc inhibited insulin secretion, we tested whether this effect correlated with the phosphorylation levels of EphA5. We noticed that treatment of islets with ephrin-A5-Fc resulted in elevated EphA5 phosphorylation under stimulatory conditions (compare column 3 with last column in Figure 6C). In addition, ephrin-A5-Fc treatment of non-confluent MIN6 cells significantly increased EphA5 phosphorylation also under basal conditions (Figure 3E and 3F; Figure 6E and 6F). Thus, EphA5 phosphorylation levels correlate with inhibition of insulin secretion.
We then tested whether a protein tyrosine phosphatase (PTP) was involved in dephosphorylating EphAs in response to glucose stimulation (Figure 6B). We observed that 10µM peroxovanadate (PV), a PTP inhibitor, effectively prevented glucose-induced EphA5 dephosphorylation (Figure 6G), and even increased EphA5 phosphorylation levels at stimulatory conditions above the levels characteristic of basal conditions (compare columns in Figure 6E and 6G). These results therefore suggest that EphA forward signaling is active at low glucose concentrations in order to inhibit insulin secretion (Figure 6A), and that EphA forward signaling is downregulated by dephosphorylation, upon glucose stimulation (Figure 6B).

### Example 10: EphA5 dephosphorylation is required for glucose-stimulated insulin secretion

lt was previously shown that treatment of islets with 10µM PV strongly reduced glucose-stimulated insulin secretion (Gogg et al. (2001) Biochem. Biophys. Res. Commun. 280, 1161). Based on our experiments, we hypothesized that PV inhibited glucose-stimulated insulin secretion partially by preventing glucose-induced EphA dephosphorylation (Figure 7A). After confirming that 10µM PV reduced glucose-stimulated insulin secretion (Figures 7D and 7E), we tested whether insulin secretion could be rescued in PV-treated MlN6 cells by overexpression of DN-EphA5 (Figure 7B). We also tested whether insulin secretion could be further reduced in PV-treated MlN6 cells by overexpression of wt EphA5 (Figure 7C). We found that DN-EphA5 partially rescued glucose-stimulated insulin secretion in PV-treated MlN6 cells (compare Empty vector/PV with DN-EphA5/PV in Figure 7E). ln contrast, wt EphA5 reduced glucose-stimulated insulin secretion in PV-treated MlN6 cells even further (compare empty vector/PV with wt EphA5/PV in Figure 7E). We conclude that downregulation of EphA forward signaling is essential for glucose-stimulated insulin secretion.

## Claims

1. A method of identifying a compound suitable as a lead compound and/or as a medicament for the treatment and/or prevention of diabetes, the method comprising the steps of
(a) contacting a test compound with a cell comprising a protein, wherein said protein
(i) comprises or consists of the amino acid sequence of any one of SEQ ID NOs: 1 to 29;
(ii) is encoded by a nucleic acid molecule comprising or consisting of the sequence of any one of SEQ ID NOs: 59 to 87;
(iii) is a fragment of the protein according to (i) or (ii) and exhibits Eph-Receptor-Tyrosine-Kinase activity; or
(iv) has a sequence at least 75% identical with the protein according to (i) or (ii) or with the fragment according to (iii) and exhibits Eph-Receptor-Tyrosine-Kinase activity; and
(b) determining whether said test compound, upon contacting in step (a) inhibits the Eph receptor tyrosine kinase activity of said protein wherein said inhibition indicates a compound suitable as a lead compound and/or as a medicament for the treatment and/or prevention of diabetes.

2. A method of identifying a compound suitable as a lead compound and/or as a medicament for the treatment and/or prevention of diabetes, the method comprising the steps of
(a) contacting a test compound with a protein, wherein said protein
(i) comprises or consists of the amino acid sequence of any one of SEQ ID NOs: 1 to 29, or
(ii) is encoded by a nucleic acid molecule comprising or consisting of the sequence of any one of SEQ ID NOs: 59 to 87, or
(iii) is a fragment of the protein according to (i) or (ii) and exhibits Eph-Receptor-Tyrosine-Kinase activity, or
(iv) has a sequence at least 75% identical with the protein according to (i) or (ii) or with the fragment according to (iii) and exhibits Eph-Receptor-Tyrosine-Kinase activity; and
(b) determining whether said test compound, upon contacting in step (a) inhibits the Eph receptor tyrosine kinase activity of said protein wherein said inhibition indicates a compound suitable as a lead compound and/or as a medicament for the treatment and/or prevention of diabetes.

3. A method of identifying a compound suitable as a lead compound and/or as a medicament for the treatment and/or prevention of diabetes, the method comprising the steps of
(a) contacting a test compound with a cell comprising a protein, wherein said protein
(i) comprises or consists of the amino acid sequence of any one of SEQ lD NOs: 48 to 58, or
(ii) is encoded by a nucleic acid molecule comprising or consisting of the sequence of any one of SEQ ID NOs: 106 to 116, or
(iii) is a fragment of the protein according to (i) or (ii) and acts as a protein tyrosine phosphatase for Eph receptor tyrosine kinases; or
(iv) has a sequence at least 75% identical with the protein according to (i) or (ii) or with the fragment according to (iii) and acts as a protein tyrosine phosphatase for Eph receptor tyrosine kinases; and
(b) determining whether said test compound, upon contacting in step (a) activates the expression and/or activity of said protein wherein said activation indicates a compound suitable as a lead compound and/or as a medicament for the treatment and/or prevention of diabetes.

4. A method of identifying a compound suitable as a lead compound and/or as a medicament for the treatment and/or prevention of diabetes, the method comprising the steps of
(a) contacting a test compound with a protein, wherein said protein
(i) comprises or consists of the amino acid sequence of any one of SEQ ID NOs: 48 to 58, or
(ii) is encoded by a nucleic acid molecule comprising or consisting of the sequence of any one of SEQ lD NOs: 106 to 116, or
(iii) is a fragment of the protein according to (i) or (ii) and acts as a protein tyrosine phosphatase for Eph receptor tyrosine kinases; or
(iv) has a sequence at least 75% identical with the protein according to (i) or (ii) or with the fragment according to (iii) and acts as a protein tyrosine phosphatase for Eph receptor tyrosine kinases; and
(b) determining whether said test compound, upon contacting in step (a) enhances the activity of said protein wherein said enhancement indicates a compound suitable as a lead compound and/or as a medicament for the treatment and/or prevention of diabetes.

5. A method of identifying a compound suitable as a lead compound and/or as a medicament for the treatment and/or prevention of diabetes, the method comprising the steps of
(a) contacting a test compound with a cell comprising a protein, wherein said protein
(i) comprises or consists of the amino acid sequence of any one of SEQ ID NOs: 30 to 47, or
(ii) is encoded by a nucleic acid molecule comprising or consisting of the sequence of any one of SEQ ID NOs: 88 to 105, or
(iii) is a fragment of the protein according to (i) or (ii) and exhibits ephrin activity, or
(iv) has a sequence at least 75% identical with the protein according to (i) or (ii) or with the fragment according to (iii) and exhibits ephrin activity; and
(b) determining whether said test compound, upon contacting in step (a) activates the expression and/or activity of said protein wherein said activation indicates a compound suitable as a lead compound and/or as a medicament for the treatment and/or prevention of diabetes.

6. The method of any one of claims 1 to 5, wherein said cell is a pancreatic beta-cell or a cell from a beta-cell line.

7. The method of any one of claims 1 to 6, wherein determining in step (b) comprises quantifying insulin secretion.

8. The method of any one of claims 1 to 6 wherein determining in step (b) comprises quantifying F-actin in said cell, wherein the quantity of F-actin is determined at stimulatory glucose concentrations and wherein a decreased quantity of F-actin in the cell contacted with the test compound indicates a compound suitable as a lead compound and/or as a medicament for the treatment and/or prevention of diabetes.

9. The method of any of the preceding claims, wherein said protein is comprised in a cell expressing, or transfected with, a nucleic acid encoding said protein.

10. The method of any of the preceding claims, wherein said cell is comprised in a non-human animal.

11. The method of claim 10, wherein determining in step (b) involves quantifying insulin secretion


SEQUENCE LISTING

<110>  Max-Planck-Gesellschaft zur Förderung der Wissenschaften
       e.V.

<120>  Screening-method for anti-diabetic compounds

<130>  M 2788 EP

<160>  156

<170>  PatentIn version 3.3

<210>  1
<211>  976
<212>  PRT
<213>  human

<400>  1

Met Glu Arg Arg Trp Pro Leu Gly Leu Gly Leu Val Leu Leu Leu Cys
1               5                   10                  15

Ala Pro Leu Pro Pro Gly Ala Arg Ala Lys Glu Val Thr Leu Met Asp
            20                  25                  30

Thr Ser Lys Ala Gln Gly Glu Leu Gly Trp Leu Leu Asp Pro Pro Lys
        35                  40                  45

Asp Gly Trp Ser Glu Gln Gln Gln Ile Leu Asn Gly Thr Pro Leu Tyr
    50                  55                  60

Met Tyr Gln Asp Cys Pro Met Gln Gly Arg Arg Asp Thr Asp His Trp
65                  70                  75                  80

Leu Arg Ser Asn Trp Ile Tyr Arg Gly Glu Glu Ala Ser Arg Val His
                85                  90                  95

Val Glu Leu Gln Phe Thr Val Arg Asp Cys Lys Ser Phe Pro Gly Gly
            100                 105                 110

Ala Gly Pro Leu Gly Cys Lys Glu Thr Phe Asn Leu Leu Tyr Met Glu
        115                 120                 125

Ser Asp Gln Asp Val Gly Ile Gln Leu Arg Arg Pro Leu Phe Gln Lys
    130                 135                 140

Val Thr Thr Val Ala Ala Asp Gln Ser Phe Thr Ile Arg Asp Leu Ala
145                 150                 155                 160

Ser Gly Ser Val Lys Leu Asn Val Glu Arg Cys Ser Leu Gly Arg Leu
                165                 170                 175

Thr Arg Arg Gly Leu Tyr Leu Ala Phe His Asn Pro Gly Ala Cys Val
            180                 185                 190

Ala Leu Val Ser Val Arg Val Phe Tyr Gln Arg Cys Pro Glu Thr Leu
        195                 200                 205

Asn Gly Leu Ala Gln Phe Pro Asp Thr Leu Pro Gly Pro Ala Gly Leu
    210                 215                 220

Val Glu Val Ala Gly Thr Cys Leu Pro His Ala Arg Ala Ser Pro Arg
225                 230                 235                 240

Pro Ser Gly Ala Pro Arg Met His Cys Ser Pro Asp Gly Glu Trp Leu
                245                 250                 255

Val Pro Val Gly Arg Cys His Cys Glu Pro Gly Tyr Glu Glu Gly Gly
            260                 265                 270

Ser Gly Glu Ala Cys Val Ala Cys Pro Ser Gly Ser Tyr Arg Met Asp
        275                 280                 285

Met Asp Thr Pro His Cys Leu Thr Cys Pro Gln Gln Ser Thr Ala Glu
    290                 295                 300

Ser Glu Gly Ala Thr Ile Cys Thr Cys Glu Ser Gly His Tyr Arg Ala
305                 310                 315                 320

Pro Gly Glu Gly Pro Gln Val Ala Cys Thr Gly Pro Pro Ser Ala Pro
                325                 330                 335

Arg Asn Leu Ser Phe Ser Ala Ser Gly Thr Gln Leu Ser Leu Arg Trp
            340                 345                 350

Glu Pro Pro Ala Asp Thr Gly Gly Arg Gln Asp Val Arg Tyr Ser Val
        355                 360                 365

Arg Cys Ser Gln Cys Gln Gly Thr Ala Gln Asp Gly Gly Pro Cys Gln
    370                 375                 380

Pro Cys Gly Val Gly Val His Phe Ser Pro Gly Ala Arg Gly Leu Thr
385                 390                 395                 400

Thr Pro Ala Val His Val Asn Gly Leu Glu Pro Tyr Ala Asn Tyr Thr
                405                 410                 415

Phe Asn Val Glu Ala Gln Asn Gly Val Ser Gly Leu Gly Ser Ser Gly
            420                 425                 430

His Ala Ser Thr Ser Val Ser Ile Ser Met Gly His Ala Glu Ser Leu
        435                 440                 445

Ser Gly Leu Ser Leu Arg Leu Val Lys Lys Glu Pro Arg Gln Leu Glu
    450                 455                 460

Leu Thr Trp Ala Gly Ser Arg Pro Arg Ser Pro Gly Ala Asn Leu Thr
465                 470                 475                 480

Tyr Glu Leu His Val Leu Asn Gln Asp Glu Glu Arg Tyr Gln Met Val
                485                 490                 495

Leu Glu Pro Arg Val Leu Leu Thr Glu Leu Gln Pro Asp Thr Thr Tyr
            500                 505                 510

Ile Val Arg Val Arg Met Leu Thr Pro Leu Gly Pro Gly Pro Phe Ser
        515                 520                 525

Pro Asp His Glu Phe Arg Thr Ser Pro Pro Val Ser Arg Gly Leu Thr
    530                 535                 540

Gly Gly Glu Ile Val Ala Val Ile Phe Gly Leu Leu Leu Gly Ala Ala
545                 550                 555                 560

Leu Leu Leu Gly Ile Leu Val Phe Arg Ser Arg Arg Ala Gln Arg Gln
                565                 570                 575

Arg Gln Gln Arg Gln Arg Asp Arg Ala Thr Asp Val Asp Arg Glu Asp
            580                 585                 590

Lys Leu Trp Leu Lys Pro Tyr Val Asp Leu Gln Ala Tyr Glu Asp Pro
        595                 600                 605

Ala Gln Gly Ala Leu Asp Phe Thr Arg Glu Leu Asp Pro Ala Trp Leu
    610                 615                 620

Met Val Asp Thr Val Ile Gly Glu Gly Glu Phe Gly Glu Val Tyr Arg
625                 630                 635                 640

Gly Thr Leu Arg Leu Pro Ser Gln Asp Cys Lys Thr Val Ala Ile Lys
                645                 650                 655

Thr Leu Lys Asp Thr Ser Pro Gly Gly Gln Trp Trp Asn Phe Leu Arg
            660                 665                 670

Glu Ala Thr Ile Met Gly Gln Phe Ser His Pro His Ile Leu His Leu
        675                 680                 685

Glu Gly Val Val Thr Lys Arg Lys Pro Ile Met Ile Ile Thr Glu Phe
    690                 695                 700

Met Glu Asn Gly Ala Leu Asp Ala Phe Leu Arg Glu Arg Glu Asp Gln
705                 710                 715                 720

Leu Val Pro Gly Gln Leu Val Ala Met Leu Gln Gly Ile Ala Ser Gly
                725                 730                 735

Met Asn Tyr Leu Ser Asn His Asn Tyr Val His Arg Asp Leu Ala Ala
            740                 745                 750

Arg Asn Ile Leu Val Asn Gln Asn Leu Cys Cys Lys Val Ser Asp Phe
        755                 760                 765

Gly Leu Thr Arg Leu Leu Asp Asp Phe Asp Gly Thr Tyr Glu Thr Gln
    770                 775                 780

Gly Gly Lys Ile Pro Ile Arg Trp Thr Ala Pro Glu Ala Ile Ala His
785                 790                 795                 800

Arg Ile Phe Thr Thr Ala Ser Asp Val Trp Ser Phe Gly Ile Val Met
                805                 810                 815

Trp Glu Val Leu Ser Phe Gly Asp Lys Pro Tyr Gly Glu Met Ser Asn
            820                 825                 830

Gln Glu Val Met Lys Ser Ile Glu Asp Gly Tyr Arg Leu Pro Pro Pro
        835                 840                 845

Val Asp Cys Pro Ala Pro Leu Tyr Glu Leu Met Lys Asn Cys Trp Ala
    850                 855                 860

Tyr Asp Arg Ala Arg Arg Pro His Phe Gln Lys Leu Gln Ala His Leu
865                 870                 875                 880

Glu Gln Leu Leu Ala Asn Pro His Ser Leu Arg Thr Ile Ala Asn Phe
                885                 890                 895

Asp Pro Arg Met Thr Leu Arg Leu Pro Ser Leu Ser Gly Ser Asp Gly
            900                 905                 910

Ile Pro Tyr Arg Thr Val Ser Glu Trp Leu Glu Ser Ile Arg Met Lys
        915                 920                 925

Arg Tyr Ile Leu His Phe His Ser Ala Gly Leu Asp Thr Met Glu Cys
    930                 935                 940

Val Leu Glu Leu Thr Ala Glu Asp Leu Thr Gln Met Gly Ile Thr Leu
945                 950                 955                 960

Pro Gly His Gln Lys Arg Ile Leu Cys Ser Ile Gln Gly Phe Lys Asp
                965                 970                 975

<210> 2
<211> 977
<212> PRT
<213> mouse

<400> 2

Met Glu Arg Arg Trp Pro Leu Gly Leu Ala Leu Leu Leu Leu Leu Leu

1               5                   10                  15

Cys Ala Pro Leu Pro Pro Gly Ala Arg Ala Glu Glu Val Thr Leu Met
            20                  25                  30

Asp Thr Ser Thr Ala Gln Gly Glu Leu Gly Trp Leu Leu Asp Pro Pro
        35                  40                  45

Glu Thr Gly Trp Ser Glu Val Gln Gln Met Leu Asn Gly Thr Pro Leu
    50                  55                  60

Tyr Met Tyr Gln Asp Cys Pro Ile Gln Glu Gly Gly Asp Thr Asp His
65                  70                  75                  80

Trp Leu Arg Ser Asn Trp Ile Tyr Arg Gly Glu Glu Ala Ser Arg Ile
                85                  90                  95

Tyr Val Glu Leu Gln Phe Thr Val Arg Asp Cys Lys Ser Phe Pro Gly
            100                 105                 110

Gly Ala Gly Pro Leu Gly Cys Lys Glu Thr Phe Asn Leu Phe Tyr Met
        115                 120                 125

Glu Ser Asp Gln Asp Val Gly Ile Gln Leu Arg Arg Pro Leu Phe Gln
    130                 135                 140

Lys Val Thr Thr Val Ala Ala Asp Gln Ser Phe Thr Ile Arg Asp Leu
145                 150                 155                 160

Ala Ser Gly Ser Val Lys Leu Asn Val Glu Arg Cys Ser Leu Gly His
                165                 170                 175

Leu Thr Arg Arg Gly Leu Tyr Leu Ala Phe His Asn Pro Gly Ser Cys
            180                 185                 190

Val Ala Leu Val Ser Val Arg Val Phe Tyr Gln Arg Cys Ala Glu Thr
        195                 200                 205

Val His Gly Leu Ala His Phe Pro Asp Thr Leu Pro Gly Pro Gly Gly
    210                 215                 220

Leu Val Glu Val Ala Gly Thr Cys Leu Ser His Ala Gln Ile Ser Leu
225                 230                 235                 240

Gly Ser Ser Gly Thr Pro Arg Met His Cys Ser Pro Asp Gly Glu Trp
                245                 250                 255

Leu Val Pro Val Gly Gln Cys Gln Cys Glu Pro Gly Tyr Glu Glu Ser
            260                 265                 270

Ser Gly Asn Val Gly Cys Thr Ala Cys Pro Thr Gly Phe Tyr Arg Val

        275                 280                 285

Asp Met Asn Thr Leu Arg Cys Leu Lys Cys Pro Gln His Ser Ile Ala
    290                 295                 300

Glu Ser Glu Gly Ser Thr Ile Cys Thr Cys Glu Asn Gly His Tyr Arg
305                 310                 315                 320

Ala Pro Gly Glu Gly Pro Gln Val Ala Cys Thr Arg Pro Pro Ser Ala
                325                 330                 335

Pro Gln Asn Leu Ser Phe Ser Thr Ser Gly Thr Gln Leu Ser Leu Arg
            340                 345                 350

Trp Glu Pro Pro Arg Asp Thr Gly Gly Arg His Asp Ile Arg Tyr Ser
        355                 360                 365

Val Glu Cys Leu Gln Cys Arg Gly Ile Ala Gln Asp Gly Gly Pro Cys
    370                 375                 380

Gln Pro Cys Gly Lys Gly Val His Phe Ser Pro Ala Ala Ser Gly Leu
385                 390                 395                 400

Thr Thr Ser Thr Val Gln Val Gln Gly Leu Glu Pro Tyr Ala Asn Tyr
                405                 410                 415

Thr Phe Thr Val Lys Ser Gln Asn Arg Val Ser Gly Leu Asp Ser Ser
            420                 425                 430

Ser Pro Ser Ser Ala Ser Leu Ser Ile Asn Met Gly His Ala Glu Ser
        435                 440                 445

Leu Ser Gly Leu Ser Leu Lys Leu Val Lys Lys Glu Pro Arg Gln Leu
    450                 455                 460

Glu Leu Thr Trp Ala Gly Ser Arg Pro Arg Asn Pro Gly Gly Asn Leu
465                 470                 475                 480

Ser Tyr Glu Leu His Val Leu Asn Gln Asp Glu Glu Trp His Gln Met
                485                 490                 495

Val Leu Glu Pro Arg Val Leu Leu Thr Lys Leu Gln Pro Asp Thr Thr
            500                 505                 510

Tyr Ile Val Arg Val Arg Thr Leu Thr Pro Leu Gly Pro Gly Pro Phe
        515                 520                 525

Ser Pro Asp His Glu Phe Arg Thr Ser Pro Pro Val Ser Arg Ser Leu
    530                 535                 540

Thr Gly Gly Glu Ile Val Ala Val Ile Phe Gly Leu Leu Leu Gly Ile

545                 550                 555                 560

Ala Leu Leu Ile Gly Ile Tyr Val Phe Arg Ser Arg Arg Gly Gln Arg
                565                 570                 575

Gln Arg Gln Gln Arg Gln Arg Glu Arg Thr Thr Asn Val Asp Arg Glu
            580                 585                 590

Asp Lys Leu Trp Leu Lys Pro Tyr Val Asp Leu Gln Ala Tyr Glu Asp
        595                 600                 605

Pro Ala Gln Gly Ala Leu Asp Phe Ala Gln Glu Leu Asp Pro Ala Trp
    610                 615                 620

Leu Ile Val Asp Thr Val Ile Gly Glu Gly Glu Phe Gly Glu Val Tyr
625                 630                 635                 640

Arg Gly Ala Leu Arg Leu Pro Ser Gln Asp Cys Lys Thr Val Ala Ile
                645                 650                 655

Lys Thr Leu Lys Asp Thr Ser Pro Asp Gly Tyr Trp Trp Asn Phe Leu
            660                 665                 670

Arg Glu Ala Thr Ile Met Gly Gln Phe Asn His Pro His Ile Leu Arg
        675                 680                 685

Leu Glu Gly Val Ile Thr Lys Arg Lys Pro Ile Met Ile Ile Thr Glu
    690                 695                 700

Phe Met Glu Asn Gly Ala Leu Asp Ala Phe Leu Lys Glu Arg Glu Asp
705                 710                 715                 720

Gln Leu Ala Pro Gly Gln Leu Val Ala Met Leu Leu Gly Ile Ala Ser
                725                 730                 735

Gly Met Asn Cys Leu Ser Gly His Asn Tyr Val His Arg Asp Leu Ala
            740                 745                 750

Ala Arg Asn Ile Leu Val Asn Gln Asn Leu Cys Cys Lys Val Ser Asp
        755                 760                 765

Phe Gly Leu Thr Arg Leu Leu Asp Asp Phe Asp Gly Thr Tyr Glu Thr
    770                 775                 780

Gln Gly Gly Lys Ile Pro Ile Arg Trp Thr Ala Pro Glu Ala Ile Ala
785                 790                 795                 800

His Arg Ile Phe Thr Thr Ala Ser Asp Val Trp Ser Phe Gly Ile Val
                805                 810                 815

Met Trp Glu Val Leu Ser Phe Gly Asp Lys Pro Tyr Gly Glu Met Ser

            820                 825                 830

Asn Gln Glu Val Met Lys Ser Ile Glu Asp Gly Tyr Arg Leu Pro Pro
        835                 840                 845

Pro Val Asp Cys Pro Ala Pro Leu Tyr Glu Leu Met Lys Asn Cys Trp
    850                 855                 860

Ala Tyr Asp Arg Ala Arg Arg Pro His Phe Leu Gln Leu Gln Ala His
865                 870                 875                 880

Leu Glu Gln Leu Leu Thr Asp Pro His Ser Leu Arg Thr Ile Ala Asn
                885                 890                 895

Phe Asp Pro Arg Val Thr Leu Arg Leu Pro Ser Leu Ser Gly Ser Asp
            900                 905                 910

Gly Ile Pro Tyr Arg Ser Val Ser Glu Trp Leu Glu Ser Ile Arg Met
        915                 920                 925

Lys Arg Tyr Ile Leu His Phe Arg Ser Ala Gly Leu Asp Thr Met Glu
    930                 935                 940

Cys Val Leu Glu Leu Thr Ala Glu Asp Leu Thr Gln Met Gly Ile Thr
945                 950                 955                 960

Leu Pro Gly His Gln Lys Arg Ile Leu Cys Ser Ile Gln Gly Phe Lys
                965                 970                 975

Asp

<210> 3
<211> 977
<212> PRT
<213> rat

<400> 3

Met Glu Arg Arg Trp Pro Leu Gly Leu Ala Leu Leu Leu Leu Leu Leu
1               5                   10                  15

Cys Ala Pro Leu Pro Pro Gly Ala Arg Ala Glu Glu Val Thr Leu Met
            20                  25                  30

Asp Thr Ser Thr Ala Gln Gly Glu Leu Gly Trp Leu Leu Asp Pro Pro
        35                  40                  45

Glu Thr Gly Trp Ser Glu Val Gln Gln Met Leu Asn Gly Thr Pro Leu
    50                  55                  60

Tyr Met Tyr Gln Asp Cys Pro Ile Gln Glu Gly Gly Asp Thr Asp His
65                  70                  75                  80

Trp Leu Arg Ser Asn Trp Ile Tyr Arg Gly Glu Glu Ala Ser Arg Val
                85                  90                  95

His Val Glu Leu Gln Phe Thr Val Arg Asp Cys Lys Ser Phe Pro Gly
            100                 105                 110

Gly Ala Gly Pro Leu Gly Cys Lys Glu Thr Phe Asn Leu Phe Tyr Met
        115                 120                 125

Glu Ser Asp Gln Asp Val Gly Ile Gln Leu Arg Arg Pro Leu Phe Gln
    130                 135                 140

Lys Val Thr Thr Val Ala Ala Asp Gln Ser Phe Thr Ile Arg Asp Leu
145                 150                 155                 160

Ala Ser Gly Ser Val Lys Leu Asn Val Glu His Cys Ser Leu Gly His
                165                 170                 175

Leu Thr Arg Arg Gly Leu Tyr Leu Ala Phe His Asn Pro Gly Ser Cys
            180                 185                 190

Val Ala Leu Val Ser Val Arg Val Phe Tyr Gln Arg Cys Ala Glu Thr
        195                 200                 205

Val His Gly Leu Ala His Phe Pro His Thr Leu Pro Gly Pro Thr Gly
    210                 215                 220

Leu Val Glu Val Ala Gly Thr Cys Leu Ser His Ala Gln Ile Ser Leu
225                 230                 235                 240

Gly Ser Ser Gly Thr Pro Arg Met His Cys Ser Pro Asp Gly Glu Trp
                245                 250                 255

Leu Val Pro Val Gly Gln Cys Gln Cys Glu Pro Gly Tyr Glu Glu Ser
            260                 265                 270

Gly Gly Ser Val Gly Cys Thr Ala Cys Pro Thr Gly Phe Tyr Arg Met
        275                 280                 285

Asp Met Asn Thr Leu His Cys Leu Lys Cys Pro Gln His Ser Val Ala
    290                 295                 300

Glu Ser Glu Gly Ser Thr Ile Cys Thr Cys Glu Asn Gly His Tyr Arg
305                 310                 315                 320

Ala Pro Gly Glu Gly Pro Gln Val Ala Cys Thr Arg Pro Pro Ser Ala
                325                 330                 335

Pro Gln Asn Leu Ser Phe Ser Ala Ser Gly Thr Gln Leu Ser Leu Arg
            340                 345                 350

Trp Glu Pro Pro Arg Asp Thr Gly Gly Arg Gln Asp Ile Arg Tyr Ser
        355                 360                 365

Val Glu Cys Leu Gln Cys Arg Gly Ile Ala Gln Asp Gly Gly Pro Cys
    370                 375                 380

Gln Pro Cys Gly Lys Gly Val His Phe Ser Pro Ala Ala Ser Gly Leu
385                 390                 395                 400

Thr Ala Ser Thr Val Gln Val Glu Gly Leu Glu Pro Tyr Ala Asn Tyr
                405                 410                 415

Thr Phe Thr Ile Lys Ser Gln Asn Arg Val Ser Gly Leu Asp Ser Ser
            420                 425                 430

Ser Pro Ser Ser Ala Ser Leu Ser Ile Asn Met Gly Gln Ala Glu Ser
        435                 440                 445

Leu Ser Gly Leu Ser Leu Lys Leu Val Lys Lys Glu Pro Arg Gln Leu
    450                 455                 460

Glu Leu Thr Trp Ala Gly Ser Arg Leu Gln Asn Pro Gly Gly Asn Leu
465                 470                 475                 480

Ser Tyr Glu Leu His Val Leu Asn Gln Asp Glu Glu Trp His Gln Met
                485                 490                 495

Val Leu Glu Pro Arg Val Leu Leu Thr Lys Leu Gln Pro Asp Thr Thr
            500                 505                 510

Tyr Ile Val Arg Val Arg Thr Leu Thr Pro Leu Gly Pro Gly Pro Phe
        515                 520                 525

Ser Pro Asp His Glu Phe Arg Thr Ser Pro Pro Val Ser Arg Ser Leu
    530                 535                 540

Thr Gly Gly Glu Ile Val Ala Ile Ile Phe Gly Leu Leu Leu Gly Ile
545                 550                 555                 560

Ala Leu Leu Ile Gly Ile Tyr Val Phe Arg Ser Arg Arg Gly Gln Arg
                565                 570                 575

Gln Arg Gln Gln Arg Gln Arg Glu Arg Thr Thr Asn Val Asp Arg Glu
            580                 585                 590

Asp Lys Leu Trp Leu Lys Pro Tyr Val Asp Leu Gln Ala Tyr Glu Asp
        595                 600                 605

Pro Ala Gln Gly Ala Leu Asp Phe Ala Gln Glu Leu Asp Pro Ala Trp
    610                 615                 620

Leu Ile Val Asp Thr Val Ile Gly Glu Gly Glu Phe Gly Glu Val Tyr
625                 630                 635                 640

Arg Gly Ala Leu Arg Ile Pro Ser Gln Asp Cys Lys Thr Val Ala Ile
                645                 650                 655

Lys Thr Leu Lys Asp Thr Ser Pro Asp Gly Tyr Trp Trp Asn Phe Leu
            660                 665                 670

Arg Glu Ala Thr Ile Met Gly Gln Phe Asn His Pro His Ile Leu Arg
        675                 680                 685

Leu Glu Gly Val Ile Thr Lys Arg Lys Pro Ile Met Ile Ile Thr Glu
    690                 695                 700

Phe Met Glu Asn Gly Ala Leu Asp Ala Phe Leu Lys Glu Arg Glu Asp
705                 710                 715                 720

Gln Leu Val Pro Gly Gln Leu Val Ala Met Leu Leu Gly Ile Ala Ser
                725                 730                 735

Gly Met Asn Tyr Leu Ser Gly His Asn Tyr Val His Arg Asp Leu Ala
            740                 745                 750

Ala Arg Asn Ile Leu Val Asn Gln Asn Leu Cys Cys Lys Val Ser Asp
        755                 760                 765

Phe Gly Leu Thr Arg Leu Leu Asp Asp Phe Asp Gly Thr Tyr Glu Thr
    770                 775                 780

Gln Gly Gly Lys Ile Pro Ile Arg Trp Thr Ala Pro Glu Ala Ile Ala
785                 790                 795                 800

His Arg Ile Phe Thr Thr Ala Ser Asp Val Trp Ser Phe Gly Ile Val
                805                 810                 815

Met Trp Glu Val Leu Ser Phe Gly Asp Lys Pro Tyr Gly Glu Met Ser
            820                 825                 830

Asn Gln Glu Val Met Lys Ser Ile Glu Asp Gly Tyr Arg Leu Pro Pro
        835                 840                 845

Pro Val Asp Cys Pro Ala Pro Leu Tyr Glu Leu Met Lys Asn Cys Trp
    850                 855                 860

Ala Tyr Asp Arg Ala Arg Arg Pro His Phe Leu Gln Leu Gln Ala His
865                 870                 875                 880

Leu Glu Gln Leu Leu Thr Asp Pro His Ser Leu Arg Thr Ile Ala Asn
                885                 890                 895

Phe Asp Pro Arg Val Thr Leu Arg Leu Pro Ser Leu Ser Gly Ser Asp
            900                 905                 910

Gly Ile Pro Tyr Arg Ser Val Ser Glu Trp Leu Glu Ser Ile Arg Met
        915                 920                 925

Lys Arg Tyr Ile Leu His Phe Arg Ser Ala Gly Leu Asp Thr Met Glu
    930                 935                 940

Cys Val Leu Glu Leu Thr Ala Glu Asp Leu Thr Gln Met Gly Ile Thr
945                 950                 955                 960

Leu Pro Gly His Gln Lys Arg Ile Leu Cys Ser Ile Gln Gly Phe Lys
                965                 970                 975

Asp

<210>   4
<211>   976
<212>   PRT
<213>   human

<400>   4

<210>  5
<211>  977
<212>  PRT
<213>  mouse

<400>  5

Met Glu Leu Arg Ala Val Gly Phe Cys Leu Ala Leu Leu Trp Gly Cys
1               5                   10                  15

Ala Leu Ala Ala Ala Ala Ala Gln Gly Lys Glu Val Val Leu Leu Asp
            20                  25                  30

Phe Ala Ala Met Lys Gly Glu Leu Gly Trp Leu Thr His Pro Tyr Gly
        35                  40                  45

Lys Gly Trp Asp Leu Met Gln Asn Ile Met Asp Asp Met Pro Ile Tyr
    50                  55                  60

Met Tyr Ser Val Cys Asn Val Val Ser Gly Asp Gln Asp Asn Trp Leu
65                  70                  75                  80

Arg Thr Asn Trp Val Tyr Arg Glu Glu Ala Glu Arg Ile Phe Ile Glu
                85                  90                  95

Leu Lys Phe Thr Val Arg Asp Cys Asn Ser Phe Pro Gly Gly Ala Ser
            100                 105                 110

Ser Cys Lys Glu Thr Phe Asn Leu Tyr Tyr Ala Glu Ser Asp Val Asp
        115                 120                 125

Tyr Gly Thr Asn Phe Gln Lys Arg Gln Phe Thr Lys Ile Asp Thr Ile
    130                 135                 140

Ala Pro Asp Glu Ile Thr Val Ser Ser Asp Phe Glu Ala Arg Asn Val
145                 150                 155                 160

Lys Leu Asn Val Glu Glu Arg Met Val Gly Pro Leu Thr Arg Lys Gly
                165                 170                 175

Phe Tyr Leu Ala Phe Gln Asp Ile Gly Ala Cys Val Ala Leu Leu Ser
            180                 185                 190

Val Arg Val Tyr Tyr Lys Lys Cys Pro Glu Met Leu Gln Ser Leu Ala
        195                 200                 205

Arg Phe Pro Glu Thr Ile Ala Val Ala Val Ser Asp Thr Gln Pro Leu
    210                 215                 220

Ala Thr Val Ala Gly Thr Cys Val Asp His Ala Val Val Pro Tyr Gly
225                 230                 235                 240

Gly Glu Gly Pro Leu Met His Cys Thr Val Asp Gly Glu Trp Leu Val
                245                 250                 255

Pro Ile Gly Gln Cys Leu Cys Gln Glu Gly Tyr Glu Lys Val Glu Asp
            260                 265                 270

Ala Cys Arg Ala Cys Ser Pro Gly Phe Phe Lys Ser Glu Ala Ser Glu
        275                 280                 285

Ser Pro Cys Leu Glu Cys Pro Glu His Thr Leu Pro Ser Thr Glu Gly
    290                 295                 300

Ala Thr Ser Cys Gln Cys Glu Glu Gly Tyr Phe Arg Ala Pro Glu Asp
305                 310                 315                 320

Pro Leu Ser Met Ser Cys Thr Arg Pro Pro Ser Ala Pro Asn Tyr Leu
                325                 330                 335

Thr Ala Ile Gly Met Gly Ala Lys Val Glu Leu Arg Trp Thr Ala Pro
            340                 345                 350

Lys Asp Thr Gly Gly Arg Gln Asp Ile Val Tyr Ser Val Thr Cys Glu
        355                 360                 365

Gln Cys Trp Pro Glu Ser Gly Glu Cys Gly Pro Cys Glu Ala Ser Val
    370                 375                 380

Arg Tyr Ser Glu Pro Pro His Ala Leu Thr Arg Thr Ser Val Thr Val
385                 390                 395                 400

Ser Asp Leu Glu Pro His Met Asn Tyr Thr Phe Ala Val Glu Ala Arg
                405                 410                 415

Asn Gly Val Ser Gly Leu Val Thr Ser Arg Ser Phe Arg Thr Ala Ser
            420                 425                 430

Val Ser Ile Asn Gln Thr Glu Pro Pro Lys Val Arg Leu Glu Asp Arg
        435                 440                 445

Ser Thr Thr Ser Leu Ser Val Thr Trp Ser Ile Pro Val Ser Gln Gln
    450                 455                 460

Ser Arg Val Trp Lys Tyr Glu Val Thr Tyr Arg Lys Lys Gly Asp Ala
465                 470                 475                 480

Asn Ser Tyr Asn Val Arg Arg Thr Glu Gly Phe Ser Val Thr Leu Asp
                485                 490                 495

Asp Leu Ala Pro Asp Thr Thr Tyr Leu Val Gln Val Gln Ala Leu Thr
            500                 505                 510

Gln Glu Gly Gln Gly Ala Gly Ser Lys Val His Glu Phe Gln Thr Leu
        515                 520                 525

Ser Thr Glu Gly Ser Ala Asn Met Ala Val Ile Gly Gly Val Ala Val
    530                 535                 540

Gly Val Val Leu Leu Leu Val Leu Ala Gly Val Gly Leu Phe Ile His
545                 550                 555                 560

Arg Arg Arg Arg Asn Leu Arg Ala Arg Gln Ser Ser Glu Asp Val Arg
                565                 570                 575

Phe Ser Lys Ser Glu Gln Leu Lys Pro Leu Lys Thr Tyr Val Asp Pro
            580                 585                 590

His Thr Tyr Glu Asp Pro Asn Gln Ala Val Leu Lys Phe Thr Thr Glu
        595                 600                 605

Ile His Pro Ser Cys Val Ala Arg Gln Lys Val Ile Gly Ala Gly Glu
    610                 615                 620

Phe Gly Glu Val Tyr Lys Gly Thr Leu Lys Ala Ser Ser Gly Lys Lys
625                 630                 635                 640

Glu Ile Pro Val Ala Ile Lys Thr Leu Lys Ala Gly Tyr Thr Glu Lys
                645                 650                 655

Gln Arg Val Asp Phe Leu Ser Glu Ala Ser Ile Met Gly Gln Phe Ser
            660                 665                 670

His His Asn Ile Ile Arg Leu Glu Gly Val Val Ser Lys Tyr Lys Pro
        675                 680                 685

Met Met Ile Ile Thr Glu Tyr Met Glu Asn Gly Ala Leu Asp Lys Phe
    690                 695                 700

Leu Arg Glu Lys Asp Gly Glu Phe Ser Val Leu Gln Leu Val Gly Met
705                 710                 715                 720

Leu Arg Gly Ile Ala Ser Gly Met Lys Tyr Leu Ala Asn Met Asn Tyr
                725                 730                 735

Val His Arg Asp Leu Ala Ala Arg Asn Ile Leu Val Asn Ser Asn Leu
            740                 745                 750

Val Cys Lys Val Ser Asp Phe Gly Leu Ser Arg Val Leu Glu Asp Asp
        755                 760                 765

Pro Glu Ala Thr Tyr Thr Thr Ser Gly Gly Lys Ile Pro Ile Arg Trp
    770                 775                 780

Thr Ala Pro Glu Ala Ile Ser Tyr Arg Lys Phe Thr Ser Ala Ser Asp
785                 790                 795                 800

Val Trp Ser Tyr Gly Ile Val Met Trp Glu Val Met Thr Tyr Gly Glu
                805                 810                 815

Arg Pro Tyr Trp Glu Leu Ser Asn His Glu Val Met Lys Ala Ile Asn
            820                 825                 830

Asp Gly Phe Arg Leu Pro Thr Pro Met Asp Cys Pro Ser Ala Ile Tyr
        835                 840                 845

Gln Leu Met Met Gln Cys Trp Gln Gln Glu Arg Ser Arg Arg Pro Lys
    850                 855                 860

Phe Ala Asp Ile Val Ser Ile Leu Asp Lys Leu Ile Arg Ala Pro Asp
865                 870                 875                 880

Ser Leu Lys Thr Leu Ala Asp Phe Asp Pro Arg Val Ser Ile Arg Leu
                885                 890                 895

Pro Ser Thr Ser Gly Ser Glu Gly Val Pro Phe Arg Thr Val Ser Glu
            900                 905                 910

Trp Leu Glu Ser Ile Lys Met Gln Gln Tyr Thr Glu His Phe Met Val
        915                 920                 925

Ala Gly Tyr Thr Ala Ile Glu Lys Val Val Gln Met Ser Asn Glu Asp
    930                 935                 940

Ile Lys Arg Ile Gly Val Arg Leu Pro Gly His Gln Lys Arg Ile Ala
945                 950                 955                 960

Tyr Ser Leu Leu Gly Leu Lys Asp Gln Val Asn Thr Val Gly Ile Pro
                965                 970                 975

Ile

<210>  6
<211>  977
<212>  PRT
<213>  rat

<400>  6

Met Lys Leu Arg Ala Ala Gly Phe Cys Leu Ala Leu Leu Trp Gly Cys
1               5                   10                  15

Ala Leu Ala Ala Ala Ala Ala Gln Gly Lys Glu Val Val Leu Leu Asp

20                  25                  30

Phe Ala Ala Met Lys Gly Glu Leu Gly Trp Leu Thr His Pro Tyr Gly
        35                  40                  45

Lys Gly Trp Asp Leu Met Gln Asn Ile Met Asn Asp Met Pro Ile Tyr
    50                  55                  60

Met Tyr Ser Val Cys Asn Val Val Ser Gly Asp Gln Asp Asn Trp Leu
65                  70                  75                  80

Arg Thr Asn Trp Val Tyr Arg Glu Glu Ala Glu Arg Ile Phe Ile Glu
                85                  90                  95

Leu Lys Phe Thr Val Arg Asp Cys Asn Ser Phe Pro Gly Gly Ala Ser
            100                 105                 110

Ser Cys Lys Glu Thr Phe Asn Leu Tyr Tyr Ala Glu Ser Asp Val Asp
        115                 120                 125

Tyr Gly Thr Asn Phe Gln Lys Arg Gln Phe Thr Lys Ile Asp Thr Ile
    130                 135                 140

Ala Pro Asp Glu Ile Thr Val Ser Ser Asp Phe Glu Ala Arg Asn Val
145                 150                 155                 160

Lys Leu Asn Val Glu Glu Arg Met Val Gly Pro Leu Thr Arg Lys Gly
                165                 170                 175

Phe Tyr Leu Ala Phe Gln Asp Ile Gly Ala Cys Val Ala Leu Leu Ser
            180                 185                 190

Val Arg Val Tyr Tyr Lys Lys Cys Pro Glu Met Leu Gln Ser Leu Ala
        195                 200                 205

Arg Phe Pro Glu Thr Ile Ala Val Ala Val Ser Asp Ala Gln Pro Leu
    210                 215                 220

Ala Thr Val Ala Gly Thr Cys Val Asp His Ala Val Val Pro Tyr Gly
225                 230                 235                 240

Gly Glu Gly Pro Leu Met His Cys Thr Val Asp Gly Glu Trp Leu Val
                245                 250                 255

Pro Ile Gly Gln Cys Leu Cys Gln Glu Gly Tyr Glu Lys Val Glu Asp
            260                 265                 270

Ala Cys Gln Ala Cys Ser Pro Gly Phe Phe Lys Ser Glu Ala Ser Glu
        275                 280                 285

Ser Pro Cys Leu Gln Cys Pro Glu His Thr Leu Pro Ser Thr Glu Gly

290                 295                 300

Ala Thr Ser Cys Gln Cys Glu Glu Gly Tyr Phe Arg Ala Pro Glu Asp
305                 310                 315                 320

Pro Leu Ser Met Pro Cys Thr Arg Pro Pro Ser Ala Pro His Tyr Leu
                325                 330                 335

Thr Ala Ile Gly Met Gly Ala Lys Val Glu Leu Arg Trp Thr Ala Pro
            340                 345                 350

Gln Asp Thr Gly Gly Arg Lys Asp Ile Val Tyr Ser Val Thr Cys Glu
        355                 360                 365

Gln Cys Trp Pro Glu Ser Gly Glu Cys Gly Ser Cys Glu Ala Ser Val
    370                 375                 380

Gln Tyr Ser Glu Pro Pro Gln Ala Leu Thr Arg Thr Ser Val Thr Val
385                 390                 395                 400

Ser Asp Leu Glu Pro His Met Asn Tyr Thr Phe Thr Val Glu Ala Arg
                405                 410                 415

Asn Gly Val Ser Asp Leu Val Asn Ser Arg Ser Phe Arg Thr Ala Ser
            420                 425                 430

Val Ser Ile Asn Gln Thr Glu Pro Pro Lys Val Arg Leu Glu Asp Arg
        435                 440                 445

Ser Thr Thr Ser Leu Ser Val Ala Trp Ser Ile Pro Val Pro Gln Gln
    450                 455                 460

Ser Arg Val Trp Lys Tyr Glu Val Thr Tyr Arg Lys Lys Gly Asp Ala
465                 470                 475                 480

Asn Ser Tyr Asn Val His Arg Thr Asp Gly Phe Ser Val Thr Leu Asp
                485                 490                 495

Gly Leu Ala Pro Gly Thr Thr Tyr Leu Val Gln Val Gln Ala Leu Thr
            500                 505                 510

Gln Glu Gly Gln Gly Ala Gly Ser Lys Val His Glu Phe Gln Thr Leu
        515                 520                 525

Ser Thr Glu Gly Ser Ala Thr Met Ala Val Ile Gly Gly Val Ala Val
    530                 535                 540

Gly Val Val Leu Leu Leu Val Leu Ala Gly Ile Gly Phe Phe Ile His
545                 550                 555                 560

Arg Arg Arg Arg Asn Leu Arg Ala Arg Gln Ser Ser Glu Asp Val Tyr

                565                 570                 575

Phe Ser Lys Ser Glu Gln Leu Lys Pro Leu Lys Thr Tyr Val Asp Pro
            580                 585                 590

His Thr Tyr Glu Asp Pro Asn Gln Ala Val Leu Lys Phe Thr Thr Glu
        595                 600                 605

Ile His Pro Ser Cys Val Ser Arg Gln Lys Val Ile Gly Ala Gly Glu
    610                 615                 620

Phe Gly Glu Val Tyr Lys Gly Thr Leu Lys Ser Ser Ser Gly Lys Lys
625                 630                 635                 640

Glu Ile Pro Val Ala Ile Lys Thr Leu Lys Ala Gly Tyr Thr Glu Lys
                645                 650                 655

Gln Arg Val Asp Phe Leu Ser Glu Ala Ser Ile Met Gly Gln Phe Ser
            660                 665                 670

His His Asn Ile Ile Arg Leu Glu Gly Val Val Ser Lys Tyr Lys Pro
        675                 680                 685

Met Met Ile Ile Thr Glu Tyr Met Glu Asn Gly Ala Leu Asp Lys Phe
    690                 695                 700

Leu Arg Glu Lys Asp Gly Glu Phe Ser Val Leu Gln Leu Val Gly Met
705                 710                 715                 720

Leu Arg Gly Ile Ala Ser Gly Met Lys Tyr Leu Ala Asn Met Asn Tyr
                725                 730                 735

Val His Arg Asp Leu Ala Ala Arg Asn Ile Leu Val Asn Ser Asn Leu
            740                 745                 750

Val Cys Lys Val Ser Asp Phe Gly Leu Ser Arg Val Leu Glu Asp Asp
        755                 760                 765

Pro Glu Ala Thr Tyr Thr Thr Ser Gly Gly Lys Ile Pro Ile Arg Trp
    770                 775                 780

Thr Ala Pro Glu Ala Ile Ser Tyr Arg Lys Phe Thr Ser Ala Ser Asp
785                 790                 795                 800

Val Trp Ser Tyr Gly Ile Val Met Trp Glu Val Met Thr Tyr Gly Glu
                805                 810                 815

Arg Pro Tyr Trp Glu Leu Ser Asn His Glu Val Met Lys Ala Ile Asn
            820                 825                 830

Asp Gly Phe Arg Leu Pro Thr Pro Met Asp Cys Pro Ser Ala Ile Tyr

835                    840                    845

Gln Leu Met Met Gln Cys Trp Gln Gln Glu Arg Ser Arg Arg Pro Lys
    850                 855                 860

Phe Ala Asp Ile Val Ser Ile Leu Asp Lys Leu Ile Arg Ala Pro Asp
865                 870                 875                 880

Ser Leu Lys Thr Leu Ala Asp Phe Asp Pro Arg Val Ser Ile Arg Leu
                885                 890                 895

Pro Ser Thr Ser Gly Ser Glu Gly Val Pro Phe Arg Thr Val Ser Glu
            900                 905                 910

Trp Leu Glu Ser Ile Lys Met Gln Gln Tyr Thr Glu His Phe Met Val
        915                 920                 925

Ala Gly Tyr Thr Ala Ile Glu Lys Val Val Gln Met Ser Asn Asp Asp
    930                 935                 940

Ile Lys Arg Ile Gly Val Arg Leu Pro Gly His Gln Lys Arg Ile Ala
945                 950                 955                 960

Tyr Ser Leu Leu Gly Leu Lys Asp Gln Val Asn Thr Val Gly Ile Pro
                965                 970                 975

Ile

<210>  7
<211>  986
<212>  PRT
<213>  human

<400>  7

<210>  8
<211>  986
<212>  PRT
<213>  mouse

<400>  8

Met Ala Gly Ile Phe Tyr Phe Ile Leu Phe Ser Phe Leu Phe Gly Ile
1               5                   10                  15

Cys Asp Ala Val Thr Gly Ser Arg Val Tyr Pro Ala Asn Glu Val Thr
            20                  25                  30

Leu Leu Asp Ser Arg Ser Val Gln Gly Glu Leu Gly Trp Ile Ala Ser
        35                  40                  45

Pro Leu Glu Gly Gly Trp Glu Glu Val Ser Ile Met Asp Glu Lys Asn
    50                  55                  60

Thr Pro Ile Arg Thr Tyr Gln Val Cys Asn Val Met Glu Ala Ser Gln
65                  70                  75                  80

Asn Asn Trp Leu Arg Thr Asp Trp Ile Thr Arg Glu Gly Ala Gln Arg
                85                  90                  95

Val Tyr Ile Glu Ile Lys Phe Thr Leu Arg Asp Cys Asn Ser Leu Pro
            100                 105                 110

Gly Val Met Gly Thr Cys Lys Glu Thr Phe Asn Leu Tyr Tyr Tyr Glu
        115                 120                 125

Ser Asp Asn Asp Lys Glu Arg Phe Ile Arg Glu Ser Gln Phe Gly Lys
    130                 135                 140

Ile Asp Thr Ile Ala Ala Asp Glu Ser Phe Thr Gln Val Asp Ile Gly
145                 150                 155                 160

Asp Arg Ile Met Lys Leu Asn Thr Glu Ile Arg Asp Val Gly Pro Leu
                165                 170                 175

Ser Lys Lys Gly Phe Tyr Leu Ala Phe Gln Asp Val Gly Ala Cys Ile
            180                 185                 190

Ala Leu Val Ser Val Arg Val Phe Tyr Lys Lys Cys Pro Leu Thr Val
        195                 200                 205

Arg Asn Leu Ala Gln Phe Pro Asp Thr Ile Thr Gly Ala Asp Thr Ser
    210                 215                 220

Ser Leu Val Glu Val Arg Gly Ser Cys Val Asn Asn Ser Glu Glu Lys
225                 230                 235                 240

Asp Val Pro Lys Met Tyr Cys Gly Ala Asp Gly Glu Trp Leu Val Pro
                245                 250                 255

Ile Gly Asn Cys Leu Cys Asn Ala Gly His Glu Glu Gln Asn Gly Glu
            260                 265                 270

Cys Gln Ala Cys Lys Ile Gly Tyr Tyr Lys Ala Leu Ser Thr Asp Ala
        275                 280                 285

Ser Cys Ala Lys Cys Pro Pro His Ser Tyr Ser Val Trp Glu Gly Ala
    290                 295                 300

Thr Ser Cys Thr Cys Asp Arg Gly Phe Phe Arg Ala Asp Asn Asp Ala
305                 310                 315                 320

Ala Ser Met Pro Cys Thr Arg Pro Pro Ser Ala Pro Leu Asn Leu Ile
                325                 330                 335

Ser Asn Val Asn Glu Thr Ser Val Asn Leu Glu Trp Ser Ser Pro Gln
            340                 345                 350

Asn Thr Gly Gly Arg Gln Asp Ile Ser Tyr Asn Val Val Cys Lys Lys
        355                 360                 365

Cys Gly Ala Gly Asp Pro Ser Lys Cys Arg Pro Cys Gly Ser Gly Val
    370                 375                 380

His Tyr Thr Pro Gln Gln Asn Gly Leu Lys Thr Thr Arg Val Ser Ile
385                 390                 395                 400

Thr Asp Leu Leu Ala His Thr Asn Tyr Thr Phe Glu Ile Trp Ala Val
                405                 410                 415

Asn Gly Val Ser Lys Tyr Asn Pro Ser Pro Asp Gln Ser Val Ser Val
            420                 425                 430

Thr Val Thr Thr Asn Gln Ala Ala Pro Ser Ser Ile Ala Leu Val Gln
        435                 440                 445

Ala Lys Glu Val Thr Arg Tyr Ser Val Ala Leu Ala Trp Leu Glu Pro
    450                 455                 460

Asp Arg Pro Asn Gly Val Ile Leu Glu Tyr Glu Val Lys Tyr Tyr Glu
465                 470                 475                 480

Lys Asp Gln Asn Glu Arg Ser Tyr Arg Ile Val Arg Thr Ala Ala Arg
                485                 490                 495

Asn Thr Asp Ile Lys Gly Leu Asn Pro Leu Thr Ser Tyr Val Phe His
            500                 505                 510

Val Arg Ala Arg Thr Ala Ala Gly Tyr Gly Asp Phe Ser Glu Pro Leu
        515                 520                 525

Glu Val Thr Thr Asn Thr Val Pro Ser Arg Ile Ile Gly Asp Gly Ala
    530                 535                 540

Asn Ser Thr Val Leu Leu Val Ser Val Ser Gly Ser Val Val Leu Val
545                 550                 555                 560

Val Ile Leu Ile Ala Ala Phe Val Ile Ser Arg Arg Arg Ser Lys Tyr
                565                 570                 575

Ser Lys Ala Lys Gln Glu Ala Asp Glu Glu Lys His Leu Asn Gln Gly
            580                 585                 590

Val Arg Thr Tyr Val Asp Pro Phe Thr Tyr Glu Asp Pro Asn Gln Ala
        595                 600                 605

Val Arg Glu Phe Ala Lys Glu Ile Asp Ala Ser Cys Ile Lys Ile Glu
    610                 615                 620

Lys Val Ile Gly Val Gly Glu Phe Gly Glu Val Cys Ser Gly Arg Leu
625                 630                 635                 640

Lys Val Pro Gly Lys Arg Glu Ile Cys Val Ala Ile Lys Thr Leu Lys
                645                 650                 655

Ala Gly Tyr Thr Asp Lys Gln Arg Arg Asp Phe Leu Ser Glu Ala Ser
            660                 665                 670

Ile Met Gly Gln Phe Asp His Pro Asn Ile Ile His Leu Glu Gly Val
        675                 680                 685

Val Thr Lys Cys Lys Pro Val Met Ile Ile Thr Glu Tyr Met Glu Asn
    690                 695                 700

Gly Ser Leu Asp Ala Phe Leu Arg Lys Asn Asp Gly Arg Phe Thr Val
705                 710                 715                 720

Ile Gln Leu Val Gly Met Leu Arg Gly Ile Gly Ser Gly Met Lys Tyr
                725                 730                 735

Leu Ser Asp Met Ser Tyr Val His Arg Asp Leu Ala Ala Arg Asn Ile
            740                 745                 750

Leu Val Asn Ser Asn Leu Val Cys Lys Val Ser Asp Phe Gly Met Ser
        755                 760                 765

Arg Val Leu Glu Asp Asp Pro Glu Ala Ala Tyr Thr Thr Arg Gly Gly
    770                 775                 780

Lys Ile Pro Ile Arg Trp Thr Ala Pro Glu Ala Ile Ala Tyr Arg Lys
785                 790                 795                 800

Phe Thr Ser Ala Ser Asp Val Trp Ser Tyr Gly Ile Val Met Trp Glu
                805                 810                 815

Val Met Ser Tyr Gly Glu Arg Pro Tyr Trp Asp Met Ser Asn Gln Asp
            820                 825                 830

Val Ile Lys Ala Ile Glu Glu Gly Tyr Arg Leu Pro Pro Pro Met Asp
        835                 840                 845

Cys Pro Ile Ala Leu His Gln Leu Met Leu Asp Cys Trp Gln Lys Glu
    850                 855                 860

Arg Ser Asp Arg Pro Lys Phe Gly Gln Ile Val Asn Met Leu Asp Lys
865                 870                 875                 880

Leu Ile Arg Asn Pro Asn Ser Leu Lys Arg Thr Gly Ser Glu Ser Ser
                885                 890                 895

Arg Pro Asn Thr Ala Leu Leu Asp Pro Ser Ser Pro Glu Phe Ser Ala
            900                 905                 910

Val Val Ser Val Gly Asp Trp Leu Gln Ala Ile Lys Met Asp Arg Tyr
        915                 920                 925

Lys Asp Asn Phe Thr Ala Ala Gly Tyr Thr Thr Leu Glu Ala Val Val
    930                 935                 940

His Met Ser Gln Asp Asp Leu Ala Arg Ile Gly Ile Thr Ala Ile Thr
945                 950                 955                 960

His Gln Asn Lys Ile Leu Ser Ser Val Gln Ala Met Arg Thr Gln Met
                965                 970                 975

Gln Gln Met His Gly Arg Met Val Pro Val
            980                 985

<210>  9
<211>  986
<212>  PRT
<213>  rat

<400>  9

Met Ala Gly Ile Phe Tyr Phe Ile Leu Phe Ser Phe Leu Phe Gly Ile
1               5                   10                  15

Cys Asp Ala Val Thr Gly Ser Arg Val Tyr Pro Ala Asn Glu Val Thr
            20                  25                  30

Leu Leu Asp Ser Arg Ser Val Gln Gly Glu Leu Gly Trp Ile Ala Ser
        35                  40                  45

Pro Leu Glu Gly Gly Trp Glu Glu Val Ser Ile Met Asp Glu Lys Asn
    50                  55                  60

Thr Pro Ile Arg Thr Tyr Gln Val Cys Asn Val Met Glu Ala Ser Gln
65                  70                  75                  80

Asn Asn Trp Leu Arg Thr Asp Trp Ile Thr Arg Glu Gly Ala Gln Arg
                85                  90                  95

Val Tyr Ile Glu Ile Lys Phe Thr Leu Arg Asp Cys Asn Ser Leu Pro
            100                 105                 110

Gly Val Met Gly Thr Cys Lys Glu Thr Phe Asn Leu Tyr Tyr Tyr Glu
        115                 120                 125

Ser Asp Asn Asp Lys Glu Arg Phe Ile Arg Glu Ser Gln Phe Gly Lys
    130                 135                 140

Ile Asp Thr Ile Ala Ala Asp Glu Ser Phe Thr Gln Val Asp Ile Gly
145                 150                 155                 160

Asp Arg Ile Met Lys Leu Asn Thr Glu Ile Arg Asp Val Gly Pro Leu
                165                 170                 175

Ser Lys Lys Gly Phe Tyr Leu Ala Phe Gln Asp Val Gly Ala Cys Ile
            180                 185                 190

Ala Leu Val Ser Val Arg Val Phe Tyr Lys Lys Cys Pro Leu Thr Val
        195                 200                 205

Arg Asn Leu Ala Gln Phe Pro Asp Thr Ile Thr Gly Ala Asp Thr Ser
    210                 215                 220

Ser Leu Val Glu Val Arg Gly Ser Cys Val Asn Asn Ser Glu Glu Lys
225                 230                 235                 240

Asp Val Pro Lys Met Tyr Cys Gly Ala Asp Gly Glu Trp Leu Val Pro
                245                 250                 255

Ile Gly Asn Cys Leu Cys Asn Ala Gly His Glu Glu Gln Asn Gly Glu
            260                 265                 270

Cys Gln Ala Cys Lys Ile Gly Tyr Tyr Lys Ala Leu Ser Thr Asp Ala
        275                 280                 285

Thr Cys Ala Lys Cys Pro Pro His Ser Tyr Ser Val Trp Glu Gly Ala
    290                 295                 300

Thr Ser Cys Thr Cys Asp Arg Gly Phe Phe Arg Ala Asp Asn Asp Ala
305                 310                 315                 320

Ala Ser Met Pro Cys Thr Arg Pro Pro Ser Ala Pro Leu Asn Leu Ile
                325                 330                 335

Ser Asn Val Asn Glu Thr Ser Val Asn Leu Glu Trp Ser Ser Pro Gln
            340                 345                 350

Asn Thr Gly Gly Arg Gln Asp Ile Ser Tyr Asn Val Val Cys Lys Lys
        355                 360                 365

Cys Gly Ala Gly Asp Pro Ser Lys Cys Arg Pro Cys Gly Ser Gly Val
    370                 375                 380

His Tyr Thr Pro Gln Gln Asn Gly Leu Lys Thr Thr Arg Val Ser Ile
385                 390                 395                 400

Thr Asp Leu Leu Ala His Thr Asn Tyr Thr Phe Glu Ile Trp Ala Val
                405                 410                 415

Asn Gly Val Ser Lys Tyr Asn Pro Ser Pro Asp Gln Ser Val Ser Val
            420                 425                 430

Thr Val Thr Thr Asn Gln Ala Ala Pro Ser Ser Ile Ala Leu Val Gln
        435                 440                 445

Ala Lys Glu Val Thr Arg Tyr Ser Val Ala Leu Ala Trp Leu Glu Pro
    450                 455                 460

Asp Arg Pro Asn Gly Val Ile Leu Glu Tyr Glu Val Lys Tyr Tyr Glu
465                 470                 475                 480

Lys Asp Gln Asn Glu Arg Ser Tyr Arg Ile Val Arg Thr Ala Ala Arg
                485                 490                 495

Asn Thr Asp Ile Lys Gly Leu Asn Pro Leu Thr Ser Tyr Val Phe His
            500                 505                 510

Val Arg Ala Arg Thr Ala Ala Gly Tyr Gly Asp Phe Ser Glu Pro Leu
        515                 520                 525

Glu Val Thr Thr Asn Thr Val Pro Ser Arg Ile Ile Gly Asp Gly Ala
    530                 535                 540

Asn Ser Thr Val Leu Leu Val Ser Val Ser Gly Ser Val Val Leu Val
545                 550                 555                 560

Val Ile Leu Ile Ala Ala Phe Val Ile Ser Arg Arg Arg Ser Lys Tyr
                565                 570                 575

Ser Gln Ala Lys Gln Glu Ala Asp Glu Glu Lys His Leu Asn Gln Gly
            580                 585                 590

Val Arg Thr Tyr Val Asp Pro Phe Thr Tyr Glu Asp Pro Asn Gln Ala
        595                 600                 605

Val Arg Glu Phe Ala Lys Glu Ile Asp Ala Ser Cys Ile Lys Ile Glu
    610                 615                 620

Lys Val Ile Gly Val Gly Glu Phe Gly Glu Val Cys Ser Gly Arg Leu
625                 630                 635                 640

Lys Val Pro Gly Lys Arg Glu Ile Cys Val Ala Ile Lys Thr Leu Lys
                645                 650                 655

Ala Gly Tyr Thr Asp Lys Gln Arg Arg Asp Phe Leu Ser Glu Ala Ser
            660                 665                 670

Ile Met Gly Gln Phe Asp His Pro Asn Ile Ile His Leu Glu Gly Val
        675                 680                 685

Val Thr Lys Cys Lys Pro Val Met Ile Ile Thr Glu Tyr Met Glu Asn
    690                 695                 700

Gly Ser Leu Asp Ala Phe Leu Arg Lys Asn Asp Gly Arg Phe Thr Val
705                 710                 715                 720

Ile Gln Leu Val Gly Met Leu Arg Gly Ile Gly Ser Gly Met Lys Tyr
                725                 730                 735

Leu Ser Asp Met Ser Tyr Val His Arg Asp Leu Ala Ala Arg Asn Ile
            740                 745                 750

Leu Val Asn Ser Asn Leu Val Cys Lys Val Ser Asp Phe Gly Met Ser
        755                 760                 765

Arg Val Leu Glu Asp Asp Pro Glu Ala Ala Tyr Thr Thr Arg Gly Gly
    770                 775                 780

Lys Ile Pro Ile Arg Trp Thr Ala Pro Glu Ala Ile Ala Tyr Arg Lys
785                 790                 795                 800

Phe Thr Ser Ala Ser Asp Val Trp Ser Tyr Gly Ile Val Met Trp Glu
                805                 810                 815

Val Met Ser Tyr Gly Glu Arg Pro Tyr Trp Asp Met Ser Asn Gln Asp
            820                 825                 830

Val Ile Lys Ala Ile Glu Glu Gly Tyr Arg Leu Pro Pro Pro Met Asp
        835                 840                 845

Cys Pro Ile Ala Leu His Gln Leu Met Leu Asp Cys Trp Gln Lys Glu
    850                 855                 860

Arg Ser Asp Arg Pro Lys Phe Gly Gln Ile Val Asn Met Leu Asp Lys
865                 870                 875                 880

Leu Ile Arg Asn Pro Asn Ser Leu Lys Arg Thr Gly Pro Glu Ser Ser
                885                 890                 895

Arg Pro Asn Thr Ala Leu Leu Asp Pro Ser Ser Pro Glu Phe Ser Ala
            900                 905                 910

Val Val Ser Val Gly Asp Trp Leu Gln Ala Ile Lys Met Asp Arg Tyr
        915                 920                 925

Lys Asp Asn Phe Thr Ala Ala Gly Tyr Thr Thr Leu Glu Ala Val Val
    930                 935                 940

His Met Ser Gln Asp Asp Leu Ala Arg Ile Gly Ile Thr Ala Ile Thr
945                 950                 955                 960

His Gln Asn Lys Ile Leu Ser Ser Val Gln Ala Met Arg Thr Gln Met
                965                 970                 975

Gln Gln Met His Gly Arg Met Val Pro Val
            980                 985

<210>  10
<211>  1037
<212>  PRT
<213>  human

<400>  10

Met Arg Gly Ser Gly Pro Arg Gly Ala Gly His Arg Arg Pro Pro Ser
1               5                   10                  15

Gly Gly Gly Asp Thr Pro Ile Thr Pro Ala Ser Leu Ala Gly Cys Tyr

            20                  25                  30

Ser Ala Pro Arg Arg Ala Pro Leu Trp Thr Cys Leu Leu Leu Cys Ala
        35                  40                  45

Ala Leu Arg Thr Leu Leu Ala Ser Pro Ser Asn Glu Val Asn Leu Leu
    50                  55                  60

Asp Ser Arg Thr Val Met Gly Asp Leu Gly Trp Ile Ala Phe Pro Lys
65                  70                  75                  80

Asn Gly Trp Glu Glu Ile Gly Glu Val Asp Glu Asn Tyr Ala Pro Ile
                85                  90                  95

His Thr Tyr Gln Val Cys Lys Val Met Glu Gln Asn Gln Asn Asn Trp
            100                 105                 110

Leu Leu Thr Ser Trp Ile Ser Asn Glu Gly Ala Ser Arg Ile Phe Ile
        115                 120                 125

Glu Leu Lys Phe Thr Leu Arg Asp Cys Asn Ser Leu Pro Gly Gly Leu
    130                 135                 140

Gly Thr Cys Lys Glu Thr Phe Asn Met Tyr Tyr Phe Glu Ser Asp Asp
145                 150                 155                 160

Gln Asn Gly Arg Asn Ile Lys Glu Asn Gln Tyr Ile Lys Ile Asp Thr
                165                 170                 175

Ile Ala Ala Asp Glu Ser Phe Thr Glu Leu Asp Leu Gly Asp Arg Val
            180                 185                 190

Met Lys Leu Asn Thr Glu Val Arg Asp Val Gly Pro Leu Ser Lys Lys
        195                 200                 205

Gly Phe Tyr Leu Ala Phe Gln Asp Val Gly Ala Cys Ile Ala Leu Val
    210                 215                 220

Ser Val Arg Val Tyr Tyr Lys Lys Cys Pro Ser Val Val Arg His Leu
225                 230                 235                 240

Ala Val Phe Pro Asp Thr Ile Thr Gly Ala Asp Ser Ser Gln Leu Leu
                245                 250                 255

Glu Val Ser Gly Ser Cys Val Asn His Ser Val Thr Asp Glu Pro Pro
            260                 265                 270

Lys Met His Cys Ser Ala Glu Gly Glu Trp Leu Val Pro Ile Gly Lys
        275                 280                 285

Cys Met Cys Lys Ala Gly Tyr Glu Glu Lys Asn Gly Thr Cys Gln Val

290                 295                 300

Cys Arg Pro Gly Phe Phe Lys Ala Ser Pro His Ile Gln Ser Cys Gly
305                 310                 315                 320

Lys Cys Pro Pro His Ser Tyr Thr His Glu Glu Ala Ser Thr Ser Cys
                325                 330                 335

Val Cys Glu Lys Asp Tyr Phe Arg Arg Glu Ser Asp Pro Pro Thr Met
            340                 345                 350

Ala Cys Thr Arg Pro Pro Ser Ala Pro Arg Asn Ala Ile Ser Asn Val
        355                 360                 365

Asn Glu Thr Ser Val Phe Leu Glu Trp Ile Pro Pro Ala Asp Thr Gly
    370                 375                 380

Gly Arg Lys Asp Val Ser Tyr Tyr Ile Ala Cys Lys Lys Cys Asn Ser
385                 390                 395                 400

His Ala Gly Val Cys Glu Glu Cys Gly Gly His Val Arg Tyr Leu Pro
                405                 410                 415

Arg Gln Ser Gly Leu Lys Asn Thr Ser Val Met Met Val Asp Leu Leu
            420                 425                 430

Ala His Thr Asn Tyr Thr Phe Glu Ile Glu Ala Val Asn Gly Val Ser
        435                 440                 445

Asp Leu Ser Pro Gly Ala Arg Gln Tyr Val Ser Val Asn Val Thr Thr
    450                 455                 460

Asn Gln Ala Ala Pro Ser Pro Val Thr Asn Val Lys Lys Gly Lys Ile
465                 470                 475                 480

Ala Lys Asn Ser Ile Ser Leu Ser Trp Gln Glu Pro Asp Arg Pro Asn
                485                 490                 495

Gly Ile Ile Leu Glu Tyr Glu Ile Lys Tyr Phe Glu Lys Asp Gln Glu
            500                 505                 510

Thr Ser Tyr Thr Ile Ile Lys Ser Lys Glu Thr Thr Ile Thr Ala Glu
        515                 520                 525

Gly Leu Lys Pro Ala Ser Val Tyr Val Phe Gln Ile Arg Ala Arg Thr
    530                 535                 540

Ala Ala Gly Tyr Gly Val Phe Ser Arg Arg Phe Glu Phe Glu Thr Thr
545                 550                 555                 560

Pro Val Phe Ala Ala Ser Ser Asp Gln Ser Gln Ile Pro Val Ile Ala

                565                 570                 575

Val Ser Val Thr Val Gly Val Ile Leu Leu Ala Val Val Ile Gly Val
            580                 585                 590

Leu Leu Ser Gly Ser Cys Cys Glu Cys Gly Cys Gly Arg Ala Ser Ser
        595                 600                 605

Leu Cys Ala Val Ala His Pro Ser Leu Ile Trp Arg Cys Gly Tyr Ser
    610                 615                 620

Lys Ala Lys Gln Asp Pro Glu Glu Glu Lys Met His Phe His Asn Gly
625                 630                 635                 640

His Ile Lys Leu Pro Gly Val Arg Thr Tyr Ile Asp Pro His Thr Tyr
                645                 650                 655

Glu Asp Pro Asn Gln Ala Val His Glu Phe Ala Lys Glu Ile Glu Ala
            660                 665                 670

Ser Cys Ile Thr Ile Glu Arg Val Ile Gly Ala Gly Glu Phe Gly Glu
        675                 680                 685

Val Cys Ser Gly Arg Leu Lys Leu Pro Gly Lys Arg Glu Leu Pro Val
    690                 695                 700

Ala Ile Lys Thr Leu Lys Val Gly Tyr Thr Glu Lys Gln Arg Arg Asp
705                 710                 715                 720

Phe Leu Gly Glu Ala Ser Ile Met Gly Gln Phe Asp His Pro Asn Ile
                725                 730                 735

Ile His Leu Glu Gly Val Val Thr Lys Ser Lys Pro Val Met Ile Val
            740                 745                 750

Thr Glu Tyr Met Glu Asn Gly Ser Leu Asp Thr Phe Leu Lys Lys Asn
        755                 760                 765

Asp Gly Gln Phe Thr Val Ile Gln Leu Val Gly Met Leu Arg Gly Ile
    770                 775                 780

Ser Ala Gly Met Lys Tyr Leu Ser Asp Met Gly Tyr Val His Arg Asp
785                 790                 795                 800

Leu Ala Ala Arg Asn Ile Leu Ile Asn Ser Asn Leu Val Cys Lys Val
                805                 810                 815

Ser Asp Phe Gly Leu Ser Arg Val Leu Glu Asp Asp Pro Glu Ala Ala
            820                 825                 830

Tyr Thr Thr Arg Gly Gly Lys Ile Pro Ile Arg Trp Thr Ala Pro Glu

835                    840                    845

Ala Ile Ala Phe Arg Lys Phe Thr Ser Ala Ser Asp Val Trp Ser Tyr
    850                 855                 860

Gly Ile Val Met Trp Glu Val Val Ser Tyr Gly Glu Arg Pro Tyr Trp
865                 870                 875                 880

Glu Met Thr Asn Gln Asp Val Ile Lys Ala Val Glu Glu Gly Tyr Arg
                885                 890                 895

Leu Pro Ser Pro Met Asp Cys Pro Ala Ala Leu Tyr Gln Leu Met Leu
            900                 905                 910

Asp Cys Trp Gln Lys Glu Arg Asn Ser Arg Pro Lys Phe Asp Glu Ile
        915                 920                 925

Val Asn Met Leu Asp Lys Leu Ile Arg Asn Pro Ser Ser Leu Lys Thr
    930                 935                 940

Leu Val Asn Ala Ser Cys Arg Val Ser Asn Leu Leu Ala Glu His Ser
945                 950                 955                 960

Pro Leu Gly Ser Gly Ala Tyr Arg Ser Val Gly Glu Trp Leu Glu Ala
                965                 970                 975

Ile Lys Met Gly Arg Tyr Thr Glu Ile Phe Met Glu Asn Gly Tyr Ser
            980                 985                 990

Ser Met Asp Ala Val Ala Gln Val  Thr Leu Glu Asp Leu  Arg Arg Leu
        995                 1000                1005

Gly Val  Thr Leu Val Gly His  Gln Lys Lys Ile Met  Asn Ser Leu
    1010                1015                1020

Gln Glu  Met Lys Val Gln Leu  Val Asn Gly Met Val  Pro Leu
    1025                1030                1035

<210>  11
<211>  876
<212>  PRT
<213>  mouse

<400>  11

Met Arg Gly Ser Gly Pro Arg Gly Ala Gly His Arg Arg Thr Gln Gly
1               5                   10                  15

Arg Gly Gly Gly Asp Asp Thr Pro Arg Val Pro Ala Ser Leu Ala Gly
            20                  25                  30

Cys Tyr Ser Ala Pro Leu Lys Gly Pro Leu Trp Thr Cys Leu Leu Leu
        35                  40                  45

Cys Ala Ala Leu Arg Thr Leu Leu Ala Ser Pro Ser Asn Glu Val Asn
    50                  55                  60

Leu Leu Asp Ser Arg Thr Val Met Gly Asp Leu Gly Trp Ile Ala Phe
65                  70                  75                  80

Pro Lys Asn Gly Trp Glu Glu Ile Gly Glu Val Asp Glu Asn Tyr Ala
                85                  90                  95

Pro Ile His Thr Tyr Gln Val Cys Lys Val Met Glu Gln Asn Gln Asn
            100                 105                 110

Asn Trp Leu Leu Thr Ser Trp Ile Ser Asn Glu Gly Ala Ser Arg Ile
        115                 120                 125

Phe Ile Glu Leu Lys Phe Thr Leu Arg Asp Cys Asn Ser Leu Pro Gly
    130                 135                 140

Gly Leu Gly Thr Cys Lys Glu Thr Phe Asn Met Tyr Tyr Phe Glu Ser
145                 150                 155                 160

Asp Asp Glu Asn Gly Arg Ser Ile Lys Glu Asn Gln Tyr Ile Lys Ile
                165                 170                 175

Asp Thr Ile Ala Ala Asp Glu Ser Phe Thr Glu Leu Asp Leu Gly Asp
            180                 185                 190

Arg Val Met Lys Leu Asn Thr Glu Val Arg Asp Val Gly Pro Leu Ser
        195                 200                 205

Lys Lys Gly Phe Tyr Leu Ala Phe Gln Asp Val Gly Ala Cys Ile Ala
    210                 215                 220

Leu Val Ser Val Arg Val Tyr Tyr Lys Lys Cys Pro Ser Val Val Arg
225                 230                 235                 240

His Leu Ala Ile Phe Pro Asp Thr Ile Thr Gly Ala Asp Ser Ser Gln
                245                 250                 255

Leu Leu Glu Val Ser Gly Ser Cys Val Asn His Ser Val Thr Asp Asp
            260                 265                 270

Pro Pro Lys Met His Cys Ser Ala Glu Gly Glu Trp Leu Val Pro Ile
        275                 280                 285

Gly Lys Cys Met Cys Lys Ala Gly Tyr Glu Glu Lys Asn Gly Thr Cys
    290                 295                 300

Gln Ala Pro Ser Pro Val Thr Asn Val Lys Lys Gly Lys Ile Ala Lys
305                 310                 315                 320

Asn Ser Ile Ser Leu Ser Trp Gln Glu Pro Asp Arg Pro Asn Gly Ile
                325                 330                 335

Ile Leu Glu Tyr Glu Ile Lys Tyr Phe Glu Lys Asp Gln Glu Thr Ser
            340                 345                 350

Tyr Thr Ile Ile Lys Ser Lys Glu Thr Ser Ile Thr Ala Glu Gly Leu
        355                 360                 365

Lys Pro Ala Ser Val Tyr Val Phe Gln Ile Arg Ala Arg Thr Ala Ala
    370                 375                 380

Gly Tyr Gly Val Phe Ser Arg Arg Phe Glu Phe Glu Thr Thr Pro Val
385                 390                 395                 400

Ser Val Ala Ala Ser Asn Asp Gln Ser Gln Ile Pro Ile Ile Ala Val
                405                 410                 415

Ser Val Thr Val Gly Val Ile Leu Leu Ala Val Met Ile Gly Phe Leu
            420                 425                 430

Leu Ser Gly Ser Cys Cys Asp Cys Gly Cys Gly Arg Ala Ser Ser Leu
        435                 440                 445

Cys Ala Val Ala His Pro Ser Leu Ile Trp Arg Cys Gly Tyr Ser Lys
    450                 455                 460

Ala Lys Gln Asp Pro Glu Glu Glu Lys Met His Phe His Asn Gly His
465                 470                 475                 480

Ile Lys Leu Pro Gly Val Arg Thr Tyr Ile Asp Pro His Thr Tyr Glu
                485                 490                 495

Asp Pro Asn Gln Ala Val His Glu Phe Ala Lys Glu Ile Glu Ala Ser
            500                 505                 510

Cys Ile Thr Ile Glu Arg Val Ile Gly Ala Gly Glu Phe Gly Glu Val
        515                 520                 525

Cys Ser Gly Arg Leu Lys Leu Pro Gly Lys Arg Glu Leu Pro Val Ala
    530                 535                 540

Ile Lys Thr Leu Lys Val Gly Tyr Thr Glu Lys Gln Arg Arg Asp Phe
545                 550                 555                 560

Leu Gly Glu Ala Ser Ile Met Gly Gln Phe Asp His Pro Asn Ile Ile
                565                 570                 575

His Leu Glu Gly Val Val Thr Lys Ser Lys Pro Val Met Ile Val Thr
            580                 585                 590

Glu Tyr Met Glu Asn Gly Ser Leu Asp Thr Phe Leu Lys Lys Asn Asp
        595                 600                 605

Gly Gln Phe Thr Val Ile Gln Leu Val Gly Met Leu Arg Gly Ile Ala
    610                 615                 620

Ala Gly Met Lys Tyr Leu Ser Asp Met Gly Tyr Val His Arg Asp Leu
625                 630                 635                 640

Ala Ala Arg Asn Ile Leu Ile Asn Ser Asn Leu Val Cys Lys Val Ser
                645                 650                 655

Asp Phe Gly Leu Ser Arg Val Leu Glu Asp Asp Pro Glu Ala Ala Tyr
            660                 665                 670

Thr Thr Arg Gly Gly Lys Ile Pro Ile Arg Trp Thr Ala Pro Glu Ala
        675                 680                 685

Ile Ala Phe Arg Lys Phe Thr Ser Ser Ser Asp Val Trp Ser Tyr Gly
    690                 695                 700

Ile Val Met Trp Glu Val Val Ser Tyr Gly Glu Arg Pro Tyr Trp Glu
705                 710                 715                 720

Met Thr Asn Gln Asp Val Ile Lys Ala Val Glu Glu Gly Tyr Arg Leu
                725                 730                 735

Pro Ser Pro Met Asp Cys Pro Ala Ala Leu Tyr Gln Leu Met Leu Asp
            740                 745                 750

Cys Trp Gln Lys Asp Arg Asn Ser Arg Pro Lys Phe Asp Glu Ile Val
        755                 760                 765

Asn Met Leu Asp Lys Leu Ile Arg Asn Pro Ser Ser Leu Lys Thr Leu
    770                 775                 780

Val Asn Ala Ser Ser Arg Val Ser Thr Leu Leu Ala Glu His Gly Ser
785                 790                 795                 800

Leu Gly Ser Gly Ala Tyr Arg Ser Val Gly Glu Trp Leu Glu Ala Ile
                805                 810                 815

Lys Met Gly Arg Tyr Thr Glu Ile Phe Met Glu Asn Gly Tyr Ser Ser
            820                 825                 830

Met Asp Ala Val Ala Gln Val Thr Leu Glu Asp Leu Arg Arg Leu Gly
        835                 840                 845

Val Thr Leu Val Gly His Gln Lys Lys Ile Met Ser Ser Leu Gln Glu
    850                 855                 860

Met Lys Val Gln Met Val Asn Gly Met Val Pro Val
865                 870                 875

<210>  12
<211>  1040
<212>  PRT
<213>  rat

<220>
<221>  misc_feature
<222>  (641)..(641)
<223>  Xaa can be any naturally occurring amino acid

<220>
<221>  misc_feature
<222>  (645)..(645)
<223>  Xaa can be any naturally occurring amino acid

<220>
<221>  misc_feature
<222>  (654)..(654)
<223>  Xaa can be any naturally occurring amino acid

<220>
<221>  misc_feature
<222>  (667)..(667)
<223>  Xaa can be any naturally occurring amino acid

<220>
<221>  misc_feature
<222>  (680)..(680)
<223>  Xaa can be any naturally occurring amino acid

<220>
<221>  misc_feature
<222>  (687)..(687)
<223>  Xaa can be any naturally occurring amino acid

<220>
<221>  misc_feature
<222>  (690)..(690)
<223>  Xaa can be any naturally occurring amino acid

<220>
<221>  misc_feature
<222>  (693)..(693)
<223>  Xaa can be any naturally occurring amino acid

<220>
<221>  misc_feature
<222>  (712)..(712)
<223>  Xaa can be any naturally occurring amino acid

<220>
<221>  misc_feature
<222>  (717)..(717)
<223>  Xaa can be any naturally occurring amino acid

<220>
<221>  misc_feature
<222>  (726)..(726)
<223>  Xaa can be any naturally occurring amino acid

<220>

<221>  misc_feature
<222>  (734)..(734)
<223>  Xaa can be any naturally occurring amino acid

<220>
<221>  misc_feature
<222>  (747)..(747)
<223>  Xaa can be any naturally occurring amino acid

<220>
<221>  misc_feature
<222>  (749)..(749)
<223>  Xaa can be any naturally occurring amino acid

<220>
<221>  misc_feature
<222>  (795)..(795)
<223>  Xaa can be any naturally occurring amino acid

<220>
<221>  misc_feature
<222>  (806)..(806)
<223>  Xaa can be any naturally occurring amino acid

<220>
<221>  misc_feature
<222>  (813)..(813)
<223>  Xaa can be any naturally occurring amino acid

<220>
<221>  misc_feature
<222>  (820)..(820)
<223>  Xaa can be any naturally occurring amino acid

<220>
<221>  misc_feature
<222>  (830)..(830)
<223>  Xaa can be any naturally occurring amino acid

<220>
<221>  misc_feature
<222>  (832)..(832)
<223>  Xaa can be any naturally occurring amino acid

<220>
<221>  misc_feature
<222>  (862)..(862)
<223>  Xaa can be any naturally occurring amino acid

<220>
<221>  misc_feature
<222>  (928)..(929)
<223>  Xaa can be any naturally occurring amino acid

<220>
<221>  misc_feature
<222>  (936)..(936)
<223>  Xaa can be any naturally occurring amino acid

<220>
<221>  misc_feature
<222>  (943)..(943)
<223>  Xaa can be any naturally occurring amino acid

<220>
<221>  misc_feature
<222>  (974)..(974)
<223>  Xaa can be any naturally occurring amino acid

<400> 12

Met Arg Gly Ser Gly Pro Arg Gly Ala Gly Arg Arg Arg Thr Gln Gly
1               5                   10                  15

Arg Gly Gly Gly Gly Asp Thr Pro Arg Val Pro Ala Ser Leu Ala Gly
            20                  25                  30

Cys Tyr Ser Ala Pro Leu Lys Gly Pro Leu Trp Thr Cys Leu Leu Leu
        35                  40                  45

Cys Ala Ala Leu Arg Thr Leu Leu Ala Ser Pro Ser Asn Glu Val Asn
    50                  55                  60

Leu Leu Asp Ser Arg Thr Val Leu Gly Asp Leu Gly Trp Ile Ala Phe
65                  70                  75                  80

Pro Lys Asn Gly Trp Glu Glu Ile Gly Glu Val Asp Glu Asn Tyr Ala
                85                  90                  95

Pro Ile His Thr Tyr Gln Val Cys Lys Val Met Glu Gln Asn Gln Asn
            100                 105                 110

Asn Trp Leu Leu Thr Ser Trp Ile Ser Asn Glu Gly Ala Ser Arg Ile
        115                 120                 125

Phe Ile Glu Leu Lys Phe Thr Leu Arg Asp Cys Asn Ser Leu Pro Gly
    130                 135                 140

Gly Leu Gly Thr Cys Lys Glu Thr Phe Asn Met Tyr Tyr Phe Glu Ser
145                 150                 155                 160

Asp Asp Glu Asn Gly Arg Asn Ile Lys Glu Asn Gln Tyr Ile Lys Ile
                165                 170                 175

Asp Thr Ile Ala Ala Asp Glu Ser Phe Thr Glu Leu Asp Leu Gly Asp
            180                 185                 190

Arg Val Met Lys Leu Asn Thr Glu Val Arg Asp Val Gly Pro Leu Ser
        195                 200                 205

Lys Lys Gly Phe Tyr Leu Ala Phe Gln Asp Val Gly Ala Cys Ile Ala
    210                 215                 220

Leu Val Ser Val Arg Val Tyr Tyr Lys Lys Cys Pro Ser Val Val Arg
225                 230                 235                 240

His Leu Ala Val Phe Pro Asp Thr Ile Thr Gly Ala Asp Ser Ser Gln
                245                 250                 255

Leu Leu Glu Val Ser Gly Ser Cys Val Asn His Ser Val Thr Asp Asp

            260                 265                 270

Pro Pro Lys Met His Cys Ser Ala Glu Gly Glu Trp Leu Val Pro Ile
        275                 280                 285

Gly Lys Cys Met Cys Lys Ala Gly Tyr Glu Glu Lys Asn Gly Thr Cys
    290                 295                 300

Gln Val Cys Arg Pro Gly Phe Phe Lys Ala Ser Pro His Ser Gln Thr
305                 310                 315                 320

Cys Ser Lys Cys Pro Pro His Ser Tyr Thr His Glu Glu Ala Ser Thr
                325                 330                 335

Ser Cys Val Cys Glu Lys Asp Tyr Phe Arg Arg Glu Ser Asp Pro Pro
            340                 345                 350

Thr Met Ala Cys Thr Arg Pro Pro Ser Ala Pro Arg Asn Ala Ile Ser
        355                 360                 365

Asn Val Asn Glu Thr Ser Val Phe Leu Glu Trp Ile Pro Pro Ala Asp
    370                 375                 380

Thr Gly Gly Arg Lys Asp Val Ser Tyr Tyr Ile Ala Cys Lys Lys Cys
385                 390                 395                 400

Asn Ser His Ala Gly Val Cys Glu Glu Cys Gly Gly His Val Arg Tyr
                405                 410                 415

Leu Pro Gln Gln Ile Gly Leu Lys Asn Thr Ser Val Met Met Ala Asp
            420                 425                 430

Leu Leu Ala His Thr Asn Tyr Thr Phe Glu Ile Glu Ala Val Asn Gly
        435                 440                 445

Val Ser Asp Leu Ser Pro Gly Thr Arg Gln Tyr Val Ser Val Asn Val
    450                 455                 460

Thr Thr Asn Gln Ala Ala Pro Ser Pro Val Thr Asn Val Lys Lys Gly
465                 470                 475                 480

Lys Ile Ala Lys Asn Ser Ile Ser Leu Ser Trp Gln Glu Pro Asp Arg
                485                 490                 495

Pro Asn Gly Ile Ile Leu Glu Tyr Glu Ile Lys Tyr Phe Glu Lys Asp
            500                 505                 510

Gln Glu Thr Ser Tyr Thr Ile Ile Lys Ser Lys Glu Thr Thr Ile Thr
        515                 520                 525

Ala Glu Gly Leu Lys Pro Ala Ser Val Tyr Val Phe Gln Ile Arg Ala

530                 535                 540

Arg Thr Ala Ala Gly Tyr Gly Val Phe Ser Arg Arg Phe Glu Phe Glu
545                 550                 555                 560

Thr Thr Pro Val Phe Ala Ala Ser Asn Asp Gln Ser Gln Ile Pro Ile
                565                 570                 575

Ile Ala Val Ser Val Thr Val Gly Val Ile Leu Leu Ala Val Met Ile
            580                 585                 590

Gly Phe Leu Leu Ser Gly Ser Cys Cys Asp Met Trp Leu Trp Glu Gly
        595                 600                 605

Phe Phe Pro Val Arg Cys Cys Pro Ser Lys Pro Asn Met Ala Val Trp
    610                 615                 620

Leu Gln Gln Ser Lys Ala Gly Ser Arg Arg Gly Lys Asp Ala Leu Ser
625                 630                 635                 640

Xaa Arg Ala His Xaa Thr Ala Arg Ser Lys Asn Val His Xaa Ser Thr
                645                 650                 655

His Leu Arg Arg Ser His Ser Ser Arg Ser Xaa Ile Cys Gln Gly Asp
            660                 665                 670

Arg Gly Leu Met His His His Xaa Glu Ser Tyr Trp Ser Arg Xaa Ile
        675                 680                 685

Trp Xaa Ser Leu Xaa Trp Thr Phe Glu Thr Thr Arg Lys Lys Arg Ile
    690                 695                 700

Ala Cys Gly Tyr Gln Asn Ser Xaa Ser Arg Leu His Xaa Lys Ala Ala
705                 710                 715                 720

Gln Arg Phe Ser Glu Xaa Ser Glu Tyr His Gly Ala Val Xaa Ser Ser
                725                 730                 735

Lys His His Pro Ser Arg Arg Arg Cys Asp Xaa Lys Xaa Thr Cys Asp
            740                 745                 750

Asp Ser Asp Arg Val His Gly Glu Trp Leu Leu Arg His Ile Phe Lys
        755                 760                 765

Glu Lys Arg Trp Pro Val His Cys Asp Ser Ala Cys Trp His Ala Glu
    770                 775                 780

Arg His Arg Cys Arg Asn Glu Val Pro Phe Xaa His Gly Leu Arg Ala
785                 790                 795                 800

Gln Arg Pro Cys Cys Xaa Lys His Leu Asn Gln Gln Xaa Pro Cys Val

                805                 810                 815

Gln Ser Val Xaa Leu Trp Thr Phe Gln Gly Ala Gly Arg Xaa Ser Xaa
            820                 825                 830

Gly Ser Leu Tyr His Lys Gly Arg Gln Asn Ser Asn Gln Val Asp Cys
        835                 840                 845

Ser Arg Ser Asn Ser Phe Ser Lys Val Tyr Leu Cys Gln Xaa Cys Leu
    850                 855                 860

Glu Leu Trp Asn Arg Asn Val Gly Ser Cys Val Leu Trp Arg Glu Thr
865                 870                 875                 880

Val Leu Gly Asp Asp Gln Ser Gly Cys Asp Gln Gly Ser Gly Arg Arg
                885                 890                 895

Leu Pro Pro Ala Lys Pro His Gly Leu Ser Cys Cys Pro Leu Ser Ile
            900                 905                 910

Asn Ala Gly Leu Leu Ala Glu Arg Ser Gln Gln Gln Ala Gln Val Xaa
        915                 920                 925

Xaa His Ser Gln His Ala Gly Xaa Ala Asp Thr Lys Pro Lys Xaa Phe
    930                 935                 940

Glu Asp Thr Gly Glu Arg Val Lys Gln Gly Ile Tyr Phe Val Gly Arg
945                 950                 955                 960

Thr Trp Ile Phe Gly Val Trp Gly Leu Gln Ile Ser Arg Xaa Met Ala
                965                 970                 975

Arg Ser Asn Gln Asn Gly Pro Val His Arg Asp Phe His Gly Lys Trp
            980                 985                 990

Ile Gln Phe Asn Gly Arg Cys Gly Ser Gly Asp Leu Gly Gly Phe Glu
        995                 1000                1005

Thr Pro Gly Ser Asp Ser Gly Arg Ser Pro Glu Glu Asp His Glu
    1010                1015                1020

Gln Pro Ser Arg Asp Glu Gly Ala Ala Gly Lys Arg Asp Gly Ala
    1025                1030                1035

Ser Val
    1040

<210> 13
<211> 998
<212> PRT
<213> human

<400>  13

Met Val Phe Gln Thr Arg Tyr Pro Ser Trp Ile Ile Leu Cys Tyr Ile
1               5                   10                  15

Trp Leu Leu Arg Phe Ala His Thr Gly Glu Ala Gln Ala Ala Lys Glu
            20                  25                  30

Val Leu Leu Leu Asp Ser Lys Ala Gln Gln Thr Glu Leu Glu Trp Ile
        35                  40                  45

Ser Ser Pro Pro Asn Gly Trp Glu Glu Ile Ser Gly Leu Asp Glu Asn
    50                  55                  60

Tyr Thr Pro Ile Arg Thr Tyr Gln Val Cys Gln Val Met Glu Pro Asn
65                  70                  75                  80

Gln Asn Asn Trp Leu Arg Thr Asn Trp Ile Ser Lys Gly Asn Ala Gln
                85                  90                  95

Arg Ile Phe Val Glu Leu Lys Phe Thr Leu Arg Asp Cys Asn Ser Leu
            100                 105                 110

Pro Gly Val Leu Gly Thr Cys Lys Glu Thr Phe Asn Leu Tyr Tyr Tyr
        115                 120                 125

Glu Thr Asp Tyr Asp Thr Gly Arg Asn Ile Arg Glu Asn Leu Tyr Val
    130                 135                 140

Lys Ile Asp Thr Ile Ala Ala Asp Glu Ser Phe Thr Gln Gly Asp Leu
145                 150                 155                 160

Gly Glu Arg Lys Met Lys Leu Asn Thr Glu Val Arg Glu Ile Gly Pro
                165                 170                 175

Leu Ser Lys Lys Gly Phe Tyr Leu Ala Phe Gln Asp Val Gly Ala Cys
            180                 185                 190

Ile Ala Leu Val Ser Val Lys Val Tyr Tyr Lys Lys Cys Trp Ser Ile
        195                 200                 205

Ile Glu Asn Leu Ala Ile Phe Pro Asp Thr Val Thr Gly Ser Glu Phe
    210                 215                 220

Ser Ser Leu Val Glu Val Arg Gly Thr Cys Val Ser Ser Ala Glu Glu
225                 230                 235                 240

Glu Ala Glu Asn Ala Pro Arg Met His Cys Ser Ala Glu Gly Glu Trp
                245                 250                 255

Leu Val Pro Ile Gly Lys Cys Ile Cys Lys Ala Gly Tyr Gln Gln Lys
            260                 265                 270

Gly Asp Thr Cys Glu Pro Cys Gly Arg Gly Phe Tyr Lys Ser Ser Ser
        275                 280                 285

Gln Asp Leu Gln Cys Ser Arg Cys Pro Thr His Ser Phe Ser Asp Lys
    290                 295                 300

Glu Gly Ser Ser Arg Cys Glu Cys Glu Asp Gly Tyr Tyr Arg Ala Pro
305                 310                 315                 320

Ser Asp Pro Pro Tyr Val Ala Cys Thr Arg Pro Pro Ser Ala Pro Gln
                325                 330                 335

Asn Leu Ile Phe Asn Ile Asn Gln Thr Thr Val Ser Leu Glu Trp Ser
            340                 345                 350

Pro Pro Ala Asp Asn Gly Gly Arg Asn Asp Val Thr Tyr Arg Ile Leu
        355                 360                 365

Cys Lys Arg Cys Ser Trp Glu Gln Gly Glu Cys Val Pro Cys Gly Ser
    370                 375                 380

Asn Ile Gly Tyr Met Pro Gln Gln Thr Gly Leu Glu Asp Asn Tyr Val
385                 390                 395                 400

Thr Val Met Asp Leu Leu Ala His Ala Asn Tyr Thr Phe Glu Val Glu
                405                 410                 415

Ala Val Asn Gly Val Ser Asp Leu Ser Arg Ser Gln Arg Leu Phe Ala
            420                 425                 430

Ala Val Ser Ile Thr Thr Gly Gln Ala Ala Pro Ser Gln Val Ser Gly
        435                 440                 445

Val Met Lys Glu Arg Val Leu Gln Arg Ser Val Glu Leu Ser Trp Gln
    450                 455                 460

Glu Pro Glu His Pro Asn Gly Val Ile Thr Glu Tyr Glu Ile Lys Tyr
465                 470                 475                 480

Tyr Glu Lys Asp Gln Arg Glu Arg Thr Tyr Ser Thr Val Lys Thr Lys
                485                 490                 495

Ser Thr Ser Ala Ser Ile Asn Asn Leu Lys Pro Gly Thr Val Tyr Val
            500                 505                 510

Phe Gln Ile Arg Ala Phe Thr Ala Ala Gly Tyr Gly Asn Tyr Ser Pro
        515                 520                 525

Arg Leu Asp Val Ala Thr Leu Glu Glu Ala Thr Gly Lys Met Phe Glu
    530                 535                 540

Ala Thr Ala Val Ser Ser Glu Gln Asn Pro Val Ile Ile Ile Ala Val
545                 550                 555                 560

Val Ala Val Ala Gly Thr Ile Ile Leu Val Phe Met Val Phe Gly Phe
                565                 570                 575

Ile Ile Gly Arg Arg His Cys Gly Tyr Ser Lys Ala Asp Gln Glu Gly
            580                 585                 590

Asp Glu Glu Leu Tyr Phe His Phe Lys Phe Pro Gly Thr Lys Thr Tyr
        595                 600                 605

Ile Asp Pro Glu Thr Tyr Glu Asp Pro Asn Arg Ala Val His Gln Phe
    610                 615                 620

Ala Lys Glu Leu Asp Ala Ser Cys Ile Lys Ile Glu Arg Val Ile Gly
625                 630                 635                 640

Ala Gly Glu Phe Gly Glu Val Cys Ser Gly Arg Leu Lys Leu Pro Gly
                645                 650                 655

Lys Arg Asp Val Ala Val Ala Ile Lys Thr Leu Lys Val Gly Tyr Thr
            660                 665                 670

Glu Lys Gln Arg Arg Asp Phe Leu Cys Glu Ala Ser Ile Met Gly Gln
        675                 680                 685

Phe Asp His Pro Asn Val Val His Leu Glu Gly Val Val Thr Arg Gly
    690                 695                 700

Lys Pro Val Met Ile Val Ile Glu Phe Met Glu Asn Gly Ala Leu Asp
705                 710                 715                 720

Ala Phe Leu Arg Lys His Asp Gly Gln Phe Thr Val Ile Gln Leu Val
                725                 730                 735

Gly Met Leu Arg Gly Ile Ala Ala Gly Met Arg Tyr Leu Ala Asp Met
            740                 745                 750

Gly Tyr Val His Arg Asp Leu Ala Ala Arg Asn Ile Leu Val Asn Ser
        755                 760                 765

Asn Leu Val Cys Lys Val Ser Asp Phe Gly Leu Ser Arg Val Ile Glu
    770                 775                 780

Asp Asp Pro Glu Ala Val Tyr Thr Thr Thr Gly Gly Lys Ile Pro Val
785                 790                 795                 800

Arg Trp Thr Ala Pro Glu Ala Ile Gln Tyr Arg Lys Phe Thr Ser Ala
                805                 810                 815

Ser Asp Val Trp Ser Tyr Gly Ile Val Met Trp Glu Val Met Ser Tyr
            820                 825                 830

Gly Glu Arg Pro Tyr Trp Asp Met Ser Asn Gln Asp Val Ile Lys Ala
        835                 840                 845

Ile Glu Glu Gly Tyr Arg Leu Pro Ala Pro Met Asp Cys Pro Ala Gly
    850                 855                 860

Leu His Gln Leu Met Leu Asp Cys Trp Gln Lys Glu Arg Ala Glu Arg
865                 870                 875                 880

Pro Lys Phe Glu Gln Ile Val Gly Ile Leu Asp Lys Met Ile Arg Asn
                885                 890                 895

Pro Asn Ser Leu Lys Thr Pro Leu Gly Thr Cys Ser Arg Pro Ile Ser
            900                 905                 910

Pro Leu Leu Asp Gln Asn Thr Pro Asp Phe Thr Thr Phe Cys Ser Val
        915                 920                 925

Gly Glu Trp Leu Gln Ala Ile Lys Met Glu Arg Tyr Lys Asp Asn Phe
    930                 935                 940

Thr Ala Ala Gly Tyr Asn Ser Leu Glu Ser Val Ala Arg Met Thr Ile
945                 950                 955                 960

Glu Asp Val Met Ser Leu Gly Ile Thr Leu Val Gly His Gln Lys Lys
                965                 970                 975

Ile Met Ser Ser Ile Gln Thr Met Arg Ala Gln Met Leu His Leu His
            980                 985                 990

Gly Thr Gly Ile Gln Val
        995

<210>  14
<211>  998
<212>  PRT
<213>  mouse

<400>  14

Met Val Val Gln Thr Arg Phe Pro Ser Trp Ile Ile Leu Cys Tyr Ile
1               5                   10                  15

Trp Leu Leu Gly Phe Ala His Thr Gly Glu Ala Gln Ala Ala Lys Glu
            20                  25                  30

Val Leu Leu Leu Asp Ser Lys Ala Gln Gln Thr Glu Leu Glu Trp Ile
        35                  40                  45

Ser Ser Pro Pro Ser Gly Trp Glu Glu Ile Ser Gly Leu Asp Glu Asn
    50                  55                  60

Tyr Thr Pro Ile Arg Thr Tyr Gln Val Cys Gln Val Met Glu Pro Asn
65                  70                  75                  80

Gln Asn Asn Trp Leu Arg Thr Asn Trp Ile Ser Lys Gly Asn Ala Gln
                85                  90                  95

Arg Ile Phe Val Glu Leu Lys Phe Thr Leu Arg Asp Cys Asn Ser Leu
            100                 105                 110

Pro Gly Val Leu Gly Thr Cys Lys Glu Thr Phe Asn Leu Tyr Tyr Tyr
        115                 120                 125

Glu Thr Asp Tyr Asp Thr Gly Arg Asn Ile Arg Glu Asn Leu Tyr Val
    130                 135                 140

Lys Ile Asp Thr Ile Ala Ala Asp Glu Ser Phe Thr Gln Gly Asp Leu
145                 150                 155                 160

Gly Glu Arg Lys Met Lys Leu Asn Thr Glu Val Arg Glu Ile Gly Pro
                165                 170                 175

Leu Ser Lys Lys Gly Phe Tyr Leu Ala Phe Gln Asp Val Gly Ala Cys
            180                 185                 190

Ile Ala Leu Val Ser Val Lys Val Tyr Tyr Lys Lys Cys Trp Thr Ile
        195                 200                 205

Val Glu Asn Leu Ala Val Phe Pro Asp Thr Val Thr Gly Ser Glu Phe
    210                 215                 220

Ser Ser Leu Val Glu Val Arg Gly Thr Cys Val Ser Ser Ala Glu Glu
225                 230                 235                 240

Glu Ala Glu Asn Ser Pro Arg Met His Cys Ser Ala Glu Gly Glu Trp
                245                 250                 255

Leu Val Pro Ile Gly Lys Cys Ile Cys Lys Ala Gly Tyr Gln Gln Lys
            260                 265                 270

Gly Asp Thr Cys Glu Pro Cys Gly Arg Arg Phe Tyr Lys Ser Ser Ser
        275                 280                 285

Gln Asp Leu Gln Cys Ser Arg Cys Pro Thr His Ser Phe Ser Asp Arg
    290                 295                 300

Glu Gly Ser Ser Arg Cys Glu Cys Glu Asp Gly Tyr Tyr Arg Ala Pro
305                 310                 315                 320

Ser Asp Pro Pro Tyr Val Ala Cys Thr Arg Pro Pro Ser Ala Pro Gln
                325                 330                 335

Asn Leu Ile Phe Asn Ile Asn Gln Thr Thr Val Ser Leu Glu Trp Ser
            340                 345                 350

Pro Pro Ala Asp Asn Gly Gly Arg Asn Asp Val Thr Tyr Arg Ile Leu
        355                 360                 365

Cys Lys Arg Cys Ser Trp Glu Gln Gly Glu Cys Val Pro Cys Gly Ser
    370                 375                 380

Asn Ile Gly Tyr Met Pro Gln Gln Thr Gly Leu Glu Asp Asn Tyr Val
385                 390                 395                 400

Thr Val Met Asp Leu Leu Ala His Ala Asn Tyr Thr Phe Glu Val Glu
                405                 410                 415

Ala Val Asn Gly Val Ser Asp Leu Ser Arg Ser Gln Arg Leu Phe Ala
            420                 425                 430

Ala Val Ser Ile Thr Thr Gly Gln Ala Ala Pro Ser Gln Val Ser Gly
        435                 440                 445

Val Met Lys Glu Arg Val Leu Gln Arg Ser Val Gln Leu Ser Trp Gln
    450                 455                 460

Glu Pro Glu His Pro Asn Gly Val Ile Thr Glu Tyr Glu Ile Lys Tyr
465                 470                 475                 480

Tyr Glu Lys Asp Gln Arg Glu Arg Thr Tyr Ser Thr Leu Lys Thr Lys
                485                 490                 495

Ser Thr Ser Ala Ser Ile Asn Asn Leu Lys Pro Gly Thr Val Tyr Val
            500                 505                 510

Phe Gln Ile Arg Ala Val Thr Ala Ala Gly Tyr Gly Asn Tyr Ser Pro
        515                 520                 525

Arg Leu Asp Val Ala Thr Leu Glu Glu Ala Ser Gly Lys Met Phe Glu
    530                 535                 540

Ala Thr Ala Val Ser Ser Glu Gln Asn Pro Val Ile Ile Ile Ala Val
545                 550                 555                 560

Val Ala Val Ala Gly Thr Ile Ile Leu Val Phe Met Val Phe Gly Phe
                565                 570                 575

Ile Ile Gly Arg Arg His Cys Gly Tyr Ser Lys Ala Asp Gln Glu Gly
            580                 585                 590

Asp Glu Glu Leu Tyr Phe His Phe Lys Phe Pro Gly Thr Lys Thr Tyr
        595                 600                 605

Ile Asp Pro Glu Thr Tyr Glu Asp Pro Asn Arg Ala Val His Gln Phe
    610                 615                 620

Ala Lys Glu Leu Asp Ala Ser Cys Ile Lys Ile Glu Arg Val Ile Gly
625                 630                 635                 640

Ala Gly Glu Phe Gly Glu Val Cys Ser Gly Arg Leu Lys Leu Pro Gly
                645                 650                 655

Gln Arg Asp Val Ala Val Ala Ile Lys Thr Leu Lys Val Gly Tyr Thr
            660                 665                 670

Glu Lys Gln Arg Arg Asp Phe Leu Cys Glu Ala Ser Ile Met Gly Gln
        675                 680                 685

Phe Asp His Pro Asn Val Val His Leu Glu Gly Val Val Thr Arg Gly
    690                 695                 700

Lys Pro Val Met Ile Val Ile Glu Phe Met Glu Asn Gly Ala Leu Asp
705                 710                 715                 720

Ala Phe Leu Arg Lys His Asp Gly Gln Phe Thr Val Ile Gln Leu Val
                725                 730                 735

Gly Met Leu Arg Gly Ile Ala Ala Gly Met Arg Tyr Leu Ala Asp Met
            740                 745                 750

Gly Tyr Val His Arg Asp Leu Ala Ala Arg Asn Ile Leu Val Asn Ser
        755                 760                 765

Asn Leu Val Cys Lys Val Ser Asp Phe Gly Leu Ser Arg Val Ile Glu
    770                 775                 780

Asp Asp Pro Glu Ala Val Tyr Thr Thr Thr Gly Gly Lys Ile Pro Val
785                 790                 795                 800

Arg Trp Thr Ala Pro Glu Ala Ile Gln Tyr Arg Lys Phe Thr Ser Ala
                805                 810                 815

Ser Asp Val Trp Ser Tyr Gly Ile Val Met Trp Glu Val Met Ser Tyr
            820                 825                 830

Gly Glu Arg Pro Tyr Trp Asp Met Ser Asn Gln Asp Val Ile Lys Ala
        835                 840                 845

Ile Glu Glu Gly Tyr Arg Leu Pro Ala Pro Met Asp Cys Pro Ala Gly
    850                 855                 860

Leu His Gln Leu Met Leu Asp Cys Trp Gln Lys Asp Arg Ala Glu Arg
865                 870                 875                 880

Pro Lys Phe Glu Gln Ile Val Gly Ile Leu Asp Lys Met Ile Arg Asn
                885                 890                 895

Pro Ser Ser Leu Lys Thr Pro Leu Gly Thr Cys Ser Arg Pro Leu Ser
            900                 905                 910

Pro Leu Leu Asp Gln Ser Thr Pro Asp Phe Thr Ala Phe Cys Ser Val
        915                 920                 925

Gly Glu Trp Leu Gln Ala Ile Lys Met Glu Arg Tyr Lys Asp Asn Phe
    930                 935                 940

Thr Ala Ala Gly Tyr Asn Ser Leu Glu Ser Val Ala Arg Met Thr Ile
945                 950                 955                 960

Asp Asp Val Met Ser Leu Gly Ile Thr Leu Val Gly His Gln Lys Lys
                965                 970                 975

Ile Met Ser Ser Ile Gln Thr Met Arg Ala Gln Met Leu His Leu His
            980                 985                 990

Gly Thr Gly Ile Gln Val
        995

<210>  15
<211>  1005
<212>  PRT
<213>  human

<400>  15

Met Ala Pro Ala Arg Gly Arg Leu Pro Pro Ala Leu Trp Val Val Thr
1               5                   10                  15

Ala Ala Ala Ala Ala Ala Thr Cys Val Ser Ala Ala Arg Gly Glu Val
            20                  25                  30

Asn Leu Leu Asp Thr Ser Thr Ile His Gly Asp Trp Gly Trp Leu Thr
        35                  40                  45

Tyr Pro Ala His Gly Trp Asp Ser Ile Asn Glu Val Asp Glu Ser Phe
    50                  55                  60

Gln Pro Ile His Thr Tyr Gln Val Cys Asn Val Met Ser Pro Asn Gln
65                  70                  75                  80

Asn Asn Trp Leu Arg Thr Ser Trp Val Pro Arg Asp Gly Ala Arg Arg
                85                  90                  95

Val Tyr Ala Glu Ile Lys Phe Thr Leu Arg Asp Cys Asn Ser Met Pro
            100                 105                 110

Gly Val Leu Gly Thr Cys Lys Glu Thr Phe Asn Leu Tyr Tyr Leu Glu
        115                 120                 125

Ser Asp Arg Asp Leu Gly Ala Ser Thr Gln Glu Ser Gln Phe Leu Lys
    130                 135                 140

Ile Asp Thr Ile Ala Ala Asp Glu Ser Phe Thr Gly Ala Asp Leu Gly
145                 150                 155                 160

Val Arg Arg Leu Lys Leu Asn Thr Glu Val Arg Ser Val Gly Pro Leu
                165                 170                 175

Ser Lys Arg Gly Phe Tyr Leu Ala Phe Gln Asp Ile Gly Ala Cys Leu
            180                 185                 190

Ala Ile Leu Ser Leu Arg Ile Tyr Tyr Lys Lys Cys Pro Ala Met Val
        195                 200                 205

Arg Asn Leu Ala Ala Phe Ser Glu Ala Val Thr Gly Ala Asp Ser Ser
    210                 215                 220

Ser Leu Val Glu Val Arg Gly Gln Cys Val Arg His Ser Glu Glu Arg
225                 230                 235                 240

Asp Thr Pro Lys Met Tyr Cys Ser Ala Glu Gly Glu Trp Leu Val Pro
                245                 250                 255

Ile Gly Lys Cys Val Cys Ser Ala Gly Tyr Glu Glu Arg Arg Asp Ala
            260                 265                 270

Cys Val Ala Cys Glu Leu Gly Phe Tyr Lys Ser Ala Pro Gly Asp Gln
        275                 280                 285

Leu Cys Ala Arg Cys Pro Pro His Ser His Ser Ala Ala Pro Ala Ala
    290                 295                 300

Gln Ala Cys His Cys Asp Leu Ser Tyr Tyr Arg Ala Ala Leu Asp Pro
305                 310                 315                 320

Pro Ser Ser Ala Cys Thr Arg Pro Pro Ser Ala Pro Val Asn Leu Ile
                325                 330                 335

Ser Ser Val Asn Gly Thr Ser Val Thr Leu Glu Trp Ala Pro Pro Leu
            340                 345                 350

Asp Pro Gly Gly Arg Ser Asp Ile Thr Tyr Asn Ala Val Cys Arg Arg
        355                 360                 365

Cys Pro Trp Ala Leu Ser Arg Cys Glu Ala Cys Gly Ser Gly Thr Arg
    370                 375                 380

Phe Val Pro Gln Gln Thr Ser Leu Val Gln Ala Ser Leu Leu Val Ala
385                 390                 395                 400

Asn Leu Leu Ala His Met Asn Tyr Ser Phe Trp Ile Glu Ala Val Asn
                405                 410                 415

Gly Val Ser Asp Leu Ser Pro Glu Pro Arg Arg Ala Ala Val Val Asn
            420                 425                 430

Ile Thr Thr Asn Gln Ala Ala Pro Ser Gln Val Val Val Ile Arg Gln
        435                 440                 445

Glu Arg Ala Gly Gln Thr Ser Val Ser Leu Leu Trp Gln Glu Pro Glu
    450                 455                 460

Gln Pro Asn Gly Ile Ile Leu Glu Tyr Glu Ile Lys Tyr Tyr Glu Lys
465                 470                 475                 480

Asp Lys Glu Met Gln Ser Tyr Ser Thr Leu Lys Ala Val Thr Thr Arg
                485                 490                 495

Ala Thr Val Ser Gly Leu Lys Pro Gly Thr Arg Tyr Val Phe Gln Val
            500                 505                 510

Arg Ala Arg Thr Ser Ala Gly Cys Gly Arg Phe Ser Gln Ala Met Glu
        515                 520                 525

Val Glu Thr Gly Lys Pro Arg Pro Arg Tyr Asp Thr Arg Thr Ile Val
    530                 535                 540

Trp Ile Cys Leu Thr Leu Ile Thr Gly Leu Val Val Leu Leu Leu Leu
545                 550                 555                 560

Leu Ile Cys Lys Lys Arg His Cys Gly Tyr Ser Lys Ala Phe Gln Asp
                565                 570                 575

Ser Asp Glu Glu Lys Met His Tyr Gln Asn Gly Gln Ala Pro Pro Pro
            580                 585                 590

Val Phe Leu Pro Leu His His Pro Pro Gly Lys Leu Pro Glu Pro Gln
        595                 600                 605

Phe Tyr Ala Glu Pro His Thr Tyr Glu Glu Pro Gly Arg Ala Gly Arg
    610                 615                 620

Ser Phe Thr Arg Glu Ile Glu Ala Ser Arg Ile His Ile Glu Lys Ile
625                 630                 635                 640

Ile Gly Ser Gly Asp Ser Gly Glu Val Cys Tyr Gly Arg Leu Arg Val
                645                 650                 655

Pro Gly Gln Arg Asp Val Pro Val Ala Ile Lys Ala Leu Lys Ala Gly
            660                 665                 670

Tyr Thr Glu Arg Gln Arg Arg Asp Phe Leu Ser Glu Ala Ser Ile Met
        675                 680                 685

Gly Gln Phe Asp His Pro Asn Ile Ile Arg Leu Glu Gly Val Val Thr
    690                 695                 700

Arg Gly Arg Leu Ala Met Ile Val Thr Glu Tyr Met Glu Asn Gly Ser
705                 710                 715                 720

Leu Asp Thr Phe Leu Arg Thr His Asp Gly Gln Phe Thr Ile Met Gln
                725                 730                 735

Leu Val Gly Met Leu Arg Gly Val Gly Ala Gly Met Arg Tyr Leu Ser
            740                 745                 750

Asp Leu Gly Tyr Val His Arg Asp Leu Ala Ala Arg Asn Val Leu Val
        755                 760                 765

Asp Ser Asn Leu Val Cys Lys Val Ser Asp Phe Gly Leu Ser Arg Val
    770                 775                 780

Leu Glu Asp Asp Pro Asp Ala Ala Tyr Thr Thr Thr Gly Gly Lys Ile
785                 790                 795                 800

Pro Ile Arg Trp Thr Ala Pro Glu Ala Ile Ala Phe Arg Thr Phe Ser
                805                 810                 815

Ser Ala Ser Asp Val Trp Ser Phe Gly Val Val Met Trp Glu Val Leu
            820                 825                 830

Ala Tyr Gly Glu Arg Pro Tyr Trp Asn Met Thr Asn Arg Asp Val Ile
        835                 840                 845

Ser Ser Val Glu Glu Gly Tyr Arg Leu Pro Ala Pro Met Gly Cys Pro
    850                 855                 860

His Ala Leu His Gln Leu Met Leu Asp Cys Trp His Lys Asp Arg Ala
865                 870                 875                 880

Gln Arg Pro Arg Phe Ser Gln Ile Val Ser Val Leu Asp Ala Leu Ile
                885                 890                 895

Arg Ser Pro Glu Ser Leu Arg Ala Thr Ala Thr Val Ser Arg Cys Pro
            900                 905                 910

Pro Pro Ala Phe Val Arg Ser Cys Phe Asp Leu Arg Gly Gly Ser Gly
        915                 920                 925

Gly Gly Gly Gly Leu Thr Val Gly Asp Trp Leu Asp Ser Ile Arg Met
    930                 935                 940

Gly Arg Tyr Arg Asp His Phe Ala Ala Gly Gly Tyr Ser Ser Leu Gly
945                 950                 955                 960

Met Val Leu Arg Met Asn Ala Gln Asp Val Arg Ala Leu Gly Ile Thr
                965                 970                 975

Leu Met Gly His Gln Lys Lys Ile Leu Gly Ser Ile Gln Thr Met Arg
            980                 985                 990

Ala Gln Leu Thr Ser Thr Gln Gly  Pro Arg Arg His Leu
        995                 1000                1005

<210>  16
<211>  1004
<212>  PRT
<213>  mouse

<400>  16

Met Ala Pro Ala Arg Ala Arg Leu Ser Pro Ala Leu Trp Val Val Thr
1               5                   10                  15

Ala Ala Ala Ala Ala Thr Cys Val Ser Ala Gly Arg Gly Glu Val Asn
            20                  25                  30

Leu Leu Asp Thr Ser Thr Ile His Gly Asp Trp Gly Trp Leu Thr Tyr
        35                  40                  45

Pro Ala His Gly Trp Asp Ser Ile Asn Glu Val Asp Glu Ser Phe Arg
    50                  55                  60

Pro Ile His Thr Tyr Gln Val Cys Asn Val Met Ser Pro Asn Gln Asn
65                  70                  75                  80

Asn Trp Leu Arg Thr Asn Trp Val Pro Arg Asp Gly Ala Arg Arg Val
                85                  90                  95

Tyr Ala Glu Ile Lys Phe Thr Leu Arg Asp Cys Asn Ser Ile Pro Gly
            100                 105                 110

Val Leu Gly Thr Cys Lys Glu Thr Phe Asn Leu His Tyr Leu Glu Ser
        115                 120                 125

Asp Arg Asp Leu Gly Ala Ser Thr Gln Glu Ser Gln Phe Leu Lys Ile
    130                 135                 140

Asp Thr Ile Ala Ala Asp Glu Ser Phe Thr Gly Ala Asp Leu Gly Val

145                 150                 155                 160

Arg Arg Leu Lys Leu Asn Thr Glu Val Arg Gly Val Gly Pro Leu Ser
                165                 170                 175

Lys Arg Gly Phe Tyr Leu Ala Phe Gln Asp Ile Gly Ala Cys Leu Ala
            180                 185                 190

Ile Leu Ser Leu Arg Ile Tyr Tyr Lys Lys Cys Pro Ala Met Val Arg
        195                 200                 205

Asn Leu Ala Ala Phe Ser Glu Ala Val Thr Gly Ala Asp Ser Ser Ser
    210                 215                 220

Leu Val Glu Val Arg Gly Gln Cys Val Arg His Ser Glu Glu Arg Asp
225                 230                 235                 240

Thr Pro Lys Met Tyr Cys Ser Ala Glu Gly Glu Trp Leu Val Pro Ile
                245                 250                 255

Gly Lys Cys Val Cys Ser Ala Gly Tyr Glu Glu Arg Arg Asp Ala Cys
            260                 265                 270

Met Ala Cys Glu Leu Gly Phe Tyr Lys Ser Ala Pro Gly Asp Gln Leu
        275                 280                 285

Cys Ala Arg Cys Pro Pro His Ser His Ser Ala Thr Pro Ala Ala Gln
    290                 295                 300

Thr Cys Arg Cys Asp Leu Ser Tyr Tyr Arg Ala Ala Leu Asp Pro Pro
305                 310                 315                 320

Ser Ala Ala Cys Thr Arg Pro Pro Ser Ala Pro Val Asn Leu Ile Ser
                325                 330                 335

Ser Val Asn Gly Thr Ser Val Thr Leu Glu Trp Ala Pro Pro Leu Asp
            340                 345                 350

Pro Gly Gly Arg Ser Asp Ile Thr Tyr Asn Ala Val Cys Arg Arg Cys
        355                 360                 365

Pro Trp Ala Leu Ser His Cys Glu Ala Cys Gly Ser Gly Thr Arg Phe
    370                 375                 380

Val Pro Gln Gln Thr Ser Leu Ala Gln Ala Ser Leu Leu Val Ala Asn
385                 390                 395                 400

Leu Leu Ala His Met Asn Tyr Ser Phe Trp Ile Glu Ala Val Asn Gly
                405                 410                 415

Val Ser Asn Leu Ser Pro Glu Pro Arg Ser Ala Ala Val Val Asn Ile

            420                 425                 430

Thr Thr Asn Gln Ala Ala Pro Ser Gln Val Val Val Ile Arg Gln Glu
        435                 440                 445

Arg Ala Gly Gln Thr Ser Val Ser Leu Leu Trp Gln Glu Pro Glu Gln
    450                 455                 460

Pro Asn Gly Ile Ile Leu Glu Tyr Glu Ile Lys Tyr Tyr Glu Lys Asp
465                 470                 475                 480

Lys Glu Met Gln Ser Tyr Ser Thr Leu Lys Ala Val Thr Thr Arg Ala
                485                 490                 495

Thr Val Ser Gly Leu Lys Pro Gly Thr Arg Tyr Val Phe Gln Val Arg
            500                 505                 510

Ala Arg Thr Ser Ala Gly Cys Gly Arg Phe Ser Gln Ala Met Glu Val
        515                 520                 525

Glu Thr Gly Lys Pro Arg Pro Arg Tyr Asp Thr Arg Thr Ile Val Trp
    530                 535                 540

Ile Cys Leu Thr Leu Ile Thr Gly Leu Val Val Leu Leu Leu Leu Leu
545                 550                 555                 560

Ile Cys Lys Lys Arg His Cys Gly Tyr Ser Lys Ala Phe Gln Asp Ser
                565                 570                 575

Asp Glu Glu Lys Met His Tyr Gln Asn Gly Gln Ala Pro Pro Pro Val
            580                 585                 590

Phe Leu Pro Leu Asn His Pro Pro Gly Lys Phe Pro Glu Thr Gln Phe
        595                 600                 605

Ser Ala Glu Pro His Thr Tyr Glu Glu Pro Gly Arg Ala Gly Arg Ser
    610                 615                 620

Phe Thr Arg Glu Ile Glu Ala Ser Arg Ile His Ile Glu Lys Ile Ile
625                 630                 635                 640

Gly Ser Gly Glu Ser Gly Glu Val Cys Tyr Gly Arg Leu Gln Val Pro
                645                 650                 655

Gly Gln Arg Asp Val Pro Val Ala Ile Lys Ala Leu Lys Ala Gly Tyr
            660                 665                 670

Thr Glu Arg Gln Arg Gln Asp Phe Leu Ser Glu Ala Ala Ile Met Gly
        675                 680                 685

Gln Phe Asp His Pro Asn Ile Ile Arg Leu Glu Gly Val Val Thr Arg

690                 695                 700

Gly Arg Leu Ala Met Ile Val Thr Glu Tyr Met Glu Asn Gly Ser Leu
705                 710                 715                 720

Asp Ala Phe Leu Arg Thr His Asp Gly Gln Phe Thr Ile Val Gln Leu
                725                 730                 735

Val Gly Met Leu Arg Gly Val Gly Ala Gly Met Arg Tyr Leu Ser Asp
            740                 745                 750

Leu Gly Tyr Ile His Arg Asp Leu Ala Ala Arg Asn Val Leu Val Asp
        755                 760                 765

Gly Arg Leu Val Cys Lys Val Ser Asp Phe Gly Leu Ser Arg Ala Leu
    770                 775                 780

Glu Asp Asp Pro Glu Ala Ala Tyr Thr Thr Ala Gly Gly Lys Ile Pro
785                 790                 795                 800

Ile Arg Trp Thr Ala Pro Glu Ala Ile Ala Phe Arg Thr Phe Ser Ser
                805                 810                 815

Ala Ser Asp Val Trp Ser Phe Gly Val Val Met Trp Glu Val Leu Ala
            820                 825                 830

Tyr Gly Glu Arg Pro Tyr Trp Asn Met Thr Asn Gln Asp Val Ile Ser
        835                 840                 845

Ser Val Glu Glu Gly Tyr Arg Leu Pro Ala Pro Met Gly Cys Pro Arg
    850                 855                 860

Ala Leu His Gln Leu Met Leu Asp Cys Trp His Lys Asp Arg Ala Gln
865                 870                 875                 880

Arg Pro Arg Phe Ala His Val Val Ser Val Leu Asp Ala Leu Val His
                885                 890                 895

Ser Pro Glu Ser Leu Arg Ala Thr Ala Thr Val Ser Arg Cys Pro Pro
            900                 905                 910

Pro Ala Phe Ala Arg Ser Cys Phe Asp Leu Arg Ala Gly Gly Ser Gly
        915                 920                 925

Asn Gly Asp Leu Thr Val Gly Asp Trp Leu Asp Ser Ile Arg Met Gly
    930                 935                 940

Arg Tyr Arg Asp His Phe Ala Ala Gly Gly Tyr Ser Ser Leu Gly Met
945                 950                 955                 960

Val Leu Arg Met Asn Ala Gln Asp Val Arg Ala Leu Gly Ile Thr Leu

965                    970                    975

Met Gly His Gln Lys Lys Ile Leu Gly Ser Ile Gln Thr Met Arg Ala
            980                 985                 990

Gln Leu Ser Ser Thr Gln Gly Arg  Arg Arg His Leu
        995                 1000

<210>  17
<211>  978
<212>  PRT
<213>  rat

<400>  17

Met Ala Gly Asp Leu Asn Leu Leu Asp Thr Ser Thr Ile His Gly Asp
1               5                   10                  15

Trp Gly Trp Leu Thr Tyr Pro Ala His Gly Trp Asp Ser Ile Asn Glu
            20                  25                  30

Val Asp Glu Ser Phe Arg Pro Ile His Thr Tyr Gln Val Cys Asn Val
        35                  40                  45

Met Ser Pro Asn Gln Asn Asn Trp Leu Arg Thr Ser Trp Val Pro Arg
    50                  55                  60

Asp Gly Ala Arg Arg Val Tyr Ala Glu Ile Lys Phe Thr Leu Arg Asp
65                  70                  75                  80

Cys Asn Ser Ile Pro Gly Val Leu Gly Thr Cys Lys Glu Thr Phe Asn
                85                  90                  95

Leu His Tyr Leu Glu Ser Asp Arg Asp Leu Gly Ala Ser Thr Gln Glu
            100                 105                 110

Ser Gln Phe Leu Lys Ile Asp Thr Ile Ala Ala Asp Glu Ser Phe Thr
        115                 120                 125

Gly Ala Asp Leu Gly Val Arg Arg Leu Lys Leu Asn Thr Glu Val Arg
    130                 135                 140

Gly Val Gly Pro Leu Ser Lys Arg Gly Phe Tyr Leu Ala Phe Gln Asp
145                 150                 155                 160

Ile Gly Ala Cys Leu Ala Ile Leu Ser Leu Arg Ile Tyr Tyr Lys Lys
                165                 170                 175

Cys Pro Ala Met Val Arg Asn Leu Ala Ala Phe Ser Glu Ala Val Thr
            180                 185                 190

Gly Ala Asp Ser Ser Ser Leu Val Glu Val Arg Gly Gln Cys Val Arg
        195                 200                 205

His Ser Glu Glu Arg Asp Thr Pro Lys Met Tyr Cys Ser Ala Glu Gly
    210                 215                 220

Glu Trp Leu Val Pro Ile Gly Lys Cys Val Cys Ser Ala Gly Tyr Glu
225                 230                 235                 240

Glu Arg Arg Asp Ala Cys Met Ala Cys Glu Leu Gly Phe Tyr Lys Ser
                245                 250                 255

Ala Pro Gly Asp Gln Leu Cys Ala Arg Cys Pro Pro His Ser His Ser
            260                 265                 270

Ala Thr Pro Ala Ala Gln Thr Cys Arg Cys Asp Leu Ser Tyr Tyr Arg
        275                 280                 285

Ala Ala Leu Asp Pro Pro Ser Ala Ala Cys Thr Arg Pro Pro Ser Ala
    290                 295                 300

Pro Val Asn Leu Ile Ser Ser Val Asn Gly Thr Ser Val Thr Leu Glu
305                 310                 315                 320

Trp Ala Pro Pro Leu Asp Pro Gly Gly Arg Ser Asp Ile Thr Tyr Asn
                325                 330                 335

Ala Val Cys Arg Arg Cys Pro Trp Ala Leu Ser His Cys Glu Ala Cys
            340                 345                 350

Gly Ser Gly Thr Arg Phe Val Pro Gln Gln Thr Ser Leu Ala Gln Ala
        355                 360                 365

Ser Leu Leu Val Ala Asn Leu Leu Ala His Met Asn Tyr Ser Phe Trp
    370                 375                 380

Ile Glu Ala Val Asn Gly Val Ser Asn Leu Ser Pro Glu Pro Arg Asn
385                 390                 395                 400

Ala Ala Val Val Asn Ile Thr Thr Asn Gln Ala Ala Pro Ser Gln Val
                405                 410                 415

Val Val Ile Arg Gln Glu Arg Ala Gly Gln Thr Ser Val Ser Leu Leu
            420                 425                 430

Trp Gln Glu Pro Glu Gln Pro Asn Gly Ile Ile Leu Glu Tyr Glu Ile
        435                 440                 445

Lys Tyr Tyr Glu Lys Asp Lys Glu Met Gln Ser Tyr Ser Thr Leu Lys
    450                 455                 460

Ala Val Thr Thr Arg Ala Thr Val Ser Gly Leu Lys Pro Gly Thr Arg
465                 470                 475                 480

Tyr Met Phe Gln Val Arg Ala Arg Thr Ser Ala Gly Cys Gly Arg Phe
                485                 490                 495

Ser Gln Ala Met Glu Val Glu Thr Gly Lys Pro Arg Pro Arg Tyr Asp
            500                 505                 510

Thr Arg Thr Ile Val Trp Ile Cys Leu Thr Leu Ile Thr Gly Leu Val
        515                 520                 525

Val Leu Leu Leu Leu Leu Ile Cys Lys Lys Arg His Cys Gly Tyr Ser
    530                 535                 540

Lys Ala Phe Gln Asp Ser Asp Glu Glu Lys Met His Tyr Gln Asn Gly
545                 550                 555                 560

Gln Ala Pro Pro Pro Val Phe Leu Pro Leu Asn His Pro Pro Gly Lys
                565                 570                 575

Leu Pro Glu Thr Gln Phe Ser Ala Glu Pro His Asn Tyr Glu Glu Pro
            580                 585                 590

Gly Arg Ala Gly Arg Ser Phe Thr Arg Glu Ile Glu Ala Ser Arg Ile
        595                 600                 605

His Ile Glu Lys Ile Ile Gly Ser Gly Glu Ser Gly Glu Val Cys Tyr
    610                 615                 620

Gly Arg Leu Gln Val Pro Gly Gln Arg Asp Val Pro Val Ala Ile Lys
625                 630                 635                 640

Ala Leu Lys Ala Gly Tyr Thr Glu Arg Gln Arg Gln Asp Phe Leu Arg
                645                 650                 655

Glu Ala Ala Ile Met Gly Gln Phe Asp His Pro Asn Ile Ile Arg Leu
            660                 665                 670

Glu Gly Val Val Thr Arg Gly Arg Leu Ala Met Ile Val Thr Glu Tyr
        675                 680                 685

Met Glu Asn Gly Ser Leu Asp Ala Phe Leu Arg Thr His Asp Gly Gln
    690                 695                 700

Phe Thr Ile Leu Gln Leu Val Gly Met Leu Lys Gly Val Gly Ala Gly
705                 710                 715                 720

Met Arg Tyr Leu Ser Asp Leu Gly Tyr Ile His Arg Asp Leu Ala Ala
                725                 730                 735

Arg Asn Ile Leu Val Asp Gly Arg Leu Val Cys Lys Val Ser Asp Phe
            740                 745                 750

Gly Leu Ser Arg Ala Leu Glu Asp Asp Pro Glu Ala Ala Tyr Thr Thr
        755                 760                 765

Ala Gly Gly Lys Ile Pro Ile Arg Trp Thr Ala Pro Glu Ala Ile Ala
    770                 775                 780

Phe Arg Thr Phe Ser Ser Ala Ser Asp Val Trp Ser Phe Gly Val Val
785                 790                 795                 800

Met Trp Glu Val Leu Ala Tyr Gly Glu Arg Pro Tyr Trp Asn Met Thr
                805                 810                 815

Asn Gln Asp Val Ile Ser Ser Val Glu Glu Gly Tyr Arg Leu Pro Ala
            820                 825                 830

Pro Met Gly Cys Pro Arg Ala Leu His Gln Leu Met Leu Asp Cys Trp
        835                 840                 845

His Lys Asp Arg Ala Gln Arg Pro Arg Phe Ser His Val Val Ser Val
    850                 855                 860

Leu Glu Ala Leu Val His Ser Pro Glu Ser Leu Arg Ala Thr Ala Thr
865                 870                 875                 880

Val Ser Arg Cys Pro Ala Pro Ala Phe Ala Arg Ser Cys Phe Asp Leu
                885                 890                 895

Arg Ala Gly Gly Asn Gly Asn Gly Asp Leu Thr Val Gly Asp Trp Leu
            900                 905                 910

Asp Ser Ile Arg Met Gly Arg Tyr Arg Asp His Phe Ala Ala Gly Gly
        915                 920                 925

Tyr Ser Ser Leu Gly Met Val Leu His Met Asn Ala Gln Asp Val Arg
    930                 935                 940

Ala Leu Gly Ile Thr Leu Met Gly His Gln Lys Lys Ile Leu Gly Ser
945                 950                 955                 960

Ile Gln Thr Met Arg Ser Gln Leu Ser Cys Thr Gln Gly Pro Arg Arg
                965                 970                 975

His Leu

<210> 18
<211> 987
<212> PRT
<213> human

<400> 18

Met Ala Leu Arg Arg Leu Gly Ala Ala Leu Leu Leu Leu Pro Leu Leu
1               5                   10                  15

Ala Ala Val Glu Glu Thr Leu Met Asp Ser Thr Thr Ala Thr Ala Glu
            20                  25                  30

Leu Gly Trp Met Val His Pro Pro Ser Gly Trp Glu Glu Val Ser Gly
        35                  40                  45

Tyr Asp Glu Asn Met Asn Thr Ile Arg Thr Tyr Gln Val Cys Asn Val
    50                  55                  60

Phe Glu Ser Ser Gln Asn Asn Trp Leu Arg Thr Lys Phe Ile Arg Arg
65                  70                  75                  80

Arg Gly Ala His Arg Ile His Val Glu Met Lys Phe Ser Val Arg Asp
                85                  90                  95

Cys Ser Ser Ile Pro Ser Val Pro Gly Ser Cys Lys Glu Thr Phe Asn
            100                 105                 110

Leu Tyr Tyr Tyr Glu Ala Asp Phe Asp Ser Ala Thr Lys Thr Phe Pro
        115                 120                 125

Asn Trp Met Glu Asn Pro Trp Val Lys Val Asp Thr Ile Ala Ala Asp
    130                 135                 140

Glu Ser Phe Ser Gln Val Asp Leu Gly Gly Arg Val Met Lys Ile Asn
145                 150                 155                 160

Thr Glu Val Arg Ser Phe Gly Pro Val Ser Arg Ser Gly Phe Tyr Leu
                165                 170                 175

Ala Phe Gln Asp Tyr Gly Gly Cys Met Ser Leu Ile Ala Val Arg Val
            180                 185                 190

Phe Tyr Arg Lys Cys Pro Arg Ile Ile Gln Asn Gly Ala Ile Phe Gln
        195                 200                 205

Glu Thr Leu Ser Gly Ala Glu Ser Thr Ser Leu Val Ala Ala Arg Gly
    210                 215                 220

Ser Cys Ile Ala Asn Ala Glu Glu Val Asp Val Pro Ile Lys Leu Tyr
225                 230                 235                 240

Cys Asn Gly Asp Gly Glu Trp Leu Val Pro Ile Gly Arg Cys Met Cys
                245                 250                 255

Lys Ala Gly Phe Glu Ala Val Glu Asn Gly Thr Val Cys Arg Gly Cys
            260                 265                 270

Pro Ser Gly Thr Phe Lys Ala Asn Gln Gly Asp Glu Ala Cys Thr His
        275                 280                 285

Cys Pro Ile Asn Ser Arg Thr Thr Ser Glu Gly Ala Thr Asn Cys Val
    290                 295                 300

Cys Arg Asn Gly Tyr Tyr Arg Ala Asp Leu Asp Pro Leu Asp Met Pro
305                 310                 315                 320

Cys Thr Thr Ile Pro Ser Ala Pro Gln Ala Val Ile Ser Ser Val Asn
                325                 330                 335

Glu Thr Ser Leu Met Leu Glu Trp Thr Pro Pro Arg Asp Ser Gly Gly
            340                 345                 350

Arg Glu Asp Leu Val Tyr Asn Ile Ile Cys Lys Ser Cys Gly Ser Gly
        355                 360                 365

Arg Gly Ala Cys Thr Arg Cys Gly Asp Asn Val Gln Tyr Ala Pro Arg
    370                 375                 380

Gln Leu Gly Leu Thr Glu Pro Arg Ile Tyr Ile Ser Asp Leu Leu Ala
385                 390                 395                 400

His Thr Gln Tyr Thr Phe Glu Ile Gln Ala Val Asn Gly Val Thr Asp
                405                 410                 415

Gln Ser Pro Phe Ser Pro Gln Phe Ala Ser Val Asn Ile Thr Thr Asn
            420                 425                 430

Gln Ala Ala Pro Ser Ala Val Ser Ile Met His Gln Val Ser Arg Thr
        435                 440                 445

Val Asp Ser Ile Thr Leu Ser Trp Ser Gln Pro Asp Gln Pro Asn Gly
    450                 455                 460

Val Ile Leu Asp Tyr Glu Leu Gln Tyr Tyr Glu Lys Glu Leu Ser Glu
465                 470                 475                 480

Tyr Asn Ala Thr Ala Ile Lys Ser Pro Thr Asn Thr Val Thr Val Gln
                485                 490                 495

Gly Leu Lys Ala Gly Ala Ile Tyr Val Phe Gln Val Arg Ala Arg Thr
            500                 505                 510

Val Ala Gly Tyr Gly Arg Tyr Ser Gly Lys Met Tyr Phe Gln Thr Met
        515                 520                 525

Thr Glu Ala Glu Tyr Gln Thr Ser Ile Gln Glu Lys Leu Pro Leu Ile
    530                 535                 540

Ile Gly Ser Ser Ala Ala Gly Leu Val Phe Leu Ile Ala Val Val Val
545                 550                 555                 560

Ile Ala Ile Val Cys Asn Arg Arg Arg Gly Phe Glu Arg Ala Asp Ser
                565                 570                 575

Glu Tyr Thr Asp Lys Leu Gln His Tyr Thr Ser Gly His Met Thr Pro
            580                 585                 590

Gly Met Lys Ile Tyr Ile Asp Pro Phe Thr Tyr Glu Asp Pro Asn Glu
        595                 600                 605

Ala Val Arg Glu Phe Ala Lys Glu Ile Asp Ile Ser Cys Val Lys Ile
    610                 615                 620

Glu Gln Val Ile Gly Ala Gly Glu Phe Gly Glu Val Cys Ser Gly His
625                 630                 635                 640

Leu Lys Leu Pro Gly Lys Arg Glu Ile Phe Val Ala Ile Lys Thr Leu
                645                 650                 655

Lys Ser Gly Tyr Thr Glu Lys Gln Arg Arg Asp Phe Leu Ser Glu Ala
            660                 665                 670

Ser Ile Met Gly Gln Phe Asp His Pro Asn Val Ile His Leu Glu Gly
        675                 680                 685

Val Val Thr Lys Ser Thr Pro Val Met Ile Ile Thr Glu Phe Met Glu
    690                 695                 700

Asn Gly Ser Leu Asp Ser Phe Leu Arg Gln Asn Asp Gly Gln Phe Thr
705                 710                 715                 720

Val Ile Gln Leu Val Gly Met Leu Arg Gly Ile Ala Ala Gly Met Lys
                725                 730                 735

Tyr Leu Ala Asp Met Asn Tyr Val His Arg Asp Leu Ala Ala Arg Asn
            740                 745                 750

Ile Leu Val Asn Ser Asn Leu Val Cys Lys Val Ser Asp Phe Gly Leu
        755                 760                 765

Ser Arg Phe Leu Glu Asp Asp Thr Ser Asp Pro Thr Tyr Thr Ser Ala
    770                 775                 780

Leu Gly Gly Lys Ile Pro Ile Arg Trp Thr Ala Pro Glu Ala Ile Gln
785                 790                 795                 800

Tyr Arg Lys Phe Thr Ser Ala Ser Asp Val Trp Ser Tyr Gly Ile Val
                805                 810                 815

Met Trp Glu Val Met Ser Tyr Gly Glu Arg Pro Tyr Trp Asp Met Thr
            820                 825                 830

Asn Gln Asp Val Ile Asn Ala Ile Glu Gln Asp Tyr Arg Leu Pro Pro
        835                 840                 845

Pro Met Asp Cys Pro Ser Ala Leu His Gln Leu Met Leu Asp Cys Trp
    850                 855                 860

Gln Lys Asp Arg Asn His Arg Pro Lys Phe Gly Gln Ile Val Asn Thr
865                 870                 875                 880

Leu Asp Lys Met Ile Arg Asn Pro Asn Ser Leu Lys Ala Met Ala Pro
                885                 890                 895

Leu Ser Ser Gly Ile Asn Leu Pro Leu Leu Asp Arg Thr Ile Pro Asp
            900                 905                 910

Tyr Thr Ser Phe Asn Thr Val Asp Glu Trp Leu Glu Ala Ile Lys Met
        915                 920                 925

Gly Gln Tyr Lys Glu Ser Phe Ala Asn Ala Gly Phe Thr Ser Phe Asp
    930                 935                 940

Val Val Ser Gln Met Met Met Glu Asp Ile Leu Arg Val Gly Val Thr
945                 950                 955                 960

Leu Ala Gly His Gln Lys Lys Ile Leu Asn Ser Ile Gln Val Met Arg
                965                 970                 975

Ala Gln Met Asn Gln Ile Gln Ser Val Glu Val
            980                 985

<210>  19
<211>  986
<212>  PRT
<213>  mouse

<400>  19

Met Ala Val Arg Arg Leu Gly Ala Ala Leu Leu Leu Leu Pro Leu Leu
1               5                   10                  15

Ala Ala Val Glu Glu Thr Leu Met Asp Ser Thr Thr Ala Thr Ala Glu
            20                  25                  30

Leu Gly Trp Met Val His Pro Pro Ser Gly Trp Glu Glu Val Ser Gly
        35                  40                  45

Tyr Asp Glu Asn Met Asn Thr Ile Arg Thr Tyr Gln Val Cys Asn Val
    50                  55                  60

Phe Glu Ser Ser Gln Asn Asn Trp Leu Arg Thr Lys Phe Ile Arg Arg
65                  70                  75                  80

Arg Gly Ala His Arg Ile His Val Glu Met Lys Phe Ser Val Arg Asp
                85                  90                  95

Cys Ser Ser Ile Pro Ser Val Pro Gly Ser Cys Lys Glu Thr Phe Asn
            100                 105                 110

Leu Tyr Tyr Tyr Glu Ala Asp Phe Asp Leu Ala Thr Lys Thr Phe Pro
        115                 120                 125

Asn Trp Met Glu Asn Pro Trp Val Lys Val Asp Thr Ile Ala Ala Asp
    130                 135                 140

Glu Ser Phe Ser Gln Val Asp Leu Gly Gly Arg Val Met Lys Ile Asn
145                 150                 155                 160

Thr Glu Val Arg Ser Phe Gly Pro Val Ser Arg Asn Gly Phe Tyr Leu
                165                 170                 175

Ala Phe Gln Asp Tyr Gly Gly Cys Met Ser Leu Ile Ala Val Arg Val
            180                 185                 190

Phe Tyr Arg Lys Cys Pro Arg Ile Ile Gln Asn Gly Ala Ile Phe Gln
        195                 200                 205

Glu Thr Leu Ser Gly Ala Glu Ser Thr Ser Leu Val Ala Ala Arg Gly
    210                 215                 220

Ser Cys Ile Ala Asn Ala Glu Glu Val Asp Val Pro Ile Lys Leu Tyr
225                 230                 235                 240

Cys Asn Gly Asp Gly Glu Trp Leu Val Pro Ile Gly Arg Cys Met Cys
                245                 250                 255

Lys Ala Gly Phe Glu Ala Val Glu Asn Gly Thr Val Cys Arg Gly Cys
            260                 265                 270

Pro Ser Gly Thr Phe Lys Ala Asn Gln Gly Asp Glu Ala Cys Thr His
        275                 280                 285

Cys Pro Ile Asn Ser Arg Thr Thr Ser Glu Gly Ala Thr Asn Cys Val
    290                 295                 300

Cys Arg Asn Gly Tyr Tyr Arg Ala Asp Leu Asp Pro Leu Asp Met Pro
305                 310                 315                 320

Cys Thr Thr Ile Pro Ser Ala Pro Gln Ala Val Ile Ser Ser Val Asn
                325                 330                 335

Glu Thr Ser Leu Met Leu Glu Trp Thr Pro Pro Arg Asp Ser Gly Gly
            340                 345                 350

Arg Glu Asp Leu Val Tyr Asn Ile Ile Cys Lys Ser Cys Gly Ser Gly
        355                 360                 365

Arg Gly Ala Cys Thr Arg Cys Gly Asp Asn Val Gln Tyr Ala Pro Arg
    370                 375                 380

Gln Leu Gly Leu Thr Glu Pro Arg Ile Tyr Ile Ser Asp Leu Leu Ala
385                 390                 395                 400

His Thr Gln Tyr Thr Phe Glu Ile Gln Ala Val Asn Gly Val Thr Asp
                405                 410                 415

Gln Ser Pro Phe Ser Pro Gln Phe Ala Ser Val Asn Ile Thr Thr Asn
            420                 425                 430

Gln Ala Ala Pro Ser Ala Val Ser Ile Met His Gln Val Ser Arg Thr
        435                 440                 445

Val Asp Ser Ile Thr Leu Ser Trp Ser Gln Pro Asp Gln Pro Asn Gly
    450                 455                 460

Val Ile Leu Asp Tyr Glu Leu Gln Tyr Tyr Glu Lys Glu Leu Ser Glu
465                 470                 475                 480

Tyr Asn Ala Thr Ala Ile Lys Ser Pro Thr Asn Thr Val Thr Val Gln
                485                 490                 495

Gly Leu Lys Ala Gly Ala Ile Tyr Val Phe Gln Val Arg Ala Arg Thr
            500                 505                 510

Val Ala Gly Tyr Gly Arg Tyr Ser Gly Lys Met Tyr Phe Gln Thr Met
        515                 520                 525

Thr Glu Ala Glu Tyr Gln Thr Ser Ile Lys Glu Lys Leu Pro Leu Ile
    530                 535                 540

Val Gly Ser Ser Ala Ala Gly Leu Val Phe Leu Ile Ala Val Val Val
545                 550                 555                 560

Ile Ala Ile Val Cys Asn Arg Arg Gly Phe Glu Arg Ala Asp Ser Glu
                565                 570                 575

Tyr Thr Asp Lys Leu Gln His Tyr Thr Ser Gly His Met Thr Pro Gly
            580                 585                 590

Met Lys Ile Tyr Ile Asp Pro Phe Thr Tyr Glu Asp Pro Asn Glu Ala
        595                 600                 605

Val Arg Glu Phe Ala Lys Glu Ile Asp Ile Ser Cys Val Lys Ile Glu
    610                 615                 620

Gln Val Ile Gly Ala Gly Glu Phe Gly Glu Val Cys Ser Gly His Leu
625                 630                 635                 640

Lys Leu Pro Gly Lys Arg Glu Ile Phe Val Ala Ile Lys Thr Leu Lys
                645                 650                 655

Ser Gly Tyr Thr Glu Lys Gln Arg Arg Asp Phe Leu Ser Glu Ala Ser
            660                 665                 670

Ile Met Gly Gln Phe Asp His Pro Asn Val Ile His Leu Glu Gly Val
        675                 680                 685

Val Thr Lys Ser Thr Pro Val Met Ile Ile Thr Glu Phe Met Glu Asn
    690                 695                 700

Gly Ser Leu Asp Ser Phe Leu Arg Gln Asn Asp Gly Gln Phe Thr Val
705                 710                 715                 720

Ile Gln Leu Val Gly Met Leu Arg Gly Ile Ala Ala Gly Met Lys Tyr
                725                 730                 735

Leu Ala Asp Met Asn Tyr Val His Arg Asp Leu Ala Ala Arg Asn Ile
            740                 745                 750

Leu Val Asn Ser Asn Leu Val Cys Lys Val Ser Asp Phe Gly Leu Ser
        755                 760                 765

Arg Phe Leu Glu Asp Asp Thr Ser Asp Pro Thr Tyr Thr Ser Ala Leu
    770                 775                 780

Gly Gly Lys Ile Pro Ile Arg Trp Thr Ala Pro Glu Ala Ile Gln Tyr
785                 790                 795                 800

Arg Lys Phe Thr Ser Ala Ser Asp Val Trp Ser Tyr Gly Ile Val Met
                805                 810                 815

Trp Glu Val Met Ser Tyr Gly Glu Arg Pro Tyr Trp Asp Met Thr Asn
            820                 825                 830

Gln Asp Val Ile Asn Ala Ile Glu Gln Asp Tyr Arg Leu Pro Pro Pro
        835                 840                 845

Met Asp Cys Pro Ser Ala Leu His Gln Leu Met Leu Asp Cys Trp Gln
    850                 855                 860

Lys Asp Arg Asn His Arg Pro Lys Phe Gly Gln Ile Val Asn Thr Leu
865                 870                 875                 880

Asp Lys Met Ile Arg Asn Pro Asn Ser Leu Lys Ala Met Ala Pro Leu
                885                 890                 895

Ser Ser Gly Ile Asn Leu Pro Leu Leu Asp Arg Thr Ile Pro Asp Tyr
            900                 905                 910

Thr Ser Phe Asn Thr Val Asp Glu Trp Leu Glu Ala Ile Lys Met Gly
        915                 920                 925

Gln Tyr Lys Glu Ser Phe Ala Asn Ala Gly Phe Thr Ser Phe Asp Val
    930                 935                 940

Val Ser Gln Met Met Met Glu Asp Ile Leu Arg Val Gly Val Thr Leu
945                 950                 955                 960

Ala Gly His Gln Lys Lys Ile Leu Asn Ser Ile Gln Val Met Arg Ala
                965                 970                 975

Gln Met Asn Gln Ile Gln Ser Val Glu Val
            980                 985

<210> 20
<211> 987
<212> PRT
<213> rat

<400> 20

Met Asp Leu Phe His Phe Lys Leu Tyr Gly Phe Phe Ser Pro Leu Thr
1               5                   10                  15

Gly Ser Asp Thr Glu Thr Leu Met Asp Ser Thr Thr Ala Thr Ala Glu
            20                  25                  30

Leu Gly Trp Met Val His Pro Pro Ser Gly Trp Glu Glu Val Ser Gly
        35                  40                  45

Tyr Asp Glu Asn Met Asn Thr Ile Arg Thr Tyr Gln Val Cys Asn Val
    50                  55                  60

Phe Glu Ser Ser Gln Asn Asn Trp Leu Arg Thr Lys Phe Ile Arg Arg
65                  70                  75                  80

Arg Gly Ala His Arg Ile His Val Glu Met Lys Phe Ser Val Arg Asp
                85                  90                  95

Cys Ser Ser Ile Pro Ser Val Pro Gly Ser Cys Lys Glu Thr Phe Asn
            100                 105                 110

Leu Tyr Tyr Tyr Glu Ala Asp Phe Asp Leu Ala Thr Lys Thr Phe Pro
        115                 120                 125

Asn Trp Met Glu Asn Pro Trp Val Lys Val Asp Thr Ile Ala Ala Asp

130                 135                 140

Glu Ser Phe Ser Gln Val Asp Leu Gly Gly Arg Val Met Lys Ile Asn
145                 150                 155                 160

Thr Glu Val Arg Ser Phe Gly Pro Val Ser Arg Asn Gly Phe Tyr Leu
                165                 170                 175

Ala Phe Gln Asp Tyr Gly Gly Cys Met Ser Leu Ile Ala Val Arg Val
            180                 185                 190

Phe Tyr Arg Lys Cys Pro Arg Val Ile Gln Asn Gly Ala Ile Phe Gln
        195                 200                 205

Glu Thr Leu Ser Gly Ala Glu Ser Thr Ser Leu Val Ala Ala Arg Gly
    210                 215                 220

Ser Cys Ile Ala Asn Ala Glu Glu Val Asp Val Pro Ile Lys Leu Tyr
225                 230                 235                 240

Cys Asn Gly Asp Gly Glu Trp Leu Val Pro Ile Gly Arg Cys Met Cys
                245                 250                 255

Lys Ala Gly Phe Glu Ala Val Glu Asn Gly Thr Val Cys Arg Gly Cys
            260                 265                 270

Pro Ser Gly Thr Phe Lys Ala Asn Gln Gly Asp Glu Ala Cys Thr His
        275                 280                 285

Cys Pro Ile Asn Ser Arg Thr Thr Ser Glu Gly Ala Thr Asn Cys Val
    290                 295                 300

Cys Arg Asn Gly Tyr Tyr Arg Ala Asp Leu Asp Pro Leu Asp Met Pro
305                 310                 315                 320

Cys Thr Thr Ile Pro Ser Ala Pro Gln Ala Val Ile Ser Ser Val Asn
                325                 330                 335

Glu Thr Ser Leu Met Leu Glu Trp Thr Pro Pro Arg Asp Ser Gly Gly
            340                 345                 350

Arg Glu Asp Leu Val Tyr Asn Ile Ile Cys Lys Ser Cys Gly Ser Gly
        355                 360                 365

Arg Gly Ala Cys Thr Arg Cys Gly Asp Asn Val Gln Tyr Ala Pro Arg
    370                 375                 380

Gln Leu Gly Leu Thr Glu Pro Arg Ile Tyr Ile Ser Asp Leu Leu Ala
385                 390                 395                 400

His Thr Gln Tyr Thr Phe Glu Ile Gln Ala Val Asn Gly Val Thr Asp

                405                 410                 415

Gln Ser Pro Phe Ser Pro Gln Phe Ala Ser Val Asn Ile Thr Thr Asn
            420                 425                 430

Gln Ala Ala Pro Ser Ala Val Ser Ile Met His Gln Val Ser Arg Thr
        435                 440                 445

Val Asp Ser Ile Thr Leu Ser Trp Ser Gln Pro Asp Gln Pro Asn Gly
    450                 455                 460

Val Ile Leu Asp Tyr Glu Leu Gln Tyr Tyr Glu Lys Glu Leu Ser Glu
465                 470                 475                 480

Tyr Asn Ala Thr Ala Ile Lys Ser Pro Thr Asn Thr Val Thr Val Gln
                485                 490                 495

Gly Leu Lys Ala Gly Ala Ile Tyr Val Phe Gln Val Arg Ala Arg Thr
            500                 505                 510

Val Ala Gly Tyr Gly Arg Tyr Ser Gly Lys Met Tyr Phe Gln Thr Met
        515                 520                 525

Thr Glu Ala Glu Tyr Gln Thr Ser Ile Lys Glu Lys Leu Pro Leu Ile
    530                 535                 540

Val Gly Ser Ser Ala Ala Gly Val Val Phe Val Ile Ala Val Val Val
545                 550                 555                 560

Ile Ala Ile Val Cys Asn Arg Arg Arg Gly Phe Glu Arg Ala Asp Ser
                565                 570                 575

Glu Tyr Thr Asp Lys Leu Gln His Tyr Thr Ser Gly His Met Thr Pro
            580                 585                 590

Gly Met Lys Ile Tyr Ile Asp Pro Phe Thr Tyr Glu Asp Pro Asn Glu
        595                 600                 605

Ala Val Arg Glu Phe Ala Lys Glu Ile Asp Ile Ser Cys Val Lys Ile
    610                 615                 620

Glu Gln Val Ile Gly Ala Gly Glu Phe Gly Glu Val Cys Ser Gly His
625                 630                 635                 640

Leu Lys Leu Pro Gly Lys Arg Glu Ile Phe Val Ala Ile Lys Thr Leu
                645                 650                 655

Lys Ser Gly Tyr Thr Glu Lys Gln Arg Arg Asp Phe Leu Ser Glu Ala
            660                 665                 670

Ser Ile Met Gly Gln Phe Asp His Pro Asn Val Ile His Leu Glu Gly

        675                 680                 685

Val Val Thr Lys Ser Thr Pro Val Met Ile Ile Thr Glu Phe Met Glu
    690                 695                 700

Asn Gly Ser Leu Asp Ser Phe Leu Arg Gln Asn Asp Gly Gln Phe Thr
705                 710                 715                 720

Val Ile Gln Leu Val Gly Met Leu Arg Gly Ile Ala Ala Gly Met Lys
                725                 730                 735

Tyr Leu Ala Asp Met Asn Tyr Val His Arg Asp Leu Ala Ala Arg Asn
            740                 745                 750

Ile Leu Val Asn Ser Asn Leu Val Cys Lys Val Ser Asp Phe Gly Leu
        755                 760                 765

Ser Arg Phe Leu Glu Asp Asp Thr Ser Asp Pro Thr Tyr Thr Ser Ala
    770                 775                 780

Leu Gly Gly Lys Ile Pro Ile Arg Trp Thr Ala Pro Glu Ala Ile Gln
785                 790                 795                 800

Tyr Arg Lys Phe Thr Ser Ala Ser Asp Val Trp Ser Tyr Gly Ile Val
                805                 810                 815

Met Trp Glu Val Met Ser Tyr Gly Glu Arg Pro Tyr Trp Asp Met Thr
            820                 825                 830

Asn Gln Asp Val Ile Asn Ala Ile Glu Gln Asp Tyr Arg Leu Pro Pro
        835                 840                 845

Pro Met Asp Cys Pro Ser Ala Leu His Gln Leu Met Leu Asp Cys Trp
    850                 855                 860

Gln Lys Asp Arg Asn His Arg Pro Lys Phe Gly Gln Ile Val Asn Thr
865                 870                 875                 880

Leu Asp Lys Met Ile Arg Asn Pro Asn Ser Leu Lys Ala Met Ala Pro
                885                 890                 895

Leu Ser Ser Gly Ile Asn Leu Pro Leu Leu Asp Arg Thr Ile Pro Asp
            900                 905                 910

Tyr Thr Ser Phe Asn Thr Val Asp Glu Trp Leu Glu Ala Ile Lys Met
        915                 920                 925

Gly Gln Tyr Lys Glu Ser Phe Thr Asn Ala Gly Phe Thr Ser Phe Asp
    930                 935                 940

Val Val Ser Gln Met Met Met Glu Asp Ile Leu Arg Val Gly Val Thr

945                 950                 955                 960

Leu Ala Gly His Gln Lys Lys Ile Leu Asn Ser Ile Gln Val Met Arg
                965                 970                 975

Ala Gln Met Asn Gln Ile Gln Ser Val Glu Val
            980                 985

<210>  21
<211>  998
<212>  PRT
<213>  human

<400>  21

Met Ala Arg Ala Arg Pro Pro Pro Pro Pro Ser Pro Pro Pro Gly Leu
1               5                   10                  15

Leu Pro Leu Leu Pro Pro Leu Leu Leu Leu Pro Leu Leu Leu Leu Pro
            20                  25                  30

Ala Gly Cys Arg Ala Leu Glu Glu Thr Leu Met Asp Thr Lys Trp Val
        35                  40                  45

Thr Ser Glu Leu Ala Trp Thr Ser His Pro Glu Ser Gly Trp Glu Glu
    50                  55                  60

Val Ser Gly Tyr Asp Glu Ala Met Asn Pro Ile Arg Thr Tyr Gln Val
65                  70                  75                  80

Cys Asn Val Arg Glu Ser Ser Gln Asn Asn Trp Leu Arg Thr Gly Phe
                85                  90                  95

Ile Trp Arg Arg Asp Val Gln Arg Val Tyr Val Glu Leu Lys Phe Thr
            100                 105                 110

Val Arg Asp Cys Asn Ser Ile Pro Asn Ile Pro Gly Ser Cys Lys Glu
        115                 120                 125

Thr Phe Asn Leu Phe Tyr Tyr Glu Ala Asp Ser Asp Val Ala Ser Ala
    130                 135                 140

Ser Ser Pro Phe Trp Met Glu Asn Pro Tyr Val Lys Val Asp Thr Ile
145                 150                 155                 160

Ala Pro Asp Glu Ser Phe Ser Arg Leu Asp Ala Gly Arg Val Asn Thr
                165                 170                 175

Lys Val Arg Ser Phe Gly Pro Leu Ser Lys Ala Gly Phe Tyr Leu Ala
            180                 185                 190

Phe Gln Asp Gln Gly Ala Cys Met Ser Leu Ile Ser Val Arg Ala Phe
        195                 200                 205

Tyr Lys Lys Cys Ala Ser Thr Thr Ala Gly Phe Ala Leu Phe Pro Glu
    210                 215                 220

Thr Leu Thr Gly Ala Glu Pro Thr Ser Leu Val Ile Ala Pro Gly Thr
225                 230                 235                 240

Cys Ile Pro Asn Ala Val Glu Val Ser Val Pro Leu Lys Leu Tyr Cys
                245                 250                 255

Asn Gly Asp Gly Glu Trp Met Val Pro Val Gly Ala Cys Thr Cys Ala
            260                 265                 270

Thr Gly His Glu Pro Ala Ala Lys Glu Ser Gln Cys Arg Pro Cys Pro
        275                 280                 285

Pro Gly Ser Tyr Lys Ala Lys Gln Gly Glu Gly Pro Cys Leu Pro Cys
    290                 295                 300

Pro Pro Asn Ser Arg Thr Thr Ser Pro Ala Ala Ser Ile Cys Thr Cys
305                 310                 315                 320

His Asn Asn Phe Tyr Arg Ala Asp Ser Asp Ser Ala Asp Ser Ala Cys
                325                 330                 335

Thr Thr Val Pro Ser Pro Pro Arg Gly Val Ile Ser Asn Val Asn Glu
            340                 345                 350

Thr Ser Leu Ile Leu Glu Trp Ser Glu Pro Arg Asp Leu Gly Gly Arg
        355                 360                 365

Asp Asp Leu Leu Tyr Asn Val Ile Cys Lys Lys Cys His Gly Ala Gly
    370                 375                 380

Gly Ala Ser Ala Cys Ser Arg Cys Asp Asp Asn Val Glu Phe Val Pro
385                 390                 395                 400

Arg Gln Leu Gly Leu Thr Glu Arg Arg Val His Ile Ser His Leu Leu
                405                 410                 415

Ala His Thr Arg Tyr Thr Phe Glu Val Gln Ala Val Asn Gly Val Ser
            420                 425                 430

Gly Lys Ser Pro Leu Pro Pro Arg Tyr Ala Ala Val Asn Ile Thr Thr
        435                 440                 445

Asn Gln Ala Ala Pro Ser Glu Val Pro Thr Leu Arg Leu His Ser Ser
    450                 455                 460

Ser Gly Ser Ser Leu Thr Leu Ser Trp Ala Pro Pro Glu Arg Pro Asn
465                 470                 475                 480

Gly Val Ile Leu Asp Tyr Glu Met Lys Tyr Phe Glu Lys Ser Glu Gly
                485                 490                 495

Ile Ala Ser Thr Val Thr Ser Gln Met Asn Ser Val Gln Leu Asp Gly
            500                 505                 510

Leu Arg Pro Asp Ala Arg Tyr Val Val Gln Val Arg Ala Arg Thr Val
        515                 520                 525

Ala Gly Tyr Gly Gln Tyr Ser Arg Pro Ala Glu Phe Glu Thr Thr Ser
    530                 535                 540

Glu Arg Gly Ser Gly Ala Gln Gln Leu Gln Glu Gln Leu Pro Leu Ile
545                 550                 555                 560

Val Gly Ser Ala Thr Ala Gly Leu Val Phe Val Val Ala Val Val Val
                565                 570                 575

Ile Ala Ile Val Cys Leu Arg Lys Gln Arg His Gly Ser Asp Ser Glu
            580                 585                 590

Tyr Thr Glu Lys Leu Gln Gln Tyr Ile Ala Pro Gly Met Lys Val Tyr
        595                 600                 605

Ile Asp Pro Phe Thr Tyr Glu Asp Pro Asn Glu Ala Val Arg Glu Phe
    610                 615                 620

Ala Lys Glu Ile Asp Val Ser Cys Val Lys Ile Glu Glu Val Ile Gly
625                 630                 635                 640

Ala Gly Glu Phe Gly Glu Val Cys Arg Gly Arg Leu Lys Gln Pro Gly
                645                 650                 655

Arg Arg Glu Val Phe Val Ala Ile Lys Thr Leu Lys Val Gly Tyr Thr
            660                 665                 670

Glu Arg Gln Arg Arg Asp Phe Leu Ser Glu Ala Ser Ile Met Gly Gln
        675                 680                 685

Phe Asp His Pro Asn Ile Ile Arg Leu Glu Gly Val Val Thr Lys Ser
    690                 695                 700

Arg Pro Val Met Ile Leu Thr Glu Phe Met Glu Asn Cys Ala Leu Asp
705                 710                 715                 720

Ser Phe Leu Arg Leu Asn Asp Gly Gln Phe Thr Val Ile Gln Leu Val
                725                 730                 735

Gly Met Leu Arg Gly Ile Ala Ala Gly Met Lys Tyr Leu Ser Glu Met
            740                 745                 750

Asn Tyr Val His Arg Asp Leu Ala Ala Arg Asn Ile Leu Val Asn Ser
        755                 760                 765

Asn Leu Val Cys Lys Val Ser Asp Phe Gly Leu Ser Arg Phe Leu Glu
    770                 775                 780

Asp Asp Pro Ser Asp Pro Thr Tyr Thr Ser Ser Leu Gly Gly Lys Ile
785                 790                 795                 800

Pro Ile Arg Trp Thr Ala Pro Glu Ala Ile Ala Tyr Arg Lys Phe Thr
                805                 810                 815

Ser Ala Ser Asp Val Trp Ser Tyr Gly Ile Val Met Trp Glu Val Met
            820                 825                 830

Ser Tyr Gly Glu Arg Pro Tyr Trp Asp Met Ser Asn Gln Asp Val Ile
        835                 840                 845

Asn Ala Val Glu Gln Asp Tyr Arg Leu Pro Pro Pro Met Asp Cys Pro
    850                 855                 860

Thr Ala Leu His Gln Leu Met Leu Asp Cys Trp Val Arg Asp Arg Asn
865                 870                 875                 880

Leu Arg Pro Lys Phe Ser Gln Ile Val Asn Thr Leu Asp Lys Leu Ile
                885                 890                 895

Arg Asn Ala Ala Ser Leu Lys Val Ile Ala Ser Ala Gln Ser Gly Met
            900                 905                 910

Ser Gln Pro Leu Leu Asp Arg Thr Val Pro Asp Tyr Thr Thr Phe Thr
        915                 920                 925

Thr Val Gly Asp Trp Leu Asp Ala Ile Lys Met Gly Arg Tyr Lys Glu
    930                 935                 940

Ser Phe Val Ser Ala Gly Phe Ala Ser Phe Asp Leu Val Ala Gln Met
945                 950                 955                 960

Thr Ala Glu Asp Leu Leu Arg Ile Gly Val Thr Leu Ala Gly His Gln
                965                 970                 975

Lys Lys Ile Leu Ser Ser Ile Gln Asp Met Arg Leu Gln Met Asn Gln
            980                 985                 990

Thr Leu Pro Val Gln Val
        995

<210>  22
<211>  993

<212>  PRT
<213>  mouse

<400>  22

Met Ala Gly Ala Arg Pro Pro Pro Gly Leu Leu Pro Leu Leu Ala Pro
1               5                   10                  15

Leu Leu Leu Pro Leu Leu Leu Pro Ala Gly Cys Trp Ala Leu Glu Glu
            20                  25                  30

Thr Leu Met Asp Thr Lys Trp Val Thr Ser Glu Leu Ala Trp Thr Ser
        35                  40                  45

His Pro Glu Ser Gly Trp Glu Glu Val Ser Gly Tyr Asp Glu Ala Met
    50                  55                  60

Asn Pro Ile Arg Thr Tyr Gln Val Cys Asn Val Arg Glu Ser Ser Gln
65                  70                  75                  80

Asn Asn Trp Leu Arg Thr Gly Phe Ile Trp Arg Arg Glu Val Gln Arg
                85                  90                  95

Val Tyr Val Glu Leu Lys Phe Thr Val Arg Asp Cys Asn Ser Ile Pro
            100                 105                 110

Asn Ile Pro Gly Ser Cys Lys Glu Thr Phe Asn Leu Phe Tyr Tyr Glu
        115                 120                 125

Ala Asp Ser Asp Val Ala Ser Ala Ser Ser Pro Phe Trp Met Glu Asn
    130                 135                 140

Pro Tyr Val Lys Val Asp Thr Ile Ala Pro Asp Glu Ser Phe Ser Arg
145                 150                 155                 160

Leu Asp Ala Gly Arg Val Asn Thr Lys Val Arg Ser Phe Gly Pro Leu
                165                 170                 175

Ser Lys Ala Gly Phe Tyr Leu Ala Phe Gln Asp Gln Gly Ala Cys Met
            180                 185                 190

Ser Leu Ile Ser Val Arg Ala Phe Tyr Lys Lys Cys Ala Ser Thr Thr
        195                 200                 205

Ala Gly Phe Ala Leu Phe Pro Glu Thr Leu Thr Gly Ala Glu Pro Thr
    210                 215                 220

Ser Leu Val Ile Ala Pro Gly Thr Cys Ile Ala Asn Ala Val Glu Val
225                 230                 235                 240

Ser Val Pro Leu Lys Leu Tyr Cys Asn Gly Asp Gly Glu Trp Met Val
                245                 250                 255

Pro Val Gly Ala Cys Thr Cys Ala Thr Gly His Glu Pro Ala Ala Lys
            260                 265                 270

Glu Ser Gln Cys Arg Ala Cys Pro Pro Gly Ser Tyr Lys Ala Lys Gln
        275                 280                 285

Gly Glu Gly Pro Cys Leu Pro Cys Pro Pro Asn Ser Arg Thr Thr Ser
    290                 295                 300

Pro Ala Ala Ser Ile Cys Thr Cys His Asn Asn Phe Tyr Arg Ala Asp
305                 310                 315                 320

Ser Asp Ser Ala Asp Ser Ala Cys Thr Thr Val Pro Ser Pro Pro Arg
                325                 330                 335

Gly Val Ile Ser Asn Val Asn Glu Thr Ser Leu Ile Leu Glu Trp Ser
            340                 345                 350

Glu Pro Arg Asp Leu Gly Gly Arg Asp Asp Leu Leu Tyr Asn Val Ile
        355                 360                 365

Cys Lys Lys Cys Arg Gly Ser Ser Gly Ala Gly Gly Pro Ala Thr Cys
    370                 375                 380

Ser Arg Cys Asp Asp Asn Val Glu Phe Val Pro Arg Gln Leu Gly Leu
385                 390                 395                 400

Thr Glu Arg Arg Val His Ile Ser His Leu Leu Ala His Thr Arg Tyr
                405                 410                 415

Thr Phe Glu Val Gln Ala Val Asn Gly Val Ser Gly Lys Ser Pro Leu
            420                 425                 430

Pro Pro Arg Tyr Ala Ala Val Asn Ile Thr Thr Asn Gln Ala Ala Pro
        435                 440                 445

Ser Glu Val Pro Thr Leu His Leu His Ser Ser Ser Gly Ser Ser Leu
    450                 455                 460

Thr Leu Ser Trp Ala Pro Pro Glu Arg Pro Asn Gly Val Ile Leu Asp
465                 470                 475                 480

Tyr Glu Met Lys Tyr Phe Glu Lys Ser Lys Gly Ile Ala Ser Thr Val
                485                 490                 495

Thr Ser Gln Lys Asn Ser Val Gln Leu Asp Gly Leu Gln Pro Asp Ala
            500                 505                 510

Arg Tyr Val Val Gln Val Arg Ala Arg Thr Val Ala Gly Tyr Gly Gln
        515                 520                 525

Tyr Ser His Pro Ala Glu Phe Glu Thr Thr Ser Glu Arg Gly Ser Gly
    530                 535                 540

Ala Gln Gln Leu Gln Glu Gln Leu Pro Leu Ile Val Gly Ser Thr Val
545                 550                 555                 560

Ala Gly Phe Val Phe Met Val Val Val Val Val Ile Ala Leu Val Cys
                565                 570                 575

Leu Arg Lys Gln Arg His Gly Pro Asp Ala Glu Tyr Thr Glu Lys Leu
            580                 585                 590

Gln Gln Tyr Ile Ala Pro Gly Met Lys Val Tyr Ile Asp Pro Phe Thr
        595                 600                 605

Tyr Glu Asp Pro Asn Glu Ala Val Arg Glu Phe Ala Lys Glu Ile Asp
    610                 615                 620

Val Ser Cys Val Lys Ile Glu Glu Val Ile Gly Ala Gly Glu Phe Gly
625                 630                 635                 640

Glu Val Cys Arg Gly Arg Leu Lys Leu Pro Gly Arg Arg Glu Val Phe
                645                 650                 655

Val Ala Ile Lys Thr Leu Lys Val Gly Tyr Thr Glu Arg Gln Arg Arg
            660                 665                 670

Asp Phe Leu Ser Glu Ala Ser Ile Met Gly Gln Phe Asp His Pro Asn
        675                 680                 685

Ile Ile Arg Leu Glu Gly Val Val Thr Lys Ser Arg Pro Val Met Ile
    690                 695                 700

Leu Thr Glu Phe Met Glu Asn Cys Ala Leu Asp Ser Phe Leu Arg Leu
705                 710                 715                 720

Asn Asp Gly Gln Phe Thr Val Ile Gln Leu Val Gly Met Leu Arg Gly
                725                 730                 735

Ile Ala Ala Gly Met Lys Tyr Leu Ser Glu Met Asn Tyr Val His Arg
            740                 745                 750

Asp Leu Ala Ala Arg Asn Ile Leu Val Asn Ser Asn Leu Val Cys Lys
        755                 760                 765

Val Ser Asp Phe Gly Leu Ser Arg Phe Leu Glu Asp Asp Pro Ser Asp
    770                 775                 780

Pro Thr Tyr Thr Ser Ser Leu Gly Gly Lys Ile Pro Ile Arg Trp Thr
785                 790                 795                 800

Ala Pro Glu Ala Ile Ala Tyr Arg Lys Phe Thr Ser Ala Ser Asp Val
                805                 810                 815

Trp Ser Tyr Gly Ile Val Met Trp Glu Val Met Ser Tyr Gly Glu Arg
            820                 825                 830

Pro Tyr Trp Asp Met Ser Asn Gln Asp Val Ile Asn Ala Val Glu Gln
        835                 840                 845

Asp Tyr Arg Leu Pro Pro Pro Met Asp Cys Pro Thr Ala Leu His Gln
    850                 855                 860

Leu Met Leu Asp Cys Trp Val Arg Asp Arg Asn Leu Arg Pro Lys Phe
865                 870                 875                 880

Ser Gln Ile Val Asn Thr Leu Asp Lys Leu Ile Arg Asn Ala Ala Ser
                885                 890                 895

Leu Lys Val Thr Ala Ser Ala Pro Ser Gly Met Ser Gln Pro Leu Leu
            900                 905                 910

Asp Arg Thr Val Pro Asp Tyr Thr Thr Phe Thr Thr Val Gly Asp Trp
        915                 920                 925

Leu Asp Ala Ile Lys Met Gly Arg Tyr Lys Glu Ser Phe Val Gly Ala
    930                 935                 940

Gly Phe Ala Ser Phe Asp Leu Val Ala Gln Met Thr Ala Glu Asp Leu
945                 950                 955                 960

Leu Arg Ile Gly Val Thr Leu Ala Gly His Gln Lys Lys Ile Leu Cys
                965                 970                 975

Ser Ile Gln Asp Met Arg Leu Gln Met Asn Gln Thr Leu Pro Val Gln
            980                 985                 990

Val

<210>  23
<211>  993
<212>  PRT
<213>  rat

<400>  23

Met Ala Gly Ala Arg Pro Pro Pro Gly Leu Leu Pro Leu Leu Ala Pro
1               5                   10                  15

Leu Leu Leu Pro Leu Leu Leu Pro Ala Gly Cys Arg Ala Leu Glu Glu
            20                  25                  30

Thr Leu Met Asp Thr Lys Trp Val Thr Ser Glu Leu Ala Trp Thr Ser
        35                  40                  45

His Pro Glu Ser Gly Trp Glu Glu Val Ser Gly Tyr Asp Glu Ala Met
    50                  55                  60

Asn Pro Ile Arg Thr Tyr Gln Val Cys Asn Val Arg Glu Ser Ser Gln
65                  70                  75                  80

Asn Asn Trp Leu Arg Thr Gly Phe Ile Trp Arg Arg Glu Val Gln Arg
                85                  90                  95

Val Tyr Val Glu Leu Lys Phe Thr Val Arg Asp Cys Asn Ser Ile Pro
            100                 105                 110

Asn Ile Pro Gly Ser Cys Lys Glu Thr Phe Asn Leu Phe Tyr Tyr Glu
        115                 120                 125

Ala Asp Ser Asp Val Ala Ser Ala Ser Ser Pro Phe Trp Met Glu Asn
    130                 135                 140

Pro Tyr Val Lys Val Asp Thr Ile Ala Pro Asp Glu Ser Phe Ser Arg
145                 150                 155                 160

Leu Asp Ala Gly Arg Val Asn Thr Lys Val Arg Ser Phe Gly Pro Leu
                165                 170                 175

Ser Lys Ala Gly Phe Tyr Leu Ala Phe Gln Asp Gln Gly Ala Cys Met
            180                 185                 190

Ser Leu Ile Ser Val Arg Ala Phe Tyr Lys Lys Cys Ala Ser Thr Thr
        195                 200                 205

Ala Gly Phe Ala Leu Phe Pro Glu Thr Leu Thr Gly Ala Glu Pro Thr
    210                 215                 220

Ser Leu Val Ile Ala Pro Gly Thr Cys Ile Ala Asn Ala Val Glu Val
225                 230                 235                 240

Ser Val Pro Leu Lys Leu Tyr Cys Asn Gly Asp Gly Glu Trp Met Val
                245                 250                 255

Pro Val Gly Ala Cys Thr Cys Ala Thr Gly His Glu Pro Ala Ala Lys
            260                 265                 270

Glu Thr Gln Cys Arg Ala Cys Pro Pro Gly Ser Tyr Lys Ala Lys Gln
        275                 280                 285

Gly Glu Gly Pro Cys Leu Pro Cys Pro Pro Asn Ser Arg Thr Thr Ser
    290                 295                 300

Pro Ala Ala Ser Ile Cys Thr Cys His Asn Asn Phe Tyr Arg Ala Asp
305                 310                 315                 320

Ser Asp Thr Ala Asp Ser Ala Cys Thr Thr Val Pro Ser Pro Pro Arg
                325                 330                 335

Gly Val Ile Ser Asn Val Asn Glu Thr Ser Leu Ile Leu Glu Trp Ser
            340                 345                 350

Glu Pro Arg Asp Leu Gly Gly Arg Asp Asp Leu Leu Tyr Asn Val Ile
        355                 360                 365

Cys Lys Lys Cys Arg Gly Ser Ser Gly Ala Gly Gly Pro Ala Thr Cys
    370                 375                 380

Ser Arg Cys Asp Asp Asn Val Glu Phe Glu Pro Arg Gln Leu Gly Leu
385                 390                 395                 400

Thr Glu Arg Arg Val His Ile Ser His Leu Leu Ala His Thr Arg Tyr
                405                 410                 415

Thr Phe Glu Val Gln Ala Val Asn Gly Val Ser Gly Lys Ser Pro Leu
            420                 425                 430

Pro Pro Arg Tyr Ala Ala Val Asn Ile Thr Thr Asn Gln Ala Ala Pro
        435                 440                 445

Ser Glu Val Pro Thr Leu His Leu His Ser Ser Ser Gly Ser Ser Leu
    450                 455                 460

Thr Leu Ser Trp Ala Pro Pro Glu Arg Pro Asn Gly Val Ile Leu Asp
465                 470                 475                 480

Tyr Glu Met Lys Tyr Phe Glu Lys Ser Lys Gly Ile Ala Ser Thr Val
                485                 490                 495

Thr Ser Gln Lys Asn Ser Val Gln Leu Asp Gly Leu Gln Pro Asp Ala
            500                 505                 510

Arg Tyr Val Val Gln Val Arg Ala Arg Thr Val Ala Gly Tyr Gly Gln
        515                 520                 525

Tyr Ser Arg Pro Ala Glu Phe Glu Thr Thr Ser Glu Arg Gly Ser Gly
    530                 535                 540

Ala Gln Gln Leu Gln Glu Gln Leu Pro Leu Ile Val Gly Ser Thr Val
545                 550                 555                 560

Ala Gly Phe Val Phe Met Val Val Val Val Val Ile Ala Leu Val Cys
                565                 570                 575

Leu Arg Lys Gln Arg Gln Gly Pro Asp Ala Glu Tyr Thr Glu Lys Leu
            580                 585                 590

Gln Gln Tyr Val Ala Pro Arg Met Lys Val Tyr Ile Asp Pro Phe Thr
        595                 600                 605

Tyr Glu Asp Pro Asn Glu Ala Val Arg Glu Phe Ala Lys Glu Ile Asp
    610                 615                 620

Val Ser Cys Val Lys Ile Glu Glu Val Ile Gly Ala Gly Glu Phe Gly
625                 630                 635                 640

Glu Val Cys Arg Gly Arg Leu Lys Leu Pro Gly Arg Arg Glu Val Phe
                645                 650                 655

Val Ala Ile Lys Thr Leu Lys Val Gly Tyr Thr Glu Arg Gln Arg Arg
            660                 665                 670

Asp Phe Leu Ser Glu Ala Ser Ile Met Gly Gln Phe Asp His Pro Asn
        675                 680                 685

Ile Ile Arg Leu Glu Gly Val Val Thr Lys Ser Arg Pro Val Met Ile
    690                 695                 700

Leu Thr Glu Phe Met Glu Asn Cys Ala Leu Asp Ser Phe Leu Arg Leu
705                 710                 715                 720

Asn Asp Gly Gln Phe Thr Val Ile Gln Leu Val Gly Met Leu Arg Gly
                725                 730                 735

Ile Ala Ala Gly Met Lys Tyr Leu Ser Glu Met Asn Tyr Val His Arg
            740                 745                 750

Asp Leu Ala Ala Arg Asn Ile Leu Val Asn Ser Asn Leu Val Cys Lys
        755                 760                 765

Val Ser Asp Phe Gly Leu Ser Arg Phe Leu Glu Asp Asp Pro Ser Asp
    770                 775                 780

Pro Thr Tyr Thr Ser Ser Leu Gly Gly Lys Ile Pro Ile Arg Trp Thr
785                 790                 795                 800

Ala Pro Glu Ala Ile Ala Tyr Arg Lys Phe Thr Ser Ala Ser Asp Val
                805                 810                 815

Trp Ser Tyr Gly Ile Val Met Trp Glu Val Met Ser Tyr Gly Glu Arg
            820                 825                 830

Pro Tyr Trp Asp Met Ser Asn Gln Asp Val Ile Asn Ala Val Glu Gln
        835                 840                 845

Asp Tyr Arg Leu Pro Pro Pro Met Asp Cys Pro Thr Ala Leu His Gln
    850                 855                 860

Leu Met Leu Asp Cys Trp Val Arg Asp Arg Asn Leu Arg Pro Lys Phe
865                 870                 875                 880

Ser Gln Ile Val Asn Thr Leu Asp Lys Leu Ile Arg Asn Ala Ala Ser
                885                 890                 895

Leu Lys Val Ile Ala Ser Ala Pro Ser Gly Met Ser Gln Pro Leu Leu
            900                 905                 910

Asp Arg Thr Val Pro Asp Tyr Thr Thr Phe Thr Thr Val Gly Asp Trp
        915                 920                 925

Leu Asp Ala Ile Lys Met Gly Arg Tyr Lys Glu Ser Phe Val Gly Ala
    930                 935                 940

Gly Phe Ala Ser Phe Asp Leu Val Ala Gln Met Thr Ala Glu Asp Leu
945                 950                 955                 960

Leu Arg Ile Gly Val Thr Leu Ala Gly His Gln Lys Lys Ile Leu Ser
                965                 970                 975

Ser Ile Gln Asp Met Arg Leu Gln Met Asn Gln Thr Leu Pro Val Gln
            980                 985                 990

Val

<210>   24
<211>   987
<212>   PRT
<213>   human

<400>   24

<210>  25
<211>  987
<212>  PRT
<213>  mouse

<400>  25

Met Glu Leu Arg Ala Leu Leu Cys Trp Ala Ser Leu Ala Thr Ala Leu
1               5                   10                  15

Glu Glu Thr Leu Leu Asn Thr Lys Leu Glu Thr Ala Asp Leu Lys Trp
            20                  25                  30

Val Thr Tyr Pro Gln Ala Glu Gly Gln Trp Glu Glu Leu Ser Gly Leu
        35                  40                  45

Asp Glu Glu Gln His Ser Val Arg Thr Tyr Glu Val Cys Asp Met Lys
    50                  55                  60

Arg Pro Gly Gly Gln Ala His Trp Leu Arg Thr Gly Trp Val Pro Arg
65                  70                  75                  80

Arg Gly Ala Val His Val Tyr Ala Thr Ile Arg Phe Thr Met Met Glu
                85                  90                  95

Cys Leu Ser Leu Pro Arg Ala Ser Arg Ser Cys Lys Glu Thr Phe Thr
            100                 105                 110

Val Phe Tyr Tyr Glu Ser Glu Ala Asp Thr Ala Thr Ala His Thr Pro
        115                 120                 125

Ala Trp Met Glu Asn Pro Tyr Ile Lys Val Asp Thr Val Ala Ala Glu
    130                 135                 140

His Leu Thr Arg Lys Arg Pro Gly Ala Glu Ala Thr Gly Lys Val Asn
145                 150                 155                 160

Ile Lys Thr Leu Arg Leu Gly Pro Leu Ser Lys Ala Gly Phe Tyr Leu
                165                 170                 175

Ala Phe Gln Asp Gln Gly Ala Cys Met Ala Leu Leu Ser Leu His Leu
            180                 185                 190

Phe Tyr Lys Lys Cys Ser Trp Leu Ile Thr Asn Leu Thr Tyr Phe Pro
        195                 200                 205

Glu Thr Val Pro Arg Glu Leu Val Val Pro Val Ala Gly Ser Cys Val
    210                 215                 220

Ala Asn Ala Val Pro Thr Ala Asn Pro Ser Pro Ser Leu Tyr Cys Arg
225                 230                 235                 240

Glu Asp Gly Gln Trp Ala Glu Gln Gln Val Thr Gly Cys Ser Cys Ala
                245                 250                 255

Pro Gly Tyr Glu Ala Ala Glu Ser Asn Lys Val Cys Arg Ala Cys Gly
            260                 265                 270

Gln Gly Thr Phe Lys Pro Gln Ile Gly Asp Glu Ser Cys Leu Pro Cys
        275                 280                 285

Pro Ala Asn Ser His Ser Asn Asn Ile Gly Ser Pro Val Cys Leu Cys
    290                 295                 300

Arg Ile Gly Tyr Tyr Arg Ala Arg Ser Asp Pro Arg Ser Ser Pro Cys
305                 310                 315                 320

Thr Thr Pro Pro Ser Ala Pro Arg Ser Val Val His His Leu Asn Gly
                325                 330                 335

Ser Thr Leu Arg Leu Glu Trp Ser Ala Pro Leu Glu Ser Gly Gly Arg
            340                 345                 350

Glu Asp Leu Thr Tyr Ala Val Arg Cys Arg Glu Cys Arg Pro Gly Gly
        355                 360                 365

Ser Cys Leu Pro Cys Gly Gly Asp Met Thr Phe Asp Pro Gly Pro Arg
    370                 375                 380

Asp Leu Val Glu Pro Trp Val Ala Ile Arg Gly Leu Arg Pro Asp Val
385                 390                 395                 400

Thr Tyr Thr Phe Glu Val Ala Ala Leu Asn Gly Val Ser Thr Leu Ala
                405                 410                 415

Thr Gly Pro Pro Pro Phe Glu Pro Val Asn Val Thr Thr Asp Arg Glu
            420                 425                 430

Val Pro Pro Ala Val Ser Asp Ile Arg Val Thr Arg Ser Ser Pro Ser
        435                 440                 445

Ser Leu Ile Leu Ser Trp Ala Leu Pro Arg Ala Pro Ser Gly Ala Val
    450                 455                 460

Leu Asp Tyr Glu Val Lys Tyr His Glu Lys Gly Ala Glu Gly Pro Ser
465                 470                 475                 480

Ser Val Arg Phe Leu Lys Thr Ser Glu Asn Arg Ala Glu Leu Arg Gly
                485                 490                 495

Leu Lys Arg Gly Ala Ser Tyr Leu Val Pro Val Arg Ala Arg Ser Glu
            500                 505                 510

Ala Gly Tyr Gly Pro Phe Gly Gln Glu His His Ser Gln Thr Gln Leu
        515                 520                 525

Asp Glu Ser Glu Ser Trp Arg Glu Gln Leu Ala Leu Ile Ala Gly Thr
    530                 535                 540

Ala Val Val Gly Val Val Leu Val Leu Val Val Val Ile Ile Ala Val
545                 550                 555                 560

Leu Cys Phe Arg Lys Gln Ser Tyr Gly Arg Glu Val Glu Tyr Ser Asp
                565                 570                 575

Lys His Gly Gln Tyr Leu Ile Gly His Gly Thr Lys Val Tyr Ile Asp
            580                 585                 590

Pro Phe Thr Tyr Glu Asp Pro Asn Glu Ala Val Arg Glu Phe Ala Lys
        595                 600                 605

Glu Ile Asp Val Ser Tyr Val Lys Ile Glu Glu Val Ile Gly Ala Gly
    610                 615                 620

Glu Phe Gly Glu Val Cys Arg Gly Arg Leu Lys Ala Pro Gly Lys Lys
625                 630                 635                 640

Glu Ser Cys Val Ala Ile Lys Thr Leu Lys Gly Gly Tyr Thr Glu Arg
                645                 650                 655

Gln Arg Arg Glu Phe Leu Ser Glu Ala Ser Ile Met Gly Gln Phe Glu
            660                 665                 670

His Pro Asn Ile Ile Arg Leu Glu Gly Val Val Thr Asn Ser Val Pro
        675                 680                 685

Val Met Ile Leu Thr Glu Phe Met Glu Asn Gly Ala Leu Asp Ser Phe
    690                 695                 700

Leu Arg Leu Asn Asp Gly Gln Phe Thr Val Ile Gln Leu Val Gly Met
705                 710                 715                 720

Leu Arg Gly Ile Ala Ser Gly Met Arg Tyr Leu Ala Glu Met Ser Tyr
                725                 730                 735

Val His Arg Asp Leu Ala Ala Arg Asn Ile Leu Val Asn Ser Asn Leu
            740                 745                 750

Val Cys Lys Val Ser Asp Phe Gly Leu Ser Arg Phe Leu Glu Glu Asn
        755                 760                 765

Ser Ser Asp Pro Thr Tyr Thr Ser Ser Leu Gly Gly Lys Ile Pro Ile
    770                 775                 780

Arg Trp Thr Ala Pro Glu Ala Ile Ala Phe Arg Lys Phe Thr Ser Ala
785                 790                 795                 800

Ser Asp Ala Trp Ser Tyr Gly Ile Val Met Trp Glu Val Met Ser Phe
                805                 810                 815

Gly Glu Arg Pro Tyr Trp Asp Met Ser Asn Gln Asp Val Ile Asn Ala
            820                 825                 830

Ile Glu Gln Asp Tyr Arg Leu Pro Pro Pro Pro Asp Cys Pro Thr Ser
        835                 840                 845

Leu His Gln Leu Met Leu Asp Cys Trp Gln Lys Asp Arg Asn Ala Arg
    850                 855                 860

Pro Arg Phe Pro Gln Val Val Ser Ala Leu Asp Lys Met Ile Arg Asn
865                 870                 875                 880

Pro Ala Ser Leu Lys Ile Val Ala Arg Glu Asn Gly Gly Ala Ser His
                885                 890                 895

Pro Leu Leu Asp Gln Arg Gln Pro His Tyr Ser Ala Phe Gly Ser Val
            900                 905                 910

Gly Glu Trp Leu Arg Ala Ile Lys Met Gly Arg Tyr Glu Glu Ser Phe
        915                 920                 925

Ala Ala Ala Gly Phe Gly Ser Phe Glu Val Val Ser Gln Ile Ser Ala
    930                 935                 940

Glu Asp Leu Leu Arg Ile Gly Val Thr Leu Ala Gly His Gln Lys Lys
945                 950                 955                 960

Ile Leu Ala Ser Val Gln His Met Lys Ser Gln Ala Lys Pro Gly Ala
                965                 970                 975

Pro Gly Gly Thr Gly Gly Pro Ala Gln Gln Phe
            980                 985

<210> 26
<211> 989
<212> PRT
<213> rat

<400> 26
   Met Glu Leu Arg Ala Leu Leu Cys Trp Ala Ser Leu Ala Thr Ala Leu
   1 5 10 15
   Glu Glu Thr Leu Leu Asn Thr Lys Leu Glu Thr Ala Asp Leu Lys Trp
   20 25 30
   Val Thr Tyr Pro Gln Ala Glu Gly Gln Trp Glu Glu Leu Ser Gly Leu
   35 40 45
   Asp Glu Glu Gln His Ser Val Arg Thr Tyr Glu Val Cys Asp Met Lys
   50 55 60
   Arg Pro Gly Gly Gln Ala His Trp Leu Arg Thr Gly Trp Val Pro Arg
   65 70 75 80
   Arg Gly Ala Val His Val Tyr Ala Thr Ile Arg Phe Thr Met Met Glu
   85 90 95
   Cys Leu Ser Leu Pro Arg Ala Ser Arg Ser Cys Lys Glu Thr Phe Thr
   100 105 110
   Val Phe Tyr Tyr Glu Ser Glu Ala Asp Thr Ala Thr Ala His Thr Pro
   115 120 125
   Ala Trp Met Glu Asn Pro Tyr Ile Lys Val Asp Thr Val Ala Ala Glu
   130 135 140
   His Leu Thr Arg Lys Arg Pro Gly Ala Glu Ala Thr Gly Lys Val Asn
   145 150 155 160
   Ile Lys Thr Leu Arg Leu Gly Pro Leu Ser Lys Ala Gly Phe Tyr Leu
   165 170 175
   Ala Phe Gln Asp Gln Gly Ala Cys Met Ala Leu Leu Ser Leu His Leu
   180 185 190
   Phe Tyr Lys Lys Cys Ser Arg Leu Val Thr Asn Leu Thr Tyr Phe Pro
   195 200 205
   Glu Thr Val Pro Arg Glu Leu Val Val Pro Val Ala Gly Ser Cys Val
   210 215 220
   Ala Asn Ala Val Pro Thr Ala Ser Pro Ser Pro Ser Leu Tyr Cys Arg
   225                 230                 235                 240
   Glu Asp Gly Gln Trp Ala Glu Gln Gln Val Thr Gly Cys Ser Cys Ala
                   245                 250                 255
   Pro Gly Tyr Glu Ala Ala Glu Gly Asn Lys Val Cys Arg Ala Cys Gly
               260                 265                 270
   Gln Gly Thr Phe Lys Pro Gln Ile Gly Glu Glu Pro Cys Leu Pro Cys
           275                 280                 285
   Pro Ala Asn Ser His Ser Asn Asn Ile Gly Ser Pro Val Cys Leu Cys
       290                 295                 300
   Arg Ile Gly Tyr Tyr Arg Ala Arg Ser Asp Pro Arg Ser Ser Pro Cys
   305                 310                 315                 320
   Thr Thr Pro Pro Ser Ala Pro Arg Ser Val Val His His Leu Asn Gly
                   325                 330                 335
   Ser Ser Leu Arg Leu Glu Trp Ser Ala Pro Leu Glu Ser Gly Gly Arg
               340                 345                 350
   Glu Asp Leu Thr Tyr Ala Val Arg Cys Arg Glu Cys Arg Pro Gly Gly
           355                 360                 365
   Ser Cys Leu Pro Cys Gly Gly Asp Leu Thr Phe Asp Pro Gly Pro Arg
       370                 375                 380
   Asp Leu Val Glu Pro Trp Val Ala Ile Arg Gly Leu Arg Pro Asp Val
   385                 390                 395                 400
   Thr Tyr Thr Phe Glu Val Ala Ala Leu Asn Gly Val Ser Thr Leu Ala
                   405                 410                 415
   Thr Gly Pro Pro Pro Phe Glu Pro Val Asn Val Thr Thr Asp Arg Glu
               420                 425                 430
   Val Pro Pro Ala Val Ser Asp Ile Arg Val Thr Arg Ser Ser Pro Ser
           435                 440                 445
   Ser Leu Ile Leu Ser Trp Ala Ile Pro Arg Ala Pro Ser Gly Ala Val
       450                 455                 460
   Leu Asp Tyr Glu Val Lys Tyr His Glu Lys Gly Ala Glu Gly Pro Ser
   465                 470                 475                 480
   Ser Val Arg Phe Leu Lys Thr Ser Glu Asn Arg Ala Glu Leu Arg Gly
                   485                 490                 495
   Leu Lys Gln Gly Ala Ser Tyr Leu Val Gln Val Arg Ala Arg Ser Glu
               500                 505                 510
   Ala Gly Tyr Gly Pro Phe Gly Gln Glu His His Ser Gln Thr Gln Leu
           515                 520                 525
   Asp Glu Ser Glu Ser Trp Arg Glu Gln Leu Ala Leu Ile Ala Gly Thr
       530                 535                 540
   Ala Val Val Gly Val Val Leu Val Leu Val Val Ile Ile Ile Ala Val
   545                 550                 555                 560
   Leu Cys Leu Arg Lys Gln Ser Ser Gly Arg Glu Val Glu Tyr Ser Asp
                   565                 570                 575
   Lys His Gly Gln Tyr Leu Ile Gly His Gly Thr Lys Val Tyr Ile Asp
               580                 585                 590
   Pro Phe Thr Tyr Glu Asp Pro Asn Glu Ala Val Arg Glu Phe Ala Lys
           595                 600                 605
   Glu Ile Asp Val Ser Tyr Val Lys Ile Glu Glu Val Ile Gly Ala Gly
       610                 615                 620
   Glu Phe Gly Glu Val Cys Arg Gly Arg Leu Lys Ala Pro Gly Lys Lys
   625                 630                 635                 640
   Glu Ser Cys Val Ala Ile Lys Thr Leu Lys Gly Gly Tyr Thr Glu Arg
                   645                 650                 655
   Gln Arg Arg Glu Phe Leu Ser Glu Ala Ser Ile Met Gly Gln Phe Glu
               660                 665                 670
   His Pro Asn Ile Ile Arg Leu Glu Gly Val Val Thr Asn Ser Val Pro
           675                 680                 685
   Val Met Ile Leu Thr Glu Phe Met Glu Asn Gly Ala Leu Asp Ser Phe
       690                 695                 700
   Leu Arg Asn Leu Pro Glu Arg Gly Trp Gly His Gly Ser Val Leu Val
   705                 710                 715                 720
   Gly Met Leu Arg Gly Ile Ala Ser Gly Met Arg Tyr Leu Ala Glu Met
                   725                 730                 735
   Ser Tyr Val His Arg Asp Leu Ala Ala Arg Asn Ile Leu Val Asn Ser
               740                 745                 750
   Asn Leu Val Cys Lys Val Ser Asp Phe Gly Leu Ser Arg Phe Leu Glu
           755                 760                 765
   Glu Asn Ser Ser Asp Pro Thr Tyr Thr Ser Ser Leu Gly Gly Lys Ile
   770 775 780
   Pro Ile Arg Trp Thr Ala Pro Glu Ala Ile Ala Phe Arg Lys Phe Thr
   785 790 795 800
   Ser Ala Ser Asp Ala Trp Ser Tyr Gly Ile Val Met Trp Glu Val Met
   805 810 815
   Ser Phe Gly Glu Arg Pro Tyr Trp Asp Met Ser Asn Gln Asp Val Ile
   820 825 830
   Asn Ala Ile Glu Gln Asp Tyr Arg Leu Pro Pro Pro Pro Asp Cys Pro
   835 840 845
   Thr Ser Leu His Gln Leu Met Leu Asp Cys Trp Gln Lys Asp Arg Asn
   850 855 860
   Ala Arg Pro Arg Phe Pro Gln Val Val Ser Ala Leu Asp Lys Met Ile
   865 870 875 880
   Arg Asn Pro Ala Ser Leu Lys Ile Val Ala Arg Glu Asn Gly Gly Ala
   885 890 895
   Ser His Pro Leu Leu Asp Gln Arg Gln Pro His Tyr Ser Ala Phe Gly
   900 905 910
   Ser Val Gly Glu Trp Leu Arg Ala Ile Lys Met Gly Arg Tyr Glu Glu
   915 920 925
   Ser Phe Ala Ala Ala Gly Phe Gly Ser Phe Glu Val Val Ser Gln Ile
   930 935 940
   Ser Ala Glu Asp Leu Leu Arg Ile Gly Val Thr Leu Ala Gly His Gln
   945 950 955 960
   Lys Lys Ile Leu Ala Ser Val Gln His Met Lys Ser Gln Ala Lys Pro
   965 970 975
   Gly Ala Pro Gly Gly Thr Gly Gly Pro Thr Gln Gln Phe
   980 985

<210>  27
<211>  1006
<212>  PRT
<213>  human

<400>  27
   Met Val Cys Ser Leu Trp Val Leu Leu Leu Val Ser Ser Val Leu Ala
   1               5                   10                  15
   Leu Glu Glu Val Leu Leu Asp Thr Thr Gly Glu Thr Ser Glu Ile Gly
               20                  25                  30
   Trp Leu Thr Tyr Pro Pro Gly Gly Trp Asp Glu Val Ser Val Leu Asp
           35                  40                  45
   Asp Gln Arg Arg Leu Thr Arg Thr Phe Glu Ala Cys His Val Ala Gly
       50                  55                  60
   Ala Pro Pro Gly Thr Gly Gln Asp Asn Trp Leu Gln Thr His Phe Val
   65                  70                  75                  80
   Glu Arg Arg Gly Ala Gln Arg Ala His Ile Arg Leu His Phe Ser Val
                   85                  90                  95
   Arg Ala Cys Ser Ser Leu Gly Val Ser Gly Gly Thr Cys Arg Glu Thr
               100                 105                 110
   Phe Thr Leu Tyr Tyr Arg Gln Ala Glu Glu Pro Asp Ser Pro Asp Ser
           115                 120                 125
   Val Ser Ser Trp His Leu Lys Arg Trp Thr Lys Val Asp Thr Ile Ala
       130                 135                 140
   Ala Asp Glu Ser Phe Pro Ser Ser Ser Ser Ser Ser Ser Ser Ser Ser
   145                 150                 155                 160
   Ser Ala Ala Trp Ala Val Gly Pro His Gly Ala Gly Gln Arg Ala Gly
                   165                 170                 175
   Leu Gln Leu Asn Val Lys Glu Arg Ser Phe Gly Pro Leu Thr Gln Arg
               180                 185                 190
   Gly Phe Tyr Val Ala Phe Gln Asp Thr Gly Ala Cys Leu Ala Leu Val
           195                 200                 205
   Ala Val Arg Leu Phe Ser Tyr Thr Cys Pro Ala Val Leu Arg Ser Phe
       210                 215                 220
   Ala Ser Phe Pro Glu Thr Gln Ala Ser Gly Ala Gly Gly Ala Ser Leu
   225                 230                 235                 240
   Val Ala Ala Val Gly Thr Cys Val Ala His Ala Glu Pro Glu Glu Asp
                   245                 250                 255
   Gly Val Gly Gly Gln Ala Gly Gly Ser Pro Pro Arg Leu His Cys Asn
               260                 265                 270
   Gly Glu Gly Lys Trp Met Val Ala Val Gly Gly Cys Arg Cys Gln Pro
           275                 280                 285
   Gly Tyr Gln Pro Ala Arg Gly Asp Lys Ala Cys Gln Ala Cys Pro Arg
       290                 295                 300
   Gly Leu Tyr Lys Ser Ser Ala Gly Asn Ala Pro Cys Ser Pro Cys Pro
   305                 310                 315                 320
   Ala Arg Ser His Ala Pro Asn Pro Ala Ala Pro Val Cys Pro Cys Leu
                   325                 330                 335
   Glu Gly Phe Tyr Arg Ala Ser Ser Asp Pro Pro Glu Ala Pro Cys Thr
               340                 345                 350
   Gly Pro Pro Ser Ala Pro Gln Glu Leu Trp Phe Glu Val Gln Gly Ser
           355                 360                 365
   Ala Leu Met Leu His Trp Arg Leu Pro Arg Glu Leu Gly Gly Arg Gly
       370                 375                 380
   Asp Leu Leu Phe Asn Val Val Cys Lys Glu Cys Glu Gly Arg Gln Glu
   385                 390                 395                 400
   Pro Ala Ser Gly Gly Gly Gly Thr Cys His Arg Cys Arg Asp Glu Val
                   405                 410                 415
   His Phe Asp Pro Arg Gln Arg Gly Leu Thr Glu Ser Arg Val Leu Val
               420                 425                 430
   Gly Gly Leu Arg Ala His Val Pro Tyr Ile Leu Glu Val Gln Ala Val
           435                 440                 445
   Asn Gly Val Ser Glu Leu Ser Pro Asp Pro Pro Gln Ala Ala Ala Ile
       450                 455                 460
   Asn Val Ser Thr Ser His Glu Val Pro Ser Ala Val Pro Val Val His
   465                 470                 475                 480
   Gln Val Ser Arg Ala Ser Asn Ser Ile Thr Val Ser Trp Pro Gln Pro
                   485                 490                 495
   Asp Gln Thr Asn Gly Asn Ile Leu Asp Tyr Gln Leu Arg Tyr Tyr Asp
               500                 505                 510
   Gln Ala Glu Asp Glu Ser His Ser Phe Thr Leu Thr Ser Glu Thr Asn
           515                 520                 525
   Thr Ala Thr Val Thr Gln Leu Ser Pro Gly His Ile Tyr Gly Phe Gln
       530                 535                 540
   Val Arg Ala Arg Thr Ala Ala Gly His Gly Pro Tyr Gly Gly Lys Val
   545                 550                 555                 560
   Tyr Phe Gln Thr Leu Pro Gln Gly Glu Leu Ser Ser Gln Leu Pro Glu
                   565                 570                 575
   Arg Leu Ser Leu Val Ile Gly Ser Ile Leu Gly Ala Leu Ala Phe Leu
               580                 585                 590
   Leu Leu Ala Ala Ile Thr Val Leu Ala Val Val Phe Gln Arg Lys Arg
           595                 600                 605
   Arg Gly Thr Gly Tyr Thr Glu Gln Leu Gln Gln Tyr Ser Ser Pro Gly
       610                 615                 620
   Leu Gly Val Lys Tyr Tyr Ile Asp Pro Ser Thr Tyr Glu Asp Pro Cys
   625                 630                 635                 640
   Gln Ala Ile Arg Glu Leu Ala Arg Glu Val Asp Pro Ala Tyr Ile Lys
                   645                 650                 655
   Ile Glu Glu Val Ile Gly Thr Gly Ser Phe Gly Glu Val Arg Gln Gly
               660                 665                 670
   Arg Leu Gln Pro Arg Gly Arg Arg Glu Gln Thr Val Ala Ile Gln Ala
           675                 680                 685
   Leu Trp Ala Gly Gly Ala Glu Ser Leu Gln Met Thr Phe Leu Gly Arg
       690                 695                 700
   Ala Ala Val Leu Gly Gln Phe Gln His Pro Asn Ile Leu Arg Leu Glu
   705                 710                 715                 720
   Gly Val Val Thr Lys Ser Arg Pro Leu Met Val Leu Thr Glu Phe Met
                   725                 730                 735
   Glu Leu Gly Pro Leu Asp Ser Phe Leu Arg Gln Arg Glu Gly Gln Phe
               740                 745                 750
   Ser Ser Leu Gln Leu Val Ala Met Gln Arg Gly Val Ala Ala Ala Met
           755                 760                 765
   Gln Tyr Leu Ser Ser Phe Ala Phe Val His Arg Ser Leu Ser Ala His
       770                 775                 780
   Ser Val Leu Val Asn Ser His Leu Val Cys Lys Val Ala Arg Leu Gly
   785                 790                 795                 800
   His Ser Pro Gln Gly Pro Ser Cys Leu Leu Arg Trp Ala Ala Pro Glu
                   805                 810                 815
   Val Ile Ala His Gly Lys His Thr Thr Ser Ser Asp Val Trp Ser Phe
               820                 825                 830
   Gly Ile Leu Met Trp Glu Val Met Ser Tyr Gly Glu Arg Pro Tyr Trp
           835                     840                     845
   Asp Met Ser Glu Gln Glu Val Leu Asn Ala Ile Glu Gln Glu Phe Arg
       850                     855                     860
   Leu Pro Pro Pro Pro Gly Cys Pro Pro Gly Leu His Leu Leu Met Leu
   865                     870                     875                     880
   Asp Thr Trp Gln Lys Asp Arg Ala Arg Arg Pro His Phe Asp Gln Leu
                   885                     890                     895
   Val Ala Ala Phe Asp Lys Met Ile Arg Lys Pro Asp Thr Leu Gln Ala
               900                     905                     910
   Gly Gly Asp Pro Gly Glu Arg Pro Ser Gln Ala Leu Leu Thr Pro Val
           915                     920                     925
   Ala Leu Asp Phe Pro Cys Leu Asp Ser Pro Gln Ala Trp Leu Ser Ala
       930                     935                     940
   Ile Gly Leu Glu Cys Tyr Gln Asp Asn Phe Ser Lys Phe Gly Leu Cys
   945                     950                     955                     960
   Thr Phe Ser Asp Val Ala Gln Leu Ser Leu Glu Asp Leu Pro Ala Leu
                   965                     970                     975
   Gly Ile Thr Leu Ala Gly His Gln Lys Lys Leu Leu His His Ile Gln
               980                     985                     990
   Leu Leu Gln Gln His Leu Arg Gln Gln Gly Ser Val Glu Val
           995                     1000                    1005

<210>  28
<211>  1014
<212>  PRT
<213>  mouse

<400>  28

Met Ala Thr Glu Gly Thr Thr Gly Ser Gly Ser Arg Val Val Ala Gly
1               5                   10                  15

Met Val Cys Ser Leu Trp Leu Leu Val Leu Gly Ser Ser Val Leu Ala
            20                  25                  30

Leu Glu Glu Val Leu Leu Asp Thr Thr Gly Glu Thr Ser Glu Ile Gly
        35                  40                  45

Trp Leu Thr Tyr Pro Pro Gly Gly Trp Asp Glu Val Ser Val Leu Asp
    50                  55                  60

Asp Gln Arg Arg Leu Thr Arg Thr Phe Glu Ala Cys His Val Ala Gly

65                  70                  75                  80

Leu Pro Pro Gly Ser Gly Gln Asp Asn Trp Leu Gln Thr His Phe Val
                85                  90                  95

Glu Arg Arg Gly Ala Gln Arg Ala His Ile Arg Leu His Phe Ser Val
            100                 105                 110

Arg Ala Cys Ser Ser Leu Gly Val Ser Gly Gly Thr Cys Arg Glu Thr
        115                 120                 125

Phe Thr Leu Tyr Tyr Arg Gln Ala Asp Glu Pro Asp Gly Pro Asp Ser
    130                 135                 140

Ile Ala Ala Trp His Leu Lys Arg Trp Thr Lys Val Asp Thr Ile Ala
145                 150                 155                 160

Ala Asp Glu Ser Phe Pro Ala Ser Ser Ser Ser Ser Ser Trp Ala Val
                165                 170                 175

Gly Pro His Arg Thr Gly Gln Arg Val Gly Leu Gln Leu Asn Val Lys
            180                 185                 190

Glu Arg Ser Phe Gly Pro Leu Thr Gln Arg Gly Phe Tyr Val Ala Phe
        195                 200                 205

Gln Asp Thr Gly Ala Cys Leu Ala Leu Val Ala Val Lys Leu Phe Ser
    210                 215                 220

Tyr Thr Cys Pro Ser Val Leu Arg Ala Phe Ala Ser Phe Pro Glu Thr
225                 230                 235                 240

Gln Ala Ser Gly Ala Gly Gly Ala Ser Leu Val Ala Ala Val Gly Thr
                245                 250                 255

Cys Val Ala His Ala Glu Pro Glu Glu Asp Gly Val Gly Gly Gln Ala
            260                 265                 270

Gly Gly Ser Pro Pro Arg Leu His Cys Asn Gly Glu Gly Arg Trp Met
        275                 280                 285

Val Ala Val Gly Gly Cys Arg Cys Gln Pro Gly His Gln Pro Ala Arg
    290                 295                 300

Gly Asp Lys Leu Cys Gln Ala Cys Pro Glu Gly Ser Tyr Lys Ala Leu
305                 310                 315                 320

Ala Gly Asn Val Pro Cys Ser Pro Cys Pro Ala Arg Ser His Ser Pro
                325                 330                 335

Asp Pro Ala Ala Pro Val Cys Pro Cys Leu Gln Gly Phe Tyr Arg Ala

            340                 345                 350

Ser Ser Asp Pro Pro Glu Ala Pro Cys Thr Gly Pro Pro Ser Ala Pro
        355                 360                 365

Arg Glu Leu Trp Phe Glu Val Gln Gly Ser Ala Leu Met Leu His Trp
    370                 375                 380

Arg Leu Pro Gln Glu Leu Gly Gly Arg Gly Asp Leu Leu Phe Asn Val
385                 390                 395                 400

Val Cys Lys Glu Cys Gly Gly His Gly Glu Pro Ser Ser Gly Gly Met
                405                 410                 415

Cys Arg Arg Cys Arg Asp Glu Val His Phe Asp Pro Arg Gln Arg Gly
            420                 425                 430

Leu Thr Glu Ser Arg Val Leu Val Gly Gly Leu Arg Ala His Val Pro
        435                 440                 445

Tyr Ile Leu Glu Val Gln Ala Val Asn Gly Val Ser Glu Leu Ser Pro
    450                 455                 460

Asp Pro Pro Gln Ala Ala Ala Ile Asn Val Ser Thr Ser His Glu Val
465                 470                 475                 480

Pro Ser Ala Val Pro Val Met His Gln Val Ser Arg Ala Ala Asn Ser
                485                 490                 495

Ile Thr Val Ser Trp Pro Gln Pro Glu Gln Thr Asn Gly Asn Ile Leu
            500                 505                 510

Asp Tyr Gln Leu Arg Tyr Tyr Asp Gln Ala Glu Asp Glu Ser Tyr Ser
        515                 520                 525

Phe Thr Met Thr Ser Glu Thr Asn Thr Ala Thr Val Thr Arg Leu Ser
    530                 535                 540

Pro Gly His Ile Tyr Gly Phe Gln Val Arg Ala Arg Thr Ala Ala Gly
545                 550                 555                 560

His Gly Pro Tyr Gly Gly Lys Val Tyr Phe Gln Thr Leu Pro Gln Gly
                565                 570                 575

Glu Leu Ser Ser Gln Leu Pro Glu Lys Leu Ser Leu Val Ile Gly Ser
            580                 585                 590

Ile Leu Gly Ala Leu Ala Phe Leu Leu Leu Ala Ala Ile Thr Val Leu
        595                 600                 605

Ala Val Ile Phe Gln Arg Lys Arg Arg Gly Thr Gly Tyr Thr Glu Gln

610                 615                 620

Leu Gln Gln Tyr Ser Ser Pro Gly Leu Gly Val Lys Tyr Tyr Ile Asp
625                 630                 635                 640

Pro Ser Thr Tyr Asp Asp Pro Cys Gln Ala Ile Arg Glu Leu Ala Arg
                645                 650                 655

Glu Val Asp Pro Thr Tyr Ile Lys Ile Glu Glu Val Ile Gly Ala Gly
            660                 665                 670

Ser Phe Gly Glu Val Arg Arg Gly Arg Leu Gln Pro Arg Gly Arg Arg
        675                 680                 685

Glu Gln Ala Val Ala Ile Gln Ala Leu Trp Ala Gly Gly Ala Glu Ser
    690                 695                 700

Leu Lys Met Thr Phe Leu Gly Arg Ala Ala Leu Leu Gly Gln Phe Gln
705                 710                 715                 720

His Pro Asn Ile Leu Arg Leu Glu Gly Val Val Thr Lys Ser Arg Pro
                725                 730                 735

Val Met Val Leu Thr Glu Leu Met Glu Leu Gly Pro Leu Asp Ser Phe
            740                 745                 750

Leu Arg Gln Arg Glu Gly Gln Phe Ser Ser Leu Gln Leu Val Ala Met
        755                 760                 765

Gln Arg Gly Val Ala Ala Ala Met Gln Tyr Leu Ser Ser Phe Ala Phe
    770                 775                 780

Val His Arg Ala Leu Ser Ala Arg Ser Val Leu Val Asn Ser His Leu
785                 790                 795                 800

Val Cys Lys Val Ala Arg Leu Gly His Ser Pro Gln Gly Ser Ser Ser
                805                 810                 815

Leu Leu Arg Trp Ala Ala Pro Glu Val Ile Thr His Gly Lys Tyr Thr
            820                 825                 830

Thr Ser Ser Asp Val Trp Ser Phe Gly Ile Leu Met Trp Glu Val Met
        835                 840                 845

Ser Tyr Gly Glu Arg Pro Tyr Trp Asp Met Asn Glu Gln Glu Val Leu
    850                 855                 860

Asn Ala Ile Glu Gln Glu Phe Arg Leu Pro Pro Pro Pro Gly Cys Pro
865                 870                 875                 880

Pro Gly Leu His Leu Leu Met Leu Asp Thr Trp Gln Lys Asp Arg Ala

                885                 890                 895

Arg Arg Pro His Phe Asp Gln Leu Val Ala Ala Phe Asp Lys Met Ile
            900                 905                 910

Arg Lys Pro Asp Thr Leu Gln Ala Glu Gly Gly Ser Gly Asp Arg Pro
        915                 920                 925

Ser Gln Ala Leu Leu Asn Pro Val Ala Leu Asp Phe Pro Cys Leu Asp
    930                 935                 940

Ser Pro Gln Ala Trp Leu Ser Ala Ile Gly Leu Glu Cys Tyr Gln Asp
945                 950                 955                 960

Asn Phe Ser Lys Phe Gly Leu Ser Thr Phe Ser Asp Val Ala Gln Leu
                965                 970                 975

Ser Leu Glu Asp Leu Pro Gly Leu Gly Ile Thr Leu Ala Gly His Gln
            980                 985                 990

Lys Lys Leu Leu His Asn Ile Gln Leu Leu Gln Gln His Leu Arg Gln
        995                 1000                1005

Pro Gly Ser Val Glu Val
    1010

<210> 29
<211> 1013
<212> PRT
<213> rat

<400> 29

Met Ala Ser Glu Asn Thr Ala Gly Ser Gly Ser Arg Val Ala Gly Met
1               5                   10                  15

Val Tyr Ser Leu Trp Leu Leu Val Leu Gly Pro Ser Val Leu Ala Leu
            20                  25                  30

Glu Glu Val Leu Leu Asp Thr Thr Gly Glu Thr Ser Glu Ile Gly Trp
        35                  40                  45

Leu Thr Tyr Pro Pro Gly Gly Trp Asp Glu Val Ser Val Leu Asp Asp
    50                  55                  60

Gln Arg Arg Leu Thr Arg Thr Phe Glu Ala Cys His Val Ala Gly Leu
65                  70                  75                  80

Pro Pro Gly Ser Gly Gln Asp Asn Trp Leu Gln Thr His Phe Val Glu
                85                  90                  95

Arg Arg Gly Ala Gln Arg Ala His Ile Arg Leu His Phe Ser Val Arg
            100                 105                 110

Ala Cys Ser Ser Leu Gly Val Ser Gly Gly Thr Cys Arg Glu Thr Phe
        115                 120                 125

Thr Leu Tyr Tyr Arg Gln Ala Asp Glu Pro Asp Ser Pro Asp Ser Ile
    130                 135                 140

Ser Ala Trp His Leu Lys Arg Trp Thr Lys Val Asp Thr Ile Ala Ala
145                 150                 155                 160

Asp Glu Ser Phe Pro Ala Ser Ser Ser Ser Ser Ser Trp Ala Val Gly
                165                 170                 175

Pro His Arg Thr Asp Gln Arg Val Gly Leu Gln Leu Asn Val Lys Glu
            180                 185                 190

Arg Ser Phe Gly Pro Leu Thr Gln Arg Gly Phe Tyr Val Ala Phe Gln
        195                 200                 205

Asp Thr Gly Ala Cys Leu Ala Leu Val Ala Val Lys Leu Phe Ser Tyr
    210                 215                 220

Thr Cys Pro Ser Val Leu Arg Ala Phe Ala Ser Phe Pro Glu Thr Gln
225                 230                 235                 240

Ala Ser Gly Ala Gly Gly Ala Ser Leu Val Ala Ala Val Gly Thr Cys
                245                 250                 255

Val Ala His Ala Glu Pro Glu Glu Asp Gly Val Gly Gly Gln Ala Gly
            260                 265                 270

Gly Ser Pro Pro Arg Leu His Cys Asn Gly Glu Gly Arg Trp Met Val
        275                 280                 285

Ala Val Gly Gly Cys Arg Cys Gln Pro Gly His Gln Pro Ala Arg Gly
    290                 295                 300

Asp Lys Leu Cys Gln Ala Cys Pro Glu Gly Ser Tyr Lys Ala Leu Pro
305                 310                 315                 320

Gly Asn Val Pro Cys Ser Pro Cys Pro Ala Arg Ser His Ser Pro Asp
                325                 330                 335

Pro Ala Ala Pro Val Cys Pro Cys Leu Gln Gly Phe Tyr Arg Ala Ser
            340                 345                 350

Ser Asp Pro Pro Glu Ala Pro Cys Thr Gly Pro Pro Ser Ala Pro Arg
        355                 360                 365

Glu Leu Trp Phe Glu Val Gln Gly Ser Ala Leu Met Leu His Trp Arg
    370                 375                 380

Leu Pro Gln Glu Leu Gly Gly Arg Gly Asp Leu Leu Phe Asn Val Val
385                 390                 395                 400

Cys Lys Glu Cys Gly Gly His Lys Glu Pro Ser Ser Gly Gly Met Cys
                405                 410                 415

Arg Arg Cys Arg Asp Glu Val His Phe Asp Pro Arg Gln Arg Gly Leu
            420                 425                 430

Thr Glu Ser Arg Val Leu Val Gly Gly Leu Arg Ala His Val Pro Tyr
        435                 440                 445

Ile Leu Glu Val Gln Ala Val Asn Gly Val Ser Glu Leu Ser Pro Asp
    450                 455                 460

Pro Pro Gln Ala Ala Ala Ile Asn Val Ser Thr Ser His Glu Val Pro
465                 470                 475                 480

Ser Ala Val Pro Val Met His Gln Val Ser Arg Ala Ala Asn Ser Ile
                485                 490                 495

Thr Val Ser Trp Pro Gln Pro Glu Gln Thr Asn Gly Asn Ile Leu Asp
            500                 505                 510

Tyr Gln Leu Arg Tyr Tyr Asp Gln Ala Glu Asp Glu Ser His Ser Phe
        515                 520                 525

Thr Met Thr Ser Glu Thr Asn Thr Ala Thr Val Thr Arg Leu Ser Pro
    530                 535                 540

Gly His Ile Tyr Gly Phe Gln Val Arg Ala Arg Thr Ala Ala Gly His
545                 550                 555                 560

Gly Pro Tyr Gly Gly Lys Val Tyr Phe Gln Thr Leu Pro Gln Gly Glu
                565                 570                 575

Leu Ser Ser Gln Leu Pro Glu Lys Leu Ser Leu Val Ile Gly Ser Ile
            580                 585                 590

Leu Gly Ala Leu Ala Phe Leu Leu Leu Ala Ala Ile Thr Val Leu Ala
        595                 600                 605

Val Ile Phe Gln Arg Lys Arg Arg Gly Thr Gly Tyr Thr Glu Gln Leu
    610                 615                 620

Gln Gln Tyr Ser Ser Pro Gly Leu Gly Val Lys Tyr Tyr Ile Asp Pro
625                 630                 635                 640

Ser Thr Tyr Asp Asp Pro Cys Gln Ala Ile Arg Glu Leu Ala Arg Glu
                645                 650                 655

Val Asp Pro Thr Tyr Ile Lys Ile Glu Glu Val Ile Gly Ala Gly Ser
            660                 665                 670

Phe Gly Glu Val Arg Arg Gly Arg Leu Gln Pro Arg Gly Arg Arg Glu
        675                 680                 685

Gln Ala Val Ala Ile Gln Ala Leu Trp Ala Gly Gly Ala Glu Ser Leu
    690                 695                 700

Lys Met Thr Phe Leu Gly Arg Ala Ala Leu Leu Gly Gln Phe Gln His
705                 710                 715                 720

Pro Asn Ile Leu Arg Leu Glu Gly Val Val Thr Arg Ser Arg Pro Val
                725                 730                 735

Met Val Leu Thr Glu Leu Met Glu Leu Gly Pro Leu Asp Ser Phe Leu
            740                 745                 750

Arg Gln Arg Glu Gly Gln Phe Ser Ser Leu Gln Leu Val Ala Met Gln
        755                 760                 765

Arg Gly Val Ala Ala Ala Met Gln Tyr Leu Ser Ser Phe Ala Phe Val
    770                 775                 780

His Arg Ala Leu Ser Ala Arg Ser Val Leu Val Asn Ser His Leu Val
785                 790                 795                 800

Cys Lys Val Ala Arg Leu Gly His Ser Pro Gln Gly Ser Ser Ser Leu
                805                 810                 815

Leu Arg Trp Ala Ala Pro Glu Val Ile Thr His Gly Lys Tyr Thr Thr
            820                 825                 830

Ser Ser Asp Val Trp Ser Phe Gly Ile Leu Met Trp Glu Val Met Ser
        835                 840                 845

Tyr Gly Glu Arg Pro Tyr Trp Asp Met Ser Asp Gln Glu Val Leu Asn
    850                 855                 860

Ala Ile Glu Gln Glu Phe Arg Leu Pro Pro Pro Pro Gly Cys Pro Thr
865                 870                 875                 880

Gly Leu His Leu Leu Met Leu Asp Thr Trp Gln Lys Asp Arg Ala Arg
                885                 890                 895

Arg Pro His Phe Asp Gln Leu Val Ala Ala Phe Asp Lys Met Ile Arg
            900                 905                 910

Lys Pro Asp Thr Leu Gln Ala Glu Gly Ser Ser Gly Asp Arg Pro Ser
        915                 920                 925

Gln Ala Leu Leu Asn Pro Val Ala Leu Asp Phe Pro Cys Leu Asp Ser
    930                 935                 940

Pro Gln Ala Trp Leu Ser Ala Ile Gly Leu Glu Cys Tyr Gln Asp Asn
945                 950                 955                 960

Phe Ser Lys Phe Gly Leu Ser Thr Phe Ser Asp Val Ala Gln Leu Ser
                965                 970                 975

Leu Glu Asp Leu Pro Gly Leu Gly Ile Thr Leu Ala Gly His Gln Lys
            980                 985                 990

Lys Leu Leu His Asn Ile Gln Leu  Leu Gln Gln His Leu  Arg Gln Pro
        995                 1000                1005

Gly Ser  Val Glu Val
    1010

<210>  30
<211>  205
<212>  PRT
<213>  mouse

<400>  30

Met Glu Phe Leu Trp Ala Pro Leu Leu Gly Leu Cys Cys Ser Leu Ala
1               5                   10                  15

Ala Ala Asp Arg His Ile Val Phe Trp Asn Ser Ser Asn Pro Lys Phe
            20                  25                  30

Arg Glu Glu Asp Tyr Thr Val His Val Gln Leu Asn Asp Tyr Leu Asp
        35                  40                  45

Ile Ile Cys Pro His Tyr Glu Asp Asp Ser Val Ala Asp Ala Ala Met
    50                  55                  60

Glu Arg Tyr Thr Leu Tyr Met Val Glu His Gln Glu Tyr Val Ala Cys
65                  70                  75                  80

Gln Pro Gln Ser Lys Asp Gln Val Arg Trp Asn Cys Asn Arg Pro Ser
                85                  90                  95

Ala Lys His Gly Pro Glu Lys Leu Ser Val Lys Phe Gln Arg Phe Thr
            100                 105                 110

Pro Phe Ile Leu Gly Lys Glu Phe Lys Glu Gly His Ser Tyr Tyr Tyr
        115                 120                 125

Ile Ser Lys Pro Ile Tyr His Gln Glu Ser Gln Cys Leu Lys Leu Lys
    130                 135                 140

Val Thr Val Asn Gly Lys Ile Thr His Asn Pro Gln Ala His Val Asn
145                 150                 155                 160

Pro Gln Glu Lys Arg Leu Gln Ala Asp Asp Pro Glu Val Gln Val Leu
                165                 170                 175

His Ser Ile Gly Tyr Ser Ala Ala Pro Arg Leu Phe Pro Leu Val Trp
            180                 185                 190

Ala Val Leu Leu Leu Pro Leu Leu Leu Leu Gln Ser Gln
        195                 200                 205

<210>  31
<211>  205
<212>  PRT
<213>  human

<400>  31

Met Glu Phe Leu Trp Ala Pro Leu Leu Gly Leu Cys Cys Ser Leu Ala
1               5                   10                  15

Ala Ala Asp Arg His Thr Val Phe Trp Asn Ser Ser Asn Pro Lys Phe
            20                  25                  30

Arg Asn Glu Asp Tyr Thr Ile His Val Gln Leu Asn Asp Tyr Val Asp
        35                  40                  45

Ile Ile Cys Pro His Tyr Glu Asp His Ser Val Ala Asp Ala Ala Met
    50                  55                  60

Glu Gln Tyr Ile Leu Tyr Leu Val Glu His Glu Glu Tyr Gln Leu Cys
65                  70                  75                  80

Gln Pro Gln Ser Lys Asp Gln Val Arg Trp Gln Cys Asn Arg Pro Ser
                85                  90                  95

Ala Lys His Gly Pro Glu Lys Leu Ser Glu Lys Phe Gln Arg Phe Thr
            100                 105                 110

Pro Phe Thr Leu Gly Lys Glu Phe Lys Glu Gly His Ser Tyr Tyr Tyr
        115                 120                 125

Ile Ser Lys Pro Ile His Gln His Glu Asp Arg Cys Leu Arg Leu Lys
    130                 135                 140

Val Thr Val Ser Gly Lys Ile Thr His Ser Pro Gln Ala His Asp Asn
145                 150                 155                 160

Pro Gln Glu Lys Arg Leu Ala Ala Asp Asp Pro Glu Val Arg Val Leu
                165                 170                 175

His Ser Ile Gly His Ser Ala Ala Pro Arg Leu Phe Pro Leu Ala Trp
                180                 185                 190

Thr Val Leu Leu Leu Pro Leu Leu Leu Leu Gln Thr Pro
        195                 200                 205

<210>  32
<211>  205
<212>  PRT
<213>  rat

<400>  32

Met Glu Phe Leu Trp Ala Pro Leu Leu Gly Leu Cys Cys Ser Leu Ala
1               5                   10                  15

Ala Ala Asp Arg His Ile Val Phe Trp Asn Ser Ser Asn Pro Lys Phe
            20                  25                  30

Arg Glu Glu Asp Tyr Thr Val His Val Gln Leu Asn Asp Tyr Leu Asp
        35                  40                  45

Ile Ile Cys Pro His Tyr Glu Asp Asp Ser Val Ala Asp Ala Ala Met
    50                  55                  60

Glu Arg Tyr Thr Leu Tyr Met Val Glu His Gln Glu Tyr Val Thr Cys
65                  70                  75                  80

Glu Pro Gln Ser Lys Asp Gln Val Arg Trp Lys Cys Asn Gln Pro Ser
                85                  90                  95

Ala Lys His Gly Pro Glu Lys Leu Ser Glu Lys Phe Gln Arg Phe Thr
            100                 105                 110

Pro Phe Thr Leu Gly Lys Glu Phe Lys Glu Gly His Ser Tyr Tyr Tyr
        115                 120                 125

Ile Ser Lys Pro Ile Tyr His Gln Glu Thr Gln Cys Leu Lys Leu Lys
    130                 135                 140

Val Thr Val Asn Gly Lys Ile Thr His Ser Pro His Ala His Ala Asn
145                 150                 155                 160

Pro Gln Glu Lys Arg Leu Gln Ala Asp Asp Pro Glu Val Gln Val Leu
                165                 170                 175

His Ser Ile Gly His Ser Ala Ala Pro Arg Leu Phe Pro Leu Val Trp
            180                 185                 190

Ala Val Leu Leu Leu Pro Leu Leu Leu Leu Gln Thr Gln
        195                 200                 205

<210>  33

<211>   209
<212>   PRT
<213>   mouse

<400>   33

Met Ala Pro Ala Gln Arg Pro Leu Leu Pro Leu Leu Leu Leu Leu Leu
1               5                   10                  15

Pro Leu Arg Ala Arg Asn Glu Asp Pro Ala Arg Ala Asn Ala Asp Arg
            20                  25                  30

Tyr Ala Val Tyr Trp Asn Arg Ser Asn Pro Arg Phe Gln Val Ser Ala
        35                  40                  45

Val Gly Asp Gly Gly Gly Tyr Thr Val Glu Val Ser Ile Asn Asp Tyr
    50                  55                  60

Leu Asp Ile Tyr Cys Pro His Tyr Gly Ala Pro Leu Pro Pro Ala Glu
65                  70                  75                  80

Arg Met Glu Arg Tyr Ile Leu Tyr Met Val Asn Gly Glu Gly His Ala
                85                  90                  95

Ser Cys Asp His Arg Gln Arg Gly Phe Lys Arg Trp Glu Cys Asn Arg
            100                 105                 110

Pro Ala Ala Pro Gly Gly Pro Leu Lys Phe Ser Glu Lys Phe Gln Leu
        115                 120                 125

Phe Thr Pro Phe Ser Leu Gly Phe Glu Phe Arg Pro Gly His Glu Tyr
    130                 135                 140

Tyr Tyr Ile Ser Ala Thr Pro Pro Asn Leu Val Asp Arg Pro Cys Gln
145                 150                 155                 160

Arg Leu Lys Val Tyr Val Arg Pro Thr Asn Glu Thr Leu Tyr Glu Ala
                165                 170                 175

Pro Glu Pro Ile Phe Thr Ser Asn Ser Ser Cys Ser Gly Leu Gly Gly
            180                 185                 190

Cys His Leu Phe Leu Thr Thr Val Pro Val Leu Trp Ser Leu Leu Gly
        195                 200                 205

Ser

<210>   34
<211>   213
<212>   PRT
<213>   human

<400>   34

Met Ala Pro Ala Gln Arg Pro Leu Leu Pro Leu Leu Leu Leu Leu Leu
1               5                   10                  15

Pro Leu Pro Pro Pro Pro Phe Ala Arg Ala Glu Asp Ala Ala Arg Ala
            20                  25                  30

Asn Ser Asp Arg Tyr Ala Val Tyr Trp Asn Arg Ser Asn Pro Arg Phe
        35                  40                  45

His Ala Gly Ala Gly Asp Asp Gly Gly Gly Tyr Thr Val Glu Val Ser
    50                  55                  60

Ile Asn Asp Tyr Leu Asp Ile Tyr Cys Pro His Tyr Gly Ala Pro Leu
65                  70                  75                  80

Pro Pro Ala Glu Arg Met Glu His Tyr Val Leu Tyr Met Val Asn Gly
                85                  90                  95

Glu Gly His Ala Ser Cys Asp His Arg Gln Arg Gly Phe Lys Arg Trp
            100                 105                 110

Glu Cys Asn Arg Pro Ala Ala Pro Gly Gly Pro Leu Lys Phe Ser Glu
        115                 120                 125

Lys Phe Gln Leu Phe Thr Pro Phe Ser Leu Gly Phe Glu Phe Arg Pro
    130                 135                 140

Gly His Glu Tyr Tyr Tyr Ile Ser Ala Thr Pro Pro Asn Ala Val Asp
145                 150                 155                 160

Arg Pro Cys Leu Arg Leu Lys Val Tyr Val Arg Pro Thr Asn Glu Thr
                165                 170                 175

Leu Tyr Glu Ala Pro Glu Pro Ile Phe Thr Ser Asn Asn Ser Cys Ser
            180                 185                 190

Ser Pro Gly Gly Cys Arg Leu Phe Leu Ser Thr Ile Pro Val Leu Trp
        195                 200                 205

Thr Leu Leu Gly Ser
    210

<210>  35
<211>  204
<212>  PRT
<213>  rat

<220>
<221>  misc_feature
<222>  (1)..(1)
<223>  Xaa can be any naturally occurring amino acid

<400>  35

Xaa Pro Leu Leu Pro Leu Leu Leu Leu Leu Leu Pro Leu Arg Ala Arg
1               5                   10                  15

Asn Glu Asp Pro Ala Arg Ala Asn Ala Asp Arg Tyr Ala Val Tyr Trp
            20                  25                  30

Asn Arg Ser Asn Pro Arg Phe Gln Val Ser Ala Val Gly Asp Gly Gly
        35                  40                  45

Gly Tyr Thr Val Glu Val Ser Ile Asn Asp Tyr Leu Asp Ile Tyr Cys
    50                  55                  60

Pro His Tyr Gly Ala Pro Leu Pro Pro Ala Glu Arg Met Glu Arg Tyr
65                  70                  75                  80

Ile Leu Tyr Met Val Asn Gly Glu Gly His Ala Ser Cys Asp His Arg
                85                  90                  95

Gln Arg Gly Phe Lys Arg Trp Glu Cys Asn Arg Pro Ala Ala Pro Gly
            100                 105                 110

Gly Pro Leu Lys Phe Ser Glu Lys Phe Gln Leu Phe Thr Pro Phe Ser
        115                 120                 125

Leu Gly Phe Glu Phe Arg Pro Gly His Glu Tyr Tyr Tyr Ile Ser Ala
    130                 135                 140

Thr Pro Pro Asn Leu Val Asp Arg Pro Cys Leu Arg Leu Lys Val Tyr
145                 150                 155                 160

Val Arg Pro Thr Asn Glu Thr Leu Tyr Glu Ala Pro Glu Pro Ile Phe
                165                 170                 175

Thr Ser Asn Ser Ser Cys Ser Gly Leu Gly Gly Cys His Leu Phe Leu
            180                 185                 190

Thr Thr Val Pro Val Leu Trp Ser Leu Leu Gly Ser
        195                 200

<210>  36
<211>  206
<212>  PRT
<213>  mouse

<400>  36

Met Arg Leu Leu Pro Leu Leu Arg Thr Val Leu Trp Ala Ala Leu Leu
1               5                   10                  15

Gly Ser Arg Leu Pro Gly Cys Ser Ser Leu Arg His Pro Ile Tyr Trp
            20                  25                  30

Asn Ser Ser Asn Pro Arg Leu Leu Arg Gly Asp Ala Val Val Glu Leu
        35                  40                  45

Gly Phe Asn Asp Tyr Leu Asp Ile Phe Cys Pro His Tyr Glu Ser Pro
    50                  55                  60

Gly Pro Pro Glu Gly Pro Glu Thr Phe Ala Leu Tyr Met Val Asp Trp
65                  70                  75                  80

Ser Gly Tyr Glu Ala Cys Thr Ala Glu Gly Ala Asn Ala Phe Gln Arg
                85                  90                  95

Trp Asn Cys Ser Met Pro Phe Ala Pro Phe Ser Pro Val Arg Phe Ser
            100                 105                 110

Glu Lys Ile Gln Arg Tyr Thr Pro Phe Pro Leu Gly Phe Glu Phe Leu
        115                 120                 125

Pro Gly Glu Thr Tyr Tyr Tyr Ile Ser Val Pro Thr Pro Glu Ser Pro
    130                 135                 140

Gly Arg Cys Leu Arg Leu Gln Val Ser Val Cys Cys Lys Glu Ser Gly
145                 150                 155                 160

Ser Ser His Glu Ser Ala His Pro Val Gly Ser Pro Gly Glu Ser Gly
                165                 170                 175

Thr Ser Gly Trp Arg Gly Gly His Ala Pro Ser Pro Leu Cys Leu Leu
            180                 185                 190

Leu Leu Leu Leu Leu Pro Ile Leu Arg Leu Leu Arg Val Leu
        195                 200                 205

<210>  37
<211>  201
<212>  PRT
<213>  human

<400>  37

Met Arg Leu Leu Pro Leu Leu Arg Thr Val Leu Trp Ala Ala Phe Leu
1               5                   10                  15

Gly Ser Pro Leu Arg Gly Gly Ser Ser Leu Arg His Val Val Tyr Trp
            20                  25                  30

Asn Ser Ser Asn Pro Arg Leu Leu Arg Gly Asp Ala Val Val Glu Leu
        35                  40                  45

Gly Leu Asn Asp Tyr Leu Asp Ile Val Cys Pro His Tyr Glu Gly Pro
    50                  55                  60

Gly Pro Pro Glu Gly Pro Glu Thr Phe Ala Leu Tyr Met Val Asp Trp
65                  70                  75                  80

Pro Gly Tyr Glu Ser Cys Gln Ala Glu Gly Pro Arg Ala Tyr Lys Arg
                85                  90                  95

Trp Val Cys Ser Leu Pro Phe Gly His Val Gln Phe Ser Glu Lys Ile
            100                 105                 110

Gln Arg Phe Thr Pro Phe Ser Leu Gly Phe Glu Phe Leu Pro Gly Glu
        115                 120                 125

Thr Tyr Tyr Tyr Ile Ser Val Pro Thr Pro Glu Ser Ser Gly Gln Cys
    130                 135                 140

Leu Arg Leu Gln Val Ser Val Cys Cys Lys Glu Arg Lys Ser Glu Ser
145                 150                 155                 160

Ala His Pro Val Gly Ser Pro Gly Glu Ser Gly Thr Ser Gly Trp Arg
                165                 170                 175

Gly Gly Asp Thr Pro Ser Pro Leu Cys Leu Leu Leu Leu Leu Leu Leu
            180                 185                 190

Leu Ile Leu Arg Leu Leu Arg Ile Leu
        195                 200

<210>  38
<211>  204
<212>  PRT
<213>  rat

<400>  38

Met Arg Leu Leu Pro Leu Leu Arg Thr Val Leu Trp Ala Ala Leu Leu
1               5                   10                  15

Gly Ser Arg Leu Arg Gly Cys Ser Ser Leu Arg His Ser Ile Tyr Trp
            20                  25                  30

Asn Ser Thr Asn Pro Arg Leu Leu Arg Gly Asp Ala Val Val Glu Leu
        35                  40                  45

Gly Leu Asn Asp Tyr Leu Asp Ile Phe Cys Pro His Tyr Glu Ser Pro
    50                  55                  60

Gly Pro Pro Glu Gly Pro Glu Thr Phe Ala Leu Tyr Ile Val Asp Trp
65                  70                  75                  80

Ser Gly Tyr Glu Ala Cys Lys Ala Glu Gly Ala Asn Ala Phe Gln Arg
                85                  90                  95

Trp Asn Cys Thr Leu Pro Phe Ala Pro Phe Val Pro Val Arg Phe Ser

            100                 105                 110

Glu Lys Ile Gln Arg Phe Thr Pro Phe Pro Leu Gly Phe Glu Phe Leu
        115                 120                 125

Pro Gly Glu Thr Tyr Tyr Tyr Ile Ser Val Pro Thr Pro Glu Ser Pro
    130                 135                 140

Gly Gln Cys Leu Arg Leu Gln Val Ser Val Cys Cys Lys Glu Asp Arg
145                 150                 155                 160

Ser Glu Ser Ala His Pro Val Gly Ser Pro Gly Glu Ser Gly Thr Ser
                165                 170                 175

Gly Trp Arg Gly Gly His Ala Pro Ser Pro Leu Ser Leu Leu Leu Leu
            180                 185                 190

Leu Leu Leu Pro Ile Leu Arg Leu Leu Arg Val Leu
        195                 200

<210> 39
<211> 228
<212> PRT
<213> human

<400> 39

Met Leu His Val Glu Met Leu Thr Leu Val Phe Leu Val Leu Trp Met
1               5                   10                  15

Cys Val Phe Ser Gln Asp Pro Gly Ser Lys Ala Val Ala Asp Arg Tyr
            20                  25                  30

Ala Val Tyr Trp Asn Ser Ser Asn Pro Arg Phe Gln Arg Gly Asp Tyr
        35                  40                  45

His Ile Asp Val Cys Ile Asn Asp Tyr Leu Asp Val Phe Cys Pro His
    50                  55                  60

Tyr Glu Asp Ser Val Pro Glu Asp Lys Thr Glu Arg Tyr Val Leu Tyr
65                  70                  75                  80

Met Val Asn Phe Asp Gly Tyr Ser Ala Cys Asp His Thr Ser Lys Gly
                85                  90                  95

Phe Lys Arg Trp Glu Cys Asn Arg Pro His Ser Pro Asn Gly Pro Leu
            100                 105                 110

Lys Phe Ser Glu Lys Phe Gln Leu Phe Thr Pro Phe Ser Leu Gly Phe
        115                 120                 125

Glu Phe Arg Pro Gly Arg Glu Tyr Phe Tyr Ile Ser Ser Ala Ile Pro
    130                 135                 140

Asp Asn Gly Arg Arg Ser Cys Leu Lys Leu Lys Val Phe Val Arg Pro
145                 150                 155                 160

Thr Asn Ser Cys Met Lys Thr Ile Gly Val His Asp Arg Val Phe Asp
                165                 170                 175

Val Asn Asp Lys Val Glu Asn Ser Leu Glu Pro Ala Asp Asp Thr Val
            180                 185                 190

His Glu Ser Ala Glu Pro Ser Arg Gly Glu Asn Ala Ala Gln Thr Pro
        195                 200                 205

Arg Ile Pro Ser Arg Leu Leu Ala Ile Leu Leu Phe Leu Leu Ala Met
    210                 215                 220

Leu Leu Thr Leu
225

<210>  40
<211>  228
<212>  PRT
<213>  mouse

<400>  40

Met Leu His Val Glu Met Leu Thr Leu Leu Phe Leu Val Leu Trp Met
1               5                   10                  15

Cys Val Phe Ser Gln Asp Pro Gly Ser Lys Val Val Ala Asp Arg Tyr
            20                  25                  30

Ala Val Tyr Trp Asn Ser Ser Asn Pro Arg Phe Gln Arg Gly Asp Tyr
        35                  40                  45

His Ile Asp Val Cys Ile Asn Asp Tyr Leu Asp Val Phe Cys Pro His
    50                  55                  60

Tyr Glu Asp Ser Val Pro Glu Asp Lys Thr Glu Arg Tyr Val Leu Tyr
65                  70                  75                  80

Met Val Asn Phe Asp Gly Tyr Ser Ala Cys Asp His Thr Ser Lys Gly
                85                  90                  95

Phe Lys Arg Trp Glu Cys Asn Arg Pro His Ser Pro Asn Gly Pro Leu
            100                 105                 110

Lys Phe Ser Glu Lys Phe Gln Leu Phe Thr Pro Phe Ser Leu Gly Phe
        115                 120                 125

Glu Phe Arg Pro Gly Arg Glu Tyr Phe Tyr Ile Ser Ser Ala Ile Pro
    130                 135                 140

Asp Asn Gly Arg Arg Ser Cys Leu Lys Leu Lys Val Phe Val Arg Pro
145                 150                 155                 160

Thr Asn Ser Cys Met Lys Thr Ile Gly Val His Asp Arg Val Phe Asp
                165                 170                 175

Val Asn Asp Lys Val Glu Asn Ser Leu Glu Pro Ala Asp Asp Thr Val
            180                 185                 190

His Glu Ser Ala Glu Pro Ser Arg Gly Glu Asn Ala Ala Gln Thr Pro
        195                 200                 205

Arg Ile Pro Ser Arg Leu Leu Ala Ile Leu Leu Phe Leu Leu Ala Met
    210                 215                 220

Leu Leu Thr Leu
225

<210>  41
<211>  228
<212>  PRT
<213>  rat

<400>  41

Met Leu His Val Glu Met Leu Thr Leu Leu Phe Leu Val Leu Trp Met
1               5                   10                  15

Cys Val Phe Ser Gln Asp Pro Gly Ser Lys Val Val Ala Asp Arg Tyr
            20                  25                  30

Ala Val Tyr Trp Asn Ser Ser Asn Pro Arg Phe Gln Arg Gly Asp Tyr
        35                  40                  45

His Ile Asp Val Cys Ile Asn Asp Tyr Leu Asp Val Phe Cys Pro His
    50                  55                  60

Tyr Glu Asp Ser Val Pro Glu Asp Lys Thr Glu Arg Tyr Val Leu Tyr
65                  70                  75                  80

Met Val Asn Phe Asp Gly Tyr Ser Ala Cys Asp His Thr Ser Lys Gly
                85                  90                  95

Phe Lys Arg Trp Glu Cys Asn Arg Pro His Ser Pro Asn Gly Pro Leu
            100                 105                 110

Lys Phe Ser Glu Lys Phe Gln Leu Phe Thr Pro Phe Ser Leu Gly Phe
        115                 120                 125

Glu Phe Arg Pro Gly Arg Glu Tyr Phe Tyr Ile Ser Ser Ala Ile Pro
    130                 135                 140

Asp Asn Gly Arg Arg Ser Cys Leu Lys Leu Lys Val Phe Val Arg Pro
145                 150                 155                 160

Thr Asn Ser Cys Met Lys Thr Ile Gly Val Arg Asp Arg Val Phe Asp
                165                 170                 175

Val Asn Asp Lys Val Glu Asn Ser Leu Glu Pro Ala Asp Asp Thr Val
            180                 185                 190

His Glu Ser Ala Glu Pro Ser Arg Gly Glu Asn Ala Ala Gln Thr Pro
        195                 200                 205

Arg Ile Pro Ser Arg Leu Leu Ala Ile Leu Leu Phe Leu Leu Ala Met
    210                 215                 220

Leu Leu Thr Leu
225

<210>  42
<211>  336
<212>  PRT
<213>  mouse

<400>  42

Met Ala Met Ala Arg Ser Arg Arg Asp Ser Val Trp Lys Tyr Cys Trp
1               5                   10                  15

Gly Leu Leu Met Val Leu Cys Arg Thr Ala Ile Ser Arg Ser Ile Val
            20                  25                  30

Leu Glu Pro Ile Tyr Trp Asn Ser Ser Asn Ser Lys Phe Leu Pro Gly
        35                  40                  45

Gln Gly Leu Val Leu Tyr Pro Gln Ile Gly Asp Lys Leu Asp Ile Ile
    50                  55                  60

Cys Pro Lys Val Asp Ser Lys Thr Val Gly Gln Tyr Glu Tyr Tyr Lys
65                  70                  75                  80

Val Tyr Met Val Asp Lys Asp Gln Ala Asp Arg Cys Thr Ile Lys Lys
                85                  90                  95

Glu Asn Thr Pro Leu Leu Asn Cys Ala Arg Pro Asp Gln Asp Val Lys
            100                 105                 110

Phe Thr Ile Lys Phe Gln Glu Phe Ser Pro Asn Leu Trp Gly Leu Glu
        115                 120                 125

Phe Gln Lys Asn Lys Asp Tyr Tyr Ile Ile Ser Thr Ser Asn Gly Ser
    130                 135                 140

Leu Glu Gly Leu Asp Asn Gln Glu Gly Gly Val Cys Gln Thr Arg Ala

145                 150                 155                 160

Met Lys Ile Leu Met Lys Val Gly Gln Asp Ala Ser Ser Ala Gly Ser
                165                 170                 175

Ala Arg Asn His Gly Pro Thr Arg Arg Pro Glu Leu Glu Ala Gly Thr
            180                 185                 190

Asn Gly Arg Ser Ser Thr Thr Ser Pro Phe Val Lys Pro Asn Pro Gly
        195                 200                 205

Ser Ser Thr Asp Gly Asn Ser Ala Gly His Ser Gly Asn Asn Leu Leu
    210                 215                 220

Gly Ser Glu Val Ala Leu Phe Ala Gly Ile Ala Ser Gly Cys Ile Ile
225                 230                 235                 240

Phe Ile Val Ile Ile Ile Thr Leu Val Val Leu Leu Leu Lys Tyr Arg
                245                 250                 255

Arg Arg His Arg Lys His Ser Pro Gln His Thr Thr Thr Leu Ser Leu
            260                 265                 270

Ser Thr Leu Ala Thr Pro Lys Arg Gly Gly Asn Asn Asn Gly Ser Glu
        275                 280                 285

Pro Ser Asp Val Ile Ile Pro Leu Arg Thr Ala Asp Ser Val Phe Cys
    290                 295                 300

Pro His Tyr Glu Lys Val Ser Gly Asp Tyr Gly His Pro Val Tyr Ile
305                 310                 315                 320

Val Gln Glu Met Pro Pro Gln Ser Pro Ala Asn Ile Tyr Tyr Lys Val
                325                 330                 335

<210>  43
<211>  333
<212>  PRT
<213>  human

<400>  43

Met Ala Val Arg Arg Asp Ser Val Trp Lys Tyr Cys Trp Gly Val Leu
1               5                   10                  15

Met Val Leu Cys Arg Thr Ala Ile Ser Lys Ser Ile Val Leu Glu Pro
            20                  25                  30

Ile Tyr Trp Asn Ser Ser Asn Ser Lys Phe Leu Pro Gly Gln Gly Leu
        35                  40                  45

Val Leu Tyr Pro Gln Ile Gly Asp Lys Leu Asp Ile Ile Cys Pro Lys
    50                  55                  60

Val Asp Ser Lys Thr Val Gly Gln Tyr Glu Tyr Tyr Lys Val Tyr Met
65                  70                  75                  80

Val Asp Lys Asp Gln Ala Asp Arg Cys Thr Ile Lys Lys Glu Asn Thr
                85                  90                  95

Pro Leu Leu Asn Cys Ala Lys Pro Asp Gln Asp Ile Lys Phe Thr Ile
            100                 105                 110

Lys Phe Gln Glu Phe Ser Pro Asn Leu Trp Gly Leu Glu Phe Gln Lys
        115                 120                 125

Asn Lys Asp Tyr Tyr Ile Ile Ser Thr Ser Asn Gly Ser Leu Glu Gly
    130                 135                 140

Leu Asp Asn Gln Glu Gly Gly Val Cys Gln Thr Arg Ala Met Lys Ile
145                 150                 155                 160

Leu Met Lys Val Gly Gln Asp Ala Ser Ser Ala Gly Ser Thr Arg Asn
                165                 170                 175

Lys Asp Pro Thr Arg Arg Pro Glu Leu Glu Ala Gly Thr Asn Gly Arg
            180                 185                 190

Ser Ser Thr Thr Ser Pro Phe Val Lys Pro Asn Pro Gly Ser Ser Thr
        195                 200                 205

Asp Gly Asn Ser Ala Gly His Ser Gly Asn Asn Ile Leu Gly Ser Glu
    210                 215                 220

Val Ala Leu Phe Ala Gly Ile Ala Ser Gly Cys Ile Ile Phe Ile Val
225                 230                 235                 240

Ile Ile Ile Thr Leu Val Val Leu Leu Leu Lys Tyr Arg Arg Arg His
                245                 250                 255

Arg Lys His Ser Pro Gln His Thr Thr Thr Leu Ser Leu Ser Thr Leu
            260                 265                 270

Ala Thr Pro Lys Arg Ser Gly Asn Asn Asn Gly Ser Glu Pro Ser Asp
        275                 280                 285

Ile Ile Ile Pro Leu Arg Thr Ala Asp Ser Val Phe Cys Pro His Tyr
    290                 295                 300

Glu Lys Val Ser Gly Asp Tyr Gly His Pro Val Tyr Ile Val Gln Glu
305                 310                 315                 320

Met Pro Pro Gln Ser Pro Ala Asn Ile Tyr Tyr Lys Val
                325                 330

<210>  44
<211>  355
<212>  PRT
<213>  rat

<400>  44

Met Ala Met Ala Arg Ser Arg Arg Asp Ser Val Trp Lys Tyr Cys Trp
1               5                   10                  15

Gly Leu Leu Met Val Leu Cys Arg Thr Ala Ile Ser Arg Ser Ile Val
            20                  25                  30

Leu Glu Pro Ile Tyr Trp Asn Ser Ser Asn Ser Lys Phe Leu Pro Gly
        35                  40                  45

Gln Gly Leu Val Leu Tyr Pro Gln Ile Gly Asp Lys Leu Asp Ile Ile
    50                  55                  60

Cys Pro Lys Val Asp Ser Lys Thr Val Gly Gln Tyr Glu Tyr Tyr Lys
65                  70                  75                  80

Val Tyr Met Val Asp Lys Glu Gln Ala Asp Arg Cys Thr Ile Lys Lys
                85                  90                  95

Glu Asn Thr Pro Leu Leu Asn Cys Ala Arg Pro Asp Gln Asp Val Lys
            100                 105                 110

Phe Thr Ile Lys Phe Gln Glu Phe Ser Pro Asn Leu Trp Gly Leu Glu
        115                 120                 125

Phe Gln Lys Asn Lys Asp Tyr Tyr Ile Ile Ser Thr Ser Asn Gly Ser
    130                 135                 140

Leu Glu Gly Leu Asp Asn Gln Glu Gly Gly Val Cys Gln Thr Arg Ala
145                 150                 155                 160

Met Lys Ile Leu Met Lys Val Gly Gln Asp Ala Ser Ser Ala Gly Ser
                165                 170                 175

Thr Arg Asn Asn Asp Pro Thr Arg Arg Pro Glu Leu Glu Ala Gly Thr
            180                 185                 190

Asn Gly Arg Ser Ser Thr Thr Ser Pro Phe Val Lys Pro Asn Pro Gly
        195                 200                 205

Thr Pro Gln Gly Lys Pro Arg Thr Leu Val Thr Gly Ser Ser Cys Ser
    210                 215                 220

Cys Ala Gly Ser Ser Thr Asp Gly Asn Ser Ala Gly His Ser Gly Asn
225                 230                 235                 240

Asn Leu Leu Gly Ser Glu Val Ala Leu Phe Ala Gly Ile Ala Ser Gly
                245                 250                 255

Cys Ile Ile Phe Ile Val Ile Ile Ile Thr Leu Val Val Leu Leu Leu
            260                 265                 270

Lys Tyr Arg Arg Arg His Arg Lys His Ser Pro Gln His Thr Thr Thr
        275                 280                 285

Leu Ser Leu Ser Thr Leu Ala Thr Pro Lys Arg Gly Gly Asn Asn Asn
    290                 295                 300

Gly Ser Glu Pro Ser Asp Val Ile Ile Pro Leu Arg Thr Ala Asp Ser
305                 310                 315                 320

Val Phe Cys Pro His Tyr Glu Lys Val Ser Gly Asp Tyr Gly His Pro
                325                 330                 335

Val Tyr Ile Val Gln Glu Met Pro Pro Gln Ser Pro Ala Asn Ile Tyr
            340                 345                 350

Tyr Lys Val
        355

<210>  45
<211>  340
<212>  PRT
<213>  human

<400>  45

Met Gly Pro Pro His Ser Gly Pro Gly Gly Val Arg Val Gly Ala Leu
1               5                   10                  15

Leu Leu Leu Gly Val Leu Gly Leu Val Ser Gly Leu Ser Leu Glu Pro
            20                  25                  30

Val Tyr Trp Asn Ser Ala Asn Lys Arg Phe Gln Ala Glu Gly Gly Tyr
        35                  40                  45

Val Leu Tyr Pro Gln Ile Gly Asp Arg Leu Asp Leu Leu Cys Pro Arg
    50                  55                  60

Ala Arg Pro Pro Gly Pro His Ser Ser Pro Asn Tyr Glu Phe Tyr Lys
65                  70                  75                  80

Leu Tyr Leu Val Gly Gly Ala Gln Gly Arg Arg Cys Glu Ala Pro Pro
                85                  90                  95

Ala Pro Asn Leu Leu Leu Thr Cys Asp Arg Pro Asp Leu Asp Leu Arg
            100                 105                 110

Phe Thr Ile Lys Phe Gln Glu Tyr Ser Pro Asn Leu Trp Gly His Glu
        115                 120                 125

Phe Arg Ser His His Asp Tyr Tyr Ile Ile Ala Thr Ser Asp Gly Thr
    130                 135                 140

Arg Glu Gly Leu Glu Ser Leu Gln Gly Gly Val Cys Leu Thr Arg Gly
145                 150                 155                 160

Met Lys Val Leu Leu Arg Val Gly Gln Ser Pro Arg Gly Gly Ala Val
                165                 170                 175

Pro Arg Lys Pro Val Ser Glu Met Pro Met Glu Arg Asp Arg Gly Ala
            180                 185                 190

Ala His Ser Leu Glu Pro Gly Lys Glu Asn Leu Pro Gly Asp Pro Thr
        195                 200                 205

Ser Asn Ala Thr Ser Arg Gly Ala Glu Gly Pro Leu Pro Pro Pro Ser
    210                 215                 220

Met Pro Ala Val Ala Gly Ala Ala Gly Gly Leu Ala Leu Leu Leu Leu
225                 230                 235                 240

Gly Val Ala Gly Ala Gly Gly Ala Met Cys Trp Arg Arg Arg Arg Ala
                245                 250                 255

Lys Pro Ser Glu Ser Arg His Pro Gly Pro Gly Ser Phe Gly Arg Gly
            260                 265                 270

Gly Ser Leu Gly Leu Gly Gly Gly Gly Gly Met Gly Pro Arg Glu Ala
        275                 280                 285

Glu Pro Gly Glu Leu Gly Ile Ala Leu Arg Gly Gly Gly Ala Ala Asp
    290                 295                 300

Pro Pro Phe Cys Pro His Tyr Glu Lys Val Ser Gly Asp Tyr Gly His
305                 310                 315                 320

Pro Val Tyr Ile Val Gln Asp Gly Pro Pro Gln Ser Pro Pro Asn Ile
                325                 330                 335

Tyr Tyr Lys Val
            340

<210>  46
<211>  340
<212>  PRT
<213>  mouse

<400>  46

Met Gly Ala Pro His Phe Gly Pro Gly Gly Val Gln Val Gly Ala Leu

1               5                   10                  15

Leu Leu Leu Gly Phe Ala Gly Leu Val Ser Gly Leu Ser Leu Glu Pro
            20                  25                  30

Val Tyr Trp Asn Ser Ala Asn Lys Arg Phe Gln Ala Glu Gly Gly Tyr
        35                  40                  45

Val Leu Tyr Pro Gln Ile Gly Asp Arg Leu Asp Leu Leu Cys Pro Arg
    50                  55                  60

Ala Arg Pro Pro Gly Pro His Ser Ser Pro Ser Tyr Glu Phe Tyr Lys
65                  70                  75                  80

Leu Tyr Leu Val Glu Gly Ala Gln Gly Arg Arg Cys Glu Ala Pro Pro
                85                  90                  95

Ala Pro Asn Leu Leu Leu Thr Cys Asp Arg Pro Asp Leu Asp Leu Arg
            100                 105                 110

Phe Thr Ile Lys Phe Gln Glu Tyr Ser Pro Asn Leu Trp Gly His Glu
        115                 120                 125

Phe Arg Ser His His Asp Tyr Tyr Ile Ile Ala Thr Ser Asp Gly Thr
    130                 135                 140

Arg Glu Gly Leu Glu Ser Leu Gln Gly Gly Val Cys Leu Thr Arg Gly
145                 150                 155                 160

Met Lys Val Leu Leu Arg Val Gly Gln Ser Pro Arg Gly Gly Ala Val
                165                 170                 175

Pro Arg Lys Pro Val Ser Glu Met Pro Met Glu Arg Asp Arg Gly Ala
            180                 185                 190

Ala His Ser Ala Glu Pro Gly Arg Asp Thr Ile Pro Gly Asp Pro Ser
        195                 200                 205

Ser Asn Ala Thr Ser Arg Gly Ala Glu Gly Pro Leu Pro Pro Pro Ser
    210                 215                 220

Met Pro Ala Val Ala Gly Ala Ala Gly Gly Met Ala Leu Leu Leu Leu
225                 230                 235                 240

Gly Val Ala Gly Ala Gly Gly Ala Met Cys Trp Arg Arg Arg Arg Ala
                245                 250                 255

Lys Pro Ser Glu Ser Arg His Pro Gly Pro Gly Ser Phe Gly Arg Gly
            260                 265                 270

Gly Ser Leu Gly Leu Gly Gly Gly Gly Gly Met Gly Pro Arg Glu Ala

275                 280                 285

Glu Pro Gly Glu Leu Gly Ile Ala Leu Arg Gly Gly Gly Thr Ala Asp
    290                 295                 300

Pro Pro Phe Cys Pro His Tyr Glu Lys Val Ser Gly Asp Tyr Gly His
305                 310                 315                 320

Pro Val Tyr Ile Val Gln Asp Gly Pro Pro Gln Ser Pro Pro Asn Ile
                325                 330                 335

Tyr Tyr Lys Val
            340

<210>  47
<211>  340
<212>  PRT
<213>  rat

<400>  47

Met Gly Gly Pro His Phe Gly Pro Gly Gly Val Gln Val Gly Ala Leu
1               5                   10                  15

Leu Leu Leu Gly Phe Ala Gly Leu Val Ser Gly Leu Ser Leu Glu Pro
            20                  25                  30

Val Tyr Trp Asn Ser Ala Asn Lys Arg Phe Gln Ala Glu Gly Gly Tyr
        35                  40                  45

Val Leu Tyr Pro Gln Ile Gly Asp Arg Leu Asp Leu Leu Cys Pro Arg
    50                  55                  60

Ala Arg Pro Pro Gly Pro His Ser Ser Pro Ser Tyr Glu Phe Tyr Lys
65                  70                  75                  80

Leu Tyr Leu Val Gly Gly Ala Gln Gly Arg Arg Cys Glu Ala Pro Pro
                85                  90                  95

Ala Pro Asn Leu Leu Leu Thr Cys Asp Arg Pro Asp Leu Asp Leu Arg
            100                 105                 110

Phe Thr Ile Lys Phe Gln Glu Tyr Ser Pro Asn Leu Trp Gly His Glu
        115                 120                 125

Phe Arg Ser His His Asp Tyr Tyr Ile Ile Ala Thr Ser Asp Gly Thr
    130                 135                 140

Arg Glu Gly Leu Glu Ser Leu Gln Gly Gly Val Cys Leu Thr Arg Gly
145                 150                 155                 160

Met Lys Val Leu Leu Arg Val Gly Gln Ser Pro Arg Gly Gly Ala Val
                165                 170                 175

Pro Arg Lys Pro Val Ser Glu Met Pro Met Glu Arg Asp Arg Gly Ala
            180                 185                 190

Ala His Ser Gln Glu Pro Gly Lys Asp Ser Ile Pro Gly Asp Pro Asn
        195                 200                 205

Ser Asn Ala Thr Ser Arg Gly Ala Glu Gly Pro Leu Pro Pro Pro Ser
    210                 215                 220

Met Pro Ala Val Ala Gly Ala Ala Gly Gly Leu Ala Leu Leu Leu Leu
225                 230                 235                 240

Gly Val Ala Gly Ala Gly Gly Ala Met Cys Trp Arg Arg Arg Arg Ala
                245                 250                 255

Lys Pro Ser Glu Ser Arg His Pro Gly Pro Gly Ser Phe Gly Arg Gly
            260                 265                 270

Gly Ser Leu Gly Leu Gly Gly Gly Gly Gly Met Gly Pro Arg Glu Ala
        275                 280                 285

Glu Pro Gly Glu Leu Gly Ile Ala Leu Arg Gly Gly Gly Ala Ala Asp
    290                 295                 300

Pro Pro Phe Cys Pro His Tyr Glu Lys Val Ser Gly Asp Tyr Gly His
305                 310                 315                 320

Pro Val Tyr Ile Val Gln Asp Gly Pro Pro Gln Ser Pro Pro Asn Ile
                325                 330                 335

Tyr Tyr Lys Val
            340

<210> 48
<211> 112
<212> PRT
<213> human

<400> 48

Met Ala Glu Gln Ala Thr Lys Ser Val Leu Phe Val Cys Leu Gly Asn
1               5                   10                  15

Ile Cys Arg Ser Pro Ile Ala Glu Ala Val Phe Arg Lys Leu Val Thr
            20                  25                  30

Asp Gln Asn Ile Ser Glu Asn Trp Arg Val Asp Ser Ala Ala Thr Ser
        35                  40                  45

Gly Tyr Glu Ile Gly Asn Pro Pro Asp Tyr Arg Gly Gln Ser Cys Met
    50                  55                  60

Lys Arg His Gly Ile Pro Met Ser His Val Ala Arg Gln Val Pro Ser
65                  70                  75                  80

Leu Asp Leu Lys Leu Cys Val Leu Cys Phe Ser Gly Ser Leu Thr Ala
                85                  90                  95

Val Leu Phe Leu Thr Gly Thr Trp Ala Gly Pro Gln Thr Gln Glu Leu
            100                 105                 110

<210>  49
<211>  158
<212>  PRT
<213>  human

<400>  49

Met Ala Glu Gln Ala Thr Lys Ser Val Leu Phe Val Cys Leu Gly Asn
1               5                   10                  15

Ile Cys Arg Ser Pro Ile Ala Glu Ala Val Phe Arg Lys Leu Val Thr
            20                  25                  30

Asp Gln Asn Ile Ser Glu Asn Trp Val Ile Asp Ser Gly Ala Val Ser
        35                  40                  45

Asp Trp Asn Val Gly Arg Ser Pro Asp Pro Arg Ala Val Ser Cys Leu
    50                  55                  60

Arg Asn His Gly Ile His Thr Ala His Lys Ala Arg Gln Ile Thr Lys
65                  70                  75                  80

Glu Asp Phe Ala Thr Phe Asp Tyr Ile Leu Cys Met Asp Glu Ser Asn
                85                  90                  95

Leu Arg Asp Leu Asn Arg Lys Ser Asn Gln Val Lys Thr Cys Lys Ala
            100                 105                 110

Lys Ile Glu Leu Leu Gly Ser Tyr Asp Pro Gln Lys Gln Leu Ile Ile
        115                 120                 125

Glu Asp Pro Tyr Tyr Gly Asn Asp Ser Asp Phe Glu Thr Val Tyr Gln
    130                 135                 140

Gln Cys Val Arg Cys Cys Arg Ala Phe Leu Glu Lys Ala His
145                 150                 155

<210>  50
<211>  158
<212>  PRT
<213>  human

<400>  50

Met Ala Glu Gln Ala Thr Lys Ser Val Leu Phe Val Cys Leu Gly Asn

1                   5                   10                  15

Ile Cys Arg Ser Pro Ile Ala Glu Ala Val Phe Arg Lys Leu Val Thr
            20                  25                  30

Asp Gln Asn Ile Ser Glu Asn Trp Arg Val Asp Ser Ala Ala Thr Ser
        35                  40                  45

Gly Tyr Glu Ile Gly Asn Pro Pro Asp Tyr Arg Gly Gln Ser Cys Met
    50                  55                  60

Lys Arg His Gly Ile Pro Met Ser His Val Ala Arg Gln Ile Thr Lys
65                  70                  75                  80

Glu Asp Phe Ala Thr Phe Asp Tyr Ile Leu Cys Met Asp Glu Ser Asn
                85                  90                  95

Leu Arg Asp Leu Asn Arg Lys Ser Asn Gln Val Lys Thr Cys Lys Ala
            100                 105                 110

Lys Ile Glu Leu Leu Gly Ser Tyr Asp Pro Gln Lys Gln Leu Ile Ile
        115                 120                 125

Glu Asp Pro Tyr Tyr Gly Asn Asp Ser Asp Phe Glu Thr Val Tyr Gln
    130                 135                 140

Gln Cys Val Arg Cys Cys Arg Ala Phe Leu Glu Lys Ala His
145                 150                 155

<210>  51
<211>  158
<212>  PRT
<213>  mouse

<400>  51

Met Ala Glu Val Gly Ser Lys Ser Val Leu Phe Val Cys Leu Gly Asn
1               5                   10                  15

Ile Cys Arg Ser Pro Ile Ala Glu Ala Val Phe Arg Lys Leu Val Thr
            20                  25                  30

Asp Glu Lys Val Ser Asp Asn Trp Arg Ile Asp Ser Ala Ala Thr Ser
        35                  40                  45

Thr Tyr Glu Val Gly Asn Pro Pro Asp Tyr Arg Gly Gln Asn Cys Met
    50                  55                  60

Arg Lys His Gly Ile His Met Gln His Ile Ala Arg Gln Ile Thr Lys
65                  70                  75                  80

Glu Asp Phe Ala Thr Phe Asp Tyr Ile Leu Cys Met Asp Glu Ser Asn
                85                  90                  95

Leu Arg Asp Leu Asn Arg Lys Ser Asn Gln Val Lys Asn Cys Lys Ala
            100                 105                 110

Lys Ile Glu Leu Leu Gly Ser Tyr Asp Pro Gln Lys Gln Leu Ile Ile
        115                 120                 125

Glu Asp Pro Tyr Tyr Gly Asn Asp Ser Asp Phe Glu Val Val Tyr Gln
    130                 135                 140

Gln Cys Leu Arg Cys Cys Lys Ala Phe Leu Glu Lys Thr Tyr
145                 150                 155

<210>  52
<211>  158
<212>  PRT
<213>  rat

<400>  52

Met Ala Glu Val Gly Ser Lys Ser Val Leu Phe Val Cys Leu Gly Asn
1               5                   10                  15

Ile Cys Arg Ser Pro Ile Ala Glu Ala Val Phe Arg Lys Leu Val Thr
            20                  25                  30

Asp Glu Asn Val Ser Asp Asn Trp Arg Ile Asp Ser Ala Ala Thr Ser
        35                  40                  45

Thr Tyr Glu Val Gly Asn Pro Pro Asp Tyr Arg Gly Gln Asn Cys Met
    50                  55                  60

Lys Lys His Gly Ile His Met Gln His Ile Ala Arg Gln Ile Thr Arg
65                  70                  75                  80

Glu Asp Phe Ala Thr Phe Asp Tyr Ile Leu Cys Met Asp Glu Ser Asn
                85                  90                  95

Leu Arg Asp Leu Asn Arg Lys Ser Asn Gln Val Lys Asn Cys Lys Ala
            100                 105                 110

Lys Ile Glu Leu Leu Gly Ser Tyr Asp Pro Gln Lys Gln Leu Ile Ile
        115                 120                 125

Glu Asp Pro Tyr Tyr Gly Asn Asp Ser Asp Phe Glu Val Val Tyr Gln
    130                 135                 140

Gln Cys Leu Arg Cys Cys Lys Ala Phe Leu Glu Lys Thr His
145                 150                 155

<210>  53
<211>  1216
<212>  PRT

<213>  human

<400>  53

Met Gly His Leu Pro Thr Gly Ile His Gly Ala Arg Arg Leu Leu Pro
1               5                   10                  15

Leu Leu Trp Leu Phe Val Leu Phe Lys Asn Ala Thr Ala Phe His Val
            20                  25                  30

Thr Val Gln Asp Asp Asn Asn Ile Val Val Ser Leu Glu Ala Ser Asp
        35                  40                  45

Val Ile Ser Pro Ala Ser Val Tyr Val Val Lys Ile Thr Gly Glu Ser
    50                  55                  60

Lys Asn Tyr Phe Phe Glu Phe Glu Glu Phe Asn Ser Thr Leu Pro Pro
65                  70                  75                  80

Pro Val Ile Phe Lys Ala Ser Tyr His Gly Leu Tyr Tyr Ile Ile Thr
                85                  90                  95

Leu Val Val Val Asn Gly Asn Val Val Thr Lys Pro Ser Arg Ser Ile
            100                 105                 110

Thr Val Leu Thr Lys Pro Leu Pro Val Thr Ser Val Ser Ile Tyr Asp
        115                 120                 125

Tyr Lys Pro Ser Pro Glu Thr Gly Val Leu Phe Glu Ile His Tyr Pro
    130                 135                 140

Glu Lys Tyr Asn Val Phe Thr Arg Val Asn Ile Ser Tyr Trp Glu Gly
145                 150                 155                 160

Lys Asp Phe Arg Thr Met Leu Tyr Lys Asp Phe Phe Lys Gly Lys Thr
                165                 170                 175

Val Phe Asn His Trp Leu Pro Gly Met Cys Tyr Ser Asn Ile Thr Phe
            180                 185                 190

Gln Leu Val Ser Glu Ala Thr Phe Asn Lys Ser Thr Leu Val Glu Tyr
        195                 200                 205

Ser Gly Val Ser His Glu Pro Lys Gln His Arg Thr Ala Pro Tyr Pro
    210                 215                 220

Pro Gln Asn Ile Ser Val Arg Ile Val Asn Leu Asn Lys Asn Asn Trp
225                 230                 235                 240

Glu Glu Gln Ser Gly Asn Phe Pro Glu Glu Ser Phe Met Arg Ser Gln
                245                 250                 255

Asp Thr Ile Gly Lys Glu Lys Leu Phe His Phe Thr Glu Glu Thr Pro
            260                 265                 270

Glu Ile Pro Ser Gly Asn Ile Ser Ser Gly Trp Pro Asp Phe Asn Ser
        275                 280                 285

Ser Asp Tyr Glu Thr Thr Ser Gln Pro Tyr Trp Trp Asp Ser Ala Ser
    290                 295                 300

Ala Ala Pro Glu Ser Glu Asp Glu Phe Val Ser Val Leu Pro Met Glu
305                 310                 315                 320

Tyr Glu Asn Asn Ser Thr Leu Ser Glu Thr Glu Lys Ser Thr Ser Gly
                325                 330                 335

Ser Phe Ser Phe Phe Pro Val Gln Met Ile Leu Thr Trp Leu Pro Pro
            340                 345                 350

Lys Pro Pro Thr Ala Phe Asp Gly Phe His Ile His Ile Glu Arg Glu
        355                 360                 365

Glu Asn Phe Thr Glu Tyr Leu Met Val Asp Glu Glu Ala His Glu Phe
    370                 375                 380

Val Ala Glu Leu Lys Glu Pro Gly Lys Tyr Lys Leu Ser Val Thr Thr
385                 390                 395                 400

Phe Ser Ser Ser Gly Ser Cys Glu Thr Arg Lys Ser Gln Ser Ala Lys
                405                 410                 415

Ser Leu Ser Phe Tyr Ile Ser Pro Ser Gly Glu Trp Ile Glu Glu Leu
            420                 425                 430

Thr Glu Lys Pro Gln His Val Ser Val His Val Leu Ser Ser Thr Thr
        435                 440                 445

Ala Leu Met Ser Trp Thr Ser Ser Gln Glu Asn Tyr Asn Ser Thr Ile
    450                 455                 460

Val Ser Val Val Ser Leu Thr Cys Gln Lys Gln Lys Glu Ser Gln Arg
465                 470                 475                 480

Leu Glu Lys Gln Tyr Cys Thr Gln Val Asn Ser Ser Lys Pro Ile Ile
                485                 490                 495

Glu Asn Leu Val Pro Gly Ala Gln Tyr Gln Val Val Ile Tyr Leu Arg
            500                 505                 510

Lys Gly Pro Leu Ile Gly Pro Pro Ser Asp Pro Val Thr Phe Ala Ile
        515                 520                 525

Val Pro Thr Gly Ile Lys Asp Leu Met Leu Tyr Pro Leu Gly Pro Thr
    530                 535                 540

Ala Val Val Leu Ser Trp Thr Arg Pro Tyr Leu Gly Val Phe Arg Lys
545                 550                 555                 560

Tyr Val Val Glu Met Phe Tyr Phe Asn Pro Ala Thr Met Thr Ser Glu
                565                 570                 575

Trp Thr Thr Tyr Tyr Glu Ile Ala Ala Thr Val Ser Leu Thr Ala Ser
            580                 585                 590

Val Arg Ile Ala Asn Leu Leu Pro Ala Trp Tyr Tyr Asn Phe Arg Val
        595                 600                 605

Thr Met Val Thr Trp Gly Asp Pro Glu Leu Ser Cys Cys Asp Ser Ser
    610                 615                 620

Thr Ile Ser Phe Ile Thr Ala Pro Val Ala Pro Glu Ile Thr Ser Val
625                 630                 635                 640

Glu Tyr Phe Asn Ser Leu Leu Tyr Ile Ser Trp Thr Tyr Gly Asp Asp
                645                 650                 655

Thr Thr Asp Leu Ser His Ser Arg Met Leu His Trp Met Val Val Ala
            660                 665                 670

Glu Gly Lys Lys Lys Ile Lys Lys Ser Val Thr Arg Asn Val Met Thr
        675                 680                 685

Ala Ile Leu Ser Leu Pro Pro Gly Asp Ile Tyr Asn Leu Ser Val Thr
    690                 695                 700

Ala Cys Thr Glu Arg Gly Ser Asn Thr Ser Met Leu Arg Leu Val Lys
705                 710                 715                 720

Leu Glu Pro Ala Pro Pro Lys Ser Leu Phe Ala Val Asn Lys Thr Gln
                725                 730                 735

Thr Ser Val Thr Leu Leu Trp Val Glu Glu Gly Val Ala Asp Phe Phe
            740                 745                 750

Glu Val Phe Cys Gln Gln Val Gly Ser Ser Gln Lys Thr Lys Leu Gln
        755                 760                 765

Glu Pro Val Ala Val Ser Ser His Val Val Thr Ile Ser Ser Leu Leu
    770                 775                 780

Pro Ala Thr Ala Tyr Asn Cys Ser Val Thr Ser Phe Ser His Asp Ser
785                 790                 795                 800

Pro Ser Val Pro Thr Phe Ile Ala Val Ser Thr Met Val Thr Glu Met
                805                 810                 815

Asn Pro Asn Val Val Val Ile Ser Val Leu Ala Ile Leu Ser Thr Leu
            820                 825                 830

Leu Ile Gly Leu Leu Leu Val Thr Leu Ile Ile Leu Arg Lys Lys His
        835                 840                 845

Leu Gln Met Ala Arg Glu Cys Gly Ala Gly Thr Phe Val Asn Phe Ala
    850                 855                 860

Ser Leu Glu Arg Asp Gly Lys Leu Pro Tyr Asn Trp Arg Arg Ser Ile
865                 870                 875                 880

Phe Ala Phe Leu Thr Leu Leu Pro Ser Cys Leu Trp Thr Asp Tyr Leu
                885                 890                 895

Leu Ala Phe Tyr Ile Asn Pro Trp Ser Lys Asn Gly Leu Lys Lys Arg
            900                 905                 910

Lys Leu Thr Asn Pro Val Gln Leu Asp Asp Phe Asp Ala Tyr Ile Lys
        915                 920                 925

Asp Met Ala Lys Asp Ser Asp Tyr Lys Phe Ser Leu Gln Phe Glu Glu
    930                 935                 940

Leu Lys Leu Ile Gly Leu Asp Ile Pro His Phe Ala Ala Asp Leu Pro
945                 950                 955                 960

Leu Asn Arg Cys Lys Asn Arg Tyr Thr Asn Ile Leu Pro Tyr Asp Phe
                965                 970                 975

Ser Arg Val Arg Leu Val Ser Met Asn Glu Glu Glu Gly Ala Asp Tyr
            980                 985                 990

Ile Asn Ala Asn Tyr Ile Pro Gly  Tyr Asn Ser Pro Gln  Glu Tyr Ile
        995                 1000                1005

Ala Thr  Gln Gly Pro Leu Pro  Glu Thr Arg Asn Asp  Phe Trp Lys
    1010                1015                1020

Met Val  Leu Gln Gln Lys Ser  Gln Ile Ile Val Met  Leu Thr Gln
    1025                1030                1035

Cys Asn  Glu Lys Arg Arg Val  Lys Cys Asp His Tyr  Trp Pro Phe
    1040                1045                1050

Thr Glu  Glu Pro Ile Ala Tyr  Gly Asp Ile Thr Val  Glu Met Ile
    1055                1060                1065

Ser Glu Glu Glu Gln Asp Asp Trp Ala Cys Arg His Phe Arg Ile
    1070                1075                1080

Asn Tyr Ala Asp Glu Met Gln Asp Val Met His Phe Asn Tyr Thr
    1085                1090                1095

Ala Trp Pro Asp His Gly Val Pro Thr Ala Asn Ala Ala Glu Ser
    1100                1105                1110

Ile Leu Gln Phe Val His Met Val Arg Gln Gln Ala Thr Lys Ser
    1115                1120                1125

Lys Gly Pro Met Ile Ile His Cys Ser Ala Gly Val Gly Arg Thr
    1130                1135                1140

Gly Thr Phe Ile Ala Leu Asp Arg Leu Leu Gln His Ile Arg Asp
    1145                1150                1155

His Glu Phe Val Asp Ile Leu Gly Leu Val Ser Glu Met Arg Ser
    1160                1165                1170

Tyr Arg Met Ser Met Val Gln Thr Glu Glu Gln Tyr Ile Phe Ile
    1175                1180                1185

His Gln Cys Val Gln Leu Met Trp Met Lys Lys Lys Gln Gln Phe
    1190                1195                1200

Cys Ile Ser Asp Val Ile Tyr Glu Asn Val Ser Lys Ser
    1205                1210                1215

<210> 54
<211> 1188
<212> PRT
<213> human

<400> 54

Met Gly His Leu Pro Thr Gly Ile His Gly Ala Arg Arg Leu Leu Pro
1               5                   10                  15

Leu Leu Trp Leu Phe Val Leu Phe Lys Asn Ala Thr Ala Phe His Val
            20                  25                  30

Thr Val Gln Asp Asp Asn Asn Ile Val Val Ser Leu Glu Ala Ser Asp
        35                  40                  45

Val Ile Ser Pro Ala Ser Val Tyr Val Val Lys Ile Thr Gly Glu Ser
    50                  55                  60

Lys Asn Tyr Phe Phe Glu Phe Glu Glu Phe Asn Ser Thr Leu Pro Pro
65                  70                  75                  80

Pro Val Ile Phe Lys Ala Ser Tyr His Gly Leu Tyr Tyr Ile Ile Thr

                85                  90                  95

Leu Val Val Val Asn Gly Asn Val Val Thr Lys Pro Ser Arg Ser Ile
            100                 105                 110

Thr Val Leu Thr Lys Pro Leu Pro Val Thr Ser Val Ser Ile Tyr Asp
        115                 120                 125

Tyr Lys Pro Ser Pro Glu Thr Gly Val Leu Phe Glu Ile His Tyr Pro
    130                 135                 140

Glu Lys Tyr Asn Val Phe Thr Arg Val Asn Ile Ser Tyr Trp Glu Gly
145                 150                 155                 160

Lys Asp Phe Arg Thr Met Leu Tyr Lys Asp Phe Phe Lys Gly Lys Thr
                165                 170                 175

Val Phe Asn His Trp Leu Pro Gly Met Cys Tyr Ser Asn Ile Thr Phe
            180                 185                 190

Gln Leu Val Ser Glu Ala Thr Phe Asn Lys Ser Thr Leu Val Glu Tyr
        195                 200                 205

Ser Gly Val Ser His Glu Pro Lys Gln His Arg Thr Ala Pro Tyr Pro
    210                 215                 220

Pro Gln Asn Ile Ser Val Arg Ile Val Asn Leu Asn Lys Asn Asn Trp
225                 230                 235                 240

Glu Glu Gln Ser Gly Asn Phe Pro Glu Glu Ser Phe Met Arg Ser Gln
                245                 250                 255

Asp Thr Ile Gly Lys Glu Lys Leu Phe His Phe Thr Glu Glu Thr Pro
            260                 265                 270

Glu Ile Pro Ser Gly Asn Ile Ser Ser Gly Trp Pro Asp Phe Asn Ser
        275                 280                 285

Ser Asp Tyr Glu Thr Thr Ser Gln Pro Tyr Trp Trp Asp Ser Ala Ser
    290                 295                 300

Ala Ala Pro Glu Ser Glu Asp Glu Phe Val Ser Val Leu Pro Met Glu
305                 310                 315                 320

Tyr Glu Asn Asn Ser Thr Leu Ser Glu Thr Glu Lys Ser Thr Ser Gly
                325                 330                 335

Ser Phe Ser Phe Phe Pro Val Gln Met Ile Leu Thr Trp Leu Pro Pro
            340                 345                 350

Lys Pro Pro Thr Ala Phe Asp Gly Phe His Ile His Ile Glu Arg Glu

        355                 360                 365

Glu Asn Phe Thr Glu Tyr Leu Met Val Asp Glu Glu Ala His Glu Phe
    370                 375                 380

Val Ala Glu Leu Lys Glu Pro Gly Lys Tyr Lys Leu Ser Val Thr Thr
385                 390                 395                 400

Phe Ser Ser Ser Gly Ser Cys Glu Thr Arg Lys Ser Gln Ser Ala Lys
                405                 410                 415

Ser Leu Ser Phe Tyr Ile Ser Pro Ser Gly Glu Trp Ile Glu Glu Leu
            420                 425                 430

Thr Glu Lys Pro Gln His Val Ser Val His Val Leu Ser Ser Thr Thr
        435                 440                 445

Ala Leu Met Ser Trp Thr Ser Ser Gln Glu Asn Tyr Asn Ser Thr Ile
    450                 455                 460

Val Ser Val Val Ser Leu Thr Cys Gln Lys Gln Lys Glu Ser Gln Arg
465                 470                 475                 480

Leu Glu Lys Gln Tyr Cys Thr Gln Val Asn Ser Ser Lys Pro Ile Ile
                485                 490                 495

Glu Asn Leu Val Pro Gly Ala Gln Tyr Gln Val Val Ile Tyr Leu Arg
            500                 505                 510

Lys Gly Pro Leu Ile Gly Pro Pro Ser Asp Pro Val Thr Phe Ala Ile
        515                 520                 525

Val Pro Thr Gly Ile Lys Asp Leu Met Leu Tyr Pro Leu Gly Pro Thr
    530                 535                 540

Ala Val Val Leu Ser Trp Thr Arg Pro Tyr Leu Gly Val Phe Arg Lys
545                 550                 555                 560

Tyr Val Val Glu Met Phe Tyr Phe Asn Pro Ala Thr Met Thr Ser Glu
                565                 570                 575

Trp Thr Thr Tyr Tyr Glu Ile Ala Ala Thr Val Ser Leu Thr Ala Ser
            580                 585                 590

Val Arg Ile Ala Asn Leu Leu Pro Ala Trp Tyr Tyr Asn Phe Arg Val
        595                 600                 605

Thr Met Val Thr Trp Gly Asp Pro Glu Leu Ser Cys Cys Asp Ser Ser
    610                 615                 620

Thr Ile Ser Phe Ile Thr Ala Pro Val Ala Pro Glu Ile Thr Ser Val

625                 630                 635                 640

Glu Tyr Phe Asn Ser Leu Leu Tyr Ile Ser Trp Thr Tyr Gly Asp Asp
                645                 650                 655

Thr Thr Asp Leu Ser His Ser Arg Met Leu His Trp Met Val Val Ala
            660                 665                 670

Glu Gly Lys Lys Lys Ile Lys Lys Ser Val Thr Arg Asn Val Met Thr
        675                 680                 685

Ala Ile Leu Ser Leu Pro Pro Gly Asp Ile Tyr Asn Leu Ser Val Thr
    690                 695                 700

Ala Cys Thr Glu Arg Gly Ser Asn Thr Ser Met Leu Arg Leu Val Lys
705                 710                 715                 720

Leu Glu Pro Ala Pro Pro Lys Ser Leu Phe Ala Val Asn Lys Thr Gln
                725                 730                 735

Thr Ser Val Thr Leu Leu Trp Val Glu Glu Gly Val Ala Asp Phe Phe
            740                 745                 750

Glu Val Phe Cys Gln Gln Val Gly Ser Ser Gln Lys Thr Lys Leu Gln
        755                 760                 765

Glu Pro Val Ala Val Ser Ser His Val Val Thr Ile Ser Ser Leu Leu
    770                 775                 780

Pro Ala Thr Ala Tyr Asn Cys Ser Val Thr Ser Phe Ser His Asp Ser
785                 790                 795                 800

Pro Ser Val Pro Thr Phe Ile Ala Val Ser Thr Met Val Thr Glu Met
                805                 810                 815

Asn Pro Asn Val Val Val Ile Ser Val Leu Ala Ile Leu Ser Thr Leu
            820                 825                 830

Leu Ile Gly Leu Leu Leu Val Thr Leu Ile Ile Leu Arg Lys Lys His
        835                 840                 845

Leu Gln Met Ala Arg Glu Cys Gly Ala Gly Thr Phe Val Asn Phe Ala
    850                 855                 860

Ser Leu Glu Arg Asp Gly Lys Leu Pro Tyr Asn Trp Ser Lys Asn Gly
865                 870                 875                 880

Leu Lys Lys Arg Lys Leu Thr Asn Pro Val Gln Leu Asp Asp Phe Asp
                885                 890                 895

Ala Tyr Ile Lys Asp Met Ala Lys Asp Ser Asp Tyr Lys Phe Ser Leu

            900                 905                 910

Gln Phe Glu Glu Leu Lys Leu Ile Gly Leu Asp Ile Pro His Phe Ala
        915                 920                 925

Ala Asp Leu Pro Leu Asn Arg Cys Lys Asn Arg Tyr Thr Asn Ile Leu
    930                 935                 940

Pro Tyr Asp Phe Ser Arg Val Arg Leu Val Ser Met Asn Glu Glu Glu
945                 950                 955                 960

Gly Ala Asp Tyr Ile Asn Ala Asn Tyr Ile Pro Gly Tyr Asn Ser Pro
                965                 970                 975

Gln Glu Tyr Ile Ala Thr Gln Gly Pro Leu Pro Glu Thr Arg Asn Asp
            980                 985                 990

Phe Trp Lys Met Val Leu Gln Gln Lys Ser Gln Ile Ile Val Met Leu
        995                 1000                1005

Thr Gln Cys Asn Glu Lys Arg Arg Val Lys Cys Asp His Tyr Trp
    1010                1015                1020

Pro Phe Thr Glu Glu Pro Ile Ala Tyr Gly Asp Ile Thr Val Glu
    1025                1030                1035

Met Ile Ser Glu Glu Glu Gln Asp Asp Trp Ala Cys Arg His Phe
    1040                1045                1050

Arg Ile Asn Tyr Ala Asp Glu Met Gln Asp Val Met His Phe Asn
    1055                1060                1065

Tyr Thr Ala Trp Pro Asp His Gly Val Pro Thr Ala Asn Ala Ala
    1070                1075                1080

Glu Ser Ile Leu Gln Phe Val His Met Val Arg Gln Gln Ala Thr
    1085                1090                1095

Lys Ser Lys Gly Pro Met Ile Ile His Cys Ser Ala Gly Val Gly
    1100                1105                1110

Arg Thr Gly Thr Phe Ile Ala Leu Asp Arg Leu Leu Gln His Ile
    1115                1120                1125

Arg Asp His Glu Phe Val Asp Ile Leu Gly Leu Val Ser Glu Met
    1130                1135                1140

Arg Ser Tyr Arg Met Ser Met Val Gln Thr Glu Glu Gln Tyr Ile
    1145                1150                1155

Phe Ile His Gln Cys Val Gln Leu Met Trp Met Lys Lys Lys Gln

1160                    1165                    1170

Gln Phe  Cys Ile Ser Asp Val  Ile Tyr Glu Asn Val  Ser Lys Ser
    1175                    1180                    1185

<210>  55
<211>  405
<212>  PRT
<213>  human

<400>  55

Met Val Thr Glu Met Asn Pro Asn Val Val Val Ile Ser Val Leu Ala
1               5                   10                  15

Ile Leu Ser Thr Leu Leu Ile Gly Leu Leu Leu Val Thr Leu Ile Ile
            20                  25                  30

Leu Arg Lys Lys His Leu Gln Met Ala Arg Glu Cys Gly Ala Gly Thr
        35                  40                  45

Phe Val Asn Phe Ala Ser Leu Glu Arg Asp Gly Lys Leu Pro Tyr Asn
    50                  55                  60

Trp Arg Arg Ser Ile Phe Ala Phe Leu Thr Leu Leu Pro Ser Cys Leu
65                  70                  75                  80

Trp Thr Asp Tyr Leu Leu Ala Phe Tyr Ile Asn Pro Trp Ser Lys Asn
                85                  90                  95

Gly Leu Lys Lys Arg Lys Leu Thr Asn Pro Val Gln Leu Asp Asp Phe
            100                 105                 110

Asp Ala Tyr Ile Lys Asp Met Ala Lys Asp Ser Asp Tyr Lys Phe Ser
        115                 120                 125

Leu Gln Phe Glu Glu Leu Lys Leu Ile Gly Leu Asp Ile Pro His Phe
    130                 135                 140

Ala Ala Asp Leu Pro Leu Asn Arg Cys Lys Asn Arg Tyr Thr Asn Ile
145                 150                 155                 160

Leu Pro Tyr Asp Phe Ser Arg Val Arg Leu Val Ser Met Asn Glu Glu
                165                 170                 175

Glu Gly Ala Asp Tyr Ile Asn Ala Asn Tyr Ile Pro Gly Tyr Asn Ser
            180                 185                 190

Pro Gln Glu Tyr Ile Ala Thr Gln Gly Pro Leu Pro Glu Thr Arg Asn
        195                 200                 205

Asp Phe Trp Lys Met Val Leu Gln Gln Lys Ser Gln Ile Ile Val Met
    210                 215                 220

Leu Thr Gln Cys Asn Glu Lys Arg Arg Val Lys Cys Asp His Tyr Trp
225                 230                 235                 240

Pro Phe Thr Glu Glu Pro Ile Ala Tyr Gly Asp Ile Thr Val Glu Met
                245                 250                 255

Ile Ser Glu Glu Glu Gln Asp Asp Trp Ala Cys Arg His Phe Arg Ile
            260                 265                 270

Asn Tyr Ala Asp Glu Met Gln Asp Val Met His Phe Asn Tyr Thr Ala
        275                 280                 285

Trp Pro Asp His Gly Val Pro Thr Ala Asn Ala Ala Glu Ser Ile Leu
    290                 295                 300

Gln Phe Val His Met Val Arg Gln Gln Ala Thr Lys Ser Lys Gly Pro
305                 310                 315                 320

Met Ile Ile His Cys Ser Ala Gly Val Gly Arg Thr Gly Thr Phe Ile
                325                 330                 335

Ala Leu Asp Arg Leu Leu Gln His Ile Arg Asp His Glu Phe Val Asp
            340                 345                 350

Ile Leu Gly Leu Val Ser Glu Met Arg Ser Tyr Arg Met Ser Met Val
        355                 360                 365

Gln Thr Glu Glu Gln Tyr Ile Phe Ile His Gln Cys Val Gln Leu Met
    370                 375                 380

Trp Met Lys Lys Lys Gln Gln Phe Cys Ile Ser Asp Val Ile Tyr Glu
385                 390                 395                 400

Asn Val Ser Lys Ser
                405

<210>  56
<211>  377
<212>  PRT
<213>  human

<400>  56

Met Val Thr Glu Met Asn Pro Asn Val Val Val Ile Ser Val Leu Ala
1               5                   10                  15

Ile Leu Ser Thr Leu Leu Ile Gly Leu Leu Leu Val Thr Leu Ile Ile
            20                  25                  30

Leu Arg Lys Lys His Leu Gln Met Ala Arg Glu Cys Gly Ala Gly Thr
        35                  40                  45

Phe Val Asn Phe Ala Ser Leu Glu Arg Asp Gly Lys Leu Pro Tyr Asn
    50                  55                  60

Trp Ser Lys Asn Gly Leu Lys Lys Arg Lys Leu Thr Asn Pro Val Gln
65                  70                  75                  80

Leu Asp Asp Phe Asp Ala Tyr Ile Lys Asp Met Ala Lys Asp Ser Asp
                85                  90                  95

Tyr Lys Phe Ser Leu Gln Phe Glu Glu Leu Lys Leu Ile Gly Leu Asp
            100                 105                 110

Ile Pro His Phe Ala Ala Asp Leu Pro Leu Asn Arg Cys Lys Asn Arg
        115                 120                 125

Tyr Thr Asn Ile Leu Pro Tyr Asp Phe Ser Arg Val Arg Leu Val Ser
    130                 135                 140

Met Asn Glu Glu Glu Gly Ala Asp Tyr Ile Asn Ala Asn Tyr Ile Pro
145                 150                 155                 160

Gly Tyr Asn Ser Pro Gln Glu Tyr Ile Ala Thr Gln Gly Pro Leu Pro
                165                 170                 175

Glu Thr Arg Asn Asp Phe Trp Lys Met Val Leu Gln Gln Lys Ser Gln
            180                 185                 190

Ile Ile Val Met Leu Thr Gln Cys Asn Glu Lys Arg Arg Val Lys Cys
        195                 200                 205

Asp His Tyr Trp Pro Phe Thr Glu Glu Pro Ile Ala Tyr Gly Asp Ile
    210                 215                 220

Thr Val Glu Met Ile Ser Glu Glu Glu Gln Asp Asp Trp Ala Cys Arg
225                 230                 235                 240

His Phe Arg Ile Asn Tyr Ala Asp Glu Met Gln Asp Val Met His Phe
                245                 250                 255

Asn Tyr Thr Ala Trp Pro Asp His Gly Val Pro Thr Ala Asn Ala Ala
            260                 265                 270

Glu Ser Ile Leu Gln Phe Val His Met Val Arg Gln Gln Ala Thr Lys
        275                 280                 285

Ser Lys Gly Pro Met Ile Ile His Cys Ser Ala Gly Val Gly Arg Thr
    290                 295                 300

Gly Thr Phe Ile Ala Leu Asp Arg Leu Leu Gln His Ile Arg Asp His
305                 310                 315                 320

Glu Phe Val Asp Ile Leu Gly Leu Val Ser Glu Met Arg Ser Tyr Arg
                325                 330                 335

Met Ser Met Val Gln Thr Glu Glu Gln Tyr Ile Phe Ile His Gln Cys
            340                 345                 350

Val Gln Leu Met Trp Met Lys Lys Lys Gln Gln Phe Cys Ile Ser Asp
        355                 360                 365

Val Ile Tyr Glu Asn Val Ser Lys Ser
    370                 375

<210> 57
<211> 1226
<212> PRT
<213> mouse

<400> 57

Met Gly His Leu Pro Arg Gly Thr Leu Gly Gly Arg Arg Leu Leu Pro
1               5                   10                  15

Leu Leu Gly Leu Phe Val Leu Leu Lys Ile Val Thr Thr Phe His Val
            20                  25                  30

Ala Val Gln Asp Asp Asn Asn Ile Val Val Ser Leu Glu Ala Ser Asp
        35                  40                  45

Ile Val Ser Pro Ala Ser Val Tyr Val Val Arg Val Ala Gly Glu Ser
    50                  55                  60

Lys Asn Tyr Phe Phe Glu Phe Glu Glu Phe Asn Ser Thr Leu Pro Pro
65                  70                  75                  80

Pro Val Val Phe Lys Ala Thr Tyr His Gly Leu Tyr Tyr Ile Ile Thr
                85                  90                  95

Leu Val Val Val Asn Gly Asn Val Val Thr Lys Pro Ser Arg Ser Ile
            100                 105                 110

Thr Val Leu Thr Lys Pro Leu Pro Val Thr Ser Val Ser Ile Tyr Asp
        115                 120                 125

Tyr Lys Pro Ser Pro Glu Thr Gly Val Leu Phe Glu Ile His Tyr Pro
    130                 135                 140

Glu Lys Tyr Asn Val Phe Ser Arg Val Asn Ile Ser Tyr Trp Glu Gly
145                 150                 155                 160

Arg Asp Phe Arg Thr Met Leu Tyr Lys Asp Phe Phe Lys Gly Lys Thr
                165                 170                 175

Val Phe Asn His Trp Leu Pro Gly Leu Cys Tyr Ser Asn Ile Thr Phe
            180                 185                 190

Gln Leu Val Ser Glu Ala Thr Phe Asn Lys Ser Thr Leu Val Glu Tyr
        195                 200                 205

Ser Gly Val Ser His Glu Pro Lys Gln His Arg Thr Ala Pro Tyr Pro
    210                 215                 220

Pro Arg Asn Ile Ser Val Arg Phe Val Asn Leu Asn Lys Asn Asn Trp
225                 230                 235                 240

Glu Glu Pro Ser Gly Ser Phe Pro Glu Asp Ser Phe Ile Lys Pro Pro
                245                 250                 255

Gln Asp Ser Ile Gly Arg Asp Arg Arg Phe His Phe Pro Glu Glu Thr
            260                 265                 270

Pro Glu Thr Pro Pro Ser Asn Val Ser Ser Gly Ser Pro Pro Ser Asn
        275                 280                 285

Val Ser Ser Ala Trp Pro Asp Pro Asn Ser Thr Asp Tyr Glu Ser Thr
    290                 295                 300

Ser Gln Pro Phe Trp Trp Asp Ser Ala Ser Ala Ala Pro Glu Asn Glu
305                 310                 315                 320

Glu Asp Phe Val Ser Ala Leu Pro Ala Asp Tyr Asp Thr Glu Thr Thr
                325                 330                 335

Leu Asp Arg Thr Glu Lys Pro Thr Ala Asp Pro Phe Ser Ala Phe Pro
            340                 345                 350

Val Gln Met Thr Leu Ser Trp Leu Pro Pro Lys Pro Pro Thr Ala Phe
        355                 360                 365

Asp Gly Phe Asn Ile Leu Ile Glu Arg Glu Glu Asn Phe Thr Asp Tyr
    370                 375                 380

Leu Thr Val Asp Glu Glu Ala His Glu Phe Val Ala Glu Leu Lys Glu
385                 390                 395                 400

Pro Gly Lys Tyr Lys Leu Ser Val Thr Thr Phe Ser Ser Ser Gly Ala
                405                 410                 415

Cys Glu Thr Arg Lys Ser Gln Ser Ala Lys Ser Leu Ser Phe Tyr Ile
            420                 425                 430

Ser Pro Thr Gly Glu Trp Ile Glu Glu Leu Thr Glu Lys Pro Gln His
        435                 440                 445

Val Ser Val His Val Leu Ser Ser Thr Thr Ala Leu Met Ser Trp Thr
    450                 455                 460

Ser Ser Gln Glu Asn Tyr Asn Ser Thr Ile Val Ser Val Val Ser Leu
465                 470                 475                 480

Thr Cys Gln Lys Gln Lys Glu Ser Gln Arg Leu Glu Lys Gln Tyr Cys
                485                 490                 495

Thr Gln Val Asn Ser Ser Lys Pro Val Ile Glu Asn Leu Val Pro Gly
            500                 505                 510

Ala Gln Tyr Gln Val Val Met Tyr Leu Arg Lys Gly Pro Leu Ile Gly
        515                 520                 525

Pro Pro Ser Asp Pro Val Thr Phe Ala Ile Val Pro Thr Gly Ile Lys
    530                 535                 540

Asp Leu Met Leu Tyr Pro Leu Gly Pro Thr Ala Val Val Leu Ser Trp
545                 550                 555                 560

Thr Arg Pro Ile Leu Gly Val Phe Arg Lys Tyr Val Val Glu Met Phe
                565                 570                 575

Tyr Phe Asn Pro Thr Thr Met Thr Ser Glu Trp Thr Thr Tyr Tyr Glu
            580                 585                 590

Ile Ala Ala Thr Val Ser Leu Thr Ala Ser Val Arg Ile Ala Ser Leu
        595                 600                 605

Leu Pro Ala Trp Tyr Tyr Asn Phe Arg Val Thr Met Val Thr Trp Gly
    610                 615                 620

Asp Pro Glu Leu Ser Cys Cys Asp Ser Ser Thr Ile Ser Phe Ile Thr
625                 630                 635                 640

Ala Pro Val Ala Pro Glu Ile Thr Ser Val Glu Tyr Phe Asn Ser Leu
                645                 650                 655

Leu Tyr Ile Ser Trp Thr Tyr Gly Asp Ala Thr Thr Asp Leu Ser His
            660                 665                 670

Ser Arg Met Leu His Trp Met Val Val Ala Glu Gly Arg Lys Lys Ile
        675                 680                 685

Lys Lys Ser Val Thr Arg Asn Val Met Thr Ala Ile Leu Ser Leu Pro
    690                 695                 700

Pro Gly Asp Ile Tyr Asn Leu Ser Val Thr Ala Cys Thr Glu Arg Gly
705                 710                 715                 720

Ser Asn Thr Ser Leu Pro Arg Leu Val Lys Leu Glu Pro Ala Pro Pro
                725                 730                 735

Lys Ser Leu Phe Ala Val Asn Lys Thr Gln Thr Ser Val Thr Leu Leu
            740                 745                 750

Trp Val Glu Glu Gly Val Ala Asp Phe Phe Glu Val Phe Cys Gln Gln
        755                 760                 765

Leu Gly Ser Gly His Asn Gly Lys Leu Gln Glu Pro Val Ala Val Ser
    770                 775                 780

Ser His Val Val Thr Ile Ser Ser Leu Leu Pro Ala Thr Ala Tyr Asn
785                 790                 795                 800

Cys Ser Val Thr Ser Phe Ser His Asp Thr Pro Ser Val Pro Thr Phe
                805                 810                 815

Ile Ala Val Ser Thr Met Val Thr Glu Val Asn Pro Asn Val Val Val
            820                 825                 830

Ile Ser Val Leu Ala Ile Leu Ser Thr Leu Leu Ile Gly Leu Leu Leu
        835                 840                 845

Val Thr Leu Val Ile Leu Arg Lys Lys His Leu Gln Met Ala Arg Glu
    850                 855                 860

Cys Gly Ala Gly Thr Phe Val Asn Phe Ala Ser Leu Glu Arg Glu Gly
865                 870                 875                 880

Lys Leu Pro Tyr Ser Trp Arg Arg Ser Val Phe Ala Leu Leu Thr Leu
                885                 890                 895

Leu Pro Ser Cys Leu Trp Thr Asp Tyr Leu Leu Ala Phe Tyr Ile Asn
            900                 905                 910

Pro Trp Ser Lys Asn Gly Leu Lys Lys Arg Lys Leu Thr Asn Pro Val
        915                 920                 925

Gln Leu Asp Asp Phe Asp Ser Tyr Ile Lys Asp Met Ala Lys Asp Ser
    930                 935                 940

Asp Tyr Lys Phe Ser Leu Gln Phe Glu Glu Leu Lys Leu Ile Gly Leu
945                 950                 955                 960

Asp Ile Pro His Phe Ala Ala Asp Leu Pro Leu Asn Arg Cys Lys Asn
                965                 970                 975

Arg Tyr Thr Asn Ile Leu Pro Tyr Asp Phe Ser Arg Val Arg Leu Val
            980                 985                 990

Ser Met Asn Glu Glu Glu Gly Ala Asp Tyr Ile Asn Ala Asn Tyr Ile
        995                 1000                1005

Pro Gly Tyr Asn Ser Pro Gln Glu Tyr Ile Ala Thr Gln Gly Pro
    1010                1015                1020

Leu Pro Glu Thr Arg Asn Asp Phe Trp Lys Met Val Leu Gln Gln
    1025                1030                1035

Lys Ser His Ile Ile Val Met Leu Thr Gln Cys Asn Glu Lys Arg
    1040                1045                1050

Arg Val Lys Cys Asp His Tyr Trp Pro Phe Thr Glu Glu Pro Ile
    1055                1060                1065

Ala Tyr Gly Asp Ile Thr Val Glu Met Val Ser Glu Glu Glu Glu
    1070                1075                1080

Glu Asp Trp Ala Ser Arg His Phe Arg Ile Asn Tyr Ala Asp Glu
    1085                1090                1095

Ala Gln Asp Val Met His Phe Asn Tyr Thr Ala Trp Pro Asp His
    1100                1105                1110

Gly Val Pro Pro Ala Asn Ala Ala Glu Ser Ile Leu Gln Phe Val
    1115                1120                1125

Phe Thr Val Arg Gln Gln Ala Ala Lys Ser Lys Gly Pro Met Ile
    1130                1135                1140

Ile His Cys Ser Ala Gly Val Gly Arg Thr Gly Thr Phe Ile Ala
    1145                1150                1155

Leu Asp Arg Leu Leu Gln His Ile Arg Asp His Glu Phe Val Asp
    1160                1165                1170

Ile Leu Gly Leu Val Ser Glu Met Arg Ser His Arg Met Ser Met
    1175                1180                1185

Val Gln Thr Glu Glu Gln Tyr Ile Phe Ile His Gln Cys Val Gln
    1190                1195                1200

Leu Met Trp Leu Arg Lys Lys Gln Gln Phe Cys Ile Ser Asp Val
    1205                1210                1215

Ile Tyr Glu Asn Val Ser Lys Ser
    1220                1225

<210> 58
<211> 1216
<212> PRT
<213> rat

<400> 58

Met Gly His Leu Pro Arg Glu Arg Arg Gly Gly Arg Arg Leu Leu Pro
1               5                   10                  15

Leu Leu Gly Leu Phe Val Leu Leu Lys Ile Val Ser Thr Phe His Val
            20                  25                  30

Thr Val Arg Asp Asp Asn Asn Ile Val Val Ser Leu Glu Ala Ser Asp
        35                  40                  45

Val Val Ser Pro Thr Ser Val Tyr Val Val Arg Val Ala Gly Glu Ser
    50                  55                  60

Lys Asn Tyr Phe Phe Glu Phe Glu Glu Ser Asn Gly Thr Leu Pro Pro
65                  70                  75                  80

Pro Val Val Phe Lys Ala Thr Tyr His Gly Leu Tyr Tyr Ile Ile Ser
                85                  90                  95

Leu Val Val Val Ser Gly Asn Val Val Thr Lys Pro Ser Arg Ser Ile
            100                 105                 110

Thr Val Leu Thr Lys Pro Leu Pro Val Thr Ser Val Ser Ile Tyr Asp
        115                 120                 125

Tyr Lys Pro Ser Pro Glu Thr Gly Val Leu Phe Glu Ile His Tyr Pro
    130                 135                 140

Glu Lys Tyr Asn Val Phe Ser Arg Val Asn Ile Ser Tyr Trp Glu Gly
145                 150                 155                 160

Lys Asp Phe Arg Thr Met Leu Tyr Lys Asp Phe Leu Lys Gly Lys Thr
                165                 170                 175

Val Phe Asn His Trp Leu Pro Gly Ile Cys Tyr Ser Asn Ile Thr Phe
            180                 185                 190

Gln Leu Val Ser Glu Ala Thr Phe Asn Lys Ser Thr Leu Val Glu Cys
        195                 200                 205

Ser Gly Val Ser His Glu Pro Lys Gln His Arg Thr Ala Pro Tyr Pro
    210                 215                 220

Pro Arg Asn Ile Ser Val Arg Phe Val Asn Leu Asn Lys Asn Asn Trp
225                 230                 235                 240

Glu Glu Pro Ser Gly Ser Phe Pro Glu Asp Ser Phe Ile Lys Pro Gln
                245                 250                 255

Asp Ala Ile Gly Arg Asp Arg Pro Phe His Phe Pro Glu Glu Thr Pro

260                    265                    270

Glu Thr Pro Ala Ser Asn Ala Ser Ser Gly Trp Pro Asp Pro Asn Gly
        275                 280                 285

Thr Glu Tyr Glu Ser Thr Ser Gln Pro Tyr Trp Trp Asp Ser Ala Ser
    290                 295                 300

Ala Ala Pro Glu Asn Glu Glu Asp Phe Val Ser Val Leu Pro Ala Asp
305                 310                 315                 320

Tyr Asp Thr Glu Ala Thr Leu Gly Arg Thr Glu Lys Pro Thr Ala Asp
                325                 330                 335

Pro Phe Ser Ala Phe Pro Val Gln Met Thr Leu Ser Trp Leu Pro Pro
            340                 345                 350

Lys Pro Pro Thr Ala Phe Asp Gly Phe Asn Ile Leu Ile Glu Arg Glu
        355                 360                 365

Gly Asn Phe Thr Asp Tyr Leu Thr Val Asp Glu Glu Ala His Glu Phe
    370                 375                 380

Val Ala Glu Leu Lys Glu Pro Gly Lys Tyr Arg Leu Ser Val Thr Thr
385                 390                 395                 400

Phe Ser Ser Ser Gly Ala Cys Glu Thr Arg Lys Ser Gln Ser Ala Lys
                405                 410                 415

Ser Leu Ser Phe Tyr Ile Ser Pro Thr Gly Glu Trp Ile Glu Glu Leu
            420                 425                 430

Thr Glu Lys Pro Gln His Val Ser Val His Val Leu Ser Ser Thr Thr
        435                 440                 445

Ala Leu Leu Ser Trp Thr Ser Ser Gln Glu Asn Tyr Asn Ser Thr Ile
    450                 455                 460

Val Ser Val Val Ser Leu Thr Cys Gln Lys Gln Lys Glu Ser Gln Arg
465                 470                 475                 480

Leu Glu Lys Gln Tyr Cys Thr Gln Val Asn Ser Ser Lys Pro Val Ile
                485                 490                 495

Glu Asn Leu Val Pro Gly Ala Gln Tyr Gln Val Val Met Tyr Leu Arg
            500                 505                 510

Lys Gly Pro Leu Ile Gly Pro Pro Ser Asp Pro Val Thr Phe Ala Ile
        515                 520                 525

Val Pro Thr Gly Ile Lys Asp Leu Met Leu Tyr Pro Leu Gly Pro Thr

530                 535                 540

Ala Val Val Leu Ser Trp Thr Arg Pro Tyr Leu Gly Val Phe Arg Lys
545                 550                 555                 560

Tyr Val Val Glu Met Phe Tyr Phe Asn Pro Thr Thr Met Thr Ser Glu
                565                 570                 575

Trp Thr Thr Tyr Tyr Glu Ile Ala Ala Thr Val Ser Leu Thr Ser Ser
            580                 585                 590

Val Arg Ile Ala Asn Leu Leu Pro Ala Trp Tyr Tyr Asn Phe Arg Val
        595                 600                 605

Thr Met Val Thr Trp Gly Asp Pro Glu Leu Ser Cys Cys Asp Ser Ser
    610                 615                 620

Thr Ile Ser Phe Ile Thr Ala Pro Val Ala Pro Glu Ile Thr Ser Val
625                 630                 635                 640

Glu Tyr Phe Asn Ser Leu Leu Tyr Ile Ser Trp Thr Tyr Gly Asp Ala
                645                 650                 655

Thr Thr Asp Leu Ser His Ser Arg Met Leu His Trp Met Val Val Ala
            660                 665                 670

Glu Gly Arg Arg Lys Ile Lys Lys Ser Val Thr Arg Asn Val Met Thr
        675                 680                 685

Ala Ile Leu Ser Leu Pro Pro Gly Asp Ile Tyr Asn Leu Ser Val Thr
    690                 695                 700

Ala Cys Thr Glu Arg Gly Ser Asn Thr Ser Met Leu Arg Leu Val Lys
705                 710                 715                 720

Leu Glu Pro Ala Pro Pro Lys Ser Leu Phe Ala Val Asn Lys Thr Gln
                725                 730                 735

Thr Ser Val Thr Leu Leu Trp Val Glu Glu Gly Val Ala Asp Phe Phe
            740                 745                 750

Glu Val Leu Cys Gln Lys Leu Gly Ser Gly His Asp Gly Lys Leu Gln
        755                 760                 765

Glu Pro Val Ala Val Ser Ser His Val Val Thr Ile Ser Ser Leu Leu
    770                 775                 780

Pro Ala Thr Ala Tyr Asn Cys Ser Val Thr Ser Phe Ser His Asp Ser
785                 790                 795                 800

Pro Ser Val Pro Thr Phe Ile Ala Val Ser Thr Met Val Thr Glu Val

                805                 810                 815

Asn Pro Asn Val Val Val Ile Ser Val Leu Ala Ile Leu Ser Ile Leu
            820                 825                 830

Leu Ile Gly Leu Leu Leu Val Thr Leu Val Ile Leu Arg Lys Lys His
        835                 840                 845

Leu Gln Met Ala Arg Glu Cys Gly Ala Gly Thr Phe Val Asn Phe Ala
    850                 855                 860

Ser Leu Glu Arg Glu Gly Lys Leu Pro Tyr Ser Trp Arg Arg Ser Val
865                 870                 875                 880

Phe Thr Phe Leu Thr Leu Leu Pro Ser Cys Leu Trp Thr Asp Tyr Leu
                885                 890                 895

Leu Ala Phe Tyr Ile Asn Pro Trp Ser Lys Asn Gly Leu Lys Lys Arg
            900                 905                 910

Lys Leu Thr Asn Pro Val Gln Leu Asp Asp Phe Asp Ser Tyr Ile Lys
        915                 920                 925

Asp Met Ala Lys Asp Ser Asp Tyr Lys Phe Ser Leu Gln Phe Glu Glu
    930                 935                 940

Leu Lys Leu Ile Gly Leu Asp Ile Pro His Phe Ala Ala Asp Leu Pro
945                 950                 955                 960

Leu Asn Arg Cys Lys Asn Arg Tyr Thr Asn Ile Leu Pro Tyr Asp Phe
                965                 970                 975

Ser Arg Val Arg Leu Val Ser Met Asn Glu Glu Glu Gly Ala Asp Tyr
            980                 985                 990

Ile Asn Ala Asn Tyr Ile Pro Gly Tyr Asn Ser Pro Gln Glu Tyr Ile
        995                 1000                1005

Ala Thr Gln Gly Pro Leu Pro Glu Thr Arg Asn Asp Phe Trp Lys
    1010                1015                1020

Met Val Leu Gln Gln Lys Ser Arg Ile Ile Val Met Leu Thr Gln
    1025                1030                1035

Cys Asn Glu Lys Arg Arg Val Lys Cys Asp His Tyr Trp Pro Phe
    1040                1045                1050

Thr Glu Glu Pro Ile Ala Tyr Gly Asp Ile Thr Val Glu Met Val
    1055                1060                1065

Ser Glu Glu Glu Gln Glu Asp Trp Ala Ser Arg His Phe Arg Ile

1070                    1075                    1080

Asn Tyr Ala Asp Glu Ala Gln Asp Val Met His Phe Asn Tyr Thr
    1085                    1090                    1095

Ala Trp Pro Asp His Gly Val Pro Pro Ala Asn Ala Ala Glu Ser
    1100                    1105                    1110

Ile Leu Gln Phe Val Tyr Thr Val Arg Gln Gln Ala Thr Lys Ser
    1115                    1120                    1125

Lys Gly Pro Met Ile Ile His Cys Ser Ala Gly Val Gly Arg Thr
    1130                    1135                    1140

Gly Thr Phe Ile Ala Leu Asp Arg Leu Leu Gln His Ile Arg Asp
    1145                    1150                    1155

His Glu Phe Val Asp Ile Leu Gly Leu Val Ser Glu Met Arg Ser
    1160                    1165                    1170

Tyr Arg Met Ser Met Val Gln Thr Glu Glu Gln Tyr Ile Phe Ile
    1175                    1180                    1185

His Gln Cys Val Gln Leu Met Trp Leu Lys Lys Lys Gln Gln Phe
    1190                    1195                    1200

Cys Ile Ser Asp Val Ile Tyr Glu Asn Val Ser Lys Ser
    1205                    1210                    1215

<210>  59
<211>  3367
<212>  DNA
<213>  human

<400>  59
gcccccgccc ggcccgcccc gctctcctag tcccttgcaa cctggcgctg ccatccgggc      60

cactgtccca ggtcccggcc cggagctatg gagcggcgct ggcccctggg gctagggctg     120

gtgctgctgc tctgcgcccc gctgcccccg ggggcgcgcg ccaaggaagt tactctgatg     180

gacacaagca aggcacaggg agagctgggc tggctgctgg atcccccaaa agatgggtgg     240

agtgaacagc aacagatact gaatgggaca cccctgtaca tgtaccagga ctgcccaatg     300

caaggacgca gagacactga ccactggctt cgctccaatt ggatctaccg cggggaggag     360

gcttcccgcg tccacgtgga gctgcagttc accgtgcggg actgcaagag tttccctggg     420

ggagccgggc ctctgggctg caaggagacc ttcaaccttc tgtacatgga gagtgaccag     480

gatgtgggca ttcagctccg acggcccttg ttccagaagg taaccacggt ggctgcagac     540

cagagcttca ccattcgaga ccttgcgtct ggctccgtga agctgaatgt ggagcgctgc     600

tctctgggcc gcctgacccg ccgtggcctc tacctcgctt tccacaaccc gggtgcctgt     660

gtggccctgg tgtctgtccg ggtcttctac cagcgctgtc ctgagaccct gaatggcttg     720

gcccaattcc cagacactct gcctggcccc gctgggttgg tggaagtggc ggggacctgc 780
ttgccccacg cgcgggccag ccccaggccc tcaggtgcac cccgcatgca ctgcagccct 840
gatggcgagt ggctggtgcc tgtaggacgg tgccactgtg agcctggcta tgaggaaggt 900
ggcagtggcg aagcatgtgt tgcctgccct agcggctcct accggatgga catggacaca 960
ccccattgtc tcacgtgccc ccagcagagc actgctgagt ctgagggggc caccatctgt 1020
acctgtgaga gcggccatta cagagctccc ggggagggcc cccaggtggc atgcacaggt 1080
cccccctcgg ccccccgaaa cctgagcttc tctgcctcag ggactcagct ctccctgcgt 1140
tgggaacccc cagcagatac ggggggacgc caggatgtca gatacagtgt gaggtgttcc 1200
cagtgtcagg gcacagcaca ggacgggggg ccctgccagc cctgtggggt gggcgtgcac 1260
ttctcgccgg gggcccgggg gctcaccaca cctgcagtgc atgtcaatgg ccttgaacct 1320
tatgccaact acacctttaa tgtggaagcc caaaatggag tgtcagggct gggcagctct 1380
ggccatgcca gcacctcagt cagcatcagc atggggcatg cagagtcact gtcaggcctg 1440
tctctgagac tggtgaagaa agaaccgagg caactagagc tgacctgggc ggggtcccgg 1500
ccccgaagcc ctggggcgaa cctgacctat gagctgcacg tgctgaacca ggatgaagaa 1560
cggtaccaga tggttctaga acccagggtc ttgctgacag agctgcagcc tgacaccaca 1620
tacatcgtca gagtccgaat gctgacccca ctgggtcctg gccctttctc ccctgatcat 1680
gagtttcgga ccagcccacc agtgtccagg ggcctgactg gaggagagat tgtagccgtc 1740
atctttgggc tgctgcttgg tgcagccttg ctgcttggga ttctcgtttt ccggtccagg 1800
agagcccagc ggcagaggca gcagaggcag cgtgaccgcg ccaccgatgt ggatcgagag 1860
gacaagctgt ggctgaagcc ttatgtggac ctccaggcat acgaggaccc tgcacaggga 1920
gccctggact ttacccggga gcttgatcca gcgtggctga tggtggacac tgtcatagga 1980
gaaggagagt ttggggaagt gtatcgaggg accctgaggc tccccagcca ggactgcaag 2040
actgtggcca ttaagacctt aaaagacaca tccccaggtg gccagtggtg gaacttcctt 2100
cgagaggcaa ctatcatggg ccagtttagc cacccgcata ttctgcatct ggaaggcgtc 2160
gtcacaaagc gaaagccgat catgatcatc acagaattta tggagaatgg agccctggat 2220
gccttcctga gggagcggga ggaccagctg gtccctgggc agctagtggc catgctgcag 2280
ggcatagcat ctggcatgaa ctacctcagt aatcacaatt atgtccaccg ggacctggct 2340
gccagaaaca tcttggtgaa tcaaaacctg tgctgcaagg tgtctgactt tggcctgact 2400
cgcctcctgg atgactttga tggcacatac gaaacccagg gaggaaagat ccctatccgt 2460
tggacagccc ctgaagccat tgcccatcgg atcttcacca cagccagcga tgtgtggagc 2520
tttgggattg tgatgtggga ggtgctgagc tttggggaca agccttatgg ggagatgagc 2580
aatcaggagg ttatgaagag cattgaggat gggtaccggt tgccccctcc tgtggactgc 2640
cctgcccctc tgtatgagct catgaagaac tgctgggcat atgaccgtgc ccgccggcca 2700
cacttccaga agcttcaggc acatctggag caactgcttg ccaaccccca ctccctgcgg 2760

accattgcca actttgaccc caggatgact cttcgcctgc ccagcctgag tggctcagat 2820

gggatcccat atcgaaccgt ctctgagtgg ctcgagtcca tacgcatgaa acgctacatc 2880

ctgcacttcc actcggctgg gctggacacc atggagtgtg tgctggagct gaccgctgag 3000

gacctgacgc agatgggaat cacactgccc gggcaccaga agcgcattct ttgcagtatt 3000

cagggattca aggactgatc cctcctctca ccccatgccc aatcagggtg caaggagcaa 3060

ggacggggcc aaggtcgctc atggtcactc cctgcgcccc ttcccacaac ctgccagact 3120

aggctatcgg tgctgcttct gcccactttc aggagaaccc tgctctgcac cccagaaaac 3180

ctctttgttt taaaagggag gtgggggtag aagtaaaagg atgatcatgg gagggagctg 3240

aggggttaat atatatacat acatacacat atatatattt ttgtaaataa acaggaactg 3300

attttctgcc tccatcccac ccatgagggc tgcaggcact acaaaagagc tgactactga 3360

gaaaaaa 3367

<210> 60
<211> 3275
<212> DNA
<213> mouse

<400> 60
gagtccctcg cgacctggcc cagccgtgag ggccgtggtc ccaggtcccg gccggcgcca 60

tggagcggcg ctggcccctg ggcttgcatt gctgctgctt gctgctctgc gccccgctgc 120

ccccgggggc gcgcgccgag gaagtcactc taatggacac aagcacagca caaggagagc 180

tgggctggct tctggatccc ccagagactg ggtggagtga ggtgcaacaa atgctaaacg 240

ggacacccct gtacatgtac caagactgcc caatacagga aggtggggac actgaccact 300

ggcttcgctc caattggatc taccgcggag aggaagcttc acgcatctac gtggagctgc 360

agttcaccgt gcgggactgt aagagtttcc cagggggagc tgggcctctg ggatgcaaag 420

agaccttcaa ccttttctac atggagagtg accaggatgt gggcattcag ctccgacgac 480

ctttgttcca aaaggtaaca actgtggcag cagaccagag cttcaccatc agagacctgg 540

catctggctc tgtaaagctg aatgtagaac gctgctcgtt gggccacctc acccgccgtg 600

gcctctactt agctttccac aacccgggtt cctgtgtggc gctagtgtct gtaagggtgt 660

tctaccagcg ctgtgccgag accgtgcatg gcttggccca cttccctgac actctccctg 720

gacctggagg gttggttgaa gtagctggaa cgtgcctctc ccatgcacag atcagcttgg 780

ggtcctcagg tacaccacga atgcactgca gccctgatgg cgagtggctg gtgcctgtgg 840

gtcagtgcca gtgcgagcct ggctatgaag aaagcagtgg aaatgtggga tgcactgcct 900

gtcctactgg tttctatcga gtggacatga atacactccg ttgtctcaag tgcccccaac 960

atagcatagc agagtctgag gggtctacca tctgtacctg tgagaatgga cattatcgag 1020

cccctgggga gggtccccag gtagcatgca cacgtccccc atcggctccc caaaatctga 1080

gcttctccac atcagggact caactctccc tgcgctggga gccccccaga gatacagggg 1140

gacgccatga tatcagatac agcgtggagt gcttgcagtg tcggggcatt gcacaggatg 1200
ggggtccctg ccaaccctgt ggaaaaggtg tgcacttttc cccggctgct tccgggctca 1260
ccacatctac cgtgcaagtg caaggcctcg agccttacgc caactacaca tttaccgtca 1320
aatcccaaaa cagagtgtca ggactggaca gttccagccc tagcagcgcc tccctcagta 1380
tcaacatggg gcacgcagag tcactctctg gcctgtcact gaagctggtg aagaaagaac 1440
cgaggcagct ggagctgact tgggcagggt cccgaccccg aaatcctgga gggaatctga 1500
gctatgagct gcacgtgctg aatcaggacg aagaatggca ccagatggtg ttggaaccca 1560
gggtcttgct gacaaaactt cagccagata ccacatacat tgtcagagtg cgaacactga 1620
ccccactggg gcctggccct ttctcccctg accatgagtt tcggacaagc ccaccagttt 1680
ccagaagcct gaccggagga gagattgtgg ccgtcatctt tggattgctg cttggaatag 1740
ctctgctgat cgggatttat gtcttccgtt caaggagagg ccagagacag agacagcaga 1800
ggcagcgtga acgcaccacc aatgtcgatc gagaggacaa gctgtggcta aaaccctatg 1860
tggacctcca ggcctatgag gaccctgcac agggagcctt agactttgcc caggaactgg 1920
acccagcctg gctgattgtg gacactgtca taggagaagg ggagtttggt gaagtgtatc 1980
ggggagccct gagactcccc agccaagatt gcaagactgt ggccattaag accttgaaag 2040
atacatcccc agatggctac tggtggaatt tccttcgaga ggcaactatc atgggccagt 2100
tcaaccaccc acacattcta cgcctagaag gtgtcatcac aaaaagaaag cccatcatga 2160
tcatcacaga atttatggaa aatggagccc tggatgcctt tctgaaggaa cgggaggacc 2220
aactagctcc tggtcagcta gtggctatgc tactgggcat agcatcaggc atgaactgcc 2280
tcagtggcca caattatgtc catagagacc tggctgccag gaacatcttg gtgaatcaga 2340
acctgtgctg caaggtgtct gactttggct tgacccgcct cctggatgac tttgacggca 2400
cctatgaaac ccagggagga aagatcccca tccgatggac agccccagaa gctattgccc 2460
atcggatctt caccacagcc agtgatgtgt ggagctttgg gattgtaatg tgggaggtgt 2520
tgagttttgg cgacaaaccc tatggggaga tgagcaacca agaggtaatg aaaagcattg 2580
aagatgggta ccggttgccc cctcctgtgg actgtcctgc ccctctctat gaactcatga 2640
agaactgctg ggcttacgat cgtgcccgtc gaccccactt cctccagctg caggcacatc 2700
tggaacagtt gcttactgac ccccattccc taaggacaat tgccaacttt gaccctaggg 2760
tgaccttacg cctgcccagc ctgagtggct ctgatgggat cccttatcga agtgtctctg 2820
agtggcttga atccatacgc atgaagcgct acatcctgca cttccgttcg gctgggctgg 2880
acaccatgga gtgtgtgctg gagctgacgg ctgaggacct gacgcagatg ggaataacgt 2940
tgccagggca ccagaaacga attctctgca gtattcaggg atttaaggac tgagcatcca 3000
ctgaaaagat gctccagccc tctgcctgcc tccattagca aggacggggc tacagtcaac 3060
tccctgggcc tttcctcagc ctacgaaatg taggctattg gtgctgctcc tgcccagtca 3120
atcagaactc tgcctttgaa ccaaggagcc tttgtttata aggggggtgg atgggtacaa 3180

gtgaagggga ctgtgggtgg gttctggggg agggtttaat atatacactt acatatgcat 3240

tatctatttt tgtaaataaa caagagttga gtttg 3275

<210> 61
<211> 3884
<212> DNA
<213> rat

<400> 61
cagtccctcg cgacctggcc cagccgtagg ggccgtggtc ccaggtcccg gccggcgcca 60

tggagcggcg ctggcccctg gggctcgcac tgctgctgct gctgctctgc gccccgctgc 120

ccccgggggc gcgcgccgag gaagtcactt taatggacac gagcacagca caaggagagt 180

tgggctggct tctggatccc ccagagactg ggtggagtga ggtgcaacaa atgctgaacg 240

ggacacccct gtacatgtac caagactgcc caatacagga aggtggagat actgaccact 300

ggcttcgctc caactggatc taccgaggag aggaagcctc acgtgtccac gtagagctgc 360

agttcactgt gcgggactgt aaaagtttcc cagggggagc tgggcctctg ggatgcaaag 420

agaccttcaa ccttttctac atggagagtg accaggatgt gggcattcaa ctccgacggc 480

ctttgttcca aaaggtaaca actgtggcag cagaccagag cttcaccatc agagacctgg 540

cctctggctc tgtaaagctg aacgtagagc actgctcctt gggtcacctc acccgccgtg 600

gtctctacct agctttccac aacccgggat cctgtgtggc gctcgtatct gtaagggtgt 660

tctaccagcg ctgcgcagag actgtgcatg gcttggccca cttccctcac actctccctg 720

gacctacagg gttggtagaa gtagctggaa cctgcctctc ccatgcacag atcagcctgg 780

ggtcctcagg tacaccacgc atgcattgca gccctgatgg cgagtggttg gtgcctgtgg 840

gtcagtgcca gtgcgagcct ggctatgagg aaagcggtgg aagtgtggga tgcactgcct 900

gtcctactgg tttctatcga atggacatga atactctcca ctgtctcaag tgcccccaac 960

atagcgtagc agagtctgag gggtctacca tctgtacctg tgagaatgga cattatcgag 1020

cccctgggga gggcccccag gtagcatgca cgcgtccccc atcagctccc caaaatctga 1080

gcttctctgc atcaggcact cagctctccc tacgctggga gcccccaaga gatacagggg 1140

gacgccaaga tatcagatac agtgtggaat gcttgcagtg tcggggcatt gcacaggatg 1200

ggggtccctg ccaaccctgt ggaaaaggtg tgcacttttc cccggcagct tccgggctca 1260

ccgcatctac tgtgcaagtt gaaggcctcg agccttatgc caactacacg tttaccatca 1320

aatcccagaa cagagtgtcg ggactggaca gttccagccc tagcagcgct tctctcagta 1380

tcaacatggg gcaagcagag tcactctctg gcctgtcact gaagctggtg aagaaagagc 1440

cgaggcagct ggagctgact tgggcagggt cccgactcca aaaccctggg gggaatctga 1500

gctatgagct gcacgtgctg aaccaggatg aagaatggca ccaaatggtg ctggaaccca 1560

gggtcttgct gacaaaactt cagccagata ccacatacat tgtcagagta cgaacactga 1620

ccccactggg gcctggccct ttctcccctg accatgagtt ccggacaagc ccaccagttt 1680

ccagaagcct gactggagga gagattgtgg ccatcatctt tggattgctg cttggaatag 1740

ctctgctgat cgggatttat gtcttccgtt caaggagagg ccagcgacag agacagcaga 1800
ggcagcgtga acgcaccacc aatgtcgatc gagaggacaa gctgtggcta aaaccctatg 1860
tggacctcca ggcctatgag gaccctgcac agggagcctt agactttgcc caggaactgg 1920
accctgcctg gctgattgtg gacactgtca taggagaagg ggagtttggg gaagtgtatc 1980
ggggagccct gagaatcccc agccaagact gcaagaccgt ggccattaag accttgaaag 2040
acacatcccc agatggctac tggtggaatt tccttcgaga agcaactatc atgggccagt 2100
tcaaccaccc acacattcta cgcctagaag gtgtcatcac gaaaagaaag ccgatcatga 2160
tcatcacaga atttatggaa aatggagccc ttgatgcctt tctgaaggaa cgggaggacc 2220
agctagttcc tggtcagcta gtggctatgc ttctgggcat agcatcaggc atgaactacc 2280
tcagtggcca caattatgtc catagagacc tggctgccag gaacatcttg gtgaatcaga 2340
acttgtgctg caaggtatct gactttggcc tgacccgcct cctggatgac ttcgatggca 2400
cctatgaaac ccagggagga aagatcccta tccgttggac agccccagaa gctattgccc 2460
atcggatctt caccacagcc agtgatgttt ggagctttgg gattgtaatg tgggaggtgt 2520
tgagttttgg cgacaaaccc tatggggaga tgagcaatca agaggtaatg aaaagcattg 2580
aggatgggta ccggctgccc cctcctgtgg attgtcctgc ccccctgtat gaactcatga 2640
agaactgctg ggcttacgac cgtgcccgtc ggccccactt cctccagctg caggcacatc 2700
tggaacagct gctcacggac ccccattccc taaggacaat tgccaacttt gaccctaggg 2760
tgaccctacg tctgcccagc ctgagtggct ctgatgggat cccttatcga agtgtctctg 2820
agtggcttga atccatacgc atgaagcgct acatcctgca cttccgctcg gcaggcctgg 2880
acaccatgga gtgtgtgctg gagctgacag ccgaggacct gacgcagatg ggaatcacat 2940
tgccagggca ccagaagcgc attctctgca gtattcaagg atttaaggac tgagcatcca 3000
ctgaaaagat gttccagccc tctgtccact ccctgggcct ttcctcagcc tacgaaatgt 3060
aggctattgg tgctgctcct gcccagtcaa tcagaactct gcctttggac caaggagcct 3120
ttgtttataa agggggtggg tggggtacaa gtgaagggga ctgtgggtgg gtgggtgctg 3180
gggggagggt ttaatatata tacttacata tgcattatct atttttgtaa ataaacagag 3240
ttgagttttt atccctcccc tgatcttttt ccccatcaag accctttatt tcaactccaa 3300
gagtataggg tattatcaaa aaaccaagtg ctagcaattc tggaaaatga ggttttataa 3360
tcttagctgt ggccacaacc tagcagcaca gagcaaggtg ggtgtccagg ctgctggagc 3420
cacgttccca gagggttggg gcttccaccc tcagcaagca cattgcatag gctgctcagg 3480
ccagccaagg gcattggtgc agcagtgaaa gcagcagcac taaatgtgac ccttgggttg 3540
ggggcctcag gatgataggt gatccctgca gaagaggcaa gggttggagc ccagggctgg 3600
gcctcagcac caggacggag cctcagtact tctaggccat gctggggccc tgggtcaggg 3660
cacttgagac acaagggagg gatttggaat gcagggaggg tggaggcaag gactggcctc 3720
tgtgggtggt ccacggtggg ggatggacct gtggtgtgga aggaggtcca tcttcaactc 3780

aggtctgggc tctagcagag tggcacttcc gacaaaggac atggccatcc agagggaaac 3840

agccgttgtc atctgcctca atggacaggg ctttcccaca gtcc 3884

<210> 62
<211> 3963
<212> DNA
<213> human

<400> 62
attaaggact cggggcagga ggggcagaag ttgcgcgcag gccggcgggc gggagcggac 60

accgaggccg gcgtgcaggc gtgcgggtgt gcgggagccg ggctcggggg gatcggaccg 120

agagcgagaa gcgcggcatg gagctccagg cagcccgcgc ctgcttcgcc ctgctgtggg 180

gctgtgcgct ggccgcggcc gcggcggcgc agggcaagga agtggtactg ctggactttg 240

ctgcagctgg aggggagctc ggctggctca cacacccgta tggcaaaggg tgggacctga 300

tgcagaacat catgaatgac atgccgatct acatgtactc cgtgtgcaac gtgatgtctg 360

gcgaccagga caactggctc cgcaccaact gggtgtaccg aggagaggct gagcgtatct 420

tcattgagct caagtttact gtacgtgact gcaacagctt ccctggtggc gccagctcct 480

gcaaggagac tttcaacctc tactatgccg agtcggacct ggactacggc accaacttcc 540

agaagcgcct gttcaccaag attgacacca ttgcgcccga tgagatcacc gtcagcagcg 600

acttcgaggc acgccacgtg aagctgaacg tggaggagcg ctccgtgggg ccgctcaccc 660

gcaaaggctt ctacctggcc ttccaggata tcggtgcctg tgtggcgctg ctctccgtcc 720

gtgtctacta caagaagtgc cccgagctgc tgcagggcct ggcccacttc cctgagacca 780

tcgccggctc tgatgcacct tccctggcca ctgtggccgg cacctgtgtg gaccatgccg 840

tggtgccacc ggggggtgaa gagccccgta tgcactgtgc agtggatggc gagtggctgg 900

tgcccattgg gcagtgcctg tgccaggcag gctacgagaa ggtggaggat gcctgccagg 960

cctgctcgcc tggatttttt aagtttgagg catctgagag cccctgcttg gagtgccctg 1020

agcacacgct gccatcccct gagggtgcca cctcctgcga gtgtgaggaa ggcttcttcc 1080

gggcacctca ggacccagcg tcgatgcctt gcacacgacc cccctccgcc ccacactacc 1140

tcacagccgt gggcatgggt gccaaggtgg agctgcgctg gacgccccct caggacagcg 1200

ggggccgcga ggacattgtc tacagcgtca cctgcgaaca gtgctggccc gagtctgggg 1260

aatgcgggcc gtgtgaggcc agtgtgcgct actcggagcc tcctcacgga ctgacccgca 1320

ccagtgtgac agtgagcgac ctggagcccc acatgaacta caccttcacc gtggaggccc 1380

gcaatggcgt ctcaggcctg gtaaccagcc gcagcttccg tactgccagt gtcagcatca 1440

accagacaga gccccccaag gtgaggctgg agggccgcag caccacctcg cttagcgtct 1500

cctggagcat ccccccgccg cagcagagcc gagtgtggaa gtacgaggtc acttaccgca 1560

agaagggaga ctccaacagc tacaatgtgc gccgcaccga gggtttctcc gtgaccctgg 1620

acgacctggc cccagacacc acctacctgg tccaggtgca ggcactgacg caggagggcc 1680

agggggccgg cagcaaggtg cacgaattcc agacgctgtc cccggaggga tctggcaact 1740
tggcggtgat tggcggcgtg gctgtcggtg tggtcctgct tctggtgctg gcaggagttg 1800
gcttctttat ccaccgcagg aggaagaacc agcgtgcccg ccagtccccg gaggacgttt 1860
acttctccaa gtcagaacaa ctgaagcccc tgaagacata cgtggacccc cacacatatg 1920
aggaccccaa ccaggctgtg ttgaagttca ctaccgagat ccatccatcc tgtgtcactc 1980
ggcagaaggt gatcggagca ggagagtttg gggaggtgta caagggcatg ctgaagacat 2040
cctcggggaa gaaggaggtg ccggtggcca tcaagacgct gaaagccggc tacacagaga 2100
agcagcgagt ggacttcctc ggcgaggccg gcatcatggg ccagttcagc caccacaaca 2160
tcatccgcct agagggcgtc atctccaaat acaagcccat gatgatcatc actgagtaca 2220
tggagaatgg ggccctggac aagttccttc gggagaagga tggcgagttc agcgtgctgc 2280
agctggtggg catgctgcgg ggcatcgcag ctggcatgaa gtacctggcc aacatgaact 2340
atgtgcaccg tgacctggct gcccgcaaca tcctcgtcaa cagcaacctg gtctgcaagg 2400
tgtctgactt tggcctgtcc cgcgtgctgg aggacgaccc cgaggccacc tacaccacca 2460
gtggcggcaa gatccccatc cgctggaccg ccccggaggc catttcctac cggaagttca 2520
cctctgccag cgacgtgtgg agctttggca ttgtcatgtg ggaggtgatg acctatggcg 2580
agcggcccta ctgggagttg tccaaccacg aggtgatgaa agccatcaat gatggcttcc 2640
ggctccccac acccatggac tgcccctccg ccatctacca gctcatgatg cagtgctggc 2700
agcaggagcg tgcccgccgc cccaagttcg ctgacatcgt cagcatcctg gacaagctca 2760
ttcgtgcccc tgactccctc aagaccctgg ctgactttga cccccgcgtg tctatccggc 2820
tccccagcac gagcggctcg gagggggtgc ccttccgcac ggtgtccgag tggctggagt 2880
ccatcaagat gcagcagtat acggagcact tcatggcggc cggctacact gccatcgaga 2940
aggtggtgca gatgaccaac gacgacatca agaggattgg ggtgcggctg cccggccacc 3000
agaagcgcat cgcctacagc ctgctgggac tcaaggacca ggtgaacact gtggggatcc 3060
ccatctgagc ctcgacaggg cctggagccc catcggccaa gaatacttga agaaacagag 3120
tggcctccct gctgtgccat gctgggccac tggggacttt atttatttct agttctttcc 3180
tccccctgca acttccgctg aggggtctcg gatgacaccc tggcctgaac tgaggagatg 3240
accagggatg ctgggctggg ccctctttcc ctgcgagacg cacacagctg agcacttagc 3300
aggcaccgcc acgtcccagc atccctggag caggagcccc gccacagcct tcggacagac 3360
atataggata ttcccaagcc gaccttccct ccgccttctc ccacatgagg ccatctcagg 3420
agatggaggg cttggcccag cgccaagtaa acagggtacc tcaagcccca tttcctcaca 3480
ctaagagggc agactgtgaa cttgactggg tgagacccaa agcggtccct gtccctctag 3540
tgccttcttt agaccctcgg gccccatcct catccctgac tggccaaacc cttgctttcc 3600
tgggcctttg caagatgctt ggttgtgttg aggtttttaa atatatattt tgtactttgt 3660
ggagagaatg tgtgtgtgtg gcagggggcc ccgccagggc tggggacaga gggtgtcaaa 3720

cattcgtgag ctggggactc agggaccggt gctgcaggag tgtcctgccc atgccccagt 3780
cggccccatc tctcatcctt ttggataagt ttctattctg tcagtgttaa agattttgtt 3840
ttgttggaca tttttttcga atcttaattt attatttttt ttatatttat tgttagaaaa 3900
tgacttattt ctgctctgga ataaagttgc agatgattca aaccgaaaaa aaaaaaaaaa 3960
aaa 3963

<210> 63
<211> 3907
<212> DNA
<213> mouse

<400> 63
attaaggact tggagcagga gggtagaagt tgtctctgtc ggcgggcggg caggattggg 60
gcaccgagac cggcgtgcgg acagcaggga tcgcggggag cgaggggtgc ggcatggagc 120
tccgggcagt cggtttctgc ctggcgctgc tgtggggttg cgcgctggcg gccgcggcgg 180
cacagggaaa ggaagttgtt ttgttggact tcgcagcaat gaagggagag ctcggctggc 240
tcacgcaccc ctatggcaaa gggtgggacc tgatgcagaa catcatggac gacatgccta 300
tctacatgta ctcggtgtgc aacgtggtat ccggcgacca ggacaactgg ctccgcacca 360
actgggtgta ccgggaggag gccgagcgca tctttattga gctcaagttc acggtgcgag 420
actgtaacag cttcccgggt ggcgccagct catgcaaaga gaccttcaac ctctactatg 480
cagagtcaga tgtggactat ggcaccaact tccagaagcg ccagttcacc aagattgaca 540
ccatcgcccc tgacgagatc acggtcagca gtgacttcga ggctcgcaat gtcaagctga 600
acgtagagga gcgcatggtg gggcccctta cccggaaggg cttctacctg gccttccagg 660
acatcggcgc ctgcgtggcg ctgctctccg ttcgcgtcta ctacaagaag tgtcccgaga 720
tgctgcagag cttggctcgc ttccccgaga ccattgctgt cgctgtctcc gatacacaac 780
ccctggccac ggtggccggt acctgcgtgg accatgccgt ggtgccttat gggggcgagg 840
ggcctctcat gcactgcacg gtggatggcg agtggctggt gcccatcggg cagtgcctgt 900
gccaggaagg ctacgagaag gtcgaggatg cctgccgagc ctgttctcca ggattcttca 960
agtctgaggc atctgagagc ccttgcctgg agtgtccaga gcataccctg ccatccacag 1020
agggtgccac ctcctgccag tgtgaagaag gctatttcag ggcacctgag gacccactgt 1080
ccatgtcttg cacacgtcca ccctctgccc ccaactacct cacggccatt ggcatgggtg 1140
ccaaagtaga actgcgttgg acagctccca aggacactgg tggccgccag gacattgtct 1200
acagtgtcac ttgcgaacag tgctggccag agtctggcga gtgtgggccc tgtgaggcga 1260
gcgtgcgcta ttcagaacct cctcacgccc tgacccgcac gagtgtgaca gtcagtgacc 1320
tggagcccca catgaactat accttcgctg tcgaagcacg caatggtgtc tcaggcctgg 1380
tgactagccg aagcttccgg actgccagcg tcagtattaa ccaaacagag ccccccaaag 1440
tgaggctgga ggaccgaagc accacctccc tgagtgtcac ctggagcatc ccggtgtcac 1500
agcagagccg tgtgtggaag tacgaagtca cctaccgcaa gaagggggat gccaacagct 1560

ataatgtgcg ccgcacggaa ggcttctccg tgaccctgga tgaccttgct ccggatacca 1620
cgtacctggt gcaggtgcag gcgctgacgc aggagggcca gggagccggc agcaaagtgc 1680
acgagttcca gacactgtcc acggaaggat ctgccaacat ggcggtgatc ggcggtgtgg 1740
ctgtaggtgt tgttttgctt ctggtactgg caggagttgg cctcttcatc catcgaagga 1800
ggaggaacct gcgggctcgc cagtcctctg aggatgtccg tttttccaag tcagaacaac 1860
taaagcccct gaagacctat gtggatcctc acacttacga agaccccaac caggctgtac 1920
tcaagtttac caccgagatc cacccatcct gtgtggcaag gcagaaggtc attggagcag 1980
gagagtttgg agaggtctat aaagggacgc tgaaggcatc ctcggggaag aaggagatac 2040
cggtggccat caagacactg aaagcgggct acactgagaa gcagcgggtg gacttcctga 2100
gcgaggccag catcatgggc cagtttagcc accacaatat catccgcctg gagggcgtgg 2160
tctctaaata caaacccatg atgattatca cagagtacat ggagaatgga gcgctagaca 2220
agttccttag ggagaaggat ggtgagttca gtgtacttca gttggtgggc atgctgaggg 2280
gtatcgcatc cggcatgaag tacctggcca acatgaacta cgtgcaccgg gacctggccg 2340
cccgcaacat cctcgtcaac agcaacctgg tgtgcaaggt gtccgatttt ggcctgtcgc 2400
gtgtgctgga agatgacccc gaggccacct acaccacaag tggcggcaag atccctattc 2460
gatggacagc cccagaggcc atttcctacc gcaagttcac ctcagccagc gatgtgtgga 2520
gctacggcat tgtcatgtgg gaagtgatga cttatggcga acggccctac tgggaactgt 2580
caaaccacga ggtcatgaaa gccatcaacg acggcttccg gctccctacg cccatggact 2640
gcccttcagc catttaccag ctcatgatgc agtgctggca gcaagagcgc tcccgccgac 2700
ccaagtttgc cgacatcgtt agcatcctgg acaagctcat ccgagccccc gactccctca 2760
agacgctggc tgactttgat ccccgagtgt ccatccggct gcccagcacc agcggctcgg 2820
agggagtccc cttccgtacg gtgtccgagt ggctggagag catcaagatg caacagtaca 2880
cggaacactt catggtggct ggctacacgg ccatcgagaa ggtggtacag atgtccaacg 2940
aagacatcaa aaggatcgga gtgcgtcttc ctggccacca gaagcgtatt gcctacagcc 3000
tgctgggact caaggaccag gtcaacacag tggggattcc tatctgagtc cattggggcc 3060
gtgcccaaca atacttgaag agccacagtg gtctccctgc cgacctggtg ctggcccact 3120
ggaactttat ttatttctgt ttcctcgtct atgcctccct gaggactctg cagggggctt 3180
ttgaatgaca ccctggcctg agcctgggaa acttggatgc tggtcagggc tctctttccc 3240
ctgcaaagga cccagctaag cacttagcag tttgccatgg ccttcccagc atcccctgag 3300
gctaaagttc caccaagacc gtcgatatcg acgagggaca tttccaaacg gacctcccca 3360
tcttcatttg gcctcctgag aagccacctc aggagctgag gctaagcact aagcccagga 3420
ccatatgact agggcactgt acgccccgcc cctagttaga gggtaggttt tggacttggc 3480
tgggtgtggt cacagcaatc tcccagtgcc ttttacagac ccagggctct gccctccgcc 3540
ctcgagggcc agcttcttgc tttcctaggg ccctctcagg atgcttggct gtgctgaggt 3600

ttttattaaa tatatatttt atacttgtgg aaagaatgag tgtgtggcag ggaccttgcc 3660

agggctggag acagaggatc ccctgcaaca gacattcccg ggctgggggc tggcggacct 3720

gcaggagact ttccgccagc cacccagtct ccagcccctt tggacaaatg tcgctgtcag 3780

tgttacagat ttcttttatt gggttgtttt tttgttgtat tttttgaacc ttaacttatt 3840

atttttttta tatttattgt tagaaaatga cttatttctg ctctggaata aagttgcaga 3900

tggttca 3907

<210> 64
<211> 3917
<212> DNA
<213> rat

<400> 64
attaaggact tggggcagga gggcagaagt tgtctgtggc gagcgggtag gagcgagaca 60

tccgggccgg cgtgtggaca gcagggatcg cggggagcga ggggtgcagc atgaagcttc 120

gggcagccgg tttctgcctg gccctgttgt ggggttgcgc gctggcggcc gcggcggcac 180

agggaaaaga agttgttttg ctggacttcg cagcaatgaa gggggaactc ggctggctca 240

cacaccccta cggaaaaggg tgggacctga tgcaaaacat catgaatgac atgcctatct 300

acatgtactc cgtgtgcaat gtggtgtctg gcgaccagga caactggctc cgtaccaact 360

gggtgtaccg ggaggaggcc gagcgcatct ttattgagct caagttcacc gtgcgagact 420

gcaatagctt tccgggtggc gccagctcct gcaaagagac cttcaacctc tactatgcag 480

agtcagacgt ggactatggc accaacttcc agaagcgcca gttcaccaag atcgacacca 540

tcgcccctga cgagatcacg gtcagcagtg acttcgaggc tcgcaatgtc aagctgaatg 600

tggaggagcg catggtgggg ccccttaccc ggaagggctt ctacctggcc ttccaggaca 660

tcggtgcctg tgtggcattg ctctctgttc gtgtctacta caagaagtgt cccgagatgc 720

ttcagagctt ggcccgcttc cctgagacca ttgctgtcgc cgtctcagat gcacagcccc 780

tggccacggt ggccggtacc tgcgtggacc acgctgtggt gccttatggg ggcgaggggc 840

ctctcatgca ctgcacggtg gatggtgagt ggctggtgcc tatcgggcag tgcctgtgcc 900

aagaaggcta cgagaaggtg gaggacgcct gccaagcctg ttctccagga ttcttcaagt 960

ctgaggcatc tgagagccct tgcctgcagt gtccggagca taccctgcca tccactgagg 1020

gtgccacctc ctgccagtgt gaagaaggct atttcagggc acctgaggac ccactgtcca 1080

tgccctgcac acgtccaccc tctgcccccc actacctcac ggccatcggc atgggtgcca 1140

aagtagaact gcgttggacg gccccccagg acaccggtgg gcgcaaggac atcgtctaca 1200

gcgtcacttg cgaacagtgc tggccagagt ccggcgagtg tgggtcctgt gaggccagtg 1260

tgcaatactc agaaccacct caggccctga cccgcacgag tgtgacagtc agcgacctgg 1320

agcctcacat gaactatacc ttcactgtcg aagcccgcaa tggcgtctca gacctggtga 1380

atagccgaag cttccggact gccagtgtca gcattaacca aacagagccc cccaaggtga 1440

gactggagga ccgaagcacc acctccctga gtgtcgcctg gagcatcccg gtgccacagc 1500
agagccgtgt gtggaagtac gaagtcacct accgcaagaa gggggatgcc aatagctaca 1560
atgtgcaccg tacagacggc ttctccgtga ccctggatgg ccttgctccc ggtactacat 1620
acctggtgca ggtgcaggcg ctgacgcagg agggccaggg cgccggcagc aaagtgcacg 1680
agttccagac gctgtccacg gaaggatctg ccaccatggc ggtgatcggc ggtgtggctg 1740
ttggcgttgt cctgctccta gtgttagcgg gaattggctt cttcatccat cgaaggagga 1800
ggaacctgcg ggcccgtcag tcctctgagg acgtctactt ttccaagtca gaacaactaa 1860
agcccctgaa gacatatgtg gatcctcaca cttacgaaga ccccaaccag gccgtgctca 1920
agtttaccac cgagatccac ccatcctgtg tctccaggca gaaggtcatt ggagcaggag 1980
agtttggaga ggtatataaa gggacgctga agtcctcctc tgggaagaag gagataccgg 2040
tggctatcaa gacgctgaaa gccggctaca ctgagaagca gcgggtggac ttcctaagtg 2100
aagccagcat catgggccag ttcagccatc acaatatcat ccgcctggag ggcgttgtct 2160
ctaaatacaa acccatgatg attatcacag agtacatgga gaatggagcg ctagacaagt 2220
tccttaggga gaaggacggc gagttcagcg tactgcagct cgtgggcatg cttcggggca 2280
tcgcgtctgg catgaagtac ctggccaaca tgaactacgt gcaccgggac ctggccgccc 2340
gcaacatcct tgtcaacagt aacctggtat gcaaggtgtc cgattttggc ctgtcgcgtg 2400
tgctggagga tgaccctgag gccacctaca ccactagtgg cggcaagatt cctattcgat 2460
ggacagcccc agaagccatt tcctaccgca agttcacctc agccagtgat gtgtggagct 2520
atggaattgt catgtgggag gtgatgacgt acggagagcg gccctattgg gagctgtcaa 2580
accatgaggt catgaaagcc atcaacgatg gcttccggct ccccacgccc atggactgcc 2640
cctcagccat ctaccaactc atgatgcaat gctggcagca agagcgctcc cgccggccca 2700
agtttgctga cattgttagc atcttggaca agctcatccg agcccccgac tccctcaaga 2760
ccctggctga cttcgatccc cgagtgtcca ttcggctacc cagcaccagc ggctcagagg 2820
gagtcccctt ccggacggtg tctgagtggc tggaaagcat caaaatgcaa cagtatacgg 2880
aacacttcat ggtagctggc tacacggcca tcgagaaagt ggtgcagatg tccaatgatg 2940
acatcaaaag gatcggagtg cgtcttcctg gccaccagaa gcgcattgcc tacagcctgc 3000
tgggactcaa agaccaagtg aacacagtgg ggattcctat ctgagtctga tggggcctcg 3060
tgcccacctt gaccaacaat acttgaagag ccacagtggt ctccctaccg acctggtgct 3120
gggccactgg aactttattt atttctgact tcctcgtcga cacctccctg aggactctgc 3180
gagggggact cttgaatgac accctggcat gagccgggga acgcctggtc agggttcctt 3240
tccccctgca aaggacccag ctaagcactt agcaggttgt cattgcactc ccagcatccc 3300
ctgaggctgg agtttggcca agactgtcga tatccacgag gggagtttcc aaactgacct 3360
ccccgtcttt tctccccaag aagccacctc aagagctgag gctaagcact aagcccagga 3420
ccatatgact agggcactgt aagccccacc tctagttaga gggtaggttt tggacttggc 3480

tgggtggggc cacaacaatc tcccagtgcc ttctacagac ccagggctct gccctctgcc 3540

ctcgagggcc agtttcttgc tttcctaggg ccctctcagg atgcttggct gtgttgaggt 3600

tttttttaaa tatatatttt atacttgtgg aaagaatgag tgtgtggcag ggaccttgcc 3660

agggctgggg acagaggata ccctgcaaca gacattcccg ggctgaggcc tggccgactt 3720

gtaggagact taccacccag ccaccccgtc tctagcccct ttggacaact gtcgctgtca 3780

gtgttacaga tctgtgttat tgggtggttt ttgtttttgt ttttggaacc ttaacttatt 3840

attatttttt tttatattta ttgttagaaa atgacttatt tctgctccag aataaagttg 3900

cagatggttc aaactga 3917

<210> 65
<211> 6364
<212> DNA
<213> human

<400> 65
ctgggataga agcggcagga gcagcgttgg caccggcgaa ccatggctgg gattttctat 60

ttcgccctat tttcgtgtct cttcgggatt tgcgacgctg tcacaggttc cagggtatac 120

cccgcgaatg aagttacctt attggattcc agatctgttc agggagaact tgggtggata 180

gcaagccctc tggaaggagg gtgggaggaa gtgagtatca tggatgaaaa aaatacacca 240

atccgaacct accaagtgtg caatgtgatg gaacccagcc agaataactg gctacgaact 300

gattggatca cccgagaagg ggctcagagg gtgtatattg agattaaatt caccttgagg 360

gactgcaata gtcttccggg cgtcatgggg acttgcaagg agacgtttaa cctgtactac 420

tatgaatcag acaacgacaa agagcgtttc atcagagaga accagtttgt caaaattgac 480

accattgctg ctgatgagag cttcacccaa gtggacattg gtgacagaat catgaagctg 540

aacaccgaga tccgggatgt agggccatta agcaaaaagg ggttttacct ggcttttcag 600

gatgtggggg cctgcatcgc cctggtatca gtccgtgtgt tctataaaaa gtgtccactc 660

acagtccgca atctggccca gtttcctgac accatcacag gggctgatac gtcttccctg 720

gtggaagttc gaggctcctg tgtcaacaac tcagaagaga aagatgtgcc aaaaatgtac 780

tgtggggcag atggtgaatg gctggtaccc attggcaact gcctatgcaa cgctgggcat 840

gaggagcgga gcggagaatg ccaagcttgc aaaattggat attacaaggc tctctccacg 900

gatgccacct gtgccaagtg cccaccccac agctactctg tctgggaagg agccacctcg 960

tgcacctgtg accgaggctt tttcagagct gacaacgatg ctgcctctat gccctgcacc 1020

cgtccaccat ctgctcccct gaacttgatt tcaaatgtca acgagacatc tgtgaacttg 1080

gaatggagta gccctcagaa tacaggtggc cgccaggaca tttcctataa tgtggtatgc 1140

aagaaatgtg gagctggtga ccccagcaag tgccgaccct gtggaagtgg ggtccactac 1200

accccacagc agaatggctt gaagaccacc aaagtctcca tcactgacct cctagctcat 1260

accaattaca cctttgaaat ctgggctgtg aatggagtgt ccaaatataa ccctaaccca 1320

gaccaatcag tttctgtcac tgtgaccacc aaccaagcag caccatcatc cattgctttg 1380

gtccaggcta aagaagtcac aagatacagt gtggcactgg cttggctgga accagatcgg 1440
cccaatgggg taatcctgga atatgaagtc aagtattatg agaaggatca gaatgagcga 1500
agctatcgta tagttcggac agctgccagg aacacagata tcaaaggcct gaaccctctc 1560
acttcctatg ttttccacgt gcgagccagg acagcagctg gctatggaga cttcagtgag 1620
cccttggagg ttacaaccaa cacagtgcct tcccggatca ttggagatgg ggctaactcc 1680
acagtccttc tggtctctgt ctcgggcagt gtggtgctgg tggtaattct cattgcagct 1740
tttgtcatca gccggagacg gagtaaatac agtaaagcca aacaagaagc ggatgaagag 1800
aaacatttga atcaaggtgt aagaacatat gtggacccct ttacgtacga agatcccaac 1860
caagcagtgc gagagtttgc caaagaaatt gacgcatcct gcattaagat tgaaaaagtt 1920
ataggagttg gtgaatttgg tgaggtatgc agtgggcgtc tcaaagtgcc tggcaagaga 1980
gagatctgtg tggctatcaa gactctgaaa gctggttata cagacaaaca gaggagagac 2040
ttcctgagtg aggccagcat catgggacag tttgaccatc cgaacatcat tcacttggaa 2100
ggcgtggtca ctaaatgtaa accagtaatg atcataacag agtacatgga gaatggctcc 2160
ttggatgcat tcctcaggaa aaatgatggc agatttacag tcattcagct ggtgggcatg 2220
cttcgtggca ttgggtctgg gatgaagtat ttatctgata tgagctatgt gcatcgtgat 2280
ctggccgcac ggaacatcct ggtgaacagc aacttggtct gcaaagtgtc tgattttggc 2340
atgtcccgag tgcttgagga tgatccggaa gcagcttaca ccaccagggg tggcaagatt 2400
cctatccggt ggactgcgcc agaagcaatt gcctatcgta aattcacatc agcaagtgat 2460
gtatggagct atggaatcgt tatgtgggaa gtgatgtcgt acggggagag gccctattgg 2520
gatatgtcca atcaagatgt gattaaagcc attgaggaag gctatcggtt accccctcca 2580
atggactgcc ccattgcgct ccaccagctg atgctagact gctggcagaa ggagaggagc 2640
gacaggccta aatttgggca gattgtcaac atgttggaca aactcatccg caaccccaac 2700
agcttgaaga ggacagggac ggagagctcc agacctaaca ctgccttgtt ggatccaagc 2760
tcccctgaat tctctgctgt ggtatcagtg ggcgattggc tccaggccat taaaatggac 2820
cggtataagg ataacttcac agctgctggt tataccacac tagaggctgt ggtgcacgtg 2880
aaccaggagg acctggcaag aattggtatc acagccatca cgcaccagaa taagattttg 2940
agcagtgtcc aggcaatgcg aacccaaatg cagcagatgc acggcagaat ggttcccgtc 3000
tgagccagta ctgaataaac tcaaaactct tgaaattagt ttacctcatc catgcacttt 3060
aattgaagaa ctgcactttt tttacttcgt cttcgccctc tgaaattaaa gaaatgaaaa 3120
aaaaaaacaa tatctgcagc gttgcttggt gcacagattg ctgaaactgt ggggcttaca 3180
gaaatgactg ccggtcattt gaatgagacc tggaacaaat cgtttctcag aagtactttt 3240
ctgttcatca ccagtctgta aaatacatgt acctatagaa atagaacact gcctctgagt 3300
tttgatgctg tatttgctgc cagacactga gcttctgaga catccctgat tctctctcca 3360
tttggaatta caaccattgt attttgtttg tggcataaat tacagtcatc tgtctttcac 3420

tggaatgaag accatgccta ggaacatttt ttaaggactc agctgtggct tttagggctt 3480
ggttcatacc atgggggaaa aaaaagtcct aggagaaagc gacgtggctc attagtgttg 3540
cctcttcagt gctcaagccg cctggtggat tcctatgaca cagggggcct ggaaagaaag 3600
ggaaagtgga tttaaaatat atatatacgt aacccaagcc ccataacccc taactggaca 3660
aatgaggtct gtttctttgg gcctgaggct gtgccatata aagtcttatt ttgggacttt 3720
acaaacttgt cctaactatc ttgtggatag tgggctgtga caatctggaa tagagaacgt 3780
tcacacttcg ctcctttaaa gaagcgaccc cagatctgca agggagtaga ttctgctatc 3840
ttggcctcac agcccttcct gttgattaca aagcccgtgg aagaaaacag aacacaccct 3900
cctcagttcc gtctaaatgt gtttcttctg cttcaattac accagttctg gggcaaagac 3960
actgatgaaa caacacccat acctgaaaag aataaatgtg tgactttcaa atcccctttc 4020
gcagtgaaag aaacagcaaa cacttaagat tcagcatctg ttctccagtt gcactgagga 4080
atgcactgtc tcgcagcacc agctctgcag agcccttgcc ccagactctt tgcggtttta 4140
tttatatgta tttccatatt tcattcctgt gtgtcactgc tgcattggtg tggcagcaag 4200
tgaccaaatg ctacaggtct tactatggac accaggtcag gtgcaaccac acaaaacaaa 4260
gccagttcca tgagctgcct atgatatgca ttgcggaagt aacattttac ccagggtgtg 4320
ccattgcagt gatataaata tatttttttc ttagactaaa tatgagctga ctatctcttt 4380
tgatgtgtgt acataggtgt gagtgtgtgt gtatgcgtgc ctgtctgtgt gcgggtgtgt 4440
gtatgtgcat agcctcatgc ttaggactac ccatgaatgt tgtggaatgc tacacctgga 4500
gagttctggt tttccaccag tttcaagatg aagaactaca tgatacagtg gacctggaga 4560
ccatcccctt ggaaagacaa cccagagatg ttcagcatcc tgtatctaca cgcatcctgt 4620
atctacacgt gtattttgta gctgtcacac taaccttaat aagaattcta cagctttgga 4680
cagaggcatt ttcaccttaa tggtgaagta atttaaaata taaatccatt caggtgacaa 4740
cccatcatca aaattacaaa ttttctgatt gaactcatct gaatcatcag ttccttgatg 4800
gagagagaga aggagatgga atgtgtctgg taaccccaaa tggagtacaa gtagcctttg 4860
ttttcctgca taaatggact tgttgaatgc gaacgaatat atgcaattca tatacttttg 4920
gagatgaacg tagatatgtg tgtcagcttt gagatggtgt gtcctggatt aatactttgt 4980
ctcccaatat cacagaaaaa tacatgccag tgactcttga ggttaaggta gttgggatga 5040
aatggcctca ggcaatttca cattccctaa ttacctggaa agttctacag taattaatat 5100
gcagctaact cctgttgccc tcacaagagc atcagccttc tagaatcgga gctccggagt 5160
gtgaagattc agtattgata tgatatgtat accaaactcc agccaactta ctgccatttt 5220
tcataatctg agtggctgcc ttgcttatcc taagctgtgg ttgcagaaac cgtggccatt 5280
tatataagct ataacatcaa atcagggaaa aatgaggaaa aaaaatagat tctgaaccat 5340
ttattgttga ataagtagag aaaatcatca ataaatattt attacattct gacagggtgt 5400
gtggcattgt gttctatgcc agagtgacaa agttgattca cccctttttg gggaccttaa 5460

tatatttttt aagggatgtg cctatgcatt gatgcctgaa aaatatgtat aaagaaatga 5520
ggttgactct tctgagcagt tcatcttttc cagaggtaag ggtaggaggc caacttcagg 5580
gtctgggtct gagcccgtgg gcaagccctg gccgagtgag ctccaatgct aactcatgtg 5640
ccgatctcta gagcagtggg aaactacccc gctgcaccaa atcaagtagc ttcaccttgt 5700
gtatgcaggc cccaagttat tttttagcaa tcttacgagt gaaatgttct ggtgggttga 5760
aaaacgttct tattttaaag aaaggttgtg ctcgctacac tgctggtgtg tgcattctga 5820
gacctcttgt attcaatctg tgaaggatat gtgtattaat ccgtacaccc gtatagcctc 5880
aatatttgtc tgaagacact taaattctga cccataaagg aaagttctag aagcaatatt 5940
ttcacttatt taacattctc caaacaacat caagcattga tacacactga agagtgcgtt 6000
tattttttgt atcactctaa gtatgttgga atatgcaagg actgtggttc aaattagaat 6060
gtataaggca tattataatt tagttcatac tgaataagaa attaacagaa cattgttcgg 6120
ttcacacgtt ccaaactttg agtgatttct ggagttagac atagattttc tattttgttt 6180
taatttgtca aggtattttt cttcccttca tgaactttag gtacacataa cttatgtcat 6240
ttatttatgg tcttttatac ctagtttgta aaattgtaaa atagcaaact aaatgcaaag 6300
agtttgcatt tgaaaataat aaagtagttg ccgtatacaa ccctgcaaaa aaaaaaaaaa 6360
aaaa 6364

<210> 66
<211> 6328
<212> DNA
<213> mouse

<400> 66
taagcattta ttccactggg atagaagcgg caggagcagc gttggcaccg gcgaaccatg 60
gctgggattt tctatttcat cctcttttcg tttctctttg gaatttgcga cgctgtcacc 120
ggttctaggg tataccccgc gaatgaagtt actttattgg attccagatc tgttcaggga 180
gagcttgggt ggatagcaag ccctctggaa ggagggtggg aggaagtgag cattatggat 240
gagaaaaata caccgatccg aacctaccag gtgtgcaacg tgatggaagc cagccagaac 300
aactggctgc gaactgactg gatcacccgc gaaggggcgc agagggtgta cattgaaatt 360
aagttcactc taagggactg caacagtctt ccgggcgtca tggggacttg caaggagacg 420
tttaacctct actactatga gtcggacaac gacaaggagc gcttcatcag agagagccag 480
tttggcaaga tcgacaccat cgcagctgat gagagcttca cgcaggtgga cattggtgac 540
cgaatcatga aactcaacac tgagatccgg gatgtagggc cactgagcaa gaaagggttt 600
tacctggctt ttcaggatgt gggtgcttgc atcgccctgg tgtctgtccg tgtgttctac 660
aagaagtgtc cactcaccgt tcgaaatcta gcccagtttc ctgataccat tactggggct 720
gatacctctt ccctggtgga agttcgaggc tcctgtgtca acaactcaga agagaaggat 780
gtgccaaaaa tgtactgtgg ggcagatggt gaatggctgg tacccattgg caactgccta 840

tgcaacgctg ggcacgagga gcagaatggt gaatgccaag cttgcaaaat tggatattac 900
aaagctcttt ccacggacgc ctcctgtgcc aagtgtccac cgcacagcta ctctgtctgg 960
gaaggagcca cgtcctgcac ctgtgaccga ggctttttca gagctgacaa cgatgcagcc 1020
tccatgccct gcacccgccc accatctgct cccctcaact tgatttctaa tgtcaacgag 1080
acatcggtga acttggaatg gagcagccct cagaacacag gtggccgtca ggacatttct 1140
tacaacgtgg tctgcaagaa atgtggagct ggtgatccca gcaagtgccg gccctgtgga 1200
agtggagtcc actacacacc acagcagaac ggactcaaga cgaccagagt ctccatcact 1260
gacctcctag cacacaccaa ttacacgttt gagatctggg cagtgaatgg agtgtctaag 1320
tataacccta gcccggacca gtccgtgtct gtcacagtga caaccaacca agcagcacca 1380
tcatccattg ctttggtcca ggctaaagaa gttacaagat atagcgtagc tctggcttgg 1440
ctggaaccag atcgaccaaa tggagtcatc ttagaatatg aagtcaaata ttatgaaaag 1500
gatcagaatg aacgcagcta tcgcatagtc cggacagctg ccaggaacac agatatcaaa 1560
ggcctgaacc ctctgacttc ctatgttttt cacgtgcgag ccaggaccgc tgctggctac 1620
ggagacttca gcgagcccct ggaagtcact actaatacag tgccttcccg catcattggc 1680
gatggagcca actccactgt cctgctggtc tccgtctctg gcagtgtggt cctcgtggtc 1740
attctcattg cagcttttgt gatcagccga agacggagta agtacagcaa agcgaaacaa 1800
gaagcagatg aagagaaaca tttgaatcaa ggtgttagaa cttatgtgga tccctttaca 1860
tacgaagacc ccaaccaggc agttcgagaa tttgccaaag aaatcgatgc atcctgcatt 1920
aaaattgaaa aggtcatagg agttggtgaa tttggagagg tctgcagtgg gcgtctcaaa 1980
gtgcccggca agagagagat ctgtgtggcc atcaagactc tgaaagctgg ctatactgac 2040
aagcagagga gagacttcct gagtgaggcc agcatcatgg gacagtttga ccacccaaac 2100
ataatccacc tggaaggcgt tgtcaccaaa tgtaaaccgg taatgatcat aacggagtac 2160
atggagaacg gctccttgga tgctttcctc aggaagaatg atggccgctt tacagtcatt 2220
cagctggtgg gcatgctccg gggcattggc tcggggatga agtacttatc tgatatgagc 2280
tatgtgcatc gagacctggc tgccaggaac atcctggtaa acagcaactt ggtctgcaag 2340
gtgtctgact ttggcatgtc cagggtgctg gaggatgacc ccgaagcagc ctacactacc 2400
aggggtggca agattcctat ccggtggact gcgccagaag caattgcgta tcgtaaattt 2460
acctcagcca gtgatgtctg gagctacgga atcgttatgt gggaagtgat gtcatatgga 2520
gagagaccgt actgggatat gtccaatcaa gatgtgatca aagccatcga ggaagggtac 2580
cggctacccc ctccaatgga ctgccccatt gccctccatc agttaatgct ggactgctgg 2640
cagaaagaga gaagtgacag gcctaaattt gggcagatcg tcaacatgct ggacaaactc 2700
atccgcaatc ccaacagcct gaagaggaca gggtcggaga gttccagacc aaacacagcc 2760
ttgttagatc ccagctcccc tgaattctct gctgtagtat cagtgggcga ctggctgcag 2820
gccatcaaaa tggaccggta taaggacaac ttcacggccg ccgggtacac gacactagaa 2880

gccgtggttc acatgagcca ggacgacctg gcgagaattg gcatcaccgc catcacgcac 2940
cagaataaga ttttgagcag cgtccaggcg atgcgaaccc agatgcagca gatgcatggc 3000
aggatggttc ctgtctgagc cagtaccgaa tagactcaaa actctcgaat agtttacctc 3060
atccatgcac tttaattgaa gaactgcact ttttttactt tgtcttcgcc ctctgaaatt 3120
aaaggaaaac aaacaaacaa aaaacaaaaa caaaaatatc tgcagcgttg cttggtgtaa 3180
gagattgctg aaaccatggg gcttataatg aatgaccttg atcatcagaa ttaaacctgg 3240
agccagtaat ttctcagaag aactctgtcc atcgctggtg tgtaaaatac atgtacttat 3300
ataaataaaa ctctgcctct gagtttcgat gctgtatttc ccgtcagagg cttttcaagt 3360
caccctaatc ctttctctgt ctggaatttc aagggaagga tttcatctgt ggcattaata 3420
acagtcatca ctccttacca gaaagaagac ctgctgtgcc caggagcact gttaaaggac 3480
tcaatggtgg ctttcagggc tcggtttata ccacagaaga aaatccaggt gaaagccact 3540
tggcccattc atgctgcctc ttcaatggcc aggacacctg gtggattttt atgacatagg 3600
gggggttaga aagaaaagaa aatggatttt taaaacatgc gaactcccaa gacctattaa 3660
ccctaactgg acaaatgagg tctctttggg gctgaggctg tgccatataa agtcttcttt 3720
gagaatgcta caaacttctc ataactacct tatggatagt gggctgagac aatcctgaat 3780
ggagaatttt cacacacaca cacacacacc ccttcaaaga ggcaactccc aaatttacaa 3840
gggaatagat tctggtatct cagcctcaca gcccttcctg ttgattacat aagcgcttgg 3900
aagaaaatag aacacaccct ccaccgttcc ttctgaatgt ttttctttgg cttctcttct 3960
gccggttttt gaggcaaaga cacgacaaaa cacctgcacc tgaacagcag aagaaatgtg 4020
tcaagtcccc ttcgcccagg aaaacgacat ccagctttgg catctgttct cagctgcact 4080
gaggaatgtg ctgtctcgag gcaccagctc tgcagagccc ttgctcccaa actccttgtg 4140
gttttattta tacgtatttc catatttcat tcctgtacgg ccccgctgca tccgtgtggc 4200
agcaagtgac catatgttac aggttttact atggacgcca ggtcaggcgc caccacgcag 4260
aacaaagcca gttccgcaag ctgcctccga tacgcaatgc taaagtgaca ttttacccag 4320
ggtgtgccat tgcagtgata taaatatatt tttttcctag actaaaatat gacctgaata 4380
tctcttttga tgtgtgtaca tagagtgtgt gtgtgtgtgt gtgtgtgtgt gtgtatgtga 4440
gtgtgtatgg ggtgtgtgtg tgtgtgtgtg tgtgtgtgtg taacctcatg cttagaactg 4500
cctgtgaatg ttgtggaatg ctatacctgg agagttctgg tttattacca gttcaaagat 4560
aaggaaagag tcacatgata tacaagaccc ggagaccatg tccgtcccct tgggaagaca 4620
gcttggaaac gctacatgtt ctttatctgt aagtgtaatg tgtctgtcac attcatccta 4680
agaagcagtt ctttggacag atgttttcac cttgatggtg gaatgatttt tagatataag 4740
cctgactcgc cgatttcaca atggagagag aaggggatgg aatccaattt gtgaccccag 4800
atggagtata agtcgccttt gtttccctgc ataaatgaac ttgttgaatg cgaacaaata 4860
tatgcaattc atatactttt ggaagatgaa cttaagatat gtgtgtcagc cttgagatga 4920

tgtgtctgga gtaatacttt gtgtcccagt gtcacagaaa aatacataca tgccagtggc 4980

tcttgaggtt aatgtagttg ggatgaaatg gcctctagca attccaggtt ccccaattac 5040

atgtggaaag ttctggtaat taatatgcag ctaactactt atgcccttga aagtatacca 5100

gtcttctaga atcggagttc tggtgtatga agattcagta tcgacaggat atgtatacca 5160

aactccagcc agcatactgc cattttactt aacctgagag gctgccttgc ttattctgag 5220

ctatggtcac agaaactgtg gccatttata taagctataa catcaaatca gggaattaga 5280

aaaaaaaaaa acggggaaaa gattctgaac catatattgt tgaataagta aagaaaatca 5340

ccaataaata tttattgcat tctgacaggg tgtatggcat tgtattctca taactatgcc 5400

agagtgacaa agttatttca ccccttttga ggggacctta atatattttt aaagggatgt 5460

gcctatgcat tgatgcctga aaaataggtg taaaaaaaaa aaaaaagatg aacctctgag 5520

tgcatcatct ttcccagagg tcaggtggga ggaggtcaac ttctgggtct gtgtccagcc 5580

caggagcaag cgctggcctt gggagctgga aggctaaaat ctagtcaaat ctactaaaca 5640

gctttgagct gttccctgca ccaaatcaag ccgtttcacc acgtgtctgc aggctctgag 5700

gtttttattt tttggcgatc ttctaaatga aatgttctgg tgtgttttaa agactagagg 5760

aaaggttgtg ttcacaacat gattactgtg tgtgttctga gacctcttgt attcatctgt 5820

gaaggggacg tgtattaatc catacactcc tatagcctcg actatttgtc tgaagacact 5880

taaattttga ccagtaaagg aaagttctag aagcaatatt ttcactcctt tagcattgtc 5940

caaataacat caagtattaa taaacgctga agagtgtatt tattttgtat tactccaagt 6000

ctgttggaat atgcaaggac tgtggttata attagaatgt ataaggcata ttataattta 6060

gttcattctg aaaaagaagt tagctgaaca tctcttggtt catacatgtt ccaaattttg 6120

aacaatcttt tgagttagcc atagattttt ctattttgtt tttatttgtc aaggtatttt 6180

tttcttccct tcatgaactt taggtacaca taacttatgt catttattta tggtctttta 6240

tacctagttt gtaaagttgt aaaatagcaa actaaatgca aagagtttgc atttgaaagt 6300

aataaagtag ttgccgtata caaacctg 6328

<210> 67
<211> 6470
<212> DNA
<213> rat

<400> 67
taagcattta ttccactggg atagaagcgg taggagcagc gttggcaccg gcgaaccatg 60

gctgggattt tctatttcat cctcttttcg tttctctttg gaatttgcga cgctgtcacc 120

ggttctaggg tataccccgc gaatgaagtt actttattgg attccagatc tgttcaggga 180

gagcttgggt ggatagcaag ccctctggaa ggagggtggg aggaagtaag cattatggat 240

gagaaaaata caccgatccg aacctaccag gtatgcaacg tgatggaagc cagtcagaac 300

aactggctgc gaactgactg gatcacccga gaaggggcgc agagggtgta cattgaaatt 360

aagttcactc tgagggactg caacagtctt ccgggcgtca tggggacttg caaggagacg 420

tttaaccttt actactatga atcggacaac gacaaggagc gcttcatcag agagagccag 480
ttcggcaaga tcgacaccat cgcagcggat gagagcttta cacaggtgga cattggggac 540
agaatcatga agctcaacac cgagatccgg gatgtagggc ccctgagcaa gaagggtttt 600
tacctggctt ttcaggatgt gggtgcttgc atcgccctgg tgtctgttcg tgtgttctac 660
aagaagtgtc ccctcaccgt tcgaaatcta gcccagttcc ccgataccat cactggggct 720
gatacctctt ccctggtgga agttcgaggc tcctgtgtca acaactcaga agagaaggat 780
gtgccaaaaa tgtactgtgg ggcagatggt gaatggctgg tacccattgg caactgccta 840
tgcaatgctg ggcacgagga gcagaatggt gaatgccaag cttgcaaaat tggatattac 900
aaagctctct ccacggacgc cacctgtgcc aagtgtccac cgcacagcta ctctgtctgg 960
gaaggagcca cgtcctgcac ctgtgaccga ggctttttca gagctgacaa cgatgccgcg 1020
tccatgccct gcacccgccc accatctgct cccctcaact tgatttctaa tgtcaacgag 1080
acgtcggtga acttggaatg gagcagtcct cagaacacag gtggccgtca ggacatttct 1140
tacaacgtgg tctgcaagaa atgtggagct ggggatccca gcaagtgtcg accctgtgga 1200
agtggagtcc actacacgcc acagcagaat ggactcaaga cgaccagagt ctccatcact 1260
gacctcctag cacacaccaa ttacacgttt gagatctggg cagtgaatgg cgtgtctaag 1320
tacaacccta gcccagacca gtcagtgtct gtcacagtga caaccaacca agcagcacca 1380
tcatccattg ctttggtcca ggctaaagaa gttacaagat acagtgtggc tctggcttgg 1440
ctggaaccag atcgaccaaa cggagtcatc ttagaatatg aagtgaaata ttatgaaaag 1500
gatcagaatg aacgaagcta tcgcatagtt cggacagctg ccaggaacac agatatcaaa 1560
ggcctgaacc ctttgacttc ttacgtcttc cacgtgcgag ccaggaccgc cgctggctat 1620
ggagacttca gcgaaccctt ggaagtcact accaatacag tgccttcccg aatcattggc 1680
gacggggcca actctaccgt cctgctggtc tccgtctctg gcagtgtagt actcgtggtc 1740
attctcattg cggcatttgt gatcagccgt agacggagta agtacagcca agcaaagcaa 1800
gaagcagatg aagagaagca tttgaatcaa ggtgttagaa catacgtgga tccctttaca 1860
tatgaagacc ccaaccaggc agttcgagaa tttgccaaag aaatcgatgc ctcctgcatt 1920
aaaatcgaaa aggtcattgg agttggcgaa tttggagaag tctgcagtgg gcgtctcaaa 1980
gtgcccggca agagagagat ctgtgtggcc atcaagactc tgaaagctgg ttatactgac 2040
aagcagagga gagacttcct gagcgaggcc agcatcatgg gacagtttga ccacccaaac 2100
ataatccacc tggaaggcgt tgtcaccaaa tgtaaaccag taatgatcat cacggagtac 2160
atggagaacg gctccttgga cgctttcctc aggaagaatg atggccgatt tacagtcatt 2220
cagctggtgg gcatgctccg aggcattggc tcggggatga agtacttatc tgatatgagc 2280
tatgtgcatc gagacctggc tgccaggaac attctggtaa acagcaactt ggtctgcaag 2340
gtgtctgatt tcggcatgtc cagggtgctt gaggatgacc cggaagcagc ctatactacc 2400
aggggcggca agattcccat ccggtggact gcaccagaag caattgcgta tcgtaaattt 2460

acctcagcca gtgacgtctg gagctacgga atcgttatgt gggaagtgat gtcatatgga 2520
gagaggccgt actgggatat gtccaatcaa gatgtgatca aagccatcga ggaagggtac 2580
cggctacccc cgccaatgga ctgccccatt gccctccatc agttaatgct ggactgttgg 2640
cagaaagaga gaagcgacag gcctaaattt gggcagatcg tcaacatgtt ggacaaactc 2700
atccgcaacc ccaacagcct gaagaggaca gggccagaga gttccagacc aaacacagcc 2760
ttgttagatc ccagctcccc tgaattctcc gccgtagtat cagtgggtga ctggctgcag 2820
gccatcaaaa tggaccggta taaggacaac ttcacagccg ccgggtacac gacactagaa 2880
gctgtggttc acatgagcca ggatgacctg gcgcgaattg gcatcaccgc aatcacgcac 2940
cagaataaga ttttgagcag cgtccaggcg atgcgaaccc agatgcagca gatgcatggc 3000
aggatggttc ctgtctgagc cagtaccgaa cagactcaaa actctcgaat agtttacctc 3060
atccatgcac tttaattgaa gaactgcact ttttttactt cgtcttcgcc ctctgaaatt 3120
aaaggaaaac aaacaaacaa acaaacaaac aaaaacacaa atattatctg cagcgttgct 3180
tggtgtaaga gattgctgaa accgcggggc ttgtaatgaa tgaccttgat catcggaatt 3240
aaacccggag ccagtaattt ctcagaagaa ctctgtccat cgctggtgtg taaaatacat 3300
gtacttatat aaataaaact ctgcctctga gtttcgatgc tgtattcccc gtcagatacg 3360
tggcttttca agtcaccgga atcctttctc tgtctggaat ttcaacggaa ggatttcatc 3420
tgtggcatta actacagtca tcactcctta ctgggacgaa gaccctctgt gccccaggag 3480
cattgttaaa ggactcaatg gtggctgtca gggcttggtt tataccacag aggaaaatgc 3540
aggtgaaaac cacttggccc attcatgctg cctcgtcagt gcccaggaca cctggcggat 3600
tgttaggaca aagaggggtg ggggtggggc tagaaagaca agaaaatgga tttttaaaaa 3660
aatgcaaact cccaagccct attaccccta accggacaaa tgaggtttct ttgggctcga 3720
ggctgtgcca tataaagtct tgtttgggaa tgctacaaac ttatcataac taccttatgg 3780
atagtgggct gagacaatcc tgaatggaga attttcacac acccctttaa agaggcaact 3840
cccaaattta caagggaata gattctggta tctcagcctc tcagcccttc ctgttgatga 3900
cataagccct tggaagaaaa gagaacacac cctccttggt tccttctgaa tgtttttctt 3960
tggcttctct tctgccagtt ctgaggcaaa gacacgacaa aacacctgta cctgaaaagc 4020
aaaagaaatg tgtcaagtcc ccttcgccca ggaaaacatc aagctttggc agctgttctc 4080
cagttgcacg gaggaacgtg cgtctcaaga caccagctct gcagagccct tgctcccaaa 4140
ctccttgtgg ttttatttat acgtatttcc atatttcatt cctgtacgtc actgctgcat 4200
ccatgtggca gcacgtgacc aaatggtaca ggttttacta tggacgccag gtcaggcgcc 4260
accacgcaga acaaagccag ttccgtaagc tgcctccgat aagcattgct gaagtgacat 4320
tttacccagg gtgtgccatt gcagtgatac aaatatattt ttttcctaga ctaaaatatg 4380
acctgaatat ctcttttgat gtgtgtaaat aggtgtgagt gagtgagtat gtgtgtgtgt 4440
gtttgtgtgt gtgtgtgtgt gtgtgtgtgc ttgcatacgt gtgtgcgtgc gtgacctcat 4500

gcttagaact gcctgtgaat gttgtggaat gctatacctg gagagttctg gtttattacc 4560
agttcaaaga tagagagtca catgatatac gagacccgga gaccatgtcc gtccccttgg 4620
gaagacagct tggaaatact acgtgttctt tatccgtaag tgtaatgtgt ctgtcacatt 4680
catcctaaga agcagttctt tggacagttt tcaccccgat ggtggaatga gtttcttgat 4740
atacacctga ctggccagtt ccacaatgga gagagaaggg gatggaatcc gtatagtgac 4800
cccggatgga gtataagtcg cctttgtttc cctgcataaa tgaccttgct gaatgcgaac 4860
aaatatatgc aattcatata ctttttggag atgaacttag atatgcgtgt cagccttgag 4920
atgacgtgtc tggagtaata ctttgtgtcc cagtgtcaca gaaaaataca tccatgccag 4980
tggctcttga ggttaataat gtagttggga tggaatggcc tctagcaaat ccaggttccc 5040
caattacatg tagaaagttc tggtaattaa tatgcagcta actacttctg cccttgaaag 5100
tacatcggtc ttctagaatc agagttctgg tgtatgaaga ttcagtattg acaggatatg 5160
ttataccaaa ctccagccag catactgcca tgttacttaa cctgagtggc tgccttgctt 5220
attccgagcg atggttacag aagctgtgac catttatata agctataaca tcaaatcagg 5280
gaattaaaaa aaaaaaaaaa acgggggaaa aattctgaac catatattgt tgaataagta 5340
aagaaaatca ccaataaata tttattgcat tctgacaggg tgtgtgtggc attgtattct 5400
cgtaactatg ccagagtgac aaagctaatt cacccctttt ggggggacct taatatattt 5460
ttaaagggat gtgcctatgc attgatgcct gaaaaatatg tgtaaaaaaa aagatgaagt 5520
tgtacctttg agtggatcat ctttcccaaa ggtcaggtgg gaggaggtca acttccgggt 5580
ctgtgttcag ctcaggatcg agcgctggtc tcgtgagctg gaaggctaaa atctgttcaa 5640
atctactaag cagcttcgag ctgttccctg caccaaatca agttgtttca ccacgtgtct 5700
gcaggctctg agttattttt ggcaatctga taaatgaaat gttctggtgt gtttaaaaga 5760
atagaggaaa ggttgtgttc gcaacctcat ttctgtgtgt tctgagacct cttgtattca 5820
tctgtgaagg gtacgtgtat caatccatac actcctatag cctcaatgac ttgtctgaag 5880
acacttaaat tttgaccagt aaaggaaagt tctagaagca atattttcac tcctttagca 5940
ttgtccaaat aacatcaagt attaataaac gctgaagagt gtatttattt tgtattactc 6000
caagtctgtt ggaatatgca aggactgtgg ttataattag aatgtataag gcataattat 6060
aatttagttc attctgaaaa agaagttagc cggacatctc ttgtttcata catgttccaa 6120
attttgagca atcttttgag ttagccatag atttttttct attttgttct tatttgtcaa 6180
ggtatttttt tcttcccttc atgaacttta ggtacacata acttatgtca tttatttatg 6240
gtcttttata cctagtttgt aaagttgtaa aatagcaaac taaatgcaaa gagtttgcat 6300
ttgaaagtaa taaagtagtt gctgtattca aacctgaaat ctggttgtct atgttctatt 6360
tcattttctt cagtgttggg gacacacatg ctgggcaaat gcttcaccaa agagctaagc 6420
ccaggctcct tttacatcaa gacaagctct tactaagttg ctgagcatgg 6470

<210>  68
<211>  3349
<212>  DNA
<213>  human

<400>  68
ggtggactga gccgctcggg acagcggcac cggaggaggc tcggagaaga tgcggggctc      60

ggggccccgg ggtgcgggac accggcggcc cccaagcggc ggcggcgaca cccccatcac     120

cccagcgtcc ctggccggct gctactctgc acctcgacgg gctcccctct ggacgtgcct     180

tctcctgtgc gccgcactcc ggaccctcct ggccagcccc agcaacgaag tgaatttatt     240

ggattcacgc actgtcatgg gggacctggg atggattgct tttccaaaaa atgggtggga     300

agagattggt gaagtggatg aaaattatgc ccctatccac acataccaag tatgcaaagt     360

gatggaacag aatcagaata actggctttt gaccagttgg atctccaatg aaggtgcttc     420

cagaatcttc atagaactca aatttaccct gcgggactgc aacagccttc ctggaggact     480

ggggacctgt aaggaaacct ttaatatgta ttactttgag tcagatgatc agaatgggag     540

aaacatcaag gaaaaccaat acatcaaaat tgataccatt gctgccgatg aaagctttac     600

agaacttgat cttggtgacc gtgttatgaa actgaataca gaggtcagag atgtaggacc     660

tctaagcaaa aagggatttt atcttgcttt tcaagatgtt ggtgcttgca ttgctctggt     720

ttctgtgcgt gtatactata aaaaatgccc ttctgtggta cgacacttgg ctgtcttccc     780

tgacaccatc actggagctg attcttccca attgctcgaa gtgtcaggct cctgtgtcaa     840

ccattctgtg accgatgaac ctcccaaaat gcactgcagc gccgaagggg agtggctggt     900

gcccatcggg aaatgcatgt gcaaggcagg atatgaagag aaaaatggca cctgtcaagt     960

gtgcagacct gggttcttca aagcctcacc tcacatccag agctgcggca aatgtccacc    1020

tcacagttat acccatgagg aagcttcaac ctcttgtgtc tgtgaaaagg attatttcag    1080

gagagagtct gatccaccca caatggcatg cacaagaccc ccctctgctc ctcggaatgc    1140

catctcaaat gttaatgaaa ctagtgtctt tctggaatgg attccgcctg ctgacactgg    1200

tggaaggaaa gacgtgtcat attatattgc atgcaagaag tgcaactccc atgcaggtgt    1260

gtgtgaggag tgtggcggtc atgtcaggta ccttccccgg caaagcggcc tgaaaaacac    1320

ctctgtcatg atggtggatc tactcgctca cacaaactat acctttgaga ttgaggcagt    1380

gaatggagtg tccgacttga gcccaggagc ccggcagtat gtgtctgtaa atgtaaccac    1440

aaatcaagca gctccatctc cagtcaccaa tgtgaaaaaa gggaaaattg caaaaaacag    1500

catctctttg tcttggcaag aaccagatcg tcccaatgga atcatcctag agtatgaaat    1560

caagtatttt gaaaaggacc aagagaccag ctacacgatt atcaaatcta aagagacaac    1620

tattactgca gagggcttga aaccagcttc agtttatgtc ttccaaattc gagcacgtac    1680

agcagcaggc tatggtgtct tcagtcgaag atttgagttt gaaaccaccc cagtgtttgc    1740

agcatccagc gatcaaagcc agattcctgt aattgctgtg tctgtgacag tgggagtcat    1800

tttgttggca gtggttatcg gcgtcctcct cagtggaagt tgctgcgaat gtggctgtgg    1860

gagggcttct tccctgtgcg ctgttgccca tccaagccta atatggcggt gtggctacag 1920
caaagcaaaa caagatccag aagaggaaaa gatgcatttt cataatgggc acattaaact 1980
gccaggagta agaacttaca ttgatccaca tacctatgag gatcccaatc aagctgtcca 2040
cgaatttgct aaggagatag aagcatcatg tatcaccatt gagagagtta ttggagcagg 2100
tgaatttggt gaagtttgta gtggacgttt gaaactacca ggaaaaagag aattacctgt 2160
ggctatcaaa acccttaaag taggctatac tgaaaagcaa cgcagagatt tcctaggtga 2220
agcaagtatc atgggacagt ttgatcatcc taacatcatc catttagaag gtgtggtgac 2280
caaaagtaaa ccagtgatga tcgtgacaga gtatatggag aatggctctt tagatacatt 2340
tttgaagaaa aacgatgggc agttcactgt gattcagctt gttggcatgc tgagaggtat 2400
ctctgcagga atgaagtacc tttctgacat gggctatgtg catagagatc ttgctgccag 2460
aaacatctta atcaacagta accttgtgtg caaagtgtct gactttggac tttcccgggt 2520
actggaagat gatcccgagg cagcctacac cacaagggga ggaaaaattc caatcagatg 2580
gactgcccca gaagcaatag ctttccgaaa gtttacttct gccagtgatg tctggagtta 2640
tggaatagta atgtgggaag ttgtgtctta tggagagaga ccctactggg agatgaccaa 2700
tcaagatgtg attaaagcgg tagaggaagg ctatcgtctg ccaagcccca tggattgtcc 2760
tgctgctctc tatcagttaa tgctggattg ctggcagaaa gagcgaaata gcaggcccaa 2820
gtttgatgaa atagtcaaca tgttggacaa gctgatacgt aacccaagta gtctgaagac 2880
gctggttaat gcatcctgca gagtatctaa tttattggca gaacatagcc cactaggatc 2940
tggggcctac agatcagtag gtgaatggct agaggcaatc aagatgggcc ggtatacaga 3000
gattttcatg gaaaatggat acagttcaat ggacgctgtg gctcaggtga ccttggagga 3060
tttgagacgg cttggagtga ctcttgtcgg tcaccagaag aagatcatga acagccttca 3120
agaaatgaag gtgcagctgg taaacggaat ggtgccattg taacttcatg taaatgtcgc 3180
ttcttcaagt gaatgattct gcactttgta aacagcactg agatttattt taacaaaaaa 3240
agggggaaaa gggaaaacag tgatttctaa accttagaaa acatttgcct cagccacaga 3300
atttgtaatc atggttttac tgaagtatcc agttcttagt ccttagtct 3349

<210> 69
<211> 4759
<212> DNA
<213> mouse

<400> 69
agacacagca ggagcgagcg agcgagcgag cgagcgagcg ccgcgcgggt gcagtcgccg 60
gttcgccctg gtcgccgcgc actccgcctc cgcgctgagc agcgggaacc gcagcggctc 120
gagcctccgg gctctgctgc gctctcgggc ccgcgctcca gcccgctccg ccggtcgcac 180
cgcccccggc cgagctcagc ccgcagcgat ttgacttccc tctgccagcc tctccagttc 240
tctagatcgc ttgctgcctg ggctccactc tactcggtgt ttgccacaca ccgtttgctc 300
tactcttctc cccttgatcc gtagacactc tgcttgattc ccccctcctc cctcctctct 360

gtctctctct ctctctctgt ctctctctct gtctctctct ctcttttatt gaaaacatca 420
tcttgattta agatgactcg tcttctttaa tctcgtccat atcggcactg aagactgaaa 480
gggaaccttc accaccactc cgaccctggt ggcacttaaa aaaaaaaaag aagaagaaga 540
agaagggggg tgccagaaaa agaaaaagtc ttaaagggca aagaagcggg actcccgacc 600
ctctacagac ccttgaccga gcccacccag gactgtacta gccatcagaa cttctaattc 660
atcttatcca actgaaaggg agggcgacag gagccagaag caaacttctt cagcggtctc 720
tgcggatctg tgattcccgc atttagaggg gtcgagagcc agaaggtaca ggaccccacc 780
aggaggagga gttccacgtc tctctcgccc cttccaccaa gcctgaacct taggctgagc 840
cacgcaggac acaagaggca gaagagggta gtagaaaatg cggggctccg ggccccgcgg 900
tgcgggacac cgacggaccc agggcagagg tggcggcgac gacacccccc gcgtccctgc 960
ctctctggca ggctgctatt cggcacctct aaagggcccc ctctggacgt gccttctctt 1020
gtgtgcggcg ctccggaccc ttttggccag ccccagcaac gaagtgaatt tgttggattc 1080
gcgcactgtc atgggggacc ttggatggat tgcttttcca aagaacgggt gggaagagat 1140
tggtgaagtt gatgagaact atgcccccat ccacacatac caagtgtgca aagttatgga 1200
acagaatcag aataattggc tgttgaccag ttggatctct aacgaaggtg cttccagaat 1260
ctttattgaa ctcaagttta ctttaaggga ctgcaacagc cttcctggag gactggggac 1320
ttgtaaggag acatttaaca tgtattattt tgaatcagat gatgagaatg ggagaagtat 1380
caaagagaac caatacatca agattgatac catcgctgca gatgagagct tcacagaact 1440
tgatcttggt gaccgtgtca tgaaactgaa tacagaggtc agagatgtcg gacctctgag 1500
caaaaaggga ttttatcttg ctttccaaga tgtcggtgct tgcattgctc tggtttctgt 1560
ccgtgtctac tataaaaagt gtccctctgt agtaagacac ttggctatct tccctgacac 1620
tatcactgga gcagattcat cacagttgtt agaggtgtca ggctcctgcg tcaaccattc 1680
tgtgacagat gatcctccca agatgcattg cagtgctgaa ggggagtggc tggttcccat 1740
tgggaaatgc atgtgcaagg ctggatatga agagaaaaat ggtacctgcc aagctccttc 1800
tccagtcacc aatgtgaaaa aggggaagat tgcaaagaac agcatttctt tgtcttggca 1860
agagccagat cgccccaatg gaattatcct ggagtatgaa atcaagtact ttgaaaagga 1920
ccaagagacc agttacacaa ttatcaagtc taaagagacc agtattacag ccgagggcct 1980
gaaacctgca tctgtgtatg tcttccaaat tcgagcacgt acagcagcag gctacggcgt 2040
cttcagtcga agatttgagt ttgaaaccac accagtgtca gttgcagcat ctaatgatca 2100
aagccagatt cccatcattg cagtgtcagt gacagtggga gtcatcttgt tggcagtgat 2160
gatcggcttc ctcctcagtg gcagttgctg cgattgtggc tgtgggaggg cttcttccct 2220
gtgcgctgtt gcccatccaa gcctaatatg gcggtgtggc tacagcaaag caaagcagga 2280
tccagaagag gaaaagatgc actttcataa cgggcacatt aaactgccag gagtcagaac 2340
ctatattgat ccgcacactt atgaagatcc caatcaagct gttcatgaat ttgcgaagga 2400

gattgaagct tcatgcatca ccattgagag agtgatcgga gcaggtgaat ttggtgaagt 2460
ttgcagtgga cgtttgaaac tacctggaaa aagagaatta cctgtggcta tcaaaactct 2520
taaagtaggc tatactgaaa agcagcgcag agatttcctg ggtgaagcaa gtattatggg 2580
gcagttcgat catccaaaca tcatccatct agaaggtgtt gtgactaaaa gcaaacctgt 2640
gatgatagtg acagagtaca tggagaacgg ctccttagac acgtttttaa agaaaaacga 2700
tgggcagttc actgtgattc agcttgttgg catgctgaga ggcatcgctg caggaatgaa 2760
gtacctttct gacatgggct acgtgcatag agaccttgct gctagaaaca tcttaatcaa 2820
cagtaacctt gtgtgcaagg tgtctgactt tggactttcc agggtactgg aagatgatcc 2880
tgaggcagcc tacaccacaa ggggaggcaa aattccaatc agatggactg ccccggaggc 2940
aatagctttt cgaaagttca cctcttccag tgatgtctgg agctatggca ttgtaatgtg 3000
ggaagttgta tcttatggag agagacccta ctgggagatg accaatcagg atgtgatcaa 3060
ggcagtagaa gaaggctacc gcctgccaag ccccatggat tgccctgctg ctctctatca 3120
attaatgctg gattgctggc agaaagatcg aaacagcagg cccaagtttg atgaaatcgt 3180
caacatgctg gacaaactga tacgaaaccc aagtagtctg aagacactgg tgaatgcgtc 3240
gagcagagtg tctacattgt tggcagaaca tggttctttg gggtctgggg cctacagatc 3300
agtaggtgaa tggctggaag caatcaaaat gggtcggtac acagagattt tcatggaaaa 3360
tggatacagt tcaatggacg ctgtggctca ggtgaccttg gaggatttga ggcgcctggg 3420
agtgactctg gtcggtcacc agaagaagat catgagcagc cttcaagaga tgaaggtgca 3480
gatggtaaac gggatggtgc cagtgtgacc cgcgcatggg tcacttctcc aagtgaacaa 3540
ctcagcactt tgtaaacaac cctgagattt attttaacag agaaagggga aagggtggtt 3600
tctaaacctt tggaggcatt tgccttaacc tttgagttta taatcaatat tttactaaaa 3660
tctccagatc ttcctcttaa tccacaatgt acaggtaacc tgcaaagaga gctaacatga 3720
cgatcaacat cctttattaa aatagtaacg aaatcttccc cacttcttcc atggagtctt 3780
gtacaggaaa tgtatcaagc tagagcacct ttagagactg ttaaggcagc ccctttcaaa 3840
acttccaggg atcaacttga aaggaaaagt tttaaagcca tgtgtgggct aagaaaagct 3900
gcattttact gacgtttact tcaagtctta attgtctaca tgagtgtatt gaagagcaat 3960
atgattagat tatttcttaa atagactttg taatttaaaa tgaaattaca tgttgttaag 4020
ttatagaaga tagtttataa acatgttgct tggtcaagga aaagttcaat acagggtgta 4080
tatttatttt tctgtgttag aaaatttact tttagttgct cttctagaga gtattaggta 4140
atgaatgtgt atatatgtat agtttgcaat atatgcagga actgacttac atcgaaattg 4200
tgtgtgtgtg tgtgtgtgcg tgcgtgcgcg tgtgtatgtg tatgtatttg aatatgtgga 4260
gggtattttt ttgcttgcct tttggatagt gttttaattt tgacgacata caggaaagtg 4320
tcttctagac tctatcatgg gtaaaagagt agaaagccgt agaccaaatt cccatccaag 4380
ttggtatctt atgctctcat ttttaaagca actaattagt ttatttaaac agctaatgcc 4440

tctagactaa attccctttg gattttacaa acactcccta cctgcctgtg acagagaagt 4500

tcttaccatt aatctttcat gcttctttga aattccacct aagttcctga aaatatcttc 4560

taaagcaaat ttgacacaaa gggcacactt gtgaagaaga agagagatgt gactgcgctg 4620

gcttctgtta ccatccaatt caggcagagg gtactctata tctcccttca tgtttatcca 4680

tttgtgacca cttccactgt gactcattca cagagagttt tatactaaga cagcattaaa 4740

aagtgaagaa aaattatga 4759

<210> 70
<211> 3201
<212> DNA
<213> rat

<400> 70
atgcggggct ccgggccccg cggtgcggga cgccgacgga cccagggcag aggtggcggc 60

ggcgacaccc cccgcgtccc tgcctctctg gcaggctgct attccgcacc tctaaagggg 120

cccctctgga cgtgccttct cctgtgtgcg gcgctccgga cccttttggc cagccccagt 180

aacgaagtga atttattgga ttcgcgcact gtcctgggag accttggatg gattgctttt 240

ccaaagaatg ggtgggaaga gattggtgaa gttgatgaaa actatgcccc catccacacc 300

tatcaagtgt gcaaagttat ggaacagaat cagaataatt ggctgttgac cagttggatc 360

tctaacgaag gtgcttccag aatttttatt gaactcaagt ttactctgag ggattgcaac 420

agccttcctg gaggactggg gacttgcaag gagaccttta acatgtatta ttttgagtcg 480

gatgatgaga atgggagaaa tatcaaagag aaccagtaca tcaagatcga taccattgct 540

gctgatgaga gcttcaccga acttgacctt ggagaccggg tcatgaagct gaatacggag 600

gtcagagatg taggacctct gagcaaaaag ggattttatc ttgctttcca agatgtcggt 660

gcttgcatcg ctctggtttc tgtccgtgtc tactataaaa aatgtccttc tgtagttaga 720

catttggctg ttttccctga cacgatcact ggagcagatt cttcccagtt gctagaggtg 780

tcaggctcct gcgtcaacca ttctgtgaca gacgatcctc ccaaaatgca ttgcagtgct 840

gaaggggagt ggctggttcc catcgggaaa tgcatgtgca aggccggata tgaagagaaa 900

aatggtacct gtcaagtgtg cagacctggg ttcttcaaag cctctcctca cagccagacc 960

tgcagcaaat gtccacctca cagttacacc catgaggaag cttccacctc ttgtgtctgt 1020

gaaaaggatt atttcaggag ggaatctgat ccgcccacaa tggcatgcac aagacccccc 1080

tcagctcctc ggaatgccat ctcaaatgtt aatgaaacta gtgtctttct ggagtggatt 1140

cctcctgctg acactggtgg acggaaagat gtgtcctatt acattgcatg caagaagtgc 1200

aactcccatg caggtgtgtg tgaggagtgt ggcggtcatg tcaggtacct cccccagcaa 1260

atcggcctga aaaatacctc tgtcatgatg gcggacctcc ttgctcacac aaactatacc 1320

tttgaaattg aggcagtgaa cggagtttcc gacttgagtc caggcacccg gcagtatgtg 1380

tctgtaaatg taaccacgaa ccaagcagct ccttctccag tcaccaacgt gaagaagggg 1440

aagatcgcaa agaacagcat ttctttgtct tggcaagagc cagatcgccc caatgggatc 1500
atcctggagt acgaaatcaa gtactttgaa aaggaccaag agaccagcta cacaattatc 1560
aagtctaaag agaccactat tacggcagag ggcctgaaac ctgcgtctgt gtatgtcttc 1620
caaattcgag cacgtacagc agcaggctac ggcgtcttca gtcgacggtt tgagtttgaa 1680
accacaccag tgtttgcagc atctaatgat caaagccaga ttcccatcat tgcggtgtca 1740
gtgacggtgg gagtcatctt gttggcagtg atgatcggct tcctcctcag tggcagttgc 1800
tgcgatatgt ggctgtggga gggcttcttc cctgtgcgct gttgcccatc caagcctaat 1860
atggcggtgt ggctacagca aagcaaagca ggatccagaa gaggaaaaga tgcactttca 1920
taacgggcac attaaactgc caggagtaag aacgtacatt gatccacaca cctacgaaga 1980
tcccactcaa gccgttcatg aatttgccaa ggagatcgag gcctcatgca tcaccattga 2040
gagagttatt ggagcaggtg aatttggtga agtttgtagt ggacgtttga aactacccgg 2100
aaaaagagaa ttgcctgtgg ctatcaaaac tcttaaagta ggctacactg aaaagcagcg 2160
cagagatttt ctgagtgaag cgagtatcat ggggcagttt gatcatccaa acatcatcca 2220
tctagaaggc gttgtgacta aaagtaaacc tgtgatgata gtgacagagt acatggagaa 2280
tggctcctta gacacatttt taaagaaaaa cgatggccag ttcactgtga ttcagcttgt 2340
tggcatgctg agaggcatcg ctgcaggaat gaagtacctt tctgacatgg gctacgtgca 2400
cagagacctt gctgctagaa acatcttaat caacagtaac cttgtgtgca aagtgtctga 2460
ctttggactt tccagggtgc tggaagatga tcctgaggca gcctatacca caaggggagg 2520
caaaattcca atcaggtgga ctgctccaga agcaatagct tttcgaaagt ttacctctgc 2580
cagtgatgtc tggagctatg gaatcgtaat gtgggaagtt gtgtcctatg gagagagacc 2640
gtactgggag atgaccaatc aggatgtgat caaggcagtg gaagaaggct accgcctgcc 2700
aagccccatg gattgtcctg ctgccctcta tcaattaatg ctggattgct ggcagaaaga 2760
tcgcaacagc aggcccaagt ttgatgacat agtcaacatg ctggataagc tgatacgaaa 2820
cccaagtagt ttgaagacac tggtgaacgc gtcaagcagg gtatctactt tgttggcaga 2880
acatggatct ttggggtctg gggcctacag atcagtaggt gaatggctag aagcaatcaa 2940
aatgggccgg tacacagaga ttttcatgga aaatggatac agttcaatgg acgctgtggc 3000
tcaggtgacc ttggaggatt tgagacgcct gggagtgact ctggtcggtc accagaagaa 3060
gatcatgaac agccttcaag agatgaaggt gcagctggta aacgggatgg tgccagtgtg 3120
acccgcacac gggtcacttc tccaagtgaa caactctgca ctttgtaaac agccctaaga 3180
tttattttaa cagagaaagg g 3201

<210> 71
<211> 5229
<212> DNA
<213> human

<400> 71
gcagtcggag acttgcaggc agcaaacacg gtgcgagcga acaggagtgg gggggaaatt 60

aaaaaaagct aaacgtggag cagccgatcg gggaccgaga aggggaatcg atgcaaggag 120
cacaataaaa caaaagctac ttcggaacaa acagcattta aaaatccacg actcaagata 180
actgaaacct aaaataaaac ctgctcatgc accatggttt ttcaaactcg gtacccttca 240
tggattattt tatgctacat ctggctgctc cgctttgcac acacagggga ggcgcaggct 300
gcgaaggaag tactactgct ggattctaaa gcacaacaaa cagagttgga gtggatttcc 360
tctccaccca atgggtggga agaaattagt ggtttggatg agaactatac cccgatacga 420
acataccagg tgtgccaagt catggagccc aaccaaaaca actggctgcg gactaactgg 480
atttccaaag gcaatgcaca aaggattttt gtagaattga aattcaccct gagggattgt 540
aacagtcttc ctggagtact gggaacttgc aaggaaacat ttaatttgta ctattatgaa 600
acagactatg acactggcag gaatataaga gaaaacctct atgtaaaaat agacaccatt 660
gctgcagatg aaagttttac ccaaggtgac cttggtgaaa gaaagatgaa gcttaacact 720
gaggtgagag agattggacc tttgtccaaa aagggattct atcttgcctt tcaggatgta 780
ggggcttgca tagctttggt ttctgtcaaa gtgtactaca agaagtgctg gtccattatt 840
gagaacttag ctatctttcc agatacagtg actggttcag aattttcctc tttagtcgag 900
gttcgaggga catgtgtcag cagtgcagag gaagaagcgg aaaacgcccc caggatgcac 960
tgcagtgcag aaggagaatg gttagtgccc attggaaaat gtatctgcaa agcaggctac 1020
cagcaaaaag gagacacttg tgaaccctgt ggccgtgggt tctacaagtc ttcctctcaa 1080
gatcttcagt gctctcgttg tccaactcac agtttttctg ataaagaagg ctcctccaga 1140
tgtgaatgtg aagatgggta ttacagggct ccatctgacc caccatacgt tgcatgcaca 1200
aggcctccat ctgcaccaca gaacctcatt ttcaacatca accaaaccac agtaagtttg 1260
gaatggagtc ctcctgcaga caatggggga agaaacgatg tgacctacag aatattgtgt 1320
aagcggtgca gttgggagca gggcgaatgt gttccctgtg ggagtaacat tggatacatg 1380
ccccagcaga ctggattaga ggataactat gtcactgtca tggacctgct agcccacgct 1440
aattatactt ttgaagttga agctgtaaat ggagtttctg acttaagccg atcccagagg 1500
ctctttgctg ctgtcagtat caccactggt caagcagctc cctcgcaagt gagcggagta 1560
atgaaggaga gagtactgca gcggagtgtc gagctttcct ggcaggaacc agagcatccc 1620
aatggagtca tcacagaata tgaaatcaag tattacgaga aagatcaaag ggaacggacc 1680
tactcaacag taaaaaccaa gtctacttca gcctccatta ataatctgaa accaggaaca 1740
gtgtatgttt tccagattcg ggcttttact gctgctggtt atggaaatta cagtcccaga 1800
cttgatgttg ctacactaga ggaagctaca ggtaaaatgt ttgaagctac agctgtctcc 1860
agtgaacaga atcctgttat tatcattgct gtggttgctg tagctgggac catcattttg 1920
gtgttcatgg tctttggctt catcattggg agaaggcact gtggttatag caaagctgac 1980
caagaaggcg atgaagagct ttactttcat tttaaatttc caggcaccaa aacctacatt 2040
gaccctgaaa cctatgagga cccaaataga gctgtccatc aattcgccaa ggagctagat 2100

gcctcctgta ttaaaattga gcgtgtgatt ggtgcaggag aattcggtga agtctgcagt 2160
ggccgtttga aacttccagg gaaaagagat gttgcagtag ccataaaaac cctgaaagtt 2220
ggttacacag aaaaacaaag gagagacttt ttgtgtgaag caagcatcat ggggcagttt 2280
gaccacccaa atgttgtcca tttggaaggg gttgttacaa gagggaaacc agtcatgata 2340
gtaatagagt tcatggaaaa tggagcccta gatgcatttc tcaggaaaca tgatgggcaa 2400
tttacagtca ttcagttagt aggaatgctg agaggaattg ctgctggaat gagatatttg 2460
gctgatatgg gatatgttca cagggacctt gcagctcgca atattcttgt caacagcaat 2520
ctcgtttgta aagtgtcaga ttttggcctg tcccgagtta tagaggatga tccagaagct 2580
gtctatacaa ctactggtgg aaaaattcca gtaaggtgga cagcacccga agccatccag 2640
taccggaaat tcacatcagc cagtgatgta tggagctatg gaatagtcat gtgggaagtt 2700
atgtcttatg gagaaagacc ttattgggac atgtcaaatc aagatgttat aaaagcaata 2760
gaagaaggtt atcgtttacc agcacccatg gactgcccag ctggccttca ccagctaatg 2820
ttggattgtt ggcaaaagga gcgtgctgaa aggccaaaat ttgaacagat agttggaatt 2880
ctagacaaaa tgattcgaaa cccaaatagt ctgaaaactc ccctgggaac ttgtagtagg 2940
ccaataagcc ctcttctgga tcaaaacact cctgatttca ctaccttttg ttcagttgga 3000
gaatggctac aagctattaa gatggaaaga tataaagata atttcacggc agctggctac 3060
aattcccttg aatcagtagc caggatgact attgaggatg tgatgagttt agggatcaca 3120
ctggttggtc atcaaaagaa aatcatgagc agcattcaga ctatgagagc acaaatgcta 3180
catttacatg gaactggcat tcaagtgtga tatgcatttc tcccttttaa gggagattac 3240
agactgcaag agaacagtac tggccttcag tatatgcata gaatgctgct agaagacaag 3300
tgatgtcctg ggtccttcca acagtgaaga gaagatttaa gaagcaccta tagacttgaa 3360
ctcctaagtg ccaccagaat atataaaaag ggaatttagg atccaccatc ggtggccagg 3420
aaaatagcag tgacaataaa caaagtacta cctgaaaaac atccaaacac cttgagctct 3480
ctaacctcct tttgtcttta tagacttttt aaaatgtaca taaagaattt aagaaagaat 3540
atatttgtca aataaaatca tgatcttatt gttaaaatta atgaaatatt ttccttaaat 3600
atgtgatttc agactattcc tttttaaaat catttgtgtt tattcttcat aaggactttg 3660
ttttagaaag ctgtttatag ctttggacct ttttagtgtt aaatctgtaa cattactaca 3720
ctgggtacct ttgaaagaat ctcaaatttc aaaagaaata gcatgattga agatacatct 3780
ctgttagaac attggtatcc tttttgtgcc attttattct gtttaatcag tgctgttttg 3840
atattgtttg ctaattggca ggtagtcaag aaaatgcaag ttgccaagag ctctgatatt 3900
ttttaaaaag aatttttttg taaagatcag acaacacact atcttttcaa tgaaaaaagc 3960
aataatgatc catacatact ataaggcact tttaacagat tgtttataga gtgattttac 4020
tagaaagaat ttaataaact cgaagtttag gtttatgagt atataaacaa atgaggcact 4080
tcatctgaag aatgttggtg aaggcaagtc tctgaaagca gaactatcca gtgttatcta 4140

aaaattaatc tgagcacatc aagatttttt cattctcgtg acattaggaa atttaggata 4200

aatagttgac atatatttta tatcctcttc tgttgaatgc agtccaaaca tgaaaggaaa 4260

taattgtttt atattataac tctgaagcat gataaagggg cagttcacaa ttttcaccat 4320

ttaaacacaa atttgctgca cagaatatca ccattgcagt tcaaaacaaa acaaaacaaa 4380

aagtcttttg tttgtgaaca ctgatgcaag aaacttgtta aatgaaagga ctctttaccc 4440

tagaaggaag aggtgaagga tctggcttgt ttttaaagct ttatttatta aaccatatta 4500

tttgattact gtgttagaat ttcataagca ataattaaat gtgtctttat agatattgca 4560

ggaatgtata catattgtga ttaatgcttt caaaacttat gaaaatcatg aactacccca 4620

gaattgaact gttgtacttc caaagagaat tgggctgttt ataatgattt taatagagaa 4680

agatcccagg gatcggtcat aattggtctt gtttgataat gtgggcatcc acaaacaaac 4740

aaacaaataa cagaaacaaa atctgtaaat gttcctttgt aaaacttgta aattttattt 4800

atactgtctt gttttgtaca cacatttctc tgtagtgggc tctgaataca ttgaaaatgc 4860

actatatttt tctattttac ttgcagagca tcacaaaaga acaggtattt tcagtgctac 4920

ataatgtgtt ttcccacatt taggaccaaa gacggctata gaaaaactca aatggattgc 4980

ttcccaaacc cctccccacc cttttttttt ggttttaaat cactgtacag tgttatttga 5040

tattttaatt tattttttga ttgactagaa aaatcatttt aatttcacta aaatgttttt 5100

tgtccctaag gaaaagtaat ctgtaaaaat aattttaatt agcataatac agtcacctag 5160

acacttccat ttgtaatctt tgtaatagac tgtaaatata tttttggaac tataaaaaaa 5220

aaaaaaaaa 5229

<210> 72
<211> 4304
<212> DNA
<213> mouse

<400> 72
aagcggccgg tctgcagtcg gagacttgca ggcagcaaac acggtgcgaa cgaaccggag 60

gggggagaga gaaatcaaac agctaagcgt ggagcagacg gcctgggacc cagaagggga 120

tcgatgcgag gagcgcaata ataacaacaa taataaccca cttcggagca aacagcatct 180

aaagagctgc gacccaactg cagcctaaaa aaatcaaacc tgctcatgca ccatggttgt 240

tcaaactcgg ttcccttcgt ggattatttt gtgttacatc tggctgcttg gctttgcaca 300

cacgggggag gcgcaggctg cgaaggaagt actattactg gactcgaaag cacaacaaac 360

agaattggaa tggatttcct ctccacccag tgggtgggaa gaaattagtg gtttggatga 420

gaactacact ccgataagaa cataccaggt gtgccaggtc atggagccca accagaacaa 480

ctggctgcgg actaactgga tttctaaagg caacgcacaa aggatttttg tagaattgaa 540

attcaccttg agggattgta atagtcttcc cggagtcctg ggaacttgca aggaaacgtt 600

taatttgtac tattatgaaa cagactacga caccggcagg aatatacgag aaaaccttta 660

tgttaaaata gacaccattg ctgcagatga aagtttcaca caaggtgacc ttggtgaaag 720
aaagatgaag ctgaacactg aggtgagaga gattggacct ttgtccaaaa agggattcta 780
tcttgccttt caggatgtag gggcttgcat agcattggtt tctgtcaaag tgtactacaa 840
gaagtgctgg accattgttg agaacttagc tgtctttcca gatacagtga ctggttcgga 900
attttcctcc ttagtcgagg tccgtgggac atgtgtcagc agtgccgagg aagaggcaga 960
aaattccccc agaatgcatt gcagtgcaga aggagagtgg ctagtaccca ttggaaaatg 1020
catctgcaaa gcaggctatc agcaaaaagg ggacacttgc gaaccctgtg gccgcaggtt 1080
ctacaaatct tcctctcagg atctccagtg ttctcgttgt ccaacccaca gcttctctga 1140
ccgagaagga tcatccaggt gtgaatgtga agatgggtac tacagagctc cttctgatcc 1200
accatacgtt gcatgcacga ggcctccctc tgcaccacag aaccttattt tcaatatcaa 1260
tcaaacgact gtaagtttgg aatggagtcc tccggctgac aacgggggaa gaaacgatgt 1320
cacctacaga atactgtgta agcggtgcag ttgggaacag ggagaatgtg tgccatgcgg 1380
aagtaacatt ggatacatgc cccagcagac gggattagag gataactatg tcactgtcat 1440
ggacctactt gcccatgcaa attacacttt cgaagttgaa gctgtaaatg gagtttcgga 1500
cttaagcaga tcccagaggc tcttcgctgc tgttagcatc accaccggtc aagcagctcc 1560
ctcgcaagtg agtggagtca tgaaggagcg agtactgcag cggagtgtgc agctttcctg 1620
gcaggagccg gagcatccca atggagtcat cacggaatat gaaatcaagt attatgagaa 1680
agatcaacgg gaaaggacgt actcaacact caaaaccaag tccacctccg cctccattaa 1740
taatctgaaa ccgggaacag tgtacgtctt tcagatccgg gcggtcactg ctgccggtta 1800
tggaaactac agccctaggc ttgatgttgc cacacttgag gaagcttcag gtaaaatgtt 1860
tgaagcgaca gcagtctcca gtgaacagaa tcctgtcatc ataattgctg tagtggctgt 1920
agcagggacc atcatcttgg tgttcatggt gttcggcttc atcattggaa gaaggcactg 1980
tggttatagc aaggctgacc aagaagggga tgaagaactc tactttcatt ttaaatttcc 2040
aggcaccaaa acctacattg accctgaaac ctatgaggac ccaaatagag ctgtccatca 2100
attcgccaag gagctagatg cctcctgtat taaaattgag cgtgtgattg gtgcaggaga 2160
atttggagaa gtttgcagtg gtcgtttgaa acttccgggc cagagagatg ttgcagtggc 2220
cataaaaacc ctgaaagttg gttacacaga aaagcaaagg agggactttt tatgcgaagc 2280
aagcatcatg gggcaatttg accacccaaa tgtcgtccat ttggaagggg ttgttacaag 2340
agggaagcct gtcatgattg tgatagagtt catggagaat ggagccctgg atgcatttct 2400
caggaaacac gatgggcagt ttacagtcat tcagttggta ggaatgttga gaggtattgc 2460
cgctgggatg cgatacttgg ctgatatggg atacgttcac agggaccttg cagcgcgcaa 2520
catccttgtc aacagcaatc ttgtttgtaa agtgtcagat tttggccttt cccgggttat 2580
agaggatgat cccgaagctg tctacaccac gactggtgga aaaattccag taaggtggac 2640
tgcaccggaa gccattcaat accggaagtt cacctcagcc agcgatgtgt ggagctatgg 2700

gattgtcatg tgggaagtga tgtcttatgg agaaagacct tactgggaca tgtcaaatca 2760

agatgtcatt aaagcgatag aagaaggtta tcgtttgccg gcgcccatgg attgcccagc 2820

tggtcttcac cagctaatgc tggattgttg gcagaaagat cgggcggaaa ggccaaagtt 2880

tgagcagata gtcggaattc tagacaaaat gattcgaaac ccaagtagtc tgaaaacacc 2940

cctgggaact tgtagtagac ccttaagccc tcttctggac cagagcactc ctgacttcac 3000

tgccttctgt tcagttggag aatggttgca agctattaaa atggaaaggt ataaggacaa 3060

cttcacagca gcgggttaca actcactcga gtcagtggcc aggatgacta tcgatgatgt 3120

gatgagttta gggatcacac tggttggcca tcaaaagaag atcatgagca gcatccagac 3180

tatgcgggca caaatgttgc atttacacgg aacaggcatc caagtgtgac acatcggcct 3240

ccctcagatg aggcttaaga ctgcaggaga acagttctgg ccttcagtat acgcatagaa 3300

tgctgctaga agacagttga tatactgggt ccttcctaca agaaagagaa gattttagaa 3360

gcacctccag acttgaactc ctaagtgcca ccagaatata caaaaaggga atttaggatc 3420

caccactggt ggccaggaac acagcagaga caataaacaa agtactacct gaaaaacatc 3480

ccaacacctt gagctctcga acctcctttt tatcttatag actttttaaa aatgtacata 3540

aagaatttaa gaaagaatat atttgtcaaa taaaaatcat gatcttattg ttaaaatcaa 3600

tgaaatattt tccttaaaat atgtgatttc agactattct tttccagaac catctgtgtt 3660

tattctgctt aaggactttg ttttagaaag ttatttgtag ctttggacct ttttagtgtt 3720

aaatttatga cacgttacta cactgggaac ctttgaagac tctcaaactt aaaggaaagc 3780

aaaactacgc acatagtcga ggatggactt tgtccttcat ggctttggta tcctggctgt 3840

gtcattttgt taaaccagtg atgttttcat attgtttgct gattggcagg tagttcaaaa 3900

ttgcaagttg ccaagagctc tgatattttt taacaggatt tttttttctt tgtaaaaatc 3960

agataacata ctaacttttc aatgaaaaaa aaaaaaaaag aagcaataat gatccataaa 4020

tactataagg cacttttaac agattgttta tagagtgatt tactaggcag aatttaataa 4080

aaaaaaaaga gagatgtcaa attttaggtt tatgtgtata tgataaaagg ctgagcttcg 4140

tctgaagatg ctggtgaaag caagactgga agcgaagctc tccagctttg gctaacccaa 4200

tccgagcaca tcaagagctt cagtcttgtg acagtaagaa atttaggaac atagttgacc 4260

tatattttgt attctttctt gttgaatgca gtccaaatac aaaa 4304

<210> 73
<211> 4996
<212> DNA
<213> human

<400> 73
gcgccggggt gtgcgcccgg ccgggtgtgc ggagagcgag ggagcgcgct ccctcccgac 60

gcgcgggccg cagcggccaa gcccgagggt gcgtggcgcc cccgcccgcc cggcccggcc 120

cggccatggc ccccgcccgg ggccgcctgc cccctgcgct ctgggtcgtc acggccgcgg 180

cggcggcggc cacctgcgtg tccgcggcgc gcggcgaagt gaatttgctg gacacgtcga 240

ccatccacgg ggactggggc tggctcacgt atccggctca tgggtgggac tccatcaacg 300
aggtggacga gtccttccag cccatccaca cgtaccaggt ttgcaacgtc atgagcccca 360
accagaacaa ctggctgcgc acgagctggg tcccccgaga cggcgcccgg cgcgtctatg 420
ctgagatcaa gtttaccctg cgcgactgca acagcatgcc tggtgtgctg ggcacctgca 480
aggagacctt caacctctac tacctggagt cggaccgcga cctgggggcc agcacacaag 540
aaagccagtt cctcaaaatc gacaccattg cggccgacga gagcttcaca ggtgccgacc 600
ttggtgtgcg gcgtctcaag ctcaacacgg aggtgcgcag tgtgggtccc ctcagcaagc 660
gcggcttcta cctggccttc caggacatag gtgcctgcct ggccatcctc tctctccgca 720
tctactataa gaagtgccct gccatggtgc gcaatctggc tgccttctcg gaggcagtga 780
cgggggccga ctcgtcctca ctggtggagg tgaggggcca gtgcgtgcgg cactcagagg 840
agcgggacac acccaagatg tactgcagcg cggagggcga gtggctcgtg cccatcggca 900
aatgcgtgtg cagtgccggc tacgaggagc ggcgggatgc ctgtgtggcc tgtgagctgg 960
gcttctacaa gtcagcccct ggggaccagc tgtgtgcccg ctgccctccc cacagccact 1020
ccgcagctcc agccgcccaa gcctgccact gtgacctcag ctactaccgt gcagccctgg 1080
acccgccgtc ctcagcctgc acccggccac cctcggcacc agtgaacctg atctccagtg 1140
tgaatgggac atcagtgact ctggagtggg cccctcccct ggacccaggt ggccgcagtg 1200
acatcaccta caatgccgtg tgccgccgct gcccctgggc actgagccgc tgcgaggcat 1260
gtgggagcgg cacccgcttt gtgccccagc agacaagcct ggtgcaggcc agcctgctgg 1320
tggccaacct gctggcccac atgaactact ccttctggat cgaggccgtc aatggcgtgt 1380
ccgacctgag ccccgagccc cgccgggccg ctgtggtcaa catcaccacg aaccaggcag 1440
ccccgtccca ggtggtggtg atccgtcaag agcgggcggg gcagaccagc gtctcgctgc 1500
tgtggcagga gcccgagcag ccgaacggca tcatcctgga gtatgagatc aagtactacg 1560
agaaggacaa ggagatgcag agctactcca ccctcaaggc cgtcaccacc agagccaccg 1620
tctccggcct caagccgggc acccgctacg tgttccaggt ccgagcccgc acctcagcag 1680
gctgtggccg cttcagccag gccatggagg tggagaccgg gaaaccccgg ccccgctatg 1740
acaccaggac cattgtctgg atctgcctga cgctcatcac gggcctggtg gtgcttctgc 1800
tcctgctcat ctgcaagaag aggcactgtg gctacagcaa ggccttccag gactcggacg 1860
aggagaagat gcactatcag aatggacagg cacccccacc tgtcttcctg cctctgcatc 1920
accccccggg aaagctccca gagccccagt tctatgcgga accccacacc tacgaggagc 1980
caggccgggc gggccgcagt ttcactcggg agatcgaggc ctctaggatc cacatcgaga 2040
aaatcatcgg ctctggagac tccggggaag tctgctacgg gaggctgcgg gtgccagggc 2100
agcgggatgt gcccgtggcc atcaaggccc tcaaagccgg ctacacggag agacagaggc 2160
gggacttcct gagcgaggcg tccatcatgg ggcaattcga ccatcccaac atcatccgcc 2220
tcgagggtgt cgtcacccgt ggccgcctgg caatgattgt gactgagtac atggagaacg 2280

gctctctgga caccttcctg aggacccacg acgggcagtt caccatcatg cagctggtgg 2340
gcatgctgag aggagtgggt gccggcatgc gctacctctc agacctgggc tatgtccacc 2400
gagacctggc cgcccgcaac gtcctggttg acagcaacct ggtctgcaag gtgtctgact 2460
tcgggctctc acgggtgctg gaggacgacc cggatgctgc ctacaccacc acgggcggga 2520
agatccccat ccgctggacg gccccagagg ccatcgcctt ccgcaccttc tcctcggcca 2580
gcgacgtgtg gagcttcggc gtggtcatgt gggaggtgct ggcctatggg gagcggccct 2640
actggaacat gaccaaccgg gatgtcatca gctctgtgga ggaggggtac cgcctgcccg 2700
cacccatggg ctgcccccac gccctgcacc agctcatgct cgactgttgg cacaaggacc 2760
gggcgcagcg gcctcgcttc tcccagattg tcagtgtcct cgatgcgctc atccgcagcc 2820
ctgagagtct cagggccacc gccacagtca gcaggtgccc accccctgcc ttcgtccgga 2880
gctgctttga cctccgaggg ggcagcggtg gcggtggggg cctcaccgtg ggggactggc 2940
tggactccat ccgcatgggc cggtaccgag accacttcgc tgcgggcgga tactcctctc 3000
tgggcatggt gctacgcatg aacgcccagg acgtgcgcgc cctgggcatc accctcatgg 3060
gccaccagaa gaagatcctg ggcagcattc agaccatgcg ggcccagctg accagcaccc 3120
aggggccccg ccggcacctc tgatgtacag ccagcagggc ccaggcagcc accaagccca 3180
ccccaggtca tgccagcggc agaggacgtg aggggctggc agcaggcagg gcggccccag 3240
gcctctgccc tcctctcagg tgctggagga gctgaaggct tcgccacagg acctggagtt 3300
atcaggggtc aggcgcctgg gaaggggcct ttggtggcca ccctggtgag gacacctgtc 3360
ccccagggca ggcaccttct cttttccaga gcctggggcc tccacgtcac agagtccaac 3420
agggacatca ctcgcctgcc tctgtgtgcg tgcatgtgtg tgtgtggtgg ggggtgttct 3480
cacaaggtca tgggatctca tgtgaacagt gtgtgatcaa gtgtgtccac cccttcgggt 3540
ctcagcatgg acgtgtgcat gttatgagcg tgtgcttatc cgttaaggct ggaggcacat 3600
gtgggtgatg gtggatgatg tgtcatgaat gaggaggtgt gtgagcaggg aactcagtgt 3660
gacaccgcca ggtccagcac ccatgggggc gggggagagg ctcaccccta cgtcccccca 3720
cacctggagg ctggagccag gggccacttc tgaactgcac cagcaccagg cccaccctcg 3780
tctctgcctg ggtgagccca ccccggctgt atctcaggtc tgggtcctcc ctctagccga 3840
ggaggccacc tgcagcctcc acccggctct caccgctgct tcaacaggaa aacagggttc 3900
ccggtcagtc cggctggccg ccttcatgga ggcatcatgg cagagcacat gagatgtcct 3960
cagctgggct tggctgcctg gccagggccg ggggctcagc agcctcctct agcctctgat 4020
gccttccctc cacggcccag gtctcctcac tcaaagtccc ttcgccaacc tttcaatgcc 4080
cagccctgac acctgccctt tgtcccccag gcctaggatc agggaccaga ggatcctatc 4140
ttctcagcac ccagcccacc ccttcctgta gcatatgggg agactaaggc ctggagagag 4200
gggtgatgcc ccgtcccagg ttgcactgca accaagtgtc agagtcgggg ctccggcctc 4260
ctgccaaggc tcttgtcccc atacaccatc ccacaagggc gctgggggtg gaagtgccct 4320

ggaagcccct cccctctcac actgacctcc ccccttacgg cccaccaggg tatgtaaata 4380

tctcttttct accatgtcag aatatttttt cctcactcct gacaatgcaa aaatggtctt 4440

caaagcacat aaaaagcacc cagggtgaga aagccccatc ccgggggccg ttggcaggca 4500

gggaagcagg aaccccaccg tgtgccccct gccagcccca gagggagtgg cgagcccagc 4560

tgcccagccc tgccccccct ccccatagcc agcacagcta tcccgcgggg acaccagcac 4620

tgagccccct ctccctcctg caataattcg gggagtctca gccccatcca ggtgccgcgg 4680

ccagctctct acacctctat atattatatt actatatagc cgagctgttc ttccttccta 4740

tggaagtcgg aaacatggtc agaacacgat ctgggggggg gatcctgtct tcctccccac 4800

cccaccccac tcttacccaa tttctgggct ctggatcctc acagtcatgg aggcaccgtg 4860

ggcctggcac ttgcaaaagt gtggcccctc actctagtgt gtggtccctc tcagggtcct 4920

ggggatctgc ctctctgtgg tctccatcct gactcttgaa cttacccaca ataagaataa 4980

attctgcctc atcttt 4996

<210> 74
<211> 4737
<212> DNA
<213> mouse

<400> 74
gaattccggg cgggagaggc aggagcgcgc cacgcgcggc cgccgggccg agcccgagga 60

gcgcggcgcc gcccggccat ggcccccgcc cgggcccgcc tgtcccccgc tctctgggtc 120

gtcacggccg cggcggcggc cacctgcgtg tccgcggggc gcggcgaagt gaacttgttg 180

gatacatcaa ccatccatgg agactggggc tggctcacgt atcccgctca tgggtgggac 240

tccatcaacg aggtagacga gtccttccgg cccatccaca cgtaccaagt atgcaacgtc 300

atgagcccca accagaacaa ctggctgcgc acgaactggg tgccccgcga cggcgccagg 360

cgtgtctacg ccgagatcaa attcaccctg cgtgactgca atagcatccc aggggtgtta 420

ggtacctgca aggagacctt caaccttcac tacctggagt ctgaccgcga cctgggggcc 480

agcacacaag agagccagtt cctcaagatt gacaccatcg cggcggatga gagcttcacg 540

ggcgcagacc tgggtgtgcg gaggctcaag ctgaacacgg aggtgcgtgg cgtgggtccg 600

ctcagcaagc gcggcttcta cctggccttc caggacatag gtgcctgcct cgccatcctc 660

tcactccgaa tctactacaa gaagtgcccc gccatggtac gcaacctggc ggccttctcg 720

gaggcggtta ctggggccga ctcgtcctcg ctggttgagg ttcgaggcca gtgtgtgcgg 780

cactcggaag agcgcgacac acccaagatg tactgcagtg ctgagggcga atggctggtg 840

cccatcggca agtgcgtgtg cagtgctggc tatgaggagc ggcgggatgc ctgcatggcc 900

tgtgagctgg gcttctacaa gtcagccccc ggggaccagc tgtgcgcccg atgcccaccc 960

cacagccact ctgccacccc tgcggcccag acctgccgtt gtgacctcag ctactaccgc 1020

gcggccttgg acccaccatc ggcagcctgc acccggccac cctcagcacc agtgaacctg 1080

atctccagcg taaatggaac gtctgtgacc ctcgagtggg cccctcctct ggacccaggt 1140
ggtcgcagcg acatcaccta caacgcagtg tgccgccgct gcccctgggc cctgagccac 1200
tgtgaggctt gtgggagtgg aactcgcttc gtcccgcagc agacaagcct ggcacaggcc 1260
agcctgctgg tggccaacct gctagcccac atgaactact ccttctggat cgaggcagtc 1320
aacggtgtct ccaacctgag ccctgagccc cgcagcgctg ctgtggtcaa catcaccacg 1380
aaccaggcag ccccgtccca ggtggtggtg atccggcagg agcgggctgg gcagaccagc 1440
gtgtcactcc tgtggcagga accagagcag cccaacggca tcattctgga gtacgagatc 1500
aagtactacg agaaggataa ggagatgcaa agctactcta ccctcaaggc tgttaccacc 1560
cgcgccaccg tgtcaggcct caaaccaggc actcgctacg tgttccaggt ccgagcccgc 1620
acctcagcag gctgtggccg cttcagccag gccatggagg tggagaccgg gaaacccagg 1680
ccccgctacg acactcggac catcgtctgg atctgcctga cactcatcac gggcctggtg 1740
gtgctcctct tgcttctcat ctgcaagaag aggcactgtg gctacagcaa ggcgttccag 1800
gactcggatg aagagaagat gcattaccag aacggacagg cacccccacc tgtcttcttg 1860
cccttgaacc acccccgggg gaagttccca gaaacccagt tctctgcaga gccccacacc 1920
tatgaggaac caggtcgggc aggccggagc tttacccgag agatcgaggc atccaggatc 1980
cacattgaga agatcatagg ctcgggggag tcaggagaag tgtgctatgg gaggctgcag 2040
gtgccaggac agcgggatgt gcccgtggcc atcaaggctc tcaaagctgg ctacacagag 2100
agacagcgac aagacttctt gagcgaggca gccatcatgg gccaatttga tcaccctaac 2160
atcatccgcc ttgagggtgt ggtcacccgt ggtcgcctgg caatgattgt gactgagtac 2220
atggaaaatg gctctctaga tgccttcctc aggacccacg atgggcagtt caccatcgtg 2280
cagctagtgg gcatgctcag aggggtgggc gctggcatgc gctacctctc agacctgggc 2340
tacattcacc gtgacctggc tgcccgcaat gtcctggtgg atggccgtct ggtgtgcaaa 2400
gtgtcagact ttgggctctc aagggctctg gaggatgacc ccgaagctgc ctacaccact 2460
gcgggaggga agatccctat ccgatggaca gcgccagagg ccattgcttt ccgcacgttc 2520
tcctcagcca gtgatgtgtg gagctttggt gtggtcatgt gggaggttct agcctatggt 2580
gaacgaccct actggaacat gaccaaccag gatgtcatca gctctgtgga ggagggttat 2640
aggctgccag cacccatggg ttgcccacgt gccctgcacc agctcatgct ggactgctgg 2700
cacaaggacc gtgcccagcg gcctcgcttc gctcatgtcg tgagcgttct ggatgccctg 2760
gtccacagtc ccgagagcct cagggccaca gccacagtca gcaggtgccc accccctgcc 2820
ttcgccagga gctgctttga cctccgagca ggtggaagtg ggaacgggga cctcacagtg 2880
ggggactggc tggactccat ccgaatgggg cgctaccggg accacttcgc ggctggcggg 2940
tactcctccc ttggcatggt gcttcggatg aacgcccagg atgtgcgcgc cctcggcatc 3000
actctcatgg gtcaccagaa gaagatactt ggaagcatcc agaccatgag ggcccagctg 3060
agcagcaccc agggacgccg ccggcacctc tgaccgatgg ccacttggca ctggttatac 3120

cagctgtcag gggctccacc cccccccccc catcagtcag ggtagccccc agagcaatac 3180
cacaggacct agagtgacag aggtcaggca cctgggacaa ggaccttggt gctccaggta 3240
gagaggacac ctgccctctg gaagcaggca caggaccttg tgtcttctag acctggggcc 3300
tccaacacca gaagccgatg gtgacatcac tcacctagct gtgggtgcat gcgtgcatgc 3360
gtagggttga tggtaggtat gggggtgctc cacaaggtcc tggcagctga tggaaacaat 3420
gtgtactcat gtctctgttc cccgccaggc cccagcacag acgtgtgcag gtcaccatga 3480
gtgtgcttat ctgctaaggg caggagcaca cgtgtgtgac cgcagataac acgtcataaa 3540
tgaggaggcg tgacagagtg tgagcagggg atctgagctg acactgccca gttcccagga 3600
cctgtgggga tcagacagtg cctgcagctg gagtttaggc tgcttccgaa tcacaccagc 3660
accaggccca gtgtccagcc tgggtgaatc caccatggca atacctcagg tccgggactc 3720
cttcctactt tggagggcgg ggcacctgca gcctctgcct ggctcctcct gctattcaac 3780
aggaaaacag ggttcctggc cagtccctct ggccaccttc cgtagaggat cacagcagag 3840
cgtgagatgc cctcggtggg gcgtgactgc ctggccatct gctctcatgg ctgtgatgcc 3900
tctgcccaac gtcccactac ccatccctgc cacccctgcc catgctggga acaggagcat 3960
ggggcagact ctcaagcccc aaacccaccc cttcctatac cagataggta atgcccagag 4020
acgaggatgc cttgcctgag gtcacatgac agccaaattt cagcgttggg gccctcatac 4080
ccccagccat ggtctgttcc ttcccaacct cccctcccca gactgctagg atgggagggg 4140
cctgggaagc tcactcgcac aggctgataa ccccttacgg cccaccaggg tatgtaaata 4200
tctttttttc taccatgtca gaatattttt ttctcctcac ttctgacaat gcaaaaatgg 4260
tcttcaaagc acatgcaaag ctccccgggt gagagagcca tcctggggga ggcagggccc 4320
aaggagcccc ggcccagagg acagggcggc ccccttccct gcccatccct gcccacagcc 4380
agcacagcac cccgtgagac accagcactg agccccctcc cttctgcaat aattcagggg 4440
cttcagcccc acccaggtgc cgtgcgctgc cagctcctgc gcttctatat attatagtac 4500
tatgtagccg agctgccctt ccttcctatg gaagtgggaa acatggtcag gacacgaggg 4560
gtggggacac cttgtcttcc accccacccc actcacccag tgtctgggct taggatcctc 4620
aggcaaggcc ctggggtggg gctcctgggg gcctctctgt ggttcccatc ctgagtctgg 4680
aatcacttgt gatgagaata aatactgtct catcttcaaa aaaaaaaaaa aaaaaaa 4737

<210> 75
<211> 4528
<212> DNA
<213> rat

<400> 75
atggctgggg acttgaacct gttggacaca tcgaccatcc acggagactg gggctggctc 60
acatatcctg ctcatgggtg ggactccatc aacgaggtgg acgagtcctt tcggcccatc 120
cacacgtatc aggtgtgcaa cgtcatgagt cccaaccaga acaactggct gcgtacaagc 180
tgggtgcccc gtgacggcgc caggcgtgtc tacgccgaga tcaaattcac cctgcgtgac 240

tgcaatagca ttcctggggt gttgggcacc tgcaaggaga ccttcaacct tcactaccta 300
gagtctgacc gggatctggg ggccagcaca caagagagcc agttcctcaa gattgacacc 360
atcgcagcgg atgagagctt caccggcgca gatctgggcg tgcggaggct caagctgaac 420
acggaggtgc gtggcgtggg cccgctcagc aagcgcggct tctacctggc cttccaggac 480
ataggtgcct gcctcgccat cctctcactc agaatctact acaagaagtg tcccgccatg 540
gtacgcaacc tggcggcctt ctcggaggcg gtaaccgggg ctgactcgtc ctcgctggta 600
gaggttcgag gccagtgtgt gcgacactca gaggagcgcg acacacccaa gatgtactgc 660
agtgccgagg gcgaatggct ggtgcccatc ggcaagtgcg tgtgcagtgc cggctatgag 720
gagcggcgcg atgcctgtat ggcctgtgag ctgggcttct acaagtcagc ccccggggac 780
cagctgtgtg cccgatgccc accccacagc cattctgcca cccctgcggc ccagacctgc 840
cgttgtgacc tcagctacta ccgtgcggcc ttggacccac catcggcagc ctgcacccgg 900
ccaccctcag caccggtgaa cctgatctcc agcgtgaacg gaacttctgt gaccctggag 960
tgggcccctc ctctggaccc aggcggccgc agtgacatca cctacaacgc ggtgtgccgc 1020
cgctgcccct gggccctgag ccactgtgag gcttgtggga gtggaactcg ctttgtcccg 1080
caacagacaa gcctggcgca ggccagcctg ctggtggcca acctcctggc ccacatgaac 1140
tactccttct ggatcgaggc agtcaacggt gtctccaacc tgagccccga gccccgcaac 1200
gctgctgtgg tcaacatcac cacaaaccag gcagccccgt cccaggtggt ggtgatccgg 1260
caggagcggg ctgggcagac cagcgtgtca ctcctgtggc aggagccaga gcaacccaac 1320
ggcatcatac tggagtacga gatcaagtac tacgagaagg ataaggagat gcaaagctac 1380
tctaccctca aggctgtcac cacccgcgcc accgtgtcgg gcctcaaacc aggcactcgc 1440
tacatgttcc aggtccgggc ccgcacctca gcaggctgtg gacgcttcag ccaggccatg 1500
gaggtggaga ccgggaaacc caggccccgc tatgacactc ggaccatcgt ctggatctgc 1560
ctgacactca tcacaggcct ggtggtactc ctcctgcttc tcatctgcaa gaagaggcac 1620
tgcggctata gcaaggcatt ccaggactct gatgaagaga aaatgcatta ccagaatggc 1680
caagcacccc cgcctgtctt cttgcccttg aaccaccccc cggggaagct cccagaaacc 1740
cagttctcgg cagagcccca caactatgag gaaccgggcc gtgcaggccg aagctttacc 1800
agagagatcg aggcctccag gatccacatt gagaagatca taggctcggg ggagtcagga 1860
gaagtatgct atgggaggct gcaggtgcca ggacagcggg atgtgcccgt ggccatcaag 1920
gctctcaaag ctggctacac ggagaggcag cgccaagact tcttgagaga ggcagccatc 1980
atgggacaat tcgatcatcc taacatcatc cgcctggagg gtgtggtcac ccgtggccgc 2040
ctggcaatga ttgtgactga gtacatggaa aatggctctc tggatgcctt cctcaggacc 2100
cacgatggtc agttcaccat cctgcagcta gtgggcatgc tcaaaggggt gggcgctggc 2160
atgcgctacc tctcggacct gggctacatt caccgtgacc tggccgcccg caacatcctg 2220
gtagatggcc gtcttgtgtg caaggtgtca gactttgggc tctcaagggc tctggaggac 2280

gaccctgagg ccgcctacac cactgcggga gggaagatcc ctatccgatg gacagcacca 2340
gaggccattg ccttccgcac tttctcctca gccagtgatg tgtggagctt tggtgtggtc 2400
atgtgggagg tgctggccta tggtgaacgg ccctactgga acatgaccaa tcaggatgtc 2460
atcagctctg tggaagaggg ttataggctt ccagcgccca tgggctgccc acgtgccctg 2520
caccaactca tgctagactg ctggcacaag gaccgtgccc agcggcctcg cttctctcac 2580
gtcgtgagcg ttctggaggc cctggtccac agccccgaga gccttagggc cacagctaca 2640
gtcagcaggt gcccagcccc tgcctttgcc aggagctgct ttgacctccg agcaggcgga 2700
aatgggaacg gggacctcac tgtgggagat tggctggatt ccatccgaat gggccggtac 2760
cgggaccact ttgcagcggg cgggtactcc tcccttggca tggtgcttca catgaacgcc 2820
caggatgttc gtgccctcgg catcaccctc atgggccacc agaagaaaat ccttggaagc 2880
atccagacaa tgaggtccca gctgagctgc actcaggggc cacgccggca cctctgaccc 2940
atggccactt ggcactggtc ataccaactg tcaggggctc tgcggggggg ggcccccagc 3000
agtcagggta gcccctgagc tctgccacag gagagtgaca gaggtcaggc acctgggaca 3060
aggaccttgg tgctttaagt agagaggaca cctgccctct ggaggaaggc acaggacctt 3120
gtgtcttcta gagcctgggg ctccaacacc agacgccaat ggtgacatca ctcgcctagc 3180
tgtgggtgca tgcgtgcatg catacgggtg acggtgtggg cgtgcccctc gaggccctgg 3240
cggttgatga aaacaatgcg taaccatgtc tctgttcccc cattaggccc cagcacagat 3300
gtgtgcaggt taccacgcgt gtgcttgtct actagggcag gagccacgtg tgtatggcca 3360
cagataacac gtcatgaatg aggaggcgtg acagagtgtg agcgacactg cccagttccc 3420
aggacctgtg ggggaacgca cagtgcctgg cggctggagc ccaggctgct tccgaatcat 3480
accagcacca ggcccaccgt ccagcctggg tgaacccacc atggctgtat ctcaggtccg 3540
ggacgccttc ctactttggg ggctctgcct ggctcctcct gctactcaat aggaaaacag 3600
ggttcctggc cagtccctct ggccgccttc cgtagaggat cacagcagag tgtgagatgc 3660
cctcggtggg ccgtgactgc ctgaccgtcg ctctcatggc tgtgatgcct ctttccaatg 3720
tctcactgcc cattcctgct gcccccctgc ccatgctggg aacaggagcc ccaaacccac 3780
cccttcctgt accagatagg tatgcccaga gaaaaggatg ccttgtctga tgtcacatga 3840
caaccaaatt tcagagttgg gaccctcttc ccccccccca accatggtct attctcccca 3900
accccaccca agacagctag gacgggaggg gcctgggaga ctcatcacca caggctgata 3960
cccctccccc ccttacggcc caccagggta tgtaaatgtc tttttttcta ccatgtcaga 4020
atattttttt ccctcactcc tgacaatgca aaaatggtct tcaaagcaca tacaaagcac 4080
cccgggtgag aacgccccat cctaggggag gcagggccca aagagccccg gcccagagga 4140
cagggcggcc ccctcccctg cccatccctg cccacagcca gcacagcacc ccgcgtgaca 4200
ccagcactga gccccctccc ttctgcaata atcgggggct tcagccccac ccaggtgccg 4260
cggccagctc ctgcgcttct atatattata gtactatgta gccgagctgc ccttccttcc 4320

tatggaagtg ggaaacatgg tcaggacacg aggggtgggg acaccttgtc ttccaccacc 4380

cccactcacc cagtgtctgg gcttaggatc ctcaggcgag gcccctgggg tgggtccccc 4440

ttggggctcc tggggcctct ctgtggttcc catcctgagt ctggaattac ttgcgatgag 4500

aataaattgt ctcatctttg cctgcgca 4528

<210> 76
<211> 4869
<212> DNA
<213> human

<400> 76
cattctgctg gctgcgcggt ggcggcggct gtgtgtgcgc cgcgccttgc cgccccccct 60

ggccccccga gcccggggcg cgcgctcccg cccgggccgt ccgggccccg cggcgccgcg 120

gcccgaggcc ccgggaagcg cagccatggc tctgcggagg ctgggggccg cgctgctgct 180

gctgccgctg ctcgccgccg tggaagaaac gctaatggac tccactacag cgactgctga 240

gctgggctgg atggtgcatc ctccatcagg gtgggaagag gtgagtggct acgatgagaa 300

catgaacacg atccgcacgt accaggtgtg caacgtgttt gagtcaagcc agaacaactg 360

gctacggacc aagtttatcc ggcgccgtgg cgcccaccgc atccacgtgg agatgaagtt 420

ttcggtgcgt gactgcagca gcatccccag cgtgcctggc tcctgcaagg agaccttcaa 480

cctctattac tatgaggctg actttgactc ggccaccaag accttcccca actggatgga 540

gaatccatgg gtgaaggtgg ataccattgc agccgacgag agcttctccc aggtggacct 600

gggtggccgc gtcatgaaaa tcaacaccga ggtgcggagc ttcggacctg tgtcccgcag 660

cggcttctac ctggccttcc aggactatgg cggctgcatg tccctcatcg ccgtgcgtgt 720

cttctaccgc aagtgccccc gcatcatcca gaatggcgcc atcttccagg aaaccctgtc 780

gggggctgag agcacatcgc tggtggctgc ccggggcagc tgcatcgcca atgcggaaga 840

ggtggatgta cccatcaagc tctactgtaa cggggacggc gagtggctgg tgcccatcgg 900

gcgctgcatg tgcaaagcag gcttcgaggc cgttgagaat ggcaccgtct gccgaggttg 960

tccatctggg actttcaagg ccaaccaagg ggatgaggcc tgtacccact gtcccatcaa 1020

cagccggacc acttctgaag gggccaccaa ctgtgtctgc cgcaatggct actacagagc 1080

agacctggac cccctggaca tgccctgcac aaccatcccc tccgcgcccc aggctgtgat 1140

ttccagtgtc aatgagacct ccctcatgct ggagtggacc cctccccgcg actccggagg 1200

ccgagaggac ctcgtctaca acatcatctg caagagctgt ggctcgggcc ggggtgcctg 1260

cacccgctgc ggggacaatg tacagtacgc accacgccag ctaggcctga ccgagccacg 1320

catttacatc agtgacctgc tggcccacac ccagtacacc ttcgagatcc aggctgtgaa 1380

cggcgttact gaccagagcc ccttctcgcc tcagttcgcc tctgtgaaca tcaccaccaa 1440

ccaggcagct ccatcggcag tgtccatcat gcatcaggtg agccgcaccg tggacagcat 1500

taccctgtcg tggtcccagc cggaccagcc caatggcgtg atcctggact atgagctgca 1560

gtactatgag aaggagctca gtgagtacaa cgccacagcc ataaaaagcc ccaccaacac 1620
ggtcaccgtg cagggcctca aagccggcgc catctatgtc ttccaggtgc gggcacgcac 1680
cgtggcaggc tacgggcgct acagcggcaa gatgtacttc cagaccatga cagaagccga 1740
gtaccagaca agcatccagg agaagttgcc actcatcatc ggctcctcgg ccgctggcct 1800
ggtcttcctc attgctgtgg ttgtcatcgc catcgtgtgt aacagaagac gggggtttga 1860
gcgtgctgac tcggagtaca cggacaagct gcaacactac accagtggcc acatgacccc 1920
aggcatgaag atctacatcg atcctttcac ctacgaggac cccaacgagg cagtgcggga 1980
gtttgccaag gaaattgaca tctcctgtgt caaaattgag caggtgatcg gagcagggga 2040
gtttggcgag gtctgcagtg gccacctgaa gctgccaggc aagagagaga tctttgtggc 2100
catcaagacg ctcaagtcgg gctacacgga gaagcagcgc cgggacttcc tgagcgaagc 2160
ctccatcatg ggccagttcg accatcccaa cgtcatccac ctggagggtg tcgtgaccaa 2220
gagcacacct gtgatgatca tcaccgagtt catggagaat ggctccctgg actcctttct 2280
ccggcaaaac gatgggcagt tcacagtcat ccagctggtg ggcatgcttc ggggcatcgc 2340
agctggcatg aagtacctgg cagacatgaa ctatgttcac cgtgacctgg ctgcccgcaa 2400
catcctcgtc aacagcaacc tggtctgcaa ggtgtcggac tttgggctct cacgctttct 2460
agaggacgat acctcagacc ccacctacac cagtgccctg ggcggaaaga tccccatccg 2520
ctggacagcc ccggaagcca tccagtaccg gaagttcacc tcggccagtg atgtgtggag 2580
ctacggcatt gtcatgtggg aggtgatgtc ctatggggag cggccctact gggacatgac 2640
caaccaggat gtaatcaatg ccattgagca ggactatcgg ctgccaccgc ccatggactg 2700
cccgagcgcc ctgcaccaac tcatgctgga ctgttggcag aaggaccgca accaccggcc 2760
caagttcggc caaattgtca acacgctaga caagatgatc cgcaatccca acagcctcaa 2820
agccatggcg cccctctcct ctggcatcaa cctgccgctg ctggaccgca cgatccccga 2880
ctacaccagc tttaacacgg tggacgagtg gctggaggcc atcaagatgg ggcagtacaa 2940
ggagagcttc gccaatgccg gcttcacctc ctttgacgtc gtgtctcaga tgatgatgga 3000
ggacattctc cgggttgggg tcactttggc tggccaccag aaaaaaatcc tgaacagtat 3060
ccaggtgatg cgggcgcaga tgaaccagat tcagtctgtg gaggtttgac attcacctgc 3120
ctcggctcac ctcttcctcc aagccccgcc ccctctgccc cacgtgccgg ccctcctggt 3180
gctctatcca ctgcagggcc agccactcgc caggaggcca cgggccacgg gaagaaccaa 3240
gcggtgccag ccacgagacg tcaccaagaa aacatgcaac tcaaacgacg gaaaaaaaaa 3300
gggaatggga aaaaagaaaa cagatcctgg gagggggcgg gaaatacaag gaatattttt 3360
taaagaggat tctcataagg aaagcaatga ctgttcttgc gggggataaa aaagggcttg 3420
ggagattcat gcgatgtgtc caatcggaga caaaagcagt ttctctccaa ctccctctgg 3480
gaaggtgacc tggccagagc caagaaacac tttcagaaaa acaaatgtga aggggagaga 3540
caggggccgc ccttggctcc tgtccctgct gctcctctag gcctcactca acaaccaagc 3600

gcctggagga cgggacagat ggacagacag ccaccctgag aacccctctg ggaaaatcta 3660

ttcctgccac cactgggcaa acagaagaat tttctgtctt ttggagagta ttttagaaac 3720

tccaatgaaa gacactgttt ctcctgttgg ctcacagggc tgaaaggggc ttttgtcctc 3780

ctgggtcagg gagaacgcgg ggaccccaga aaggtcagcc ttcctgagga tgggcaaccc 3840

ccaggtctgc agctccaggt acatatcacg cgcacagcct ggcagcctgg ccctcctggt 3900

gcccactccc gccagcccct gcctcgagga ctgatactgc agtgactgcc gtcagctccg 3960

actgccgctg agaagggttg atcctgcatc tgggtttgtt tacagcaatt cctggactcg 4020

ggggtatttt ggtcacaggg tggttttggt ttagggggtt tgtttgttgg gttgtttttt 4080

gttttttggt tttttttaat gacaatgaag tgacactttg acatttccta ccttttgagg 4140

acttgatcct tctccaggaa gaaggtgctt tctgcttact gacttaggca atacaccaag 4200

ggcgagattt tatatgcaca tttctggatt tttttatacg gttttcattg acactcttcc 4260

ctcctcccac ctgccaccag gcctcaccaa agcccactgc catggggcca tctgggccat 4320

tcagagactg gagtgagatt tgggtgtgga gggggaggcg ccaaggtgga ggagcttccc 4380

actccaggac tgttgatgaa agggacagat tgaggaggaa gtgggctctg aggctgcagg 4440

gctggaagtc cttgcccact tcccactctc ctgccccaat ctatctagta cttcccaggc 4500

aaataggccc ctttgaggct cctgagtgcc ctcagatggt caaaacccag ttttccctct 4560

gggagcctaa accaggctgc atcggaggcc aggacccgga tcattcactg tgataccctg 4620

ccctccagag ggtgcgctca gagacacggg caagcatgcc tcttcccttc cctggagaga 4680

aagtgtgtga tttctctccc acctccttcc ccccaccaga cctttgctgg gcctaaaggt 4740

cttggccatg gggacgccct cagtctaggg atctggccac agactccctc ctgtgaacca 4800

acacagacac ccaagcagag caatcagtta gtgaattgaa tggaaataaa cgctttagtt 4860

ataatatga 4869

<210> 77
<211> 4780
<212> DNA
<213> mouse

<400> 77
tgcgccgcgc cccgccgctc cccgccggcc ccccgagccc ggggcgcgcg ctccagcccg 60

ggggtccccg cggcgccgcg gccggaggct ccgggcagcg cagcaatggc cgtgcgcagg 120

ctgggggccg cgctgctgct gctgccgctg ctagccgccg tggaagaaac cctgatggac 180

tctacgacag caacggctga gctgggctgg atggtacatc ccccatcagg gtgggaagag 240

gtgagcggct acgacgagaa catgaacact atccgtacct accaggtgtg caatgtcttt 300

gagtcaagcc agaacaactg gctgcggacc aaattcatcc ggcgccgcgg cgcccaccgc 360

atccacgtgg agatgaagtt ctcggtgcgt gactgcagca gcattcccag cgtgccgggc 420

tcctgcaagg agaccttcaa cctctactac tatgaggctg attttgactt agccaccaaa 480

acctttccca actggatgga gaatccgtgg gtgaaggtgg acaccatcgc ggccgatgag 540

agcttctctc aggtggacct gggtggccgc gtcatgaaaa tcaacactga ggtgcgaagc 600
ttcggtcccg tgtcccgcaa tggtttctac ctggccttcc aggactatgg cggctgtatg 660
tccctcattg ctgtgcgcgt cttctaccgg aagtgccccc gaatcatcca gaatggtgcc 720
atcttccagg agacactgtc gggggctgag agcacttcgc tggtggcagc tcggggcagc 780
tgcatcgcca atgctgaaga agtggatgtg cccatcaaac tctactgtaa cggggacggc 840
gaatggctgg tgcccatagg tcgctgcatg tgcaaggcgg gcttcgaggc tgtggagaac 900
ggcaccgtct gccgaggttg tccatcagga accttcaagg ccaaccaagg ggacgaagcc 960
tgcacccact gtcccatcaa cagccgcacc acctccgagg gtgccaccaa ctgtgtatgc 1020
cgcaacggct actacagggc cgacctggac cccttagaca tgccttgcac aaccatcccc 1080
tctgcgcccc aggctgtgat ctccagcgtc aacgagacgt ccctcatgct agagtggacc 1140
ccaccccgag actcgggggg tcgcgaggat cttgtttaca acatcatctg caagagctgt 1200
ggctccggcc ggggcgcatg cacgcgctgc ggggacaacg tgcagtacgc gccccgccag 1260
ctgggcctga ctgagccgcg catctacatc agtgacctgc tggcacacac gcagtacacc 1320
ttcgagatcc aggccgtgaa cggtgtgact gaccagagtc ccttctcacc tcagttcgcc 1380
tctgtgaaca tcaccaccaa ccaagcagca ccatcggccg tgtccatcat gcaccaggtg 1440
agccgcactg tggacagcat caccctgtcg tggtcccagc cagaccagcc caacggtgtg 1500
atcctggact acgagctgca gtactatgag aaggagctca gtgagtacaa cgccacggcc 1560
ataaaaagcc ccaccaacac agtcactgtg cagggcctca aagccggcgc catctatgtc 1620
ttccaggtgc gggcacgcac cgttgcaggc tatgggcgct acagtggcaa gatgtacttc 1680
caaaccatga cagaagccga gtaccagacc agcatcaagg aaaagctacc cctcatcgtt 1740
ggctcctccg ccgccggctt agtcttcctc atcgctgtgg tcgtcattgc catcgtatgt 1800
aacagacggg ggtttgagcg tgccgactca gagtacacgg acaagctaca acactacacc 1860
agcggacaca tgaccccagg catgaagatc tatatagacc ctttcaccta tgaagatcct 1920
aatgaggcag tgcgggagtt tgccaaggaa attgacatct cctgtgtcaa gattgagcag 1980
gtgatcggag caggggaatt tggtgaggtc tgcagtggcc atttgaagct gccaggcaag 2040
agagagatct ttgtagccat caagaccctc aagtcaggat acacggagaa acagcgccgg 2100
gacttcctga gtgaggcatc catcatgggc cagttcgacc accccaatgt catccatctg 2160
gaaggggttg tcaccaagag cacacctgtc atgatcatca ctgaattcat ggagaacgga 2220
tctctggact ccttcctccg gcaaaacgat gggcagttca cagtcatcca actggtgggc 2280
atgctgaggg gcattgcagc cggcatgaag tacctggcgg acatgaacta cgtgcaccgt 2340
gaccttgctg ctcgaaacat cctcgtcaac agcaacctgg tgtgtaaggt gtctgatttt 2400
gggctctcac gcttcctgga ggatgacacg tctgacccca cctataccag cgctctgggt 2460
gggaagatcc ccatccgttg gacggcaccg gaagccatcc agtaccggaa attcacctcg 2520
gccagtgatg tgtggagcta tggcatcgtc atgtgggagg tgatgtccta cggggaacga 2580

ccctactggg acatgaccaa tcaagacgta atcaacgcca ttgaacagga ctacagacta 2640
cctccgccca tggactgccc tagtgccctg caccagctca tgctggactg ctggcagaag 2700
gaccgcaacc accggcccaa gttcggccag attgtcaaca cgctggacaa gatgatccga 2760
aaccccaaca gcctcaaagc catggcaccc ctgtcctctg gcatcaacct gccactgctg 2820
gaccgcacga taccggacta caccagcttt aacacggtgg atgagtggct agaggccatc 2880
aagatgggcc agtacaagga gagctttgcc aacgccggct ttacctcttt cgacgttgta 2940
tctcagatga tgatggagga cattctccgc gttggggtca ctctagctgg ccaccagaaa 3000
aaaatcctga acagtatcca ggtgatgcgg gcccagatga accagatcca gtctgtagag 3060
gtttgacatt cgcctgcctc ggttctcctc ttcctccacg ccgccccctg agccccccta 3120
catcggtccc cctgctgctc tgtccactac aaggtcagcc acctgccagg aggccacaga 3180
caacaggaag aaccaagcag cactccagcc aagccgtcac caagagccca tgcaagtcaa 3240
aggaaggaaa aggggattgg gtggggggga cacgcaccta gatctgggag ggggcgggac 3300
aggcaagaga cgttcgttcc aagaggattc tcataaggga agtgacggtt cttagaggag 3360
aaaagcaaaa ggcttgggag atccacatgg cctgtccccg tgaaggccaa aaagcatttt 3420
ctctccaact ccttctggga aggttgagct ggccagagcc aagaaacact tccaggaaca 3480
ttaacatgaa gggaagaggt gagccaccct ccttgcctgg ccttagccgt ttcttggagc 3540
atcactaatg gcccagcccc caggcacaag gggcagagag acaggcagtc gccccagata 3600
ggctccgggg aagtccattc cagctgccac cggcagacag gaggatagtt gttctgtctt 3660
gggagacggt ttccaaaaac tccgagagac tttctgctaa tggttcagga ggcggaagag 3720
gagctcctgg cctcccaggt cacgccaaac aggctagatc ttcctgagga cagccaccca 3780
ggtctgcagc cccacaaggc cctgctcctg atcagcctca gccagaggac gaggaggcag 3840
gtgactgtca tcagcctcga ctgccacaga gaagcatact cttgcatctg ggtttgttta 3900
cagcgattcc gtggaccggg ggtattttga tcaggggtgg ttttggtctg ggagggtttt 3960
gtttgttggg tttttttaaa aaaaaatgac aatgaagaga cactttgaca tttcctacct 4020
tttgaggact tgatccttct ccaggaagaa ggtgctttct gcttactgac ttaggcaata 4080
caccaagggc gagattttat atgcacattt ctggattttt ttatatgctt ttcattgata 4140
cccttccctc ctcccacctg ctgccaggcc tcaccaaagc caactgtcac agggccacct 4200
gggccactca gagacctcag tgggactggg agagtggagg gaactacatc agcgcccaca 4260
ctggaccaca gacggaggca gatgagggct ccaaggccgg aagtcctccc ttcctccctg 4320
tctgctctca tgctctccgc cctgttctat ctggcacttc ccaggcacag aggccccttc 4380
aggttcctgg gtgctcttca atggccaaaa ctcgatttca tgtctgggag cctggaccag 4440
gctacccggg aagtcaggac cctgatcatt cactgtgaca ccccagccct ccaagggtac 4500
cccgggagac cacgtgactc tctctctctc tctgtctctc tctctctctg tctctctctc 4560
tctctctctc tctctgtctc tctctctctc tgtctctctc tgtctctctc ccactccttc 4620

tttccccgag gcctttgcct gggtctgaag gtcttggcca gaggaacacc atcatcagtc 4680

aaggggtctg gccgcaggct cccctgtgag caaatgcagg gaatccgagc agaacaatcg 4740

gttagtgaat tgaatggaaa taaatgcttt agttataaaa 4780

<210> 78
<211> 4610
<212> DNA
<213> rat

<400> 78
atggatctct ttcactttaa gctttatggc ttcttctcac cacttactgg ttctgacact 60

gaaacactga tggactctac tacagcaacg gctgagctgg gctggatggt acatccccca 120

tcagggtggg aagaggtgag cggctacgat gagaacatga acactatccg tacctaccag 180

gtgtgcaatg tctttgagtc aagccagaac aactggctac ggaccaaatt catccggcgc 240

cgcggcgccc accgcatcca cgtggagatg aagttctcgg tgcgtgactg tagcagcatt 300

cccagcgtgc ccggctcctg caaggagacc ttcaacctct actactatga ggctgatttt 360

gacttagcca ccaagacctt tcccaactgg atggagaatc cgtgggtgaa ggtggacacc 420

attgcggctg atgagagctt ttcccaggtg gatctgggtg gccgcgtcat gaaaatcaac 480

actgaggtgc gaagcttcgg tcccgtgtcc cgcaatggtt tctacctggc cttccaggac 540

tacggcggct gtatgtccct cattgcggtg cgcgtcttct accggaagtg cccccgagtc 600

atccagaatg gtgccatctt ccaggagaca ctgtcggggg ctgagagcac ctcactggta 660

gctgctcggg gcagctgcat cgccaacgct gaagaagtgg atgtacccat caaactctac 720

tgtaacgggg acggcgaatg gctggtgccc atcggtcgct gcatgtgcaa ggcgggcttc 780

gaggctgtgg agaacggtac cgtctgccga ggttgtccat caggaacctt caaggccaac 840

caaggggatg aagcctgtac ccactgtccc atcaacagcc gtaccacctc cgagggtgcc 900

accaactgtg tgtgccgtaa cggctactac agggctgacc tggacccctt agacatgcct 960

tgcacaacca tcccctctgc gccccaggct gtgatctcca gtgtcaacga gacgtccctc 1020

atgctagagt ggaccccgcc acgagactcg gggggccgtg aggaccttgt ttataacatc 1080

atctgcaaga gctgtggctc aggccggggt gcctgcacgc gctgtggaga caatgtgcag 1140

tacgcacccc gccagctagg cctgaccgag ccacgcatct acatcagtga cctgctggca 1200

cacacacagt acaccttcga gatccaggcc gtgaatggtg tcaccgacca gagtcccttc 1260

tcacctcagt tcgcctctgt gaacatcacc accaaccaag cagcaccgtc ggccgtgtcc 1320

atcatgcacc aggtgagccg caccgtggac agcatcaccc tgtcgtggtc ccagccagac 1380

cagcccaacg gtgtgatcct ggactatgaa ctacagtact atgagaagga gctcagtgag 1440

tacaacgcca cagccataaa aagccccacc aacacagtca ctgtgcaggg cctcaaagcc 1500

ggcgccatct atgtcttcca ggtgcgggca cgcaccgtcg caggctatgg gcgttacagt 1560

ggcaagatgt acttccaaac catgacagaa gccgagtacc agaccagcat caaggaaaag 1620

ctgcccctca tcgtcggctc ctccgctgcc ggcgtagtct tcgtcatcgc cgtggtcgtc 1680
attgccatcg tatgtaacag aagacggggg tttgagcgtg ccgactcaga atacacggac 1740
aagctgcagc actacaccag cggccacatg accccaggca tgaagatcta tattgaccct 1800
ttcacctatg aagatcctaa tgaggcagtg cgggagtttg ccaaggaaat cgacatctcc 1860
tgtgtcaaga ttgagcaggt gattggagca ggggaatttg gtgaggtctg cagtggccac 1920
ttgaagctgc cagggaagag agagatcttt gtagccatca agaccctcaa gtcaggatac 1980
acagagaaac agcgccggga cttcctgagt gaggcatcca tcatgggcca gttcgaccac 2040
cccaatgtca tccacctgga aggggttgtc accaagagca cacctgtcat gatcatcact 2100
gaattcatgg agaacggttc cctggactcc ttcctccggc aaaacgatgg gcagttcacg 2160
gtcatccaac tggtgggcat gctgaggggc atcgcagctg gcatgaagta cctggcggac 2220
atgaactatg tgcaccggga ccttgctgca cgcaacatcc ttgtcaatag caacctggtg 2280
tgcaaggtgt ctgacttcgg gctctcacgc ttcctggagg acgacacatc tgaccccacc 2340
tacaccagcg ctctgggtgg gaagatcccc atccgttgga cagcaccgga agccatccag 2400
taccggaaat tcacctcagc cagtgatgtg tggagctatg gcattgtcat gtgggaggtg 2460
atgtcctacg gggagcgacc ctactgggac atgaccaacc aagacgtaat caatgccatc 2520
gagcaggact accggctacc tccgcccatg gactgcccca gcgcgctgca ccagctcatg 2580
ctggattgct ggcagaagga ccgcaaccac cggcccaagt tcggccagat cgtcaacacg 2640
ctggacaaga tgatccgaaa ccccaacagc ctcaaagcta tggcgcccct gtcctctggt 2700
atcaacctgc cactactgga ccgcacgata cccgactaca ccagctttaa cacagtggat 2760
gagtggctgg aggccatcaa gatgggccag tacaaggaga gctttaccaa cgccggcttc 2820
acctcttttg acgtcgtatc tcagatgatg atggaggaca ttctccgggt tggggtcact 2880
ctagctggcc accagaaaaa aatcctgaac agtatccagg tgatgcgggc ccagatgaac 2940
cagatccagt cggtagaggt ttgatggtcg cctgcctcgg ctctcctcgt cctccatgcg 3000
cccctaagcc ccttacgtcg gtccccctgc tgctctgtcc actgcaaggt cagccacctg 3060
ccagcaggcc acaggcaaca ggaagaacca agcagcactc cagtcaagcc gtcaccaaga 3120
acccatgcaa gtcaaaggaa ggaaaaggga atggggggga cacgcaccta gatctgggag 3180
ggggcgggac aggcaagaaa cgtttgttca aagaggattc tcataagaag agtgatggtt 3240
cttaggggag aaaagcaaaa aggcttggga gacccacatg gcctgtcccg gtggaggcca 3300
aaagcatttt ctctccaact ccttctggga aggttgagct ggtcagagcc aagggggaaa 3360
aaaaaatgct tccaggaaca ttaacatgga gggaagaggt gggccaccct ccttgcctgg 3420
ccttggctgt ttcttggagc atcactaatg gcccagtccc aggcacaagg ggcagagaga 3480
caggcagtcg ccccaaataa gctctgggga agtccattcc agtcgccacc ggcagacaga 3540
acagttctgt cttgggagcc ggtttcaaaa actccgaggg acttttcttc tgctaatggt 3600
tcaggaggcg acacacagag gagctcctgg ccttccggat cacactgaac aggccagatc 3660

ttcctgagga cagccaccca ggtctgcagc cccacaaggc cctgccctgc acgggtgccc 3720

gctcctgccc tgctgcaggt gactgtcttg agcctcgact gccactgaga agcattctct 3780

tgcatctggg tttgtttaca gcaattcgtg gacttggggg tattttgatc aggggtggtt 3840

ttggtctggg agggtttgtt tgttgggttt ttttaaatga caatgaagag acactttgac 3900

atttcctacc ttttgaggac ttgatccttc tccaggaaga aggtgctttc tgcttactga 3960

cttaggcaat acaccaaggg caagatttta tatgcacatt tctggatttt tatacgcttt 4020

tcattgacac ccttccctcc ccgcacctgc tgccaggcct caccaaagcc aactgtcaca 4080

gggccacctg ggccattcag agacctcagt gggtctgggg gaatggaggg agccgcacca 4140

acaccccccc ccccccgcat tggaccccag actgagtgag gcagatgagg gcttcctctg 4200

caggaagtcc tctcttcctc cctgcacatt cccatgctct ctgccctgtt ctatctggta 4260

cttcccaggc acagaggccc cttcaggttc ctgggtgctc tccgatggcc aaaacccaat 4320

ttcacctctg ggagcctgga ccaggctacc caggaagtca ggaccctgat cattcactgt 4380

gacaccctag ccctccaagg gtaccccggg agaccatgtg actctctctc ccactccttc 4440

tccctacccc ccacccccgc ctttgcccag gtctgaaggt cttggccaga ggaatatcat 4500

cagtcagggg tctggcctca gactctcctg tgagcaaaag caggagaccc gagcagaaca 4560

atcggttagt gaattgaacg gaaataaatg ctttagttat aaaatgaccc 4610

<210> 79
<211> 4234
<212> DNA
<213> human

<400> 79
cgtgagcggc gcagcaagat cccagctcgg accccggacg gcgcgcgccc ccgaagcccc 60

ggatcccagt cgggcccgca gctgaccgcc agattactgt gcatcccgaa tcacgaccac 120

ctgcaccctc ctgccccggc ccgcccccca agtcctcagg cacccagctc cccggcgccc 180

cggatcctcc tggaccggtc cgtccagatt cccgcgggac cgacctgtcc gcatccccag 240

gaccgccggg ctcggtgcac cgcctcggtc ccggagccgc ccgcctggat tgcattccct 300

cctctcctgg atctcctggg acccgacgcg agcctgcccc ggagcccgcc gagcgcaccc 360

tctctcgggt gcctgcagcc ccgccggcgc ggcccggccc ggcgcggccc ggctcggctc 420

ctagagctgc cacggccatg gccagagccc gcccgccgcc gccgccgtcg ccgccgccgg 480

ggcttctgcc gctgctccct ccgctgctgc tgctgccgct gctgctgctg cccgccggct 540

gccgggcgct ggaagagacc ctcatggaca caaaatgggt aacatctgag ttggcgtgga 600

catctcatcc agaaagtggg tgggaagagg tgagtggcta cgatgaggcc atgaatccca 660

tccgcacata ccaggtgtgt aatgtgcgcg agtcaagcca gaacaactgg cttcgcacgg 720

ggttcatctg gcggcgggat gtgcagcggg tctacgtgga gctcaagttc actgtgcgtg 780

actgcaacag catccccaac atccccggct cctgcaagga gaccttcaac ctcttctact 840

acgaggctga cagcgatgtg gcctcagcct cctccccctt ctggatggag aacccctacg 900

tgaaagtgga caccattgca cccgatgaga gcttctcgcg gctggatgcc ggccgtgtca 960
acaccaaggt gcgcagcttt gggccacttt ccaaggctgg cttctacctg gccttccagg 1020
accagggcgc ctgcatgtcg ctcatctccg tgcgcgcctt ctacaagaag tgtgcatcca 1080
ccaccgcagg cttcgcactc ttccccgaga ccctcactgg ggcggagccc acctcgctgg 1140
tcattgctcc tggcacctgc atccctaacg ccgtggaggt gtcggtgcca ctcaagctct 1200
actgcaacgg cgatggggag tggatggtgc ctgtgggtgc ctgcacctgt gccaccggcc 1260
atgagccagc tgccaaggag tcccagtgcc gcccctgtcc ccctgggagc tacaaggcga 1320
agcagggaga ggggccctgc ctcccatgtc cccccaacag ccgtaccacc tccccagccg 1380
ccagcatctg cacctgccac aataacttct accgtgcaga ctcggactct gcggacagtg 1440
cctgtaccac cgtgccatct ccaccccgag gtgtgatctc caatgtgaat gaaacctcac 1500
tgatcctcga gtggagtgag ccccgggacc tgggtggccg ggatgacctc ctgtacaatg 1560
tcatctgcaa gaagtgccat ggggctggag gggcctcagc ctgctcacgc tgtgatgaca 1620
acgtggagtt tgtgcctcgg cagctgggcc tgacggagcg ccgggtccac atcagccatc 1680
tgctggccca cacgcgctac acctttgagg tgcaggcggt caacggtgtc tcgggcaaga 1740
gccctctgcc gcctcgttat gcggccgtga atatcaccac aaaccaggct gccccgtctg 1800
aagtgcccac actacgcctg cacagcagct caggcagcag cctcacccta tcctgggcac 1860
ccccagagcg gcccaacgga gtcatcctgg actacgagat gaagtacttt gagaagagcg 1920
agggcatcgc ctccacagtg accagccaga tgaactccgt gcagctggac gggcttcggc 1980
ctgacgcccg ctatgtggtc caggtccgtg cccgcacagt agctggctat gggcagtaca 2040
gccgccctgc cgagtttgag accacaagtg agagaggctc tggggcccag cagctccagg 2100
agcagcttcc cctcatcgtg ggctccgcta cagctgggct tgtcttcgtg gtggctgtcg 2160
tggtcatcgc tatcgtctgc ctcaggaagc agcgacacgg ctctgattcg gagtacacgg 2220
agaagctgca gcagtacatt gctcctggaa tgaaggttta tattgaccct tttacctacg 2280
aggaccctaa tgaggctgtt cgggagtttg ccaaggagat cgacgtgtcc tgcgtcaaga 2340
tcgaggaggt gatcggagct ggggaatttg gggaagtgtg ccgtggtcga ctgaaacagc 2400
ctggccgccg agaggtgttt gtggccatca agacgctgaa ggtgggctac accgagaggc 2460
agcggcggga cttcctaagc gaggcctcca tcatgggtca gtttgatcac cccaatataa 2520
tccggctcga gggcgtggtc accaaaagtc ggccagttat gatcctcact gagttcatgg 2580
aaaactgcgc cctggactcc ttcctccggc tcaacgatgg gcagttcacg gtcatccagc 2640
tggtgggcat gttgcggggc attgctgccg gcatgaagta cctgtccgag atgaactatg 2700
tgcaccgcga cctggctgct cgcaacatcc ttgtcaacag caacctggtc tgcaaagtct 2760
cagactttgg cctctcccgc ttcctggagg atgacccctc cgatcctacc tacaccagtt 2820
ccctgggcgg gaagatcccc atccgctgga ctgccccaga ggccatagcc tatcggaagt 2880
tcacttctgc tagtgatgtc tggagctacg gaattgtcat gtgggaggtc atgagctatg 2940

gagagcgacc ctactgggac atgagcaacc aggatgtcat caatgccgtg gagcaggatt 3000
accggctgcc accacccatg gactgtccca cagcactgca ccagctcatg ctggactgct 3060
gggtgcggga ccggaacctc aggcccaaat tctcccagat tgtcaatacc ctggacaagc 3120
tcatccgcaa tgctgccagc ctcaaggtca ttgccagcgc tcagtctggc atgtcacagc 3180
ccctcctgga ccgcacggtc ccagattaca caaccttcac gacagttggt gattggctgg 3240
atgccatcaa gatggggcgg tacaaggaga gcttcgtcag tgcggggttt gcatcttttg 3300
acctggtggc ccagatgacg gcagaagacc tgctccgtat tggggtcacc ctggccggcc 3360
accagaagaa gatcctgagc agtatccagg acatgcggct gcagatgaac cagacgctgc 3420
ctgtgcaggt ctgacaccgg ctcccacggg gaccctgagg accgtgcagg gatgccaagc 3480
agccggctgg actttcggac tcttggactt ttggatgcct ggccttaggc tgtggcccag 3540
aagctggaag tttgggaaag gcccaagctg ggacttctcc aggcctgtgt tccctcccca 3600
ggaagtgcgc cccaaacctc ttcatattga agatggatta ggagaggggg tgatgacccc 3660
tccccaagcc cctcagggcc cagaccttcc tgctctccag caggggatcc ccacaacctc 3720
acacttgtct gttcttcagt gctggaggtc ctggcagggt caggctgggg taagccgggg 3780
ttccacaggg cccagccctg gcaggggtct ggccccccag gtaggcggag agcagtccct 3840
ccctcaggaa ctggaggagg ggactccagg aatggggaaa tgtgacacca ccatcctgaa 3900
gccagcttgc acctccagtt tgcacaggga tttgttctgg gggctgaggg ccctgtcccc 3960
acccccgccc ttggtgctgt cataaaaggg caggcagggg caggctgagg agttgccctt 4020
tgccccccag agactgactc tcagagccag agatgggatg tgtgagtgtg tgtgtgtgtg 4080
tgtgtgtgtg cgcgcgcgcg cgcgtgtgtg tgtgcacgca ctggcctgca cagagagcat 4140
gggtgagcgt gtaaaagctt ggccctgtgc cctacaatgg ggccagctgg gccgacagca 4200
gaataaaggc aataagatga aaaaaaaaaa aaaa 4234

<210> 80
<211> 4164
<212> DNA
<213> mouse

<400> 80
agcggcgcag ccagataccg gctcctaccc cggacggcga gcgcacaagg atcccgggat 60
ctgagctcgg cctcagtgga ctgtgccccg agaacctgcg accacctgcg ccctccgctt 120
cccgagtcat tgggcatcaa gccacccagc gccccggatc ctcctggacc ggtccgtcca 180
gattcccgcg ggaacgacct gccggcatct acccggagtc ccgggttggg cgacacctcg 240
gttacagagc agcccgtctg ctttgctccc cctccctgac gggatctctt ggactgtgcg 300
cgagccttct tcctcgggcg tccccagccc cgccggcgcg gcccggctcg gccccccaac 360
cgagcgcccc gggctatggc cggagcccgc ccgccgccgg ggcttctgcc gctgctcgct 420
ccgctgctgc tgccgctgct gcttcccgcc ggctgctggg cgctggaaga gactctcatg 480

gacacgaaat gggtgacgtc tgagctggca tggacatctc atccagagag tgggtgggaa 540
gaagtaagcg gctatgatga agccatgaat cctatccgca cgtatcaggt gtgtaatgtg 600
cgtgagtcga gccagaacaa ctggctgcgg acgggtttca tctggcggcg ggaagtccag 660
cgcgtctacg tggagctgaa gttcaccgta agagactgta acagcattcc caacatccct 720
ggctcctgca aggaaacctt caaccttttc tactacgagg ctgatagcga tgtggcctca 780
gcctcctctc ccttctggat ggagaacccc tacgtgaaag tggacaccat tgcgccagat 840
gagagcttct cgcggctcga tgccgggcgt gtcaacacca aggtgcgcag cttcgggccg 900
ctttccaagg ctggcttcta cttggccttc caggaccaag gtgcctgcat gtcactcatc 960
tctgtgcgtg ccttctacaa gaagtgtgca tccaccactg caggcttcgc actcttcccc 1020
gagaccctca cgggggctga gcccacttcg ctggtcattg cccccggcac ctgcatcgct 1080
aacgctgtgg aggtctctgt accactcaag ctctactgca atggcgacgg ggagtggatg 1140
gtgcccgtcg gtgcctgcac ctgcgctact ggccatgagc cagccgccaa ggagtcccag 1200
tgccgtgcct gtcctcctgg gagttacaaa gcaaagcaag gagaagggcc ctgcctcccc 1260
tgtcccccca atagccgcac cacctcgccg gctgccagca tctgcacctg ccataataac 1320
ttctaccgtg cagactcaga ctcggccgac agcgcctgca ccacggtgcc atctccaccc 1380
cggggtgtga tctccaatgt gaatgagacc tcgctaatcc tcgaatggag tgagccccgg 1440
gaccttggcg gacgagatga cctcctttat aatgttatct gcaagaagtg ccgtgggagc 1500
tctggggctg ggggtccggc aacctgttca cgctgcgatg acaacgtaga gtttgtacct 1560
cggcagctgg gcctgacaga gcgccgggtc cacattagcc acctgttggc ccacacacgc 1620
tacacctttg aggtgcaggc tgtcaacggt gtctcaggca aaagcccttt gccaccccgc 1680
tatgcagctg tgaatatcac caccaaccag gctgctccat cagaagtgcc tacactccac 1740
ctgcacagca gctcggggag cagcctgacc ctgtcctggg cacccccaga gcggcctaat 1800
ggagtcatct tggactatga gatgaagtac tttgagaaga gtaaaggcat cgcctccact 1860
gtaaccagcc agaagaactc cgtacaactg gacgggctgc agcctgatgc ccgctacgta 1920
gttcaggtcc gggctcgcac agtagcgggt tacggacagt atagccaccc ggctgagttt 1980
gagaccacaa gtgaaagagg ttcaggtgcc cagcagcttc aagaacagct tcccctgatt 2040
gtgggatcca ccgtagccgg gtttgtcttc atggtggtcg tcgtggtcat cgctcttgtc 2100
tgcctcagga agcagcgcca tggccccgat gcagaataca cagagaagct gcagcaatac 2160
attgctcctg ggatgaaagt ttacattgac ccctttacct atgaggatcc caatgaggcc 2220
gtccgagagt tcgccaagga gatcgatgtg tcctgtgtca agatcgagga ggtgattgga 2280
gctggggagt ttggggaagt gtgccggggt cgactgaaac tgcccggccg ccgggaggtg 2340
ttcgtggcta tcaagacgct gaaggtggga tacacggaga ggcagcggcg ggacttccta 2400
agtgaggctt ccattatggg tcagtttgac catcccaata taatccgtct agagggtgtg 2460
gtcaccaaaa gtcgtccagt catgatcctc actgagttca tggagaactg tgccctggac 2520

tcctttctac ggctcaatga cggacagttc acggtcatcc agcttgtggg catgttacga 2580

ggcattgctg ctggcatgaa gtacttgtct gagatgaact acgtgcaccg tgaccttgct 2640

gcccgaaaca tccttgtcaa cagcaacttg gtctgcaaag tctctgactt tggcctctcc 2700

cgcttcctgg aggatgaccc atcagacccc acctacacca gctccctggg tgggaagatc 2760

cctatccgct ggaccgcccc ggaggccata gcctatcgga agtttacgtc tgccagcgat 2820

gtctggagct atgggattgt catgtgggag gtcatgagct acggagagcg accctactgg 2880

gatatgagca accaggatgt cattaatgct gtagagcaag actatcggtt accacccccc 2940

atggactgcc ccaccgcgct gcaccagctc atgctggact gttgggtgcg ggaccggaac 3000

ctcaggccca agttctctca aatcgtcaac acactagaca aacttatccg caatgctgcc 3060

agcctcaagg tcaccgccag tgccccatct ggcatgtccc agcccctcct ggaccgcaca 3120

gtcccagatt atacgacctt cacgacagtg ggtgactggc tagatgccat caagatgggg 3180

aggtataaag agagctttgt cggtgctggg tttgcatcct ttgacctggt ggcccagatg 3240

actgcagaag atctgctaag gattggggtc actttggctg ggcaccagaa gaagatcctc 3300

tgcagtatcc aggacatgcg gctgcagatg aaccagaccc tgcctgtgca ggtctgacac 3360

tcagctccaa cgaggggggg gtgccccccc aggactgcac aaggattctg accagccagc 3420

tggacttttg gatgcctggc ctctggctgt ggcccagaag atggaagttt gggggaggac 3480

cctagctgtg acttctccag gcctgtgctc cctcccagga agtgtgcccc aaacctcttc 3540

atattgaaga tggattagaa gagggggtga tgtcccctcc ccagatgcct tggggcccag 3600

gcctgcctgc tctccagtgg ggagtcttca caactcagat tgggctgcgc ttcagtaatg 3660

gacgtcctgg tagggtcagg tggggataag cctgggttct tcaggcccca gccctggcag 3720

gggtctgacc ccaccaggta agcagagagt actccctccc ccaggaagtg gaggaggaga 3780

ctctggggat ggggagatat ggtgccccat cctgaagcca gctggtacct ccagtttgca 3840

cagggacttg atgggggctg agggccctgc ctacccttgg tgctgtcata aaagggcagg 3900

caggagcagg ctgagaagca gcctgtgcct cccagagact gactcagaga gccagagact 3960

gtgtgtgtgt gtgtgtgtgt gtgtgtgtgt gtgtgtgtgt gtgtgtgaaa gacgggggtg 4020

ggggtatgta tgtgtgtgtt gtgcacatgc ttgcctgcac agagagcatg agtgtgtaca 4080

agcttggccc tgtgccctgt agtggggcca gctgggcaga cagcaaaata aaggcaataa 4140

gatgaaaaaa aaaaaaaaaa aaaa 4164

<210> 81
<211> 4176
<212> DNA
<213> rat

<400> 81
ccgccgctcc acggcgagga ccagcagaag tgagtacgtg agcggagcag tctgataccg 60

gctcctaccc cggacggcgc gctcaccggg agcccgggat ccgagctcgg cctcagtgga 120

ctgtgccccg agaaccgcga ccatctgcgc cctctgcctc ccgagtcatc gggcatcaag 180

ccacccagcg ccccggatcc tcctggaccg gtccgtccag attcccgcgg gaacgacctg 240
ccggcatcta ccctgagtac cgggttgggc gacacctcag atacagagcc gcccgtctgc 300
tttgcgcccc ctccctgccg ggatctcttg gactgtgcgc gagccttctt cctcaggcgc 360
ccccagcccc gccggcgcgg cccggctcgg cgccccccga gcgctccggg ctatggccgg 420
agcccgcccg ccgccggggc ttctgccgct gctcgctccg ctgctgctgc cgctgctgct 480
gcccgccggc tgccgggcgc tggaagagac tctcatggac acgaaatggg tgacgtccga 540
gctggcatgg acatctcatc cagagagtgg gtgggaagaa gtgagcggct acgatgaagc 600
catgaatcct atccgcacgt atcaggtgtg taacgtgcgc gagtccagcc agaacaactg 660
gctgcggacc ggtttcatct ggcggcggga agtccagcgc gtctacgtgg agctgaagtt 720
taccgtgaga gattgcaaca gcatccccaa catccctggc tcctgcaagg aaaccttcaa 780
ccttttttac tacgaggctg atagcgatgt ggcgtcagcc tcctctccct tctggatgga 840
gaacccctac gtgaaagtgg acaccattgc gccagatgag agcttctcgc ggctagacgc 900
tgggcgcgtt aacaccaaag tgcgcagctt cgggccgctt tccaaagccg gcttctactt 960
ggccttccag gaccagggtg cctgcatgtc actcatctct gtgcgcgcct tctacaagaa 1020
gtgtgcatcc accactgcag gcttcgcact cttccccgag accctcacgg gggctgagcc 1080
cacttcgctg gtcattgccc ctggcacctg catcgctaac gctgtggagg tgtctgtacc 1140
gctcaagctc tactgcaatg gcgacgggga gtggatggtg cccgttggtg cctgcacctg 1200
cgctactggc catgagccag ccgccaagga gacccagtgc cgcgcctgtc cccctgggag 1260
ctacaaggca aagcaaggag aggggccctg cctcccctgt ccccccaata gccgcaccac 1320
ctcgccggct gccagcatct gcacctgtca caataatttc taccgcgcag actcagacac 1380
agcggacagc gcctgcacca cggtgccgtc tcccccccgg ggtgtgatct ccaatgtgaa 1440
tgagacctcg ctgatcctcg agtggagtga gccccgggac cttggcggac gagatgacct 1500
cctttataat gttatctgta agaagtgccg tggcagctct ggggctggag gtccggcgac 1560
ctgttcacgc tgtgatgaca acgtggagtt cgagccccga cagctgggcc tgaccgagcg 1620
ccgggtccac atcagccacc tgttggccca cacccgctac acctttgagg tgcaggctgt 1680
caacggcgtc tctggcaaaa gccctttgcc gccccgctat gcagctgtga atatcaccac 1740
caaccaggcc gccccatcag aagtgcctac gctccacttg cacagcagtt cagggagcag 1800
cctgaccctg tcctgggcac ccccggagcg gcctaacgga gtcatcttgg actatgagat 1860
gaagtacttt gagaagagta aaggcatcgc ctccactgtc accagccaga agaactctgt 1920
acaactggac ggactgcagc ccgacgcccg ctatgtagtt caggtccggg ctcgcacagt 1980
agcaggttac ggacagtata gccgcccagc tgagtttgag accacgagtg aaagaggctc 2040
aggggcccag cagcttcaag agcagcttcc cctaattgtg ggatccaccg tagctggctt 2100
tgtcttcatg gtggtcgtcg tggtcattgc tcttgtctgc ctcaggaagc agcgccaggg 2160
ccctgatgca gaatacacgg agaagttgca gcaatacgtt gcccccagga tgaaagttta 2220

cattgacccc tttacctacg aggatcccaa tgaggccgtc cgagagttcg ccaaggagat 2280
cgatgtgtcc tgcgtcaaga tcgaggaggt gattggagct ggggagtttg gggaagtgtg 2340
ccggggtcgg ctgaaactgc ccggccgccg ggaggtgttc gtggccatca agacactgaa 2400
ggtgggatac acggagaggc agcggcggga cttcctgagt gaggcttcca tcatgggtca 2460
atttgaccat ccaaatataa tccgtctaga gggcgtggtc accaaaagtc gtccagtcat 2520
gatcctcact gagttcatgg agaactgtgc cctggactcc ttcctacggc tcaatgacgg 2580
gcagttcaca gtcatccagc ttgtgggcat gttgcgtggc attgctgccg gcatgaagta 2640
cttgtctgag atgaactacg tgcaccgtga cctcgctgcc cgcaacatcc ttgtcaacag 2700
taacttggtc tgcaaagtat ctgactttgg gctctcccgc ttcctggagg acgacccctc 2760
agaccccacc tacaccagct ccctgggtgg gaagatccct atccgttgga ccgccccaga 2820
ggccatagcc tatcggaagt tcacgtctgc cagcgatgtc tggagctacg ggatcgtcat 2880
gtgggaggtc atgagctacg gagagcgacc atactgggac atgagcaacc aggatgtcat 2940
caatgccgta gagcaagact atcggttacc accccccatg gactgcccca cggcgctgca 3000
ccagctcatg ctggactgtt gggtgcggga ccggaacctc aggcccaagt tctcccaaat 3060
cgtcaacacg ctagacaagc ttatccgcaa tgctgccagc ctcaaggtca tcgccagtgc 3120
cccatctggc atgtcccagc ccctcctaga ccgcacggtc ccagattata cgaccttcac 3180
gacggtgggc gactggctag atgccatcaa gatggggagg tataaagaga gcttcgtcgg 3240
tgcgggtttt gcctcctttg acctggtggc ccagatgact gcagaagatc tgctaaggat 3300
cggggtcact ttggccggcc accagaagaa gatcctcagc agtatccagg acatgcggct 3360
gcagatgaac cagacactgc ccgtgcaggt ctgacgctca gctccagcga ggggcgtggc 3420
cccccgggac tgcacaagga ttctgaccag ccagctggac ttttggatac ctggcctttg 3480
gctgtggccc agaagacaga agttcggggg agaaccctag ctgtgacttc tccaagcctg 3540
tgctccctcc caggaagtgt gccccaaacc tcttcatatt gaagatggat tagaagaggg 3600
ggtgatatcc cctccccaga tgcctcaggg cccaggcctg cctgctctcc agtcggggat 3660
cttcacaact cagatttggt tgtgcttcag tagtggaggt cctggtaggg tcgggtgggg 3720
ataagcctgg gttcttcagg ccccagccct ggcaggggtc tgaccccagc aggtaagcag 3780
agagtactcc ctccccagga agtggaggag gggactctgg gaatggggaa atatggtgcc 3840
ccatcctgaa gccggctggt acctccagtt tgcacaggga cttgttgggg gctgagggcc 3900
ctgcctaccc ttggtgctgt cataaaaggg caggcgggag caggctgaga agcagcctgt 3960
gcctcccaga gactgactca gagagccaga gacgtgtgtg tgtgtgtgtg tgtgtgtgtg 4020
tgtgtgtgtg tgtgtgtgaa agacgggggt ggggtatgta tgcgtgtgtt gtgcacatgc 4080
ttgcctgcac agagagcatg agtgtgtaca agcttagccc tgtgccctgt agtggggcca 4140
gctgggcaga cagcgaaata aaaggcaata agttga 4176

<210> 82
<211> 4369
<212> DNA
<213> human

<400> 82
ttccagcgca gctcagcccc tgcccggccc ggcccgcccg gctccgcgcc gcagtctccc 60

tccctcccgc tccgtccccg ctcgggctcc caccatcccc gcccgcgagg agagcactcg 120

gcccggcggc gcgagcagag ccactccagg gaggggggga gaccgcgagc ggccggctca 180

gcccccgcca cccggggcgg gaccccgagg ccccggaggg accccaactc cagccacgtc 240

ttgctgcgcg cccgcccggc gcggccactg ccagcacgct ccgggcccgc cgcccgcgcg 300

cgcggcacag acgcggggcc acacttggcg ccgccgcccg gtgccccgca cgctcgcatg 360

ggcccgcgct gagggccccg acgaggagtc ccgcgcggag tatcggcgtc cacccgccca 420

gggagagtca gacctggggg ggcgagggcc ccccaaactc agttcggatc ctacccgagt 480

gaggcggcgc catggagctc cgggtgctgc tctgctgggc ttcgttggcc gcagctttgg 540

aagagaccct gctgaacaca aaattggaaa ctgctgatct gaagtgggtg acattccctc 600

aggtggacgg gcagtgggag gaactgagcg gcctggatga ggaacagcac agcgtgcgca 660

cctacgaagt gtgtgacgtg cagcgtgccc cgggccaggc ccactggctt cgcacaggtt 720

gggtcccacg gcggggcgcc gtccacgtgt acgccacgct gcgcttcacc atgctcgagt 780

gcctgtccct gcctcgggct gggcgctcct gcaaggagac cttcaccgtc ttctactatg 840

agagcgatgc ggacacggcc acggccctca cgccagcctg gatggagaac ccctacatca 900

aggtggacac ggtggccgcg gagcatctca cccggaagcg ccctggggcc gaggccaccg 960

ggaaggtgaa tgtcaagacg ctgcgtctgg gaccgctcag caaggctggc ttctacctgg 1020

ccttccagga ccagggtgcc tgcatggccc tgctatccct gcacctcttc tacaaaaagt 1080

gcgcccagct gactgtgaac ctgactcgat tcccggagac tgtgcctcgg gagctggttg 1140

tgcccgtggc cggtagctgc gtggtggatg ccgtccccgc ccctggcccc agccccagcc 1200

tctactgccg tgaggatggc cagtgggccg aacagccggt cacgggctgc agctgtgctc 1260

cggggttcga ggcagctgag gggaacacca agtgccgagc ctgtgcccag ggcaccttca 1320

agcccctgtc aggagaaggg tcctgccagc catgcccagc caatagccac tctaacacca 1380

ttggatcagc cgtctgccag tgccgcgtcg ggtacttccg ggcacgcaca gacccccggg 1440

gtgcaccctg caccacccct ccttcggctc cgcggagcgt ggtttcccgc ctgaacggct 1500

cctccctgca cctggaatgg agtgcccccc tggagtctgg tggccgagag gacctcacct 1560

acgccctccg ctgccgggag tgccgacccg gaggctcctg tgcgccctgc gggggagacc 1620

tgacttttga ccccggcccc cgggacctgg tggagccctg ggtggtggtt cgagggctac 1680

gtcctgactt cacctatacc tttgaggtca ctgcattgaa cggggtatcc tccttagcca 1740

cggggcccgt cccatttgag cctgtcaatg tcaccactga ccgagaggta cctcctgcag 1800

tgtctgacat ccgggtgacg cggtcctcac ccagcagctt gagcctggcc tgggctgttc 1860

cccgggcacc cagtggggct gtgctggact acgaggtcaa ataccatgag aagggcgccg 1920
agggtcccag cagcgtgcgg ttcctgaaga cgtcagaaaa ccgggcagag ctgcgggggc 1980
tgaagcgggg agccagctac ctggtgcagg tacgggcgcg ctctgaggcc ggctacgggc 2040
ccttcggcca ggaacatcac agccagaccc aactggatga gagcgagggc tggcgggagc 2100
agctggccct gattgcgggc acggcagtcg tgggtgtggt cctggtcctg gtggtcattg 2160
tggtcgcagt tctctgcctc aggaagcaga gcaatgggag agaagcagaa tattcggaca 2220
aacacggaca gtatctcatc ggacatggta ctaaggtcta catcgacccc ttcacttatg 2280
aagaccctaa tgaggctgtg agggaatttg caaaagagat cgatgtctcc tacgtcaaga 2340
ttgaagaggt gattggtgca ggtgagtttg gcgaggtgtg ccgggggcgg ctcaaggccc 2400
cagggaagaa ggagagctgt gtggcaatca agaccctgaa gggtggctac acggagcggc 2460
agcggcgtga gtttctgagc gaggcctcca tcatgggcca gttcgagcac cccaatatca 2520
tccgcctgga gggcgtggtc accaacagca tgcccgtcat gattctcaca gagttcatgg 2580
agaacggcgc cctggactcc ttcctgcggc taaacgacgg acagttcaca gtcatccagc 2640
tcgtgggcat gctgcggggc atcgcctcgg gcatgcggta ccttgccgag atgagctacg 2700
tccaccgaga cctggctgct cgcaacatcc tagtcaacag caacctcgtc tgcaaagtgt 2760
ctgactttgg cctttcccga ttcctggagg agaactcttc cgatcccacc tacacgagct 2820
ccctgggagg aaagattccc atccgatgga ctgccccgga ggccattgcc ttccggaagt 2880
tcacttccgc cagtgatgcc tggagttacg ggattgtgat gtgggaggtg atgtcatttg 2940
gggagaggcc gtactgggac atgagcaatc aggacgtgat caatgccatt gaacaggact 3000
accggctgcc cccgccccca gactgtccca cctccctcca ccagctcatg ctggactgtt 3060
ggcagaaaga ccggaatgcc cggccccgct tcccccaggt ggtcagcgcc ctggacaaga 3120
tgatccggaa ccccgccagc ctcaaaatcg tggcccggga gaatggcggg gcctcacacc 3180
ctctcctgga ccagcggcag cctcactact cagcttttgg ctctgtgggc gagtggcttc 3240
gggccatcaa aatgggaaga tacgaagaaa gtttcgcagc cgctggcttt ggctccttcg 3300
agctggtcag ccagatctct gctgaggacc tgctccgaat cggagtcact ctggcgggac 3360
accagaagaa aatcttggcc agtgtccagc acatgaagtc ccaggccaag ccgggaaccc 3420
cgggtgggac aggaggaccg gccccgcagt actgacctgc aggaactccc caccccaggg 3480
acaccgcctc cccattttcc ggggcagagt ggggactcac agaggccccc agccctgtgc 3540
cccgctggat tgcactttga gcccgtgggg tgaggagttg gcaatttgga gagacaggat 3600
ttgggggttc tgccataata ggaggggaaa atcacccccc agccacctcg gggaactcca 3660
gaccaagggt gagggcgcct ttccctcagg actgggtgtg accagaggaa aaggaagtgc 3720
ccaacatctc ccagcctccc caggtgcccc cctcaccttg atgggtgcgt tcccgcagac 3780
caaagagagt gtgactccct tgccagctcc agagtggggg ggctgtccca gggggcaaga 3840
aggggtgtca gggcccagtg acaaaatcat tggggtttgt agtcccaact tgctgctgtc 3900

accaccaaac tcaatcattt ttttcccttg taaatgcccc tcccccagct gctgccttca 3960
tattgaaggt tttgagtttt tgtttttggt cttaattttt ctccccgttc ccttttttgtt 4020
tcttcgtttt gtttttctac cgtccttgtc ataactttgt gttggaggga acctgtttca 4080
ctatggcctc ctttgcccaa gttgaaacag gggcccatca tcatgtctgt ttccagaaca 4140
gtgccttggt catcccacat ccccggaccc cgcctgggac ccccaagctg tgtcctatga 4200
aggggtgtgg ggtgaggtag tgaaaagggc ggtagttggt ggtggaaccc agaaacggac 4260
gccggtgctt ggaggggttc ttaaattata tttaaaaaag taactttttg tataaataaa 4320
agaaaatggg acgtgtccca gctccagggg taaaaaaaaa aaaaaaaaa 4369

<210> 83
<211> 4270
<212> DNA
<213> mouse

<400> 83
ctcggttccc actttggttt ccaccttcct tgaccgcgac cggcgcacta accccggcgg 60
cgcgacatcc accaaccact ccagggagcg agagagaccg cgagcaatcg gcccacccca 120
gccagccagg gcgggacccc cgaagccccg aagggacccc taactctcag ccacgtcccg 180
atctccgccc gcccagcgac ggccactgcc aggacgtccc ccctcaaggc tggcctcccc 240
gcgcgcgcgc gctgcggcgg ggacgtgggg gccgcacctg agcgcgggtg cctggcgcgc 300
ccgcatgggc catcgttgag aggccctcga cggaaagtcc cagcagcaga gtcggtgtgc 360
acccgcccag gggagggtca gacctggggg ggccagggcc ccccaaactc ggatcgcatt 420
cagccaaagt gaggcggcgc catggagctc cgagcgctgc tgtgctgggc ttccctcgcc 480
actgctttag aagagaccct gttgaacaca aaactggaaa cggcggatct gaaatgggtg 540
acttaccctc aggcagaggg ccagtgggag gagctaagcg gcctggatga ggaacagcac 600
agcgtccgca cctatgaggt gtgcgacatg aagcgtccag ggggccaggc tcactggctg 660
cgcactggct gggtcccaag gcgaggtgct gtccacgtgt atgccacgat acgcttcacc 720
atgatggaat gcctgtccct gccgagggcc agtcgctcct gcaaggagac attcactgtc 780
ttctattacg agagcgaagc tgatacggcc acggcccata cgcccgcctg gatggagaac 840
ccctacatca aggtggacac agtggccgca gaacatctga ctcggaagcg ccctggagct 900
gaagccacag ggaaagttaa tatcaagacg ctgcgcctgg gtcctctcag caaagctggc 960
ttctacctgg ctttccagga ccaaggagcc tgcatggctc tgctctccct gcatctcttt 1020
tacaagaagt gctcctggct gatcacgaac ttgacctact tccccgagac ggtgcctcgg 1080
gagctcgtgg tgccggtggc aggtagctgc gtggccaacg cggtccctac cgccaacccc 1140
agccccagcc tctactgccg ggaagatggt caatgggctg agcagcaggt cacgggctgc 1200
agctgcgcgc cagggtacga ggctgcggaa agcaacaaag tatgcagagc ctgtggccag 1260
ggaaccttca agccccaaat aggagacgag tcctgcctgc cgtgcccagc caacagccac 1320
tcgaataaca ttgggtctcc tgtctgcctg tgtcgaattg ggtattaccg ggcccgctca 1380

gacccccgga gttcaccttg cactacccca ccctctgctc caagaagcgt ggttcaccat 1440
ttgaatggtt ccaccctgcg cctggaatgg agtgctcccc ttgagtccgg aggccgagag 1500
gacctcactt atgctgttcg ctgccgagag tgccgtcctg ggggttcctg cttgccctgt 1560
gggggcgaca tgaccttcga ccccggtcct cgagacctgg ttgagccctg ggtggcaatc 1620
cgagggctgc gtcctgatgt cacctatacc tttgaggttg ctgctttgaa tggtgtgtct 1680
accttagcca ctggaccacc tccttttgag cctgtcaatg tcaccactga ccgtgaggtg 1740
cctcctgcag tgtctgacat ccgagtgact cggtcgtcac ccagcagctt gatcctgtca 1800
tgggctctcc ccagagcacc cagtggggcc gtgctggact acgaggtcaa gtatcatgag 1860
aagggcgcag agggccccag cagtgttcgt ttcctgaaga catcagaaaa ccgagctgag 1920
ctccgggggc tgaagcgggg agccagctat ctggtcccgg tacgcgcacg gtccgaggct 1980
ggctacggtc ccttcggcca ggagcatcac agtcagactc aactggatga gagcgagagc 2040
tggcgggagc agctggccct gattgcaggc actgcggttg tgggtgtggt cctggtcctg 2100
gtggtcgtca tcattgcagt tctctgcttc aggaagcaga gctatgggag ggaagttgag 2160
tactcggata agcatgggca gtatctcatc gggcacggta ccaaggtcta cattgatcct 2220
tttacttacg aagaccctaa tgaggcagtg agggaatttg ccaaagagat cgatgtctcc 2280
tatgtcaaga ttgaagaggt aattggtgca ggtgagttcg gcgaggtgtg ccggggtcgg 2340
ctgaaggcac cagggaaaaa ggagagctgt gtggccatca agactctgaa gggtggctac 2400
accgagcgcc agaggcgtga gttcctgagc gaggcctcca tcatgggcca gttcgagcat 2460
cccaacatca tccgcctcga gggcgtggtc accaacagtg tgccggttat gatcctcacg 2520
gaattcatgg agaacggagc cctggactcc ttcctgcggc tgaacgacgg gcagttcaca 2580
gtcatccagc tggtgggcat gctgaggggc atcgcctcgg gcatgcggta cctggctgaa 2640
atgagctatg tccaccgaga cctggctgct cggaacatct tggtcaacag taacctggtc 2700
tgcaaggtgt ccgactttgg cctctccaga ttcttggagg agaactcctc tgatcccacc 2760
tacacaagtt ccctgggagg caagattccc atccgatgga ccgcccctga agccattgcc 2820
ttcaggaagt tcacctctgc cagtgatgcc tggagctatg ggatcgtcat gtgggaggtc 2880
atgtcttttg gggaacggcc atactgggac atgagcaacc aggatgtgat caatgccatt 2940
gaacaggact accggctgcc tcctcctcca gactgcccca cctccctcca ccagctcatg 3000
ctggactgtt ggcagaagga ccggaatgcc cggccccgct ttccccaggt ggtcagcgct 3060
ctggacaaga tgatccggaa tcccgctagc ctcaaaatcg tggccaggga gaatggcggg 3120
gcctcacatc cactcttgga ccaacggcag cctcactact ctgctttcgg ttctgtgggc 3180
gagtggcttc gagccatcaa gatgggaaga tacgaggaaa gttttgcagc ggctggattc 3240
ggctcctttg aggtggtcag tcagatctct gccgaggacc ttctccgaat tggagtcact 3300
ctggcaggac accagaagaa aatcttggcc agtgtgcagc atatgaagtc ccaagctaag 3360
ccaggagccc ctggtgggac agggggacca gcccagcagt tctgacctcc aaggactcac 3420

caccgtggca gattcttctt tccgggaggc agagttgggt ggggactcac aagatgaccc 3480

cctccccctc gtcacagcct tcccattgga ttgcactttg aacagagggg gtcggagaca 3540

caggatttgg ggaaccgtgc catatgggat catacatgtg ccctccaggc ggggaacccc 3600

aaactcagag tgagtctttc cctcaagact gggcaaagaa acatccctac gtctctaacc 3660

tcccatcttc ccagagggct ctctccccaa gcgccttcca cctcaacggg catgtccctg 3720

cagaccaaag agaaagggtg accagcctgc caacttggga gtggaaaatg ccgtcccagg 3780

aggcaggaag gggctgtcag gacccggtga tgtaatcatt gggttttgat gtcctgactt 3840

gctgtcacca ccaaaggcaa tcatttttcc cttgtaaatg cccctcccct catctgcctt 3900

catattgaag gttctgaagt tttactgttt tttattttgt taattttttc ctccttcccc 3960

cctccctccc cttcttgtcc agattttgtg tgttaaaggg cacctggttc cactatctcc 4020

tgttgggaac aaggacccat cgatatgttc tagaacagtg ccttggaaat gccacagcct 4080

tagacagttc cttcttcctt tcctcccccc aagtgccccc aagctgtgtt gtgtggaggg 4140

aaggggggtg atgttgtaaa agggacagga ggttggtggt ggtcaacaga ggcagacaac 4200

ggtgcttgga aggggattct taaattatat ttaaaaagaa actttttttt tttataaaaa 4260

aaaaaaaaaa 4270

<210> 84
<211> 4364
<212> DNA
<213> rat

<400> 84
agtctcaact cggcccagct ctggcccagc ctgtctccct cgctctgttc ggtccccact 60

ttggcttcta cccgctttga ccgcaaccgg cgcactcagc ttggcggcgc gagagccacc 120

taccactcca gggagcgagc aagatcgcga gcaatcggcc taccccagcc agccagggcg 180

ggacctccga agccccgaag ggacccctaa ctctcagcca cgtcccgatc tccgcccgcc 240

cagcgacggc cactgccagg acgtcccccc ttaaggctgg cctccccgcg cgcgcgcgct 300

gcggcgggga cgtgggggcc gcacctgagc gcgggtgccc ggcgcgcccg catgggccat 360

cgctgagagg cccccgacgg aaagtcccag ctgcagagtc ggtgtgcacc cgcccagggg 420

agggtcagac ctgggggggc cagggccccc caaactcgga tcgtattcag ccaaagtgag 480

gcggcgccat ggagctcaga gcgctgctgt gctgggcttc cctcgccact gctttagaag 540

agaccctgct gaacacaaaa ctggagacgg cagatctgaa atgggtgact taccctcagg 600

cagaaggcca gtgggaggag ctaagcggcc tggatgagga acagcacagc gtgcgcacct 660

atgaggtgtg cgatatgaag cgtccagggg gccaggcaca ctggctgcgc actggctggg 720

tcccacggcg aggtgctgtc cacgtgtatg ccacgatacg cttcaccatg atggaatgtc 780

tgtccctgcc gagggccagt cgctcctgca aggagacatt caccgtcttc tattatgaga 840

gcgaagccga tactgccacg gcccacacgc cagcctggat ggagaacccc tacatcaagg 900

tggacacagt ggctgcggaa catctgactc ggaagcgccc tggagctgaa gcaacaggga 960
aagttaacat caagacgctg cgcctgggtc ctctcagcaa agccggcttc tacctggctt 1020
tccaggacca aggagcctgc atggccctgc tctccttgca tctcttttac aagaagtgct 1080
cccggctggt cacgaacttg acctacttcc ccgagacggt gcctcgggag ctcgtggtgc 1140
cagtggcagg tagctgcgtg gccaatgccg tccctacggc cagccccagc cccagcctct 1200
actgccggga agatggtcag tgggctgagc agcaggtcac gggctgcagc tgcgcgccag 1260
ggtacgaggc cgcggaaggg aacaaagtgt gcagagcctg tggccagggg accttcaagc 1320
cccaaatagg agaagagccc tgcctgccat gcccagccaa cagccactct aataacattg 1380
ggtctcctgt ctgcctgtgt cggattgggt attaccgggc ccgctcggat ccccgcagtt 1440
cgccttgtac caccccaccc tctgctccaa gaagcgtagt tcaccatctg aatggctcct 1500
ccctgcgcct ggagtggagt gcccccttgg agtctggagg ccgagaggac ctcacttatg 1560
ctgtacgctg ccgagaatgc cgtcctgggg gttcctgctt gccctgtggg ggcgacctga 1620
ctttcgaccc tggtcctcga gacctggttg agccctgggt ggcaatccga gggctgcgtc 1680
ctgatgtcac ctataccttt gaggttgctg ctttgaatgg tgtgtctacc ttagccaccg 1740
gaccacctcc ttttgagcct gtcaatgtca ccactgaccg tgaagtgcct cccgcggtgt 1800
ctgacatccg agtcactcgg tcctcaccca gcagcttgat cctgtcatgg gctatcccca 1860
gagcacccag tggggccgtg ctggactacg aggtcaagta tcacgagaag ggcgcagagg 1920
gccctagcag tgtacgtttc ctgaagacat cagaaaaccg agctgagctc cgggggctga 1980
agcagggagc cagctacctg gtccaggtcc gtgcacggtc cgaggccggc tatggtccct 2040
ttggccaaga gcatcacagt cagactcaac tggatgagag cgagagttgg cgggaacagc 2100
tggccctgat cgcaggcacc gcggttgtag gcgtggtcct ggtcctggtg gtcattatta 2160
tcgcagttct ttgcctcagg aagcagagca gtgggaggga agtcgagtac tccgataaac 2220
atgggcaata tctcatcggg cacggtacca aggtctacat tgatcccttt acttacgaag 2280
accctaatga ggcagtgagg gaatttgcca aagagattga tgtctcctat gtcaagattg 2340
aagaggtaat tggtgcaggt gagttcggtg aggtgtgccg gggtcggctg aaggcaccag 2400
ggaaaaagga gagctgtgtg gccatcaaga ccttgaaggg tggctacacg gagcgccaga 2460
gacgtgagtt cctgagcgag gcctccatca tgggtcagtt cgagcatccc aacatcatcc 2520
gcctcgaggg tgtggtcact aacagcgtgc ccgtcatgat cctcacagaa ttcatggaga 2580
atggagccct agactctttc ctgcggaatc tcccagagag ggctgggggg catggctcag 2640
tgctggtggg catgctgaga ggcattgcct caggcatgcg gtacctggcc gaaatgagct 2700
atgtccatcg agacctggcc gctcggaaca tcctggtcaa cagtaacctg gtctgcaagg 2760
tgtccgactt tggcctctcc agattcctgg aggagaactc ctctgatccc acctacacaa 2820
gttccctggg aggaaagatt cccatccgat ggaccgcccc tgaggccatt gccttcagga 2880
aattcacgtc tgccagtgat gcctggagct atgggatcgt catgtgggag gtcatgtctt 2940

tcggggaacg gccatactgg gacatgagca accaggacgt gatcaatgct attgaacagg 3000
actaccggct gccccctccc ccagattgcc ccacctccct ccaccagctc atgctggact 3060
gttggcagaa ggaccggaat gcccggcccc gattccccca ggtggtcagc gctctggaca 3120
agatgatccg gaatcccgct agcctcaaaa tcgtggccag ggagaatggc ggggcctcac 3180
atccactcct ggaccaacgg cagcctcact actctgcttt tggttctgtg ggcgagtggc 3240
ttcgagccat caagatggga agatacgagg aaagttttgc agcggctgga ttcggctcct 3300
ttgaggtggt cagccagatc tctgccgagg accttctccg aattggagtc actctggcag 3360
gacaccagaa gaaaatctta gccagtgtgc agcatatgaa gtcccaagct aagccaggag 3420
cccccggtgg gacaggagga ccaacccagc agttctgacc tccaagggct caccacccca 3480
gagctgaggg gggcagagtt gggtggggac tcacaagatg agcccctccc cctcatcaca 3540
gcttcccatt ggattgcact ttgaacagaa ggggtaggag ctgttaatcc tggagaccca 3600
gccttgagga aactgtgcca tatgggatgg gaagaatcac acgggtgccc tccaggcggg 3660
aaaccccaaa ctcaacagtg agtctttcct tcaagactgg gcaaagaaca tccctacgtc 3720
tctaatctcc catcttccca gagggctctc tcctcaagcg tcttccacct caacgggcat 3780
gtccctgcag accaaagaga aaaggtgacc agcctgccaa cttgggagtg gaaaatgccg 3840
tcccagaagg caggacgggg ctgtcaggac tcggtgatgt aatcattgga ttctgatgtc 3900
ccaacttgct gtcaccacca aaggcaatca ttttcccttg taaatgctcc ttccctcatc 3960
tgccttcata ttgaaggttc tggagtttta ctatttttta ttttgttatt ttttttcctc 4020
cttctcttcc ctccccaccc cccttgtcca gattttgtgc gttgaagggt acctggttcg 4080
actatctcct gttgggaaca aggacccgtt gatatgttct agaacagtgc ctttgaaatg 4140
ccatagcctc agacagttag ttccttcctc cttccctccc cctaagtgcc cccaagctgt 4200
gttgtgagtg gagggaaggg ggggtgatgt tgtaaaaggg gcaggagggt ggtggcggtc 4260
aacaggaggc agacaccggt gcttggaagg gggttcttaa attatattta aaaagaaact 4320
tttttttttt gtataaataa aagaaaacgg gatctgtctc agta 4364

<210> 85
<211> 4059
<212> DNA
<213> human

<400> 85
ggggcgggcc gggccggctt gggaggggac gcggaggggg cgggccgggc tgcgttcgct 60
ccagccgcgg ctctacagca gcgggcggcg ggacccggga cccagcttgg cgacggcgat 120
ctcgacgcgg gcccccagga tctcccggcg ccccacctct ggagcagccc ctgccgccag 180
cgtcaggtcc accccggaat cccagggact ctcggcgccg aacggacccg ggccgggtgc 240
aacggggtcc ccggactgga gaagacgcgg gtggcaccgt gcgagctcca ggagccccgg 300
gtccactgcg aggcctcggg gggcgcagac ctgcagagac tgcggccaac gggaagaaat 360
aaagggatta tagtccaccc aattcacaga cttctgagac tcagacacga ggagagatag 420

agaaccgcca atctctagat caacaagcaa aggaggtgcc aagcctgttt gtcttcattg 480
tgacactgga gtctagatgc tgggaagtcc aagatcaggg tgccggcatg gtcagttcct 540
ggcgaagcct ctcttctagg tttcagactg ccctcttctt tgttgtgtcc tcgaatggca 600
gaaaaagggg tggctgttgg aggaagggag gagagtaaat gaagagaaag aactggaata 660
accccttgca gaaaaaaaaa aaaagggaag caagcttagc tgtacaccct gagtcttgca 720
aaagctgcag ccccacccag gagcagggtg gtggctgggg cgatggtgga cgccctgaag 780
atgtcccatg gctactgaag ggctgcccca gttagggaac agagtggcgg gcatggtgtg 840
tagcctatgg gtgctgctcc tggtgtcttc agttctggct ctggaagagg tattgctgga 900
caccaccgga gagacatctg agattggctg gctcacctac ccaccagggg ggtgggacga 960
ggtgagtgtt ctggacgacc agcgacgcct gactcggacc tttgaggcat gtcatgtggc 1020
aggggcccct ccaggcaccg ggcaggacaa ttggttgcag acacactttg tggagcggcg 1080
cggggcccag agggcgcaca ttcgactcca cttctctgtg cgggcatgct ccagcctggg 1140
tgtgagcggc ggcacctgcc gggagacctt caccctttac taccgtcagg ctgaggagcc 1200
cgacagccct gacagcgttt cctcctggca cctcaaacgc tggaccaagg tggacacaat 1260
tgcagcagac gagagctttc cctcctcctc ctcctcctcc tcctcttctt cctctgcagc 1320
gtgggctgtg ggaccccacg gggctgggca gcgggctgga ctgcaactga acgtcaaaga 1380
gcggagcttt gggcctctca cccaacgcgg cttctacgtg gccttccagg acacgggggc 1440
ctgcctggcc ctggtcgctg tcaggctctt ctcctacacc tgccctgccg tgctccgatc 1500
ctttgcttcc tttccagaga cgcaggccag tggggctggg ggggcctccc tggtggcagc 1560
tgtgggcacc tgtgtggctc atgcagagcc agaggaggat ggagtagggg gccaggcagg 1620
aggcagcccc cccaggctgc actgcaacgg ggagggcaag tggatggtag ctgtcggggg 1680
ctgccgctgc cagcctggat accaaccagc acgaggagac aaggcctgcc aagcctgccc 1740
acgggggctc tataagtctt ctgctgggaa tgctccctgc tcaccatgcc ctgcccgcag 1800
tcacgctccc aacccagcag cccccgtttg cccctgcctg gagggcttct accgggccag 1860
ttccgaccca ccagaggccc cctgcactgg tcctccatcg gctccccagg agctttggtt 1920
tgaggtgcaa ggctcagcac tcatgctaca ctggcgcctg cctcgggagc tggggggtcg 1980
aggggacctg ctcttcaatg tcgtgtgcaa ggagtgtgaa ggccgccagg aacctgccag 2040
cggtggtggg ggcacttgtc accgctgcag ggatgaggtc cacttcgacc ctcgccagag 2100
aggcctgact gagagccgag tgttagtggg gggactccgg gcacacgtac cctacatctt 2160
agaggtgcag gctgttaatg gggtgtctga gctcagccct gaccctcctc aggctgcagc 2220
catcaatgtc agcaccagcc atgaagtgcc ctctgctgtc cctgtggtgc accaggtgag 2280
ccgggcatcc aacagcatca cggtgtcctg gccgcagccc gaccagacca atgggaacat 2340
cctggactat cagctccgct actatgacca ggcagaagac gaatcccact ccttcaccct 2400
gaccagcgag accaacactg ccaccgtgac acagctgagc cctggccaca tctatggttt 2460

ccaggtgcgg gcccggactg ctgccggcca cggcccctac gggggcaaag tctatttcca 2520
gacacttcct caaggggagc tgtcttccca gcttccggaa agactctcct tggtgatcgg 2580
ctccatcctg ggggctttgg ccttcctcct gctggcagcc atcaccgtgc tggcggtcgt 2640
cttccagcgg aagcggcgtg ggactggcta cacggagcag ctgcagcaat acagcagccc 2700
aggactcggg gtgaagtatt acatcgaccc ctccacctac gaggacccct gtcaggccat 2760
ccgagaactt gcccgggaag tcgatcctgc ttatatcaag attgaggagg tcattgggac 2820
aggctctttt ggagaagtgc gccagggccg cctgcagcca cggggacgga gggagcagac 2880
tgtggccatc caggccctgt gggccggggg cgccgaaagc ctgcagatga ccttcctggg 2940
ccgggccgca gtgctgggtc agttccagca ccccaacatc ctgcggctgg agggcgtggt 3000
caccaagagc cgacccctca tggtgctgac ggagttcatg gagcttggcc ccctggacag 3060
cttcctcagg cagcgggagg gccagttcag cagcctgcag ctggtggcca tgcagcgggg 3120
agtggctgct gccatgcagt acctgtccag ctttgccttc gtccatcgct cgctgtctgc 3180
ccacagcgtg ctggtgaata gccacttggt gtgcaaggtg gcccgtcttg gccacagtcc 3240
tcagggccca agttgtttgc ttcgctgggc agccccagag gtcattgcac atggaaagca 3300
tacaacatcc agtgatgtct ggagctttgg gatactcatg tgggaagtga tgagttatgg 3360
agaacggcct tactgggaca tgagtgagca ggaggtacta aatgcaatag agcaggagtt 3420
ccggctgccc ccgcctccag gctgtcctcc tggattacat ctacttatgt tggacacttg 3480
gcagaaggac cgtgcccggc ggcctcattt tgaccagctg gtggctgcat ttgacaagat 3540
gatccgcaag ccagataccc tgcaggctgg cggggaccca ggggaaaggc cttcccaggc 3600
ccttctgacc cctgtggccc tggactttcc ttgtctggac tcaccccagg cctggctttc 3660
agccattgga ctggagtgct accaggacaa cttctccaag tttggcctct gtaccttcag 3720
tgatgtggct cagctcagcc tagaagacct gcctgccctg ggcatcaccc tggctggcca 3780
ccagaagaag ctgctgcacc acatccagct ccttcagcaa cacctgaggc agcagggctc 3840
agtggaggtc tgagaatgac gatacccgtg actcagccct ggacactggt ccgagaaggg 3900
acatgtggga cgtgagccgg gctccaacag cctctgtgag agatgcccca caccaaaccc 3960
aaccctcccg atggctgcat tccctggtcc tccgcctctc caccagcccc ctcctcatta 4020
aagggaaaga agggaatttg caaaaaaaaa aaaaaaaaa 4059

<210> 86
<211> 3666
<212> DNA
<213> mouse

<400> 86
gagcgactcc gatcgtgaca ccggcccccg cacgattccc cagggccccg cctagggagc 60
agtccctgct tgcagccttg ggtcctctcc cagaatccca gagactctaa gctgcgagca 120
gacgcgggcc cctgggaagc gaggtcccca gaccagagag gacacgcgcg acgctaggaa 180

agatctgcga ggtgcagatc caccgcgggg gcctcggggg gcgcagacct gcagagctgg 240
aggccggcgg gaaggttggc tgctggagga acggtgcaga gtaaatgaag agagcaaact 300
ggaataactc ctaccagaca agaaggcaag cctggctgta gacaccaggt cttctgaaag 360
ccatagcagt gcctcagaac agggcagtgg ccgaggcaat ggtgggagtg ccgaagagac 420
cgcatggcta ctgagggcac gactggctca gggagcagag tggtggcggg catggtgtgc 480
agtctgtggc tcctggttct agggtcatca gttctagctc tggaagaggt gctgctggat 540
accacaggag agacctctga gattggctgg ctcacctacc cgccaggtgg gtgggatgag 600
gtgagtgttc tagatgacca gcgccgtctg actcggacct tcgaagcatg ccacgttgca 660
gggctccctc ctggttctgg ccaggacaat tggctgcaga cacactttgt ggaacggcga 720
ggggcccaga gggcacacat ccgccttcac ttctctgtac gagcctgttc cagcctgggt 780
gtgagtgggg gtacctgccg agagaccttc accctttact accggcaggc tgatgagcca 840
gacggccccg acagtattgc ggcctggcac ctcaaacgct ggaccaaagt ggacacaatt 900
gccgcagatg agagcttccc tgcctcctct tcctcttcct catgggctgt gggaccccac 960
aggaccggtc agcgggtggg actgcagctg aatgtcaaag agcgcagctt tggtcctctc 1020
actcagcgtg gcttctatgt ggccttccag gacacagggg cctgcctggc ccttgtggca 1080
gtcaagctct tctcctacac ctgtccctcc gtcctgcgtg cctttgcctc ttttcctgag 1140
acacaggcca gtggagctgg aggggcttcc ttagtggcag ctgtgggcac ctgtgtagct 1200
catgcagaac cagaggagga tggagtagga ggccaggcag ggggcagccc cccaaggtta 1260
cactgtaatg gcgaaggcag gtggatggta gctgtgggag gctgccgctg ccagcctgga 1320
caccagcctg ctcgtggaga caagctctgc caagcctgcc ctgagggatc ctataaggcc 1380
ctggctggga atgttccctg ttcaccgtgt ccagcccgca gccactcccc tgaccctgca 1440
gccccagttt gcccatgtct ccaaggcttc tacagggcca gttcggaccc accagaggct 1500
ccctgcactg gtcctccatc ggctccccgg gagctatggt ttgaggtgca aggctcggca 1560
ctcatgctgc actggcgtct gcctcaggag ctgggtggac gaggggatct gctcttcaat 1620
gttgtgtgca aggagtgtgg aggtcacgga gagcccagca gtggggggat gtgtcgccgc 1680
tgcagggacg aggtgcattt cgacccccgc cagaggggac tgactgagag tcgagtgtta 1740
gttggggggc tccgagcaca cgtgccatac atcttggaag tgcaggctgt caatggggtg 1800
tctgagctca gccctgaccc tccccaggca gcagccatca atgtcagcac cagccacgaa 1860
gtcccttctg cagtccctgt gatgcaccag gtgagccggg cagccaacag catcacagta 1920
tcctggccac agcctgagca aacaaatggg aacatcctcg actaccagct gcgctactat 1980
gaccaggcag aagatgaatc ctactccttc accatgacca gtgagaccaa cactgccact 2040
gtgacacggt tgagccctgg ccacatctat ggcttccagg tgcgggcacg gactgcagct 2100
ggccacggcc cctatggggg caaagtctat tttcagacat tgcctcaagg tgagctgtcc 2160
tcccagcttc cagagaagct gtccttggtg attggttcca tcctgggggc cttggccttc 2220

cttctactgg cagccatcac tgtactggct gtcatcttcc agcggaagcg ccgtgggacc 2280
ggctacacag aacaactgca acaatacagc agcccaggac ttggagtgaa gtattacatt 2340
gacccttcta catacgatga cccctgccag gccatcagag agcttgctcg agaggttgac 2400
cctacatata tcaagattga agaagtcatt ggggcaggtt cctttggaga agtgcgtcgg 2460
ggccgactgc aaccccgggg aaggagggaa caagctgtgg ctatccaggc cttgtgggca 2520
gggggtgctg agagcctgaa gatgaccttt ctgggtcggg cagcactgct gggccagttc 2580
cagcacccca atatcctgcg gctggaaggt gtagtcacta agagtcggcc tgtcatggtg 2640
ctgacagaac tcatggaact tggtcccctg gacagctttc ttaggcagcg agagggccag 2700
ttcagtagcc tgcagctggt ggccatgcag cggggtgtgg ctgctgccat gcagtacttg 2760
tccagctttg cctttgttca ccgagcactc tcggcccgaa gtgtgctagt gaatagccac 2820
ctggtgtgta aggtggcacg tctcggccac agtcctcagg gttcaagttc cttgcttcgc 2880
tgggcagccc cagaggtcat tacacatgga aagtatacaa catctagcga tgtctggagc 2940
tttggcatac tcatgtggga agtgatgagc tatggagaac ggccctactg ggacatgaac 3000
gagcaggagg tactaaatgc aatagagcaa gagttccggc tgcccccacc tccagggtgc 3060
ccccctggac tacatctact gatgctagac acttggcaga aggaccgtgc ccgtcggcct 3120
cattttgacc agctggtagc tgcatttgac aagatgattc gcaagcctga tactctccag 3180
gctgaagggg gctccgggga caggccttcc caggctcttc tgaaccctgt ggccttggac 3240
ttcccttgcc tggactctcc ccaggcctgg ctttcagcca ttggactaga gtgctaccag 3300
gacaacttct ccaagtttgg tctttccacc ttcagtgatg tggctcagct cagcctggaa 3360
gacctgccag gcctgggcat caccctagct ggccaccaga agaaacttct gcacaacatt 3420
cagctcctcc agcagcacct gaggcagcca ggctcagtgg aagtgtaaag cctgctcagg 3480
agtctgtgag ccgccgatgc cccaacccag tcctggccat agggtggaga aggggcgtgt 3540
gagactgagt caggctctac tccagcttcc atgcctgtca cattaaaccc agccctctgg 3600
acattgcacc cccaaatcct gctcttccaa cagcctcccc catattaaag ggaaagaagg 3660
gaattt 3666

<210> 87
<211> 4067
<212> DNA
<213> rat

<400> 87
gatgctccgg cgcgagctac aggattttaa acacgccttg cagcggcgcc cactggccac 60
ctcctcccgg accctaactc gtgcagtcct tctgcccacg tcccctccgc ccgccaggac 120
ccacgggagg aggccgaccc tcctcagcac acaggcgggc ccagggcgcg cccagcatct 180
ccactgcacg ccccgctggg gatcgggcgc cgcgcacttg ggtcctaggc ccagagggcg 240
ggctcggctc ccggaagagg agagccaggg cggcagaagg ggccggagag gggcgggcca 300
ggctgggagg gacgagaggg ggcgggcccg gctgcgtttg cccagccagc gcttcggagc 360

agcgggcgtc cgaacccaag agcagagctc agcgactccg atcgtgacac cggcccccgc 420
acgattcccc agggccccgc ctagggagca gtccctgctt gcagcctcgg gtcctctccc 480
agaatcccag agactccaag ctgcgagcag acgcgggccg ctgggaagcg aggtcctcag 540
accagagagg acacgcgcga cgctaggaaa gatctgcgag gtgcagatcc accgcggggg 600
cctcgggggc gcagacctgc agagctggag gccggcggga aggttggctg ctggaggaca 660
ggtgcagagt aaatgaagag ctcaaactgg aataactcct tccagacaca aagggaaaca 720
agcttgtctg tagacaacgg gtcttccgaa aaccacagct gcgcctcaga acagggcagt 780
ggccgaggca atggtgggag tgccgaagac accgcatggc ttctgagaac acggctgggt 840
cagggagcag agtggcgggc atggtgtaca gtctatggct cctggttctg gggccctcag 900
ttctagctct ggaagaggtg ctgctggata ccacaggaga gacctctgag attggctggc 960
tcacctaccc accaggtggg tgggatgagg tgagtgttct agacgaccag cgccgtctga 1020
ctcggacctt cgaagcatgc cacgtcgcag ggctccctcc cggttctggg caggacaatt 1080
ggctacagac acactttgtg gagcggcgag gggcccagag ggcacacatc cgtcttcact 1140
tctctgtgcg agcctgttcc agcctgggcg tgagtggagg tacctgccga gagaccttca 1200
ccctttacta ccggcaggct gatgaaccag acagccctga cagtatttcg gcctggcacc 1260
tcaaacgctg gaccaaagtg gacacaatcg cagcagatga gagcttccct gcctcctctt 1320
cctcttcctc ctgggctgtg ggaccccaca ggactgatca gcgggtggga ctgcagctga 1380
atgtcaaaga gcgcagtttt ggtcctctca ctcagcgtgg cttctacgtg gccttccagg 1440
acacaggggc ctgcctggcc cttgtagcag tcaagctctt ctcctatacc tgtccctccg 1500
tcctgcgcgc ctttgcctct tttcctgaga cacaggccag cggagctgga ggtgcctcct 1560
tagtagctgc tgtgggcacc tgtgtagctc atgcggaacc cgaggaggat ggcgtaggag 1620
gccaggcagg gggcagccca ccaaggttac actgtaatgg ggaaggaagg tggatggtag 1680
ctgtgggagg ctgtcgctgc cagcctggac accagcctgc tcgtggagac aagctctgcc 1740
aagcctgccc tgaggggtcc tataaggcac tgcctggaaa tgtcccctgc tcaccgtgtc 1800
cagcccgcag ccactcccct gaccctgcag ccccagtttg tccctgtctc caaggtttct 1860
acagggccag ttctgaccca ccagaggctc cctgcactgg tcctccatca gctccccggg 1920
agctttggtt tgaggttcaa ggctcagcac ttatgctgca ctggcgcctg cctcaggagc 1980
tgggcggacg aggggacctg ctcttcaatg ttgtgtgcaa ggaatgtgga ggtcacaagg 2040
agcccagcag tgggggaatg tgtcgccgct gcagggatga ggtgcatttc gacccccgcc 2100
agaggggcct gaccgagagt cgagtgttag ttggtgggct ccgagcacac gtgccataca 2160
tcttggaagt gcaggctgtc aatggggtgt ctgagctcag ccctgaccct ccccaggcag 2220
cagccatcaa tgtcagcacc agccatgaag ttccttctgc agtccctgtg atgcaccagg 2280
tgagccgggc cgccaacagc atcacagtgt cctggccaca gcctgaacaa acaaatggaa 2340
acatccttga ctatcagctg cgctactatg accaggcaga agatgaatcc cactccttca 2400

ccatgaccag tgagactaac actgccactg tgacacggtt gagccctggc cacatttatg 2460
gcttccaggt tcgagcacgg actgcggctg gccatggccc ctatgggggc aaagtctatt 2520
ttcagacact gcctcaaggt gagctgtcct cccagcttcc agagaagctg tccttggtga 2580
ttggttccat cctgggggcc ttggccttcc ttctactggc agccatcact gtactggctg 2640
tcatcttcca gcggaagcgg cgtgggaccg gctacacaga acaactgcaa cagtacagca 2700
gcccaggact tggagtgaag tattacattg acccttccac gtatgacgac ccctgccagg 2760
ccatcagaga gcttgctcga gaggttgacc ctacatatat caagattgag gaagtcattg 2820
gggcaggttc ctttggagaa gtgcgtcggg gccgactgca accccgggga aggcgggaac 2880
aggctgtggc tatccaggcc ctgtgggcag ggggtgctga gagcctgaag atgacctttc 2940
tgggtcgggc agccctgctg ggccagttcc agcaccccaa tatcctgcgc ctggaaggtg 3000
tagtcactag gagtcggcct gtcatggtgc tgacagaact catggagctt ggtcccctgg 3060
acagcttcct taggcagcga gagggccagt tcagtagcct gcagctggtg gccatgcagc 3120
ggggtgtggc tgctgccatg cagtacctgt ccagctttgc ctttgttcac cgagcactct 3180
cagcccgaag tgtgctagtg aatagccacc tggtgtgtaa ggtggcccgt cttggccaca 3240
gtcctcaggg ttcaagttcc ttgcttcgct gggcagcccc agaggtcatc acacacggga 3300
agtacacaac atctagcgat gtctggagct ttggcatact catgtgggaa gtgatgagct 3360
atggagaacg gccctactgg gacatgagtg accaggaggt actaaatgcg atagagcaag 3420
agttccggct gcccccacct ccggggtgcc ctactggact acatctgctg atgctagaca 3480
cttggcagaa ggaccgtgcc cgtcggcctc actttgacca gctcgtagcc gcgtttgaca 3540
agatgatacg caagcctgat accctccagg ctgaagggag ctccggggac aggccttccc 3600
aggctcttct gaaccctgtg gccttggact ttccttgcct ggactctccc caggcctggc 3660
tttcagccat tggactagag tgctaccagg acaacttctc taagtttggt ctttccacct 3720
tcagtgatgt ggctcagctc agccttgaag acctgccagg cctgggcatc accctagctg 3780
gccaccagaa gaaactactg cataacatcc agctcctcca gcagcacctg aggcagccag 3840
gctcggtgga agtgtaaagc ctggtcagca atctgtgagc tgccgctgat gccccaaccc 3900
agccctggcc atagggtgga ggaggggcgt gtgagactga gccagactct attccagcct 3960
ccatgcctgc cacattaaac ccagccctct ggacattgca cccccaaatc ctgctcttcc 4020
aacagcctcc cccatattaa agggaaagaa gggaatttat aagttga 4067

<210> 88
<211> 1474
<212> DNA
<213> mouse

<400> 88
gagatttgtg atcgtggtgc ctactgtacc gtggagaagc ctgtgggaac cttacactgc 60
agagctctcg tcctggcctg gctcggcccg gccccgcgct atggagttcc tttgggcccc 120

tctcttgggt ctgtgctgca gtctggccgc tgctgaccgc cacatcgtct tctggaacag 180
ttcaaatccc aagttccgtg aggaggacta cacggtgcac gtgcagctga atgactacct 240
agacatcatc tgcccacatt acgaggacga ctctgtggca gatgcagcca tggagcgata 300
cacactgtac atggtggaac accaggagta tgtggcatgc caaccccagt ccaaggacca 360
ggtccgttgg aattgcaacc ggcccagtgc caagcatggc ccggagaagc tgtctgtgaa 420
attccagcgc ttcacgcctt ttatcttggg caaggagttc aaggaaggac acagctacta 480
ctacatctcc aaacctatct accatcagga atcccagtgc ttgaagctga aggtgactgt 540
caatggcaaa atcactcata atccccaggc ccatgtcaac ccacaggaga agagactcca 600
agcagatgac ccggaagtac aggttttgca cagcattggt tacagtgccg ccccccgcct 660
cttcccactg gtctgggcag tattgctcct accactgctg ctgctgcaat ctcagtgagg 720
gtgtacgctt gccctggctt atggattgga atgggactaa ggggcagccc cagccctggg 780
aacctctccc actacaccca caagacgcca ccatgaagcc tcaaaaggtt cagtattaag 840
ggttttaacc gaaaagagtt taaccagccc aactgtgcca tccctgcctc cacttcagag 900
ggatggagaa agaagtggag aaggtcctta acctgcagtt tctgccttta agccaaagaa 960
acaagctgtg cggacctggc ccattaagag gcctcagtgg gagaagggct aaaaagagcc 1020
tgaggtcatg cggttggaca ctgaacttgg taaagacgtc cttccccaag gaagccataa 1080
tgtgcagatg aactgaccga aggaaaagct tgagacagct tcctgcgggg gagccaggta 1140
cagaagaggc agcttgggct gacccagcat ctctgcaaga tttccacctg ctgggctgcc 1200
agagaggttt gtagcccggc cctgactgca tcctccccat ccctggggca acattccctg 1260
gagctgtgcc aacaggagga ctgaagcagc ctggttttag agtgtagctg taagggcagt 1320
gcccgtgtgt acagtctgtg gagtttagct taacgggcag ggcccacatg tacagtgtct 1380
gtatataaat gtgctgtatc tgttcatttc tatgactgca gggttttttt atacagtgtt 1440
tttggattca taataaattg atggtttttt atgg 1474

<210> 89
<211> 1590
<212> DNA
<213> human

<400> 89
gccagatctg tgagcccagc gctgactgcg ccgcggagaa agccagtggg aacccagacc 60
cataggagac ccgcgtcccc gctcggcctg gccaggcccc gcgctatgga gttcctctgg 120
gcccctctct tgggtctgtg ctgcagtctg gccgctgctg atcgccacac cgtcttctgg 180
aacagttcaa atcccaagtt ccggaatgag gactacacca tacatgtgca gctgaatgac 240
tacgtggaca tcatctgtcc gcactatgaa gatcactctg tggcagacgc tgccatggag 300
cagtacatac tgtacctggt ggagcatgag gagtaccagc tgtgccagcc ccagtccaag 360
gaccaagtcc gctggcagtg caaccggccc agtgccaagc atggcccgga gaagctgtct 420
gagaagttcc agcgcttcac acctttcacc ctgggcaagg agttcaaaga aggacacagc 480

tactactaca tctccaaacc catccaccag catgaagacc gctgcttgag gttgaaggtg 540
actgtcagtg gcaaaatcac tcacagtcct caggcccatg acaatccaca ggagaagaga 600
cttgcagcag atgacccaga ggtgcgggtt ctacatagca tcggtcacag tgctgcccca 660
cgcctcttcc cacttgcctg gactgtgctg ctccttccac ttctgctgct gcaaaccccg 720
tgaaggtgta tgccacacct ggccttaaag agggacaggc tgaagagagg gacaggcact 780
ccaaacctgt cttggggcca ctttcagagc ccccagccct gggaaccact cccaccacag 840
gcataagcta tcacctagca gcctcaaaac gggtcagtat taaggttttc aaccggaagg 900
aggccaacca gcccgacagt gccatcccca ccttcacctc ggagggatgg agaaagaagt 960
ggagacagtc ctttcccacc attcctgcct ttaagccaaa gaaacaagct gtgcaggcat 1020
ggtcccttaa ggcacagtgg gagctgagct ggaaggggcc acgtggatgg gcaaagcttg 1080
tcaaagatgc cccctccagg agagagccag gatgcccaga tgaactgact gaaggaaaag 1140
caagaaacag tttcttgctt ggaagccagg tacaggagag gcagcatgct tgggctgacc 1200
cagcatctcc cagcaagacc tcatctgtgg agctgccaca gagaagtttg tagccaggta 1260
ctgcattctc tcccatcctg gggcagcact ccccagagct gtgccagcag gggggctgtg 1320
ccaacctgtt cttagagtgt agctgtaagg gcagtgccca tgtgtacatt ctgcctagag 1380
tgtagcctaa agggcagggc ccacgtgtat agtatctgta tataagttgc tgtgtgtctg 1440
tcctgatttc tacaactgga gtttttttat acaatgttct ttgtctcaaa ataaagcaat 1500
gtgttttttc ggacatgctt ttctgccact ccatattaaa acatatgacc attgagtccc 1560
tgctaaaaaa aaaaaaaaaa aaaaaaaaaa 1590

<210> 90
<211> 1485
<212> DNA
<213> rat

<400> 90
gccagtggga accttacact gcggagcttt cgtcctggcc tggcccggcc cggccccgcg 60
ctatggagtt cctttgggcc cctctcttgg gtctgtgctg cagtctggcc gctgctgacc 120
gccacatcgt cttctggaac agttcaaatc ccaagttccg agaggaggac tacaccgtgc 180
acgtgcagct gaatgactat ctggacatca tctgcccaca ttacgaggac gactctgtgg 240
cagatgctgc catggagaga tacacgctgt acatggtgga acaccaggag tatgtgacat 300
gcgagcccca gtccaaggac caggttcgct ggaagtgcaa ccagcccagt gccaagcacg 360
gcccggagaa gctgtctgag aaattccagc gcttcacgcc tttcaccttg ggcaaggagt 420
tcaaggaagg acacagctac tactacatct ccaaacctat ctaccatcag gaaacccagt 480
gcctgaagtt gaaggtgact gtcaatggca aaatcactca cagcccccac gcccatgcca 540
acccacagga gaagagactc caagcagatg acccggaagt gcaggttttg cacagcattg 600
gtcacagcgc cgccccccgc ctcttcccat tggtctgggc agtattgctc ctaccactgc 660

tgctgctgca aactcagtga aggtatttgc ttgccctggc ttacggattg gaacgggact 720
aaggggcagg gttaagcacc cccaaagccc cagccctggg aacctctccc actacacgca 780
caagacgcca ccacgaagcc tcagaaaggt tcagtattaa gggttttaac cgaaaggagt 840
tcaaccagcc caactgggcc atccctgcct ccacttcaga gggatggaga aagaagtgga 900
gaaggtcctt tccctgtggt ttctaccttt aagccaaaga aacaagctgt gcggacctgg 960
cccattaaga gcacccactg ggagatgcgc tgaaagagcc tgaggccatg cagttggaca 1020
ctgaacttgg taaaaatgtc cttccccaag gaagccataa tgtgcagatg aactgaccaa 1080
aggaaaagct tgagacagct tcctgcgggg aagccaggta caagagagac agcttgggct 1140
gacccagcat ctctgcaaga tttccacctg ctgggctgcc agagagtttt gtagcccggc 1200
actgactgca tcctcccctt ccctggggca gcattccctg aagctgtgcc agcaggagga 1260
ctatagcagc ctggttttgg agtatagctg taagggcagt gcccatgtgt atagtctgtg 1320
gagtttagct taaagggcag ggcccacatg tacagtgtct gtatataaac gtgctgtctg 1380
ttcatttcta cgactggagg ttttttatac agtgttcttg gattcataat aaagtgatgt 1440
ttttggtttt ttaaaaaaaa aaaaaaaaaa aaaaaaaaaa aaaaa 1485

<210> 91
<211> 1766
<212> DNA
<213> mouse

<400> 91
ggggagcccc cgcgcccggg gaacccggga gagcgccttg cgcagtgcac cgctcgggcc 60
tctgccccgc gccgccgtcg ccgccgccgc cgccgccgcc gccgccgcct ggtttctcgg 120
cgcccgcggt ctacggcggg ggccgcgcgc agaggcgggg cggcagatct ccaagcgcgc 180
gcggcgctcc gcccgctcgg cggcggaggc ggaggcggag acggcggggc caggcggcgg 240
ctggagagcg agggagcgag cgcggcggca gcaccggggc catggcgccc gcgcagcgcc 300
cgctgctgcc gctgctgctg ctgctgctgc cgctgcgtgc gcgcaacgag gacccggccc 360
gggccaacgc tgaccgatac gcagtctact ggaaccgtag caaccccagg tttcaggtga 420
gcgctgtggg tgatggcggc ggctataccg tggaggtgag catcaacgac tacctggata 480
tctactgccc acactacggg gcgccgctgc ccccggctga gcgcatggag cggtacatcc 540
tgtacatggt gaatggtgag ggccacgcct cctgtgacca ccggcagcga ggcttcaagc 600
gctgggaatg caaccggccc gcagcgcccg ggggacccct caagttctca gagaagttcc 660
aactcttcac ccccttttcc ctgggctttg agttccggcc tggccacgaa tactactaca 720
tctctgccac acctcccaac ctcgtggacc gaccctgcca gcgactcaag gtttatgtgc 780
gtccaaccaa tgagaccctg tatgaggctc cagagcccat cttcaccagt aacagctcct 840
gcagcggcct gggtggctgc cacctcttcc tcaccaccgt ccctgtgctg tggtcccttc 900
tgggctccta gtgtcaggcc ggagaacacc agccccacct ggaccccgtg acctttgccc 960
tctgacctgc cacggccacc tccgagacaa aatccttgct gcttctcttt catggtgctg 1020

tcccgccgga ggaggccatc catccgtccc tgggatgcaa catggggtcc caatgcctga 1080

ggagaagacc cccccccaag gctgactcgc tttcaccagg gccaccaggg ccatccagtg 1140

ttgtttaatt acagtcggaa agacttaagg tttttctttt aatttaattt attccctgac 1200

attgctggtg acactgggaa gggaacaagc cacagggatg aggtgaagcc atctctgtcc 1260

ttcctggaat accggagatc caggggcctc cagctgctcc tttcttctgt gtcctgttat 1320

ttgggtccca gatggagccc accgcggact tgccttgcat tcctcaggcc aggcaagcct 1380

gagccagaaa gggcgcacgg tgccagcccc tctcggggac tctgggggtg ccatccccca 1440

ctcttcttcc agccactctc gggccccact cccacatcat ctcagaaacc cttcagccct 1500

cgcaactcgc ccctccgggc ccccccacca ggcacaacca tccccggggc cagccgggac 1560

gttgtcggtt tatttctgta aatagaaacc agcaagtgta tactgtgatt tattttaatg 1620

tattcttaag gacagaatgg aaattcttta aaaaaaattt tttttccgac cttcaattca 1680

aggggtcatt tattttggtg gggggagtgg ggtggacttt ttaggataga agcaacactt 1740

tgcaataaac tcattttttt ttgttc 1766

<210> 92
<211> 642
<212> DNA
<213> human

<400> 92
atggcgcccg cgcagcgccc gctgctcccg ctgctgctcc tgctgttacc gctgccgccg 60

ccgcccttcg cgcgcgccga ggacgccgcc cgcgccaact cggaccgcta cgccgtctac 120

tggaaccgca gcaaccccag gttccacgca ggcgcggggg acgacggcgg gggctacacg 180

gtggaggtga gcatcaatga ctacctggac atctactgcc cgcactatgg ggcgccgctg 240

ccgccggccg agcgcatgga gcactacgtg ctgtacatgg tcaacggcga gggccacgcc 300

tcctgcgacc accgccagcg cggcttcaag cgctgggagt gcaaccggcc cgcggcgccc 360

ggggggccgc tcaagttctc ggagaagttc cagctcttca cgcccttctc cctgggcttc 420

gagttccggc ccggccacga gtattactac atctctgcca cgcctcccaa tgctgtggac 480

cggccctgcc tgcgactgaa ggtgtacgtg cggccgacca acgagaccct gtacgaggct 540

cctgagccca tcttcaccag caataactcg tgtagcagcc cgggcggctg ccgcctcttc 600

ctcagcacca tccccgtgct ctggaccctc ctgggttcct ag 642

<210> 93
<211> 1718
<212> DNA
<213> rat

<220>
<221> misc_feature
<222> (1)..(1)
<223> n is a, c, g, or t

<400> 93
ngcccgctgc tgccgctgct gctgttgctg ctgccgctgc gtgcgcgcaa cgaggacccg 60

gcccgtgcca acgctgaccg ctacgcagtc tactggaacc gcagcaaccc caggtttcag 120

gtgagcgctg tgggtgatgg cggcggctac accgtggagg tcagcatcaa tgactacctg 180

gacatctatt gcccgcacta tggggcgcca ctgcccccgg cagagcgcat ggaacggtac 240

atcctataca tggtgaacgg cgagggccat gcctcctgtg accaccggca gcgaggcttc 300

aagcgctggg aatgcaaccg gcccgctgcg cctgggggac ccctcaaatt ctccgagaag 360

ttccaactct tcaccccctt ttccctgggc tttgagttcc gacctggaca cgagtactac 420

tacatctctg ccacaccccc caaccttgtg gaccgaccct gcctgcggct gaaagtttac 480

gtgcgtccaa ccaatgagac cctgtatgag gccccagagc ccatcttcac cagtaacagc 540

tcctgcagcg gcctgggtgg ctgccacctc ttcctcacta cagtccctgt gctgtggtcc 600

ctactgggct cctagtgtcc agccccacct ggaccccttg accttcgccc tctgacctgc 660

cacggccacc tccgagacaa aatccttgct gcttctcttt catggtgctg tccctccgga 720

ggaggccatc gaccggtccc tgggatgcaa catggggtcc cagtgcctga gactgagatc 780

caggctgagc cacttttgcc aggaccacca gggccgtcca gtgttgtttg attacggtca 840

gaaagactta gggtttctct tttaatttaa tttattccct gatattgctg gtgacattgg 900

gaaggaaaca agtcacaggg gtgaggtgga gccgtccctg gtcttccagt cggtcctttc 960

ttctgtgtcc tgttatttgg gtcccaggtg gaccccactg aggacttgcc ttgggttcct 1020

caggccaggc aagcctgagc cagaaagggt gcatggtgcc agcccctcat ggggactctg 1080

ggggtgtcat cccccactct tctagccact gtcgagcccc ccctgggtcc ccttcaccag 1140

gcacaaccat ctccagggcc gtccgggaag tcgttttatt tctgtaaata gaaaccagca 1200

agtgtatact gtgatttatt ttaatgtatt cttaaggaca gaatggaaat tcttttaaaa 1260

acattttccg accttcaatt caaggggtct tttattttgg tggggaggag tggggtggcc 1320

tttttaggat agaagcaaca cttcgcaata aactcatttt tttttttttg gtccgttgga 1380

gccccccccc catgtgacct agtaatgttt gtaagaaaaa aaaagtaaaa aaaaaaaaaa 1440

tcacaggact ggaaaggaag gggagctacc taccaaaaac agaaggcaaa ggactgagtg 1500

aagctggcca caccacacgt ttctatggat ttcggatttc tacccacgtt tcctcactct 1560

gttttctata tattctatat aaatatatat tgtggatggc cgccatggca aggggtggct 1620

ggggcttttg taggggcggg ggacgggtga cccggccccc tcatcgcaat cagctttgaa 1680

gtctgtattt ttttataata aaatggacaa actgtaaa 1718

<210> 94
<211> 1596
<212> DNA
<213> mouse

<400> 94
cacttttgta cctttctcgc cgggacagag aagtgggccg ggaccagccg ggccagacca 60

gactggaccc caggggcgat gcggctgctg cccctgctgc ggactgtgct ctgggccgcg 120

ctgctcggct cgcgcctgcc ggggtgctcc agcctccgcc acccaatcta ctggaactcc 180

tctaacccca ggttgctccg aggagatgcc gtggtggagc tgggcttcaa cgattaccta 240

gacatcttct gcccacatta tgaaagccca gggcccccag aaggcccgga aacctttgca 300

ttatacatgg tggactggtc aggctacgag gcctgcacgg cagagggggc aaatgccttc 360

cagcgctgga attgctcgat gccttttgcc cctttcagcc ctgttcgatt ctcagaaaag 420

attcagcgct acacaccctt cccgctgggc tttgagttct tgcctggaga gacttactac 480

tacatctcgg tgccgactcc ggagagtcct ggccggtgcc tgagactcca ggtgtctgtc 540

tgctgcaagg agagcgggtc atcacatgag tcagctcatc ctgttgggag tcctggagaa 600

agcggtacgt ccgggtggcg gggaggacac gcgcccagcc ccctgtgtct cttgctgctg 660

ctgctgctcc caatcctccg tctcctgaga gttctgtgag ccaagcacac cttccaagtc 720

accccaagga accgcagccc ttgtcaagag cctctgtgga aggggttcct gccaccatac 780

aggcgatgcc aaaccaggca gacaggatga aactttgatg tgcaaagtcg gaggatctga 840

ggtgaccctt gtaggtgact gccccctcac cacaggctga agagggacct gtaaggcagc 900

tgccgacagt ccgacagtcc gggggcgccc aggaacatag catagctgct ttgatggtct 960

tgtctcctcc ccctggaaga ctgggattcc aggggatcaa ctccttgaga cactgagggc 1020

ccaagccaaa gacctgggaa caggtgggtt cctggccaag aagcaaagga gaagccatgg 1080

cgcctgtgcc ctccaggcct cttcccagga gcaattgttg ccccttcctg ggggccactc 1140

gcttgtcttc tgtgaagacg gacttgccat gaggttgaat ttcatgctgg tttgtatttt 1200

ttataaagtg tttggaccaa accttcaata aatcacccgt tctagtgggg catggtggct 1260

cacacctgta attccagcac tgggagggtg agggtgggga gagtgagcag gaggtcagat 1320

gggtatggga gaccctgtct caaaaaaaaa aaaaaacaag aacaaagcaa accaccacct 1380

tggagagcag tgagcaggct cagcaggtga aagtgtgagc tcaccacaga gccttgacca 1440

cctgagcgag gtcaatccct ggaacccaca ctgtgaagag agagaacaca ccccaaaaag 1500

ttgtccttcg aacatgctac acggcgtgtc aatggatatg ccacacacat gcgcgggcac 1560

gcgtgcacac acactcaaat aataaactta ggttaa 1596

<210> 95
<211> 1276
<212> DNA
<213> human

<400> 95
cttccctctt cactttgtac ctttctctcc tcgactgtga agcgggccgg gacctgccag 60

gccagaccaa accggacctc gggggcgatg cggctgctgc ccctgctgcg gactgtcctc 120

tgggccgcgt tcctcggctc ccctctgcgc gggggctcca gcctccgcca cgtagtctac 180

tggaactcca gtaaccccag gttgcttcga ggagacgccg tggtggagct gggcctcaac 240

gattacctag acattgtctg cccccactac gaaggcccag ggccccctga gggccccgag 300

acgtttgctt tgtacatggt ggactggcca ggctatgagt cctgccaggc agagggcccc 360
cgggcctaca agcgctgggt gtgctccctg ccctttggcc atgttcaatt ctcagagaag 420
attcagcgct tcacaccctt ctccctcggc tttgagttct tacctggaga gacttactac 480
tacatctcgg tgcccactcc agagagttct ggccagtgct tgaggctcca ggtgtctgtc 540
tgctgcaagg agaggaagtc tgagtcagcc catcctgttg ggagccctgg agagagtggc 600
acatcagggt ggcgaggggg ggacactccc agccccctct gtctcttgct attactgctg 660
cttctgattc ttcgtcttct gcgaattctg tgagccaagc agaccttccc tctcatccca 720
aggagccaga gtcctcccaa gatcccctgg aggaggaggg atccctgctg cctgcactgg 780
gggtgccaat tcagaccgac aagatggagc attgatgggg gagatcagag ggtctgaggt 840
gactcttgca ggagcctgtc ccctcatcac aggctaaaga agagcagtag acagccctgg 900
acactctgaa gcagaggcaa gacaaacaca ggcgctttgc aggctgctct gagggtctca 960
gcccatcccc caggaggact gggatttggt atgatcaaat cctcaagcca gctgggggcc 1020
caggctgaag acctggggac aggtcgattg ctggaccagg gcaaagaaga agccctgcca 1080
tctgtgccct gtgggccttt tccctggggc agcaccttgc cctccccagg ggatcactca 1140
cttgtcttct atgaagacgg actcttcatg aggttgaatt tcatgccagt ttgtattttt 1200
ataagtatct agaccaaacc ttcaataaac cactcatctt tttgttgccc tccccaaaaa 1260
aaaaaaaaaa aaaaaa 1276

<210> 96
<211> 1322
<212> DNA
<213> rat

<400> 96
   tccacttttg tacctttctc gccgggacag agaagtgggc cgggaccagc cgggccagac 60
   cagactggac cccaggggcg atgcggctgt tgcccctgct gcggactgtg ctctgggccg 120
   cgctgctcgg ctcgcgcctg cgggggtgct ccagcctccg ccactctatc tactggaact 180
   ccactaaccc caggttgctt cgaggagatg ccgtggtgga gctgggcctc aacgattacc 240
   tagacatctt ctgcccacat tatgagagcc cagggccccc agagggcccg gaaacgtttg 300
   cattatacat agtggactgg tcaggctacg aggcctgcaa ggcagagggg gcaaatgcct 360
   tccagcgctg gaattgcacg ctaccttttg cgccttttgt ccctgttcga ttctccgaaa 420
   agattcagcg cttcacaccg ttcccgctgg gcttcgagtt cttgcctgga gagacttact 480
   actacatctc ggtgccaact ccggagagtc ctggccagtg cctgagactc caggtgtctg 540
   tctgctgcaa ggaggacaga tccgagtcag ctcatcctgt cgggagccct ggagaaagtg 600
   gcacatctgg gtggcgggga ggacacgccc ccagccctct gtctctcctg ctgttgctgc 660
   tgctcccaat actccgtctt ctgcgagttc tgtgagccaa gtacaccttc ccagtcaccc 720
   caaggagcca gagcccttcc caggatcctc tgttgaaggg gttcctgcca ccgtacaggc 780
   gatgccaagc caggcagaca tgatgaagct ttgatgtgca aagtcggagg atctgaggtg 840
   acccttgtag gtgactgctc cctcactaca ggctgaagag gaactgcaag gaagctgcag 900
   acagtcgggc acccagaagc atagctgctt tgatggtctt gtctcctccc cctggaagac 960
   tgggatttca ggggatcaaa tccttgagac actgggggcc aaagccaaag acctgggaac 1020
   cggtgggttc ctggccaaga gcagagaagc cacggtctct gtggcctcca ggcctcttcc 1080
   caggagcaat tgttgcccct tccagggagc cactctcttg tcttctgtga agacggactt 1140
   gccatgaggt tgaatttcat gctggtttgt attttttata aagtgtttgg accaaacctt 1200
   caataaatca cccgttctag tgggggcatg gtggctcaca cctgtaatcc cagcactggg 1260
   ggtggggggt gggggtgggg actgtgagcg ggaagtcagt cttgtatggg aggccctgtc 1320
   tc 1322

<210> 97
<211> 1574
<212> DNA
<213> human

<400> 97
gcttctctcc atcttgtgat tcctttttcc tcctgaaccc tccagtgggg gtgcgagttt 60
gtctttatca ccccccatcc caccgccttc ttttcttctc gctctcctac ccctccccag 120
cttggtgggc gcctctttcc tttctcgccc cctttcattt ttatttattc atatttattt 180
ggcgcccgct ctctctctgt ccctttgcct gcctccctcc ctccggatcc ccgctctctc 240
cccggagtgg cgcgtcgggg gctccgccgc tggccaggcg tgatgttgca cgtggagatg 300
ttgacgctgg tgtttctggt gctctggatg tgtgtgttca gccaggaccc gggctccaag 360
gccgtcgccg accgctacgc tgtctactgg aacagcagca accccagatt ccagaggggt 420
gactaccata ttgatgtctg tatcaatgac tacctggatg ttttctgccc tcactatgag 480
gactccgtcc cagaagataa gactgagcgc tatgtcctct acatggtgaa ctttgatggc 540
tacagtgcct gcgaccacac ttccaaaggg ttcaagagat gggaatgtaa ccggcctcac 600
tctccaaatg gaccgctgaa gttctctgaa aaattccagc tcttcactcc cttttctcta 660
ggatttgaat tcaggccagg ccgagaatat ttctacatct cctctgcaat cccagataat 720
ggaagaaggt cctgtctaaa gctcaaagtc tttgtgagac caacaaatag ctgtatgaaa 780
actataggtg ttcatgatcg tgttttcgat gttaacgaca aagtagaaaa ttcattagaa 840
ccagcagatg acaccgtaca tgagtcagcc gagccatccc gcggcgagaa cgcggcacaa 900
acaccaagga tacccagccg ccttttggca atcctactgt tcctcctggc gatgcttttg 960
acattatagc acagtctcct cccatcactt gtcacagaaa acatcagggt cttggaacac 1020
cagagatcca cctaactgct catcctaaga agggacttgt tattgggttt tggcagatgt 1080
cagatttttg ttttctttct ttcagcctga attctaagca acaacttcag gttgggggcc 1140
taaacttgtt cctgcctccc tcaccccacc ccgccccacc cccagccctg gcccttggct 1200
tctctcaccc ctcccaaatt aaatggactc cagatgaaaa tgccaaattg tcatagtgac 1260

accagtggtt cgtcagctcc tgtgcattct cctctaagaa ctcacctccg ttagcgcact 1320

gtgtcagcgg gctatggaca aggaagaata gtggcagatg cagccagcgc tggctagggc 1380

tgggagggtt ttgctctcct atgcaatatt tatgccttct cattcagaac tgtaagatga 1440

tcgcgcaggg catcatgtca ccatgtcagg tccggagggg aggtattaag aatagatacg 1500

atattacacc atttcctata ggagtatgta aatgaacagg cttctaaaag gttgagacac 1560

tggttttttt tttt 1574

<210> 98
<211> 3473
<212> DNA
<213> mouse

<400> 98
cctccttctt ctctctctcc tcttcctctt cctcccgcgc tcgccgcgcg cctctcgccc 60

cttcatcgta tttattccta tttatttggc gtccgctctc ttcgtctgcc cctctccctc 120

cctccccctg gatccccgat ctctccccgg agtggcgcgt cgggggctcc gccgctggct 180

aggcgtgatg ttgcacgtgg agatgttgac gctgctcttt ctggtgctct ggatgtgtgt 240

gttcagccag gacccgggct ccaaagtcgt cgccgaccgc tacgccgtct actggaacag 300

cagcaacccc agattccaga ggggtgacta ccacattgat gtctgtatca atgactacct 360

ggatgttttc tgccctcact atgaggactc tgtcccagaa gacaagactg agcgctacgt 420

cctgtacatg gtgaattttg atgggtacag tgcctgcgac cacacgtcca aagggttcaa 480

gagatgggaa tgtaaccggc ctcactcccc aaacggaccg ctgaagttct cggaaaaatt 540

ccagctcttc actccctttt ctttaggatt tgaattcagg ccaggccgag agtatttcta 600

catctcctct gcaatcccag acaacggaag aaggtcctgt ctaaagctca aagtctttgt 660

gagaccaaca aatagctgta tgaaaactat aggtgttcat gatcgtgttt tcgatgttaa 720

cgacaaagta gaaaattcat tagaaccagc agatgacacc gtacatgagt cagccgagcc 780

atcccgcggt gagaacgcgg cgcagacacc aaggataccc agccgccttt tggcaatcct 840

actgttcctc ctggcgatgc ttttgacatt atagcacagt ctcctcccgt cgcctgtcac 900

agaaaatgtc agggtcttgg aacaccagag atccacctaa ctgctcatcc caagaaggga 960

cttgttattg gtttttggca gatgtcagat ttttgttttc tttccttcag cctgatttct 1020

aagcaacaac ttggggttgt tgggtgggtg gggggggggt aaaccagttg ctgcctcctt 1080

caccccaccc taccctaagc ccttgccctt gactcctctc atccccttcc caatcaaacg 1140

gagtccagat aaaaatgcca gattgtcaga gtgacagcag caggtcttgg cctcctgtgc 1200

gttttctaga gctcacctcc ttaggttcag ggtatccacc atatgcacac aagaaagaat 1260

ggcggcagat gctgcctgca atgtccaggc ctgagagggg tgccctctcc tatacaatac 1320

ttatgccttc acggtcagga cagtaaggtg attgtgtgga gcttggtgtt ggcctgtcga 1380

gtccagaggg ggcggagtta agaataggcc taatactatc ccacatcctc taggagtgtg 1440

gaagtgagca ggctttgcaa aggttcagat actagttttt atttcatcaa atgggactgt 1500
cttaaagcat tttttttttt tttttttttt tttgccagca aaacttgggc tgtgttgaag 1560
aagccttttc tcccaggagg aaggttggat gatgcaggga tgctaataag aggaagtgtg 1620
gatattgaga aaatgatctg ttgctggagg tgtgagtgcc tctaactttt taggaccggg 1680
cagtgcacac ctgagggttt cttttctttt cttttttttt ttaaatcttt gaatacagtt 1740
caccatggtg ctacaacttt tttttctttt ttaggacttc aaagaggcat ttttatgaat 1800
aaagtgacct tccccaaggc tgccaagcca gggttgatga gtagagagtg gaatagcttt 1860
ggctactcct ctggggacaa tatgtacaag gaaagagagt ctgaccagag gagatgcaat 1920
ttggctttgc tggagctcca gtgtgctgtg gcctttcccc aattcccacc ccgataccca 1980
cgttctgctc cttgctccgt gaccacaggg gactcagaca cagaccccta tcaccaggag 2040
agtcagtcag cactacttgg gagggctaaa gggaaatttg gaatacaaat tccaaatttt 2100
ggaataagaa attcaagtgt tgatgttcta tcctttccct ttctgacaca gcctaaagcg 2160
tagggggaaa cgcgtgttta tctgtgggag ataaacaaga tggagtccca aagactttga 2220
caaaatattt ttttaaaaat ccactagaat agaaaataca ttatttagat atactttatg 2280
ctgagagtga gtatatatgc ttgtcctatt taaacttgtg agaaaaagtg gtatcccttg 2340
gtacatttag aaacacaggg ggttatcttg tttcatagtg gggtttgggg cagaagaatg 2400
aacatgggat gaccctgttg aaagaaactt agcatggccg aggggttggt tccagctgat 2460
tgccaggaaa cacaagcagt gggggtctcc ttgtggggtg tcgggagctt taaaccccaa 2520
agccaagcga gacagggaat agtgtcagac caatggaaaa gtgatctagc ctatttttgc 2580
tattgcacga catcaataga aaccatgcct ttgtaaacaa gcaagtcagt aaatgcaatg 2640
atcatgtaat gtggaaaatt gaaagcattc cagtttaaaa aatatggaat tttttatata 2700
ttcgttttta agatgtatat gtaaaaaaaa gagttcacat tatagatgga cttcatggat 2760
cggaaattgc ttagaatttc gagtggtttc aaattagaaa aatatgtgag tggaaggcag 2820
ctgtgaacga actgcaccct agagagttgt acgcacgttg gaatgtaagc tgggcttact 2880
tacctggtcc ctttggagtg gaggcaactg gagtctgctc tcacctgaac catgtgaccg 2940
gagtcgccag cctcaaagat atagttagtc ctctcccctc ttctcccaac atctgagact 3000
tctctggagt agcagagtct catcacagaa ggcacccaac atttccactg aaacagaact 3060
tcaaagcatt ggatttcttt gcctttattt atgcgctcga cactctcagt gatatccaga 3120
tgtattcttt ttattaatag ttacaggttt gtgggttagt gggatttcag taaagggaca 3180
aaaaaaaaaa tcccttctaa gatggatcga tagaaccaag ataatactgc atgtactaca 3240
gtcaaggaca ccaagtgatc ctatggtgat tccagttagt cctaacgaca ttagtggttc 3300
taatgtcatt gtagaaatgg tgagtcctgt gattctagca tttgtctcta accaatgatg 3360
aaacaattcc tcatggccct ctctgccatt gtctttcatt tcccactgtg caattagacc 3420
cttttacttc actgccatgc caccaattat taagtttaaa aaaaaaaaaa aaa 3473

<210> 99
<211> 768
<212> DNA
<213> rat

<400> 99
gagaggcgcg tcgggggctc cgccgctggc taggcgtgat gttgcacgtg gagatgttga 60

cgctgctctt tctggtgctc tggatgtgtg tgttcagcca ggacccgggc tccaaagtcg 120

tcgccgaccg ctacgccgtc tactggaaca gcagcaaccc cagattccag aggggtgact 180

accacatcga tgtctgtatc aatgactacc tggatgtttt ctgccctcac tatgaggact 240

ctgtaccaga ggataagact gagcgctatg tcctgtacat ggtgaatttt gatggctaca 300

gtgcctgcga ccacacatcc aaagggttca agagatggga atgtaaccgg cctcactctc 360

caaacggacc gctgaagttc tcggagaaat tccagctctt cactcccttt tctttaggat 420

ttgaattcag gccaggccga gagtatttct acatctcctc tgcaatccca gacaatggaa 480

gaagatcctg cctaaagctc aaagtctttg tgaggccaac aaacagctgt atgaaaacta 540

taggtgttcg tgatcgtgtt ttcgatgtta acgacaaagt agaaaattca ttagaaccag 600

cagatgacac cgtacatgag tcagccgagc catcccgcgg agagaacgcg gcacaaacac 660

caaggatacc cagccgcctt ttggcaatcc tactgttcct cctggcgatg cttttgacat 720

tatagcacag tctcctcccg tcacctgtca cagaaaacat cagggtct 768

<210> 100
<211> 4342
<212> DNA
<213> mouse

<400> 100
cccacgcgtc cgcgcgcgct gagggacgcg cagggtgagc gcacctggcc tcggcgaccg 60

cgggagcggc gcggcgtgtc cgccccggag gattgggggt cgctgcccgc ggccggtccc 120

aacgcgtccc ggagtgcgca gaactgggag cggcttgggc atggccatgg cccggtccag 180

gagggactct gtgtggaagt actgttgggg acttttgatg gttttgtgca gaactgcgat 240

ctccagatcg atagttttag agcctatcta ctggaattcc tcgaactcca aatttctacc 300

cggacaaggc ctggtactat acccacagat aggagacaaa ttggatatta tttgccccaa 360

agtggactct aaaactgttg gccagtatga atattataaa gtttatatgg ttgataaaga 420

ccaagcagac agatgcacaa ttaagaagga gaataccccg ctgctcaact gtgccagacc 480

agaccaagat gtgaaattca ccatcaagtt tcaagaattc agccctaacc tctggggtct 540

agaatttcag aagaacaaag attactacat tatatctaca tcaaatgggt ctttggaggg 600

cctggataac caggagggag gggtgtgcca gacaagagcc atgaagatcc tcatgaaagt 660

tggacaagat gcaagttctg ctggatcagc caggaatcac ggtccaacaa gacgtccaga 720

gctagaagct ggtacaaatg ggagaagttc aacaacaagt ccctttgtga agccaaatcc 780

aggttctagc accgatggca acagcgcggg gcattccggg aacaatctcc tgggttccga 840

agtggcctta ttcgcaggga tcgcatcagg atgcatcatc ttcatcgtca tcatcatcac 900
tttggtggtg ctgctgctca agtaccgcag gagacaccgc aaacactctc cacagcacac 960
gaccacgctg tctctcagca cactggccac gcccaagcga ggtggcaaca acaatggctc 1020
ggagcccagt gacgttatca taccactaag gactgcagac agcgtcttct gcccgcacta 1080
cgagaaggtc agcggggact atgggcaccc ggtgtacatc gtgcaggaga tgcccccaca 1140
gagtcctgcc aacatttact acaaggtctg aggcctgaga cctgcgcctc ccaagggaac 1200
tcgcaccttg ttcttgggca cgcagggact gcctgagcct ggctgtgggg gcaggatgcc 1260
tcctggaaga gcctggatct ggacagtttt gtagtctgta gcttttccga ccctggggac 1320
cacagaccct ccccggaagc tggaagactg ctaggagatc cccacttgga ctgccgcggc 1380
cccacgcgga cctccaagcc atgcacccag ccactcaggc ctctgcagag cccggggagg 1440
acacggtagg ctatggatgg cgcagcagca tcttaggaga aggttgcgca ccaggccggc 1500
ccctgcctcc acgtttcctg ccgtgcacac tggacttatc acttggacct cgggttcagt 1560
aaggttttca aagatctcta gtgtttagtc ctcactcact cactcactca ctcactcctt 1620
ctcttgccag ggctctgcag caaactccct agacccctca ctccacgtac tgcatcatta 1680
cgggacactc accacagagt cccagctcca ccctttacac caagatcaaa ttagatgggt 1740
attaggtaca gaagaaccct gcttgcctgg aggccgggtc agccgggaag cgcagatgtg 1800
tggaggagtg aggaggtgct ggctgccacg gcaggtcaag gctgcttgct gcccctggag 1860
catagtaggg atgcaggaag gaaatagata atgctttggt ttttctgaga ggacagagac 1920
aggtgggagg tgactgactg gtgagtggtg gggagccttt cactaccaca cagctatgca 1980
gcagggaatc aaaagtccct ctcctgcggg gaacaaaggg gccattgttg tgaaaggacc 2040
agctagagca cagagggaga gggcaggcct ccggtgaagt gctggggcag aactgcagag 2100
gtactggaaa taaaaagcgc agcgcagagc tgtgggagag tccgtctgct ttgggagatg 2160
ttttaagcag actcagctgc tatattacca cgtttttatt aaaaacacag ggaaagcatt 2220
taggagaaga gcagagagcc aaatctgacc tagaagttga aaagccaaag gtcaaacagg 2280
ctgtaagtcc atcaccactg aggttattgg agaattctca ttaggaaagg caggtcagat 2340
tccccaggcc ccataagtgc cccttccccc tccctgattg agccttacac gttggttttt 2400
ggtttatggc cgtgctgtcc gggctccaag gcagtacccg ggctccatgt caaagcaaag 2460
cacacatggc ccacctctta gagtccttga gatggaagta agttatgccg cggaaggaaa 2520
ggcgaagata ggacatattt ataataggtg tatagaacac aagggatata aaatgaaaga 2580
tttttactaa tatatatttt aagattacac acaatacaca ccagaagacg tggagttcgg 2640
tggtggtggt ggtcgtggtg gtggtgatta aagtgacccc agcgcttagt gctttaaaaa 2700
gtgaaagatt gggtagctac tccccgaaac gtaccaatag caagaaaagt atccataatg 2760
agagcaaatg gcaaaaataa cacggtcctg cgggaatctc gcagaaccgt agactaggaa 2820
tgccagcccc ccaaattgat gtgaccctgc cccgggttag acaatgataa aatgcgctgg 2880

cctttatttt ctgtgttggg ttttcccttg ccttatgggc tgaagtgttc tctagaattt 2940
agcaggtcac actgagggga ttccagttta actgtgggtc cctcctcctc tcctacccca 3000
tccctgccct tccagagaat aacaggaagc cttccttttt tttttttttt aagtgctatg 3060
caaaagagac atctttaaca gagtcctgtt actatggtaa cattttgctt tctgaattgg 3120
gaggaaataa aaattgtaat gacagcattt gaaggttctc agacctccag tgagtacctg 3180
caaaaatgag ttgtcacaga gattattccc tacttctcaa acctgaaaat gatgttggtt 3240
cgatgtgtgt gtgtgtgtat gagtgggtgt gtggtacatg tgtgtacata tatgtataat 3300
atatatctcc agtatatatt atatatatct atatcatatt tctgtggagg gttgccatgg 3360
caatcaactg cagtacatat gtagttcttt ccatcaccct aacctctcct gcgcattcac 3420
acaagagttt cttgtaagcc atcaaaagtt aattctaggg ggagagggat gaggcgggga 3480
gacatgggaa accgtctgat tttaatgaaa tcaaatgtct gtgtcatcgg ttggctacgt 3540
tttggttcta tgctaaactg tgaagaatcg gatgaattga tgaagagttg agttacctgc 3600
aacccattga gaagtgtcct gtgcgtctgt tttgtgtctg gtgcagaaaa tgacaatcta 3660
ccaactgtcc ccttatttgg agttggttca gctttggaaa gttactgtaa atgccttgct 3720
tgtattatca tccctagtca cctgacttcg gagcttgcac catcgtgttt taagtgaaga 3780
cgctgtaaat aggttcagat cttaccgtct atggattcgg gtgttacagt agccttattc 3840
acctttttaa taaaaataca catgaaaacg agacagtaat ggcttttctt acccagattg 3900
tgtacataga gcaatgttgg ttttttataa agtctaagca agatgttttg tataaaatct 3960
gaattttgca atgtatttag ctacagcttt taacggcagt gtcatcccct ttgcactgta 4020
atgaggaaaa aaaaaaggta taaaaggttg ccaaattgct gcatatttgt gccgtaatta 4080
tgtaccatga atatttattt aatttcgttg tccaatttgt aagtaacaca gtattatgct 4140
tgagttataa atattttttc tttgttttat tttaatagcc tgtcataggt ttttttttaa 4200
tctgctttag ttccacatga cagttaagcc ccagaaatga gatccgagca gccacattcc 4260
acgtctgttt caaaatgaat ttgttcttaa aaaaaataaa atattttttt cctatggaaa 4320
aaaaaaaaaa aagggcggcc gc 4342

<210> 101
<211> 4335
<212> DNA
<213> human

<400> 101
gcgcggagct gggagtggct tcgccatggc tgtgagaagg gactccgtgt ggaagtactg 60
ctggggtgtt ttgatggttt tatgcagaac tgcgatttcc aaatcgatag ttttagagcc 120
tatctattgg aattcctcga actccaaatt tctacctgga caaggactgg tactataccc 180
acagatagga gacaaattgg atattatttg ccccaaagtg gactctaaaa ctgttggcca 240
gtatgaatat tataaagttt atatggttga taaagaccaa gcagacagat gcactattaa 300
gaaggaaaat acccctctcc tcaactgtgc caaaccagac caagatatca aattcaccat 360

caagtttcaa gaattcagcc ctaacctctg gggtctagaa tttcagaaga acaaagatta 420
ttacattata tctacatcaa atgggtcttt ggagggcctg gataaccagg agggaggggt 480
gtgccagaca agagccatga agatcctcat gaaagttgga caagatgcaa gttctgctgg 540
atcaaccagg aataaagatc caacaagacg tccagaacta gaagctggta caaatggaag 600
aagttcgaca acaagtccct ttgtaaaacc aaatccaggt tctagcacag acggcaacag 660
cgccggacat tcggggaaca acatcctcgg ttccgaagtg gccttatttg cagggattgc 720
ttcaggatgc atcatcttca tcgtcatcat catcacgctg gtggtcctct tgctgaagta 780
ccggaggaga cacaggaagc actcgccgca gcacacgacc acgctgtcgc tcagcacact 840
ggccacaccc aagcgcagcg gcaacaacaa cggctcagag cccagtgaca ttatcatccc 900
gctaaggact gcggacagcg tcttctgccc tcactacgag aaggtcagcg gggactacgg 960
gcacccggtg tacatcgtcc aggagatgcc cccgcagagc ccggcgaaca tttactacaa 1020
ggtctgagag ggaccctggt ggtacctgtg ctttcccaga ggacacctaa tgtcccgatg 1080
cctcccttga gggtttgaga gcccgcgtgc tggagaattg actgaagcac agcaccgggg 1140
gagagggaca ctcctcctcg gaagagcccg tcgcgctgga cagcttacct agtcttgtag 1200
cattcggcct tggtgaacac acacgctccc tggaagctgg aagactgtgc agaagacgcc 1260
cattcggact gctgtgccgc gtcccacgtc tcctcctcga agccatgtgc tgcggtcact 1320
caggcctctg cagaagccaa gggaagacag tggtttgtgg acgagagggc tgtgagcatc 1380
ctggcaggtg ccccaggatg ccacgcctgg aagggccggc ttctgcctgg ggtgcatttc 1440
ccccgcagtg cataccggac ttgtcacacg gacctcgggc tagttaaggt gtgcaaagat 1500
ctctagagtt tagtccttac tgtctcactc gttctgttac ccagggctct gcagcacctc 1560
acctgagacc tccactccac atctgcatca ctcatggaac actcatgtct ggagtcccct 1620
cctccagccg ctggcaacaa cagcttcagt ccatgggtaa tccgttcata gaaattgtgt 1680
ttgctaacaa ggtgcccttt agccagatgc taggctgtct gcgaagaagg ctaggagttc 1740
atagaaggga gtggggctgg ggaaagggct ggctgcaatt gcagctcact gctgctgcct 1800
ctgaaacaga aagttggaaa ggaaaaaaga aaaaagcaat taggtagcac agcactttgg 1860
ttttgctgag atcgaagagg ccagtaggag acacgacagc acacacagtg gattccagtg 1920
catggggagg cactcgctgt tatcaaatag cgatgtgcag gaagaaaagc ccctcttcat 1980
tccggggaac aaagacgggt attgttggga aaggaacagg cttggaggga agggagaaag 2040
taggccgctg atgatatatt cgggcaggac tgttgtggta ctggcaataa gatacacagc 2100
tccgagctgt aggagagtcg gtctgctttg gatgattttt taagcagact cagctgctat 2160
acttatcaca ttttattaaa cacagggaaa gcatttagga gaatagcaga gagccaaatc 2220
tgacctaaaa gttgaaaagc caaaggtcaa acaggctgta attccatcat catcgttgtt 2280
attaaagaat ccttatctat aaaaggtagg tcagatcccc ctccccccag gttcctcctt 2340
cccctcccga ttgagcctta cgacactttg gtttatgcgg tgctgtccgg gtgccagggc 2400

tgcagggtcg gtactgatgg aggctgcagc gcccggtgct ctgtgtcaag gtgaagcaca 2460
tacggcagac ctcttagagt ccttaagacg gaagtaaatt atgatgtcca gggggagaag 2520
gaagatagga cgtatttata ataggtatat agaacacaag ggatataaaa tgaaagattt 2580
ttactaatat atattttaag gttgcacaca gtacacacca gaagatgtga aattcatttg 2640
tggcaattaa gtggtcccaa tgctcagcgc ttaaaaaaac aaattggaca gctacttctg 2700
ggaaaaacaa catcattcca aaaagaacaa taatgagagc aaatgcaaaa ataaccaagt 2760
cctccgaagg catctcacgg aaccgtagac taggaagtac gagccccaca gagcaggaag 2820
ccgatgtgac tgcatcatat atttaacaat gacaagatgt tccggcgttt atttctgcgt 2880
tgggttttcc cttgccttat gggctgaagt gttctctaga atccagcagg tcacactggg 2940
ggcttcaggt gacgatttag ctgtggctcc ctcctcctgt cctcccccgc accccctccc 3000
ttctgggaaa caagaagagt aaacaggaaa cctacttttt atgtgctatg caaaatagac 3060
atctttaaca tagtcctgtt actatggtaa cactttgctt tctgaattgg aagggaaaaa 3120
aaatgtagcg acagcatttt aaggttctca gacctccagt gagtacctgc aaaaatgagt 3180
tgtcacagaa attatgatcc tctatttcct gaacctggaa atgatgttgg tccaaagtgc 3240
gtgtgtgtat gtgtgagtgg gtgcgtggta tacatgtgta catatatgta taatatatat 3300
ctacaatata tattatatat atctatatca tatttctgtg gagggttgcc atggtaacca 3360
gccacagtac atatgtaatt ctttccatca ccccaacctc tcctttctgt gcattcatgc 3420
aagagtttct tgtaagccat cagaagttac ttttaggatg ggggagaggg gcgagaaggg 3480
gaaaaatggg aaatagtctg attttaatga aatcaaatgt atgtatcatc agttggctac 3540
gttttggttc tatgctaaac tgtgaaaaat cagatgaatt gataaaagag ttccctgcaa 3600
ccaattgaaa agtgttctgt gcgtctgttt tgtgtctggt gcagaatatg acaatctacc 3660
aactgtccct ttgtttgaag ttggtttagc tttggaaagt tactgtaaat gccttgcttg 3720
tatgatcgtc cctggtcacc cgactttgga atttgcacca tcatgtttca gtgaagatgc 3780
tgtaaatagg ttcagatttt actgtctatg gatttggggt gttacagtag ccttattcac 3840
ctttttaata aaaatacaca tgaaaacaag aaagaaatgg cttttcttac ccagattgtg 3900
tacatagagc aatgttggtt ttttataaag tctaagcaag atgttttgta taaaatctga 3960
attttgcaat gtatttagct acagcttgtt taacggcagt gtcattcccc tttgcactgt 4020
aatgaggaaa aaatggtata aaaggttgcc aaattgctgc atatttgtgc cgtaattatg 4080
taccatgaat atttatttaa aatttcgttg tccaatttgt aagtaacaca gtattatgcc 4140
tgagttataa atattttttt ctttctttgt tttattttaa tagcctgtca taggttttaa 4200
atctgcttta gtttcacatt gcagttagcc ccagaaaatg aaatccgtga agtcacattc 4260
cacatctgtt tcaaactgaa tttgttctta aaaaaataaa atattttttt cctatggaaa 4320
aaaaaaaaaa aaaaa 4335

<210> 102
<211> 2040
<212> DNA
<213> rat

<400> 102
cgcgcgtcag acgcctgcgg gggtcccagt gctctcctga ctgcttagcg gcggcgagtt 60

gcgaggattc aggaggcgcc ctttctctct gcgctccagg acatggaacc gcgccgacga 120

ggcgctcgct tgcccgggcc gctgcccgct gtcctggatc tatagtcagg cggcccctct 180

cggggagcag cacccgccac ctcacaccga ctggtctctc cgctgcgggc ctttgtcccg 240

gcccagtgcc ctcgccgcgc ccgccctccg cggctggccc ggcaccctgg agccgcacgg 300

gagccggctg tcccagctgc gtccggcgcg gcgccccgga gccccgagtc cgccgagccg 360

ccgcgccccg cgcgttcgac cccaccccgc gcgctgaggg cagcgcaggg tgagcgcacc 420

tggcctcggc gaccgcggga gcggcgcgtc cgccggggag gattggtggt cgcgaccggc 480

ggccggtccc atcgcgcccc ggagtacgca gaactgggag cggtttggcc atggccatgg 540

cccggtccag gagggactct gtgtggaagt actgttgggg acttttgatg gttttgtgca 600

gaactgcgat ctccagatcg atagttttag agcctatcta ctggaattcc tcgaactcca 660

aatttctacc cggacaaggc ctggtactat acccacagat aggagacaaa ttggatatta 720

tttgccccaa agtggactct aaaactgttg gccagtatga atattataaa gtttatatgg 780

ttgataaaga gcaagccgac agatgcacta ttaagaagga aaatacccca ctgctcaact 840

gtgccagacc agaccaagat gtgaaattca ccatcaagtt ccaagaattc agccctaacc 900

tctggggtct agagtttcag aagaacaaag attactacat tatatctaca tcaaatgggt 960

ctttggaggg cctggataac caggagggag gggtgtgcca gacaagagcc atgaagatcc 1020

tcatgaaagt tggacaagat gcaagttctg ctggatcaac caggaataat gatccaacaa 1080

gacgtccaga gctagaagct ggtacgaatg ggagaagttc aacaacaagt ccctttgtga 1140

agccaaatcc aggtacgcca cagggcaaac ccaggactct ggtaaccggc tcttcttgct 1200

cctgtgcagg ttctagcacc gatggcaaca gcgcggggca ttccgggaac aatctcctgg 1260

gttccgaagt ggccttattc gcagggattg catcaggatg catcatcttc atcgtcatca 1320

tcatcacttt ggtggtgctg ctgctcaagt accgcaggag acaccgcaaa cactctccac 1380

agcacacgac cacgctgtct ctcagcacac tggccacgcc caagcgaggt ggcaacaaca 1440

atggctcgga gcccagtgac gttatcatac cgctaaggac tgcagacagt gtcttctgcc 1500

cacactacga gaaggtcagc ggggactatg ggcacccggt gtacatcgtg caggagatgc 1560

ccccacagag tcctgccaac atttactaca aggtctgagg cctgagacct gcgcctccca 1620

agggaactcg caccttgttc ttgggcacgc agggactgcc tgagcctggc tgttgggggc 1680

aggatgcctc ccggaagagc ccggatctgg acagttttgt agtctgtagc ttttcgaccc 1740

tggggaccac agaccctccc cggaagctgg aagactgcta ggagatcccc acttggactg 1800

ccgcgttccc acgcggacct ccaagccatg cacccagcca ctcaggcctc tgcagagccc 1860

ggggggtggg ggggacgaca cggcaggcta tggatagtgc agccacatct taggagaagg 1920

ttgcgcacca ggccggcctc tgcctccacg tttcctgccg tgcacactgg acttatcact 1980

cggacctcgg gttcagtatg gttttcaaag atctctagtg tttagtcctc actcactcct 2040

<210> 103
<211> 3236
<212> DNA
<213> human

<400> 103
ggtttcctcc cttagcccgc tgccctcaat cccagcgagg ctggggctcc ggctcggcgc 60

ccccttcctc gctccctggt ccggcgcccc atgccgcccc cgcccggtcc ccggctcccc 120

cagtccccca cttaggcggg ctcacagatc ccggggtgct ggcgcgtggg ccgggggcgc 180

gtagggcgcc tgcagacggc ccctggaagg gctctggtgg ggctgagcgc tctgccgcgg 240

gggcgcgggc acagcaggaa gcaggtccgc gtgggcgctg ggggcatcag ctaccggggt 300

ggtccgggct gaagagccag gcagccaagg cagccacccc ggggggtggg cgactttggg 360

ggagttggtg ccccgccccc caggccttgg cggggtcatg gggccccccc attctgggcc 420

ggggggcgtg cgagtcgggg ccctgctgct gctgggggtt ttggggctgg tgtctgggct 480

cagcctggag cctgtctact ggaactcggc gaataagagg ttccaggcag agggtggtta 540

tgtgctgtac cctcagatcg gggaccggct agacctgctc tgcccccggg cccggcctcc 600

tggccctcac tcctctccta attatgagtt ctacaagctg tacctggtag ggggtgctca 660

gggccggcgc tgtgaggcac cccctgcccc aaacctcctt ctcacttgtg atcgcccaga 720

cctggatctc cgcttcacca tcaagttcca ggagtatagc cctaatctct ggggccacga 780

gttccgctcg caccacgatt actacatcat tgccacatcg gatgggaccc gggagggcct 840

ggagagcctg cagggaggtg tgtgcctaac cagaggcatg aaggtgcttc tccgagtggg 900

acaaagtccc cgaggagggg ctgtcccccg aaaacctgtg tctgaaatgc ccatggaaag 960

agaccgaggg gcagcccaca gcctggagcc tgggaaggag aacctgccag gtgaccccac 1020

cagcaatgca acctcccggg gtgctgaagg ccccctgccc cctcccagca tgcctgcagt 1080

ggctggggca gcaggggggc tggcgctgct cttgctgggc gtggcagggg ctgggggtgc 1140

catgtgttgg cggagacggc gggccaagcc ttcggagagt cgccaccctg gtcctggctc 1200

cttcgggagg ggagggtctc tgggcctggg gggtggaggt gggatgggac ctcgggaggc 1260

tgagcctggg gagctaggga tagctctgcg gggtggcggg gctgcagatc cccccttctg 1320

cccccactat gagaaggtga gtggtgacta tgggcatcct gtgtatatcg tgcaggatgg 1380

gcccccccag agccctccaa acatctacta caaggtatga gggctcctct cacgtggcta 1440

tcctgaatcc agcccttctt ggggtgctcc tccagtttaa ttcctggttt gagggacacc 1500

tctaacatct cggccccctg tgccccccca gccccttcac tcctcccggc tgctgtcctc 1560

gtctccactt ttaggattcc ttaggattcc cactgcccca cttcctgccc tcccgtttgg 1620

ccatgggtgc ccccctctgt ctcagtgtcc ctggatcctt tttccttggg gaggggcaca 1680

ggctcagcct cctctctgac catgacccag gcatccttgt ccccctcacc cacccagagc 1740
taggggcggg aacagcccac cttttggttg gcaccgcctt ctttctgcct ctcactggtt 1800
ttctcttctc tatctcttat tctttccctc tcttccgtct ctaggtctgt tcttcttccc 1860
tagcatcctc ctccccacat ctcctttcac cctcttggct tcttatcctg tgcctctccc 1920
atctcctggg tgggggcatc aaagcatttc tccccttagc tttcagcccc ccttctgacc 1980
tctcatacca accactcccc tcagtctgcc aaaaatgggg gccttatggg gaaggctctg 2040
acactccacc ccagctcagg ccatgggcag cagggctcca ttctctggcc tggcccaggc 2100
ctctacatac ttactccagc catttggggt ggttgggtca tgacagctac catgagaaga 2160
agtgtcccgt tttgtccagt ggccaatagc aagatatgaa ccggtcggga catgtatgga 2220
cttggtctga tgctgaatgg gccacttggg accggaagtg acttgctcca gacaagaggt 2280
gaccaggccc ggacagaaat ggcctgggaa gtagcagaag cagtgcagca ggaactggaa 2340
gtgccttcat ccaggacagg aagtagcact tctgaaacag gaagtggtct ggctggaact 2400
ccaagtggct tagtctgggg gatcaggagg tgggaggtgg atggttctta ttctgtggag 2460
aagaagggcg ggaagaactt cctttcagga ggaagctgga acttactgac tgtaagaggt 2520
tagaggtgga ccgagaagga cttttcccag tcttcagtgg cacttcccaa gatctccctt 2580
cccttgtgct ctgtgctgat tttaggacag ctaagatgac tgccatgtgc tgtggcaggc 2640
ctaatttgtc ttgttctttc ctttccatat cccagtataa tctctgttaa tcaacaggac 2700
taccccaaga acccatgtgc tctcccgagt aacccagatg gctgtcttgt tcattccatc 2760
ctacatttct gactcctttc agactcaaca cagttccctt cttagtgacc aaaatggtgg 2820
cctactggct ggtctagctg acagtggtac ttagcaaagg ccactgtttc catagtgacc 2880
agctgatacc tcttcctgcc ctctagtgtg caattgggtg ttgcctcagt ttcctcccag 2940
ctcagtttta ttagatcaaa gctgttgttg ggcaccaggt tggccacctc aatcaccagc 3000
caagatggtt gctttgtcca ccagaggtca agttcacctc tctggtgctg tagttcccag 3060
ctccttcctg atttttctaa tcgctccttc tggggaacag gaagttgata ttgccatggt 3120
ggcggggtat gccgtcacct cagtagtttt actgtaaaag ggaaatttga acaacaaaaa 3180
ccaaaaaaaa taaaaataaa aaacttcaaa agttgacaaa aaaaaaaaaa aaaaaa 3236

<210> 104
<211> 3126
<212> DNA
<213> mouse

<400> 104
gaatcccggc gaggctgggg ctccagctcg gtgtctcctt cttcgctccc ttgcccggcg 60
cctcatgttg cccccgcccg gccctcggct ccccatgtcc cccacgtagc ggggcgcaca 120
aatcccgagg tgcggcgcgt gggccggggg cgcgtaaggc gcctgcagac ggcccctgca 180
agggttcttg tggggctgag cgctctggag ccggggagca agcatcgcag aaaagagggt 240

ccttgtggga gctgggggtg actagccacc caccagggtg actggggttc cacagcgtgg 300
tagccacccc tgggcggtga gagactttgg gggagttggt gccccgcccc caggcccttg 360
gtagggtcat gggggccccc cattttgggc cagggggtgt gcaagtcggg gccctgctgc 420
tgttaggttt tgcggggctg gtatctggac tcagcctgga gcctgtctac tggaactcgg 480
cgaataagag gttccaggca gagggcggtt acgtgcttta tcctcagatc ggggaccggc 540
tagatctact ttgtccccgg gctcggcctc ctggccccca ctcctctcct agttatgagt 600
tctacaaact gtacctggta gagggtgccc agggtcggcg ttgtgaggca ccccctgccc 660
caaaccttct tctcacatgt gaccggccag acctggacct ccgcttcacc atcaagttcc 720
aggaatacag ccctaacctc tggggccacg agttccgatc ccaccacgat tactacataa 780
ttgccacatc agacgggacc cgggaaggcc ttgagagctt gcaaggaggc gtgtgcctaa 840
ccagaggcat gaaggtgctt ctgcgagtgg gacaaagtcc ccgaggagga gctgtacccc 900
gaaaacctgt gtctgaaatg cccatggaga gagacagagg ggcagctcac agcgcggaac 960
ctgggaggga caccatacca ggtgacccca gcagcaatgc aacctcccgg ggtgctgaag 1020
gccccctgcc ccctcccagc atgcccgcag tggctggggc agcagggggg atggcgctgc 1080
tcttgctggg cgtggcaggg gctgggggtg ccatgtgttg gcggagacgg cgggccaagc 1140
cttcggagag tcgccaccct ggtcctggct cctttgggag gggagggtct ctgggcctgg 1200
gtggtggagg agggatgggg cctcgggaag ctgagcctgg ggagctagga atagccctgc 1260
ggggtggtgg gactgcagac ccccccttct gccctcacta cgagaaggtg agcggtgact 1320
atgggcaccc tgtgtacatt gtgcaggatg ggccccccca gagccctccg aacatctatt 1380
acaaggtatg agggctcctc ccacctggct ctcctcaatc tagccgtcca tggggtgctc 1440
ctccagtgta attcatggta tgagggacac ctgcgccatg actttggccc cctttgccct 1500
accagctctt tggctcctcc tggctatagc cctcatctcc aattttagga tcccttaaga 1560
cttgactaca ccttttcctg acctccccga ctgaccctca tttgactgtg ggcactcctc 1620
tgtttctgcc tcttagggga ggggggtcac aggctcagcc tcccttaatc actacccagg 1680
tgtcctttct ccctcaccca cccagagtgg gggtggggac agtctgtctt ttggttggca 1740
cctctttctg cctctcacca tttttctctt ctctctattt cttattctct ctctctcctg 1800
tgtctaggtc tgttctcttc ctcctcccta gcctcctcct tacatctcct tttaccctct 1860
cggcttcttg tccttcctgt gcctctcacg tgtctcctgg gttggggcat caaagcatct 1920
ctcctctttg ctcttggccc tttccaacct ttcataccaa ctgctcccct ctgtccgcca 1980
aaaacggggg cttgcggggg gtggggggga gattctggaa ctctaccccc ccagctccag 2040
gcatgggcac agggcctctt tccctgcctt tctgtgtact tactccagcc atttggggtg 2100
gttattgggc cacaactact accatgagaa gaagtgtcct gttgtggcca gtggctgata 2160
ggaagacatg aagcagtgtg gacatgatgg actgtgtctg atgccgaatg ggcatcagat 2220
aggaagtgac tcgttccaga caagaagtga ccaggcctgg acagaaatgg cctgggaagt 2280

gtccgcagca ggaactggaa gtgccttcat ccagaacagg aagtcgcact tctgaaatag 2340
gaagtggtct ggctggaatt ccaagtggct tagtctgggg aggtggcctg ggtgggggtg 2400
ggggcatggg aagtggatgg ttcttaccct gtggagaaag ggcaggaggg aactccctgc 2460
tttaggagga agctggaact taccactgtg agaggacaga tgtggactga gagttttctt 2520
agtgttcagt ggcacttccc aaggacccct tcccttgtgc tctgtgcagt ttttaggaca 2580
gctaagatga ctgccacttg ctgtggcagg cccaatttgt cttgttctcc ccttactgta 2640
ccccagtata atctctgttg atcaacagaa ctaccccaag aacccacatg ttctccccac 2700
ctaaccagac ggctgtctgg ctgatgcctt cctcccttca agcccaacac agcgcccttg 2760
tcagtgaaga ggtggtccat ggactggtct agctgatggc agttattagc actggttgct 2820
gtttccatag tgacaagcct atacctcttc ccgctcttta gtgtgcagtg ggctgctgcc 2880
tcggtgttct cccgcctcag ttttattaga tcaaagctgc ccttggccat catgttggcc 2940
acctcaatca ccagccaaaa tggttgcttt gtccaccaga ggtcaagcca tctctctggc 3000
gctgtagttc ccagctcctt ctagggaaca ggaagttgat attgccattg gggaggtggc 3060
ggggtatggc cgtcacctcg atagttttat tgtaaaaggg aaatttgaac aacaacaaaa 3120
aaactg 3126

<210> 105
<211> 3287
<212> DNA
<213> rat

<400> 105
gtggggggcg tgggccgcgg gatgcgacgc ccgctccgac tgagctccag ctccggtttt 60
tccctccacc atgctctccc cctccccgcc tctcctttaa ctcccccctc ctgggctcgg 120
gactggtttc ctcccttagc ccgctgccct caatcccggc gaggctgggg ctccagctcg 180
gtgtctcctt cttcgctgcc ttgcccggcg ccccatgtcg cccccgcccg gccctctgct 240
ccccatgtcc cccacgtagc ccggcgcaca aatcccgagg tgcggcgcgt gggccggggg 300
tgcatagggc gcctgcagac ggcccctgca agggttctag tggggctgag cgctctggag 360
ccggggcgca agcatcgcag aaaagagggt ccttgtggga gctgggggtg actagccacc 420
caccagggtg actggggctc cacagcgtgg tagccacccc tgggggtgag agactttggg 480
ggagttggtg ccccgccccc aggccttggt agggtcatgg ggggccccca ttttgggcca 540
gggggtgtgc aagtcggggc cctgctgctg ttaggttttg cggggctggt gtctggactc 600
agcctggagc ctgtctactg gaactcggcg aataagaggt tccaggcaga gggtggttac 660
gtgctttacc ctcagatcgg ggaccggcta gatctacttt gtccccgggc ccggcctcct 720
ggcccccact cctctcctag ttatgagttc tacaaactgt acctggtagg gggtgcccag 780
ggtcggcgtt gtgaggcacc ccctgcccca aaccttcttc tcacatgtga ccggccagac 840
ctggacctcc gcttcaccat caagttccag gaatacagcc ctaacctctg gggccacgag 900
ttcagatccc accacgatta ctacataatt gccacatcag atgggacccg ggaaggcctg 960

gagagcttgc agggaggtgt gtgcctaacc agaggcatga aggtgcttct gcgagtggga 1020
caaagtcccc gaggaggagc tgtaccccga aaacctgtgt ctgaaatgcc catggagaga 1080
gaccgagggg cagctcacag ccaggagcct gggaaggaca gcataccagg tgaccccaac 1140
agcaatgcaa cctcccgggg tgctgaaggc cccctgcccc ctcccagcat gcccgcggtg 1200
gctggggcag caggggggct ggcgctgctc ttgctgggcg tggcaggggc tgggggtgcc 1260
atgtgttggc ggagacggcg ggccaagcct tcggagagtc gccaccctgg tcctggctcc 1320
tttgggagag gagggtctct gggcctgggt ggtggagggg ggatgggacc tcgggaggct 1380
gagcctgggg agctaggaat agccctgcgg ggtggtgggg ctgcagatcc ccccttctgc 1440
cctcactatg agaaggtgag tggtgactac ggacaccctg tgtacattgt gcaggatggc 1500
cccccccaga gccctccaaa catctactac aaggtatgag ggctcctccc acttggctct 1560
cctcaatcca gtcgcccatg gggtgctcct ccagtgtaat ttgtggtatg aggtacacct 1620
gcgccatgac tttggccccc tttgccccac cggctctttg gctccttctg gctatagccc 1680
ccatctccag ttttaggatc ccttaagact cgactacacc tcttcctgcc ctccctctgt 1740
ctctgcctct taggggaggg gggcacaggc tcagcctcct tcaatcacta cacaggtgtc 1800
cttttccttc gcccacccag agtaggggtg ggaacggtct gtcttttggt tggcacctct 1860
ttctgcctct caccgttttt ctcttctcta tttcttattc tctctctccc tctcctgtgt 1920
ctaggtctgt tctcttcctc ctccctagcc tcctcctcct tacatctcct ttaaccctct 1980
tggcttcttg tccttcctgt gcctctcacg tgtctcctag gttggggcat caaagtatct 2040
ctccctcggc tcttggccct ttccaacttt tcataccaac tgctcccctc cgtctgccaa 2100
aaacgggggc ttctgggggt tggaggggag attctggcac tctacccccc cagctccagg 2160
catgggcaca gggcctcttt ccctgccttt ctatgtactt actccagcca tttggggtgg 2220
ttactgggcc acagcaaact accctgagaa gaagtgtcct gttgtatcca gtggctgata 2280
ggaagacatg aagcagtgaa gacatgatgg actggatctg atgctaaatg ggcatcagat 2340
aggaagtgac ttgttccaga caagaagtga ccaggcctgg acagaaatgg cctgggaagt 2400
aagtggcagc agcaggaact ggaagtgcct tcgtccagaa caggaagtcg cacttctgaa 2460
ataggaagtg gtctggctgg aattccaagt ggcttagtct ggggaggtgg tgggggtggg 2520
ggtgggggca tggggaagtg gatggttctt accctgtgga gaaaggggca ggaggaacct 2580
cctgtgttag gaggaagctg gagcttacca ctgtgagagg acagatgtgg actgagaatt 2640
ttcttagtgc tcagtggcac ttcccaagga ctcccctccc cttgtgctct gtgcggtttt 2700
taggacagct aagatgactg ccacctgttg tggcaggccc gatttgtctt gttctcccct 2760
tactgtaccc cgatataatc tctgttgatc aacaggacta ccccaagaat ccacatgttc 2820
tcccccgtaa ccaggcagct gtctggttca tgccttcttc ccttcaaacc caacccagcg 2880
cccttgttag tgaagaggtg gtccatggac tgatgacagt tattagcact ggatgctgtt 2940
tccatagtga caagcctata cctcttccca ccctttagtg cgcagtgggc tgctgcttca 3000

gtatcctccc agctcagttt tattagatca aagctgccct tgggcaccat gttggccacc 3060

tcaatcacca gccaaaatgg tcgctttgtc caccagaggt caagccatct ttctggcgct 3120

gtagttccca gctccttcta gggaacagga agttgatatt gccatggggg aggtggcggg 3180

gtgtggccgt cacctcaata gttttactgt aaaagggaaa tttgaacaag aacaacaaca 3240

acaaaaaaaa aaaaacaaag aaaaaaataa aaaactttaa aagttga 3287

<210> 106
<211> 655
<212> DNA
<213> human

<400> 106
gcgcaggcgc gcggggcaag agggtggcag tgcgcctgcg ccgcgtcggc gtgcggaacg 60

ccgcggtgtc tcggcgcctc tgcgcgcggg aagatggcgg aacaggctac caagtccgtg 120

ctgtttgtgt gtctgggtaa catttgtcga tcacccattg cagaagcagt tttcaggaaa 180

cttgtaaccg atcaaaacat ctcagagaat tggagggtag acagcgcggc aacttccggg 240

tatgagatag ggaacccccc tgactaccga gggcagagct gcatgaagag gcacggcatt 300

cccatgagcc acgttgcccg gcaggtaccg tccttggact tgaagttgtg tgttttgtgt 360

ttcagtgggt cattgacagc ggtgctgttt ctgactggaa cgtgggccgg tccccagacc 420

caagagctgt gagctgccta agaaatcatg gcattcacac agcccataaa gcaagacagg 480

tagacaagct cttgttcaat ttctaatata tagagtccag taacttgaga agtagcgaaa 540

ggattaacca gacttgtata ttaatgaatg tgtttattta gggtgagctt aaccagctat 600

ggtgtgtcca ttttgtttca cttctggttg cacggtgttg aaagacttgc ctgac 655

<210> 107
<211> 1549
<212> DNA
<213> human

<400> 107
tggcagtgcg cctgcgccgc gtcggcgtgc ggaacgccgc ggtgtctcgg cgcctctgcg 60

cgcgggaaga tggcggaaca ggctaccaag tccgtgctgt ttgtgtgtct gggtaacatt 120

tgtcgatcac ccattgcaga agcagttttc aggaaacttg taaccgatca aaacatctca 180

gagaattggg tcattgacag cggtgctgtt tctgactgga acgtgggccg gtccccagac 240

ccaagagctg tgagctgcct aagaaatcat ggcattcaca cagcccataa agcaagacag 300

attaccaaag aagattttgc cacatttgat tatatactat gtatggatga aagcaatctg 360

agagatttga atagaaaaag taatcaagtt aaaacctgca aagctaaaat tgaactactt 420

gggagctatg atccacaaaa acaacttatt attgaagatc cctattatgg gaatgactct 480

gactttgaga cggtgtacca gcagtgtgtc aggtgctgca gagcgttctt ggagaaggcc 540

cactgaggca ggttcgtgcc ctgctgcggc cagcctgact agaccccacc ctgaggtcct 600

gcatttctca gtcggtgtgt aatcacgttc cagggcccaa agcccagctc tttgttcagt 660

tgacttactg tttcttacct taaaaagtaa ttgtagatgg aaatcagttg tgtttggcag 720

gagaatcaat aaaaatcttt gattcagaca gcttatgggg tattttaagc attcttagac 780

tagttgaaca tctcactttg ccccagttac aaaaatagta gaacaagcaa cataaaacaa 840

tgaaggaaaa cctcacttga aggcccaggt caacatctaa gcctgttgag acttagataa 900

tcgagtctac ctcttcagta ggtttgtgtg gatggcctgg agggcaggtg ccctctgctc 960

cccagtgcta cctctctctt ccctagggcc ttttgtggat tgacagtagt cccctccgta 1020

ggagctcaca gtctagatta gaagtgtttt aatttctaca cacccatagt gcacacttgt 1080

atattgaaaa gatagggaag agagaaacat ttatggaatc agtcgttggc accttcaata 1140

cttcatgatt tttgtcgagt ttacttcatg aggaggtcag cccattggct cccatctgaa 1200

ccactttgcc tctgaaactt aattacatcc agaaagaagg acacttgtat gctagtctat 1260

ggtcagttga ggaatatgac tgtttttata tgcacatgta acccaaatgt ccaatataaa 1320

ttggcttatt ttttaaaata attttaaaag ttgggaaaag tgttattatt tggcatgctt 1380

aaatattgaa taagtattct tcatcagcat ttaataaatg tataggcaga tgtaaggtaa 1440

tttctgtgta ttttgagata atgtcaaaat catgaatatt tcaaaataaa ctggggagtt 1500

ataaaaatac aactagagat ataaaaaaaa aaaaaaaaaa aaaaaaaaa 1549

<210> 108
<211> 1549
<212> DNA
<213> human

<400> 108
tggcagtgcg cctgcgccgc gtcggcgtgc ggaacgccgc ggtgtctcgg cgcctctgcg 60

cgcgggaaga tggcggaaca ggctaccaag tccgtgctgt ttgtgtgtct gggtaacatt 120

tgtcgatcac ccattgcaga agcagttttc aggaaacttg taaccgatca aaacatctca 180

gagaattgga gggtagacag cgcggcaact tccgggtatg agatagggaa cccccctgac 240

taccgagggc agagctgcat gaagaggcac ggcattccca tgagccacgt tgcccggcag 300

attaccaaag aagattttgc cacatttgat tatatactat gtatggatga aagcaatctg 360

agagatttga atagaaaaag taatcaagtt aaaacctgca aagctaaaat tgaactactt 420

gggagctatg atccacaaaa acaacttatt attgaagatc cctattatgg gaatgactct 480

gactttgaga cggtgtacca gcagtgtgtc aggtgctgca gagcgttctt ggagaaggcc 540

cactgaggca ggttcgtgcc ctgctgcggc cagcctgact agaccccacc ctgaggtcct 600

gcatttctca gtcggtgtgt aatcacgttc cagggcccaa agcccagctc tttgttcagt 660

tgacttactg tttcttacct taaaaagtaa ttgtagatgg aaatcagttg tgtttggcag 720

gagaatcaat aaaaatcttt gattcagaca gcttatgggg tattttaagc attcttagac 780

tagttgaaca tctcactttg ccccagttac aaaaatagta gaacaagcaa cataaaacaa 840

tgaaggaaaa cctcacttga aggcccaggt caacatctaa gcctgttgag acttagataa 900

tcgagtctac ctcttcagta ggtttgtgtg gatggcctgg agggcaggtg ccctctgctc 960
cccagtgcta cctctctctt ccctagggcc ttttgtggat tgacagtagt cccctccgta 1020
ggagctcaca gtctagatta gaagtgtttt aatttctaca cacccatagt gcacacttgt 1080
atattgaaaa gatagggaag agagaaacat ttatggaatc agtcgttggc accttcaata 1140
cttcatgatt tttgtcgagt ttacttcatg aggaggtcag cccattggct cccatctgaa 1200
ccactttgcc tctgaaactt aattacatcc agaaagaagg acacttgtat gctagtctat 1260
ggtcagttga ggaatatgac tgtttttata tgcacatgta acccaaatgt ccaatataaa 1320
ttggcttatt ttttaaaata attttaaaag ttgggaaaag tgttattatt tggcatgctt 1380
aaatattgaa taagtattct tcatcagcat ttaataaatg tataggcaga tgtaaggtaa 1440
tttctgtgta ttttgagata atgtcaaaat catgaatatt tcaaaataaa ctggggagtt 1500
ataaaaatac aactagagat ataaaaaaaa aaaaaaaaaa aaaaaaaaa 1549

<210> 109
<211> 3055
<212> DNA
<213> mouse

<400> 109
ggggcgccag ggtctgcacc gaaacatggc agaggttggg tccaagtcag tgctgttcgt 60
gtgtctcggt aacatttgcc ggtcacccat tgcagaagca gtattcagga aactggtaac 120
tgatgaaaag gtttcagata attggaggat agacagtgcg gctacatcca cctatgaagt 180
ggggaaccct cctgactatc gagggcagaa ctgcatgaga aaacatggca tccacatgca 240
gcacattgca cggcagatta caaaagaaga ctttgccaca ttcgattata tactatgtat 300
ggatgaaagc aatctgagag atctcaatag aaaaagtaat caagttaaaa actgcaaagc 360
taaaattgag ctacttggga gctatgatcc acagaaacag ctcatcattg aagatcccta 420
ttatggcaat gactctgact tcgaggtggt gtaccagcaa tgccttaggt gctgcaaggc 480
cttcctggag aagacttact agctggtcct aagccccacc attgagcagc tcactcatca 540
gtgctgtgcc caagggtggt ggcagtcctt agccccatac cccacctctc ttttcagctg 600
acttactgta tatctttaaa ataattgtag gtgggaatta ggcatatgtt cagaaggata 660
aaagcatttg agtcagacag tttgaggtgt ggctaagcat tcttagacta actaaacctc 720
tgaccttgcg gtgattacaa aacagtggaa caagcaaata tggaacaaaa gaaaacaaaa 780
acaaaaacaa aaacccagaa agtaagagtg acctagaagg tccatatcag cctctgagct 840
cggcaagcct gggtcgtctg gtctaagtgg agtgtgtgca tgacccgcac ccagtgtgcc 900
gtttgcttgc cgtgtactct cttctctagg ctctcgttgt gacaatagct ccattcacgg 960
cagccttcca gttaacactg gcagtttaag ctcagacaca ctgagggtgt tgagggattt 1020
gagagaggag acgctggatg tgctgatggg cctgggactc cagcaggccg tcctgggctg 1080
gacagtgaca ccctgtctaa aaggggaaca aaaataacaa cggatgggag cagcagatgg 1140
ataaagttac catcttcagt cttacttggt ttttgtctac tttcctgagc tttgttcttg 1200

tttgagccac tttgctttaa aaaaaaagta agtgtgctta aatactggta tgtgtttgta 1260
agtgttctct catgggcaat ttacaaagtt ataggcaagc aagagtaatt tttgtgtatt 1320
ttcagaaaag agacctcaaa tttatatgaa tgtatgtcag aaaggaacat gaattcaact 1380
gggaagttat caaatacaac tgagatacaa atccagtgtc tgcctgcttc ttactgacaa 1440
gtgaacaaga tacctaaatg ttgcctccat gtgccttttt atttgcttga gtgtgaagtt 1500
tgggctctca gcctctgtgt tagaaagtaa agtgatgaga tggatacaca caatgctgtg 1560
ttggatggcg atgggtgtct aattgagaga ctccaggcac tatccttatc cttgggcctt 1620
cttcatgtag ctggtgctcc ctacccgctt cccagctgaa aaggtggtaa ctgtctgtct 1680
tagcctgtct tagctcaggt cactactggt gagctccagt cacgcagaac ttgtagttag 1740
aagagccatc ctgacgtctg aaaggaagga agcccagggg cctaggaata tgcagtctct 1800
tcttggccag ggctcctctc tcgaaggagg aaagcttaca cctgactctt ccagaagaaa 1860
gcagctatcc cagcaccctc tcacagaaag gccatgatgt cccagcaaag gcagacactt 1920
cagctgcctt tttgggctcc tgtggggttt tggtcagagt cttcacaaaa ttaaaaccga 1980
ggaagggaga ggacaggtag tcagccacaa ggaggaaatg gattaagtca aagctctcca 2040
ccctacttca gtgcttgctt cagaatctca gatttctctt tcaggtcata agatcctatt 2100
gttttcttga aattttacct gtgtatctta cacgcagatc cttggaatgt caggttctgc 2160
tttgttctac ggctgtctca aacagcacag tgtgtttata agggctcatg aacagagaaa 2220
ttaacttttt acattcaatc aaattacatt tgtttaaaat acagccatta aattttaaaa 2280
ctgttctcag ggtgcttctt ggtttgacat tctcttacaa tcaggtagag atggtagatg 2340
tggagtattg atggctaaat gagaccaagc gatcaggagt ttatgttatt cagtacttag 2400
tcacatctac acacacacac acacgcacac gtgtatatat atagtgaaca atgtgtatgt 2460
atgtttatat atacatatat tctgagcctt gacattgtta tttaagagtc attctgaaga 2520
ctgttcatta ttccaaacac ttcatttatt tccttgtatt tattcacagt atttacatat 2580
tgtacaatac ctggaatgta cttttacatt tacagaggac aagtgggact ggccttggca 2640
tagccacaga tacatatgcc actgttttca acacaactga ggtttttttg gttttcatta 2700
aaatttttca tgcagttcca aactatactt tgggatttta attgtagaat acaaaatgtc 2760
aaatcatact atatgctcta tgaaaataca gtttaatgtt ctgcctatgt ttctaaagaa 2820
atattccttg tggttccacc taatcttaaa aagaaaatac ctcattttac agaacaatac 2880
atcaaatgtg gaatgttgtc tgtttttaca atcataagag tggcaaatct cactgacaga 2940
tacactgatt cactgaatgc atatttgtaa actgtcagca acatagaaaa tgcaaaggaa 3000
ttatggaaga gtgcaaaaat aaatctctgt ccacaggaag tgggcatgaa gatgc 3055

<210> 110
<211> 1436
<212> DNA
<213> rat

<400>  110
gggggcggtt tctgggcgcc agggtctgca ccgaaacatg gcagaggttg ggtccaagtc      60

agtgctgttc gtgtgtctcg gtaacatttg ccggtcaccc attgcagaag cagtgttcag     120

aaaattggta actgatgaaa acgtttcaga taactggagg atagacagtg cggctacatc     180

cacctatgaa gtggggaacc cccctgacta tcgagggcag aactgcatga aaaaacatgg     240

catccacatg caacacattg cacggcagat tacaagagaa gactttgcca cattcgatta     300

tatactgtgt atggatgaaa gcaatctgag agatctgaat agaaaaagta atcaagttaa     360

aaactgcaaa gctaaaatcg agctacttgg gagctatgat ccacagaagc agctcattat     420

tgaagatccc tattatggca atgactctga cttcgaggtg gtgtaccagc aatgccttag     480

gtgctgcaag gccttcctgg agaagactca ctagctggtc ctaaccacca ccactgagca     540

acccactcct cagtgctgtg cccaagggtg gtggcagtcc ttagccccat accccacctc     600

tcttttcagc tgacttactg tatatcttta aaataattgt aggtggaaat caggcatttg     660

ttcagaagga taaaaacatt tgaggcagac atttgaggtg tggctcagta ttcttagact     720

aacaaaagct ctggcctcgc cataattaca aaatagtgga acgagcaact gtggaacaaa     780

agaaaaccca ataaagtaag aatgacccac aaggtccaga tcagcctttg agcccagcga     840

gcctgggtag tctggtctaa ctggagtgtg agcatggcca gcacccagtg tgctgtttgc     900

ttgccttaca ctctctattc tatattgtct cttattgtga caatatctcc atccatggca     960

gccttccatt taacactggg agagtttaaa cccagacaca ccgagggttc agatatttga    1020

gagaggagaa cctggatgtg atgatgggcc tggaactcca gcaggccatc ctgggctgta    1080

cagtgactcc ctgtctgaaa gataaaaatg ctaacagatg ggagcaacag gttggtgaag    1140

ttaacacctt taatctcact tggtttttgt gtagtttctt gagcttggtt cctatttgag    1200

ccactttgct tctttaaaga gaaagtaagt gtgcttaaat agtggtgtgc gtttgtaagt    1260

gttctctcat caacatttta caaagttaca ggcaagcatg agtaatttta gtgtattttc    1320

agaaaaggga cctcaaattt atgtggatat atgtcagaaa agattgtgaa ttaaactggg    1380

atgttatcaa atacaactaa aaatacaaaa aaaaaaaaaa aaaaaaaaaa aaaaaa        1436

<210>  111
<211>  5153
<212>  DNA
<213>  human

<400>  111
gggcgcagag gaggaaaggg agcaggcgca gggggactgg aaaggcagca tgcgctcgcc      60

aggagcaacc tcggcgccca gggtctgagg ctgcagcccc agttcgccat tgtgagccgc     120

cgccggggga gtccgctagc gcagccgtgc ccccgagtcc ccgtccgcgc agcgatgggg     180

cacctgccca cggggataca cggcgcccgc cgcctcctgc ctctgctctg gctctttgtg     240

ctgttcaaga atgctacagc tttccatgta actgtccaag atgataataa catcgttgtc     300

tcattagaag cttcagacgt catcagtcca gcatctgtgt atgttgtgaa gataactggt     360

gaatccaaaa attatttctt cgaatttgag gaattcaaca gcactttgcc tcctcctgtt 420
attttcaagg ccagttatca tggcctttat tatataatca ctctggtagt ggtaaatgga 480
aatgtggtga ccaagccatc cagatcaatc actgtgttaa caaaacctct acctgtaacc 540
agtgtttcca tatatgacta taaaccttct cctgaaacag gagtcctgtt tgaaatacat 600
tatccagaaa aatataacgt tttcacaaga gtgaacatta gctactggga aggtaaagac 660
ttccggacaa tgctatataa agatttcttt aagggaaaaa cagtatttaa tcactggctg 720
ccaggaatgt gttatagtaa tatcaccttt cagctggtat ctgaggcaac ttttaataaa 780
agtacccttg ttgagtacag tggtgtcagt cacgaaccca aacagcacag aactgcccct 840
tatccacctc aaaatatttc cgttcgtatc gtaaacttga acaaaaacaa ctgggaagaa 900
cagagtggca atttcccaga agaatccttc atgagatcac aagatacaat aggaaaagaa 960
aaactcttcc attttacaga agaaacccct gaaattccct cgggcaacat ttcttccggt 1020
tggcctgatt ttaatagcag tgactatgaa actacgtctc agccatattg gtgggacagt 1080
gcatctgcag ctcctgaaag tgaagatgaa tttgtcagcg tacttcccat ggaatacgaa 1140
aataacagta cactcagtga gacagagaag tcaacatcag gctctttctc ctttttccct 1200
gtgcaaatga tattgacctg gttaccaccc aaaccaccca ctgcttttga tgggttccat 1260
atccatattg aacgagaaga gaactttact gaatatttga tggtggatga agaagcacat 1320
gaatttgttg cagaactgaa ggaacctggg aaatataagt tatctgtgac aacctttagt 1380
tcctcaggat cttgtgaaac tcgaaaaagt cagtcagcaa aatcactcag cttttatatc 1440
agtccttcag gagagtggat tgaagaactg accgagaagc cgcagcacgt gagtgtccac 1500
gttttaagct caaccactgc cttgatgtcc tggacatctt cccaagagaa ctacaacagc 1560
accattgtgt ctgtggtgtc gctgacctgc cagaaacaaa aggagagcca gaggcttgaa 1620
aagcagtact gcactcaggt gaactcaagc aaacctatta ttgaaaatct ggttcctggt 1680
gcccagtacc aggttgtaat atacctaagg aaaggccctt tgattggacc accttcagat 1740
cctgtgacat ttgctattgt tcccacagga ataaaggatt taatgctcta tcctttgggt 1800
cctacggccg tggttctgag ctggaccaga ccttatttag gcgtgttcag aaaatacgtg 1860
gttgaaatgt tttatttcaa ccctgctaca atgacatcag agtggaccac ctactatgaa 1920
atagcagcaa ctgtttcctt aactgcatcc gtgagaatag ctaatctgct gccagcatgg 1980
tactacaact tccgggttac catggtgacg tggggagatc cagaattgag ctgctgtgac 2040
agctctacca tcagcttcat aacagcccca gtggctccgg aaatcacttc tgtggaatat 2100
ttcaacagtc tgttatatat cagttggaca tatggggatg atacaacgga cttgtcccat 2160
tctagaatgc ttcactggat ggtggttgca gaaggaaaaa agaaaattaa aaagagtgta 2220
acacgcaatg tcatgactgc aattctcagc ttgcctccag gcgacatcta taacctctca 2280
gtaactgctt gtactgaaag aggaagtaat acctccatgc tccgccttgt caagctagaa 2340
ccagctccac ccaaatcact cttcgcagtg aacaaaaccc agacttcagt gactttgctg 2400

tgggtggaag agggagtagc tgatttcttt gaagttttct gtcaacaagt tggctccagt 2460
cagaaaacca aacttcagga accagttgct gtttcttccc atgtcgtgac catctccagc 2520
cttcttcctg ccactgccta caattgtagt gtcaccagct ttagccatga cagccccagt 2580
gtccctacgt tcatagccgt ctcaacaatg gttacagaga tgaatcccaa tgtggtagtg 2640
atctccgtgc tggccatcct tagcacactt ttaattggac tgttgcttgt taccctcatt 2700
attcttagga aaaagcatct gcagatggct agggagtgtg gagctggtac atttgtcaat 2760
tttgcatcct tagagaggga tggaaagctt ccatacaact ggcgtaggag tatatttgct 2820
ttcttaaccc tgctaccctc atgtctttgg actgattatc ttttggcatt ttatattaat 2880
ccttggagta aaaatggttt aaagaagagg aaactgacaa acccggttca actggatgac 2940
tttgatgcct atattaagga tatggccaaa gactctgact ataaattttc tcttcagttt 3000
gaggagttga aattgattgg actggatatc ccacactttg ctgcagatct tccactgaat 3060
cgatgtaaaa accgttacac aaacatccta ccatatgact tcagccgtgt gagattagtc 3120
tccatgaatg aagaggaagg tgcagactac atcaatgcca actatattcc tggatacaac 3180
tcaccccagg agtatattgc cacccagggg ccactgcctg aaaccagaaa tgacttctgg 3240
aagatggtcc tgcaacaaaa gtctcagatt attgtcatgc tcactcagtg taatgagaaa 3300
aggagggtga aatgtgacca ttactggcca ttcacggaag aacctatagc ctatggagac 3360
atcactgtgg agatgatttc agaggaagag caggacgact gggcctgtag acacttccgg 3420
atcaactatg ctgacgagat gcaggatgtg atgcatttta actacactgc atggcctgat 3480
catggtgtgc ccacagcaaa tgctgcagaa agtatcctgc agtttgtaca catggtccga 3540
cagcaagcta ccaagagcaa aggtcccatg atcattcact gcagtgctgg cgtgggacgg 3600
acaggaacat tcattgccct ggacaggctc ttgcagcaca ttcgggatca tgagtttgtt 3660
gacatcttag ggctggtgtc agaaatgagg tcataccgga tgtctatggt acagacagag 3720
gagcagtaca tttttatcca tcagtgtgtg caactgatgt ggatgaagaa gaagcagcag 3780
ttctgcatca gtgatgtcat atacgagaat gttagcaagt cctagttcag aatccggagc 3840
agagaggaca tgatgtgcgc ccatcctccc ttgcttccag attgttttag tgggccctga 3900
tggtcatttt tctaaacaga ggccctgctt tgtaatatgt ggccaaggag ataatttatc 3960
tcacagaagc accgggaaga cttagcctta aagagcctac agtgtccttt tggactcttt 4020
cacttcggga catttaataa tggaccaaat tcaacagaac accaggaagg tcaagacgct 4080
ctccaaaggg caggaagtac agcacttccg aagagtttag ttggcccttt gctggttggg 4140
ctgagttttt tatttttaag tgtttgtttt tcagtgcaat aatttttgtg tgtgtgtgat 4200
tcttatcaga aagttgaatt gttttctgcc tacaccgttc atcagcccca taacccagga 4260
aggaacaggc attgttagca tcagattata cctcattatt aaaaggaggc atggccacac 4320
atgaagaaat ggtcattcta cttcaaagaa attgagccag cactatctgt actccaacat 4380
taccggatct ggattgggga ggttggtcag ggaagagagg ggttctaccc acagatcaac 4440

tgtgtaatct tttactattc aagctataat tcagcttcaa agtagagtag aaaaaaaatt 4500
gtcttaactg ttctagttct tgatggtttt cttccttatt aacagttggt gtttcttcct 4560
tggccctttt ggactaatgt tactgtccaa gttctttctc aagaaaccac atctggttca 4620
gaagagtgtc aagttggact ctttgaactc tgttgctgtc tgagcaatcg tggtgcctag 4680
actttgcatt ccttgttctg ttgacctgca tacatgtgag agctatttct ttaagaacta 4740
tataggctgt gaaaacgcac tttctttccc ccaaagagct gggaatttat gaagttatgg 4800
caatgaactg cagcatgctg ggacaattat ttgactactt ttttttgtaa tattgtcaaa 4860
tgtctctatg gattctgaca gagatttctt tttgttttgt tattcttttg gttgtcagtt 4920
tcattttaac gagtgtaact agtaacattt tattctttgg attttgtata attacagtac 4980
atgattgtgt attgtgacat gaatgctgtc aaaatgacat tgatggcatt gtgaagcctg 5040
ttactttgtg tcacttcctg ataaataaga ggtgatgaca tggatataca acagaaaaca 5100
ctttgagttg aaagtaaaca caagctggct gcttccctgt ggcaactgtg gct 5153

<210> 112
<211> 5069
<212> DNA
<213> human

<400> 112
gggcgcagag gaggaaaggg agcaggcgca gggggactgg aaaggcagca tgcgctcgcc 60
aggagcaacc tcggcgccca gggtctgagg ctgcagcccc agttcgccat tgtgagccgc 120
cgccggggga gtccgctagc gcagccgtgc ccccgagtcc ccgtccgcgc agcgatgggg 180
cacctgccca cggggataca cggcgcccgc cgcctcctgc ctctgctctg gctctttgtg 240
ctgttcaaga atgctacagc tttccatgta actgtccaag atgataataa catcgttgtc 300
tcattagaag cttcagacgt catcagtcca gcatctgtgt atgttgtgaa gataactggt 360
gaatccaaaa attatttctt cgaatttgag gaattcaaca gcactttgcc tcctcctgtt 420
attttcaagg ccagttatca tggcctttat tatataatca ctctggtagt ggtaaatgga 480
aatgtggtga ccaagccatc cagatcaatc actgtgttaa caaaacctct acctgtaacc 540
agtgtttcca tatatgacta taaaccttct cctgaaacag gagtcctgtt tgaaatacat 600
tatccagaaa aatataacgt tttcacaaga gtgaacatta gctactggga aggtaaagac 660
ttccggacaa tgctatataa agatttcttt aagggaaaaa cagtatttaa tcactggctg 720
ccaggaatgt gttatagtaa tatcaccttt cagctggtat ctgaggcaac ttttaataaa 780
agtacccttg ttgagtacag tggtgtcagt cacgaaccca aacagcacag aactgcccct 840
tatccacctc aaaatatttc cgttcgtatc gtaaacttga acaaaaacaa ctgggaagaa 900
cagagtggca atttcccaga agaatccttc atgagatcac aagatacaat aggaaaagaa 960
aaactcttcc attttacaga agaaacccct gaaattccct cgggcaacat ttcttccggt 1020
tggcctgatt ttaatagcag tgactatgaa actacgtctc agccatattg gtgggacagt 1080

gcatctgcag ctcctgaaag tgaagatgaa tttgtcagcg tacttcccat ggaatacgaa 1140
aataacagta cactcagtga gacagagaag tcaacatcag gctctttctc ctttttccct 1200
gtgcaaatga tattgacctg gttaccaccc aaaccaccca ctgcttttga tgggttccat 1260
atccatattg aacgagaaga gaactttact gaatatttga tggtggatga agaagcacat 1320
gaatttgttg cagaactgaa ggaacctggg aaatataagt tatctgtgac aacctttagt 1380
tcctcaggat cttgtgaaac tcgaaaaagt cagtcagcaa aatcactcag cttttatatc 1440
agtccttcag gagagtggat tgaagaactg accgagaagc cgcagcacgt gagtgtccac 1500
gttttaagct caaccactgc cttgatgtcc tggacatctt cccaagagaa ctacaacagc 1560
accattgtgt ctgtggtgtc gctgacctgc cagaaacaaa aggagagcca gaggcttgaa 1620
aagcagtact gcactcaggt gaactcaagc aaacctatta ttgaaaatct ggttcctggt 1680
gcccagtacc aggttgtaat atacctaagg aaaggccctt tgattggacc accttcagat 1740
cctgtgacat ttgctattgt tcccacagga ataaaggatt taatgctcta tcctttgggt 1800
cctacggccg tggttctgag ctggaccaga ccttatttag gcgtgttcag aaaatacgtg 1860
gttgaaatgt tttatttcaa ccctgctaca atgacatcag agtggaccac ctactatgaa 1920
atagcagcaa ctgtttcctt aactgcatcc gtgagaatag ctaatctgct gccagcatgg 1980
tactacaact tccgggttac catggtgacg tggggagatc cagaattgag ctgctgtgac 2040
agctctacca tcagcttcat aacagcccca gtggctccgg aaatcacttc tgtggaatat 2100
ttcaacagtc tgttatatat cagttggaca tatggggatg atacaacgga cttgtcccat 2160
tctagaatgc ttcactggat ggtggttgca gaaggaaaaa agaaaattaa aaagagtgta 2220
acacgcaatg tcatgactgc aattctcagc ttgcctccag gcgacatcta taacctctca 2280
gtaactgctt gtactgaaag aggaagtaat acctccatgc tccgccttgt caagctagaa 2340
ccagctccac ccaaatcact cttcgcagtg aacaaaaccc agacttcagt gactttgctg 2400
tgggtggaag agggagtagc tgatttcttt gaagttttct gtcaacaagt tggctccagt 2460
cagaaaacca aacttcagga accagttgct gtttcttccc atgtcgtgac catctccagc 2520
cttcttcctg ccactgccta caattgtagt gtcaccagct ttagccatga cagccccagt 2580
gtccctacgt tcatagccgt ctcaacaatg gttacagaga tgaatcccaa tgtggtagtg 2640
atctccgtgc tggccatcct tagcacactt ttaattggac tgttgcttgt taccctcatt 2700
attcttagga aaaagcatct gcagatggct agggagtgtg gagctggtac atttgtcaat 2760
tttgcatcct tagagaggga tggaaagctt ccatacaact ggagtaaaaa tggtttaaag 2820
aagaggaaac tgacaaaccc ggttcaactg gatgactttg atgcctatat taaggatatg 2880
gccaaagact ctgactataa attttctctt cagtttgagg agttgaaatt gattggactg 2940
gatatcccac actttgctgc agatcttcca ctgaatcgat gtaaaaaccg ttacacaaac 3000
atcctaccat atgacttcag ccgtgtgaga ttagtctcca tgaatgaaga ggaaggtgca 3060
gactacatca atgccaacta tattcctgga tacaactcac cccaggagta tattgccacc 3120

caggggccac tgcctgaaac cagaaatgac ttctggaaga tggtcctgca acaaaagtct 3180
cagattattg tcatgctcac tcagtgtaat gagaaaagga gggtgaaatg tgaccattac 3240
tggccattca cggaagaacc tatagcctat ggagacatca ctgtggagat gatttcagag 3300
gaagagcagg acgactgggc ctgtagacac ttccggatca actatgctga cgagatgcag 3360
gatgtgatgc attttaacta cactgcatgg cctgatcatg gtgtgcccac agcaaatgct 3420
gcagaaagta tcctgcagtt tgtacacatg gtccgacagc aagctaccaa gagcaaaggt 3480
cccatgatca ttcactgcag tgctggcgtg ggacggacag gaacattcat tgccctggac 3540
aggctcttgc agcacattcg ggatcatgag tttgttgaca tcttagggct ggtgtcagaa 3600
atgaggtcat accggatgtc tatggtacag acagaggagc agtacatttt tatccatcag 3660
tgtgtgcaac tgatgtggat gaagaagaag cagcagttct gcatcagtga tgtcatatac 3720
gagaatgtta gcaagtccta gttcagaatc cggagcagag aggacatgat gtgcgcccat 3780
cctcccttgc ttccagattg ttttagtggg ccctgatggt catttttcta aacagaggcc 3840
ctgctttgta atatgtggcc aaggagataa tttatctcac agaagcaccg ggaagactta 3900
gccttaaaga gcctacagtg tccttttgga ctctttcact tcgggacatt taataatgga 3960
ccaaattcaa cagaacacca ggaaggtcaa gacgctctcc aaagggcagg aagtacagca 4020
cttccgaaga gtttagttgg ccctttgctg gttgggctga gtttttattt tttaagtgtt 4080
tgtttttcag tgcaataatt tttgtgtgtg tgtgattctt atcagaaagt tgaattgttt 4140
tctgcctaca ccgttcatca gccccataac ccaggaagga acaggcattg ttagcatcag 4200
attatacctc attattaaaa ggaggcatgg ccacacatga agaaatggtc attctacttc 4260
aaagaaattg agccagcact atctgtactc caacattacc ggatctggat tggggaggtt 4320
ggtcagggaa gagaggggtt ctacccacag atcaactgtg taatctttta ctattcaagc 4380
tataattcag cttcaaagta gagtagaaaa aaaattgtct taactgttct agttcttgat 4440
ggttttcttc cttattaaca gttggtgttt cttccttggc ccttttggac taatgttact 4500
gtccaagttc tttctcaaga aaccacatct ggttcagaag agtgtcaagt tggactcttt 4560
gaactctgtt gctgtctgag caatcgtggt gcctagactt tgcattcctt gttctgttga 4620
cctgcataca tgtgagagct atttctttaa gaactatata ggctgtgaaa acgcactttc 4680
tttcccccaa agagctggga atttatgaag ttatggcaat gaactgcagc atgctgggac 4740
aattatttga ctactttttt ttgtaatatt gtcaaatgtc tctatggatt ctgacagaga 4800
tttctttttg ttttgttatt cttttggttg tcagtttcat tttaacgagt gtaactagta 4860
acattttatt ctttggattt tgtataatta cagtacatga ttgtgtattg tgacatgaat 4920
gctgtcaaaa tgacattgat ggcattgtga agcctgttac tttgtgtcac ttcctgataa 4980
ataagaggtg atgacatgga tatacaacag aaaacacttt gagttgaaag taaacacaag 5040
ctggctgctt ccctgtggca actgtggct 5069

<210> 113

<211>  4064
<212>  DNA
<213>  human

<400>  113
aagtaaaaaa atggaagggg gaaaaggcaa agtattctgc cttccaagtt agatagctat      60

ctttatgaaa ggacatattt caaaggcaat ataaagtctt tatggtgctc ttacctacct     120

atatttgaat tgggggatgc ttcacctgct tattagtgaa agaagaaaga atacacttag     180

taaattaaat tttttttaaa actttgtctt tgctcagaac cagctccacc caaatcactc     240

ttcgcagtga acaaaaccca gacttcagtg actttgctgt gggtggaaga gggagtagct     300

gatttctttg aagttttctg tcaacaagtt ggctccagtc agaaaaccaa acttcaggaa     360

ccagttgctg tttcttccca tgtcgtgacc atctccagcc ttcttcctgc cactgcctac     420

aattgtagtg tcaccagctt tagccatgac agccccagtg tccctacgtt catagccgtc     480

tcaacaatgg ttacagagat gaatcccaat gtggtagtga tctccgtgct ggccatcctt     540

agcacacttt taattggact gttgcttgtt accctcatta ttcttaggaa aaagcatctg     600

cagatggcta gggagtgtgg agctggtaca tttgtcaatt ttgcatcctt agagagggat     660

ggaaagcttc catacaactg gcgtaggagt atatttgctt tcttaaccct gctaccctca     720

tgtctttgga ctgattatct tttggcattt tatattaatc cttggagtaa aaatggttta     780

aagaagagga aactgacaaa cccggttcaa ctggatgact ttgatgccta tattaaggat     840

atggccaaag actctgacta taaattttct cttcagtttg aggagttgaa attgattgga     900

ctggatatcc cacactttgc tgcagatctt ccactgaatc gatgtaaaaa ccgttacaca     960

aacatcctac catatgactt cagccgtgtg agattagtct ccatgaatga agaggaaggt    1020

gcagactaca tcaatgccaa ctatattcct ggatacaact caccccagga gtatattgcc    1080

acccaggggc cactgcctga aaccagaaat gacttctgga agatggtcct gcaacaaaag    1140

tctcagatta ttgtcatgct cactcagtgt aatgagaaaa ggagggtgaa atgtgaccat    1200

tactggccat tcacggaaga acctatagcc tatggagaca tcactgtgga gatgatttca    1260

gaggaagagc aggacgactg ggcctgtaga cacttccgga tcaactatgc tgacgagatg    1320

caggatgtga tgcattttaa ctacactgca tggcctgatc atggtgtgcc cacagcaaat    1380

gctgcagaaa gtatcctgca gtttgtacac atggtccgac agcaagctac caagagcaaa    1440

ggtcccatga tcattcactg cagtgctggc gtgggacgga caggaacatt cattgccctg    1500

gacaggctct tgcagcacat tcgggatcat gagtttgttg acatcttagg gctggtgtca    1560

gaaatgaggt cataccggat gtctatggta cagacagagg agcagtacat ttttatccat    1620

cagtgtgtgc aactgatgtg gatgaagaag aagcagcagt tctgcatcag tgatgtcata    1680

tacgagaatg ttagcaagtc ctagttcaga atccggagca gtaagtggag aagagctctc    1740

cacgagtgtt cagtcttaga actattagag ggggatgtga tggatgggac aaaagacagc    1800

agtagccatt tgtattgact ctgacttcac atgagtattt cttcccagct cctggcctca    1860

catgggtggt cacagggaga aacaagtact gtagcagatc tttgagtttc aatgcacctt    1920

ggttggtgtt ggcaacaaac aggagcttct gcacatcatg atgactttac ctccattccc 1980
tctacccaga accctaaatt attctgttat tggatctgtg cctgtaatat caggatacta 2040
ccatccaaat ctatctagat atcttgtgca aatattttat tgattacaag ggctgtatac 2100
ggggttacaa aaagacatga gctggattgg acactcctaa aaccaaaaac aacagtgaca 2160
atagagtctc aggatagcag agtctgaaaa taccttggtc tcccccatta tgtgagccct 2220
tatttgagag cttcatgaag acacttgtaa catattcaac gtcctgtact ccagttttga 2280
ctacgagatc agattttgga ggtgtaagta atcttggctg aggtaacagt aatctgccca 2340
tttagttcag taaacagact gtagagtgtc tgactatact gggggaagag gagccaatga 2400
cagggtaggg agatgacagt ggtgcatgat gggagaatca tgaaagtctg ggaagcatat 2460
ggaccagtgg gaaggatagg attagcactg cacctctaca tccatcaaga gagacagagc 2520
gcacaatgga gcaggaagga taaaaaataa agaaagacag agacaaacaa atttgctggg 2580
agagggaaag acattcagac ccacagaaag gaggcagcac acagagaaag atgcacagat 2640
caacccacat ctgcatgggt tgtgagactg agcatagtga gagcagtggt ggagcaatag 2700
aagattgctg tgacaccagg ctgcttactg tctttctttg tctcattcac aggagaggac 2760
atgatgtgcg cccatcctcc cttgcttcca gattgtttta gtgggccctg atggtcattt 2820
ttctaaacag aggccctgct ttgtaatatg tggccaagga gataatttat ctcacagaag 2880
caccgggaag acttagcctt aaagagccta cagtgtcctt ttggactctt tcacttcggg 2940
acatttaata atggaccaaa ttcaacagaa caccaggaag gtcaagacgc tctccaaagg 3000
gcaggaagta cagcacttcc gaagagttta gttggccctt tgctggttgg gctgagtttt 3060
ttatttttaa gtgtttgttt ttcagtgcaa taatttttgt gtgtgtgtga ttcttatcag 3120
aaagttgaat tgttttctgc ctacaccgtt catcagcccc ataacccagg aaggaacagg 3180
cattgttagc atcagattat acctcattat taaaaggagg catggccaca catgaagaaa 3240
tggtcattct acttcaaaga aattgagcca gcactatctg tactccaaca ttaccggatc 3300
tggattgggg aggttggtca gggaagagag gggttctacc cacagatcaa ctgtgtaatc 3360
ttttactatt caagctataa ttcagcttca aagtagagta gaaaaaaaat tgtcttaact 3420
gttctagttc ttgatggttt tcttccttat taacagttgg tgtttcttcc ttggcccttt 3480
tggactaatg ttactgtcca agttctttct caagaaacca catctggttc agaagagtgt 3540
caagttggac tctttgaact ctgttgctgt ctgagcaatc gtggtgccta gactttgcat 3600
tccttgttct gttgacctgc atacatgtga gagctatttc tttaagaact atataggctg 3660
tgaaaacgca ctttctttcc cccaaagagc tgggaattta tgaagttatg gcaatgaact 3720
gcagcatgct gggacaatta tttgactact tttttttgta atattgtcaa atgtctctat 3780
ggattctgac agagatttct ttttgttttg ttattctttt ggttgtcagt ttcattttaa 3840
cgagtgtaac tagtaacatt ttattctttg gattttgtat aattacagta catgattgtg 3900
tattgtgaca tgaatgctgt caaaatgaca ttgatggcat tgtgaagcct gttactttgt 3960

gtcacttcct gataaataag aggtgatgac atggatatac aacagaaaac actttgagtt 4020

gaaagtaaac acaagctggc tgcttccctg tggcaactgt ggct 4064

<210> 114
<211> 3980
<212> DNA
<213> human

<400> 114
aagtaaaaaa atggaagggg gaaaaggcaa agtattctgc cttccaagtt agatagctat 60

ctttatgaaa ggacatattt caaaggcaat ataaagtctt tatggtgctc ttacctacct 120

atatttgaat tgggggatgc ttcacctgct tattagtgaa agaagaaaga atacacttag 180

taaattaaat tttttttaaa actttgtctt tgctcagaac cagctccacc caaatcactc 240

ttcgcagtga acaaaaccca gacttcagtg actttgctgt gggtggaaga gggagtagct 300

gatttctttg aagttttctg tcaacaagtt ggctccagtc agaaaaccaa acttcaggaa 360

ccagttgctg tttcttccca tgtcgtgacc atctccagcc ttcttcctgc cactgcctac 420

aattgtagtg tcaccagctt tagccatgac agccccagtg tccctacgtt catagccgtc 480

tcaacaatgg ttacagagat gaatcccaat gtggtagtga tctccgtgct ggccatcctt 540

agcacacttt taattggact gttgcttgtt accctcatta ttcttaggaa aaagcatctg 600

cagatggcta gggagtgtgg agctggtaca tttgtcaatt ttgcatcctt agagagggat 660

ggaaagcttc catacaactg gagtaaaaat ggtttaaaga agaggaaact gacaaacccg 720

gttcaactgg atgactttga tgcctatatt aaggatatgg ccaaagactc tgactataaa 780

ttttctcttc agtttgagga gttgaaattg attggactgg atatcccaca ctttgctgca 840

gatcttccac tgaatcgatg taaaaaccgt tacacaaaca tcctaccata tgacttcagc 900

cgtgtgagat tagtctccat gaatgaagag gaaggtgcag actacatcaa tgccaactat 960

attcctggat acaactcacc ccaggagtat attgccaccc aggggccact gcctgaaacc 1020

agaaatgact tctggaagat ggtcctgcaa caaaagtctc agattattgt catgctcact 1080

cagtgtaatg agaaaaggag ggtgaaatgt gaccattact ggccattcac ggaagaacct 1140

atagcctatg gagacatcac tgtggagatg atttcagagg aagagcagga cgactgggcc 1200

tgtagacact tccggatcaa ctatgctgac gagatgcagg atgtgatgca ttttaactac 1260

actgcatggc ctgatcatgg tgtgcccaca gcaaatgctg cagaaagtat cctgcagttt 1320

gtacacatgg tccgacagca agctaccaag agcaaaggtc ccatgatcat tcactgcagt 1380

gctggcgtgg gacggacagg aacattcatt gccctggaca ggctcttgca gcacattcgg 1440

gatcatgagt ttgttgacat cttagggctg gtgtcagaaa tgaggtcata ccggatgtct 1500

atggtacaga cagaggagca gtacattttt atccatcagt gtgtgcaact gatgtggatg 1560

aagaagaagc agcagttctg catcagtgat gtcatatacg agaatgttag caagtcctag 1620

ttcagaatcc ggagcagtaa gtggagaaga gctctccacg agtgttcagt cttagaacta 1680

ttagaggggg atgtgatgga tgggacaaaa gacagcagta gccatttgta ttgactctga 1740
cttcacatga gtatttcttc ccagctcctg gcctcacatg ggtggtcaca gggagaaaca 1800
agtactgtag cagatctttg agtttcaatg caccttggtt ggtgttggca acaaacagga 1860
gcttctgcac atcatgatga ctttacctcc attccctcta cccagaaccc taaattattc 1920
tgttattgga tctgtgcctg taatatcagg atactaccat ccaaatctat ctagatatct 1980
tgtgcaaata ttttattgat tacaagggct gtatacgggg ttacaaaaag acatgagctg 2040
gattggacac tcctaaaacc aaaaacaaca gtgacaatag agtctcagga tagcagagtc 2100
tgaaaatacc ttggtctccc ccattatgtg agcccttatt tgagagcttc atgaagacac 2160
ttgtaacata ttcaacgtcc tgtactccag ttttgactac gagatcagat tttggaggtg 2220
taagtaatct tggctgaggt aacagtaatc tgcccattta gttcagtaaa cagactgtag 2280
agtgtctgac tatactgggg gaagaggagc caatgacagg gtagggagat gacagtggtg 2340
catgatggga gaatcatgaa agtctgggaa gcatatggac cagtgggaag gataggatta 2400
gcactgcacc tctacatcca tcaagagaga cagagcgcac aatggagcag gaaggataaa 2460
aaataaagaa agacagagac aaacaaattt gctgggagag ggaaagacat tcagacccac 2520
agaaaggagg cagcacacag agaaagatgc acagatcaac ccacatctgc atgggttgtg 2580
agactgagca tagtgagagc agtggtggag caatagaaga ttgctgtgac accaggctgc 2640
ttactgtctt tctttgtctc attcacagga gaggacatga tgtgcgccca tcctcccttg 2700
cttccagatt gttttagtgg gccctgatgg tcatttttct aaacagaggc cctgctttgt 2760
aatatgtggc caaggagata atttatctca cagaagcacc gggaagactt agccttaaag 2820
agcctacagt gtccttttgg actctttcac ttcgggacat ttaataatgg accaaattca 2880
acagaacacc aggaaggtca agacgctctc caaagggcag gaagtacagc acttccgaag 2940
agtttagttg gccctttgct ggttgggctg agttttttat ttttaagtgt ttgtttttca 3000
gtgcaataat ttttgtgtgt gtgtgattct tatcagaaag ttgaattgtt ttctgcctac 3060
accgttcatc agccccataa cccaggaagg aacaggcatt gttagcatca gattatacct 3120
cattattaaa aggaggcatg gccacacatg aagaaatggt cattctactt caaagaaatt 3180
gagccagcac tatctgtact ccaacattac cggatctgga ttggggaggt tggtcaggga 3240
agagaggggt tctacccaca gatcaactgt gtaatctttt actattcaag ctataattca 3300
gcttcaaagt agagtagaaa aaaaattgtc ttaactgttc tagttcttga tggttttctt 3360
ccttattaac agttggtgtt tcttccttgg cccttttgga ctaatgttac tgtccaagtt 3420
ctttctcaag aaaccacatc tggttcagaa gagtgtcaag ttggactctt tgaactctgt 3480
tgctgtctga gcaatcgtgg tgcctagact ttgcattcct tgttctgttg acctgcatac 3540
atgtgagagc tatttcttta agaactatat aggctgtgaa aacgcacttt ctttccccca 3600
aagagctggg aatttatgaa gttatggcaa tgaactgcag catgctggga caattatttg 3660
actacttttt tttgtaatat tgtcaaatgt ctctatggat tctgacagag atttcttttt 3720

gttttgttat tcttttggtt gtcagtttca ttttaacgag tgtaactagt aacattttat 3780

tctttggatt ttgtataatt acagtacatg attgtgtatt gtgacatgaa tgctgtcaaa 3840

atgacattga tggcattgtg aagcctgtta ctttgtgtca cttcctgata aataagaggt 3900

gatgacatgg atatacaaca gaaaacactt tgagttgaaa gtaaacacaa gctggctgct 3960

tccctgtggc aactgtggct 3980

<210> 115
<211> 5075
<212> DNA
<213> mouse

<400> 115
cattaaacaa cgccggctcc aagcacgttc tagcaggact cgggcacaga agaggagagc 60

gatccatccg ggggcacagg acgacctgcc aggcgacatg cggccgcccg gagccgcccg 120

ggcgcccgaa gtctgaggct gccagccggg actggtcatt gtaagcgcca cggagaactg 180

ctggcgctgc cgttccctcc ccggtccctg gtcgcgccgc gatggggcac ctgcctaggg 240

gaacgctcgg gggccgccgc ctgctacctc tgctcgggct ctttgtgctg ctcaagattg 300

ttacgacgtt ccacgtggct gtgcaagatg acaacaatat cgttgtgtct ttagaagctt 360

ctgatatagt cagcccagca tctgtgtatg ttgtgagggt agctggcgaa tcaaaaaact 420

atttcttcga atttgaggaa tttaacagca cattgcctcc tcctgtggtc tttaaggcca 480

catatcacgg tctttattac ataatcactc tggtggtagt caacgggaat gtggtcacca 540

aaccatccag atcaatcacc gtgttgacaa aacccttgcc tgtaaccagt gtgtctatct 600

atgactataa accttctcct gagacaggag tcctgtttga aatccattat ccggaaaaat 660

acaatgtgtt cagcagagtg aacatcagct actgggaagg gagggacttc aggacgatgc 720

tgtacaaaga tttctttaag gggaaaacgg tgtttaatca ctggctacca ggactatgtt 780

acagtaacat cactttccag ctggtatcgg aggcaacttt taataaaagt acccttgtgg 840

agtacagtgg tgtgagccat gaacccaaac agcacagaac agcaccatat ccacctcgaa 900

acatctctgt tcgctttgtc aacttgaaca agaacaactg ggaggagccg agcgggagct 960

tccccgagga ctcgttcatc aaaccaccgc aagattcaat aggaagagac agacgcttcc 1020

atttccccga agaaactccg gagactcccc ccagcaatgt gtcctccggt tctcccccca 1080

gcaatgtgtc ctccgcttgg cctgacccga atagcacaga ctatgaaagc acatcacagc 1140

ccttctggtg ggacagtgca tccgcggccc ctgaaaacga ggaggacttt gtcagtgcgc 1200

tgccagcaga ctatgacact gagaccacac tcgataggac agagaagccc acagccgacc 1260

ctttctctgc cttccctgtg cagatgactc tgagctggtt accacccaaa ccgcccacag 1320

cctttgatgg cttcaatatc ctcatagaga gggaagagaa ctttactgac tatttgacag 1380

tggatgaaga agcccatgaa ttcgttgcag aactgaagga gcctgggaaa tacaaactct 1440

cagtgacaac ctttagctcc tcgggggcct gtgagactcg gaaaagccag tcagcaaaat 1500

cgctcagctt ctacatcagc cccacaggcg agtggattga agaactgacc gagaaacctc 1560

agcatgtgag tgtccacgtc ttaagctcaa ccaccgcctt gatgtcgtgg acatcttctc 1620
aggagaacta caacagcacc attgtgtctg tggtgtcctt gacctgccag aaacagaagg 1680
agagccagcg gctggagaag cagtattgta cccaggtgaa ctcaagcaaa cctgtaattg 1740
agaacctggt tcctggtgcc cagtaccagg ttgtgatgta cttaagaaaa ggccctttga 1800
ttgggccacc ttccgatcct gtgacatttg cgattgttcc cactgggatc aaagatttaa 1860
tgctctaccc cttgggtccc actgctgtcg tgctgagctg gacccgacct atcctgggag 1920
tcttcagaaa atacgtggtt gaaatgttct acttcaaccc caccaccatg acctcagagt 1980
ggacgaccta ctatgagata gcagccacag tttccttaac ggcatccgtg agaatagcaa 2040
gcctattgcc agcgtggtac tacaacttcc gcgtaaccat ggtgacatgg ggagatccag 2100
agctgagctg ttgtgacagt tccaccatca gcttcataac agcccccgtt gctccagaaa 2160
tcacgtctgt ggagtacttc aacagcctgc tgtacatcag ttggacctat ggggatgcca 2220
ccactgacct gtcccactct agaatgctgc actggatggt cgttgcagaa gggaggaaga 2280
aaattaaaaa gagtgtgaca cgcaatgtca tgacggccat ccttagcctg cctccaggag 2340
atatctacaa tctgtctgtc acggcctgca ctgagagagg gagcaacacc tccttgcccc 2400
gccttgtcaa gctcgaacca gcccctccga agtcactctt cgcagtgaac aaaacacaga 2460
cgtcagtgac cctgctgtgg gttgaggagg gtgttgctga tttctttgaa gtcttctgtc 2520
agcagctcgg ctctggccac aatggcaaac tccaggagcc agtagctgtg tcgtcccacg 2580
tggtgaccat ctccagcctc ctcccagcca ctgcctacaa ctgcagtgtc accagcttca 2640
gccacgacac tcccagtgtc cctacattca tagctgtctc cacaatggtt acagaggtga 2700
accctaacgt ggtggtgatc tcggtgctgg ccatcctcag cacactttta attggactgc 2760
tcttagtgac ccttgtcatt ctccgaaaga agcacctgca gatggctagg gaatgtggag 2820
ctggcacgtt tgttaacttt gcatccttag aaagggaagg gaaactcccc tacagttggc 2880
gtaggagtgt gtttgctctc ttaaccctgc tgccttcatg tctttggact gactatcttt 2940
tggcatttta tattaaccct tggagtaaaa atggcttaaa gaagaggaaa ctaacaaacc 3000
ccgttcagct ggatgacttc gattcttaca tcaaggatat ggccaaggac tcggactata 3060
aattctctct tcagtttgag gagttgaagt tgattggact ggatattccg cactttgctg 3120
cagatctacc gctgaaccga tgtaaaaacc gctacacaaa catcctgccg tatgacttta 3180
gccgggtgag gctagtctcc atgaacgaag aggaaggagc agactacatt aatgccaact 3240
atattcctgg atacaactca ccccaggagt acattgccac ccagggtccc ctgccagaaa 3300
ccagaaatga cttctggaag atggtcctac aacagaagtc ccacatcatc gtcatgctca 3360
ctcaatgcaa tgagaaaagg agggtaaaat gtgaccacta ctggccattc acagaagaac 3420
ccattgctta tggggacatc accgtggaga tggtctctga ggaagaggag gaggactggg 3480
ccagtagaca cttccggatc aactatgcgg acgaagcgca ggacgtgatg catttaaact 3540
acacagcctg gcccgatcac ggcgtgcctc cagcaaacgc cgccgagagc atcctgcagt 3600

ttgtgttcac agtgcgacag caagccgcca agagcaaagg gcccatgatc atccactgca 3660
gtgcgggtgt gggacggaca ggaaccttca ttgccctgga caggctcctg caacacattc 3720
gagatcatga atttgtggac atcttagggc tggtatcaga gatgcgctca caccgaatgt 3780
caatggtaca gacagaggag cagtacattt ttatccatca gtgtgtgcag ctgatgtggc 3840
tgaggaagaa gcaacagttc tgcatcagcg acgtcatcta cgagaacgtc agcaaatcct 3900
agttcggaat ctggagcggt gaggacatga tacctgcgca tcctcccttg cttccagagt 3960
ttttataggg gcttttagtc actttgctaa atcgagtccc tgctgtgcag tatatggaca 4020
aggaggagat ttacctccta gaaccaagaa gaccttaagg agcctacagt atcatttgga 4080
gtcttttcac ttctagatgt ttgatggacc aaattcagta gaattccaga aaggtcacca 4140
atgctccttg aggggcagga agcgcaatac ttcctgatgg ctcagttggc tctttgctgc 4200
tcgggttggg ttgatttttt ttttaagtgc aataatttct gtataattgt gattttttac 4260
attcacaatt cagaatgttg aattgttatc tgcccacacc atcgatcagt gccacagcct 4320
tggaaataac caacatcatt acggagtttt atctcatccc tggaaaggca tggctacaca 4380
ggaagacgtg gtgatgaatc tccttcaaag aaactgagcg agtgtgctcc tcgctccaac 4440
cttgaagcta cgggtcagga gtaggcaggc agagggaaga gagcggttct actcacccat 4500
cagccagctt atcttttcct atttcaaata tgaaaacctg tgtttcaaag tagagtagga 4560
aaaacattat atgtctgact tgtcatagtg ggtttcttcc ttgttgacag ttgggagttt 4620
cttccgtggc tcttttgaac taatgtcacg gtcctttttg aacagccaga gttgattcaa 4680
cagttttgag tctgactctg aactctgaga tatcttggtg cctagccttc acgtttattt 4740
ttctctgtgt ccacctgtgt acacaaaaac agtttctcca agagctatag gctgtaaaaa 4800
tgcactttct ttctccaccc agaggagctg ggaattcaga agtcacgaca acaacaaact 4860
cagcatgtaa ggacaggtat tggacaccat cccttaagaa cattgttgag tgtctctatg 4920
gattccgaca ggagattctc tgggtcttct ttgcatttct gttgtcagag tcattttaac 4980
ctgtgtagct agtggcatta tattctttgg attttgtatg attaaagtac atgattgtgt 5040
gttgtgacca tgaaaaaaaa aaaaaaaaaa aaaaa 5075

<210> 116
<211> 4871
<212> DNA
<213> rat

<400> 116
ttttaagcgc cacggggacc cgctggcgct gccatccctc ccgggttcct ggtcgcgcca 60
cgatggggca cctgcctagg gaaaggcgcg ggggccgccg cctgctacct ctgctcgggc 120
tctttgtgct gctcaagatt gtttcaacgt tccacgtgac tgtccgagac gacaacaaca 180
tcgttgtgtc tttagaagct tctgatgtag tcagcccaac ctccgtgtat gttgtgaggg 240
tagctggcga atccaaaaac tatttcttcg aatttgagga atccaatggc acactgcctc 300

ctcctgtggt ctttaaggcc acgtatcatg gtctttatta catcatcagt ctggtggtag 360
tcagtgggaa tgtggtcacc aaaccatccc gatcaatcac cgtgctgaca aaacccttgc 420
ccgtaaccag tgtgtctata tatgactata agccttctcc tgagacagga gtactgtttg 480
aaattcacta tccggaaaaa tacaacgtgt tcagcagagt gaacatcagc tactgggagg 540
ggaaggattt caggacgatg ctgtacaaag acttcctgaa ggggaaaacc gtgtttaatc 600
actggctccc ggggatctgc tacagtaaca tcactttcca gctggtttct gaggcgacct 660
ttaacaaaag caccctggtg gagtgcagtg gtgtgagtca cgaacccaaa caacacagaa 720
cagcgccgta tccccctcga aacatctctg ttcgctttgt caacttgaac aagaacaact 780
gggaagaacc aagcgggagc ttccccgagg actcgttcat caaaccgcaa gacgcgatag 840
gaagagacag acccttccat ttccccgagg aaacccctga gacgcccgcc agcaatgcgt 900
cttccggctg gcctgacccg aacggcactg agtatgagag cacatctcag ccctactggt 960
gggacagtgc atccgcggcc cctgaaaacg aggaggactt cgtcagcgtg ctgccagcgg 1020
actatgacac tgaggccacg ctcggcagga cagagaaacc cacagctgac cctttctctg 1080
ccttccccgt gcagatgact ctgagctggt taccaccaaa accgcctaca gccttcgatg 1140
gcttcaatat cctcatagag agggaaggga actttaccga ctatttgaca gtggatgaag 1200
aagcccacga gtttgttgcc gaactgaagg agcctggaaa gtacagactc tcagtgacaa 1260
ccttcagctc ctcgggagcc tgtgagactc ggaaaagcca gtcagcaaaa tcgctcagct 1320
tctacatcag ccccacagga gagtggattg aagaactgac tgagaaaccg cagcacgtga 1380
gtgtccacgt cttaagctca accactgcct tgttgtcgtg gacatcttcc caggagaact 1440
acaacagcac cattgtgtct gtggtgtccc tgacatgcca gaaacagaaa gagagccagc 1500
ggctggagaa gcagtattgt acccaggtga actcaagcaa acctgtaatt gagaacctgg 1560
ttcccggtgc ccagtaccag gttgtgatgt acctaagaaa aggccctttg attgggccac 1620
cttctgatcc tgtgacattt gccattgttc ccacggggat aaaggattta atgctctatc 1680
cattgggtcc cacggcagta gtgctgagct ggacccgacc ttacttggga gtgttcagaa 1740
aatacgtggt tgaaatgttt tatttcaacc ccaccaccat gacctccgag tggacgacct 1800
actacgaaat agcagcgacc gtctccttaa catcgtccgt gagaatagca aatctattgc 1860
cggcgtggta ctacaacttt cgcgtaacca tggtgacgtg gggagatcca gaactgagct 1920
gctgtgacag ttccaccatc agcttcataa cggcccccgt cgctccagaa atcacgtctg 1980
tggagtactt caacagcctg ctgtacatca gctggaccta cggggacgcc accactgacc 2040
tgtcccattc gagaatgcta cactggatgg tcgtggcaga agggaggagg aaaattaaaa 2100
agagtgtgac acgcaatgtc atgacggcca tccttagcct gcctccagga gatatctaca 2160
acctgtctgt cacagcctgc actgagaggg ggagtaacac ctccatgctc cgccttgtca 2220
agctagaacc agctcctcca aaatcactct tcgcagtgaa caaaacgcag acatcggtca 2280
ccctgctttg ggttgaggag ggcgtagctg atttctttga agtcctctgt cagaagctcg 2340

ggtctggcca cgatggcaaa ctccaggaac cggtagctgt ttcctcccac gtggtgacca 2400
tctccagcct cctcccagcc actgcctaca actgcagtgt caccagcttc agccacgaca 2460
gtcccagtgt ccctacgttc atagctgtct ccacaatggt tacagaggtg aaccctaatg 2520
tggtggtgat ctcggtgctg gccatcctca gcatactttt aattggactg ctgttagtga 2580
cccttgtcat tctgagaaag aagcacctgc agatggccag ggagtgtggg gctggcacgt 2640
ttgtcaattt tgcatccttg gagagggaag ggaaactccc ctacagttgg cgtaggagtg 2700
tctttacttt cttaaccctg ctgccctcat gtctgtggac tgactatctg ttggcatttt 2760
atattaaccc ttggagtaaa aatggcttaa agaagaggaa actgacgaac cctgttcagc 2820
tggacgattt tgattcttac atcaaggaca tggccaagga ctctgactat aaattctctc 2880
ttcagtttga ggagttgaag ttgattggac tggatattcc acactttgct gcagatcttc 2940
cgctgaaccg atgtaaaaac cgctatacaa acatcctgcc gtacgacttc agcagagtga 3000
ggctggtctc catgaatgaa gaagaaggag cagactacat taatgccaat tatattcctg 3060
gctacaactc acctcaggag tacattgcca cccagggacc actgccggaa accagaaatg 3120
atttctggaa gatggtcctg cagcagaagt ctcgcatcat tgtcatgctc actcagtgca 3180
atgagaaaag aagggtaaaa tgcgaccact actggccatt cacggaagaa cccattgctt 3240
atggagacat caccgtggag atggtctctg aggaagaaca ggaggactgg gccagcagac 3300
acttccggat caattatgct gacgaggccc aggatgtgat gcactttaac tacacggcct 3360
ggccagacca tggcgtgcct ccagcaaatg ctgccgagag catcctgcag ttcgtgtaca 3420
ctgtccggca gcaagccacc aagagcaaag gacccatgat catccactgc agcgcgggcg 3480
tgggacggac aggcaccttc atagcccttg acaggctctt gcaacacatc cgagatcacg 3540
aatttgtgga catcttgggg ctggtgtcag aaatgcgctc ataccgaatg tcgatggtac 3600
agacagagga gcagtacatc ttcatccatc agtgcgtgca gctgatgtgg ctgaagaaga 3660
agcaacagtt ctgcatcagt gacgtcatct acgagaacgt cagcaagtcc tagttcagac 3720
tcgggagcgg tgaggccatg atgtctaccc atcctccctt gcttccagag gttttatagg 3780
ggcctttagt cactttgtca aatcgcgtcc ccgctgcgca gtttgtggac aaggaggtga 3840
tttaacctcc tagaaccgag aagacttagc cttaaggagc ctacaatacc gtttggagtc 3900
ttttcacttc tagacgtttg atggtccaaa ttcactagga tttccagaaa tgctcctcct 3960
cgaagggcgg gaagtgccct tcctgatggt tcagtcggct ctttgctgct tgggctgggt 4020
ttggtttttt ctttttttta agtgcaataa tttctgtata atcgtgattt tttttttaca 4080
ttcgcaattc agaatgttga attgttatcc gcccacatca ctgatcagtg ccacagcctt 4140
gggaaggcct tatcgcagag ttttatctca ccactgaaga ggcatggcta cacacaggaa 4200
gactcggtga caaatcctca aaggaaatga gcgcgtgtgt ccctccctcc aacctctcga 4260
agctatgggt caggagtagg tgggcagaga gagaagagag gggttctact cacacgtcag 4320
ccggtttatc tcttcctatt tcgaatatga gaacctgtgt ttcgaagtag agtaggaaaa 4380

acattacttg tctgacttgt cgtagtgggt ttcttccttg ttgacagttg ggagtttctt 4440

ccgtggctct tttgaactag tgtcactgtc atggtccttt tcaaacagcc agagctgact 4500

caacagtttt gagtctgact ctgaactctg agaaatcttg gtgcctagcc ttctttattt 4560

ttctgtccac ccgtgtacac aagaacagtt tctccaagag ctataggctg taaaaatgca 4620

ctttctttcc ccacccagag gagctgggaa ttcagaagtc aggacaacga actcagcatg 4680

ctaggacagt cactggacac cgtcccttaa gaataccgtt gagcgtctcc gtggattccg 4740

acaggatatt ctctgggttc tttgcatttc tgttgtcaga ctcattttaa cccgtgtagc 4800

tagtggcatt ttattctttg gattttgtat gattaaagta catgattatg tgttgtgacc 4860

agcgccgcga a 4871

<210> 117
<211> 22
<212> DNA
<213> artificial sequence

<220>
<223> ephrin-A1f

<400> 117
ttcaaatccc aagttccgtg ag 22

<210> 118
<211> 21
<212> DNA
<213> artificial sequence

<220>
<223> ephrin-A2f

<400> 118
aactcttcac cccctttcc c 21

<210> 119
<211> 24
<212> DNA
<213> artificial sequence

<220>
<223> ephrin-A3f

<400> 119
tacatctcca cgcccactca caac 24

<210> 120
<211> 20
<212> DNA
<213> artificial sequence

<220>
<223> ephrin-A4f

<400> 120
cgatgccttt tgcccctttc 20

<210> 121

<211>  21
<212>  DNA
<213>  artificial sequence

<220>
<223>  ephrin-A5f

<400>  121
aacagcagca accccagatt c                                                21


<210>  122
<211>  25
<212>  DNA
<213>  artificial sequence

<220>
<223>  EphA1f

<400>  122
gccttacgcc aactacacat ttacc                                            25


<210>  123
<211>  22
<212>  DNA
<213>  artificial sequence

<220>
<223>  EphA2f

<400>  123
tgaggatgtc cgtttttcca ag                                               22


<210>  124
<211>  22
<212>  DNA
<213>  artificial sequence

<220>
<223>  EphA3f

<400>  124
gcaatgctgg gtatgaagaa cg                                               22


<210>  125
<211>  22
<212>  DNA
<213>  artificial sequence

<220>
<223>  EphA4f

<400>  125
gaacaactgg ctgcgaactg ac                                               22


<210>  126
<211>  22
<212>  DNA
<213>  artificial sequence

<220>
<223>  EphA5f

<400>  126

gcaagtatta tggggcagtt cg                                                22

<210>  127
<211>  22
<212>  DNA
<213>  artificial sequence

<220>
<223>  EphA6f

<400>  127
tcctctttgg ttgaagtgcg gg                                                22

<210>  128
<211>  24
<212>  DNA
<213>  artificial sequence

<220>
<223>  EphA7f

<400>  128
agcagtctcc agtgaacaga atcc                                              24

<210>  129
<211>  24
<212>  DNA
<213>  artificial sequence

<220>
<223>  EphA8f

<400>  129
tccatcaacg aggtagacga gtcc                                              24

<210>  130
<211>  24
<212>  DNA
<213>  artificial sequence

<220>
<223>  ephrin-A1r

<400>  130
cagcagcagt ggtaggagca atac                                              24

<210>  131
<211>  24
<212>  DNA
<213>  artificial sequence

<220>
<223>  ephrin-A2r

<400>  131
ctcattggtt ggacgcacat aaac                                              24

<210>  132
<211>  22
<212>  DNA
<213>  artificial sequence

<220>
<223>  ephrin-A3r

<400>  132
tccctcaaag tcttccaaca cg                                                22


<210>  133
<211>  22
<212>  DNA
<213>  artificial sequence

<220>
<223>  ephrin-A4r

<400>  133
atgtgatgac ccgctctcct tg                                                22


<210>  134
<211>  24
<212>  DNA
<213>  artificial sequence

<220>
<223>  ephrin-A5r

<400>  134
aagcatcgcc aggaggaaca gtag                                              24


<210>  135
<211>  24
<212>  DNA
<213>  artificial sequence

<220>
<223>  EphA1r

<400>  135
gtccacatag ggttttagcc acag                                              24


<210>  136
<211>  22
<212>  DNA
<213>  artificial sequence

<220>
<223>  EphA2r

<400>  136
tgctgttgac gaggatgttg cg                                                22


<210>  137
<211>  22
<212>  DNA
<213>  artificial sequence

<220>
<223>  EphA3r

<400>  137
tagttgtgat gctgactgcg gc                                                22


<210>  138

<211>  22
<212>  DNA
<213>  artificial sequence

<220>
<223>  EphA4r

<400>  138
ttcaccattc tgctcctcgt gc                                                22


<210>  139
<211>  22
<212>  DNA
<213>  artificial sequence

<220>
<223>  EphA5r

<400>  139
atagagagca gcagggcaat cc                                                22


<210>  140
<211>  22
<212>  DNA
<213>  artificial sequence

<220>
<223>  EphA6r

<400>  140
cagtgtgtct tgggatgaag cg                                                22


<210>  141
<211>  24
<212>  DNA
<213>  artificial sequence

<220>
<223>  EphA7r

<400>  141
atcccagcgg caatacctct caac                                              24


<210>  142
<211>  24
<212>  DNA
<213>  artificial sequence

<220>
<223>  EphA8r

<400>  142
ggggcacttc ttgtagtaga ttcg                                              24


<210>  143
<211>  42
<212>  DNA
<213>  artificial sequence

<220>
<223>  ephrin-A5 siRNA 1f

<400>  143

attaatacga ctcactatag gcaagccagg gttgatgagt ag 42

<210> 144
<211> 41
<212> DNA
<213> artificial sequence

<220>
<223> ephrin-A5 siRNA 2f

<400> 144
attaatacga ctcactatag gagctgattg ccaggaaaca c 41

<210> 145
<211> 45
<212> DNA
<213> artificial sequence

<220>
<223> ephrin-A5 siRNA 1r

<400> 145
cgtaatacga ctcactatag gtcacaagtt taaataggac aagca 45

<210> 146
<211> 41
<212> DNA
<213> artificial sequence

<220>
<223> ephrin-A5 siRNA 2r

<400> 146
cgtaatacga ctcactatag gaaatgttgg gtgccttctg t 41

<210> 147
<211> 23
<212> DNA
<213> artificial sequence

<220>
<223> ephrin-A5f

<400> 147
tgttgacgct gctctttctg gtg 23

<210> 148
<211> 21
<212> DNA
<213> artificial sequence

<220>
<223> a-tubulinf

<400> 148
ctttgagcca gccaaccaga t 21

<210> 149
<211> 22
<212> DNA
<213> artificial sequence

<220>
<223>  ephrin-A5r

<400>  149
aatctggggt tgctgctgtt cc                                                22


<210>  150
<211>  19
<212>  DNA
<213>  artificial sequence

<220>
<223>  a-tubulinr

<400>  150
ttgatggtgg caatggcag                                                    19


<210>  151
<211>  39
<212>  DNA
<213>  artificial sequence

<220>
<223>  Cx36 siRNA 1f

<400>  151
attaatacga ctcactatag gagggcaggt ttggggaag                              39


<210>  152
<211>  41
<212>  DNA
<213>  artificial sequence

<220>
<223>  Cx36 siRNA 2f

<400>  152
attaatacga ctcactatag ggcatgccag cttttctttt t                           41


<210>  153
<211>  41
<212>  DNA
<213>  artificial sequence

<220>
<223>  Cx36 siRNA 1r

<400>  153
cgtaatacga ctcactatag gggccagatg aaggaaaaag a                           41


<210>  154
<211>  41
<212>  DNA
<213>  artificial sequence

<220>
<223>  Cx36 siRNA 2r

<400>  154
cgtaatacga ctcactatag gcgggctgat taaggtctct g                           41


<210>  155

<211>  20
<212>  DNA
<213>  artificial sequence

<220>
<223>  Cx36f

<400>  155
acggtgtacg atgatgagca                                                    20


<210>  156
<211>  20
<212>  DNA
<213>  artificial sequence

<220>
<223>  Cx36r

<400>  156
gtagagtacc ggcgttctcg                                                    20